# EUROPEAN PATENT SPECIFICATION

(11) **EP 1 311 682 B1**
(45) Date of publication and mention of the grant of the patent: **12.12.2007**
(21) Application number: 01959607.1
(22) Date of filing: 07.08.2001
(51) Int. Cl.: C12N 15/31, C07K 14/20, C12N 15/62, C07K 19/00, C12N 15/63, A61K 48/00, A61K 39/02, G01N 33/53

(54) **RECOMBINANT CONSTRUCTS OF BORRELIA BURGDORFERI**
BORRELIA BURGDORFERI REKOMBINANTE GENKONSTRUKTE
CONSTRUCTIONS RECOMBINANTES DE BORRELIA BURGDORFERI

(30) Priority: 18.08.2000 US 226484 P; 19.09.2000 US 666017
(43) Date of publication of application: 21.05.2003
(73) Proprietor: RESEARCH FOUNDATION OF STATE UNIVERSITY OF NEW YORK, Stony Brook, NY 11794-3369 (US); Brookhaven Sciences Associates, LLC, Upton, New York 11973-5000 (US)
(72) Inventor: LUFT, Benjamin, J., East Setauket, NY 11733-3951 (US); DUNN, John, J., Bellport, NY 11713-2417 (US)
(74) Representative: Kirkham, Nicholas Andrew
(86) International application number: PCT/US2001/024736
(87) International publication number: WO 2002/016422

(56) References cited:
- WO-A-00/06745
- WO-A-95/12676
- MARIA J.C. GOMES-SOLECKI ET AL.: "Recombinant chimeric Borrelia proteins for diagnosis of Lyme disease" JOURNAL OF CLINICAL MICROBIOLOGY, vol. 38, no. 7, July 2000 (2000-07), pages 2530-2535, XP002205998
- R.S. KALISH ET AL.: "Lyme disease: Human T-cell response to OspA and OspC Borrelia lipoproteins includes both CD8+ and CD4+ T-Cells" THE JOURNAL OF INVESTIGATIVE DERMATOLOGY, vol. 114, no. 4, April 2000 (2000-04), page 836 XP008004711
- REINHARD WALLICHT ET AL.: "DNA vaccines expressing a fusion product of outer surface proteins A and C from Borrelia burgdorferi induce protective antibodies suitable for prophylaxis but not for resolution of Lyme disease" INFECTION AND IMMUNITY, vol. 69, no. 4, April 2001 (2001-04), pages 2130-2136, XP002205999
- B.J. LUFT ET AL.: "A new multi-target OspA-OspC vaccine for Lyme disease" ABSTRACTS OF THE INTERSCIENCE CONFERENCE ON ANTIMICROBIAL AGENTS AND CHEMOTHERAPY, vol. 40, 17 - 20 September 2000, page 248 XP008004693

### SEQUENCE LISTING

<110> Research Foundation of the State University of New York
   Brookhaven Sciences Associates, LLC
   Benjamin J. Luft
   John J. Dunn
<120> Recombinant Constructs of Borrelia burgdorferi
<130> 2631.1001010
<140> PCT/US01/24736
   <141> 2001-08-07
<150> US 60/226,484
   <151> 2000-08-18
<150> US 09/666,017
   <151> 2000-09-19
<160> 186
<170> FastSEQ for Windows Version 4.0
<210> 1
   <211> 23
   <212> DNA
   <213> Artificial Sequence
<220>
   <223> Oligonucleotide Primer
<400> 1
   cttaatgact ctgacactag tgc 23
<210> 2
   <211> 30
   <212> DNA
   <213> Artificial Sequence
<220>
   <223> Oligonucleotide Primer
<400> 2
   gctactaaaa aaaccgggaa atggaattca 30
<210> 3
   <211> 33
   <212> DNA
   <213> Artificial Sequence
<220>
   <223> Oligonucleotide Primer
<400> 3
   gcagcttggg attcaaaaac atccacttta aca 33
<210> 4
   <211> 18
   <212> DNA
   <213> Artificial Sequence
<220>
   <223> Oligonucleotide Primer
<400> 4
   ggagaatata ttatgaaa 18
<210> 5
   <211> 17
   <212> DNA
   <213> Artificial Sequence
<220>
   <223> Oligonucleotide Primer
<400> 5
   ctccttattt taaagcg 17
<210> 6
   <211> 822
   <212> DNA
   <213> Borrelia burgdorferi
<220>
   <221> CDS
   <222> (1)... (822)
<400> 6
   atg aaa aaa tat tta ttg gga ata ggt cta ata tta gcc tta ata gca    48
   Met Lys Lys Tyr Leu Leu Gly Ile Gly Leu Ile Leu Ala Leu Ile Ala
    1               5                  10                  15
   tgt aag caa aat gtt agc agc ctt gac gag aaa aac agc gtt tca gta    96
   Cys Lys Gln Asn Val Ser Ser Leu Asp Glu Lys Asn Ser Val Ser Val
               20                  25                  30
   gat ttg cct ggt gaa atg aaa gtt ctt gta agc aaa gaa aaa aac aaa    144
   Asp Leu Pro Gly Glu Met Lys Val Leu Val Ser Lys Glu Lys Asn Lys
           35                  40                  45
   gac ggc aag tac gat cta att gca aca gta gac aag ctt gag ctt aaa    192
   Asp Gly Lys Tyr Asp Leu Ile Ala Thr Val Asp Lys Leu Glu Leu Lys
       50                  55                  60
   gga act tct gat aaa aac aat gga tct gga gta ctt gaa ggc gta aaa    240
   Gly Thr Ser Asp Lys Asn Asn Gly Ser Gly Val Leu Glu Gly Val Lys
   65                  70                  75                  80
   gct gac aaa agt aaa gta aaa tta aca att tct gac gat cta ggt caa    288
   Ala Asp Lys Ser Lys Val Lys Leu Thr Ile Ser Asp Asp Leu Gly Gln
                   85                  90                  95
   acc aca ctt gaa gtt ttc aaa gaa gat ggc aaa aca cta gta tca aaa    336
   Thr Thr Leu Glu Val Phe Lys Glu Asp Gly Lys Thr Leu Val Ser Lys
               100                 105                 110
   aaa gta act tcc aaa gac aag tca tca aca gaa gaa aaa ttc aat gaa      384
   Lys Val Thr Ser Lys Asp Lys Ser Ser Thr Glu Glu Lys Phe Asn Glu
           115                 120                 125
   aaa ggt gaa gta tct gaa aaa ata ata aca aga gca gac gga acc aga      432
   Lys Gly Glu Val Ser Glu Lys Ile Ile Thr Arg Ala Asp Gly Thr Arg
       130                 135                 140
   ctt gaa tac aca gga att aaa agc gat gga tct gga aaa gct aaa gag      480
   Leu Glu Tyr Thr Gly Ile Lys Ser Asp Gly Ser Gly Lys Ala Lys Glu
   145                 150                 155                 160
   gtt tta aaa ggc tat gtt ctt gaa gga act cta act gct gaa aaa aca      528
   Val Leu Lys Gly Tyr Val Leu Glu Gly Thr Leu Thr Ala Glu Lys Thr
                   165                 170                 175
   aca ttg gtg gtt aaa gaa gga act gtt act tta agc aaa aat att tca      576
   Thr Leu Val Val Lys Glu Gly Thr Val Thr Leu Ser Lys Asn Ile Ser
               180                 185                 190
   aaa tct ggg gaa gtt tca gtt gaa ctt aat gac act gac agt agt gct      624
   Lys Ser Gly Glu Val Ser Val Glu Leu Asn Asp Thr Asp Ser Ser Ala
           195                 200                 205
   gct act aaa aaa act gca gct tgg aat tca ggc act tca act tta aca      672
   Ala Thr Lys Lys Thr Ala Ala Trp Asn Ser Gly Thr Ser Thr Leu Thr
       210                 215                 220
   att act gta aac agt aaa aaa act aaa gac ctt gtg ttt aca aaa gaa      720
   Ile Thr Val Asn Ser Lys Lys Thr Lys Asp Leu Val Phe Thr Lys Glu
   225                 230                 235                 240
   aac aca att aca gta caa caa tac gac tca aat ggc acc aaa tta gag      768
   Asn Thr Ile Thr Val Gln Gln Tyr Asp Ser Asn Gly Thr Lys Leu Glu
                   245                 250                 255
   ggg tca gca gtt gaa att aca aaa ctt gat gaa att aaa aac gct tta      816
   Gly Ser Ala Val Glu Ile Thr Lys Leu Asp Glu Ile Lys Asn Ala Leu
               260                 265                 270
   aaa taa                                                              822
   Lys  *
<210> 7
   <211> 273
   <212> PRT
   <213> Borrelia burgdorferi
<400> 7
   Met Lys Lys Tyr Leu Leu Gly Ile Gly Leu Ile Leu Ala Leu Ile Ala
    1               5                  10                  15
   Cys Lys Gln Asn Val Ser Ser Leu Asp Glu Lys Asn Ser Val Ser Val
               20                  25                  30
   Asp Leu Pro Gly Glu Met Lys Val Leu Val Ser Lys Glu Lys Asn Lys
           35                  40                  45
   Asp Gly Lys Tyr Asp Leu Ile Ala Thr Val Asp Lys Leu Glu Leu Lys
       50                  55                  60
   Gly Thr Ser Asp Lys Asn Asn Gly Ser Gly Val Leu Glu Gly Val Lys
   65                  70                  75                  80
   Ala Asp Lys Ser Lys Val Lys Leu Thr Ile Ser Asp Asp Leu Gly Gln
                   85                  90                  95
   Thr Thr Leu Glu Val Phe Lys Glu Asp Gly Lys Thr Leu Val Ser Lys
               100                 105                 110
   Lys Val Thr Ser Lys Asp Lys Ser Ser Thr Glu Glu Lys Phe Asn Glu
           115                 120                 125
   Lys Gly Glu Val Ser Glu Lys Ile Ile Thr Arg Ala Asp Gly Thr Arg
       130                 135                 140
   Leu Glu Tyr Thr Gly Ile Lys Ser Asp Gly Ser Gly Lys Ala Lys Glu
   145                 150                 155                 160
   Val Leu Lys Gly Tyr Val Leu Glu Gly Thr Leu Thr Ala Glu Lys Thr
                   165                 170                 175
   Thr Leu Val Val Lys Glu Gly Thr Val Thr Leu Ser Lys Asn Ile Ser
               180                 185                 190
   Lys Ser Gly Glu Val Ser Val Glu Leu Asn Asp Thr Asp Ser Ser Ala
           195                 200                 205
   Ala Thr Lys Lys Thr Ala Ala Trp Asn Ser Gly Thr Ser Thr Leu Thr
       210                 215                 220
   Ile Thr Val Asn Ser Lys Lys Thr Lys Asp Leu Val Phe Thr Lys Glu
   225                 230                 235                 240
   Asn Thr Ile Thr Val Gln Gln Tyr Asp Ser Asn Gly Thr Lys Leu Glu
                   245                 250                 255
   Gly Ser Ala Val Glu Ile Thr Lys Leu Asp Glu Ile Lys Asn Ala Leu
               260                 265                 270
   Lys
<210> 8
   <211> 825
   <212> DNA
   <213> Borrelia burgdorferi
<220>
   <221> CDS
   <222> (1)...(825)
<400> 8
   atg aaa aaa tat tta ttg gga ata ggt cta ata tta gcc tta ata gca      48
   Met Lys Lys Tyr Leu Leu Gly Ile Gly Leu Ile Leu Ala Leu Ile Ala
    1               5                  10                  15
   tgt aag caa aat gtt agc agc ctt gat gaa aaa aat agc gtt tca gta      96
   Cys Lys Gln Asn Val Ser Ser Leu Asp Glu Lys Asn Ser Val Ser Val
               20                  25                  30
   gat tta cct ggt gga atg aca gtt ctt gta agt aaa gaa aaa gac aaa     144
   Asp Leu Pro Gly Gly Met Thr Val Leu Val Ser Lys Glu Lys Asp Lys
           35                  40                  45
   gac ggt aaa tac agt cta gag gca aca gta gac aag ctt gag ctt aaa     192
   Asp Gly Lys Tyr Ser Leu Glu Ala Thr Val Asp Lys Leu Glu Leu Lys
       50                  55                  60
   gga act tct gat aaa aac aac ggt tct gga aca ctt gaa ggt gaa aaa     240
   Gly Thr Ser Asp Lys Asn Asn Gly Ser Gly Thr Leu Glu Gly Glu Lys
   65                  70                  75                  80
   act gac aaa agt aaa gta aaa tta aca att gct gat gac cta agt caa     288
   Thr Asp Lys Ser Lys Val Lys Leu Thr Ile Ala Asp Asp Leu Ser Gln
                   85                  90                  95
   act aaa ttt gaa att ttc aaa gaa gat gcc aaa aca tta gta tca aaa    336
   Thr Lys Phe Glu Ile Phe Lys Glu Asp Ala Lys Thr Leu Val Ser Lys
               100                 105                 110
   aaa gta acc ctt aaa gac aag tca tca aca gaa gaa aaa ttc aac gaa    384
   Lys Val Thr Leu Lys Asp Lys Ser Ser Thr Glu Glu Lys Phe Asn Glu
           115                 120                 125
   aag ggt gaa aca tct gaa aaa aca ata gta aga gca aat gga acc aga    432
   Lys Gly Glu Thr Ser Glu Lys Thr Ile Val Arg Ala Asn Gly Thr Arg
       130                 135                 140
   ctt gaa tac aca gac ata aaa agc gat gga tcc gga aaa gct aaa gaa    480
   Leu Glu Tyr Thr Asp Ile Lys Ser Asp Gly Ser Gly Lys Ala Lys Glu
   145                 150                 155                 160
   gtt tta aaa gac ttt act ctt gaa gga act cta gct gct gac ggc aaa    528
   Val Leu Lys Asp Phe Thr Leu Glu Gly Thr Leu Ala Ala Asp Gly Lys
                   165                 170                 175
   aca aca ttg aaa gtt aca gaa ggc act gtt gtt tta agc aag aac att    576
   Thr Thr Leu Lys Val Thr Glu Gly Thr Val Val Leu Ser Lys Asn Ile
               180                 185                 190
   tta aaa tcc gga gaa ata aca gtt gca ctt gat gac tct gac act act    624
   Leu Lys Ser Gly Glu Ile Thr Val Ala Leu Asp Asp Ser Asp Thr Thr
           195                 200                 205
   cag gct act aaa aaa act gga aaa tgg gat tca aaa act tcc act tta    672
   Gln Ala Thr Lys Lys Thr Gly Lys Trp Asp Ser Lys Thr Ser Thr Leu
       210                 215                 220
   aca att agt gtg aat agc caa aaa acc aaa aac ctt gta ttc aca aaa    720
   Thr Ile Ser Val Asn Ser Gln Lys Thr Lys Asn Leu Val Phe Thr Lys
   225                 230                 235                 240
   gaa gac aca ata aca gta caa aaa tac gac tca gca ggc acc aat cta    768
   Glu Asp Thr Ile Thr Val Gln Lys Tyr Asp Ser Ala Gly Thr Asn Leu
                   245                 250                 255
   gaa ggc aaa gca gtc gaa att aca aca ctt aaa gaa ctt aaa aac gct    816
   Glu Gly Lys Ala Val Glu Ile Thr Thr Leu Lys Glu Leu Lys Asn Ala
               260                 265                 270
   tta aaa taa                                                        825
   Leu Lys  *
<210> 9
   <211> 274
   <212> PRT
   <213> Borrelia burgdorferi
<400> 9
   Met Lys Lys Tyr Leu Leu Gly Ile Gly Leu Ile Leu Ala Leu Ile Ala
    1               5                  10                  15
   Cys Lys Gln Asn Val Ser Ser Leu Asp Glu Lys Asn Ser Val Ser Val
               20                  25                  30
   Asp Leu Pro Gly Gly Met Thr Val Leu Val Ser Lys Glu Lys Asp Lys
           35                  40                  45
   Asp Gly Lys Tyr Ser Leu Glu Ala Thr Val Asp Lys Leu Glu Leu Lys
       50                  55                  60
   Gly Thr Ser Asp Lys Asn Asn Gly Ser Gly Thr Leu Glu Gly Glu Lys
   65                  70                  75                  80
   Thr Asp Lys Ser Lys Val Lys Leu Thr Ile Ala Asp Asp Leu Ser Gln
                   85                  90                  95
   Thr Lys Phe Glu Ile Phe Lys Glu Asp Ala Lys Thr Leu Val Ser Lys
               100                 105                 110
   Lys Val Thr Leu Lys Asp Lys Ser Ser Thr Glu Glu Lys Phe Asn Glu
           115                 120                 125
   Lys Gly Glu Thr Ser Glu Lys Thr Ile Val Arg Ala Asn Gly Thr Arg
       130                 135                 140
   Leu Glu Tyr Thr Asp Ile Lys Ser Asp Gly Ser Gly Lys Ala Lys Glu
   145                 150                 155                 160
   Val Leu Lys Asp Phe Thr Leu Glu Gly Thr Leu Ala Ala Asp Gly Lys
                   165                 170                 175
   Thr Thr Leu Lys Val Thr Glu Gly Thr Val Val Leu Ser Lys Asn Ile
               180                 185                 190
   Leu Lys Ser Gly Glu Ile Thr Val Ala Leu Asp Asp Ser Asp Thr Thr
           195                 200                 205
   Gln Ala Thr Lys Lys Thr Gly Lys Trp Asp Ser Lys Thr Ser Thr Leu
       210                 215                 220
   Thr Ile Ser Val Asn Ser Gln Lys Thr Lys Asn Leu Val Phe Thr Lys
   225                 230                 235                 240
   Glu Asp Thr Ile Thr Val Gln Lys Tyr Asp Ser Ala Gly Thr Asn Leu
                   245                 250                 255
   Glu Gly Lys Ala Val Glu Ile Thr Thr Leu Lys Glu Leu Lys Asn Ala
               260                 265                 270
   Leu Lys
<210> 10
   <211> 822
   <212> DNA
   <213> Borrelia burgdorferi
<220>
   <221> CDS
   <222> (1)...(822)
<400> 10
   atg aaa aaa tat tta ttg gga ata ggt cta ata tta gcc tta ata gca    48
   Met Lys Lys Tyr Leu Leu Gly Ile Gly Leu Ile Leu Ala Leu Ile Ala
    1               5                  10                  15
   tgc aag caa aat gtt agc agc ctt gat gaa aaa aac agc gct tca gta    96
   Cys Lys Gln Asn Val Ser Ser Leu Asp Glu Lys Asn Ser Ala Ser Val
               20                  25                  30
   gat ttg cct ggt gag atg aaa gtt ctt gta agt aaa gaa aaa gac aaa    144
   Asp Leu Pro Gly Glu Met Lys Val Leu Val Ser Lys Glu Lys Asp Lys
           35                  40                  45
   gac ggt aag tac agt cta aag gca aca gta gac aag att gag cta aaa    192
   Asp Gly Lys Tyr Ser Leu Lys Ala Thr Val Asp Lys Ile Glu Leu Lys
       50                  55                  60
   gga act tct gat aaa gac aat ggt tct gga gtg ctt gaa ggt aca aaa    240
   Gly Thr Ser Asp Lys Asp Asn Gly Ser Gly Val Leu Glu Gly Thr Lys
   65                  70                  75                  80
   gat gac aaa agt aaa gca aaa tta aca att gct gac gat cta agt aaa      288
   Asp Asp Lys Ser Lys Ala Lys Leu Thr Ile Ala Asp Asp Leu Ser Lys
                   85                  90                  95
   acc aca ttc gaa ctt tta aaa gaa gat ggc aaa aca tta gtg tca aga      336
   Thr Thr Phe Glu Leu Leu Lys Glu Asp Gly Lys Thr Leu Val Ser Arg
               100                 105                 110
   aaa gta agt tct aga gac aaa aca tca aca gat gaa atg ttc aat gaa      384
   Lys Val Ser Ser Arg Asp Lys Thr Ser Thr Asp Glu Met Phe Asn Glu
           115                 120                 125
   aaa ggt gaa ttg tct gca aaa acc atg aca aga gaa aat gga acc aaa      432
   Lys Gly Glu Leu Ser Ala Lys Thr Met Thr Arg Glu Asn Gly Thr Lys
       130                 135                 140
   ctt gaa tat aca gaa atg aaa agc gat gga acc gga aaa gct aaa gaa      480
   Leu Glu Tyr Thr Glu Met Lys Ser Asp Gly Thr Gly Lys Ala Lys Glu
   145                 150                 155                 160
   gtt tta aaa aag ttt act ctt gaa gga aaa gta gct aat gat aaa gta      528
   Val Leu Lys Lys Phe Thr Leu Glu Gly Lys Val Ala Asn Asp Lys Val
                   165                 170                 175
   aca ttg gaa gta aaa gaa gga acc gtt act tta agt aag gaa att gca      576
   Thr Leu Glu Val Lys Glu Gly Thr Val Thr Leu Ser Lys Glu Ile Ala
               180                 185                 190
   aaa tct gga gaa gta aca gtt gct ctt aat gac act aac act act cag      624
   Lys Ser Gly Glu Val Thr Val Ala Leu Asn Asp Thr Asn Thr Thr Gln
           195                 200                 205
   gct act aaa aaa act ggc gca tgg gat tca aaa act tct act tta aca      672
   Ala Thr Lys Lys Thr Gly Ala Trp Asp Ser Lys Thr Ser Thr Leu Thr
       210                 215                 220
   att agt gtt aac agc aaa aaa act aca caa ctt gtg ttt act aaa caa      720
   Ile Ser Val Asn Ser Lys Lys Thr Thr Gln Leu Val Phe Thr Lys Gln
   225                 230                 235                 240
   tac aca ata act gta aaa caa tac gac tcc gca ggt acc aat tta gaa      768
   Tyr Thr Ile Thr Val Lys Gln Tyr Asp Ser Ala Gly Thr Asn Leu Glu
                   245                 250                 255
   ggc aca gca gtc gaa att aaa aca ctt gat gaa ctt aaa aac gct tta      816
   Gly Thr Ala Val Glu Ile Lys Thr Leu Asp Glu Leu Lys Asn Ala Leu
               260                 265                 270
   aaa taa                                                              822
   Lys  *
<210> 11
   <211> 273
   <212> PRT
   <213> Borrelia burgdorferi
<400> 11
   Met Lys Lys Tyr Leu Leu Gly Ile Gly Leu Ile Leu Ala Leu Ile Ala
   1               5                   10                  15
   Cys Lys Gln Asn Val Ser Ser Leu Asp Glu Lys Asn Ser Ala Ser Val
               20                  25                  30
   Asp Leu Pro Gly Glu Met Lys Val Leu Val Ser Lys Glu Lys Asp Lys
           35                  40                  45
   Asp Gly Lys Tyr Ser Leu Lys Ala Thr Val Asp Lys Ile Glu Leu Lys
       50                  55                  60
   Gly Thr Ser Asp Lys Asp Asn Gly Ser Gly Val Leu Glu Gly Thr Lys
   65                  70                  75                  80
   Asp Asp Lys Ser Lys Ala Lys Leu Thr Ile Ala Asp Asp Leu Ser Lys
                   85                  90                  95
   Thr Thr Phe Glu Leu Leu Lys Glu Asp Gly Lys Thr Leu Val Ser Arg
               100                 105                 110
   Lys Val Ser Ser Arg Asp Lys Thr Ser Thr Asp Glu Met Phe Asn Glu
           115                 120                 125
   Lys Gly Glu Leu Ser Ala Lys Thr Met Thr Arg Glu Asn Gly Thr Lys
       130                 135                 140
   Leu Glu Tyr Thr Glu Met Lys Ser Asp Gly Thr Gly Lys Ala Lys Glu
   145                 150                 155                 160
   Val Leu Lys Lys Phe Thr Leu Glu Gly Lys Val Ala Asn Asp Lys Val
                   165                 170                 175
   Thr Leu Glu Val Lys Glu Gly Thr Val Thr Leu Ser Lys Glu Ile Ala
               180                 185                 190
   Lys Ser Gly Glu Val Thr Val Ala Leu Asn Asp Thr Asn Thr Thr Gln
           195                 200                 205
   Ala Thr Lys Lys Thr Gly Ala Trp Asp Ser Lys Thr Ser Thr Leu Thr
       210                 215                 220
   Ile Ser Val Asn Ser Lys Lys Thr Thr Gln Leu Val Phe Thr Lys Gln
   225                 230                 235                 240
   Tyr Thr Ile Thr Val Lys Gln Tyr Asp Ser Ala Gly Thr Asn Leu Glu
                   245                 250                 255
   Gly Thr Ala Val Glu Ile Lys Thr Leu Asp Glu Leu Lys Asn Ala Leu
               260                 265                 270
   Lys
<210> 12
   <211> 819
   <212> DNA
   <213> Borrelia burgdorferi
<220>
   <221> CDS
   <222> (1)...(819)
<400> 12
   atg aaa aaa tat tta ttg gga ata ggt cta ata tta gct tta ata gca    48
   Met Lys Lys Tyr Leu Leu Gly Ile Gly Leu Ile Leu Ala Leu Ile Ala
    1               5                  10                  15
   tgt aag caa aat gtt agc agc ctt gac gag aaa aac agc gtt tca gta    96
   Cys Lys Gln Asn Val Ser Ser Leu Asp Glu Lys Asn Ser Val Ser Val
               20                  25                  30
   gat ttg cct ggt gaa atg aaa gtt ctt gta agc aaa gaa aaa gac aaa   144
   Asp Leu Pro Gly Glu Met Lys Val Leu Val Ser Lys Glu Lys Asp Lys
           35                  40                  45
   gac ggc aag tac agt cta atg gca aca gta gac aag ctt gag ctt aaa   192
   Asp Gly Lys Tyr Ser Leu Met Ala Thr Val Asp Lys Leu Glu Leu Lys
       50                  55                  60
   gga aca tct gat aaa aac aat gga tct ggg gtg ctt gaa ggc gta aaa    240
   Gly Thr Ser Asp Lys Asn Asn Gly Ser Gly Val Leu Glu Gly Val Lys
    65                 70                  75                  80
   gct gac aaa agc aaa gta aaa tta aca gtt tct gac gat cta agc aca    288
   Ala Asp Lys Ser Lys Val Lys Leu Thr Val Ser Asp Asp Leu Ser Thr
                   85                  90                  95
   acc aca ctt gaa gtt tta aaa gaa gat ggc aaa aca tta gtg tca aaa    336
   Thr Thr Leu Glu Val Leu Lys Glu Asp Gly Lys Thr Leu Val Ser Lys
               100                 105                 110
   aaa aga act tct aaa gat aag tca tca aca gaa gaa aag ttc aat gaa    384
   Lys Arg Thr Ser Lys Asp Lys Ser Ser Thr Glu Glu Lys Phe Asn Glu
           115                 120                 125
   aaa ggc gaa tta gtt gaa aaa ata atg gca aga gca aac gga acc ata    432
   Lys Gly Glu Leu Val Glu Lys Ile Met Ala Arg Ala Asn Gly Thr Ile
       130                 135                 140
   ctt gaa tac aca gga att aaa agc gat gga tcc gga aaa gct aaa gaa    480
   Leu Glu Tyr Thr Gly Ile Lys Ser Asp Gly Ser Gly Lys Ala Lys Glu
   145                 150                 155                 160
   act tta aaa gaa tat gtt ctt gaa gga act cta act gct gaa aaa gca    528
   Thr Leu Lys Glu Tyr Val Leu Glu Gly Thr Leu Thr Ala Glu Lys Ala
                   165                 170                 175
   aca ttg gtg gtt aaa gaa gga act gtt act tta agt aag cac att tca    576
   Thr Leu Val Val Lys Glu Gly Thr Val Thr Leu Ser Lys His Ile Ser
               180                 185                 190
   aaa tct gga gaa gta aca gct gaa ctt aat gac act gac agt act caa    624
   Lys Ser Gly Glu Val Thr Ala Glu Leu Asn Asp Thr Asp Ser Thr Gln
           195                 200                 205
   gct act aaa aaa act ggg aaa tgg gat gca ggc act tca act tta aca    672
   Ala Thr Lys Lys Thr Gly Lys Trp Asp Ala Gly Thr Ser Thr Leu Thr
       210                 215                 220
   att act gta aac aac aaa aaa act aaa gcc ctt gta ttt aca aaa caa    720
   Ile Thr Val Asn Asn Lys Lys Thr Lys Ala Leu Val Phe Thr Lys Gln
   225                 230                 235                 240
   gac aca att aca tca caa aaa tac gac tca gca gga acc aac ttg gaa    768
   Asp Thr Ile Thr Ser Gln Lys Tyr Asp Ser Ala Gly Thr Asn Leu Glu
                   245                 250                 255
   ggc aca gca gtc gaa att aaa aca ctt gat gaa ctt aaa aac gct tta    816
   Gly Thr Ala Val Glu Ile Lys Thr Leu Asp Glu Leu Lys Asn Ala Leu
               260                 265                 270
   aga                                                                819
   Arg
<210> 13
   <211> 273
   <212> PRT
   <213> Borrelia burgdorferi
<400> 13
   Met Lys Lys Tyr Leu Leu Gly Ile Gly Leu Ile Leu Ala Leu Ile Ala
   1               5                   10                  15
   Cys Lys Gln Asn Val Ser Ser Leu Asp Glu Lys Asn Ser Val Ser Val
               20                  25                  30
   Asp Leu Pro Gly Glu Met Lys Val Leu Val Ser Lys Glu Lys Asp Lys
           35                  40                  45
   Asp Gly Lys Tyr Ser Leu Met Ala Thr Val Asp Lys Leu Glu Leu Lys
       50                  55                  60
   Gly Thr Ser Asp Lys Asn Asn Gly Ser Gly Val Leu Glu Gly Val Lys
   65                  70                  75                  80
   Ala Asp Lys Ser Lys Val Lys Leu Thr Val Ser Asp Asp Leu Ser Thr
                   85                  90                  95
   Thr Thr Leu Glu Val Leu Lys Glu Asp Gly Lys Thr Leu Val Ser Lys
               100                 105                 110
   Lys Arg Thr Ser Lys Asp Lys Ser Ser Thr Glu Glu Lys Phe Asn Glu
           115                 120                 125
   Lys Gly Glu Leu Val Glu Lys Ile Met Ala Arg Ala Asn Gly Thr Ile
       130                 135                 140
   Leu Glu Tyr Thr Gly Ile Lys Ser Asp Gly Ser Gly Lys Ala Lys Glu
   145                 150                 155                 160
   Thr Leu Lys Glu Tyr Val Leu Glu Gly Thr Leu Thr Ala Glu Lys Ala
                   165                 170                 175
   Thr Leu Val Val Lys Glu Gly Thr Val Thr Leu Ser Lys His Ile Ser
               180                 185                 190
   Lys Ser Gly Glu Val Thr Ala Glu Leu Asn Asp Thr Asp Ser Thr Gln
           195                 200                 205
   Ala Thr Lys Lys Thr Gly Lys Trp Asp Ala Gly Thr Ser Thr Leu Thr
       210                 215                 220
   Ile Thr Val Asn Asn Lys Lys Thr Lys Ala Leu Val Phe Thr Lys Gln
   225                 230                 235                 240
   Asp Thr Ile Thr Ser Gln Lys Tyr Asp Ser Ala Gly Thr Asn Leu Glu
                   245                 250                 255
   Gly Thr Ala Val Glu Ile Lys Thr Leu Asp Glu Leu Lys Asn Ala Leu
               260                 265                 270
   Arg
<210> 14
   <211> 21
   <212> DNA
   <213> Artificial Sequence
<220>
   <223> Oligonucleotide Primer
<400> 14
   gtctgcaaaa accatgacaa g 21
<210> 15
   <211> 22
   <212> DNA
   <213> Artificial Sequence
<220>
   <223> Oligonucleotide Primer
<400> 15
   gtcatcaaca gaagaaaaat tc 22
<210> 16
   <211> 30
   <212> DNA
   <213> Artificial Sequence
<220>
   <223> Oligonucleotide Primer
<400> 16
   ccggatccat atgaaaaaat atttattggg 30
<210> 17
   <211> 32
   <212> DNA
   <213> Artificial Sequence
<220>
   <223> Oligonucleotide Primer
<400> 17
   ccgggatcca tatggctaag caaaatgtta gc 32
<210> 18
   <211> 18
   <212> DNA
   <213> Artificial Sequence
<220>
   <223> Oligonucleotide Primer
<400> 18
   gcgttcaagt actccaga 18
<210> 19
   <211> 27
   <212> DNA
   <213> Artificial Sequence
<220>
   <223> Oligonucleotide Primer
<400> 19
   gatatctaga tcttatttta aagcgtt 27
<210> 20
   <211> 31
   <212> DNA
   <213> Artificial Sequence
<220>
   <223> Oligonucleotide Primer
<400> 20
   ggatccggtg accttttaaa gcgtttttaa t 31
<210> 21
   <211> 891
   <212> DNA
   <213> Borrelia burgdorferi
<220>
   <221> CDS
   <222> (1)...(891)
<400> 21
   atg aga tta tta ata gga ttt gct tta gcg tta gct tta ata gga tgt      48
   Met Arg Leu Leu Ile Gly Phe Ala Leu Ala Leu Ala Leu Ile Gly Cys
    1               5                  10                  15
   gca caa aaa ggt gct gag tca att ggt tct caa aaa gaa aat gat cta      96
   Ala Gln Lys Gly Ala Glu Ser Ile Gly Ser Gln Lys Glu Asn Asp Leu
               20                  25                  30
   aac ctt gaa gac tct agt aaa aaa tca cat caa aac gct aaa caa gac     144
   Asn Leu Glu Asp Ser Ser Lys Lys Ser His Gln Asn Ala Lys Gln Asp
           35                  40                  45
   ctt cct gcg gtg aca gaa gac tca gtg tct ttg ttt aat ggt aat aaa     192
   Leu Pro Ala Val Thr Glu Asp Ser Val Ser Leu Phe Asn Gly Asn Lys
       50                  55                  60
   att ttt gta agc aaa gaa aaa aat agc tcc ggc aaa tat gat tta aga     240
   Ile Phe Val Ser Lys Glu Lys Asn Ser Ser Gly Lys Tyr Asp Leu Arg
   65                  70                  75                  80
   gca aca att gat cag gtt gaa ctt aaa gga act tcc gat aaa aac aat     288
   Ala Thr Ile Asp Gln Val Glu Leu Lys Gly Thr Ser Asp Lys Asn Asn
                   85                  90                  95
   ggt tct gga acc ctt gaa ggt tca aag cct gac aag agt aaa gta aaa     336
   Gly Ser Gly Thr Leu Glu Gly Ser Lys Pro Asp Lys Ser Lys Val Lys
               100                 105                 110
   tta aca gtt tct gct gat tta aac aca gta acc tta gaa gca ttt gat     384
   Leu Thr Val Ser Ala Asp Leu Asn Thr Val Thr Leu Glu Ala Phe Asp
           115                 120                 125
   gcc agc aac caa aaa att tca agt aaa gtt act aaa aaa cag ggg tca     432
   Ala Ser Asn Gln Lys Ile Ser Ser Lys Val Thr Lys Lys Gln Gly Ser
       130                 135                 140
   ata aca gag gaa act ctc aaa gct aat aaa tta gac tca aag aaa tta     480
   Ile Thr Glu Glu Thr Leu Lys Ala Asn Lys Leu Asp Ser Lys Lys Leu
   145                 150                 155                 160
   aca aga tca aac gga act aca ctt gaa tac tca caa ata aca gat gct    528
   Thr Arg Ser Asn Gly Thr Thr Leu Glu Tyr Ser Gln Ile Thr Asp Ala
                   165                 170                 175
   gac aat gct aca aaa gca gta gaa act cta aaa aat agc att aag ctt    576
   Asp Asn Ala Thr Lys Ala Val Glu Thr Leu Lys Asn Ser Ile Lys Leu
               180                 185                 190
   gaa gga agt ctt gta gtc gga aaa aca aca gtg gaa att aaa gaa ggt    624
   Glu Gly Ser Leu Val Val Gly Lys Thr Thr Val Glu Ile Lys Glu Gly
           195                 200                 205
   act gtt act cta aaa aga gaa att gaa aaa gat gga aaa gta aaa gtc    672
   Thr Val Thr Leu Lys Arg Glu Ile Glu Lys Asp Gly Lys Val Lys Val
       210                 215                 220
   ttt ttg aat gac act gca ggt tct aac aaa aaa aca ggt aaa tgg gaa    720
   Phe Leu Asn Asp Thr Ala Gly Ser Asn Lys Lys Thr Gly Lys Trp Glu
   225                 230                 235                 240
   gac agt act agc act tta aca att agt gct gac agc aaa aaa act aaa    768
   Asp Ser Thr Ser Thr Leu Thr Ile Ser Ala Asp Ser Lys Lys Thr Lys
                   245                 250                 255
   gat ttg gtg ttc tta aca gat ggt aca att aca gta caa caa tac aac    816
   Asp Leu Val Phe Leu Thr Asp Gly Thr Ile Thr Val Gln Gln Tyr Asn
               260                 265                 270
   aca gct gga acc agc cta gaa gga tca gca agt gaa att aaa aat ctt    864
   Thr Ala Gly Thr Ser Leu Glu Gly Ser Ala Ser Glu Ile Lys Asn Leu
           275                 280                 285
   tca gag ctt aaa aac gct tta aaa taa                                891
   Ser Glu Leu Lys Asn Ala Leu Lys  *
       290                 295
<210> 22
   <211> 296
   <212> PRT
   <213> Borrelia burgdorferi
<400> 22
   Met Arg Leu Leu Ile Gly Phe Ala Leu Ala Leu Ala Leu Ile Gly Cys
   1               5                   10                  15
   Ala Gln Lys Gly Ala Glu Ser Ile Gly Ser Gln Lys Glu Asn Asp Leu
               20                  25                  30
   Asn Leu Glu Asp Ser Ser Lys Lys Ser His Gln Asn Ala Lys Gln Asp
           35                  40                  45
   Leu Pro Ala Val Thr Glu Asp Ser Val Ser Leu Phe Asn Gly Asn Lys
       50                  55                  60
   Ile Phe Val Ser Lys Glu Lys Asn Ser Ser Gly Lys Tyr Asp Leu Arg
   65                  70                  75                  80
   Ala Thr Ile Asp Gln Val Glu Leu Lys Gly Thr Ser Asp Lys Asn Asn
                   85                  90                  95
   Gly Ser Gly Thr Leu Glu Gly Ser Lys Pro Asp Lys Ser Lys Val Lys
               100                 105                 110
   Leu Thr Val Ser Ala Asp Leu Asn Thr Val Thr Leu Glu Ala Phe Asp
           115                 120                 125
   Ala Ser Asn Gln Lys Ile Ser Ser Lys Val Thr Lys Lys Gln Gly Ser
       130                 135                 140
   Ile Thr Glu Glu Thr Leu Lys Ala Asn Lys Leu Asp Ser Lys Lys Leu
   145 150 155 160
   Thr Arg Ser Asn Gly Thr Thr Leu Glu Tyr Ser Gln Ile Thr Asp Ala
   165 170 175
   Asp Asn Ala Thr Lys Ala Val Glu Thr Leu Lys Asn Ser Ile Lys Leu
   180 185 190
   Glu Gly Ser Leu Val Val Gly Lys Thr Thr Val Glu Ile Lys Glu Gly
   195 200 205
   Thr Val Thr Leu Lys Arg Glu Ile Glu Lys Asp Gly Lys Val Lys Val
   210 215 220
   Phe Leu Asn Asp Thr Ala Gly Ser Asn Lys Lys Thr Gly Lys Trp Glu
   225 230 235 240
   Asp Ser Thr Ser Thr Leu Thr Ile Ser Ala Asp Ser Lys Lys Thr Lys
   245 250 255
   Asp Leu Val Phe Leu Thr Asp Gly Thr Ile Thr Val Gln Gln Tyr Asn
   260 265 270
   Thr Ala Gly Thr Ser Leu Glu Gly Ser Ala Ser Glu Ile Lys Asn Leu
   275 280 285
   Ser Glu Leu Lys Asn Ala Leu Lys
   290 295
<210> 23
   <211> 18
   <212> DNA
   <213> Artificial Sequence
<220>
   <223> Oligonucleotide Primer
<400> 23
   ggtacaatta cagtacaa 18
<210> 24
   <211> 42
   <212> DNA
   <213> Artificial Sequence
<220>
   <223> Oligonucleotide Primer
<400> 24
   ccgagaatct catatggcac aaaaaggtgc tgagtcaatt gg 42
<210> 25
   <211> 35
   <212> DNA
   <213> Artificial Sequence
<220>
   <223> Oligonucleotide Primer
<400> 25
   ccgatatcgg atcctatttt aaagcgtttt taagc 35
<210> 26
   <211> 31
   <212> DNA
   <213> Artificial Sequence
<220>
   <223> Oligonucleotide Primer
<400> 26
   ggatccggtg accttttaaa gcgtttttaa g 31
<210> 27
   <211> 36
   <212> DNA
   <213> Artificial Sequence
<220>
   <223> Oligonucleotide Primer
<400> 27
   gtgcgcgacc atatgaaaaa gaatacatta agtgcg 36
<210> 28
   <211> 36
   <212> DNA
   <213> Artificial Sequence
<220>
   <223> Oligonucleotide Primer
<400> 28
   gtcggcggat ccttaaggtt tttttggact ttctgc 36
<210> 29
   <211> 633
   <212> DNA
   <213> Borrelia burgdorferi
<220>
   <221> CDS
   <222> (1)...(633)
<400> 29
   atg aaa aag aat aca tta agt gcg ata tta atg act tta ttt tta ttt    48
   Met Lys Lys Asn Thr Leu Ser Ala Ile Leu Met Thr Leu Phe Leu Phe
    1               5                  10                  15
   ata tct tgt aat aat tca ggg aaa gat ggg aat aca tct gca aat tct    96
   Ile Ser Cys Asn Asn Ser Gly Lys Asp Gly Asn Thr Ser Ala Asn Ser
               20                  25                  30
   gct gat gag tct gtt aaa ggg cct aat ctt aca gaa ata agt aaa aaa   144
   Ala Asp Glu Ser Val Lys Gly Pro Asn Leu Thr Glu Ile Ser Lys Lys
           35                  40                  45
   att acg gat tct aat gcg gtt tta ctt gct gtg aaa gag gtt gaa gcg   192
   Ile Thr Asp Ser Asn Ala Val Leu Leu Ala Val Lys Glu Val Glu Ala
       50                  55                  60
   ttg ctg tca tct ata gat gaa att gct gct aaa gct att ggt aaa aaa   240
   Leu Leu Ser Ser Ile Asp Glu Ile Ala Ala Lys Ala Ile Gly Lys Lys
   65                  70                  75                  80
   ata cac caa aat aat ggt ttg gat acc gaa tat aat cac aat gga tca   288
   Ile His Gln Asn Asn Gly Leu Asp Thr Glu Tyr Asn His Asn Gly Ser
                   85                  90                  95
   ttg tta gcg gga cgt tat gca ata tca acc cta ata aaa caa aaa tta   336
   Leu Leu Ala Gly Arg Tyr Ala Ile Ser Thr Leu Ile Lys Gln Lys Leu
               100                 105                 110
   gat gga ttg aaa aat gaa gga tta aag gaa aaa att gat gcg gct aag   384
   Asp Gly Leu Lys Asn Glu Gly Leu Lys Glu Lys Ile Asp Ala Ala Lys
           115                 120                 125
   aaa tgt tct gaa aca ttt act aat aaa tta aaa gaa aaa cac aca gat   432
   Lys Cys Ser Glu Thr Phe Thr Asn Lys Leu Lys Glu Lys His Thr Asp
       130                 135                 140
   ctt ggt aaa gaa ggt gtt act gat gct gat gca aaa gaa gcc att tta   480
   Leu Gly Lys Glu Gly Val Thr Asp Ala Asp Ala Lys Glu Ala Ile Leu
   145                 150                 155                 160
   aaa aca aat ggt act aaa act aaa ggt gct gaa gaa ctt gga aaa tta   528
   Lys Thr Asn Gly Thr Lys Thr Lys Gly Ala Glu Glu Leu Gly Lys Leu
                   165                 170                 175
   ttt gaa tca gta gag gtc ttg tca aaa gca gct aaa gag atg ctt gct   576
   Phe Glu Ser Val Glu Val Leu Ser Lys Ala Ala Lys Glu Met Leu Ala
               180                 185                 190
   aat tca gtt aaa gag ctt aca agc cct gtt gtg gca gaa agt cca aaa   624
   Asn Ser Val Lys Glu Leu Thr Ser Pro Val Val Ala Glu Ser Pro Lys
           195                 200                 205
   aaa cct taa                                                       633
   Lys Pro  *
       210
<210> 30
   <211> 210
   <212> PRT
   <213> Borrelia burgdorferi
<400> 30
   Met Lys Lys Asn Thr Leu Ser Ala Ile Leu Met Thr Leu Phe Leu Phe
    1               5                  10                  15
   Ile Ser Cys Asn Asn Ser Gly Lys Asp Gly Asn Thr Ser Ala Asn Ser
               20                  25                  30
   Ala Asp Glu Ser Val Lys Gly Pro Asn Leu Thr Glu Ile Ser Lys Lys
           35                  40                  45
   Ile Thr Asp Ser Asn Ala Val Leu Leu Ala Val Lys Glu Val Glu Ala
       50                  55                  60
   Leu Leu Ser Ser Ile Asp Glu Ile Ala Ala Lys Ala Ile Gly Lys Lys
   65                  70                  75                  80
   Ile His Gln Asn Asn Gly Leu Asp Thr Glu Tyr Asn His Asn Gly Ser
                   85                  90                  95
   Leu Leu Ala Gly Arg Tyr Ala Ile Ser Thr Leu Ile Lys Gln Lys Leu
               100                 105                 110
   Asp Gly Leu Lys Asn Glu Gly Leu Lys Glu Lys Ile Asp Ala Ala Lys
           115                 120                 125
   Lys Cys Ser Glu Thr Phe Thr Asn Lys Leu Lys Glu Lys His Thr Asp
       130                 135                 140
   Leu Gly Lys Glu Gly Val Thr Asp Ala Asp Ala Lys Glu Ala Ile Leu
   145                 150                 155                 160
   Lys Thr Asn Gly Thr Lys Thr Lys Gly Ala Glu Glu Leu Gly Lys Leu
                   165                 170                 175
   Phe Glu Ser Val Glu Val Leu Ser Lys Ala Ala Lys Glu Met Leu Ala
               180                 185                 190
   Asn Ser Val Lys Glu Leu Thr Ser Pro Val Val Ala Glu Ser Pro Lys
           195                 200                 205
   Lys Pro
       210
<210> 31
   <211> 630
   <212> DNA
   <213> Borrelia burgdorferi
<220>
   <221> CDS
   <222> (1)...(630)
<400> 31
   atg aaa aag aat aca tta agt gcg ata tta atg act tta ttt tta ttt      48
   Met Lys Lys Asn Thr Leu Ser Ala Ile Leu Met Thr Leu Phe Leu Phe
    1               5                  10                  15
   ata tct tgt aat aat tca ggt ggg gat acc gca tct act aat cct gat      96
   Ile Ser Cys Asn Asn Ser Gly Gly Asp Thr Ala Ser Thr Asn Pro Asp
               20                  25                  30
   gag tct gca aaa gga cct aat ctt aca gta ata agc aaa aaa att aca     144
   Glu Ser Ala Lys Gly Pro Asn Leu Thr Val Ile Ser Lys Lys Ile Thr
           35                  40                  45
   gat tct aat gca ttt gta ctg gct gtg aaa gaa gtt gag gct ttg atc     192
   Asp Ser Asn Ala Phe Val Leu Ala Val Lys Glu Val Glu Ala Leu Ile
       50                  55                  60
   tca tct ata gat gaa ctt gct aat aaa gct att ggt aaa gta ata cat     240
   Ser Ser Ile Asp Glu Leu Ala Asn Lys Ala Ile Gly Lys Val Ile His
   65                  70                  75                  80
   caa aat aat ggt tta aat gct aat gcg ggt caa aac gga tca ttg tta     288
   Gln Asn Asn Gly Leu Asn Ala Asn Ala Gly Gln Asn Gly Ser Leu Leu
                   85                  90                  95
   gca gga gcc tat gca ata tca acc cta ata aca gaa aaa tta agt aaa     336
   Ala Gly Ala Tyr Ala Ile Ser Thr Leu Ile Thr Glu Lys Leu Ser Lys
               100                 105                 110
   ttg aaa aat tca gaa gag tta aat aaa aaa att gaa gag gct aag aac     384
   Leu Lys Asn Ser Glu Glu Leu Asn Lys Lys Ile Glu Glu Ala Lys Asn
           115                 120                 125
   cat tct gaa gca ttt act aat aga cta aaa ggt tct cat gca caa ctt     432
   His Ser Glu Ala Phe Thr Asn Arg Leu Lys Gly Ser His Ala Gln Leu
       130                 135                 140
   gga gtt gct gct gct act gat gat cat gca aaa gaa gct att tta aag     480
   Gly Val Ala Ala Ala Thr Asp Asp His Ala Lys Glu Ala Ile Leu Lys
   145                 150                 155                 160
   tca aat cct act aaa gat aag ggt gct aaa gca ctt aaa gac tta tct    528
   Ser Asn Pro Thr Lys Asp Lys Gly Ala Lys Ala Leu Lys Asp Leu Ser
                   165                 170                 175
   gaa tca gta gaa agc ttg gca aaa gca gcg caa gaa gca tta gct aat    576
   Glu Ser Val Glu Ser Leu Ala Lys Ala Ala Gln Glu Ala Leu Ala Asn
               180                 185                 190
   tca gtt aaa gaa ctt aca aat cct gtt gtg gca gaa agt cca aaa aaa    624
   Ser Val Lys Glu Leu Thr Asn Pro Val Val Ala Glu Ser Pro Lys Lys
           195                 200                 205
   cct taa                                                            630
   Pro  *
<210> 32
   <211> 209
   <212> PRT
   <213> Borrelia burgdorferi
<400> 32
   Met Lys Lys Asn Thr Leu Ser Ala Ile Leu Met Thr Leu Phe Leu Phe
    1               5                  10                  15
   Ile Ser Cys Asn Asn Ser Gly Gly Asp Thr Ala Ser Thr Asn Pro Asp
               20                  25                  30
   Glu Ser Ala Lys Gly Pro Asn Leu Thr Val Ile Ser Lys Lys Ile Thr
           35                  40                  45
   Asp Ser Asn Ala Phe Val Leu Ala Val Lys Glu Val Glu Ala Leu Ile
       50                  55                  60
   Ser Ser Ile Asp Glu Leu Ala Asn Lys Ala Ile Gly Lys Val Ile His
   65                  70                  75                  80
   Gln Asn Asn Gly Leu Asn Ala Asn Ala Gly Gln Asn Gly Ser Leu Leu
                   85                  90                  95
   Ala Gly Ala Tyr Ala Ile Ser Thr Leu Ile Thr Glu Lys Leu Ser Lys
               100                 105                 110
   Leu Lys Asn Ser Glu Glu Leu Asn Lys Lys Ile Glu Glu Ala Lys Asn
           115                 120                 125
   His Ser Glu Ala Phe Thr Asn Arg Leu Lys Gly Ser His Ala Gln Leu
       130                 135                 140
   Gly Val Ala Ala Ala Thr Asp Asp His Ala Lys Glu Ala Ile Leu Lys
   145                 150                 155                 160
   Ser Asn Pro Thr Lys Asp Lys Gly Ala Lys Ala Leu Lys Asp Leu Ser
                   165                 170                 175
   Glu Ser Val Glu Ser Leu Ala Lys Ala Ala Gln Glu Ala Leu Ala Asn
               180                 185                 190
   Ser Val Lys Glu Leu Thr Asn Pro Val Val Ala Glu Ser Pro Lys Lys
           195                 200                 205
   Pro
<210> 33
   <211> 639
   <212> DNA
   <213> Borrelia burgdorferi
<220>
   <221> CDS
   <222> (1) ... (639)
<400> 33
   atg aaa aag aat aca tta agt gcg ata tta atg act tta ttt tta ttt      48
   Met Lys Lys Asn Thr Leu Ser Ala Ile Leu Met Thr Leu Phe Leu Phe
   1               5                   10                  15
   ata tct tgt agt aat tca ggg aaa ggt ggg gat tct gca tct act aat      96
   Ile Ser Cys Ser Asn Ser Gly Lys Gly Gly Asp Ser Ala Ser Thr Asn
               20                  25                  30
   cct gct gac gag tct gcg aaa ggg cct aat ctt aca gaa ata agc aaa     144
   Pro Ala Asp Glu Ser Ala Lys Gly Pro Asn Leu Thr Glu Ile Ser Lys
           35                  40                  45
   aaa att aca gat tct aat gca ttt gta ctt gct gtt aaa gaa gtt gag     192
   Lys Ile Thr Asp Ser Asn Ala Phe Val Leu Ala Val Lys Glu Val Glu
       50                  55                  60
   act ttg gtt tta tct ata gat gaa ctt gct aag aaa gct att ggt caa     240
   Thr Leu Val Leu Ser Ile Asp Glu Leu Ala Lys Lys Ala Ile Gly Gln
   65                  70                  75                  80
   aaa ata gac aat aat aat ggt tta gct gct tta aat aat cag aat gga     288
   Lys Ile Asp Asn Asn Asn Gly Leu Ala Ala Leu Asn Asn Gln Asn Gly
                   85                  90                  95
   tcg ttg tta gca gga gcc tat gca ata tca acc cta ata aca gaa aaa     336
   Ser Leu Leu Ala Gly Ala Tyr Ala Ile Ser Thr Leu Ile Thr Glu Lys
               100                 105                 110
   ttg agt aaa ttg aaa aat tta gaa gaa tta aag aca gaa att gca aag     384
   Leu Ser Lys Leu Lys Asn Leu Glu Glu Leu Lys Thr Glu Ile Ala Lys
           115                 120                 125
   gct aag aaa tgt tcc gaa gaa ttt act aat aaa cta aaa agt ggt cat     432
   Ala Lys Lys Cys Ser Glu Glu Phe Thr Asn Lys Leu Lys Ser Gly His
       130                 135                 140
   gca gat ctt ggc aaa cag gat gct acc gat gat cat gca aaa gca gct     480
   Ala Asp Leu Gly Lys Gln Asp Ala Thr Asp Asp His Ala Lys Ala Ala
   145                 150                 155                 160
   att tta aaa aca cat gca act acc gat aaa ggt gct aaa gaa ttt aaa     528
   Ile Leu Lys Thr His Ala Thr Thr Asp Lys Gly Ala Lys Glu Phe Lys
                   165                 170                 175
   gat tta ttt gaa tca gta gaa ggt ttg tta aaa gca gct caa gta gca     576
   Asp Leu Phe Glu Ser Val Glu Gly Leu Leu Lys Ala Ala Gln Val Ala
               180                 185                 190
   cta act aat tca gtt aaa gaa ctt aca agt cct gtt gta gca gaa agt     624
   Leu Thr Asn Ser Val Lys Glu Leu Thr Ser Pro Val Val Ala Glu Ser
           195                 200                 205
   cca aaa aaa cct taa                                                 639
   Pro Lys Lys Pro  *
       210
<210> 34
   <211> 212
   <212> PRT
   <213> Borrelia burgdorferi
<400> 34
   Met Lys Lys Asn Thr Leu Ser Ala Ile Leu Met Thr Leu Phe Leu Phe
    1               5                  10                  15
   Ile Ser Cys Ser Asn Ser Gly Lys Gly Gly Asp Ser Ala Ser Thr Asn
               20                  25                  30
   Pro Ala Asp Glu Ser Ala Lys Gly Pro Asn Leu Thr Glu Ile Ser Lys
           35                  40                  45
   Lys Ile Thr Asp Ser Asn Ala Phe Val Leu Ala Val Lys Glu Val Glu
       50                  55                  60
   Thr Leu Val Leu Ser Ile Asp Glu Leu Ala Lys Lys Ala Ile Gly Gln
   65                  70                  75                  80
   Lys Ile Asp Asn Asn Asn Gly Leu Ala Ala Leu Asn Asn Gln Asn Gly
                   85                  90                  95
   Ser Leu Leu Ala Gly Ala Tyr Ala Ile Ser Thr Leu Ile Thr Glu Lys
               100                 105                 110
   Leu Ser Lys Leu Lys Asn Leu Glu Glu Leu Lys Thr Glu Ile Ala Lys
           115                 120                 125
   Ala Lys Lys Cys Ser Glu Glu Phe Thr Asn Lys Leu Lys Ser Gly His
       130                 135                 140
   Ala Asp Leu Gly Lys Gln Asp Ala Thr Asp Asp His Ala Lys Ala Ala
   145                 150                 155                 160
   Ile Leu Lys Thr His Ala Thr Thr Asp Lys Gly Ala Lys Glu Phe Lys
                   165                 170                 175
   Asp Leu Phe Glu Ser Val Glu Gly Leu Leu Lys Ala Ala Gln Val Ala
               180                 185                 190
   Leu Thr Asn Ser Val Lys Glu Leu Thr Ser Pro Val Val Ala Glu Ser
           195                 200                 205
   Pro Lys Lys Pro
       210
<210> 35
   <211> 624
   <212> DNA
   <213> Borrelia burgdorferi
<220>
   <221> CDS
   <222> (1)... (624)
<400> 35
   atg aaa aag aat aca tta agt gcg ata tta atg act tta ttt tta ttt    48
   Met Lys Lys Asn Thr Leu Ser Ala Ile Leu Met Thr Leu Phe Leu Phe
    1               5                  10                  15
   ata tct tgt aat aat tca ggt ggg gat tct gca tct act aat cct gat    96
   Ile Ser Cys Asn Asn Ser Gly Gly Asp Ser Ala Ser Thr Asn Pro Asp
               20                  25                  30
   gag tct gca aaa gga cct aat ctt acc gta ata agc aaa aaa att aca     144
   Glu Ser Ala Lys Gly Pro Asn Leu Thr Val Ile Ser Lys Lys Ile Thr
           35                  40                  45
   gat tct aat gca ttt tta ctg gct gtg aaa gaa gtt gag gct ttg ctt     192
   Asp Ser Asn Ala Phe Leu Leu Ala Val Lys Glu Val Glu Ala Leu Leu
       50                  55                  60
   tca tct ata gat gaa ctt tct aaa gct att ggt aaa aaa ata aaa aat     240
   Ser Ser Ile Asp Glu Leu Ser Lys Ala Ile Gly Lys Lys Ile Lys Asn
   65                  70                  75                  80
   gat ggt act tta gat aac gaa gca aat cga aac gaa tca ttg ata gca     288
   Asp Gly Thr Leu Asp Asn Glu Ala Asn Arg Asn Glu Ser Leu Ile Ala
                   85                  90                  95
   gga gct tat gaa ata tca aaa cta ata aca caa aaa tta agt gta ttg     336
   Gly Ala Tyr Glu Ile Ser Lys Leu Ile Thr Gln Lys Leu Ser Val Leu
               100                 105                 110
   aat tca gaa gaa tta aag aaa aaa att aaa gag gct aag gat tgt tcc     384
   Asn Ser Glu Glu Leu Lys Lys Lys Ile Lys Glu Ala Lys Asp Cys Ser
           115                 120                 125
   gaa aaa ttt act act aag cta aaa gat agt cat gca gag ctt ggt ata     432
   Glu Lys Phe Thr Thr Lys Leu Lys Asp Ser His Ala Glu Leu Gly Ile
       130                 135                 140
   caa agc gtt cag gat gat aat gca aaa aaa gct att tta aaa aca cat     480
   Gln Ser Val Gln Asp Asp Asn Ala Lys Lys Ala Ile Leu Lys Thr His
   145                 150                 155                 160
   gga act aaa gac aag ggt gct aaa gaa ctt gaa gag tta ttt aaa tca     528
   Gly Thr Lys Asp Lys Gly Ala Lys Glu Leu Glu Glu Leu Phe Lys Ser
                   165                 170                 175
   cta gaa agc ttg tca aaa gca gcg caa gca gca tta act aat tca gtt     576
   Leu Glu Ser Leu Ser Lys Ala Ala Gln Ala Ala Leu Thr Asn Ser Val
               180                 185                 190
   aaa gag ctt aca aat cct gtt gtg gca gaa agt cca aaa aaa cct taa     624
   Lys Glu Leu Thr Asn Pro Val Val Ala Glu Ser Pro Lys Lys Pro  *
           195                 200                 205
<210> 36
   <211> 207
   <212> PRT
   <213> Borrelia burgdorferi
<400> 36
   Met Lys Lys Asn Thr Leu Ser Ala Ile Leu Met Thr Leu Phe Leu Phe
   1               5                   10                  15
   Ile Ser Cys Asn Asn Ser Gly Gly Asp Ser Ala Ser Thr Asn Pro Asp
               20                  25                  30
   Glu Ser Ala Lys Gly Pro Asn Leu Thr Val Ile Ser Lys Lys Ile Thr
           35                  40                  45
   Asp Ser Asn Ala Phe Leu Leu Ala Val Lys Glu Val Glu Ala Leu Leu
       50                  55                  60
   Ser Ser Ile Asp Glu Leu Ser Lys Ala Ile Gly Lys Lys Ile Lys Asn
   65                  70                  75                  80
   Asp Gly Thr Leu Asp Asn Glu Ala Asn Arg Asn Glu Ser Leu Ile Ala
                   85                  90                  95
   Gly Ala Tyr Glu Ile Ser Lys Leu Ile Thr Gln Lys Leu Ser Val Leu
               100                 105                 110
   Asn Ser Glu Glu Leu Lys Lys Lys Ile Lys Glu Ala Lys Asp Cys Ser
           115                 120                 125
   Glu Lys Phe Thr Thr Lys Leu Lys Asp Ser His Ala Glu Leu Gly Ile
       130                 135                 140
   Gln Ser Val Gln Asp Asp Asn Ala Lys Lys Ala Ile Leu Lys Thr His
   145                 150                 155                 160
   Gly Thr Lys Asp Lys Gly Ala Lys Glu Leu Glu Glu Leu Phe Lys Ser
                   165                 170                 175
   Leu Glu Ser Leu Ser Lys Ala Ala Gln Ala Ala Leu Thr Asn Ser Val
               180                 185                 190
   Lys Glu Leu Thr Asn Pro Val Val Ala Glu Ser Pro Lys Lys Pro
           195                 200                 205
<210> 37
   <211> 36
   <212> DNA
   <213> Artificial Sequence
<220>
   <223> Oligonucleotide Primer
<400> 37
   gtgcgcgacc atatggctaa taattcaggg aaagat 36
<210> 38
   <211> 36
   <212> DNA
   <213> Artificial Sequence
<220>
   <223> Oligonucleotide Primer
<400> 38
   gtgcgcgacc atatggctag taattcaggg aaaggt 36
<210> 39
   <211> 36
   <212> DNA
   <213> Artificial Sequence
<220>
   <223> Oligonucleotide Primer
<400> 39
   gtgcgcgacc atatggctaa taattcaggt ggggat 36
<210> 40
   <211> 18
   <212> DNA
   <213> Artificial Sequence
<220>
   <223> Oligonucleotide Primer
<400> 40
   cttggaaaat tatttgaa 18
<210> 41
   <211> 35
   <212> DNA
   <213> Artificial Sequence
<220>
   <223> Oligonucleotide Primer
<400> 41
   cacggtcacc ccatgggaaa taattcaggg aaagg 35
<210> 42
   <211> 18
   <212> DNA
   <213> Artificial Sequence
<220>
   <223> Oligonucleotide Primer
<400> 42
   tatagatgac agcaacgc 18
<210> 43
   <211> 40
   <212> DNA
   <213> Artificial Sequence
<220>
   <223> Oligonucleotide Primer
<400> 43
   ccggtgaccc catggtacca ggtttttttg gactttctgc 40
<210> 44
   <211> 36
   <212> DNA
   <213> Artificial Sequence
<220>
   <223> Oligonucleotide Primer
<400> 44
   ccggatccat atggttaaaa aaataatatt tatttc 36
<210> 45
   <211> 37
   <212> DNA
   <213> Artificial Sequence
<220>
   <223> Oligonucleotide Primer
<400> 45
   gatatctaga tctttaattg ctctgctcac tctcttc 37
<210> 46
   <211> 34
   <212> DNA
   <213> Artificial Sequence
<220>
   <223> Oligonucleotide Primer
<400> 46
   ccgggatcca tatggctagt gcaattggtc gtgg 34
<210> 47
   <211> 18
   <212> DNA
   <213> Artificial Sequence
<220>
   <223> Oligonucleotide Primer
<400> 47
   atgattatca atcataat 18
<210> 48
   <211> 18
   <212> DNA
   <213> Artificial Sequence
<220>
   <223> Oligonucleotide Primer
<400> 48
   tctgaacaat gacaaaac 18
<210> 49
   <211> 825
   <212> DNA
   <213> Borrelia burgdorferi
<400> 49
   atgaaaaaat atttattggg aataggtcta atattagcct taatagcatg taagcaaaat 60
   gttagcagcc ttgatgaaaa aaatagcgtt tcagtagatt tacctggtgg aatgacagtt 120
   cttgtaagta aagaaaaaga caaagacggt aaatacagtc tagaggcaac agtagacaag 180
   cttgagctta aaggaacttc tgataaaaac aacggttctg gaacacttga aggtgaaaaa 240
   actgacaaaa gtaaagtaaa atcaacaatt gctgatgacc taagtcaaac taaatttgaa 300
   attttcaaag aagatggcaa aacattagta tcaaaaaaag taacccttaa agacaagtca 360
   tcaacagaag aaaaattcaa cggaaagggt gaaacatctg aaaaaacaat agtaagagca 420
   aatggaacca gacttgaata cacagacata aaaagcgatg gatccggaaa agctaaagaa 480
   gttttaaaag actttactct tgaaggaact ctagctgctg acggcaaaac aacattgaaa 540
   gttacagaag gcactgttgt tttaagcaag aacattttaa aatccggaga aataacagct 600
   gcacttgatg actctgacac tactcgggct actaaaaaaa ctggaaaatg ggattcaaag 660
   acttccactt taacaattag tgtgaatagc caaaaaacca aaaaccttgt attcacaaaa 720
   gaagacacaa taacagtaca aagatacgac tcagcaggca ccaatctaga aggcaaagca 780
   gtcgaaatta caacacttaa agaacttaaa aacgctttaa aataa                 825
<210> 50
   <211> 824
   <212> DNA
   <213> Borrelia burgdorferi
<400> 50
   atgaaaaaat atttattggg aataggtcta atattagcat taatagcatg taagcaaaat 60
   gttagcagcc ttgatgaaaa aaatagcgtt tcagtagatt tacctggtgg aatgcaagtt 120
   cttgtaagta aagaaaaaga caaagatggt aaatacagtc taatggcaac agtagacaag 180
   cttgagctta aaggaacttc tgataaaaac aacggttctg gaacacttga aggtgaaaaa 240
   actgacaaaa gtaaagcaaa attaacaatt gctgaggatc taagtaaaac cacatttgaa 300
   atcttcaaag aagatggcaa aacattagta tcaaaaaaag taacccttaa agacaagtca 360
   tcaacagaag aaaaattcaa cgcaaagggt gaagcatctg aaaaaacaat agtaagagca 420
   aatggaacca gacttgaata cacagacata aaaagcgata aaaccggaaa agctaaagaa 480
   gttttaaaag actttgctct tgaaggaact ctagctgctg acggcaaaac aacattaaaa 540
   gttacagaag gcactgttgt tttaagcaaa cacatttcaa actctggaga aataacagtt 600
   gagcttaatg actctgacac tactcaggct actaaaaaaa ctggaacatg ggattcaaag 660
   acttccactt taacaattag tgtgaatagc cgaaaaacca aaaaccttgt attcacaaaa 720
   gaagacacaa taacagtaca aaaatacgac tcagcaggca ccaatctaga aggcaaagca 780
   gtcgaaatta caacgcttaa agaacttaaa gatgctttaa aata                  824
<210> 51
   <211> 1011
   <212> DNA
   <213> Borrelia burgdorferi
<220>
   <221> CDS
   <222> (1)...(1011)
<400> 51
   atg att atc aat cat aat aca tca gct att aat gct tca aga aat aat      48
   Met Ile Ile Asn His Asn Thr Ser Ala Ile Asn Ala Ser Arg Asn Asn
    1               5                  10                  15
   gcc att aat gct gct aat ctt agt aaa acc caa gag aag ctt tct agt      96
   Ala Ile Asn Ala Ala Asn Leu Ser Lys Thr Gln Glu Lys Leu Ser Ser
               20                  25                  30
   ggt tac aga att aat cga gct tct gat gat gct gct ggt atg ggg gtt     144
   Gly Tyr Arg Ile Asn Arg Ala Ser Asp Asp Ala Ala Gly Met Gly Val
           35                  40                  45
   tct ggc aag att aat gct caa ata aca ggc tta tca caa gct tct aga     192
   Ser Gly Lys Ile Asn Ala Gln Ile Thr Gly Leu Ser Gln Ala Ser Arg
       50                  55                  60
   aac act tca aaa gct atc aat ttt att cag aca aca gaa gga aat tta     240
   Asn Thr Ser Lys Ala Ile Asn Phe Ile Gln Thr Thr Glu Gly Asn Leu
   65                  70                  75                  80
   aat gaa gta gaa aaa gtt tta gta aga atg aaa gaa tta gca gtt caa     288
   Asn Glu Val Glu Lys Val Leu Val Arg Met Lys Glu Leu Ala Val Gln
                   85                  90                  95
   tca ggt aac gga acg tat tca gac gca gac aga ggt tct ata cag att     336
   Ser Gly Asn Gly Thr Tyr Ser Asp Ala Asp Arg Gly Ser Ile Gln Ile
               100                 105                 110
   gaa ata gag caa ctt aca gac gaa att aat aga att gct gat cag gct     384
   Glu Ile Glu Gln Leu Thr Asp Glu Ile Asn Arg Ile Ala Asp Gln Ala
           115                 120                 125
   caa tat aac caa atg cac atg ttg tca aac aaa tct gct tcc caa aat    432
   Gln Tyr Asn Gln Met His Met Leu Ser Asn Lys Ser Ala Ser Gln Asn
       130                 135                 140
   gta aaa aca gct gaa gag ctt gga atg cag cct gca aaa att aac aca    480
   Val Lys Thr Ala Glu Glu Leu Gly Met Gln Pro Ala Lys Ile Asn Thr
   145                 150                 155                 160
   cca gca tca ctt tca gga tct caa gct tct tgg act tta aga gtt cat    528
   Pro Ala Ser Leu Ser Gly Ser Gln Ala Ser Trp Thr Leu Arg Val His
                   165                 170                 175
   gtg gga gca aat caa gat gaa gca att gct gta aat att tat tca gct    576
   Val Gly Ala Asn Gln Asp Glu Ala Ile Ala Val Asn Ile Tyr Ser Ala
               180                 185                 190
   aat gtt gca aat ctt ttt gct ggt gag gga gct caa gct gct cag gct    624
   Asn Val Ala Asn Leu Phe Ala Gly Glu Gly Ala Gln Ala Ala Gln Ala
           195                 200                 205
   gca cct gtt caa gag ggt gct caa gaa gaa gga gct cag caa cca aca    672
   Ala Pro Val Gln Glu Gly Ala Gln Glu Glu Gly Ala Gln Gln Pro Thr
       210                 215                 220
   cct gct aca gca cct act caa ggt gga gtt aat tct cct gtt aat gtt    720
   Pro Ala Thr Ala Pro Thr Gln Gly Gly Val Asn Ser Pro Val Asn Val
   225                 230                 235                 240
   aca acc aca gtt gat gct aat aca tca ctt gct aaa ata gaa aat gct    768
   Thr Thr Thr Val Asp Ala Asn Thr Ser Leu Ala Lys Ile Glu Asn Ala
                   245                 250                 255
   att aga atg ata agt gat caa aga gca aat tta ggt gct ttc caa aat    816
   Ile Arg Met Ile Ser Asp Gln Arg Ala Asn Leu Gly Ala Phe Gln Asn
               260                 265                 270
   aga ctt gaa tct ata aag aat agc act gag tat gct att gaa aat cta    864
   Arg Leu Glu Ser Ile Lys Asn Ser Thr Glu Tyr Ala Ile Glu Asn Leu
           275                 280                 285
   aaa gca tct tat gct caa ata aaa gat gct aca atg aca gat gag gtt    912
   Lys Ala Ser Tyr Ala Gln Ile Lys Asp Ala Thr Met Thr Asp Glu Val
       290                 295                 300
   gta gca gct aca act aat agt att tta act caa tct gca atg gca atg    960
   Val Ala Ala Thr Thr Asn Ser Ile Leu Thr Gln Ser Ala Met Ala Met
   305                 310                 315                 320
   att gca cag gct aat caa gtt cct caa tat gtt ttg tca ttg ctt aga    1008
   Ile Ala Gln Ala Asn Gln Val Pro Gln Tyr Val Leu Ser Leu Leu Arg
                   325                 330                 335
   taa                                                                1011
    *
<210> 52
   <211> 336
   <212> PRT
   <213> Borrelia burgdorferi
<400> 52
   Met Ile Ile Asn His Asn Thr Ser Ala Ile Asn Ala Ser Arg Asn Asn
   1               5                   10                  15
   Ala Ile Asn Ala Ala Asn Leu Ser Lys Thr Gln Glu Lys Leu Ser Ser
               20                  25                  30
   Gly Tyr Arg Ile Asn Arg Ala Ser Asp Asp Ala Ala Gly Met Gly Val
           35                  40                  45
   Ser Gly Lys Ile Asn Ala Gln Ile Thr Gly Leu Ser Gln Ala Ser Arg
       50                  55                  60
   Asn Thr Ser Lys Ala Ile Asn Phe Ile Gln Thr Thr Glu Gly Asn Leu
   65                  70                  75                  80
   Asn Glu Val Glu Lys Val Leu Val Arg Met Lys Glu Leu Ala Val Gln
                   85                  90                  95
   Ser Gly Asn Gly Thr Tyr Ser Asp Ala Asp Arg Gly Ser Ile Gln Ile
               100                 105                 110
   Glu Ile Glu Gln Leu Thr Asp Glu Ile Asn Arg Ile Ala Asp Gln Ala
           115                 120                 125
   Gln Tyr Asn Gln Met His Met Leu Ser Asn Lys Ser Ala Ser Gln Asn
       130                 135                 140
   Val Lys Thr Ala Glu Glu Leu Gly Met Gln Pro Ala Lys Ile Asn Thr
   145                 150                 155                 160
   Pro Ala Ser Leu Ser Gly Ser Gln Ala Ser Trp Thr Leu Arg Val His
                   165                 170                 175
   Val Gly Ala Asn Gln Asp Glu Ala Ile Ala Val Asn Ile Tyr Ser Ala
               180                 185                 190
   Asn Val Ala Asn Leu Phe Ala Gly Glu Gly Ala Gln Ala Ala Gln Ala
           195                 200                 205
   Ala Pro Val Gln Glu Gly Ala Gln Glu Glu Gly Ala Gln Gln Pro Thr
       210                 215                 220
   Pro Ala Thr Ala Pro Thr Gln Gly Gly Val Asn Ser Pro Val Asn Val
   225                 230                 235                 240
   Thr Thr Thr Val Asp Ala Asn Thr Ser Leu Ala Lys Ile Glu Asn Ala
                   245                 250                 255
   Ile Arg Met Ile Ser Asp Gln Arg Ala Asn Leu Gly Ala Phe Gln Asn
               260                 265                 270
   Arg Leu Glu Ser Ile Lys Asn Ser Thr Glu Tyr Ala Ile Glu Asn Leu
           275                 280                 285
   Lys Ala Ser Tyr Ala Gln Ile Lys Asp Ala Thr Met Thr Asp Glu Val
       290                 295                 300
   Val Ala Ala Thr Thr Asn Ser Ile Leu Thr Gln Ser Ala Met Ala Met
   305                 310                 315                 320
   Ile Ala Gln Ala Asn Gln Val Pro Gln Tyr Val Leu Ser Leu Leu Arg
                   325                 330                 335
<210> 53
   <211> 1008
   <212> DNA
   <213> Borrelia burgdorferi
<220>
   <221> CDS
   <222> (1)...(1008)
<400> 53
   atg att atc aat cat aat aca tca gct att aat gct tca aga aat aat      48
   Met Ile Ile Asn His Asn Thr Ser Ala Ile Asn Ala Ser Arg Asn Asn
   1               5                   10                  15
   ggt att aat gct gct aat ctt agt aaa act caa gag aag ctt tct agt      96
   Gly Ile Asn Ala Ala Asn Leu Ser Lys Thr Gln Glu Lys Leu Ser Ser
               20                  25                  30
   ggt tac aga att aat aga gct tct gat gat gct gct ggt atg ggg gtt      144
   Gly Tyr Arg Ile Asn Arg Ala Ser Asp Asp Ala Ala Gly Met Gly Val
           35                  40                  45
   tct ggg aag att aat gct caa ata aga ggt tta tca caa gct tct aga      192
   Ser Gly Lys Ile Asn Ala Gln Ile Arg Gly Leu Ser Gln Ala Ser Arg
       50                  55                  60
   aac act tca aaa gct att aat ttt att cag aca aca gaa gga aat ttg      240
   Asn Thr Ser Lys Ala Ile Asn Phe Ile Gln Thr Thr Glu Gly Asn Leu
   65                  70                  75                  80
   aat gaa gta gaa aaa gtt tta gta aga atg aaa gaa tta gca gtt caa      288
   Asn Glu Val Glu Lys Val Leu Val Arg Met Lys Glu Leu Ala Val Gln
                   85                  90                  95
   tca ggt aac ggt aca tat tca gac gca gac aga ggt tct ata caa att      336
   Ser Gly Asn Gly Thr Tyr Ser Asp Ala Asp Arg Gly Ser Ile Gln Ile
               100                 105                 110
   gaa ata gag caa ctt aca gac gaa att aat aga att gct gat caa gct      384
   Glu Ile Glu Gln Leu Thr Asp Glu Ile Asn Arg Ile Ala Asp Gln Ala
           115                 120                 125
   caa tat aac caa atg cac atg ttg tca aac aaa tct gct tcc caa aat      432
   Gln Tyr Asn Gln Met His Met Leu Ser Asn Lys Ser Ala Ser Gln Asn
       130                 135                 140
   gta aga aca gct gaa gaa ctt gga atg caa cct gca aaa atc aac aca      480
   Val Arg Thr Ala Glu Glu Leu Gly Met Gln Pro Ala Lys Ile Asn Thr
   145                 150                 155                 160
   cca gcg tca ctt tca gga tct caa gct tct tgg act tta aga gtt cat      528
   Pro Ala Ser Leu Ser Gly Ser Gln Ala Ser Trp Thr Leu Arg Val His
                   165                 170                 175
   gtg gga gca aat caa gat gaa gcg att gct gta aat att tat gct gct      576
   Val Gly Ala Asn Gln Asp Glu Ala Ile Ala Val Asn Ile Tyr Ala Ala
               180                 185                 190
   aat gtt gca aat cta ttc tct ggt gaa gga gct cag gct gct cag act      624
   Asn Val Ala Asn Leu Phe Ser Gly Glu Gly Ala Gln Ala Ala Gln Thr
           195                 200                 205
   gca cct gtt caa gaa ggt gct caa caa gaa gga gct caa caa cca gca      672
   Ala Pro Val Gln Glu Gly Ala Gln Gln Glu Gly Ala Gln Gln Pro Ala
       210                 215                 220
   cct gct aca gcg cct tct cag ggt gga gtt aat tct cct gtt aat gtt      720
   Pro Ala Thr Ala Pro Ser Gln Gly Gly Val Asn Ser Pro Val Asn Val
   225                 230                 235                 240
   aca act aca gtt gac gct aat aca tct ctt gct aaa ata gaa aat gct      768
   Thr Thr Thr Val Asp Ala Asn Thr Ser Leu Ala Lys Ile Glu Asn Ala
                   245                 250                 255
   att aga atg ata agt gat caa aga gca aat tta ggt gct ttc caa aat      816
   Ile Arg Met Ile Ser Asp Gln Arg Ala Asn Leu Gly Ala Phe Gln Asn
               260                 265                 270
   aga ctt gag tct ata aag gat agt act gag tat gct att gaa aac cta      864
   Arg Leu Glu Ser Ile Lys Asp Ser Thr Glu Tyr Ala Ile Glu Asn Leu
           275                 280                 285
   aaa gca tct tat gct caa ata aaa gat gct aca atg aca gat gag gtt      912
   Lys Ala Ser Tyr Ala Gln Ile Lys Asp Ala Thr Met Thr Asp Glu Val
       290                 295                 300
   gta gca gct aca act aat agt att tta aca caa tgt gca atg gca atg      960
   Val Ala Ala Thr Thr Asn Ser Ile Leu Thr Gln Cys Ala Met Ala Met
   305                 310                 315                 320
   att gcg caa gct aat caa gtt cct caa tat gtt ttg tca ttg ctt aga      1008
   Ile Ala Gln Ala Asn Gln Val Pro Gln Tyr Val Leu Ser Leu Leu Arg
                   325                 330                 335
<210> 54
   <211> 336
   <212> PRT
   <213> Borrelia burgdorferi
<400> 54
   Met Ile Ile Asn His Asn Thr Ser Ala Ile Asn Ala Ser Arg Asn Asn
   1               5                   10                  15
   Gly Ile Asn Ala Ala Asn Leu Ser Lys Thr Gln Glu Lys Leu Ser Ser
               20                  25                  30
   Gly Tyr Arg Ile Asn Arg Ala Ser Asp Asp Ala Ala Gly Met Gly Val
           35                  40                  45
   Ser Gly Lys Ile Asn Ala Gln Ile Arg Gly Leu Ser Gln Ala Ser Arg
       50                  55                  60
   Asn Thr Ser Lys Ala Ile Asn Phe Ile Gln Thr Thr Glu Gly Asn Leu
   65                  70                  75                  80
   Asn Glu Val Glu Lys Val Leu Val Arg Met Lys Glu Leu Ala Val Gln
                   85                  90                  95
   Ser Gly Asn Gly Thr Tyr Ser Asp Ala Asp Arg Gly Ser Ile Gln Ile
               100                 105                 110
   Glu Ile Glu Gln Leu Thr Asp Glu Ile Asn Arg Ile Ala Asp Gln Ala
           115                 120                 125
   Gln Tyr Asn Gln Met His Met Leu Ser Asn Lys Ser Ala Ser Gln Asn
       130                 135                 140
   Val Arg Thr Ala Glu Glu Leu Gly Met Gln Pro Ala Lys Ile Asn Thr
   145                 150                 155                 160
   Pro Ala Ser Leu Ser Gly Ser Gln Ala Ser Trp Thr Leu Arg Val His
                   165                 170                 175
   Val Gly Ala Asn Gln Asp Glu Ala Ile Ala Val Asn Ile Tyr Ala Ala
               180                 185                 190
   Asn Val Ala Asn Leu Phe Ser Gly Glu Gly Ala Gln Ala Ala Gln Thr
           195                 200                 205
   Ala Pro Val Gln Glu Gly Ala Gln Gln Glu Gly Ala Gln Gln Pro Ala
       210                 215                 220
   Pro Ala Thr Ala Pro Ser Gln Gly Gly Val Asn Ser Pro Val Asn Val
   225                 230                 235                 240
   Thr Thr Thr Val Asp Ala Asn Thr Ser Leu Ala Lys Ile Glu Asn Ala
                   245                 250                 255
   Ile Arg Met Ile Ser Asp Gln Arg Ala Asn Leu Gly Ala Phe Gln Asn
               260                 265                 270
   Arg Leu Glu Ser Ile Lys Asp Ser Thr Glu Tyr Ala Ile Glu Asn Leu
           275                 280                 285
   Lys Ala Ser Tyr Ala Gln Ile Lys Asp Ala Thr Met Thr Asp Glu Val
       290                 295                 300
   Val Ala Ala Thr Thr Asn Ser Ile Leu Thr Gln Cys Ala Met Ala Met
   305                 310                 315                 320
   Ile Ala Gln Ala Asn Gln Val Pro Gln Tyr Val Leu Ser Leu Leu Arg
                   325                 330                 335
<210> 55
   <211> 821
   <212> DNA
   <213> Borrelia burgdorferi
<400> 55
   atgaaaaaat atttattggg aataggtcta atattagcct taatagcatg caagcaaaat 60
   gttagcagcc ttgatgaaaa aaacagcgct tcagtagatt tgcctggtga gatgaaagtt 120
   cttgtaagta aagaaaaaga caaagacggt aagtacagtc taaaggcaac agtagacaag 180
   attgagctaa aaggaacttc tgataaagac aatggttctg gggtgcttga aggtacaaaa 240
   gatgacaaaa gtaaagcaaa attaacaatt gctgacgatc taggtaaaac cacattcgaa 300
   cttttcaaag aagatggcaa aacattagtg tcaagaaaag taagttctaa agacaaaaca 360
   tcaacagatg aaatgttcaa tgaaaaaggt gaattgtctg caaaaaccat gacaagagaa 420
   aatggaacca aacttgaata tacagaaatg aaaagcgatg gaaccggaaa agctaaagaa 480
   gttttaaaaa actttactct tgaaggaaaa gtagctaatg ataaagtaac attggaagta 540
   aaagaaggaa ccgttacttt aagtaaggaa attgcaaaat ctggagaagt aacagttgct 600
   cttaatgaca ctaacactac tcaggctact aaaaaaactg gcgcatggga ttcaaaaact 660
   tctactttaa caattagtgt taacagcaaa aaaactacac aacttgtgtt tactaaacaa 720
   gacacaataa ctgtacaaaa atacgactcc gcaggtacca atttagaagg cacagcagtc 780
   gaaattaaaa cacttgatga acttaaaaac gctttaaaat a                     821
<210> 56
   <211> 821
   <212> DNA
   <213> Borrelia burgdorferi
<400> 56
   atgaaaaaat atttattggg aataggtcta atattagcct taatagcatg caagcaaaat 60
   gttagcagcc ttgatgaaaa aaacagcgct tcagtagatt tgcctggtga gattaaagtt 120
   cttgtaagta aagaaaaaga caaagacggt aagtacagtc taaaggcaac agtagacaag 180
   attgagctaa aaggaacttc tgataaagac aatggttctg gagtgcttga aggtacaaaa 240
   gatgacaaaa gtaaagcaaa attaacaatt gctgacgatc taagtaaaac cacattcgaa 300
   cttttcaaag aagatggcaa aacattagtg tcaagaaaag taagttctaa agacaaaaca 360
   tcaacagatg aaatgttcaa tgaaaaaggt gaattgtctg caaaaaccat gacaagagaa 420
   aatggaacca aacttgaata tacagaaatg aaaagcgatg gaaccggaaa agctaaagaa 480
   gttttaaaaa actttactct tgaaggaaaa gtagctaatg ataaagtaac attggaagta 540
   aaagaaggaa ccgttacttt aagtaaggaa attgcaaaat ctggagaagt aacagttgct 600
   cttaatgaca ctaacactac tcaggctact aaaaaaactg gcgcatggga ttcaaaaact 660
   tctactttaa caattagtgt taacagtaaa aaaactacac aacttgtgtt tactaaacaa 720
   gacacaataa ctgtacaaaa atacgactcc gcaggtacca atttagaagg cacagcagtc 780
   gaaattaaaa cacttgatga acttaaaaac gctttaaaat a 821
<210> 57
   <211> 821
   <212> DNA
   <213> Borrelia burgdorferi
<400> 57
   atgaaaaaat atttattggg aataggtcta atattagcct taatagcatg caagcaaaat 60
   gttagcagcc ttgatgaaaa aaacagcgct tcagtagatt tgcctggtga gatgaaagtt 120
   cttgtaagta aagaaaaaga caaagacggt aagtacagtc taaaggcaac agtagacaag 180
   attgagctaa aaggaacttc tgataaagac aatggttctg gggtgcttga aggtacaaaa 240
   gatgacaaaa gtaaagcaaa attaacaatt gctgacgatc taggtaaaac cacattcgaa 300
   cttttcaaag aagatggcaa aacattagtg tcaagaaaag taagttctaa agacaaaaca 360
   tcaacagatg aaatgttcaa tgaaaaaggt gaattgtctg caaaaaccat gacaagagaa 420
   aatggaacca aacttgaata tacagaaatg aaaagcgatg gaaccggaaa agctaaagaa 480
   gttttaaaaa actttactct tgaaggaaaa gtagctaatg ataaagtaac attggaagta 540
   aaagaaggaa ccgttacttt aagtaaggaa attgcaaaat ctggagaagt aacagttgct 600
   cttaatgaca ctaacactac tcaggctact aaaaaaactg gcgcatggga ttcaaaaact 660
   tctactttaa caattagtgt taacagcaaa aaaactacac aacttgtgtt tactaaacaa 720
   gacacaataa ctgtacaaaa atacgactcc gcaggtacca atttagaagg cacagcagtc 780
   gaaattaaaa cacttgatga acttaaaaac gctttaaaat a 821
<210> 58
   <211> 822
   <212> DNA
   <213> Borrelia burgdorferi
<400> 58
   atgaaaaaat atttattggg aataggtcta atattagcct taatagcatg caagcaaaat 60
   gttagcagcc ttgatgaaaa aaacagcgct tcagtagatt tgcctggtga gatgaaagtt 120
   cttgtaagta aagaaaaaga caaagacggt aagtacagtc taaaggcaac agtagacaag 180
   attgagctaa aaggaacttc tgataaagac aatggttctg gagtgcttga aggtacaaaa 240
   gatgacaaaa gtaaagcaaa attaacaatt gctgacgatc taagtaaaac cacattcgaa 300
   cttttcaaag aagatggcaa aacattagtg tcaagaaaag taagttctaa agacaaaaca 360
   tcaacagatg aaatgttcaa tgaaaaaggt gaattgtctg caaaaaccat gacaagagaa 420
   aatggaacca aacttgaata tacagaaatg aaaagcgatg gaaccggaaa agctaaagaa 480
   gttttaaaaa actttactct tgaaggaaaa gtagctaatg ataaagtaac attggaagta 540
   aaagaaggaa ccgttacttt aagtaaggaa attgcaaaat ctggagaagt aacagttgct 600
   cttaatgaca ctaacactac tcaggctact aaaaaaactg gcgcatggga ttcaaaaact 660
   tctactttaa caattagtgt taacagcaaa aaaactacac aacttgtgtt tactaaacaa 720
   gacacaataa ctgtacaaaa atacgactcc gcaggtacca atttagaagg cacagcagtc 780
   gaaattaaaa cacttgatga acttaaaaac gctttgaaat aa 822
<210> 59
   <211> 37
   <212> DNA
   <213> Artificial Sequence
<220>
   <223> Oligonucleotide Primer
<400> 59
   ttggatccgg tcaccccatg gctcaatata accaatg 37
<210> 60
   <211> 37
   <212> DNA
   <213> Artificial Sequence
<220>
   <223> Oligonucleotide Primer
<400> 60
   ttggatccgg tcaccccatg gcttctcaaa atgtaag 37
<210> 61
   <211> 33
   <212> DNA
   <213> Artificial Sequence
<220>
   <223> Oligonucleotide Primer
<400> 61
   ttggatccgg tgaccaactc cgccttgaga agg 33
<210> 62
   <211> 33
   <212> DNA
   <213> Artificial Sequence
<220>
   <223> Oligonucleotide Primer
<400> 62
   ttggatccgg tgacctattt gagcataaga tgc 33
<210> 63
   <211> 20
   <212> DNA
   <213> Artificial Sequence
<220>
   <223> Oligonucleotide Primer
<400> 63
   ggtgaattta gttggtaagg 20
<210> 64
   <211> 26
   <212> DNA
   <213> Artificial Sequence
<220>
   <223> Oligonucleotide Primer
<400> 64
   caccagtttc tttaagctgc tcctgc 26
<210> 65
   <211> 2102
   <212> DNA
   <213> Borrelia burgdorferi
<220>
   <221> CDS
   <222> (1)...(2100)
<400> 65
   atg aaa aaa atg tta cta atc ttt agt ttt ttt ctt att ttc ttg aat    48
   Met Lys Lys Met Leu Leu Ile Phe Ser Phe Phe Leu Ile Phe Leu Asn
    1               5                  10                  15
   gga ttt cct gtt agt gca aga gaa gtt gat agg gaa aaa tta aag gac    96
   Gly Phe Pro Val Ser Ala Arg Glu Val Asp Arg Glu Lys Leu Lys Asp
               20                  25                  30
   ttt gtt aat atg gat ctt gag ttt gta aat tat aaa ggc cct tat gat    144
   Phe Val Asn Met Asp Leu Glu Phe Val Asn Tyr Lys Gly Pro Tyr Asp
           35                  40                  45
   tct aca aat aca tat gaa caa ata gtg ggt att ggg gag ttt tta gca    192
   Ser Thr Asn Thr Tyr Glu Gln Ile Val Gly Ile Gly Glu Phe Leu Ala
       50                  55                  60
   aga ccg ttg acc aat tcc aat agc aac tca agt tat tat ggt aaa tat    240
   Arg Pro Leu Thr Asn Ser Asn Ser Asn Ser Ser Tyr Tyr Gly Lys Tyr
   65                  70                  75                  80
   ttt att aat aga ttt att gat gat caa gat aaa aaa gca agc gtt gat    288
   Phe Ile Asn Arg Phe Ile Asp Asp Gln Asp Lys Lys Ala Ser Val Asp
                   85                  90                  95
   gtt ttt tct att ggt agt aag tca gag ctt gac agt ata ttg aat tta    336
   Val Phe Ser Ile Gly Ser Lys Ser Glu Leu Asp Ser Ile Leu Asn Leu
               100                 105                 110
   aga aga att ctt aca ggg tat tta ata aag tct ttc gat tat gac agg    384
   Arg Arg Ile Leu Thr Gly Tyr Leu Ile Lys Ser Phe Asp Tyr Asp Arg
           115                 120                 125
   tct agt gca gaa tta att gct aag gtt att aca ata tat aat gct gtt    432
   Ser Ser Ala Glu Leu Ile Ala Lys Val Ile Thr Ile Tyr Asn Ala Val
       130                 135                 140
   tat aga gga gat ttg gat tat tat aaa ggg ttt tat att gag gct gct    480
   Tyr Arg Gly Asp Leu Asp Tyr Tyr Lys Gly Phe Tyr Ile Glu Ala Ala
   145                 150                 155                 160
   tta aag tct tta agt aaa gaa aat gca ggt ctt tct agg gtt tat agt    528
   Leu Lys Ser Leu Ser Lys Glu Asn Ala Gly Leu Ser Arg Val Tyr Ser
                   165                 170                 175
   cag tgg gct gga aag aca caa ata ttt att cct ctt aaa aag gat att    576
   Gln Trp Ala Gly Lys Thr Gln Ile Phe Ile Pro Leu Lys Lys Asp Ile
               180                 185                 190
   ttg tct gga aat att gag tct gac att gat att gac agt tta gtt aca    624
   Leu Ser Gly Asn Ile Glu Ser Asp Ile Asp Ile Asp Ser Leu Val Thr
           195                 200                 205
   gat aag gtg gtg gca gct ctt tta agt gaa aat gaa gca ggt gtt aac      672
   Asp Lys Val Val Ala Ala Leu Leu Ser Glu Asn Glu Ala Gly Val Asn
       210                 215                 220
   ttt gca aga gat att aca gat att caa ggc gaa act cat aag gca gat      720
   Phe Ala Arg Asp Ile Thr Asp Ile Gln Gly Glu Thr His Lys Ala Asp
   225                 230                 235                 240
   caa gat aaa att gat att gaa tta gac aat att cat gaa agt gat tcc      768
   Gln Asp Lys Ile Asp Ile Glu Leu Asp Asn Ile His Glu Ser Asp Ser
                   245                 250                 255
   aat ata aca gaa act att gaa aat tta agg gat cag ctt gaa aaa gct      816
   Asn Ile Thr Glu Thr Ile Glu Asn Leu Arg Asp Gln Leu Glu Lys Ala
               260                 265                 270
   aca gat gaa gag cat aaa aaa gag att gaa agt cag gtt gat gct aaa      864
   Thr Asp Glu Glu His Lys Lys Glu Ile Glu Ser Gln Val Asp Ala Lys
           275                 280                 285
   aag aaa caa aag gaa gag cta gat aaa aag gca ata aat ctt gat aaa      912
   Lys Lys Gln Lys Glu Glu Leu Asp Lys Lys Ala Ile Asn Leu Asp Lys
       290                 295                 300
   gct cag caa aaa tta gat tct gct gaa gat aat tta gat gtt caa aga      960
   Ala Gln Gln Lys Leu Asp Ser Ala Glu Asp Asn Leu Asp Val Gln Arg
   305                 310                 315                 320
   aat act gtt aga gag aaa att caa gag gat att aac gaa att aac aag     1008
   Asn Thr Val Arg Glu Lys Ile Gln Glu Asp Ile Asn Glu Ile Asn Lys
                   325                 330                 335
   gaa aag aat tta cca aag cct ggt gat gta agt tct cct aaa gtt gat     1056
   Glu Lys Asn Leu Pro Lys Pro Gly Asp Val Ser Ser Pro Lys Val Asp
               340                 345                 350
   aag caa cta caa ata aaa gag agc ctg gaa gat ttg cag gag cag ctt     1104
   Lys Gln Leu Gln Ile Lys Glu Ser Leu Glu Asp Leu Gln Glu Gln Leu
           355                 360                 365
   aaa gaa act ggt gat gaa aat cag aaa aga gaa att gaa aag caa att     1152
   Lys Glu Thr Gly Asp Glu Asn Gln Lys Arg Glu Ile Glu Lys Gln Ile
       370                 375                 380
   gaa atc aaa aaa agt gat gaa aag ctt tta aaa agt aaa gat gat aaa     1200
   Glu Ile Lys Lys Ser Asp Glu Lys Leu Leu Lys Ser Lys Asp Asp Lys
   385                 390                 395                 400
   gca agt aaa gat ggt aaa gcc ttg gat ctt gat cga gaa tta aat tct     1248
   Ala Ser Lys Asp Gly Lys Ala Leu Asp Leu Asp Arg Glu Leu Asn Ser
                   405                 410                 415
   aaa gct tct agc aaa gaa aaa agt aaa gcc aag gaa gaa gaa ata acc     1296
   Lys Ala Ser Ser Lys Glu Lys Ser Lys Ala Lys Glu Glu Glu Ile Thr
               420                 425                 430
   aag ggt aag tca cag aaa agc tta ggc gat ttg aat aat gat gaa aat     1344
   Lys Gly Lys Ser Gln Lys Ser Leu Gly Asp Leu Asn Asn Asp Glu Asn
           435                 440                 445
   ctt atg atg cca gaa gat caa aaa tta cct gag gtt aaa aaa tta gat     1392
   Leu Met Met Pro Glu Asp Gln Lys Leu Pro Glu Val Lys Lys Leu Asp
       450                 455                 460
   agc aaa aaa gaa ttt aaa cct gtt tct gag gtt gag aaa tta gat aag     1440
   Ser Lys Lys Glu Phe Lys Pro Val Ser Glu Val Glu Lys Leu Asp Lys
   465                 470                 475                 480
   att ttc aag tct aat aac aat gtt gga gaa tta tca ccg tta gat aaa     1488
   Ile Phe Lys Ser Asn Asn Asn Val Gly Glu Leu Ser Pro Leu Asp Lys
                   485                 490                 495
   tct tct tat aaa gac att gat tca aaa gag gag aca gtt aat aaa gat     1536
   Ser Ser Tyr Lys Asp Ile Asp Ser Lys Glu Glu Thr Val Asn Lys Asp
               500                 505                 510
   gtt aat ttg caa aag act aag cct cag gtt aaa gac caa gtt act tct     1584
   Val Asn Leu Gln Lys Thr Lys Pro Gln Val Lys Asp Gln Val Thr Ser
           515                 520                 525
   ttg aat gaa gat ttg act act atg tct ata gat tcc agt agt cct gta     1632
   Leu Asn Glu Asp Leu Thr Thr Met Ser Ile Asp Ser Ser Ser Pro Val
       530                 535                 540
   ttt tta gag gtt att gat cca att aca aat tta gga act ctt caa ctt     1680
   Phe Leu Glu Val Ile Asp Pro Ile Thr Asn Leu Gly Thr Leu Gln Leu
   545                 550                 555                 560
   att gat tta aat act ggt gtt agg ctt aaa gaa agc act cag caa ggc     1728
   Ile Asp Leu Asn Thr Gly Val Arg Leu Lys Glu Ser Thr Gln Gln Gly
                   565                 570                 575
   att cag cgg tat gga att tat gaa cgt gaa aaa gat ttg gtt gtt att     1776
   Ile Gln Arg Tyr Gly Ile Tyr Glu Arg Glu Lys Asp Leu Val Val Ile
               580                 585                 590
   aaa atg gat tca gga aaa gct aag ctt cag ata ctt gat aaa ctt gaa     1824
   Lys Met Asp Ser Gly Lys Ala Lys Leu Gln Ile Leu Asp Lys Leu Glu
           595                 600                 605
   aat tta aaa gtg gta tca gag tct aat ttt gag att aat aaa aat tca     1872
   Asn Leu Lys Val Val Ser Glu Ser Asn Phe Glu Ile Asn Lys Asn Ser
       610                 615                 620
   tct ctt tat gtt gat tct aaa atg att tta gta gct gtt agg gat aaa     1920
   Ser Leu Tyr Val Asp Ser Lys Met Ile Leu Val Ala Val Arg Asp Lys
   625                 630                 635                 640
   gat agt agt aat gat tgg aga ttg gcc aaa ttt tct cct aaa aat tta     1968
   Asp Ser Ser Asn Asp Trp Arg Leu Ala Lys Phe Ser Pro Lys Asn Leu
                   645                 650                 655
   gat gag ttt att ctt tca gag aat aaa att atg cct ttt act agc ttt     2016
   Asp Glu Phe Ile Leu Ser Glu Asn Lys Ile Met Pro Phe Thr Ser Phe
               660                 665                 670
   tct gtg aga aaa aat ttt att tat ttg caa gat gag ttt aaa agt cta     2064
   Ser Val Arg Lys Asn Phe Ile Tyr Leu Gln Asp Glu Phe Lys Ser Leu
           675                 680                 685
   gtt att tta gat gta aat act tta aaa aaa gtt aag ta                  2102
   Val Ile Leu Asp Val Asn Thr Leu Lys Lys Val Lys
       690                 695                 700
<210> 66
   <211> 700
   <212> PRT
   <213> Borrelia burgdorferi
<400> 66
   Met Lys Lys Met Leu Leu Ile Phe Ser Phe Phe Leu Ile Phe Leu Asn
   1               5                   10                  15
   Gly Phe Pro Val Ser Ala Arg Glu Val Asp Arg Glu Lys Leu Lys Asp
               20                  25                  30
   Phe Val Asn Met Asp Leu Glu Phe Val Asn Tyr Lys Gly Pro Tyr Asp
           35                  40                  45
   Ser Thr Asn Thr Tyr Glu Gln Ile Val Gly Ile Gly Glu Phe Leu Ala
       50                  55                  60
   Arg Pro Leu Thr Asn Ser Asn Ser Asn Ser Ser Tyr Tyr Gly Lys Tyr
   65                  70                  75                  80
   Phe Ile Asn Arg Phe Ile Asp Asp Gln Asp Lys Lys Ala Ser Val Asp
                   85                  90                  95
   Val Phe Ser Ile Gly Ser Lys Ser Glu Leu Asp Ser Ile Leu Asn Leu
               100                 105                 110
   Arg Arg Ile Leu Thr Gly Tyr Leu Ile Lys Ser Phe Asp Tyr Asp Arg
           115                 120                 125
   Ser Ser Ala Glu Leu Ile Ala Lys Val Ile Thr Ile Tyr Asn Ala Val
       130                 135                 140
   Tyr Arg Gly Asp Leu Asp Tyr Tyr Lys Gly Phe Tyr Ile Glu Ala Ala
   145                 150                 155                 160
   Leu Lys Ser Leu Ser Lys Glu Asn Ala Gly Leu Ser Arg Val Tyr Ser
                   165                 170                 175
   Gln Trp Ala Gly Lys Thr Gln Ile Phe Ile Pro Leu Lys Lys Asp Ile
               180                 185                 190
   Leu Ser Gly Asn Ile Glu Ser Asp Ile Asp Ile Asp Ser Leu Val Thr
           195                 200                 205
   Asp Lys Val Val Ala Ala Leu Leu Ser Glu Asn Glu Ala Gly Val Asn
       210                 215                 220
   Phe Ala Arg Asp Ile Thr Asp Ile Gln Gly Glu Thr His Lys Ala Asp
   225                 230                 235                 240
   Gln Asp Lys Ile Asp Ile Glu Leu Asp Asn Ile His Glu Ser Asp Ser
                   245                 250                 255
   Asn Ile Thr Glu Thr Ile Glu Asn Leu Arg Asp Gln Leu Glu Lys Ala
               260                 265                 270
   Thr Asp Glu Glu His Lys Lys Glu Ile Glu Ser Gln Val Asp Ala Lys
           275                 280                 285
   Lys Lys Gln Lys Glu Glu Leu Asp Lys Lys Ala Ile Asn Leu Asp Lys
       290                 295                 300
   Ala Gln Gln Lys Leu Asp Ser Ala Glu Asp Asn Leu Asp Val Gln Arg
   305                 310                 315                 320
   Asn Thr Val Arg Glu Lys Ile Gln Glu Asp Ile Asn Glu Ile Asn Lys
                   325                 330                 335
   Glu Lys Asn Leu Pro Lys Pro Gly Asp Val Ser Ser Pro Lys Val Asp
               340                 345                 350
   Lys Gln Leu Gln Ile Lys Glu Ser Leu Glu Asp Leu Gln Glu Gln Leu
           355                 360                 365
   Lys Glu Thr Gly Asp Glu Asn Gln Lys Arg Glu Ile Glu Lys Gln Ile
       370                 375                 380
   Glu Ile Lys Lys Ser Asp Glu Lys Leu Leu Lys Ser Lys Asp Asp Lys
   385                 390                 395                 400
   Ala Ser Lys Asp Gly Lys Ala Leu Asp Leu Asp Arg Glu Leu Asn Ser
                   405                 410                 415
   Lys Ala Ser Ser Lys Glu Lys Ser Lys Ala Lys Glu Glu Glu Ile Thr
               420                 425                 430
   Lys Gly Lys Ser Gln Lys Ser Leu Gly Asp Leu Asn Asn Asp Glu Asn
           435                 440                 445
   Leu Met Met Pro Glu Asp Gln Lys Leu Pro Glu Val Lys Lys Leu Asp
       450                 455                 460
   Ser Lys Lys Glu Phe Lys Pro Val Ser Glu Val Glu Lys Leu Asp Lys
   465                 470                 475                 480
   Ile Phe Lys Ser Asn Asn Asn Val Gly Glu Leu Ser Pro Leu Asp Lys
                   485                 490                 495
   Ser Ser Tyr Lys Asp Ile Asp Ser Lys Glu Glu Thr Val Asn Lys Asp
               500                 505                 510
   Val Asn Leu Gln Lys Thr Lys Pro Gln Val Lys Asp Gln Val Thr Ser
           515                 520                 525
   Leu Asn Glu Asp Leu Thr Thr Met Ser Ile Asp Ser Ser Ser Pro Val
       530                 535                 540
   Phe Leu Glu Val Ile Asp Pro Ile Thr Asn Leu Gly Thr Leu Gln Leu
   545                 550                 555                 560
   Ile Asp Leu Asn Thr Gly Val Arg Leu Lys Glu Ser Thr Gln Gln Gly
                   565                 570                 575
   Ile Gln Arg Tyr Gly Ile Tyr Glu Arg Glu Lys Asp Leu Val Val Ile
               580                 585                 590
   Lys Met Asp Ser Gly Lys Ala Lys Leu Gln Ile Leu Asp Lys Leu Glu
           595                 600                 605
   Asn Leu Lys Val Val Ser Glu Ser Asn Phe Glu Ile Asn Lys Asn Ser
       610                 615                 620
   Ser Leu Tyr Val Asp Ser Lys Met Ile Leu Val Ala Val Arg Asp Lys
   625                 630                 635                 640
   Asp Ser Ser Asn Asp Trp Arg Leu Ala Lys Phe Ser Pro Lys Asn Leu
                   645                 650                 655
   Asp Glu Phe Ile Leu Ser Glu Asn Lys Ile Met Pro Phe Thr Ser Phe
               660                 665                 670
   Ser Val Arg Lys Asn Phe Ile Tyr Leu Gln Asp Glu Phe Lys Ser Leu
           675                 680                 685
   Val Ile Leu Asp Val Asn Thr Leu Lys Lys Val Lys
       690                 695                 700
<210> 67
   <211> 2081
   <212> DNA
   <213> Borrelia burgdorferi
<220>
   <221> CDS
   <222> (1)...(2079)
<400> 67
   atg aaa aaa ttg tta cta atc ttt agt ttt ttt ctt att tct ttg aat      48
   Met Lys Lys Leu Leu Leu Ile Phe Ser Phe Phe Leu Ile Ser Leu Asn
   1               5                   10                  15
   gga ttt cct ctt aat tca agg gaa gtt gat aag gaa aaa tta aag gat      96
   Gly Phe Pro Leu Asn Ser Arg Glu Val Asp Lys Glu Lys Leu Lys Asp
               20                  25                  30
   ttt gtt aat atg gat ctt gag ttt gta aac tat aaa ggt cct tat gat      144
   Phe Val Asn Met Asp Leu Glu Phe Val Asn Tyr Lys Gly Pro Tyr Asp
           35                  40                  45
   tct aca aat aca tat gaa caa ata gta ggt att ggt gag ttt tta gca     192
   Ser Thr Asn Thr Tyr Glu Gln Ile Val Gly Ile Gly Glu Phe Leu Ala
        50                  55                  60
   aga cca ttg att aat tcc aat agc aac tca att tat tat ggt aaa tat     240
   Arg Pro Leu Ile Asn Ser Asn Ser Asn Ser Ile Tyr Tyr Gly Lys Tyr
    65                  70                  75                  80
   ttt att aat aga ttt att gat gat caa gat aaa aaa gca agc gtt gat     288
   Phe Ile Asn Arg Phe Ile Asp Asp Gln Asp Lys Lys Ala Ser Val Asp
                    85                  90                  95
   gtt ttt tct att ggt agt agg tca cag ctt gac agt ata ttg aat cta     336
   Val Phe Ser Ile Gly Ser Arg Ser Gln Leu Asp Ser Ile Leu Asn Leu
               100                 105                 110
   aga aga att ctt aca ggg tat ttg ata aag tct ttt gat tat gaa aga     384
   Arg Arg Ile Leu Thr Gly Tyr Leu Ile Lys Ser Phe Asp Tyr Glu Arg
           115                 120                 125
   tct agt gct gaa tta att gct aag gtt att aca ata cat aat gct gtt     432
   Ser Ser Ala Glu Leu Ile Ala Lys Val Ile Thr Ile His Asn Ala Val
       130                 135                 140
   tat aga ggg gat tta aat tat tat aaa gag gtt tat att gag gct gct     480
   Tyr Arg Gly Asp Leu Asn Tyr Tyr Lys Glu Val Tyr Ile Glu Ala Ala
   145                 150                 155                 160
   tta aag tct tta act aaa gaa aat gca ggt ctt tct aga gtg tac agt     528
   Leu Lys Ser Leu Thr Lys Glu Asn Ala Gly Leu Ser Arg Val Tyr Ser
                   165                 170                 175
   caa tgg gct gga aag aca caa ata ttt att cct ctt aaa aag aat att     576
   Gln Trp Ala Gly Lys Thr Gln Ile Phe Ile Pro Leu Lys Lys Asn Ile
               180                 185                 190
   tta tct gga aaa gtt gag tct gac att gat att gac agt ttg gtt aca     624
   Leu Ser Gly Lys Val Glu Ser Asp Ile Asp Ile Asp Ser Leu Val Thr
           195                 200                 205
   gat aag gtt gtg gca gct ctt tta agc gag aat gaa gca ggt gtt aac     672
   Asp Lys Val Val Ala Ala Leu Leu Ser Glu Asn Glu Ala Gly Val Asn
       210                 215                 220
   ttt gca aga gat att aca gat att caa ggc gaa act cat aaa gca gat     720
   Phe Ala Arg Asp Ile Thr Asp Ile Gln Gly Glu Thr His Lys Ala Asp
   225                 230                 235                 240
   caa gat aaa att gat att gaa tta gat aat gtt cat aaa agt gat tcc     768
   Gln Asp Lys Ile Asp Ile Glu Leu Asp Asn Val His Lys Ser Asp Ser
                   245                 250                 255
   aat ata aca gag act att gag aat tta aga gat cag ctt gaa aag gct     816
   Asn Ile Thr Glu Thr Ile Glu Asn Leu Arg Asp Gln Leu Glu Lys Ala
               260                 265                 270
   aca gat gaa gag cat aga aaa gag att gaa agt cag gtt gat gct aaa     864
   Thr Asp Glu Glu His Arg Lys Glu Ile Glu Ser Gln Val Asp Ala Lys
           275                 280                 285
   aag aaa caa aaa gaa gaa cta gat aaa aag gca atc gat ctt gat aaa    912
   Lys Lys Gln Lys Glu Glu Leu Asp Lys Lys Ala Ile Asp Leu Asp Lys
       290                 295                 300
   gcc caa caa aaa tta gat tct tct gaa gat aat tta gat att caa agg    960
   Ala Gln Gln Lys Leu Asp Ser Ser Glu Asp Asn Leu Asp Ile Gln Arg
   305                 310                 315                 320
   gat act gtt aga gag aag att caa gag gat att gac gag att aat aaa   1008
   Asp Thr Val Arg Glu Lys Ile Gln Glu Asp Ile Asp Glu Ile Asn Lys
                   325                 330                 335
   gaa aag aat ttg cca aaa cct ggt gat gta agt tct cct aaa gtt gat   1056
   Glu Lys Asn Leu Pro Lys Pro Gly Asp Val Ser Ser Pro Lys Val Asp
               340                 345                 350
   aag cag cta caa ata aaa gag agt cta gaa gac ttg cag gaa cag ctt   1104
   Lys Gln Leu Gln Ile Lys Glu Ser Leu Glu Asp Leu Gln Glu Gln Leu
           355                 360                 365
   aaa gaa act agc gat gaa aat caa aaa aga gaa att gaa aag caa att   1152
   Lys Glu Thr Ser Asp Glu Asn Gln Lys Arg Glu Ile Glu Lys Gln Ile
       370                 375                 380
   gaa atc aaa aaa agt gat gaa gaa ctt tta aaa agt aaa gat cct aaa   1200
   Glu Ile Lys Lys Ser Asp Glu Glu Leu Leu Lys Ser Lys Asp Pro Lys
   385                 390                 395                 400
   gca tta gat ctt aat gga gat tta aat tct aaa gtt tct agt aaa gaa   1248
   Ala Leu Asp Leu Asn Gly Asp Leu Asn Ser Lys Val Ser Ser Lys Glu
                   405                 410                 415
   aaa att aaa ggc aaa gaa gga gaa ata gtc aaa gag gaa tca aag gca   1296
   Lys Ile Lys Gly Lys Glu Gly Glu Ile Val Lys Glu Glu Ser Lys Ala
               420                 425                 430
   agt tta gct gat ttg aat aat gac gaa aat ctt atg agg ccg gaa gat   1344
   Ser Leu Ala Asp Leu Asn Asn Asp Glu Asn Leu Met Arg Pro Glu Asp
           435                 440                 445
   caa aaa tta tct gag gat aaa aaa tta gat agt aaa aaa aat tta aaa   1392
   Gln Lys Leu Ser Glu Asp Lys Lys Leu Asp Ser Lys Lys Asn Leu Lys
       450                 455                 460
   cct gtt tct gag att gag aga gta aat gaa att tcg aag tct aac aac   1440
   Pro Val Ser Glu Ile Glu Arg Val Asn Glu Ile Ser Lys Ser Asn Asn
   465                 470                 475                 480
   aat gag att agt gaa tca tca cca tta tat aag cct tct tat agc gat   1488
   Asn Glu Ile Ser Glu Ser Ser Pro Leu Tyr Lys Pro Ser Tyr Ser Asp
                   485                 490                 495
   atg gat tca aaa gag ggt ata gat aat aaa gat gtt aac ttg caa gaa   1536
   Met Asp Ser Lys Glu Gly Ile Asp Asn Lys Asp Val Asn Leu Gln Glu
               500                 505                 510
   acc aag tct caa act aaa agt caa cct act tct tta aat caa gat ttg   1584
   Thr Lys Ser Gln Thr Lys Ser Gln Pro Thr Ser Leu Asn Gln Asp Leu
           515                 520                 525
   act act atg tct ata gat tct agt aat cct gta ttt tta gag gtt att      1632
   Thr Thr Met Ser Ile Asp Ser Ser Asn Pro Val Phe Leu Glu Val Ile
       530                 535                 540
   gat cct att aca aat tta gga acg ctt caa ctt att gat ttg aat acc      1680
   Asp Pro Ile Thr Asn Leu Gly Thr Leu Gln Leu Ile Asp Leu Asn Thr
   545                 550                 555                 560
   ggt gtt aga ctt aaa gaa agt act cag caa ggc att cag cgg tat gga      1728
   Gly Val Arg Leu Lys Glu Ser Thr Gln Gln Gly Ile Gln Arg Tyr Gly
                   565                 570                 575
   att tat gaa cgt gaa aaa gat tta gtt gtt att aaa atg gat tca gga      1776
   Ile Tyr Glu Arg Glu Lys Asp Leu Val Val Ile Lys Met Asp Ser Gly
               580                 585                 590
   aaa gcc aag ctt caa ata ctt aat aaa ctt gag aat tta aaa gtg ata      1824
   Lys Ala Lys Leu Gln Ile Leu Asn Lys Leu Glu Asn Leu Lys Val Ile
           595                 600                 605
   tcg gag tct aat ttt gag att aat aaa aat tca tct ctt tat gtt gac      1872
   Ser Glu Ser Asn Phe Glu Ile Asn Lys Asn Ser Ser Leu Tyr Val Asp
       610                 615                 620
   tct aaa atg att tta gta gtt gtg aga gat agt ggt aat gtt tgg aga      1920
   Ser Lys Met Ile Leu Val Val Val Arg Asp Ser Gly Asn Val Trp Arg
   625                 630                 635                 640
   ttg gct aaa ttt tct cct aaa aat tta aat gag ttt att ctt tca gag      1968
   Leu Ala Lys Phe Ser Pro Lys Asn Leu Asn Glu Phe Ile Leu Ser Glu
                   645                 650                 655
   aat aaa att ttg cct ttt act agc ttt tct gtg aga aag aat ttt att      2016
   Asn Lys Ile Leu Pro Phe Thr Ser Phe Ser Val Arg Lys Asn Phe Ile
               660                 665                 670
   tat ttg cag gat gag ttt aaa agt ctt att act tta gat gta aat act      2064
   Tyr Leu Gln Asp Glu Phe Lys Ser Leu Ile Thr Leu Asp Val Asn Thr
           675                 680                 685
   tta aaa aaa gtt aag ta                                               2081
   Leu Lys Lys Val Lys
       690
<210> 68
   <211> 693
   <212> PRT
   <213> Borrelia burgdorferi
<400> 68
   Met Lys Lys Leu Leu Leu Ile Phe Ser Phe Phe Leu Ile Ser Leu Asn
   1               5                   10                  15
   Gly Phe Pro Leu Asn Ser Arg Glu Val Asp Lys Glu Lys Leu Lys Asp
               20                  25                  30
   Phe Val Asn Met Asp Leu Glu Phe Val Asn Tyr Lys Gly Pro Tyr Asp
           35                  40                  45
   Ser Thr Asn Thr Tyr Glu Gln Ile Val Gly Ile Gly Glu Phe Leu Ala
       50                  55                  60
   Arg Pro Leu Ile Asn Ser Asn Ser Asn Ser Ile Tyr Tyr Gly Lys Tyr
   65                  70                  75                  80
   Phe Ile Asn Arg Phe Ile Asp Asp Gln Asp Lys Lys Ala Ser Val Asp
                   85                  90                  95
   Val Phe Ser Ile Gly Ser Arg Ser Gln Leu Asp Ser Ile Leu Asn Leu
               100                 105                 110
   Arg Arg Ile Leu Thr Gly Tyr Leu Ile Lys Ser Phe Asp Tyr Glu Arg
           115                 120                 125
   Ser Ser Ala Glu Leu Ile Ala Lys Val Ile Thr Ile His Asn Ala Val
       130                 135                 140
   Tyr Arg Gly Asp Leu Asn Tyr Tyr Lys Glu Val Tyr Ile Glu Ala Ala
   145                 150                 155                 160
   Leu Lys Ser Leu Thr Lys Glu Asn Ala Gly Leu Ser Arg Val Tyr Ser
                   165                 170                 175
   Gln Trp Ala Gly Lys Thr Gln Ile Phe Ile Pro Leu Lys Lys Asn Ile
               180                 185                 190
   Leu Ser Gly Lys Val Glu Ser Asp Ile Asp Ile Asp Ser Leu Val Thr
           195                 200                 205
   Asp Lys Val Val Ala Ala Leu Leu Ser Glu Asn Glu Ala Gly Val Asn
       210                 215                 220
   Phe Ala Arg Asp Ile Thr Asp Ile Gln Gly Glu Thr His Lys Ala Asp
   225                 230                 235                 240
   Gln Asp Lys Ile Asp Ile Glu Leu Asp Asn Val His Lys Ser Asp Ser
                   245                 250                 255
   Asn Ile Thr Glu Thr Ile Glu Asn Leu Arg Asp Gln Leu Glu Lys Ala
               260                 265                 270
   Thr Asp Glu Glu His Arg Lys Glu Ile Glu Ser Gln Val Asp Ala Lys
           275                 280                 285
   Lys Lys Gln Lys Glu Glu Leu Asp Lys Lys Ala Ile Asp Leu Asp Lys
       290                 295                 300
   Ala Gln Gln Lys Leu Asp Ser Ser Glu Asp Asn Leu Asp Ile Gln Arg
   305                 310                 315                 320
   Asp Thr Val Arg Glu Lys Ile Gln Glu Asp Ile Asp Glu Ile Asn Lys
                   325                 330                 335
   Glu Lys Asn Leu Pro Lys Pro Gly Asp Val Ser Ser Pro Lys Val Asp
               340                 345                 350
   Lys Gln Leu Gln Ile Lys Glu Ser Leu Glu Asp Leu Gln Glu Gln Leu
           355                 360                 365
   Lys Glu Thr Ser Asp Glu Asn Gln Lys Arg Glu Ile Glu Lys Gln Ile
       370                 375                 380
   Glu Ile Lys Lys Ser Asp Glu Glu Leu Leu Lys Ser Lys Asp Pro Lys
   385                 390                 395                 400
   Ala Leu Asp Leu Asn Gly Asp Leu Asn Ser Lys Val Ser Ser Lys Glu
                   405                 410                 415
   Lys Ile Lys Gly Lys Glu Gly Glu Ile Val Lys Glu Glu Ser Lys Ala
               420                 425                 430
   Ser Leu Ala Asp Leu Asn Asn Asp Glu Asn Leu Met Arg Pro Glu Asp
           435                 440                 445
   Gln Lys Leu Ser Glu Asp Lys Lys Leu Asp Ser Lys Lys Asn Leu Lys
       450                 455                 460
   Pro Val Ser Glu Ile Glu Arg Val Asn Glu Ile Ser Lys Ser Asn Asn
   465                 470                 475                 480
   Asn Glu Ile Ser Glu Ser Ser Pro Leu Tyr Lys Pro Ser Tyr Ser Asp
                   485                 490                 495
   Met Asp Ser Lys Glu Gly Ile Asp Asn Lys Asp Val Asn Leu Gln Glu
               500                 505                 510
   Thr Lys Ser Gln Thr Lys Ser Gln Pro Thr Ser Leu Asn Gln Asp Leu
           515                 520                 525
   Thr Thr Met Ser Ile Asp Ser Ser Asn Pro Val Phe Leu Glu Val Ile
       530                 535                 540
   Asp Pro Ile Thr Asn Leu Gly Thr Leu Gln Leu Ile Asp Leu Asn Thr
   545                 550                 555                 560
   Gly Val Arg Leu Lys Glu Ser Thr Gln Gln Gly Ile Gln Arg Tyr Gly
                   565                 570                 575
   Ile Tyr Glu Arg Glu Lys Asp Leu Val Val Ile Lys Met Asp Ser Gly
               580                 585                 590
   Lys Ala Lys Leu Gln Ile Leu Asn Lys Leu Glu Asn Leu Lys Val Ile
           595                 600                 605
   Ser Glu Ser Asn Phe Glu Ile Asn Lys Asn Ser Ser Leu Tyr Val Asp
       610                 615                 620
   Ser Lys Met Ile Leu Val Val Val Arg Asp Ser Gly Asn Val Trp Arg
   625                 630                 635                 640
   Leu Ala Lys Phe Ser Pro Lys Asn Leu Asn Glu Phe Ile Leu Ser Glu
               645                 650                 655
   Asn Lys Ile Leu Pro Phe Thr Ser Phe Ser Val Arg Lys Asn Phe Ile
               660                 665                 670
   Tyr Leu Gln Asp Glu Phe Lys Ser Leu Ile Thr Leu Asp Val Asn Thr
           675                 680                 685
   Leu Lys Lys Val Lys
       690
<210> 69
   <211> 1991
   <212> DNA
   <213> Borrelia burgdorferi
<220>
   <221> CDS
   <222> (1)...(1989)
<400> 69
   atg aaa aaa atg tta cta atc ttt agt ttt ttt ctt gtt ttt tta aat      48
   Met Lys Lys Met Leu Leu Ile Phe Ser Phe Phe Leu Val Phe Leu Asn
    1               5                  10                  15
   gga ttt cct ctt aat gca agg gaa gtt gat aag gaa aaa tta aag gac      96
   Gly Phe Pro Leu Asn Ala Arg Glu Val Asp Lys Glu Lys Leu Lys Asp
               20                  25                  30
   ttt gtt aat atg gat ctt gaa ttt gtt aat tac aag ggt cct tat gat     144
   Phe Val Asn Met Asp Leu Glu Phe Val Asn Tyr Lys Gly Pro Tyr Asp
           35                  40                  45
   tct aca gat aca tat gaa caa ata gta ggt att ggg gag ttt tta gca     192
   Ser Thr Asp Thr Tyr Glu Gln Ile Val Gly Ile Gly Glu Phe Leu Ala
       50                  55                  60
   agg ccg ttg aac aat tcc aat agt aat tca agt tat tat ggt aaa tat     240
   Arg Pro Leu Asn Asn Ser Asn Ser Asn Ser Ser Tyr Tyr Gly Lys Tyr
   65                  70                  75                  80
   ttt gtt aat aga ttt att gac gat caa gat aaa aaa gca agt gtt gat     288
   Phe Val Asn Arg Phe Ile Asp Asp Gln Asp Lys Lys Ala Ser Val Asp
                   85                  90                  95
   att ttt tct att ggt agt aag tca gag ctt gat agt ata tta aat cta     336
   Ile Phe Ser Ile Gly Ser Lys Ser Glu Leu Asp Ser Ile Leu Asn Leu
               100                 105                 110
   aga aga att ctt aca ggg tat tta atg aag tct ttt gat tat gag agg     384
   Arg Arg Ile Leu Thr Gly Tyr Leu Met Lys Ser Phe Asp Tyr Glu Arg
           115                 120                 125
   tct agt gcg gaa tta att gct aaa gct att aca ata tat aat gct gtt     432
   Ser Ser Ala Glu Leu Ile Ala Lys Ala Ile Thr Ile Tyr Asn Ala Val
       130                 135                 140
   tat aga gga gat tta gat tat tac aaa gag ttt tat att gag gct tct     480
   Tyr Arg Gly Asp Leu Asp Tyr Tyr Lys Glu Phe Tyr Ile Glu Ala Ser
   145                 150                 155                 160
   ttg aag tct ttg act aaa gaa aat gca ggt ctt tct agg gtg tac agt     528
   Leu Lys Ser Leu Thr Lys Glu Asn Ala Gly Leu Ser Arg Val Tyr Ser
                   165                 170                 175
   caa tgg gct ggg aag aca caa ata ttt att cct ctt aaa aag aat att     576
   Gln Trp Ala Gly Lys Thr Gln Ile Phe Ile Pro Leu Lys Lys Asn Ile
               180                 185                 190
   tta tct gga aat gtt gag tct gac att gat att gat agt ttg gtt aca     624
   Leu Ser Gly Asn Val Glu Ser Asp Ile Asp Ile Asp Ser Leu Val Thr
           195                 200                 205
   gat aag gtg gtg gca gct ctt tta agt gag aat gaa tca ggt gtt aac     672
   Asp Lys Val Val Ala Ala Leu Leu Ser Glu Asn Glu Ser Gly Val Asn
       210                 215                 220
   ttt gca aga gat att aca gac att caa ggc gaa act cat aaa gca gat     720
   Phe Ala Arg Asp Ile Thr Asp Ile Gln Gly Glu Thr His Lys Ala Asp
   225                 230                 235                 240
   caa gat aaa att gat att gaa tta gat aat ttt cat gaa agt gat tcc     768
   Gln Asp Lys Ile Asp Ile Glu Leu Asp Asn Phe His Glu Ser Asp Ser
                   245                 250                 255
   aat ata aca gaa act att gag aat tta agg gat cag ctt gaa aaa gct     816
   Asn Ile Thr Glu Thr Ile Glu Asn Leu Arg Asp Gln Leu Glu Lys Ala
               260                 265                 270
   aca gat gaa gag cat aaa aaa gag att gaa agt cag gtt gat gct aaa     864
   Thr Asp Glu Glu His Lys Lys Glu Ile Glu Ser Gln Val Asp Ala Lys
           275                 280                 285
   aag aaa caa aag gaa gaa tta gat aaa aag gca att gat ctt gat aaa     912
   Lys Lys Gln Lys Glu Glu Leu Asp Lys Lys Ala Ile Asp Leu Asp Lys
       290                 295                 300
   gct caa caa aaa tta gat ttt gct gaa gat aat cta gat att caa agg     960
   Ala Gln Gln Lys Leu Asp Phe Ala Glu Asp Asn Leu Asp Ile Gln Arg
   305                 310                 315                 320
   gat act gtt aga gag aag ctt caa gaa aat att aac gag act aat aag    1008
   Asp Thr Val Arg Glu Lys Leu Gln Glu Asn Ile Asn Glu Thr Asn Lys
                   325                 330                 335
   gaa aag aat tta cca aag cct ggt gat gta agt tct cct aag gtt gat    1056
   Glu Lys Asn Leu Pro Lys Pro Gly Asp Val Ser Ser Pro Lys Val Asp
               340                 345                 350
   aag cag ttg cag ata aaa gag agt cta gaa gat ttg caa gag cag ctt    1104
   Lys Gln Leu Gln Ile Lys Glu Ser Leu Glu Asp Leu Gln Glu Gln Leu
           355                 360                 365
   aaa gaa gct agt gat gaa aat caa aaa aga gaa ata gaa aag caa att      1152
   Lys Glu Ala Ser Asp Glu Asn Gln Lys Arg Glu Ile Glu Lys Gln Ile
       370                 375                 380
   gaa atc aaa aaa aat gat gaa gaa ctt ttt aaa aat aaa gat cat aaa      1200
   Glu Ile Lys Lys Asn Asp Glu Glu Leu Phe Lys Asn Lys Asp His Lys
   385                 390                 395                 400
   gca tta gat ctt aag caa gaa tta aat tct aaa gct tct agt aaa gaa      1248
   Ala Leu Asp Leu Lys Gln Glu Leu Asn Ser Lys Ala Ser Ser Lys Glu
                   405                 410                 415
   aaa att gaa ggc gaa gaa gag gat aaa gaa tta gat agt aaa aaa aat      1296
   Lys Ile Glu Gly Glu Glu Glu Asp Lys Glu Leu Asp Ser Lys Lys Asn
               420                 425                 430
   tta gag cct gtt tct gag gct gat aaa gta gat aaa att tcc aag tct      1344
   Leu Glu Pro Val Ser Glu Ala Asp Lys Val Asp Lys Ile Ser Lys Ser
           435                 440                 445
   aac aac aat gag gtt agt aaa tta tcc ccg tta gat gag cct tct tat      1392
   Asn Asn Asn Glu Val Ser Lys Leu Ser Pro Leu Asp Glu Pro Ser Tyr
       450                 455                 460
   agc gac att gat tcg aaa gag ggt gta gat aac aaa gat gtt gat ttg      1440
   Ser Asp Ile Asp Ser Lys Glu Gly Val Asp Asn Lys Asp Val Asp Leu
   465                 470                 475                 480
   caa aaa act aaa ccc caa gtt gaa agt caa cct act tcg tta aat gaa      1488
   Gln Lys Thr Lys Pro Gln Val Glu Ser Gln Pro Thr Ser Leu Asn Glu
                   485                 490                 495
   gat ttg att gat gtg tct ata gat tcc agt aat cct gtc ttt tta gag      1536
   Asp Leu Ile Asp Val Ser Ile Asp Ser Ser Asn Pro Val Phe Leu Glu
               500                 505                 510
   gtt atc gat ccg att aca aat tta gga acg ctt caa ctt att gat ttg      1584
   Val Ile Asp Pro Ile Thr Asn Leu Gly Thr Leu Gln Leu Ile Asp Leu
           515                 520                 525
   aat acc ggt gtt aga ctt aaa gaa agt gct caa caa ggt att cag cga      1632
   Asn Thr Gly Val Arg Leu Lys Glu Ser Ala Gln Gln Gly Ile Gln Arg
       530                 535                 540
   tat gga att tat gaa cgt gaa aaa gat ttg gtt gtt att aaa ata gat      1680
   Tyr Gly Ile Tyr Glu Arg Glu Lys Asp Leu Val Val Ile Lys Ile Asp
   545                 550                 555                 560
   tca gga aaa gct aag ctt cag ata ctt gat aaa ctc gag aat tta aaa      1728
   Ser Gly Lys Ala Lys Leu Gln Ile Leu Asp Lys Leu Glu Asn Leu Lys
                   565                 570                 575
   gtg ata tca gag tct aat ttt gag att aat aaa aat tca tct ctt tat      1776
   Val Ile Ser Glu Ser Asn Phe Glu Ile Asn Lys Asn Ser Ser Leu Tyr
               580                 585                 590
   gtt gac tct aga atg att tta gta gtt gtt aag gac gat agt aat gct      1824
   Val Asp Ser Arg Met Ile Leu Val Val Val Lys Asp Asp Ser Asn Ala
           595                 600                 605
   tgg aga ttg gct aaa ttt tct cct aaa aat tta gat gaa ttt att ctg    1872
   Trp Arg Leu Ala Lys Phe Ser Pro Lys Asn Leu Asp Glu Phe Ile Leu
       610                 615                 620
   tca gaa aat aaa att ttg cct ttt act agc ttt gct gtg aga aag aat    1920
   Ser Glu Asn Lys Ile Leu Pro Phe Thr Ser Phe Ala Val Arg Lys Asn
   625                 630                 635                 640
   ttt att tat ttg caa gat gaa ctt aaa agc tta gtt act tta gat gta    1968
   Phe Ile Tyr Leu Gln Asp Glu Leu Lys Ser Leu Val Thr Leu Asp Val
                   645                 650                 655
   aat act tta aaa aaa gtt aag ta                                     1991
   Asn Thr Leu Lys Lys Val Lys
               660
<210> 70
   <211> 663
   <212> PRT
   <213> Borrelia burgdorferi
<400> 70
   Met Lys Lys Met Leu Leu Ile Phe Ser Phe Phe Leu Val Phe Leu Asn
   1               5                   10                  15
   Gly Phe Pro Leu Asn Ala Arg Glu Val Asp Lys Glu Lys Leu Lys Asp
               20                  25                  30
   Phe Val Asn Met Asp Leu Glu Phe Val Asn Tyr Lys Gly Pro Tyr Asp
           35                  40                  45
   Ser Thr Asp Thr Tyr Glu Gln Ile Val Gly Ile Gly Glu Phe Leu Ala
       50                  55                  60
   Arg Pro Leu Asn Asn Ser Asn Ser Asn Ser Ser Tyr Tyr Gly Lys Tyr
   65                  70                  75                  80
   Phe Val Asn Arg Phe Ile Asp Asp Gln Asp Lys Lys Ala Ser Val Asp
                   85                  90                  95
   Ile Phe Ser Ile Gly Ser Lys Ser Glu Leu Asp Ser Ile Leu Asn Leu
               100                 105                 110
   Arg Arg Ile Leu Thr Gly Tyr Leu Met Lys Ser Phe Asp Tyr Glu Arg
           115                 120                 125
   Ser Ser Ala Glu Leu Ile Ala Lys Ala Ile Thr Ile Tyr Asn Ala Val
       130                 135                 140
   Tyr Arg Gly Asp Leu Asp Tyr Tyr Lys Glu Phe Tyr Ile Glu Ala Ser
   145                 150                 155                 160
   Leu Lys Ser Leu Thr Lys Glu Asn Ala Gly Leu Ser Arg Val Tyr Ser
                   165                 170                 175
   Gln Trp Ala Gly Lys Thr Gln Ile Phe Ile Pro Leu Lys Lys Asn Ile
               180                 185                 190
   Leu Ser Gly Asn Val Glu Ser Asp Ile Asp Ile Asp Ser Leu Val Thr
           195                 200                 205
   Asp Lys Val Val Ala Ala Leu Leu Ser Glu Asn Glu Ser Gly Val Asn
       210                 215                 220
   Phe Ala Arg Asp Ile Thr Asp Ile Gln Gly Glu Thr His Lys Ala Asp
   225                 230                 235                 240
   Gln Asp Lys Ile Asp Ile Glu Leu Asp Asn Phe His Glu Ser Asp Ser
                   245                 250                 255
   Asn Ile Thr Glu Thr Ile Glu Asn Leu Arg Asp Gln Leu Glu Lys Ala
               260                 265                 270
   Thr Asp Glu Glu His Lys Lys Glu Ile Glu Ser Gln Val Asp Ala Lys
           275                 280                 285
   Lys Lys Gln Lys Glu Glu Leu Asp Lys Lys Ala Ile Asp Leu Asp Lys
       290                 295                 300
   Ala Gln Gln Lys Leu Asp Phe Ala Glu Asp Asn Leu Asp Ile Gln Arg
   305                 310                 315                 320
   Asp Thr Val Arg Glu Lys Leu Gln Glu Asn Ile Asn Glu Thr Asn Lys
                   325                 330                 335
   Glu Lys Asn Leu Pro Lys Pro Gly Asp Val Ser Ser Pro Lys Val Asp
               340                 345                 350
   Lys Gln Leu Gln Ile Lys Glu Ser Leu Glu Asp Leu Gln Glu Gln Leu
           355                 360                 365
   Lys Glu Ala Ser Asp Glu Asn Gln Lys Arg Glu Ile Glu Lys Gln Ile
       370                 375                 380
   Glu Ile Lys Lys Asn Asp Glu Glu Leu Phe Lys Asn Lys Asp His Lys
   385                 390                 395                 400
   Ala Leu Asp Leu Lys Gln Glu Leu Asn Ser Lys Ala Ser Ser Lys Glu
                   405                 410                 415
   Lys Ile Glu Gly Glu Glu Glu Asp Lys Glu Leu Asp Ser Lys Lys Asn
               420                 425                 430
   Leu Glu Pro Val Ser Glu Ala Asp Lys Val Asp Lys Ile Ser Lys Ser
           435                 440                 445
   Asn Asn Asn Glu Val Ser Lys Leu Ser Pro Leu Asp Glu Pro Ser Tyr
       450                 455                 460
   Ser Asp Ile Asp Ser Lys Glu Gly Val Asp Asn Lys Asp Val Asp Leu
   465                 470                 475                 480
   Gln Lys Thr Lys Pro Gln Val Glu Ser Gln Pro Thr Ser Leu Asn Glu
                   485                 490                 495
   Asp Leu Ile Asp Val Ser Ile Asp Ser Ser Asn Pro Val Phe Leu Glu
               500                 505                 510
   Val Ile Asp Pro Ile Thr Asn Leu Gly Thr Leu Gln Leu Ile Asp Leu
           515                 520                 525
   Asn Thr Gly Val Arg Leu Lys Glu Ser Ala Gln Gln Gly Ile Gln Arg
       530                 535                 540
   Tyr Gly Ile Tyr Glu Arg Glu Lys Asp Leu Val Val Ile Lys Ile Asp
   545                 550                 555                 560
   Ser Gly Lys Ala Lys Leu Gln Ile Leu Asp Lys Leu Glu Asn Leu Lys
                   565                 570                 575
   Val Ile Ser Glu Ser Asn Phe Glu Ile Asn Lys Asn Ser Ser Leu Tyr
               580                 585                 590
   Val Asp Ser Arg Met Ile Leu Val Val Val Lys Asp Asp Ser Asn Ala
           595                 600                 605
   Trp Arg Leu Ala Lys Phe Ser Pro Lys Asn Leu Asp Glu Phe Ile Leu
       610                 615                 620
   Ser Glu Asn Lys Ile Leu Pro Phe Thr Ser Phe Ala Val Arg Lys Asn
   625                 630                 635                 640
   Phe Ile Tyr Leu Gln Asp Glu Leu Lys Ser Leu Val Thr Leu Asp Val
                   645                 650                 655
   Asn Thr Leu Lys Lys Val Lys
               660
<210> 71
   <211> 2081
   <212> DNA
   <213> Borrelia burgdorferi
<220>
   <221> CDS
   <222> (1) ... (2079)
<400> 71
   atg aaa aaa atg tta cta atc ttt agt ttt ttt ctt att tct ttg aat     48
   Met Lys Lys Met Leu Leu Ile Phe Ser Phe Phe Leu Ile Ser Leu Asn
    1               5                  10                  15
   gga ttt ccc ctt aat gca agg gaa gtt gat aag gaa aaa tta aag gac      96
   Gly Phe Pro Leu Asn Ala Arg Glu Val Asp Lys Glu Lys Leu Lys Asp
               20                  25                  30
   ttt gtt aat atg gat ctt gag ttt gta aac tat aaa ggt cct tat gat     144
   Phe Val Asn Met Asp Leu Glu Phe Val Asn Tyr Lys Gly Pro Tyr Asp
           35                  40                  45
   tct aca aat aca tat gaa caa ata gta ggt att ggt gag ttt tta gca     192
   Ser Thr Asn Thr Tyr Glu Gln Ile Val Gly Ile Gly Glu Phe Leu Ala
       50                  55                  60
   aga cca ttg att aat ttc aat agc aac tca agt tat tat ggt aaa tat     240
   Arg Pro Leu Ile Asn Phe Asn Ser Asn Ser Ser Tyr Tyr Gly Lys Tyr
   65                  70                  75                  80
   ttt att aat aga ttt att gac gat caa gat aaa aaa gca agc gtt gat     288
   Phe Ile Asn Arg Phe Ile Asp Asp Gln Asp Lys Lys Ala Ser Val Asp
                   85                  90                  95
   gtt ttt tct att agt agt aag tca cag ctt gac agt ata ttg aat tta     336
   Val Phe Ser Ile Ser Ser Lys Ser Gln Leu Asp Ser Ile Leu Asn Leu
               100                 105                 110
   aga aga att ctt aca ggg tat ttg ata aag tct ttt gat tat gaa aga     384
   Arg Arg Ile Leu Thr Gly Tyr Leu Ile Lys Ser Phe Asp Tyr Glu Arg
           115                 120                 125
   tct agt gct gaa tta att gcc aag gtt att aca ata cat aat gct gtt     432
   Ser Ser Ala Glu Leu Ile Ala Lys Val Ile Thr Ile His Asn Ala Val
       130                 135                 140
   tat aga ggt gat tta aat tat tat aaa gag ttt tat att gag tct gct     480
   Tyr Arg Gly Asp Leu Asn Tyr Tyr Lys Glu Phe Tyr Ile Glu Ser Ala
   145                 150                 155                 160
   tta aag tct tta act aaa gaa aat gca ggt ctt tct aga gtg tac agt     528
   Leu Lys Ser Leu Thr Lys Glu Asn Ala Gly Leu Ser Arg Val Tyr Ser
                   165                 170                 175
   caa tgg gct gga aag aca caa ata ttt att cct ctt aaa aag aat att     576
   Gln Trp Ala Gly Lys Thr Gln Ile Phe Ile Pro Leu Lys Lys Asn Ile
               180                 185                 190
   tta tct gga aaa att gag tct gac att gat att gat agt ttg gtt aca     624
   Leu Ser Gly Lys Ile Glu Ser Asp Ile Asp Ile Asp Ser Leu Val Thr
           195                 200                 205
   gat aag gtt gtg gca gct ctt tta agc gaa aat gaa gca ggt gtt aac     672
   Asp Lys Val Val Ala Ala Leu Leu Ser Glu Asn Glu Ala Gly Val Asn
       210                 215                 220
   ttt gca agg gat att aca gat att caa gga gaa act cat aaa gca gat     720
   Phe Ala Arg Asp Ile Thr Asp Ile Gln Gly Glu Thr His Lys Ala Asp
   225                 230                 235                 240
   caa gat aaa att gat att gaa tta gat aat gtt cat gaa agt gat tcc     768
   Gln Asp Lys Ile Asp Ile Glu Leu Asp Asn Val His Glu Ser Asp Ser
                   245                 250                 255
   aat ata aca gaa act att gag aat tta aga gat cag ctt gaa aag gct      816
   Asn Ile Thr Glu Thr Ile Glu Asn Leu Arg Asp Gln Leu Glu Lys Ala
               260                 265                 270
   aca gat gaa gag cat aga aaa gag att gaa agt caa gtt gat gct aaa      864
   Thr Asp Glu Glu His Arg Lys Glu Ile Glu Ser Gln Val Asp Ala Lys
           275                 280                 285
   aag aaa caa aaa gaa gaa cta gat aaa aag gca atc gat ctt gat aaa      912
   Lys Lys Gln Lys Glu Glu Leu Asp Lys Lys Ala Ile Asp Leu Asp Lys
       290                 295                 300
   gcc caa caa aaa tta gat ttt tct gaa gat aat tta gat att caa agg      960
   Ala Gln Gln Lys Leu Asp Phe Ser Glu Asp Asn Leu Asp Ile Gln Arg
   305                 310                 315                 320
   gat act gtt aga gag aag att caa gag gat att aac gag att aat aag     1008
   Asp Thr Val Arg Glu Lys Ile Gln Glu Asp Ile Asn Glu Ile Asn Lys
                   325                 330                 335
   gaa aag aat tta cca aaa cct ggt gat gta agt tct cct aaa gtt gat     1056
   Glu Lys Asn Leu Pro Lys Pro Gly Asp Val Ser Ser Pro Lys Val Asp
               340                 345                 350
   aag cag cta caa ata aaa gag agt cta gaa gac ttg cag gag cag ctt     1104
   Lys Gln Leu Gln Ile Lys Glu Ser Leu Glu Asp Leu Gln Glu Gln Leu
           355                 360                 365
   aaa gaa act agc gat gaa aat caa aaa aga gaa att gaa aag caa att     1152
   Lys Glu Thr Ser Asp Glu Asn Gln Lys Arg Glu Ile Glu Lys Gln Ile
       370                 375                 380
   gaa atc aaa aaa agt gat gaa gaa ctt tta aaa agc aaa gat cct aaa     1200
   Glu Ile Lys Lys Ser Asp Glu Glu Leu Leu Lys Ser Lys Asp Pro Lys
   385                 390                 395                 400
   gca tta gat ctt aat cga gat tta aat tct aaa gct tct agt aaa gaa     1248
   Ala Leu Asp Leu Asn Arg Asp Leu Asn Ser Lys Ala Ser Ser Lys Glu
                   405                 410                 415
   aaa att aaa ggc aaa gaa aaa gaa ata gtc aaa gag aaa tca aag gta     1296
   Lys Ile Lys Gly Lys Glu Lys Glu Ile Val Lys Glu Lys Ser Lys Val
               420                 425                 430
   agt tta ggt gat ttg gat aat gac gaa acc ctt atg acg ccg gaa gat     1344
   Ser Leu Gly Asp Leu Asp Asn Asp Glu Thr Leu Met Thr Pro Glu Asp
           435                 440                 445
   caa aaa tta tct gag gat aaa aaa tta gat agt aaa aaa aat tta aaa     1392
   Gln Lys Leu Ser Glu Asp Lys Lys Leu Asp Ser Lys Lys Asn Leu Lys
       450                 455                 460
   cct gtt tct gag att gag aga gta aat gaa att tca aag tct aac aac     1440
   Pro Val Ser Glu Ile Glu Arg Val Asn Glu Ile Ser Lys Ser Asn Asn
   465                 470                 475                 480
   aat gag gtt agc aaa tca tca cca tta gat aag cct tct tat agt gat     1488
   Asn Glu Val Ser Lys Ser Ser Pro Leu Asp Lys Pro Ser Tyr Ser Asp
                   485                 490                 495
   atc gat tca aaa gag gtt gta gat aat aaa gat gtt aat ttg caa gaa     1536
   Ile Asp Ser Lys Glu Val Val Asp Asn Lys Asp Val Asn Leu Gln Glu
               500                 505                 510
   acc aag cct caa gct aaa agt caa tct act tct tta aat caa gat ttg     1584
   Thr Lys Pro Gln Ala Lys Ser Gln Ser Thr Ser Leu Asn Gln Asp Leu
           515                 520                 525
   att act atg tct ata gat tct agt aat cct gta ttt tta gag gtt att     1632
   Ile Thr Met Ser Ile Asp Ser Ser Asn Pro Val Phe Leu Glu Val Ile
       530                 535                 540
   gat cct att aca aat tta gga atg ctt caa ctt att gat tta aat act     1680
   Asp Pro Ile Thr Asn Leu Gly Met Leu Gln Leu Ile Asp Leu Asn Thr
   545                 550                 555                 560
   ggt gtt aga ctt aaa gaa agc act cag caa ggc att cag cgt tat gga     1728
   Gly Val Arg Leu Lys Glu Ser Thr Gln Gln Gly Ile Gln Arg Tyr Gly
                   565                 570                 575
   att tat gaa cgt gaa aaa gat tta gtt gtt att aaa atg gat tca gga     1776
   Ile Tyr Glu Arg Glu Lys Asp Leu Val Val Ile Lys Met Asp Ser Gly
               580                 585                 590
   aaa gct aag ctt caa ata ctt aat aaa ctt gag aat tta aaa gtg ata     1824
   Lys Ala Lys Leu Gln Ile Leu Asn Lys Leu Glu Asn Leu Lys Val Ile
           595                 600                 605
   tca gag tct aat ttt gag att aat aaa aat tca tct ctt tat gtt gac     1872
   Ser Glu Ser Asn Phe Glu Ile Asn Lys Asn Ser Ser Leu Tyr Val Asp
       610                 615                 620
   tct aaa atg att tta gta gct gtg aaa gat agt ggt aat gtt tgg aga     1920
   Ser Lys Met Ile Leu Val Ala Val Lys Asp Ser Gly Asn Val Trp Arg
   625                 630                 635                 640
   ttg gct aaa ttt tct cct aaa aat tta gat gag ttt att ctt tca gag     1968
   Leu Ala Lys Phe Ser Pro Lys Asn Leu Asp Glu Phe Ile Leu Ser Glu
                   645                 650                 655
   aat aaa att ttg cct ttt act agc ttt tct gtg aga aag aat ttt att     2016
   Asn Lys Ile Leu Pro Phe Thr Ser Phe Ser Val Arg Lys Asn Phe Ile
               660                 665                 670
   tat ttg caa gat gag ttt aaa agt ctt att act tta gat gta aat act     2064
   Tyr Leu Gln Asp Glu Phe Lys Ser Leu Ile Thr Leu Asp Val Asn Thr
           675                 680                 685
   tta aaa aaa gtt aag ta                                              2081
   Leu Lys Lys Val Lys
       690
<210> 72
   <211> 693
   <212> PRT
   <213> Borrelia burgdorferi
<400> 72
   Met Lys Lys Met Leu Leu Ile Phe Ser Phe Phe Leu Ile Ser Leu Asn
   1               5                   10                  15
   Gly Phe Pro Leu Asn Ala Arg Glu Val Asp Lys Glu Lys Leu Lys Asp
               20                  25                  30
   Phe Val Asn Met Asp Leu Glu Phe Val Asn Tyr Lys Gly Pro Tyr Asp
   35 40 45
   Ser Thr Asn Thr Tyr Glu Gln Ile Val Gly Ile Gly Glu Phe Leu Ala
   50 55 60
   Arg Pro Leu Ile Asn Phe Asn Ser Asn Ser Ser Tyr Tyr Gly Lys Tyr
   65 70 75 80
   Phe Ile Asn Arg Phe Ile Asp Asp Gln Asp Lys Lys Ala Ser Val Asp
   85 90 95
   Val Phe Ser Ile Ser Ser Lys Ser Gln Leu Asp Ser Ile Leu Asn Leu
   100 105 110
   Arg Arg Ile Leu Thr Gly Tyr Leu Ile Lys Ser Phe Asp Tyr Glu Arg
   115 120 125
   Ser Ser Ala Glu Leu Ile Ala Lys Val Ile Thr Ile His Asn Ala Val
   130 135 140
   Tyr Arg Gly Asp Leu Asn Tyr Tyr Lys Glu Phe Tyr Ile Glu Ser Ala
   145 150 155 160
   Leu Lys Ser Leu Thr Lys Glu Asn Ala Gly Leu Ser Arg Val Tyr Ser
   165 170 175
   Gln Trp Ala Gly Lys Thr Gln Ile Phe Ile Pro Leu Lys Lys Asn Ile
   180 185 190
   Leu Ser Gly Lys Ile Glu Ser Asp Ile Asp Ile Asp Ser Leu Val Thr
   195 200 205
   Asp Lys Val Val Ala Ala Leu Leu Ser Glu Asn Glu Ala Gly Val Asn
   210 215 220
   Phe Ala Arg Asp Ile Thr Asp Ile Gln Gly Glu Thr His Lys Ala Asp
   225 230 235 240
   Gln Asp Lys Ile Asp Ile Glu Leu Asp Asn Val His Glu Ser Asp Ser
   245 250 255
   Asn Ile Thr Glu Thr Ile Glu Asn Leu Arg Asp Gln Leu Glu Lys Ala
   260 265 270
   Thr Asp Glu Glu His Arg Lys Glu Ile Glu Ser Gln Val Asp Ala Lys
   275 280 285
   Lys Lys Gln Lys Glu Glu Leu Asp Lys Lys Ala Ile Asp Leu Asp Lys
   290 295 300
   Ala Gln Gln Lys Leu Asp Phe Ser Glu Asp Asn Leu Asp Ile Gln Arg
   305 310 315 320
   Asp Thr Val Arg Glu Lys Ile Gln Glu Asp Ile Asn Glu Ile Asn Lys
   325 330 335
   Glu Lys Asn Leu Pro Lys Pro Gly Asp Val Ser Ser Pro Lys Val Asp
   340 345 350
   Lys Gln Leu Gln Ile Lys Glu Ser Leu Glu Asp Leu Gln Glu Gln Leu
   355 360 365
   Lys Glu Thr Ser Asp Glu Asn Gln Lys Arg Glu Ile Glu Lys Gln Ile
   370 375 380
   Glu Ile Lys Lys Ser Asp Glu Glu Leu Leu Lys Ser Lys Asp Pro Lys
   385 390 395 400
   Ala Leu Asp Leu Asn Arg Asp Leu Asn Ser Lys Ala Ser Ser Lys Glu
   405 410 415
   Lys Ile Lys Gly Lys Glu Lys Glu Ile Val Lys Glu Lys Ser Lys Val
   420 425 430
   Ser Leu Gly Asp Leu Asp Asn Asp Glu Thr Leu Met Thr Pro Glu Asp
   435 440 445
   Gln Lys Leu Ser Glu Asp Lys Lys Leu Asp Ser Lys Lys Asn Leu Lys
   450 455 460
   Pro Val Ser Glu Ile Glu Arg Val Asn Glu Ile Ser Lys Ser Asn Asn
   465 470 475 480
   Asn Glu Val Ser Lys Ser Ser Pro Leu Asp Lys Pro Ser Tyr Ser Asp
   485 490 495
   Ile Asp Ser Lys Glu Val Val Asp Asn Lys Asp Val Asn Leu Gln Glu
   500 505 510
   Thr Lys Pro Gln Ala Lys Ser Gln Ser Thr Ser Leu Asn Gln Asp Leu
   515 520 525
   Ile Thr Met Ser Ile Asp Ser Ser Asn Pro Val Phe Leu Glu Val Ile
       530                 535                 540
   Asp Pro Ile Thr Asn Leu Gly Met Leu Gln Leu Ile Asp Leu Asn Thr
   545                 550                 555                 560
   Gly Val Arg Leu Lys Glu Ser Thr Gln Gln Gly Ile Gln Arg Tyr Gly
                   565                 570                 575
   Ile Tyr Glu Arg Glu Lys Asp Leu Val Val Ile Lys Met Asp Ser Gly
               580                 585                 590
   Lys Ala Lys Leu Gln Ile Leu Asn Lys Leu Glu Asn Leu Lys Val Ile
           595                 600                 605
   Ser Glu Ser Asn Phe Glu Ile Asn Lys Asn Ser Ser Leu Tyr Val Asp
       610                 615                 620
   Ser Lys Met Ile Leu Val Ala Val Lys Asp Ser Gly Asn Val Trp Arg
   625                 630                 635                 640
   Leu Ala Lys Phe Ser Pro Lys Asn Leu Asp Glu Phe Ile Leu Ser Glu
                   645                 650                 655
   Asn Lys Ile Leu Pro Phe Thr Ser Phe Ser Val Arg Lys Asn Phe Ile
               660                 665                 670
   Tyr Leu Gln Asp Glu Phe Lys Ser Leu Ile Thr Leu Asp Val Asn Thr
           675                 680                 685
   Leu Lys Lys Val Lys
       690
<210> 73
   <211> 2107
   <212> DNA
   <213> Borrelia burgdorferi
<220>
   <221> CDS
   <222> (1)...(2100)
<400> 73
   atg aaa aaa atg tta cta atc ttt agt ttt ttt ctt gtt ttt tta aat     48
   Met Lys Lys Met Leu Leu Ile Phe Ser Phe Phe Leu Val Phe Leu Asn
    1               5                  10                  15
   gga ttt cct ctt aat gca agg gaa gtt gat aag gaa aaa tta aag gac     96
   Gly Phe Pro Leu Asn Ala Arg Glu Val Asp Lys Glu Lys Leu Lys Asp
               20                  25                  30
   ttt gtt aat atg gat ctt gaa ttt gtt aat tac aag ggt cct tat gat    144
   Phe Val Asn Met Asp Leu Glu Phe Val Asn Tyr Lys Gly Pro Tyr Asp
           35                  40                  45
   tct aca aat aca tat gaa caa ata gta ggt att ggg gag ttt tta gca    192
   Ser Thr Asn Thr Tyr Glu Gln Ile Val Gly Ile Gly Glu Phe Leu Ala
       50                  55                  60
   agg ccg ttg atc aat tcc aat agt aat tca agt tat tat ggt aaa tat    240
   Arg Pro Leu Ile Asn Ser Asn Ser Asn Ser Ser Tyr Tyr Gly Lys Tyr
   65                  70                  75                  80
   ttt gtt aat aga ttt att gac gat caa gat aaa aaa gca agt gtt gat    288
   Phe Val Asn Arg Phe Ile Asp Asp Gln Asp Lys Lys Ala Ser Val Asp
                   85                  90                  95
   att ttt tct att ggt agt aag tca gag ctt gat agt ata tta aat cta    336
   Ile Phe Ser Ile Gly Ser Lys Ser Glu Leu Asp Ser Ile Leu Asn Leu
               100                 105                 110
   aga aga att ctt aca ggg tat tta atg aag tct ttt gat tat gag agg     384
   Arg Arg Ile Leu Thr Gly Tyr Leu Met Lys Ser Phe Asp Tyr Glu Arg
           115                 120                 125
   tct agt gcg gaa tta att gct aaa gct att aca ata tat aat gct gtt     432
   Ser Ser Ala Glu Leu Ile Ala Lys Ala Ile Thr Ile Tyr Asn Ala Val
       130                 135                 140
   tat aga gga gat tta gat tat tac aaa gag ttt tat att gag gct tct     480
   Tyr Arg Gly Asp Leu Asp Tyr Tyr Lys Glu Phe Tyr Ile Glu Ala Ser
   145                 150                 155                 160
   ttg aag tct ttg act aaa gaa aat gca ggt ctt tct agg gtg tac agt     528
   Leu Lys Ser Leu Thr Lys Glu Asn Ala Gly Leu Ser Arg Val Tyr Ser
                   165                 170                 175
   caa tgg gct ggg aag aca caa ata ttt att cct ctt aaa aag aat att     576
   Gln Trp Ala Gly Lys Thr Gln Ile Phe Ile Pro Leu Lys Lys Asn Ile
               180                 185                 190
   tta tct gga aat gtt gag tct gac att gat att gat agt ttg gtt aca     624
   Leu Ser Gly Asn Val Glu Ser Asp Ile Asp Ile Asp Ser Leu Val Thr
           195                 200                 205
   gat aag gtg gtg gca gct ctt tta agt gag aat gaa tca ggt gtt aac     672
   Asp Lys Val Val Ala Ala Leu Leu Ser Glu Asn Glu Ser Gly Val Asn
       210                 215                 220
   ttt gca aga gat att aca gac att caa ggc gaa act cat aaa gca gat     720
   Phe Ala Arg Asp Ile Thr Asp Ile Gln Gly Glu Thr His Lys Ala Asp
   225                 230                 235                 240
   caa gat aaa att gat att gaa tta gat aat att cat gaa agt gat tcc     768
   Gln Asp Lys Ile Asp Ile Glu Leu Asp Asn Ile His Glu Ser Asp Ser
                   245                 250                 255
   aat ata aca gaa act att gag aat tta agg gat cag ctt gaa aaa gct     816
   Asn Ile Thr Glu Thr Ile Glu Asn Leu Arg Asp Gln Leu Glu Lys Ala
               260                 265                 270
   aca gat gaa gag cat aaa aaa gag att gaa agt cag gtt gat gct aaa     864
   Thr Asp Glu Glu His Lys Lys Glu Ile Glu Ser Gln Val Asp Ala Lys
           275                 280                 285
   aag aaa caa aag gaa gaa tta gat aaa aag gca att gat ctt gat aaa     912
   Lys Lys Gln Lys Glu Glu Leu Asp Lys Lys Ala Ile Asp Leu Asp Lys
       290                 295                 300
   gct caa caa aaa tta gat ttt gct gaa gat aat cta gat att caa agg     960
   Ala Gln Gln Lys Leu Asp Phe Ala Glu Asp Asn Leu Asp Ile Gln Arg
   305                 310                 315                 320
   gat act gtt aga gag aag ctt caa gag aat att aac gag act aat aag    1008
   Asp Thr Val Arg Glu Lys Leu Gln Glu Asn Ile Asn Glu Thr Asn Lys
                   325                 330                 335
   gaa aag aat tta cca aag cct ggt gat gta agt tct cct aaa gtt gat    1056
   Glu Lys Asn Leu Pro Lys Pro Gly Asp Val Ser Ser Pro Lys Val Asp
               340                 345                 350
   aag caa cta caa ata aaa gag agc ctg gaa gat ttg cag gag cag ctt      1104
   Lys Gln Leu Gln Ile Lys Glu Ser Leu Glu Asp Leu Gln Glu Gln Leu
           355                 360                 365
   aaa gaa act ggt gat gaa aat cag aaa aga gaa att gaa aag caa att      1152
   Lys Glu Thr Gly Asp Glu Asn Gln Lys Arg Glu Ile Glu Lys Gln Ile
       370                 375                 380
   gaa atc aaa aaa agt gat gaa aag ctt tta aaa agt aaa gat gat aaa      1200
   Glu Ile Lys Lys Ser Asp Glu Lys Leu Leu Lys Ser Lys Asp Asp Lys
   385                 390                 395                 400
   gca agt aaa gat ggt aaa gcc ttg gat ctt gat cga gaa tta aat tct      1248
   Ala Ser Lys Asp Gly Lys Ala Leu Asp Leu Asp Arg Glu Leu Asn Ser
                   405                 410                 415
   aaa gct tct agc aaa gaa aaa agt aaa gcc aag gaa gaa gaa ata acc      1296
   Lys Ala Ser Ser Lys Glu Lys Ser Lys Ala Lys Glu Glu Glu Ile Thr
               420                 425                 430
   aag ggt aag tca cag aaa agc tta ggc gat ttg aat aat gat gaa aat      1344
   Lys Gly Lys Ser Gln Lys Ser Leu Gly Asp Leu Asn Asn Asp Glu Asn
           435                 440                 445
   ctt atg atg cca gaa gat caa aaa tta cct gag gtt aaa aaa tta gat      1392
   Leu Met Met Pro Glu Asp Gln Lys Leu Pro Glu Val Lys Lys Leu Asp
       450                 455                 460
   agc aaa aaa gaa ttt aaa cct gtt tct gag gtt gag aaa tta gat aag      1440
   Ser Lys Lys Glu Phe Lys Pro Val Ser Glu Val Glu Lys Leu Asp Lys
   465                 470                 475                 480
   att ttc aag tct aat aac aat gtt gga gaa tta tca ccg tta gat aaa      1488
   Ile Phe Lys Ser Asn Asn Asn Val Gly Glu Leu Ser Pro Leu Asp Lys
                   485                 490                 495
   tct tct tat aaa gac att gat tca aaa gag gag aca gtt aat aaa gat      1536
   Ser Ser Tyr Lys Asp Ile Asp Ser Lys Glu Glu Thr Val Asn Lys Asp
               500                 505                 510
   gtt aat ttg caa aag act aag cct cag gtt aaa gac caa gtt act tct      1584
   Val Asn Leu Gln Lys Thr Lys Pro Gln Val Lys Asp Gln Val Thr Ser
           515                 520                 525
   ttg aat gaa gat ttg act act atg tct ata gat tcc agt agt cct gta      1632
   Leu Asn Glu Asp Leu Thr Thr Met Ser Ile Asp Ser Ser Ser Pro Val
       530                 535                 540
   ttt tta gag gtt att gat cca att aca aat tta gga act ctt caa ctt      1680
   Phe Leu Glu Val Ile Asp Pro Ile Thr Asn Leu Gly Thr Leu Gln Leu
   545                 550                 555                 560
   att gat tta aat act ggt gtt agg ctt aaa gaa agc act cag caa ggc      1728
   Ile Asp Leu Asn Thr Gly Val Arg Leu Lys Glu Ser Thr Gln Gln Gly
                   565                 570                 575
   att cag cgg tat gga att tat gaa cgt gaa aaa gat ttg gtt gtt att      1776
   Ile Gln Arg Tyr Gly Ile Tyr Glu Arg Glu Lys Asp Leu Val Val Ile
               580                 585                 590
   aaa atg gat tca gga aaa gct aag ctt cag ata ctt gat aaa ctt gaa     1824
   Lys Met Asp Ser Gly Lys Ala Lys Leu Gln Ile Leu Asp Lys Leu Glu
           595                 600                 605
   aat tta aaa gtg gta tca gag tct aat ttt gag att aat aaa aat tca     1872
   Asn Leu Lys Val Val Ser Glu Ser Asn Phe Glu Ile Asn Lys Asn Ser
       610                 615                 620
   tct ctt tat gtt gat tct aaa atg att tta gta gct gtt agg gat aaa     1920
   Ser Leu Tyr Val Asp Ser Lys Met Ile Leu Val Ala Val Arg Asp Lys
   625                 630                 635                 640
   gat agt agt aat gat tgg aga ttg gcc aaa ttt tct cct aaa aat tta     1968
   Asp Ser Ser Asn Asp Trp Arg Leu Ala Lys Phe Ser Pro Lys Asn Leu
                   645                 650                 655
   gat gag ttt att ctt tca gag aat aaa att atg cct ttt act agc ttt     2016
   Asp Glu Phe Ile Leu Ser Glu Asn Lys Ile Met Pro Phe Thr Ser Phe
               660                 665                 670
   tct gtg aga aaa aat ttt att tat ttg caa gat gag ttt aaa agt cta     2064
   Ser Val Arg Lys Asn Phe Ile Tyr Leu Gln Asp Glu Phe Lys Ser Leu
           675                 680                 685
   gtt att tta gat gta aat act tta aaa aaa gtt aag taaagcc             2107
   Val Ile Leu Asp Val Asn Thr Leu Lys Lys Val Lys
       690                 695                 700
<210> 74
   <211> 700
   <212> PRT
   <213> Borrelia burgdorferi
<400> 74
   Met Lys Lys Met Leu Leu Ile Phe Ser Phe Phe Leu Val Phe Leu Asn
   1               5                   10                  15
   Gly Phe Pro Leu Asn Ala Arg Glu Val Asp Lys Glu Lys Leu Lys Asp
               20                  25                  30
   Phe Val Asn Met Asp Leu Glu Phe Val Asn Tyr Lys Gly Pro Tyr Asp
           35                  40                  45
   Ser Thr Asn Thr Tyr Glu Gln Ile Val Gly Ile Gly Glu Phe Leu Ala
       50                  55                  60
   Arg Pro Leu Ile Asn Ser Asn Ser Asn Ser Ser Tyr Tyr Gly Lys Tyr
   65                  70                  75                  80
   Phe Val Asn Arg Phe Ile Asp Asp Gln Asp Lys Lys Ala Ser Val Asp
                   85                  90                  95
   Ile Phe Ser Ile Gly Ser Lys Ser Glu Leu Asp Ser Ile Leu Asn Leu
               100                 105                 110
   Arg Arg Ile Leu Thr Gly Tyr Leu Met Lys Ser Phe Asp Tyr Glu Arg
           115                 120                 125
   Ser Ser Ala Glu Leu Ile Ala Lys Ala Ile Thr Ile Tyr Asn Ala Val
       130                 135                 140
   Tyr Arg Gly Asp Leu Asp Tyr Tyr Lys Glu Phe Tyr Ile Glu Ala Ser
   145                 150                 155                 160
   Leu Lys Ser Leu Thr Lys Glu Asn Ala Gly Leu Ser Arg Val Tyr Ser
                   165                 170                 175
   Gln Trp Ala Gly Lys Thr Gln Ile Phe Ile Pro Leu Lys Lys Asn Ile
               180                 185                 190
   Leu Ser Gly Asn Val Glu Ser Asp Ile Asp Ile Asp Ser Leu Val Thr
           195                 200                 205
   Asp Lys Val Val Ala Ala Leu Leu Ser Glu Asn Glu Ser Gly Val Asn
       210                 215                 220
   Phe Ala Arg Asp Ile Thr Asp Ile Gln Gly Glu Thr His Lys Ala Asp
   225                 230                 235                 240
   Gln Asp Lys Ile Asp Ile Glu Leu Asp Asn Ile His Glu Ser Asp Ser
                   245                 250                 255
   Asn Ile Thr Glu Thr Ile Glu Asn Leu Arg Asp Gln Leu Glu Lys Ala
               260                 265                 270
   Thr Asp Glu Glu His Lys Lys Glu Ile Glu Ser Gln Val Asp Ala Lys
           275                 280                 285
   Lys Lys Gln Lys Glu Glu Leu Asp Lys Lys Ala Ile Asp Leu Asp Lys
       290                 295                 300
   Ala Gln Gln Lys Leu Asp Phe Ala Glu Asp Asn Leu Asp Ile Gln Arg
   305                 310                 315                 320
   Asp Thr Val Arg Glu Lys Leu Gln Glu Asn Ile Asn Glu Thr Asn Lys
                   325                 330                 335
   Glu Lys Asn Leu Pro Lys Pro Gly Asp Val Ser Ser Pro Lys Val Asp
               340                 345                 350
   Lys Gln Leu Gln Ile Lys Glu Ser Leu Glu Asp Leu Gln Glu Gln Leu
           355                 360                 365
   Lys Glu Thr Gly Asp Glu Asn Gln Lys Arg Glu Ile Glu Lys Gln Ile
       370                 375                 380
   Glu Ile Lys Lys Ser Asp Glu Lys Leu Leu Lys Ser Lys Asp Asp Lys
   385                 390                 395                 400
   Ala Ser Lys Asp Gly Lys Ala Leu Asp Leu Asp Arg Glu Leu Asn Ser
                   405                 410                 415
   Lys Ala Ser Ser Lys Glu Lys Ser Lys Ala Lys Glu Glu Glu Ile Thr
               420                 425                 430
   Lys Gly Lys Ser Gln Lys Ser Leu Gly Asp Leu Asn Asn Asp Glu Asn
           435                 440                 445
   Leu Met Met Pro Glu Asp Gln Lys Leu Pro Glu Val Lys Lys Leu Asp
       450                 455                 460
   Ser Lys Lys Glu Phe Lys Pro Val Ser Glu Val Glu Lys Leu Asp Lys
   465                 470                 475                 480
   Ile Phe Lys Ser Asn Asn Asn Val Gly Glu Leu Ser Pro Leu Asp Lys
                   485                 490                 495
   Ser Ser Tyr Lys Asp Ile Asp Ser Lys Glu Glu Thr Val Asn Lys Asp
               500                 505                 510
   Val Asn Leu Gln Lys Thr Lys Pro Gln Val Lys Asp Gln Val Thr Ser
           515                 520                 525
   Leu Asn Glu Asp Leu Thr Thr Met Ser Ile Asp Ser Ser Ser Pro Val
       530                 535                 540
   Phe Leu Glu Val Ile Asp Pro Ile Thr Asn Leu Gly Thr Leu Gln Leu
   545                 550                 555                 560
   Ile Asp Leu Asn Thr Gly Val Arg Leu Lys Glu Ser Thr Gln Gln Gly
                   565                 570                 575
   Ile Gln Arg Tyr Gly Ile Tyr Glu Arg Glu Lys Asp Leu Val Val Ile
               580                 585                 590
   Lys Met Asp Ser Gly Lys Ala Lys Leu Gln Ile Leu Asp Lys Leu Glu
           595                 600                 605
   Asn Leu Lys Val Val Ser Glu Ser Asn Phe Glu Ile Asn Lys Asn Ser
       610                 615                 620
   Ser Leu Tyr Val Asp Ser Lys Met Ile Leu Val Ala Val Arg Asp Lys
   625                 630                 635                 640
   Asp Ser Ser Asn Asp Trp Arg Leu Ala Lys Phe Ser Pro Lys Asn Leu
                   645                 650                 655
   Asp Glu Phe Ile Leu Ser Glu Asn Lys Ile Met Pro Phe Thr Ser Phe
               660                 665                 670
   Ser Val Arg Lys Asn Phe Ile Tyr Leu Gln Asp Glu Phe Lys Ser Leu
           675                 680                 685
   Val Ile Leu Asp Val Asn Thr Leu Lys Lys Val Lys
       690                 695                 700
<210> 75
   <211> 2126
   <212> DNA
   <213> Borrelia burgdorferi
<220>
   <221> CDS
   <222> (1) ... (2124)
<400> 75
   atg aaa aaa atg tta cta atc ttt agt ttt ttt ctt att ttt ttg aat      48
   Met Lys Lys Met Leu Leu Ile Phe Ser Phe Phe Leu Ile Phe Leu Asn
    1               5                  10                  15
   gga ttt cct ctt aat gca agg aaa gtt gat aag gaa aaa tta aag gat      96
   Gly Phe Pro Leu Asn Ala Arg Lys Val Asp Lys Glu Lys Leu Lys Asp
               20                  25                  30
   ttt gtt aat atg gat ctt gag ttt gta aat tat aaa ggt cct tat gat     144
   Phe Val Asn Met Asp Leu Glu Phe Val Asn Tyr Lys Gly Pro Tyr Asp
           35                  40                  45
   tct aca aat acg tat gaa caa ata gtg ggt att ggg gag ttt tta gca     192
   Ser Thr Asn Thr Tyr Glu Gln Ile Val Gly Ile Gly Glu Phe Leu Ala
       50                  55                  60
   aga ccg ctg acc aat tcc aat agc aac tca agt tat tat ggc aaa tat     240
   Arg Pro Leu Thr Asn Ser Asn Ser Asn Ser Ser Tyr Tyr Gly Lys Tyr
   65                  70                  75                  80
   ttt att aat aga ttt att gat gat caa gat aaa aaa gca agt gtt gat     288
   Phe Ile Asn Arg Phe Ile Asp Asp Gln Asp Lys Lys Ala Ser Val Asp
                   85                  90                  95
   gtt ttt tct ata agc agc aaa tca gag ctt gac agt ata ttg aat tta     336
   Val Phe Ser Ile Ser Ser Lys Ser Glu Leu Asp Ser Ile Leu Asn Leu
               100                 105                 110
   aga aga att ctt aca ggg tat ata ata aag tct ttc gat tat gac agg     384
   Arg Arg Ile Leu Thr Gly Tyr Ile Ile Lys Ser Phe Asp Tyr Asp Arg
           115                 120                 125
   tct agt gca gaa tta att gct aag gtt att aca ata tat aat gct gtt     432
   Ser Ser Ala Glu Leu Ile Ala Lys Val Ile Thr Ile Tyr Asn Ala Val
       130                 135                 140
   tat aga gga gat ttg gat tat tat aaa ggg ttt tat att gag cct gct     480
   Tyr Arg Gly Asp Leu Asp Tyr Tyr Lys Gly Phe Tyr Ile Glu Pro Ala
   145                 150                 155                 160
   ttg aag tct tta act aaa gaa aac gca ggt ctt tct agg gtt tac agt     528
   Leu Lys Ser Leu Thr Lys Glu Asn Ala Gly Leu Ser Arg Val Tyr Ser
                   165                 170                 175
   cag tgg gct gga aag act caa ata ttt att cct ctt aaa aag gat att     576
   Gln Trp Ala Gly Lys Thr Gln Ile Phe Ile Pro Leu Lys Lys Asp Ile
               180                 185                 190
   ttg tct gga aat att gaa tct gac att gat att gac agt ttg gtt aca     624
   Leu Ser Gly Asn Ile Glu Ser Asp Ile Asp Ile Asp Ser Leu Val Thr
           195                 200                 205
   gat aag gtg ata gca gct ctt tta agc gaa aat gaa gca ggc gtt aac     672
   Asp Lys Val Ile Ala Ala Leu Leu Ser Glu Asn Glu Ala Gly Val Asn
       210                 215                 220
   ttt gca aga gat att aca gat att caa ggc gaa act cat aag gca gat     720
   Phe Ala Arg Asp Ile Thr Asp Ile Gln Gly Glu Thr His Lys Ala Asp
   225                 230                 235                 240
   caa gat aag att gat act gaa tta gac aat atc cat gaa agc gat tct     768
   Gln Asp Lys Ile Asp Thr Glu Leu Asp Asn Ile His Glu Ser Asp Ser
                   245                 250                 255
   aat ata aca gaa act att gaa aat tta agg gat cag ctt gaa aaa gct     816
   Asn Ile Thr Glu Thr Ile Glu Asn Leu Arg Asp Gln Leu Glu Lys Ala
               260                 265                 270
   aca gat gaa gag cat aaa aaa gag att gaa agt cag gtt gat gct aaa     864
   Thr Asp Glu Glu His Lys Lys Glu Ile Glu Ser Gln Val Asp Ala Lys
           275                 280                 285
   aag aaa gaa aag gaa gag cta gat aaa aag gca atc aat ctt gat aaa     912
   Lys Lys Glu Lys Glu Glu Leu Asp Lys Lys Ala Ile Asn Leu Asp Lys
       290                 295                 300
   gct cag caa aaa tta gac tct gct gaa gat aat tta gat gtt caa aga     960
   Ala Gln Gln Lys Leu Asp Ser Ala Glu Asp Asn Leu Asp Val Gln Arg
   305                 310                 315                 320
   gat act gtt aga gag aaa att caa gag gat att aat gag att aat aag    1008
   Asp Thr Val Arg Glu Lys Ile Gln Glu Asp Ile Asn Glu Ile Asn Lys
                   325                 330                 335
   gaa aag aat ttg cca aaa cct ggt gat gta agt tct cct aaa gtt gat    1056
   Glu Lys Asn Leu Pro Lys Pro Gly Asp Val Ser Ser Pro Lys Val Asp
               340                 345                 350
   aag caa ctg caa ata aaa gag agt cta gaa gat ttg cag gag cag ctt    1104
   Lys Gln Leu Gln Ile Lys Glu Ser Leu Glu Asp Leu Gln Glu Gln Leu
           355                 360                 365
   aaa gaa gct ggt gat gaa aat cag aaa aga gaa att gag aag caa att    1152
   Lys Glu Ala Gly Asp Glu Asn Gln Lys Arg Glu Ile Glu Lys Gln Ile
       370                 375                 380
   gaa atc aaa aaa agg gac gaa gaa ctt tta aaa agt aaa gat ggc aaa    1200
   Glu Ile Lys Lys Arg Asp Glu Glu Leu Leu Lys Ser Lys Asp Gly Lys
   385                 390                 395                 400
   gta agt aaa gat tat gaa gca tta gat ctt gat cga gaa tta tcc aaa    1248
   Val Ser Lys Asp Tyr Glu Ala Leu Asp Leu Asp Arg Glu Leu Ser Lys
                   405                 410                 415
   gct tct agt aaa gaa aaa agt aag gtc aag gaa gaa gaa ata act aaa    1296
   Ala Ser Ser Lys Glu Lys Ser Lys Val Lys Glu Glu Glu Ile Thr Lys
               420                 425                 430
   ggt aaa tca cgg gca agc tta ggc gat ttg aat aat gat aaa aac ctt    1344
   Gly Lys Ser Arg Ala Ser Leu Gly Asp Leu Asn Asn Asp Lys Asn Leu
           435                 440                 445
   atg ttg cca gaa gat caa aaa tta cct gaa gat aaa aaa ttg gat agt    1392
   Met Leu Pro Glu Asp Gln Lys Leu Pro Glu Asp Lys Lys Leu Asp Ser
       450                 455                 460
   aaa tta gat ggt aaa aaa gaa ttt aaa cca gtt tct gag gtt gaa aaa    1440
   Lys Leu Asp Gly Lys Lys Glu Phe Lys Pro Val Ser Glu Val Glu Lys
   465                 470                 475                 480
   tta gat aag att tcc aag tct aat aac aat gag gtt ggc aag tta tca    1488
   Leu Asp Lys Ile Ser Lys Ser Asn Asn Asn Glu Val Gly Lys Leu Ser
                   485                 490                 495
   cca tta gat aag cct tct tat gat gat att gat tca aaa gag gag gta    1536
   Pro Leu Asp Lys Pro Ser Tyr Asp Asp Ile Asp Ser Lys Glu Glu Val
               500                 505                 510
   gat aat aaa gct att aat ttg caa aag atc gac cct aaa gtt aaa gac    1584
   Asp Asn Lys Ala Ile Asn Leu Gln Lys Ile Asp Pro Lys Val Lys Asp
           515                 520                 525
   caa act act tct ttg aat gaa gat ttg gat aaa gat ttg act act atg    1632
   Gln Thr Thr Ser Leu Asn Glu Asp Leu Asp Lys Asp Leu Thr Thr Met
       530                 535                 540
   tct ata gat tcc agc agt cct gta ttt cta gag gtt att gat cct att    1680
   Ser Ile Asp Ser Ser Ser Pro Val Phe Leu Glu Val Ile Asp Pro Ile
   545                 550                 555                 560
   aca aat tta gga acc ctg cag ctt att gat tta aat act ggg gtt agg    1728
   Thr Asn Leu Gly Thr Leu Gln Leu Ile Asp Leu Asn Thr Gly Val Arg
                   565                 570                 575
   ctt aag gaa agc act cag caa ggc att cag cgg tat gga att tat gaa    1776
   Leu Lys Glu Ser Thr Gln Gln Gly Ile Gln Arg Tyr Gly Ile Tyr Glu
               580                 585                 590
   cgt gaa aaa gat ttg gtt gtt att aaa atg gat tca gga aag gct aag    1824
   Arg Glu Lys Asp Leu Val Val Ile Lys Met Asp Ser Gly Lys Ala Lys
           595                 600                 605
   ctt caa ata ctt aat aag ctt gaa aat ttg aaa gtg gta tca gag tct    1872
   Leu Gln Ile Leu Asn Lys Leu Glu Asn Leu Lys Val Val Ser Glu Ser
       610                 615                 620
   aat ttt gag atc aat aaa aat tca tct ctt tat gtt gac tct aaa atg    1920
   Asn Phe Glu Ile Asn Lys Asn Ser Ser Leu Tyr Val Asp Ser Lys Met
   625                 630                 635                 640
   att ttg gca gct gtt aga gat aag gat gat agc aat gct tgg aga ttg    1968
   Ile Leu Ala Ala Val Arg Asp Lys Asp Asp Ser Asn Ala Trp Arg Leu
                   645                 650                 655
   gct aaa ttt tct cct aaa aat ttg gat gag ttt att ctt tca gag aat    2016
   Ala Lys Phe Ser Pro Lys Asn Leu Asp Glu Phe Ile Leu Ser Glu Asn
               660                 665                 670
   aaa att ttg cct ttt act agc ttt tct gtg aga aaa aat ttt att tat    2064
   Lys Ile Leu Pro Phe Thr Ser Phe Ser Val Arg Lys Asn Phe Ile Tyr
           675                 680                 685
   ttg caa gat gag ctt aaa aat cta gtt att tta gat gta aat act tta      2112
   Leu Gln Asp Glu Leu Lys Asn Leu Val Ile Leu Asp Val Asn Thr Leu
       690                 695                 700
   aaa aaa gtt aag ta                                                   2126
   Lys Lys Val Lys
   705
<210> 76
   <211> 708
   <212> PRT
   <213> Borrelia burgdorferi
<400> 76
   Met Lys Lys Met Leu Leu Ile Phe Ser Phe Phe Leu Ile Phe Leu Asn
   1               5                   10                  15
   Gly Phe Pro Leu Asn Ala Arg Lys Val Asp Lys Glu Lys Leu Lys Asp
               20                  25                  30
   Phe Val Asn Met Asp Leu Glu Phe Val Asn Tyr Lys Gly Pro Tyr Asp
           35                  40                  45
   Ser Thr Asn Thr Tyr Glu Gln Ile Val Gly Ile Gly Glu Phe Leu Ala
       50                  55                  60
   Arg Pro Leu Thr Asn Ser Asn Ser Asn Ser Ser Tyr Tyr Gly Lys Tyr
   65                  70                  75                  80
   Phe Ile Asn Arg Phe Ile Asp Asp Gln Asp Lys Lys Ala Ser Val Asp
                   85                  90                  95
   Val Phe Ser Ile Ser Ser Lys Ser Glu Leu Asp Ser Ile Leu Asn Leu
               100                 105                 110
   Arg Arg Ile Leu Thr Gly Tyr Ile Ile Lys Ser Phe Asp Tyr Asp Arg
           115                 120                 125
   Ser Ser Ala Glu Leu Ile Ala Lys Val Ile Thr Ile Tyr Asn Ala Val
       130                 135                 140
   Tyr Arg Gly Asp Leu Asp Tyr Tyr Lys Gly Phe Tyr Ile Glu Pro Ala
   145                 150                 155                 160
   Leu Lys Ser Leu Thr Lys Glu Asn Ala Gly Leu Ser Arg Val Tyr Ser
                   165                 170                 175
   Gln Trp Ala Gly Lys Thr Gln Ile Phe Ile Pro Leu Lys Lys Asp Ile
               180                 185                 190
   Leu Ser Gly Asn Ile Glu Ser Asp Ile Asp Ile Asp Ser Leu Val Thr
           195                 200                 205
   Asp Lys Val Ile Ala Ala Leu Leu Ser Glu Asn Glu Ala Gly Val Asn
       210                 215                 220
   Phe Ala Arg Asp Ile Thr Asp Ile Gln Gly Glu Thr His Lys Ala Asp
   225                 230                 235                 240
   Gln Asp Lys Ile Asp Thr Glu Leu Asp Asn Ile His Glu Ser Asp Ser
                   245                 250                 255
   Asn Ile Thr Glu Thr Ile Glu Asn Leu Arg Asp Gln Leu Glu Lys Ala
               260                 265                 270
   Thr Asp Glu Glu His Lys Lys Glu Ile Glu Ser Gln Val Asp Ala Lys
           275                 280                 285
   Lys Lys Glu Lys Glu Glu Leu Asp Lys Lys Ala Ile Asn Leu Asp Lys
       290                 295                 300
   Ala Gln Gln Lys Leu Asp Ser Ala Glu Asp Asn Leu Asp Val Gln Arg
   305                 310                 315                 320
   Asp Thr Val Arg Glu Lys Ile Gln Glu Asp Ile Asn Glu Ile Asn Lys
                   325                 330                 335
   Glu Lys Asn Leu Pro Lys Pro Gly Asp Val Ser Ser Pro Lys Val Asp
               340                 345                 350
   Lys Gln Leu Gln Ile Lys Glu Ser Leu Glu Asp Leu Gln Glu Gln Leu
           355                 360                 365
   Lys Glu Ala Gly Asp Glu Asn Gln Lys Arg Glu Ile Glu Lys Gln Ile
       370                 375                 380
   Glu Ile Lys Lys Arg Asp Glu Glu Leu Leu Lys Ser Lys Asp Gly Lys
   385                 390                 395                 400
   Val Ser Lys Asp Tyr Glu Ala Leu Asp Leu Asp Arg Glu Leu Ser Lys
                   405                 410                 415
   Ala Ser Ser Lys Glu Lys Ser Lys Val Lys Glu Glu Glu Ile Thr Lys
               420                 425                 430
   Gly Lys Ser Arg Ala Ser Leu Gly Asp Leu Asn Asn Asp Lys Asn Leu
           435                 440                 445
   Met Leu Pro Glu Asp Gln Lys Leu Pro Glu Asp Lys Lys Leu Asp Ser
       450                 455                 460
   Lys Leu Asp Gly Lys Lys Glu Phe Lys Pro Val Ser Glu Val Glu Lys
   465                 470                 475                 480
   Leu Asp Lys Ile Ser Lys Ser Asn Asn Asn Glu Val Gly Lys Leu Ser
                   485                 490                 495
   Pro Leu Asp Lys Pro Ser Tyr Asp Asp Ile Asp Ser Lys Glu Glu Val
               500                 505                 510
   Asp Asn Lys Ala Ile Asn Leu Gln Lys Ile Asp Pro Lys Val Lys Asp
           515                 520                 525
   Gln Thr Thr Ser Leu Asn Glu Asp Leu Asp Lys Asp Leu Thr Thr Met
       530                 535                 540
   Ser Ile Asp Ser Ser Ser Pro Val Phe Leu Glu Val Ile Asp Pro Ile
   545                 550                 555                 560
   Thr Asn Leu Gly Thr Leu Gln Leu Ile Asp Leu Asn Thr Gly Val Arg
                   565                 570                 575
   Leu Lys Glu Ser Thr Gln Gln Gly Ile Gln Arg Tyr Gly Ile Tyr Glu
               580                 585                 590
   Arg Glu Lys Asp Leu Val Val Ile Lys Met Asp Ser Gly Lys Ala Lys
           595                 600                 605
   Leu Gln Ile Leu Asn Lys Leu Glu Asn Leu Lys Val Val Ser Glu Ser
       610                 615                 620
   Asn Phe Glu Ile Asn Lys Asn Ser Ser Leu Tyr Val Asp Ser Lys Met
   625                 630                 635                 640
   Ile Leu Ala Ala Val Arg Asp Lys Asp Asp Ser Asn Ala Trp Arg Leu
                   645                 650                 655
   Ala Lys Phe Ser Pro Lys Asn Leu Asp Glu Phe Ile Leu Ser Glu Asn
               660                 665                 670
   Lys Ile Leu Pro Phe Thr Ser Phe Ser Val Arg Lys Asn Phe Ile Tyr
           675                 680                 685
   Leu Gln Asp Glu Leu Lys Asn Leu Val Ile Leu Asp Val Asn Thr Leu
       690                 695                 700
   Lys Lys Val Lys
   705
<210> 77
   <211> 1991
   <212> DNA
   <213> Borrelia burgdorferi
<220>
   <221> CDS
   <222> (1)... (1989)
<400> 77
   atg aaa aaa atg tta cta atc ttt agt ttt ttt ctt gtt ttt tta aat    48
   Met Lys Lys Met Leu Leu Ile Phe Ser Phe Phe Leu Val Phe Leu Asn
    1               5                  10                  15
   gga ttt cct ctt aat gca agg gaa gtt gat aag gaa aaa tta aag gac      96
   Gly Phe Pro Leu Asn Ala Arg Glu Val Asp Lys Glu Lys Leu Lys Asp
               20                  25                  30
   ttt gtt aat atg gat ctt gaa ttt gtt aat tac aag ggt cct tat gat     144
   Phe Val Asn Met Asp Leu Glu Phe Val Asn Tyr Lys Gly Pro Tyr Asp
           35                  40                  45
   tct aca aat aca tat gaa caa ata gta ggt att ggg gag ttt tta gca     192
   Ser Thr Asn Thr Tyr Glu Gln Ile Val Gly Ile Gly Glu Phe Leu Ala
       50                  55                  60
   agg ccg ttg atc aat tcc aat agt aat tca agt tat tat ggt aaa tat     240
   Arg Pro Leu Ile Asn Ser Asn Ser Asn Ser Ser Tyr Tyr Gly Lys Tyr
   65                  70                  75                  80
   ttt gtt aat aga ttt att gac gat caa gat aaa aaa gca agt gtt gat     288
   Phe Val Asn Arg Phe Ile Asp Asp Gln Asp Lys Lys Ala Ser Val Asp
                   85                  90                  95
   att ttt tct att ggt agt aag tca gag ctt gat agt ata tta aat cta     336
   Ile Phe Ser Ile Gly Ser Lys Ser Glu Leu Asp Ser Ile Leu Asn Leu
               100                 105                 110
   aga aga att ctt aca ggg tat tta atg aag tct ttt gat tat gag agg     384
   Arg Arg Ile Leu Thr Gly Tyr Leu Met Lys Ser Phe Asp Tyr Glu Arg
           115                 120                 125
   tct agt gcg gaa tta att gct aaa gct att aca ata tat aat gct gtt     432
   Ser Ser Ala Glu Leu Ile Ala Lys Ala Ile Thr Ile Tyr Asn Ala Val
       130                 135                 140
   tat aga gga gat tta gat tat tac aaa gag ttt tat att gag gct tct     480
   Tyr Arg Gly Asp Leu Asp Tyr Tyr Lys Glu Phe Tyr Ile Glu Ala Ser
   145                 150                 155                 160
   ttg aag tct ttg act aaa gaa aat gca ggt ctt tct agg gtg tac agt     528
   Leu Lys Ser Leu Thr Lys Glu Asn Ala Gly Leu Ser Arg Val Tyr Ser
                   165                 170                 175
   caa tgg gct ggg aag aca caa ata ttt att cct ctt aaa aag aat att     576
   Gln Trp Ala Gly Lys Thr Gln Ile Phe Ile Pro Leu Lys Lys Asn Ile
               180                 185                 190
   tta tct gga aat gtt gag tct gac att gat att gat agt ttg gtt aca     624
   Leu Ser Gly Asn Val Glu Ser Asp Ile Asp Ile Asp Ser Leu Val Thr
           195                 200                 205
   gat aag gtg gtg gca gct ctt tta agt gag aat gaa tca ggt gtt aac     672
   Asp Lys Val Val Ala Ala Leu Leu Ser Glu Asn Glu Ser Gly Val Asn
       210                 215                 220
   ttt gca aga gat att aca gac att caa ggc gaa act cat aaa gca gat     720
   Phe Ala Arg Asp Ile Thr Asp Ile Gln Gly Glu Thr His Lys Ala Asp
   225                 230                 235                 240
   caa gat aaa att gat att gaa tta gat aat ttt cat gaa agt gat tcc     768
   Gln Asp Lys Ile Asp Ile Glu Leu Asp Asn Phe His Glu Ser Asp Ser
                   245                 250                 255
   aat ata aca gaa act att gag aat tta agg gat cag ctt gaa aaa gct      816
   Asn Ile Thr Glu Thr Ile Glu Asn Leu Arg Asp Gln Leu Glu Lys Ala
               260                 265                 270
   aca gat gaa gag cat aaa aaa gag att gaa agt cag gtt gat gct aaa      864
   Thr Asp Glu Glu His Lys Lys Glu Ile Glu Ser Gln Val Asp Ala Lys
           275                 280                 285
   aag aaa caa aag gaa gaa tta gat aaa aag gca att gat ctt gat aaa      912
   Lys Lys Gln Lys Glu Glu Leu Asp Lys Lys Ala Ile Asp Leu Asp Lys
       290                 295                 300
   gct caa caa aaa tta gat ttt gct gaa gat aat cta gat att caa agg      960
   Ala Gln Gln Lys Leu Asp Phe Ala Glu Asp Asn Leu Asp Ile Gln Arg
   305                 310                 315                 320
   gat act gtt aga gag aag ctt caa gaa aat att aac gag act aat aag     1008
   Asp Thr Val Arg Glu Lys Leu Gln Glu Asn Ile Asn Glu Thr Asn Lys
                   325                 330                 335
   gaa aag aat tta cca aag cct ggt gat gta agt tct cct aag gtt gat     1056
   Glu Lys Asn Leu Pro Lys Pro Gly Asp Val Ser Ser Pro Lys Val Asp
               340                 345                 350
   aag cag ttg cag ata aaa gag agt cta gaa gat ttg caa gag cag ctt     1104
   Lys Gln Leu Gln Ile Lys Glu Ser Leu Glu Asp Leu Gln Glu Gln Leu
           355                 360                 365
   aaa gaa gct agt gat gaa aat caa aaa aga gaa ata gaa aag caa att     1152
   Lys Glu Ala Ser Asp Glu Asn Gln Lys Arg Glu Ile Glu Lys Gln Ile
       370                 375                 380
   gaa atc aaa aaa aat gat gaa gaa ctt ttt aaa aat aaa gat cat aaa     1200
   Glu Ile Lys Lys Asn Asp Glu Glu Leu Phe Lys Asn Lys Asp His Lys
   385                 390                 395                 400
   gca tta gat ctt aag caa gaa tta aat tct aaa gct tct agt aaa gaa     1248
   Ala Leu Asp Leu Lys Gln Glu Leu Asn Ser Lys Ala Ser Ser Lys Glu
                   405                 410                 415
   aaa att gaa ggc gaa gaa gag gat aaa gaa tta gat agt aaa aaa aat     1296
   Lys Ile Glu Gly Glu Glu Glu Asp Lys Glu Leu Asp Ser Lys Lys Asn
               420                 425                 430
   tta gag cct gtt tct gag gct gat aaa gta gat aaa att tcc aag tct     1344
   Leu Glu Pro Val Ser Glu Ala Asp Lys Val Asp Lys Ile Ser Lys Ser
           435                 440                 445
   aac aac aat gag gtt agt aaa tta tcc ccg tta gat gag cct tct tat     1392
   Asn Asn Asn Glu Val Ser Lys Leu Ser Pro Leu Asp Glu Pro Ser Tyr
       450                 455                 460
   agc gac att gat tcg aaa gag ggt gta gat aac aaa gat gtt gat ttg     1440
   Ser Asp Ile Asp Ser Lys Glu Gly Val Asp Asn Lys Asp Val Asp Leu
   465                 470                 475                 480
   caa aaa act aaa ccc caa gtt gaa agt caa cct act tcg tta aat gaa     1488
   Gln Lys Thr Lys Pro Gln Val Glu Ser Gln Pro Thr Ser Leu Asn Glu
                   485                 490                 495
   gac ttg att gat gtg tct ata gat tcc agt aat cct gtc ttt tta gag    1536
   Asp Leu Ile Asp Val Ser Ile Asp Ser Ser Asn Pro Val Phe Leu Glu
               500                 505                 510
   gtt atc gat ccg att aca aat tta gga acg ctt caa ctt att gat ttg    1584
   Val Ile Asp Pro Ile Thr Asn Leu Gly Thr Leu Gln Leu Ile Asp Leu
           515                 520                 525
   aat acc ggt gtt aga ctt aaa gaa agt gct caa caa ggt att cag cga    1632
   Asn Thr Gly Val Arg Leu Lys Glu Ser Ala Gln Gln Gly Ile Gln Arg
       530                 535                 540
   tat gga att tat gaa cgt gaa aaa gat ttg gtt gtt att aaa ata gat    1680
   Tyr Gly Ile Tyr Glu Arg Glu Lys Asp Leu Val Val Ile Lys Ile Asp
   545                 550                 555                 560
   tca gga aaa gct aag ctt cag ata ctt gat aaa ctc gag aat tta aaa    1728
   Ser Gly Lys Ala Lys Leu Gln Ile Leu Asp Lys Leu Glu Asn Leu Lys
                   565                 570                 575
   gtg ata tca gag tct aat ttt gag att aat aaa aat tca tct ctt tat    1776
   Val Ile Ser Glu Ser Asn Phe Glu Ile Asn Lys Asn Ser Ser Leu Tyr
               580                 585                 590
   gtt gac tct aga atg att tta gta gtt gtt aag gac gat agt aat gct    1824
   Val Asp Ser Arg Met Ile Leu Val Val Val Lys Asp Asp Ser Asn Ala
           595                 600                 605
   tgg aga ttg gct aaa ttt tct cct aaa aat tta gat gaa ttt att ctg    1872
   Trp Arg Leu Ala Lys Phe Ser Pro Lys Asn Leu Asp Glu Phe Ile Leu
       610                 615                 620
   tca gaa aat aaa att ttg cct ttt act agc ttt gct gtg aga aag aat    1920
   Ser Glu Asn Lys Ile Leu Pro Phe Thr Ser Phe Ala Val Arg Lys Asn
   625                 630                 635                 640
   ttt att tat ttg caa gat gaa ctt aaa agc tta gtt act tta gat gta    1968
   Phe Ile Tyr Leu Gln Asp Glu Leu Lys Ser Leu Val Thr Leu Asp Val
                   645                 650                 655
   aat act tta aaa aaa gtt aag ta                                     1991
   Asn Thr Leu Lys Lys Val Lys
               660
<210> 78
   <211> 663
   <212> PRT
   <213> Borrelia burgdorferi
<400> 78
   Met Lys Lys Met Leu Leu Ile Phe Ser Phe Phe Leu Val Phe Leu Asn
   1               5                   10                  15
   Gly Phe Pro Leu Asn Ala Arg Glu Val Asp Lys Glu Lys Leu Lys Asp
               20                  25                  30
   Phe Val Asn Met Asp Leu Glu Phe Val Asn Tyr Lys Gly Pro Tyr Asp
           35                  40                  45
   Ser Thr Asn Thr Tyr Glu Gln Ile Val Gly Ile Gly Glu Phe Leu Ala
       50                  55                  60
   Arg Pro Leu Ile Asn Ser Asn Ser Asn Ser Ser Tyr Tyr Gly Lys Tyr
   65                  70                  75                  80
   Phe Val Asn Arg Phe Ile Asp Asp Gln Asp Lys Lys Ala Ser Val Asp
                   85                  90                  95
   Ile Phe Ser Ile Gly Ser Lys Ser Glu Leu Asp Ser Ile Leu Asn Leu
               100                 105                 110
   Arg Arg Ile Leu Thr Gly Tyr Leu Met Lys Ser Phe Asp Tyr Glu Arg
           115                 120                 125
   Ser Ser Ala Glu Leu Ile Ala Lys Ala Ile Thr Ile Tyr Asn Ala Val
       130                 135                 140
   Tyr Arg Gly Asp Leu Asp Tyr Tyr Lys Glu Phe Tyr Ile Glu Ala Ser
   145                 150                 155                 160
   Leu Lys Ser Leu Thr Lys Glu Asn Ala Gly Leu Ser Arg Val Tyr Ser
                   165                 170                 175
   Gln Trp Ala Gly Lys Thr Gln Ile Phe Ile Pro Leu Lys Lys Asn Ile
               180                 185                 190
   Leu Ser Gly Asn Val Glu Ser Asp Ile Asp Ile Asp Ser Leu Val Thr
           195                 200                 205
   Asp Lys Val Val Ala Ala Leu Leu Ser Glu Asn Glu Ser Gly Val Asn
       210                 215                 220
   Phe Ala Arg Asp Ile Thr Asp Ile Gln Gly Glu Thr His Lys Ala Asp
   225                 230                 235                 240
   Gln Asp Lys Ile Asp Ile Glu Leu Asp Asn Phe His Glu Ser Asp Ser
                   245                 250                 255
   Asn Ile Thr Glu Thr Ile Glu Asn Leu Arg Asp Gln Leu Glu Lys Ala
               260                 265                 270
   Thr Asp Glu Glu His Lys Lys Glu Ile Glu Ser Gln Val Asp Ala Lys
           275                 280                 285
   Lys Lys Gln Lys Glu Glu Leu Asp Lys Lys Ala Ile Asp Leu Asp Lys
       290                 295                 300
   Ala Gln Gln Lys Leu Asp Phe Ala Glu Asp Asn Leu Asp Ile Gln Arg
   305                 310                 315                 320
   Asp Thr Val Arg Glu Lys Leu Gln Glu Asn Ile Asn Glu Thr Asn Lys
                   325                 330                 335
   Glu Lys Asn Leu Pro Lys Pro Gly Asp Val Ser Ser Pro Lys Val Asp
               340                 345                 350
   Lys Gln Leu Gln Ile Lys Glu Ser Leu Glu Asp Leu Gln Glu Gln Leu
           355                 360                 365
   Lys Glu Ala Ser Asp Glu Asn Gln Lys Arg Glu Ile Glu Lys Gln Ile
       370                 375                 380
   Glu Ile Lys Lys Asn Asp Glu Glu Leu Phe Lys Asn Lys Asp His Lys
   385                 390                 395                 400
   Ala Leu Asp Leu Lys Gln Glu Leu Asn Ser Lys Ala Ser Ser Lys Glu
                   405                 410                 415
   Lys Ile Glu Gly Glu Glu Glu Asp Lys Glu Leu Asp Ser Lys Lys Asn
               420                 425                 430
   Leu Glu Pro Val Ser Glu Ala Asp Lys Val Asp Lys Ile Ser Lys Ser
           435                 440                 445
   Asn Asn Asn Glu Val Ser Lys Leu Ser Pro Leu Asp Glu Pro Ser Tyr
       450                 455                 460
   Ser Asp Ile Asp Ser Lys Glu Gly Val Asp Asn Lys Asp Val Asp Leu
   465                 470                 475                 480
   Gln Lys Thr Lys Pro Gln Val Glu Ser Gln Pro Thr Ser Leu Asn Glu
                   485                 490                 495
   Asp Leu Ile Asp Val Ser Ile Asp Ser Ser Asn Pro Val Phe Leu Glu
               500                 505                 510
   Val Ile Asp Pro Ile Thr Asn Leu Gly Thr Leu Gln Leu Ile Asp Leu
           515                 520                 525
   Asn Thr Gly Val Arg Leu Lys Glu Ser Ala Gln Gln Gly Ile Gln Arg
       530                 535                 540
   Tyr Gly Ile Tyr Glu Arg Glu Lys Asp Leu Val Val Ile Lys Ile Asp
   545                 550                 555                 560
   Ser Gly Lys Ala Lys Leu Gln Ile Leu Asp Lys Leu Glu Asn Leu Lys
                   565                 570                 575
   Val Ile Ser Glu Ser Asn Phe Glu Ile Asn Lys Asn Ser Ser Leu Tyr
               580                 585                 590
   Val Asp Ser Arg Met Ile Leu Val Val Val Lys Asp Asp Ser Asn Ala
           595                 600                 605
   Trp Arg Leu Ala Lys Phe Ser Pro Lys Asn Leu Asp Glu Phe Ile Leu
       610                 615                 620
   Ser Glu Asn Lys Ile Leu Pro Phe Thr Ser Phe Ala Val Arg Lys Asn
   625                 630                 635                     640
   Phe Ile Tyr Leu Gln Asp Glu Leu Lys Ser Leu Val Thr Leu Asp Val
                   645                 650                 655
   Asn Thr Leu Lys Lys Val Lys
               660
<210> 79
   <211> 32
   <212> DNA
   <213> Artificial Sequence
<220>
   <223> Oligonucleotide Primer
<400> 79
   ccggtcaccc catggctgct ttaaagtctt ta 32
<210> 80
   <211> 32
   <212> DNA
   <213> Artificial Sequence
<220>
   <223> Oligonucleotide Primer
<400> 80
   ccggtcaccc catgaatctt gataaagctc ag 32
<210> 81
   <211> 35
   <212> DNA
   <213> Artificial Sequence
<220>
   <223> Oligonucleotide Primer
<400> 81
   ccggtcaccc catggatgaa aagcttttaa aaagt 35
<210> 82
   <211> 33
   <212> DNA
   <213> Artificial Sequence
<220>
   <223> Oligonucleotide Primer
<400> 82
   ccggtcaccc ccatggttga gaaattagat aag 33
<210> 83
   <211> 32
   <212> DNA
   <213> Artificial Sequence
<220>
   <223> Oligonucleotide Primer
<400> 83
   ttggatccgg tgacccttaa ctttttttaa ag 32
<210> 84
   <211> 38
   <212> DNA
   <213> Artificial Sequence
<220>
   <223> Oligonucleotide Primer
<400> 84
   aaagtagaag tttttgaatc ccattttcca gttttttt 38
<210> 85
   <211> 825
   <212> DNA
   <213> Artificial Sequence
<220>
   <221> CDS
   <222> (1)...(825)
   <223> Chimeric nucleic acid
<400> 85
   atg aaa aaa tat tta ttg gga ata ggt cta ata tta gcc tta ata gca      48
   Met Lys Lys Tyr Leu Leu Gly Ile Gly Leu Ile Leu Ala Leu Ile Ala
    1               5                  10                  15
   tgt aag caa aat gtt agc agc ctt gat gaa aaa aat agc gtt tca gta      96
   Cys Lys Gln Asn Val Ser Ser Leu Asp Glu Lys Asn Ser Val Ser Val
               20                  25                  30
   gat tta cct ggt gga atg aca gtt ctt gta agt aaa gaa aaa gac aaa     144
   Asp Leu Pro Gly Gly Met Thr Val Leu Val Ser Lys Glu Lys Asp Lys
           35                  40                  45
   gac ggt aaa tac agt cta gag gca aca gta gac aag ctt gag ctt aaa     192
   Asp Gly Lys Tyr Ser Leu Glu Ala Thr Val Asp Lys Leu Glu Leu Lys
       50                  55                  60
   gga act tct gat aaa aac aac ggt tct gga aca ctt gaa ggt gaa aaa     240
   Gly Thr Ser Asp Lys Asn Asn Gly Ser Gly Thr Leu Glu Gly Glu Lys
   65                  70                  75                      80
   act gac aaa agt aaa gta aaa tta aca att gct gat gac cta agt caa     288
   Thr Asp Lys Ser Lys Val Lys Leu Thr Ile Ala Asp Asp Leu Ser Gln
                   85                  90                  95
   act aaa ttt gaa att ttc aaa gaa gat gcc aaa aca tta gta tca aaa     336
   Thr Lys Phe Glu Ile Phe Lys Glu Asp Ala Lys Thr Leu Val Ser Lys
               100                 105                 110
   aaa gta acc ctt aaa gac aag tca tca aca gaa gaa aaa ttc aac gaa      384
   Lys Val Thr Leu Lys Asp Lys Ser Ser Thr Glu Glu Lys Phe Asn Glu
           115                 120                 125
   aag ggt gaa aca tct gaa aaa aca ata gta aga gca aat gga acc aga      432
   Lys Gly Glu Thr Ser Glu Lys Thr Ile Val Arg Ala Asn Gly Thr Arg
       130                 135                 140
   ctt gaa tac aca gac ata aaa agc gat gga tcc gga aaa gct aaa gaa      480
   Leu Glu Tyr Thr Asp Ile Lys Ser Asp Gly Ser Gly Lys Ala Lys Glu
   145                 150                 155                 160
   gtt tta aaa gac ttt act ctt gaa gga act cta gct gct gac ggc aaa      528
   Val Leu Lys Asp Phe Thr Leu Glu Gly Thr Leu Ala Ala Asp Gly Lys
                   165                 170                 175
   aca aca ttg aaa gtt aca gaa ggc act gtt gtt tta agc aag aac att      576
   Thr Thr Leu Lys Val Thr Glu Gly Thr Val Val Leu Ser Lys Asn Ile
               180                 185                 190
   tta aaa tcc gga gaa ata aca gtt gca ctt gat gac tct gac act act      624
   Leu Lys Ser Gly Glu Ile Thr Val Ala Leu Asp Asp Ser Asp Thr Thr
           195                 200                 205
   cag gct act aaa aaa act gga aaa tgg gat tca aaa act tct act tta      672
   Gln Ala Thr Lys Lys Thr Gly Lys Trp Asp Ser Lys Thr Ser Thr Leu
       210                 215                 220
   aca att agt gtt aac agc aaa aaa act aca caa ctt gtg ttt act aaa      720
   Thr Ile Ser Val Asn Ser Lys Lys Thr Thr Gln Leu Val Phe Thr Lys
   225                 230                 235                 240
   caa tac aca ata act gta aaa caa tac gac tcc gca ggt acc aat tta      768
   Gln Tyr Thr Ile Thr Val Lys Gln Tyr Asp Ser Ala Gly Thr Asn Leu
                   245                 250                 255
   gaa ggc aca gca gtc gaa att aaa aca ctt gat gaa ctt aaa aac gct      816
   Glu Gly Thr Ala Val Glu Ile Lys Thr Leu Asp Glu Leu Lys Asn Ala
               260                 265                 270
   tta aaa taa                                                           825
   Leu Lys  *
<210> 86
   <211> 274
   <212> PRT
   <213> Artificial Sequence
<220>
   <223> Chimeric Protein
<400> 86
   Met Lys Lys Tyr Leu Leu Gly Ile Gly Leu Ile Leu Ala Leu Ile Ala
    1               5                  10                  15
   Cys Lys Gln Asn Val Ser Ser Leu Asp Glu Lys Asn Ser Val Ser Val
               20                  25                  30
   Asp Leu Pro Gly Gly Met Thr Val Leu Val Ser Lys Glu Lys Asp Lys
           35                  40                  45
   Asp Gly Lys Tyr Ser Leu Glu Ala Thr Val Asp Lys Leu Glu Leu Lys
       50                  55                  60
   Gly Thr Ser Asp Lys Asn Asn Gly Ser Gly Thr Leu Glu Gly Glu Lys
   65                  70                  75                  80
   Thr Asp Lys Ser Lys Val Lys Leu Thr Ile Ala Asp Asp Leu Ser Gln
                   85                  90                  95
   Thr Lys Phe Glu Ile Phe Lys Glu Asp Ala Lys Thr Leu Val Ser Lys
               100                 105                 110
   Lys Val Thr Leu Lys Asp Lys Ser Ser Thr Glu Glu Lys Phe Asn Glu
           115                 120                 125
   Lys Gly Glu Thr Ser Glu Lys Thr Ile Val Arg Ala Asn Gly Thr Arg
       130                 135                 140
   Leu Glu Tyr Thr Asp Ile Lys Ser Asp Gly Ser Gly Lys Ala Lys Glu
   145                 150                 155                 160
   Val Leu Lys Asp Phe Thr Leu Glu Gly Thr Leu Ala Ala Asp Gly Lys
                   165                 170                 175
   Thr Thr Leu Lys Val Thr Glu Gly Thr Val Val Leu Ser Lys Asn Ile
               180                 185                 190
   Leu Lys Ser Gly Glu Ile Thr Val Ala Leu Asp Asp Ser Asp Thr Thr
           195                 200                 205
   Gln Ala Thr Lys Lys Thr Gly Lys Trp Asp Ser Lys Thr Ser Thr Leu
       210                 215                 220
   Thr Ile Ser Val Asn Ser Lys Lys Thr Thr Gln Leu Val Phe Thr Lys
   225                 230                 235                 240
   Gln Tyr Thr Ile Thr Val Lys Gln Tyr Asp Ser Ala Gly Thr Asn Leu
                   245                 250                 255
   Glu Gly Thr Ala Val Glu Ile Lys Thr Leu Asp Glu Leu Lys Asn Ala
               260                 265                 270
   Leu Lys
<210> 87
   <211> 35
   <212> DNA
   <213> Artificial Sequence
<220>
   <223> Oligonucleotide Primer
<400> 87
   aaagtagaag tttttgaatt ccaagctgca gtttt 35
<210> 88
   <211> 822
   <212> DNA
   <213> Artificial Sequence
<220>
   <221> CDS
   <222> (1)...(822)
   <223> Chimeric nucleic acid
<400> 88
   atg aaa aaa tat tta ttg gga ata ggt cta ata tta gcc tta ata gca    48
   Met Lys Lys Tyr Leu Leu Gly Ile Gly Leu Ile Leu Ala Leu Ile Ala
    1               5                  10                  15
   tgc aag caa aat gtt agc agc ctt gat gaa aaa aac agc gct tca gta    96
   Cys Lys Gln Asn Val Ser Ser Leu Asp Glu Lys Asn Ser Ala Ser Val
               20                  25                  30
   gat ttg cct ggt gag atg aaa gtt ctt gta agt aaa gaa aaa gac aaa      144
   Asp Leu Pro Gly Glu Met Lys Val Leu Val Ser Lys Glu Lys Asp Lys
           35                  40                  45
   gac ggt aag tac agt cta aag gca aca gta gac aag att gag cta aaa      192
   Asp Gly Lys Tyr Ser Leu Lys Ala Thr Val Asp Lys Ile Glu Leu Lys
       50                  55                  60
   gga act tct gat aaa gac aat ggt tct gga gtg ctt gaa ggt aca aaa      240
   Gly Thr Ser Asp Lys Asp Asn Gly Ser Gly Val Leu Glu Gly Thr Lys
   65                  70                  75                  80
   gat gac aaa agt aaa gca aaa tta aca att gct gac gat cta agt aaa      288
   Asp Asp Lys Ser Lys Ala Lys Leu Thr Ile Ala Asp Asp Leu Ser Lys
                   85                  90                  95
   acc aca ttc gaa ctt tta aaa gaa gat ggc aaa aca tta gtg tca aga      336
   Thr Thr Phe Glu Leu Leu Lys Glu Asp Gly Lys Thr Leu Val Ser Arg
               100                 105                 110
   aaa gta agt tct aga gac aaa aca tca aca gat gaa atg ttc aat gaa      384
   Lys Val Ser Ser Arg Asp Lys Thr Ser Thr Asp Glu Met Phe Asn Glu
           115                 120                 125
   aaa ggt gaa ttg tct gca aaa acc atg aca aga gaa aat gga acc aaa      432
   Lys Gly Glu Leu Ser Ala Lys Thr Met Thr Arg Glu Asn Gly Thr Lys
       130                 135                 140
   ctt gaa tat aca gaa atg aaa agc gat gga acc gga aaa gct aaa gaa      480
   Leu Glu Tyr Thr Glu Met Lys Ser Asp Gly Thr Gly Lys Ala Lys Glu
   145                 150                 155                 160
   gtt tta aaa aag ttt act ctt gaa gga aaa gta gct aat gat aaa gta      528
   Val Leu Lys Lys Phe Thr Leu Glu Gly Lys Val Ala Asn Asp Lys Val
                   165                 170                 175
   aca ttg gaa gta aaa gaa gga acc gtt act tta agt aag gaa att gca      576
   Thr Leu Glu Val Lys Glu Gly Thr Val Thr Leu Ser Lys Glu Ile Ala
               180                 185                 190
   aaa tct gga gaa gta aca gtt gct ctt aat gac act aac act act cag      624
   Lys Ser Gly Glu Val Thr Val Ala Leu Asn Asp Thr Asn Thr Thr Gln
           195                 200                 205
   gct act aaa aaa act ggc gca tgg gat tca aaa act tct act tta aca      672
   Ala Thr Lys Lys Thr Gly Ala Trp Asp Ser Lys Thr Ser Thr Leu Thr
       210                 215                 220
   att agt gtt aac agc aaa aaa act aca caa ctt gtg ttt act aaa caa      720
   Ile Ser Val Asn Ser Lys Lys Thr Thr Gln Leu Val Phe Thr Lys Gln
   225                 230                 235                 240
   tac aca ata act gta aaa caa tac gac tcc gca ggt acc aat tta gaa      768
   Tyr Thr Ile Thr Val Lys Gln Tyr Asp Ser Ala Gly Thr Asn Leu Glu
                   245                 250                 255
   ggc aca gca gtc gaa att aaa aca ctt gat gaa ctt aaa aac gct tta      816
   Gly Thr Ala Val Glu Ile Lys Thr Leu Asp Glu Leu Lys Asn Ala Leu
               260                 265                 270
   aaa taa                                                              822
   Lys  *
<210> 89
   <211> 273
   <212> PRT
   <213> Artificial Sequence
<220>
   <223> Chimeric Protein
<400> 89
   Met Lys Lys Tyr Leu Leu Gly Ile Gly Leu Ile Leu Ala Leu Ile Ala
   1               5                   10                  15
   Cys Lys Gln Asn Val Ser Ser Leu Asp Glu Lys Asn Ser Ala Ser Val
               20                  25                  30
   Asp Leu Pro Gly Glu Met Lys Val Leu Val Ser Lys Glu Lys Asp Lys
           35                  40                  45
   Asp Gly Lys Tyr Ser Leu Lys Ala Thr Val Asp Lys Ile Glu Leu Lys
       50                  55                  60
   Gly Thr Ser Asp Lys Asp Asn Gly Ser Gly Val Leu Glu Gly Thr Lys
   65                  70                  75                  80
   Asp Asp Lys Ser Lys Ala Lys Leu Thr Ile Ala Asp Asp Leu Ser Lys
                   85                  90                  95
   Thr Thr Phe Glu Leu Leu Lys Glu Asp Gly Lys Thr Leu Val Ser Arg
               100                 105                 110
   Lys Val Ser Ser Arg Asp Lys Thr Ser Thr Asp Glu Met Phe Asn Glu
           115                 120                 125
   Lys Gly Glu Leu Ser Ala Lys Thr Met Thr Arg Glu Asn Gly Thr Lys
       130                 135                 140
   Leu Glu Tyr Thr Glu Met Lys Ser Asp Gly Thr Gly Lys Ala Lys Glu
   145                 150                 155                 160
   Val Leu Lys Lys Phe Thr Leu Glu Gly Lys Val Ala Asn Asp Lys Val
                   165                 170                 175
   Thr Leu Glu Val Lys Glu Gly Thr Val Thr Leu Ser Lys Glu Ile Ala
               180                 185                 190
   Lys Ser Gly Glu Val Thr Val Ala Leu Asn Asp Thr Asn Thr Thr Gln
           195                 200                 205
   Ala Thr Lys Lys Thr Gly Ala Trp Asp Ser Lys Thr Ser Thr Leu Thr
       210                 215                 220
   Ile Ser Val Asn Ser Lys Lys Thr Thr Gln Leu Val Phe Thr Lys Gln
   225                 230                 235                 240
   Tyr Thr Ile Thr Val Lys Gln Tyr Asp Ser Ala Gly Thr Asn Leu Glu
                   245                 250                 255
   Gly Thr Ala Val Glu Ile Lys Thr Leu Asp Glu Leu Lys Asn Ala Leu
               260                 265                 270
   Lys
<210> 90
   <211> 38
   <212> DNA
   <213> Artificial Sequence
<220>
   <223> Oligonucleotide Primer
<400> 90
   aaagtggaag tttttgaatt ccaagctgca gttttttt 38
<210> 91
   <211> 822
   <212> DNA
   <213> Artificial Sequence
<220>
   <221> CDS
   <222> (1)...(822)
   <223> Chimeric nucleic acid
<400> 91
   atg aaa aaa tat tta ttg gga ata ggt cta ata tta gcc tta ata gca      48
   Met Lys Lys Tyr Leu Leu Gly Ile Gly Leu Ile Leu Ala Leu Ile Ala
   1               5                   10                  15
   tgt aag caa aat gtt agc agc ctt gac gag aaa aac agc gtt tca gta      96
   Cys Lys Gln Asn Val Ser Ser Leu Asp Glu Lys Asn Ser Val Ser Val
               20                  25                  30
   gat ttg cct ggt gaa atg aaa gtt ctt gta agc aaa gaa aaa aac aaa      144
   Asp Leu Pro Gly Glu Met Lys Val Leu Val Ser Lys Glu Lys Asn Lys
           35                  40                  45
   gac ggc aag tac gat cta att gca aca gta gac aag ctt gag ctt aaa      192
   Asp Gly Lys Tyr Asp Leu Ile Ala Thr Val Asp Lys Leu Glu Leu Lys
       50                  55                  60
   gga act tct gat aaa aac aat gga tct gga gta ctt gaa ggc gta aaa      240
   Gly Thr Ser Asp Lys Asn Asn Gly Ser Gly Val Leu Glu Gly Val Lys
   65                  70                  75                  80
   gct gac aaa agt aaa gta aaa tta aca att tct gac gat cta ggt caa      288
   Ala Asp Lys Ser Lys Val Lys Leu Thr Ile Ser Asp Asp Leu Gly Gln
                   85                  90                  95
   acc aca ctt gaa gtt ttc aaa gaa gat ggc aaa aca cta gta tca aaa      336
   Thr Thr Leu Glu Val Phe Lys Glu Asp Gly Lys Thr Leu Val Ser Lys
               100                 105                 110
   aaa gta act tcc aaa gac aag tca tca aca gaa gaa aaa ttc aat gaa      384
   Lys Val Thr Ser Lys Asp Lys Ser Ser Thr Glu Glu Lys Phe Asn Glu
           115                 120                 125
   aaa ggt gaa gta tct gaa aaa ata ata aca aga gca gac gga acc aga      432
   Lys Gly Glu Val Ser Glu Lys Ile Ile Thr Arg Ala Asp Gly Thr Arg
       130                 135                 140
   ctt gaa tac aca gga att aaa agc gat gga tct gga aaa gct aaa gag      480
   Leu Glu Tyr Thr Gly Ile Lys Ser Asp Gly Ser Gly Lys Ala Lys Glu
   145                 150                 155                 160
   gtt tta aaa ggc tat gtt ctt gaa gga act cta act gct gaa aaa aca      528
   Val Leu Lys Gly Tyr Val Leu Glu Gly Thr Leu Thr Ala Glu Lys Thr
                   165                 170                 175
   aca ttg gtg gtt aaa gaa gga act gtt act tta agc aaa aat att tca      576
   Thr Leu Val Val Lys Glu Gly Thr Val Thr Leu Ser Lys Asn Ile Ser
               180                 185                 190
   aaa tct ggg gaa gtt tca gtt gaa ctt aat gac act gac agt agt gct     624
   Lys Ser Gly Glu Val Ser Val Glu Leu Asn Asp Thr Asp Ser Ser Ala
           195                 200                 205
   gct act aaa aaa act gca gct tgg aat tca aaa act tcc act tta aca     672
   Ala Thr Lys Lys Thr Ala Ala Trp Asn Ser Lys Thr Ser Thr Leu Thr
       210                 215                 220
   att agt gtg aat agc caa aaa acc aaa aac ctt gta ttc aca aaa gaa     720
   Ile Ser Val Asn Ser Gln Lys Thr Lys Asn Leu Val Phe Thr Lys Glu
   225                 230                 235                 240
   gac aca ata aca gta caa aaa tac gac tca gca ggc acc aat cta gaa     768
   Asp Thr Ile Thr Val Gln Lys Tyr Asp Ser Ala Gly Thr Asn Leu Glu
                   245                 250                 255
   ggc aaa gca gtc gaa att aca aca ctt aaa gaa ctt aaa aac gct tta     816
   Gly Lys Ala Val Glu Ile Thr Thr Leu Lys Glu Leu Lys Asn Ala Leu
               260                 265                 270
   aaa taa                                                             822
   Lys  *
<210> 92
   <211> 273
   <212> PRT
   <213> Artificial Sequence
<220>
   <223> Chimeric Protein
<400> 92
   Met Lys Lys Tyr Leu Leu Gly Ile Gly Leu Ile Leu Ala Leu Ile Ala
    1               5                  10                  15
   Cys Lys Gln Asn Val Ser Ser Leu Asp Glu Lys Asn Ser Val Ser Val
               20                  25                  30
   Asp Leu Pro Gly Glu Met Lys Val Leu Val Ser Lys Glu Lys Asn Lys
           35                  40                  45
   Asp Gly Lys Tyr Asp Leu Ile Ala Thr Val Asp Lys Leu Glu Leu Lys
       50                  55                  60
   Gly Thr Ser Asp Lys Asn Asn Gly Ser Gly Val Leu Glu Gly Val Lys
   65                  70                  75                  80
   Ala Asp Lys Ser Lys Val Lys Leu Thr Ile Ser Asp Asp Leu Gly Gln
                   85                  90                  95
   Thr Thr Leu Glu Val Phe Lys Glu Asp Gly Lys Thr Leu Val Ser Lys
               100                 105                 110
   Lys Val Thr Ser Lys Asp Lys Ser Ser Thr Glu Glu Lys Phe Asn Glu
           115                 120                 125
   Lys Gly Glu Val Ser Glu Lys Ile Ile Thr Arg Ala Asp Gly Thr Arg
       130                 135                 140
   Leu Glu Tyr Thr Gly Ile Lys Ser Asp Gly Ser Gly Lys Ala Lys Glu
   145                 150                 155                 160
   Val Leu Lys Gly Tyr Val Leu Glu Gly Thr Leu Thr Ala Glu Lys Thr
                   165                 170                 175
   Thr Leu Val Val Lys Glu Gly Thr Val Thr Leu Ser Lys Asn Ile Ser
               180                 185                 190
   Lys Ser Gly Glu Val Ser Val Glu Leu Asn Asp Thr Asp Ser Ser Ala
           195                 200                 205
   Ala Thr Lys Lys Thr Ala Ala Trp Asn Ser Lys Thr Ser Thr Leu Thr
       210                 215                 220
   Ile Ser Val Asn Ser Gln Lys Thr Lys Asn Leu Val Phe Thr Lys Glu
   225                 230                 235                 240
   Asp Thr Ile Thr Val Gln Lys Tyr Asp Ser Ala Gly Thr Asn Leu Glu
                   245                 250                 255
   Gly Lys Ala Val Glu Ile Thr Thr Leu Lys Glu Leu Lys Asn Ala Leu
               260                 265                 270
   Lys
<210> 93
   <211> 35
   <212> DNA
   <213> Artificial Sequence
<220>
   <223> oligonucleotide Primer
<400> 93
   taaagttgaa gtgcctgcat tccaagctgc agttt 35
<210> 94
   <211> 819
   <212> DNA
   <213> Artificial Sequence
<220>
   <221> CDS
   <222> (1)...(819)
   <223> Chimeric nucleic acid
<400> 94
   atg aaa aaa tat tta ttg gga ata ggt cta ata tta gcc tta ata gca      48
   Met Lys Lys Tyr Leu Leu Gly Ile Gly Leu Ile Leu Ala Leu Ile Ala
    1               5                  10                  15
   tgt aag caa aat gtt agc agc ctt gac gag aaa aac agc gtt tca gta      96
   Cys Lys Gln Asn Val Ser Ser Leu Asp Glu Lys Asn Ser Val Ser Val
               20                  25                  30
   gat ttg cct ggt gaa atg aaa gtt ctt gta agc aaa gaa aaa aac aaa     144
   Asp Leu Pro Gly Glu Met Lys Val Leu Val Ser Lys Glu Lys Asn Lys
           35                  40                  45
   gac ggc aag tac gat cta att gca aca gta gac aag ctt gag ctt aaa     192
   Asp Gly Lys Tyr Asp Leu Ile Ala Thr Val Asp Lys Leu Glu Leu Lys
       50                  55                  60
   gga act tct gat aaa aac aat gga tct gga gta ctt gaa ggc gta aaa     240
   Gly Thr Ser Asp Lys Asn Asn Gly Ser Gly Val Leu Glu Gly Val Lys
   65                  70                  75                  80
   gct gac aaa agt aaa gta aaa tta aca att tct gac gat cta ggt caa     288
   Ala Asp Lys Ser Lys Val Lys Leu Thr Ile Ser Asp Asp Leu Gly Gln
                   85                  90                  95
   acc aca ctt gaa gtt ttc aaa gaa gat ggc aaa aca cta gta tca aaa     336
   Thr Thr Leu Glu Val Phe Lys Glu Asp Gly Lys Thr Leu Val Ser Lys
               100                 105                 110
   aaa gta act tcc aaa gac aag tca tca aca gaa gaa aaa ttc aat gaa    384
   Lys Val Thr Ser Lys Asp Lys Ser Ser Thr Glu Glu Lys Phe Asn Glu
           115                 120                     125
   aaa ggt gaa gta tct gaa aaa ata ata aca aga gca gac gga acc aga    432
   Lys Gly Glu Val Ser Glu Lys Ile Ile Thr Arg Ala Asp Gly Thr Arg
       130                 135                     140
   ctt gaa tac aca gga att aaa agc gat gga tct gga aaa gct aaa gag    480
   Leu Glu Tyr Thr Gly Ile Lys Ser Asp Gly Ser Gly Lys Ala Lys Glu
   145                 150                     155                 160
   gtt tta aaa ggc tat gtt ctt gaa gga act cta act gct gaa aaa aca    528
   Val Leu Lys Gly Tyr Val Leu Glu Gly Thr Leu Thr Ala Glu Lys Thr
                   165                     170                 175
   aca ttg gtg gtt aaa gaa gga act gtt act tta agc aaa aat att tca    576
   Thr Leu Val Val Lys Glu Gly Thr Val Thr Leu Ser Lys Asn Ile Ser
               180                     185                 190
   aaa tct ggg gaa gtt tca gtt gaa ctt aat gac act gac agt agt gct    624
   Lys Ser Gly Glu Val Ser Val Glu Leu Asn Asp Thr Asp Ser Ser Ala
           195                 200                     205
   gct act aaa aaa act gca gct tgg aat gca ggc act tca act tta aca    672
   Ala Thr Lys Lys Thr Ala Ala Trp Asn Ala Gly Thr Ser Thr Leu Thr
       210                 215                     220
   att act gta aac aac aaa aaa act aaa gcc ctt gta ttt aca aaa caa    720
   Ile Thr Val Asn Asn Lys Lys Thr Lys Ala Leu Val Phe Thr Lys Gln
   225                 230                     235                 240
   gac aca att aca tca caa aaa tac gac tca gca gga acc aac ttg gaa    768
   Asp Thr Ile Thr Ser Gln Lys Tyr Asp Ser Ala Gly Thr Asn Leu Glu
                   245                     250                 255
   ggc aca gca gtc gaa att aaa aca ctt gat gaa ctt aaa aac gct tta    816
   Gly Thr Ala Val Glu Ile Lys Thr Leu Asp Glu Leu Lys Asn Ala Leu
               260                     265                 270
   aga                                                                819
   Arg
<210> 95
   <211> 273
   <212> PRT
   <213> Artificial Sequence
<220>
   <223> Chimeric Protein
<400> 95
   Met Lys Lys Tyr Leu Leu Gly Ile Gly Leu Ile Leu Ala Leu Ile Ala
   1               5                   10                  15
   Cys Lys Gln Asn Val Ser Ser Leu Asp Glu Lys Asn Ser Val Ser Val
               20                  25                  30
   Asp Leu Pro Gly Glu Met Lys Val Leu Val Ser Lys Glu Lys Asn Lys
           35                  40                  45
   Asp Gly Lys Tyr Asp Leu Ile Ala Thr Val Asp Lys Leu Glu Leu Lys
       50                  55                  60
   Gly Thr Ser Asp Lys Asn Asn Gly Ser Gly Val Leu Glu Gly Val Lys
   65                  70                  75                  80
   Ala Asp Lys Ser Lys Val Lys Leu Thr Ile Ser Asp Asp Leu Gly Gln
                   85                  90                  95
   Thr Thr Leu Glu Val Phe Lys Glu Asp Gly Lys Thr Leu Val Ser Lys
               100                 105                 110
   Lys Val Thr Ser Lys Asp Lys Ser Ser Thr Glu Glu Lys Phe Asn Glu
           115                 120                 125
   Lys Gly Glu Val Ser Glu Lys Ile Ile Thr Arg Ala Asp Gly Thr Arg
       130                 135                 140
   Leu Glu Tyr Thr Gly Ile Lys Ser Asp Gly Ser Gly Lys Ala Lys Glu
   145                 150                 155                 160
   Val Leu Lys Gly Tyr Val Leu Glu Gly Thr Leu Thr Ala Glu Lys Thr
                   165                 170                 175
   Thr Leu Val Val Lys Glu Gly Thr Val Thr Leu Ser Lys Asn Ile Ser
               180                 185                 190
   Lys Ser Gly Glu Val Ser Val Glu Leu Asn Asp Thr Asp Ser Ser Ala
           195                 200                 205
   Ala Thr Lys Lys Thr Ala Ala Trp Asn Ala Gly Thr Ser Thr Leu Thr
       210                 215                 220
   Ile Thr Val Asn Asn Lys Lys Thr Lys Ala Leu Val Phe Thr Lys Gln
   225                 230                 235                 240
   Asp Thr Ile Thr Ser Gln Lys Tyr Asp Ser Ala Gly Thr Asn Leu Glu
                   245                 250                 255
   Gly Thr Ala Val Glu Ile Lys Thr Leu Asp Glu Leu Lys Asn Ala Leu
               260                 265                 270
   Arg
<210> 96
   <211> 31
   <212> DNA
   <213> Artificial Sequence
<220>
   <223> Oligonucleotide Primer
<400> 96
   ccccagattt tgaaatcttg cttaaaacaa c 31
<210> 97
   <211> 822
   <212> DNA
   <213> Artificial Sequence
<220>
   <221> CDS
   <222> (1)...(822)
   <223> Chimeric nucleic acid
<400> 97
   atg aaa aaa tat tta ttg gga ata ggt cta ata tta gcc tta ata gca    48
   Met Lys Lys Tyr Leu Leu Gly Ile Gly Leu Ile Leu Ala Leu Ile Ala
    1               5                  10                  15
   tgt aag caa aat gtt agc agc ctt gat gaa aaa aat agc gtt tca gta    96
   Cys Lys Gln Asn Val Ser Ser Leu Asp Glu Lys Asn Ser Val Ser Val
               20                  25                  30
   gat tta cct ggt gga atg aca gtt ctt gta agt aaa gaa aaa gac aaa    144
   Asp Leu Pro Gly Gly Met Thr Val Leu Val Ser Lys Glu Lys Asp Lys
            35                  40                  45
   gac ggt aaa tac agt cta gag gca aca gta gac aag ctt gag ctt aaa    192
   Asp Gly Lys Tyr Ser Leu Glu Ala Thr Val Asp Lys Leu Glu Leu Lys
        50                  55                  60
   gga act tct gat aaa aac aac ggt tct gga aca ctt gaa ggt gaa aaa    240
   Gly Thr Ser Asp Lys Asn Asn Gly Ser Gly Thr Leu Glu Gly Glu Lys
    65                  70                  75                  80
   act gac aaa agt aaa gta aaa tta aca att gct gat gac cta agt caa    288
   Thr Asp Lys Ser Lys Val Lys Leu Thr Ile Ala Asp Asp Leu Ser Gln
                    85                  90                  95
   act aaa ttt gaa att ttc aaa gaa gat gcc aaa aca tta gta tca aaa    336
   Thr Lys Phe Glu Ile Phe Lys Glu Asp Ala Lys Thr Leu Val Ser Lys
               100                 105                 110
   aaa gta acc ctt aaa gac aag tca tca aca gaa gaa aaa ttc aac gaa    384
   Lys Val Thr Leu Lys Asp Lys Ser Ser Thr Glu Glu Lys Phe Asn Glu
           115                 120                 125
   aag ggt gaa aca tct gaa aaa aca ata gta aga gca aat gga acc aga    432
   Lys Gly Glu Thr Ser Glu Lys Thr Ile Val Arg Ala Asn Gly Thr Arg
       130                 135                 140
   ctt gaa tac aca gac ata aaa agc gat gga tcc gga aaa gct aaa gaa    480
   Leu Glu Tyr Thr Asp Ile Lys Ser Asp Gly Ser Gly Lys Ala Lys Glu
   145                 150                 155                 160
   gtt tta aaa gac ttt act ctt gaa gga act cta gct gct gac ggc aaa    528
   Val Leu Lys Asp Phe Thr Leu Glu Gly Thr Leu Ala Ala Asp Gly Lys
                   165                 170                 175
   aca aca ttg aaa gtt aca gaa ggc act gtt gtt tta agc aag att tca    576
   Thr Thr Leu Lys Val Thr Glu Gly Thr Val Val Leu Ser Lys Ile Ser
               180                 185                 190
   aaa tct ggg gaa gtt tca gtt gaa ctt aat gac act gac agt agt gct    624
   Lys Ser Gly Glu Val Ser Val Glu Leu Asn Asp Thr Asp Ser Ser Ala
           195                 200                 205
   gct act aaa aaa act gca gct tgg aat tca aaa act tcc act tta aca    672
   Ala Thr Lys Lys Thr Ala Ala Trp Asn Ser Lys Thr Ser Thr Leu Thr
       210                 215                 220
   att agt gtg aat agc caa aaa acc aaa aac ctt gta ttc aca aaa gaa    720
   Ile Ser Val Asn Ser Gln Lys Thr Lys Asn Leu Val Phe Thr Lys Glu
   225                 230                 235                 240
   gac aca ata aca gta caa aaa tac gac tca gca ggc acc aat cta gaa    768
   Asp Thr Ile Thr Val Gln Lys Tyr Asp Ser Ala Gly Thr Asn Leu Glu
                   245                 250                 255
   ggc aaa gca gtc gaa att aca aca ctt aaa gaa ctt aaa aac gct tta    816
   Gly Lys Ala Val Glu Ile Thr Thr Leu Lys Glu Leu Lys Asn Ala Leu
               260                 265                 270
   aaa taa                                                            822
   Lys  *
<210> 98
   <211> 273
   <212> PRT
   <213> Artificial Sequence
<220>
   <223> Chimeric Protein
<400> 98
   Met Lys Lys Tyr Leu Leu Gly Ile Gly Leu Ile Leu Ala Leu Ile Ala
   1               5                   10                  15
   Cys Lys Gln Asn Val Ser Ser Leu Asp Glu Lys Asn Ser Val Ser Val
               20                  25                  30
   Asp Leu Pro Gly Gly Met Thr Val Leu Val Ser Lys Glu Lys Asp Lys
           35                  40                  45
   Asp Gly Lys Tyr Ser Leu Glu Ala Thr Val Asp Lys Leu Glu Leu Lys
       50                  55                  60
   Gly Thr Ser Asp Lys Asn Asn Gly Ser Gly Thr Leu Glu Gly Glu Lys
   65                  70                  75                  80
   Thr Asp Lys Ser Lys Val Lys Leu Thr Ile Ala Asp Asp Leu Ser Gln
                   85                  90                  95
   Thr Lys Phe Glu Ile Phe Lys Glu Asp Ala Lys Thr Leu Val Ser Lys
               100                 105                 110
   Lys Val Thr Leu Lys Asp Lys Ser Ser Thr Glu Glu Lys Phe Asn Glu
           115                 120                 125
   Lys Gly Glu Thr Ser Glu Lys Thr Ile Val Arg Ala Asn Gly Thr Arg
       130                 135                 140
   Leu Glu Tyr Thr Asp Ile Lys Ser Asp Gly Ser Gly Lys Ala Lys Glu
   145                 150                 155                 160
   Val Leu Lys Asp Phe Thr Leu Glu Gly Thr Leu Ala Ala Asp Gly Lys
                   165                 170                 175
   Thr Thr Leu Lys Val Thr Glu Gly Thr Val Val Leu Ser Lys Ile Ser
               180                 185                 190
   Lys Ser Gly Glu Val Ser Val Glu Leu Asn Asp Thr Asp Ser Ser Ala
           195                 200                 205
   Ala Thr Lys Lys Thr Ala Ala Trp Asn Ser Lys Thr Ser Thr Leu Thr
       210                 215                 220
   Ile Ser Val Asn Ser Gln Lys Thr Lys Asn Leu Val Phe Thr Lys Glu
   225                 230                 235                 240
   Asp Thr Ile Thr Val Gln Lys Tyr Asp Ser Ala Gly Thr Asn Leu Glu
                   245                 250                 255
   Gly Lys Ala Val Glu Ile Thr Thr Leu Lys Glu Leu Lys Asn Ala Leu
               260                 265                 270
   Lys
<210> 99
   <211> 32
   <212> DNA
   <213> Artificial Sequence
<220>
   <223> Oligonucleotide Primer
<400> 99
   caagtctggt tccaatttgc tcttgttatt at 32
<210> 100
   <211> 822
   <212> DNA
   <213> Artificial Sequence
<220>
   <221> CDS
   <222> (1)...(822)
   <223> Chimeric nucleic acid
<400> 100
   atg aaa aaa tat tta ttg gga ata ggt cta ata tta gcc tta ata gca      48
   Met Lys Lys Tyr Leu Leu Gly Ile Gly Leu Ile Leu Ala Leu Ile Ala
    1               5                  10                  15
   tgt aag caa aat gtt agc agc ctt gat gaa aaa aat agc gtt tca gta      96
   Cys Lys Gln Asn Val Ser Ser Leu Asp Glu Lys Asn Ser Val Ser Val
               20                  25                  30
   gat tta cct ggt gga atg aca gtt ctt gta agt aaa gaa aaa gac aaa     144
   Asp Leu Pro Gly Gly Met Thr Val Leu Val Ser Lys Glu Lys Asp Lys
           35                  40                  45
   gac ggt aaa tac agt cta gag gca aca gta gac aag ctt gag ctt aaa     192
   Asp Gly Lys Tyr Ser Leu Glu Ala Thr Val Asp Lys Leu Glu Leu Lys
       50                  55                  60
   gga act tct gat aaa aac aac ggt tct gga aca ctt gaa ggt gaa aaa     240
   Gly Thr Ser Asp Lys Asn Asn Gly Ser Gly Thr Leu Glu Gly Glu Lys
   65                  70                  75                  80
   act gac aaa agt aaa gta aaa tta aca att gct gat gac cta agt caa     288
   Thr Asp Lys Ser Lys Val Lys Leu Thr Ile Ala Asp Asp Leu Ser Gln
                   85                  90                  95
   act aaa ttt gaa att ttc aaa gaa gat gcc aaa aca tta gta tca aaa     336
   Thr Lys Phe Glu Ile Phe Lys Glu Asp Ala Lys Thr Leu Val Ser Lys
               100                 105                 110
   aaa gta acc ctt aaa gac aag tca tca aca gaa gaa aaa ttc aac gaa     384
   Lys Val Thr Leu Lys Asp Lys Ser Ser Thr Glu Glu Lys Phe Asn Glu
           115                 120                 125
   aag ggt gaa aca tct gaa aaa aca ata gta aga gca aat gga acc aga     432
   Lys Gly Glu Thr Ser Glu Lys Thr Ile Val Arg Ala Asn Gly Thr Arg
       130                 135                 140
   ctt gaa tac aca gac ata aaa agc gat gga tcc gga aaa gct aaa gaa     480
   Leu Glu Tyr Thr Asp Ile Lys Ser Asp Gly Ser Gly Lys Ala Lys Glu
   145                 150                 155                 160
   gtt tta aaa gac ttt act ctt gaa gga act cta gct gct gac ggc aaa     528
   Val Leu Lys Asp Phe Thr Leu Glu Gly Thr Leu Ala Ala Asp Gly Lys
                   165                 170                 175
   aca aca ttg aaa gtt aca gaa ggc act gtt gtt tta agc aag att tca     576
   Thr Thr Leu Lys Val Thr Glu Gly Thr Val Val Leu Ser Lys Ile Ser
               180                 185                 190
   aaa tct ggg gaa gtt tca gtt gaa ctt aat gac act gac agt agt gct    624
   Lys Ser Gly Glu Val Ser Val Glu Leu Asn Asp Thr Asp Ser Ser Ala
           195                 200                 205
   gct act aaa aaa act gca gct tgg aat tca aaa act tcc act tta aca    672
   Ala Thr Lys Lys Thr Ala Ala Trp Asn Ser Lys Thr Ser Thr Leu Thr
       210                 215                 220
   att agt gtg aat agc caa aaa acc aaa aac ctt gta ttc aca aaa gaa    720
   Ile Ser Val Asn Ser Gln Lys Thr Lys Asn Leu Val Phe Thr Lys Glu
   225                 230                 235                 240
   gac aca ata aca gta caa aaa tac gac tca gca ggc acc aat cta gaa    768
   Asp Thr Ile Thr Val Gln Lys Tyr Asp Ser Ala Gly Thr Asn Leu Glu
                   245                 250                 255
   ggc aaa gca gtc gaa att aca aca ctt aaa gaa ctt aaa aac gct tta    816
   Gly Lys Ala Val Glu Ile Thr Thr Leu Lys Glu Leu Lys Asn Ala Leu
               260                 265                 270
   aaa taa                                                            822
   Lys  *
<210> 101
   <211> 273
   <212> PRT
   <213> Artificial Sequence
<220>
   <223> Chimeric Protein
<400> 101
   Met Lys Lys Tyr Leu Leu Gly Ile Gly Leu Ile Leu Ala Leu Ile Ala
   1               5                   10                  15
   Cys Lys Gln Asn Val Ser Ser Leu Asp Glu Lys Asn Ser Val Ser Val
               20                  25                  30
   Asp Leu Pro Gly Gly Met Thr Val Leu Val Ser Lys Glu Lys Asp Lys
           35                  40                  45
   Asp Gly Lys Tyr Ser Leu Glu Ala Thr Val Asp Lys Leu Glu Leu Lys
       50                  55                  60
   Gly Thr Ser Asp Lys Asn Asn Gly Ser Gly Thr Leu Glu Gly Glu Lys
   65                  70                  75                  80
   Thr Asp Lys Ser Lys Val Lys Leu Thr Ile Ala Asp Asp Leu Ser Gln
                   85                  90                  95
   Thr Lys Phe Glu Ile Phe Lys Glu Asp Ala Lys Thr Leu Val Ser Lys
               100                 105                 110
   Lys Val Thr Leu Lys Asp Lys Ser Ser Thr Glu Glu Lys Phe Asn Glu
           115                 120                 125
   Lys Gly Glu Thr Ser Glu Lys Thr Ile Val Arg Ala Asn Gly Thr Arg
       130                 135                 140
   Leu Glu Tyr Thr Asp Ile Lys Ser Asp Gly Ser Gly Lys Ala Lys Glu
   145                 150                 155                 160
   Val Leu Lys Asp Phe Thr Leu Glu Gly Thr Leu Ala Ala Asp Gly Lys
                   165                 170                 175
   Thr Thr Leu Lys Val Thr Glu Gly Thr Val Val Leu Ser Lys Ile Ser
               180                 185                 190
   Lys Ser Gly Glu Val Ser Val Glu Leu Asn Asp Thr Asp Ser Ser Ala
           195                 200                 205
   Ala Thr Lys Lys Thr Ala Ala Trp Asn Ser Lys Thr Ser Thr Leu Thr
       210                 215                 220
   Ile Ser Val Asn Ser Gln Lys Thr Lys Asn Leu Val Phe Thr Lys Glu
   225                 230                 235                 240
   Asp Thr Ile Thr Val Gln Lys Tyr Asp Ser Ala Gly Thr Asn Leu Glu
                   245                 250                 255
   Gly Lys Ala Val Glu Ile Thr Thr Leu Lys Glu Leu Lys Asn Ala Leu
               260                 265                 270
   Lys
<210> 102
   <211> 41
   <212> DNA
   <213> Artificial Sequence
<220>
   <223> Oligonucleotide Primer
<400> 102
   gttaaagtgc tagtactgtc attccaagct gcagtttttt t 41
<210> 103
   <211> 822
   <212> DNA
   <213> Artificial Sequence
<220>
   <221> CDS
   <222> (1)...(822)
   <223> Chimeric nucleic acid
<400> 103
<210> 104
   <211> 273
   <212> PRT
   <213> Artificial Sequence
<220>
   <223> Chimeric Protein
<400> 104
   Met Lys Lys Tyr Leu Leu Gly Ile Gly Leu Ile Leu Ala Leu Ile Ala
    1               5                  10                  15
   Cys Lys Gln Asn Val Ser Ser Leu Asp Glu Lys Asn Ser Val Ser Val
               20                  25                  30
   Asp Leu Pro Gly Glu Met Lys Val Leu Val Ser Lys Glu Lys Asn Lys
           35                  40                  45
   Asp Gly Lys Tyr Asp Leu Ile Ala Thr Val Asp Lys Leu Glu Leu Lys
       50                  55                  60
   Gly Thr Ser Asp Lys Asn Asn Gly Ser Gly Val Leu Glu Gly Val Lys
   65                  70                  75                  80
   Ala Asp Lys Ser Lys Val Lys Leu Thr Ile Ser Asp Asp Leu Gly Gln
                   85                  90                  95
   Thr Thr Leu Glu Val Phe Lys Glu Asp Gly Lys Thr Leu Val Ser Lys
               100                 105                 110
   Lys Val Thr Ser Lys Asp Lys Ser Ser Thr Glu Glu Lys Phe Asn Glu
           115                 120                 125
   Lys Gly Glu Val Ser Glu Lys Ile Ile Thr Arg Ala Asp Gly Thr Arg
       130                 135                 140
   Leu Glu Tyr Thr Gly Ile Lys Ser Asp Gly Ser Gly Lys Ala Lys Glu
   145                 150                 155                 160
   Val Leu Lys Gly Tyr Val Leu Glu Gly Thr Leu Thr Ala Glu Lys Thr
                   165                 170                 175
   Thr Leu Val Val Lys Glu Gly Thr Val Thr Leu Ser Lys Asn Ile Ser
               180                 185                 190
   Lys Ser Gly Glu Val Ser Val Glu Leu Asn Asp Thr Asp Ser Ser Ala
           195                 200                 205
   Ala Thr Lys Lys Thr Ala Ala Trp Asn Asp Ser Thr Ser Thr Leu Thr
       210                 215                 220
   Ile Ser Ala Asp Ser Lys Lys Thr Lys Asp Leu Val Phe Leu Thr Asp
   225                 230                 235                 240
   Gly Thr Ile Thr Val Gln Gln Tyr Asn Thr Ala Gly Thr Ser Leu Glu
                   245                 250                 255
   Gly Ser Ala Ser Glu Ile Lys Asn Leu Ser Glu Leu Lys Asn Ala Leu
               260                 265                 270
   Lys
<210> 105
   <211> 39
   <212> DNA
   <213> Artificial Sequence
<220>
   <223> Oligonucleotide Primer
<400> 105
   tgcagatgta atcccatccg ccatttttaa agcgttttt 39
<210> 106
   <211> 1401
   <212> DNA
   <213> Artificial Sequence
<220>
   <221> CDS
   <222> (1)...(1401)
   <223> Chimeric nucleic acid
<400> 106
   atg aaa aaa tat tta ttg gga ata ggt cta ata tta gcc tta ata gca      48
   Met Lys Lys Tyr Leu Leu Gly Ile Gly Leu Ile Leu Ala Leu Ile Ala
    1               5                  10                  15
   tgt aag caa aat gtt agc agc ctt gac gag aaa aac agc gtt tca gta      96
   Cys Lys Gln Asn Val Ser Ser Leu Asp Glu Lys Asn Ser Val Ser Val
               20                  25                  30
   gat ttg cct ggt gaa atg aaa gtt ctt gta agc aaa gaa aaa aac aaa     144
   Asp Leu Pro Gly Glu Met Lys Val Leu Val Ser Lys Glu Lys Asn Lys
           35                  40                  45
   gac ggc aag tac gat cta att gca aca gta gac aag ctt gag ctt aaa      192
   Asp Gly Lys Tyr Asp Leu Ile Ala Thr Val Asp Lys Leu Glu Leu Lys
        50                  55                  60
   gga act tct gat aaa aac aat gga tct gga gta ctt gaa ggc gta aaa      240
   Gly Thr Ser Asp Lys Asn Asn Gly Ser Gly Val Leu Glu Gly Val Lys
    65                  70                  75                  80
   gct gac aaa agt aaa gta aaa tta aca att tct gac gat cta ggt caa      288
   Ala Asp Lys Ser Lys Val Lys Leu Thr Ile Ser Asp Asp Leu Gly Gln
                    85                  90                  95
   acc aca ctt gaa gtt ttc aaa gaa gat ggc aaa aca cta gta tca aaa      336
   Thr Thr Leu Glu Val Phe Lys Glu Asp Gly Lys Thr Leu Val Ser Lys
               100                 105                 110
   aaa gta act tcc aaa gac aag tca tca aca gaa gaa aaa ttc aat gaa      384
   Lys Val Thr Ser Lys Asp Lys Ser Ser Thr Glu Glu Lys Phe Asn Glu
           115                 120                 125
   aaa ggt gaa gta tct gaa aaa ata ata aca aga gca gac gga acc aga      432
   Lys Gly Glu Val Ser Glu Lys Ile Ile Thr Arg Ala Asp Gly Thr Arg
       130                 135                 140
   ctt gaa tac aca gga att aaa agc gat gga tct gga aaa gct aaa gag      480
   Leu Glu Tyr Thr Gly Ile Lys Ser Asp Gly Ser Gly Lys Ala Lys Glu
   145                 150                 155                 160
   gtt tta aaa ggc tat gtt ctt gaa gga act cta act gct gaa aaa aca      528
   Val Leu Lys Gly Tyr Val Leu Glu Gly Thr Leu Thr Ala Glu Lys Thr
                   165                 170                 175
   aca ttg gtg gtt aaa gaa gga act gtt act tta agc aaa aat att tca      576
   Thr Leu Val Val Lys Glu Gly Thr Val Thr Leu Ser Lys Asn Ile Ser
               180                 185                 190
   aaa tct ggg gaa gtt tca gtt gaa ctt aat gac act gac agt agt gct      624
   Lys Ser Gly Glu Val Ser Val Glu Leu Asn Asp Thr Asp Ser Ser Ala
           195                 200                 205
   gct act aaa aaa act gca gct tgg aat gac agt act agc act tta aca      672
   Ala Thr Lys Lys Thr Ala Ala Trp Asn Asp Ser Thr Ser Thr Leu Thr
       210                 215                 220
   att agt gct gac agc aaa aaa act aaa gat ttg gtg ttc tta aca gat      720
   Ile Ser Ala Asp Ser Lys Lys Thr Lys Asp Leu Val Phe Leu Thr Asp
   225                 230                 235                 240
   ggt aca att aca gta caa caa tac aac aca gct gga acc agc cta gaa      768
   Gly Thr Ile Thr Val Gln Gln Tyr Asn Thr Ala Gly Thr Ser Leu Glu
                   245                 250                 255
   gga tca gca agt gaa att aaa aat ctt tca gag ctt aaa aac gct tta      816
   Gly Ser Ala Ser Glu Ile Lys Asn Leu Ser Glu Leu Lys Asn Ala Leu
               260                 265                 270
   aaa atg gct aat aat tca ggg aaa gat ggg aat aca tct gca aat tct      864
   Lys Met Ala Asn Asn Ser Gly Lys Asp Gly Asn Thr Ser Ala Asn Ser
           275                 280                 285
   gct gat gag tct gtt aaa ggg cct aat ctt aca gaa ata agt aaa aaa    912
   Ala Asp Glu Ser Val Lys Gly Pro Asn Leu Thr Glu Ile Ser Lys Lys
       290                 295                 300
   att acg gat tct aat gcg gtt tta ctt gct gtg aaa gag gtt gaa gcg    960
   Ile Thr Asp Ser Asn Ala Val Leu Leu Ala Val Lys Glu Val Glu Ala
   305                 310                 315                 320
   ttg ctg tca tct ata gat gaa att gct gct aaa gct att ggt aaa aaa    1008
   Leu Leu Ser Ser Ile Asp Glu Ile Ala Ala Lys Ala Ile Gly Lys Lys
                   325                 330                 335
   ata cac caa aat aat ggt ttg gat acc gaa tat aat cac aat gga tca    1056
   Ile His Gln Asn Asn Gly Leu Asp Thr Glu Tyr Asn His Asn Gly Ser
               340                 345                 350
   ttg tta gcg gga cgt tat gca ata tca acc cta ata aaa caa aaa tta    1104
   Leu Leu Ala Gly Arg Tyr Ala Ile Ser Thr Leu Ile Lys Gln Lys Leu
           355                 360                 365
   gat gga ttg aaa aat gaa gga tta aag gaa aaa att gat gcg gct aag    1152
   Asp Gly Leu Lys Asn Glu Gly Leu Lys Glu Lys Ile Asp Ala Ala Lys
       370                 375                 380
   aaa tgt tct gaa aca ttt act aat aaa tta aaa gca aaa cac aca gat    1200
   Lys Cys Ser Glu Thr Phe Thr Asn Lys Leu Lys Ala Lys His Thr Asp
   385                 390                 395                 400
   ctt ggt aaa gaa ggt gtt act gat gct gat gca aaa gaa gcc att tta    1248
   Leu Gly Lys Glu Gly Val Thr Asp Ala Asp Ala Lys Glu Ala Ile Leu
                   405                 410                 415
   aaa aca aat ggt act aaa act aaa ggt gct gaa gaa ctt gga aaa tta    1296
   Lys Thr Asn Gly Thr Lys Thr Lys Gly Ala Glu Glu Leu Gly Lys Leu
               420                 425                 430
   ttt gaa tca gta gag gtc ttg tca aaa gca gct aaa gag atg ctt gct    1344
   Phe Glu Ser Val Glu Val Leu Ser Lys Ala Ala Lys Glu Met Leu Ala
           435                 440                 445
   aat tca gtt aaa gag ctt aca agc cct gtt gtg gca gaa agt cca aaa    1392
   Asn Ser Val Lys Glu Leu Thr Ser Pro Val Val Ala Glu Ser Pro Lys
       450                 455                 460
   aaa cct taa                                                        1401
   Lys Pro  *
   465
<210> 107
   <211> 466
   <212> PRT
   <213> Artificial Sequence
<220>
   <223> Chimeric Protien
<400> 107
   Met Lys Lys Tyr Leu Leu Gly Ile Gly Leu Ile Leu Ala Leu Ile Ala
   1               5                   10                  15
   Cys Lys Gln Asn Val Ser Ser Leu Asp Glu Lys Asn Ser Val Ser Val
               20                  25                  30
   Asp Leu Pro Gly Glu Met Lys Val Leu Val Ser Lys Glu Lys Asn Lys
           35                  40                  45
   Asp Gly Lys Tyr Asp Leu Ile Ala Thr Val Asp Lys Leu Glu Leu Lys
       50                  55                  60
   Gly Thr Ser Asp Lys Asn Asn Gly Ser Gly Val Leu Glu Gly Val Lys
   65                  70                  75                  80
   Ala Asp Lys Ser Lys Val Lys Leu Thr Ile Ser Asp Asp Leu Gly Gln
                   85                  90                  95
   Thr Thr Leu Glu Val Phe Lys Glu Asp Gly Lys Thr Leu Val Ser Lys
               100                 105                 110
   Lys Val Thr Ser Lys Asp Lys Ser Ser Thr Glu Glu Lys Phe Asn Glu
           115                 120                 125
   Lys Gly Glu Val Ser Glu Lys Ile Ile Thr Arg Ala Asp Gly Thr Arg
       130                 135                 140
   Leu Glu Tyr Thr Gly Ile Lys Ser Asp Gly Ser Gly Lys Ala Lys Glu
   145                 150                 155                 160
   Val Leu Lys Gly Tyr Val Leu Glu Gly Thr Leu Thr Ala Glu Lys Thr
                   165                 170                 175
   Thr Leu Val Val Lys Glu Gly Thr Val Thr Leu Ser Lys Asn Ile Ser
               180                 185                 190
   Lys Ser Gly Glu Val Ser Val Glu Leu Asn Asp Thr Asp Ser Ser Ala
           195                 200                 205
   Ala Thr Lys Lys Thr Ala Ala Trp Asn Asp Ser Thr Ser Thr Leu Thr
       210                 215                 220
   Ile Ser Ala Asp Ser Lys Lys Thr Lys Asp Leu Val Phe Leu Thr Asp
   225                 230                 235                 240
   Gly Thr Ile Thr Val Gln Gln Tyr Asn Thr Ala Gly Thr Ser Leu Glu
                   245                 250                 255
   Gly Ser Ala Ser Glu Ile Lys Asn Leu Ser Glu Leu Lys Asn Ala Leu
               260                 265                 270
   Lys Met Ala Asn Asn Ser Gly Lys Asp Gly Asn Thr Ser Ala Asn Ser
           275                 280                 285
   Ala Asp Glu Ser Val Lys Gly Pro Asn Leu Thr Glu Ile Ser Lys Lys
       290                 295                 300
   Ile Thr Asp Ser Asn Ala Val Leu Leu Ala Val Lys Glu Val Glu Ala
   305                 310                 315                 320
   Leu Leu Ser Ser Ile Asp Glu Ile Ala Ala Lys Ala Ile Gly Lys Lys
                   325                 330                 335
   Ile His Gln Asn Asn Gly Leu Asp Thr Glu Tyr Asn His Asn Gly Ser
               340                 345                 350
   Leu Leu Ala Gly Arg Tyr Ala Ile Ser Thr Leu Ile Lys Gln Lys Leu
           355                 360                 365
   Asp Gly Leu Lys Asn Glu Gly Leu Lys Glu Lys Ile Asp Ala Ala Lys
       370                 375                 380
   Lys Cys Ser Glu Thr Phe Thr Asn Lys Leu Lys Ala Lys His Thr Asp
   385                 390                 395                 400
   Leu Gly Lys Glu Gly Val Thr Asp Ala Asp Ala Lys Glu Ala Ile Leu
                   405                 410                 415
   Lys Thr Asn Gly Thr Lys Thr Lys Gly Ala Glu Glu Leu Gly Lys Leu
               420                 425                 430
   Phe Glu Ser Val Glu Val Leu Ser Lys Ala Ala Lys Glu Met Leu Ala
           435                 440                 445
   Asn Ser Val Lys Glu Leu Thr Ser Pro Val Val Ala Glu Ser Pro Lys
       450                 455                 460
   Lys Pro
   465
<210> 108
   <211> 36
   <212> DNA
   <213> Artificial Sequence
<220>
   <223> Oligonucleotide Primer
<400> 108
   gctgctaaca ttttgcttag gtttttttgg actttc 36
<210> 109
   <211> 1401
   <212> DNA
   <213> Artificial Sequence
<220>
   <221> CDS
   <222> (1)... (1401)
   <223> Chimeric Nucleic Acid
<400> 109
   atg aaa aag aat aca tta agt gcg ata tta atg act tta ttt tta ttt    48
   Met Lys Lys Asn Thr Leu Ser Ala Ile Leu Met Thr Leu Phe Leu Phe
    1               5                  10                  15
   ata tct tgt aat aat tca ggg aaa gat ggg aat aca tct gca aat tct    96
   Ile Ser Cys Asn Asn Ser Gly Lys Asp Gly Asn Thr Ser Ala Asn Ser
               20                  25                  30
   gct gat gag tct gtt aaa ggg cct aat ctt aca gaa ata agt aaa aaa    144
   Ala Asp Glu Ser Val Lys Gly Pro Asn Leu Thr Glu Ile Ser Lys Lys
           35                  40                  45
   att acg gat tct aat gcg gtt tta ctt gct gtg aaa gag gtt gaa gcg    192
   Ile Thr Asp Ser Asn Ala Val Leu Leu Ala Val Lys Glu Val Glu Ala
       50                  55                  60
   ttg ctg tca tct ata gat gaa att gct gct aaa gct att ggt aaa aaa    240
   Leu Leu Ser Ser Ile Asp Glu Ile Ala Ala Lys Ala Ile Gly Lys Lys
   65                  70                  75                  80
   ata cac caa aat aat ggt ttg gat acc gaa tat aat cac aat gga tca    288
   Ile His Gln Asn Asn Gly Leu Asp Thr Glu Tyr Asn His Asn Gly Ser
                   85                  90                  95
   ttg tta gcg gga cgt tat gca ata tca acc cta ata aaa caa aaa tta    336
   Leu Leu Ala Gly Arg Tyr Ala Ile Ser Thr Leu Ile Lys Gln Lys Leu
               100                 105                 110
   gat gga ttg aaa aat gaa gga tta aag gaa aaa att gat gcg gct aag    384
   Asp Gly Leu Lys Asn Glu Gly Leu Lys Glu Lys Ile Asp Ala Ala Lys
           115                 120                 125
   aaa tgt tct gaa aca ttt act aat aaa tta aaa gca aaa cac aca gat    432
   Lys Cys Ser Glu Thr Phe Thr Asn Lys Leu Lys Ala Lys His Thr Asp
       130                 135                 140
   ctt ggt aaa gaa ggt gtt act gat gct gat gca aaa gaa gcc att tta    480
   Leu Gly Lys Glu Gly Val Thr Asp Ala Asp Ala Lys Glu Ala Ile Leu
   145                 150                 155                 160
   aaa aca aat ggt act aaa act aaa ggt gct gaa gaa ctt gga aaa tta    528
   Lys Thr Asn Gly Thr Lys Thr Lys Gly Ala Glu Glu Leu Gly Lys Leu
                   165                 170                 175
   ttt gaa tca gta gag gtc ttg tca aaa gca gct aaa gag atg ctt gct    576
   Phe Glu Ser Val Glu Val Leu Ser Lys Ala Ala Lys Glu Met Leu Ala
               180                 185                 190
   aat tca gtt aaa gag ctt aca agc cct gtt gtg gca gaa agt cca aaa    624
   Asn Ser Val Lys Glu Leu Thr Ser Pro Val Val Ala Glu Ser Pro Lys
           195                 200                 205
   aaa cct aag caa aat gtt agc agc ctt gac gag aaa aac agc gtt tca    672
   Lys Pro Lys Gln Asn Val Ser Ser Leu Asp Glu Lys Asn Ser Val Ser
       210                 215                 220
   gta gat ttg cct ggt gaa atg aaa gtt ctt gta agc aaa gaa aaa aac    720
   Val Asp Leu Pro Gly Glu Met Lys Val Leu Val Ser Lys Glu Lys Asn
   225                 230                 235                 240
   aaa gac ggc aag tac gat cta att gca aca gta gac aag ctt gag ctt    768
   Lys Asp Gly Lys Tyr Asp Leu Ile Ala Thr Val Asp Lys Leu Glu Leu
                   245                 250                 255
   aaa gga act tct gat aaa aac aat gga tct gga gta ctt gaa ggc gta    816
   Lys Gly Thr Ser Asp Lys Asn Asn Gly Ser Gly Val Leu Glu Gly Val
               260                 265                 270
   aaa gct gac aaa agt aaa gta aaa tta aca att tct gac gat cta ggt    864
   Lys Ala Asp Lys Ser Lys Val Lys Leu Thr Ile Ser Asp Asp Leu Gly
           275                 280                 285
   caa acc aca ctt gaa gtt ttc aaa gaa gat ggc aaa aca cta gta tca    912
   Gln Thr Thr Leu Glu Val Phe Lys Glu Asp Gly Lys Thr Leu Val Ser
       290                 295                 300
   aaa aaa gta act tcc aaa gac aag tca tca aca gaa gaa aaa ttc aat    960
   Lys Lys Val Thr Ser Lys Asp Lys Ser Ser Thr Glu Glu Lys Phe Asn
   305                 310                 315                 320
   gaa aaa ggt gaa gta tct gaa aaa ata ata aca aga gca gac gga acc    1008
   Glu Lys Gly Glu Val Ser Glu Lys Ile Ile Thr Arg Ala Asp Gly Thr
                   325                 330                 335
   aga ctt gaa tac aca gga att aaa agc gat gga tct gga aaa gct aaa    1056
   Arg Leu Glu Tyr Thr Gly Ile Lys Ser Asp Gly Ser Gly Lys Ala Lys
               340                 345                 350
   gag gtt tta aaa ggc tat gtt ctt gaa gga act cta act gct gaa aaa    1104
   Glu Val Leu Lys Gly Tyr Val Leu Glu Gly Thr Leu Thr Ala Glu Lys
           355                 360                 365
   aca aca ttg gtg gtt aaa gaa gga act gtt act tta agc aaa aat att    1152
   Thr Thr Leu Val Val Lys Glu Gly Thr Val Thr Leu Ser Lys Asn Ile
       370                 375                 380
   tca aaa tct ggg gaa gtt tca gtt gaa ctt aat gac act gac agt agt    1200
   Ser Lys Ser Gly Glu Val Ser Val Glu Leu Asn Asp Thr Asp Ser Ser
   385                 390                 395                 400
   gct gct act aaa aaa act gca gct tgg aat gac agt act agc act tta    1248
   Ala Ala Thr Lys Lys Thr Ala Ala Trp Asn Asp Ser Thr Ser Thr Leu
                   405                     410                     415
   aca att agt gct gac agc aaa aaa act aaa gat ttg gtg ttc tta aca    1296
   Thr Ile Ser Ala Asp Ser Lys Lys Thr Lys Asp Leu Val Phe Leu Thr
               420                     425                     430
   gat ggt aca att aca gta caa caa tac aac aca gct gga acc agc cta    1344
   Asp Gly Thr Ile Thr Val Gln Gln Tyr Asn Thr Ala Gly Thr Ser Leu
           435                     440                     445
   gaa gga tca gca agt gaa att aaa aat ctt tca gag ctt aaa aac gct    1392
   Glu Gly Ser Ala Ser Glu Ile Lys Asn Leu Ser Glu Leu Lys Asn Ala
       450                     455                     460
   tta aaa taa                                                         1401
   Leu Lys  *
   465
<210> 110
   <211> 466
   <212> PRT
   <213> Artificial Sequence
<220>
   <223> Chimeric Protein
<400> 110
   Met Lys Lys Asn Thr Leu Ser Ala Ile Leu Met Thr Leu Phe Leu Phe
    1               5                  10                  15
   Ile Ser Cys Asn Asn Ser Gly Lys Asp Gly Asn Thr Ser Ala Asn Ser
               20                  25                  30
   Ala Asp Glu Ser Val Lys Gly Pro Asn Leu Thr Glu Ile Ser Lys Lys
           35                  40                  45
   Ile Thr Asp Ser Asn Ala Val Leu Leu Ala Val Lys Glu Val Glu Ala
       50                  55                  60
   Leu Leu Ser Ser Ile Asp Glu Ile Ala Ala Lys Ala Ile Gly Lys Lys
   65                  70                  75                  80
   Ile His Gln Asn Asn Gly Leu Asp Thr Glu Tyr Asn His Asn Gly Ser
                   85                  90                  95
   Leu Leu Ala Gly Arg Tyr Ala Ile Ser Thr Leu Ile Lys Gln Lys Leu
               100                 105                 110
   Asp Gly Leu Lys Asn Glu Gly Leu Lys Glu Lys Ile Asp Ala Ala Lys
           115                 120                 125
   Lys Cys Ser Glu Thr Phe Thr Asn Lys Leu Lys Ala Lys His Thr Asp
       130                 135                 140
   Leu Gly Lys Glu Gly Val Thr Asp Ala Asp Ala Lys Glu Ala Ile Leu
   145                 150                 155                 160
   Lys Thr Asn Gly Thr Lys Thr Lys Gly Ala Glu Glu Leu Gly Lys Leu
                   165                 170                 175
   Phe Glu Ser Val Glu Val Leu Ser Lys Ala Ala Lys Glu Met Leu Ala
               180                 185                 190
   Asn Ser Val Lys Glu Leu Thr Ser Pro Val Val Ala Glu Ser Pro Lys
           195                 200                 205
   Lys Pro Lys Gln Asn Val Ser Ser Leu Asp Glu Lys Asn Ser Val Ser
       210                 215                 220
   Val Asp Leu Pro Gly Glu Met Lys Val Leu Val Ser Lys Glu Lys Asn
   225                 230                 235                 240
   Lys Asp Gly Lys Tyr Asp Leu Ile Ala Thr Val Asp Lys Leu Glu Leu
                   245                 250                 255
   Lys Gly Thr Ser Asp Lys Asn Asn Gly Ser Gly Val Leu Glu Gly Val
               260                 265                 270
   Lys Ala Asp Lys Ser Lys Val Lys Leu Thr Ile Ser Asp Asp Leu Gly
           275                 280                 285
   Gln Thr Thr Leu Glu Val Phe Lys Glu Asp Gly Lys Thr Leu Val Ser
       290                 295                 300
   Lys Lys Val Thr Ser Lys Asp Lys Ser Ser Thr Glu Glu Lys Phe Asn
   305                 310                 315                 320
   Glu Lys Gly Glu Val Ser Glu Lys Ile Ile Thr Arg Ala Asp Gly Thr
                   325                 330                 335
   Arg Leu Glu Tyr Thr Gly Ile Lys Ser Asp Gly Ser Gly Lys Ala Lys
               340                 345                 350
   Glu Val Leu Lys Gly Tyr Val Leu Glu Gly Thr Leu Thr Ala Glu Lys
           355                 360                 365
   Thr Thr Leu Val Val Lys Glu Gly Thr Val Thr Leu Ser Lys Asn Ile
       370                 375                 380
   Ser Lys Ser Gly Glu Val Ser Val Glu Leu Asn Asp Thr Asp Ser Ser
   385                 390                 395                 400
   Ala Ala Thr Lys Lys Thr Ala Ala Trp Asn Asp Ser Thr Ser Thr Leu
                   405                 410                 415
   Thr Ile Ser Ala Asp Ser Lys Lys Thr Lys Asp Leu Val Phe Leu Thr
               420                 425                 430
   Asp Gly Thr Ile Thr Val Gln Gln Tyr Asn Thr Ala Gly Thr Ser Leu
           435                 440                 445
   Glu Gly Ser Ala Ser Glu Ile Lys Asn Leu Ser Glu Leu Lys Asn Ala
       450                 455                 460
   Leu Lys
   465
<210> 111
   <211> 1720
   <212> DNA
   <213> Artificial Sequence
<220>
   <221> CDS
   <222> (1)...(1719)
   <223> Chimeric Nucleic Acid
<400> 111
   aag caa aat gtt agc agc ctt gac gag aaa aac agc gtt tca gta gat      48
   Lys Gln Asn Val Ser Ser Leu Asp Glu Lys Asn Ser Val Ser Val Asp
    1               5                  10                  15
   ttg cct ggt gaa atg aaa gtt ctt gta agc aaa gaa aaa aac aaa gac      96
   Leu Pro Gly Glu Met Lys Val Leu Val Ser Lys Glu Lys Asn Lys Asp
               20                  25                  30
   ggc aag tac gat cta att gca aca gta gac aag ctt gag ctt aaa gga     144
   Gly Lys Tyr Asp Leu Ile Ala Thr Val Asp Lys Leu Glu Leu Lys Gly
           35                  40                  45
   act tct gat aaa aac aat gga tct gga gta ctt gaa ggc gta aaa gct     192
   Thr Ser Asp Lys Asn Asn Gly Ser Gly Val Leu Glu Gly Val Lys Ala
       50                  55                  60
   gac aaa agt aaa gta aaa tta aca att tct gac gat cta ggt caa acc     240
   Asp Lys Ser Lys Val Lys Leu Thr Ile Ser Asp Asp Leu Gly Gln Thr
   65                  70                  75                  80
   aca ctt gaa gtt ttc aaa gaa gat ggc aaa aca cta gta tca aaa aaa     288
   Thr Leu Glu Val Phe Lys Glu Asp Gly Lys Thr Leu Val Ser Lys Lys
                   85                  90                  95
   gta act tcc aaa gac aag tca tca aca gaa gaa aaa ttc aat gaa aaa     336
   Val Thr Ser Lys Asp Lys Ser Ser Thr Glu Glu Lys Phe Asn Glu Lys
               100                 105                 110
   ggt gaa gta tct gaa aaa ata ata aca aga gca gac gga acc aga ctt     384
   Gly Glu Val Ser Glu Lys Ile Ile Thr Arg Ala Asp Gly Thr Arg Leu
           115                 120                 125
   gaa tac aca gga att aaa agc gat gga tct gga aaa gct aaa gag gtt     432
   Glu Tyr Thr Gly Ile Lys Ser Asp Gly Ser Gly Lys Ala Lys Glu Val
       130                 135                 140
   tta aaa ggc tat gtt ctt gaa gga act cta act gct gaa aaa aca aca     480
   Leu Lys Gly Tyr Val Leu Glu Gly Thr Leu Thr Ala Glu Lys Thr Thr
   145                 150                 155                 160
   ttg gtg gtt aaa gaa gga act gtt act tta agc aaa aat att tca aaa     528
   Leu Val Val Lys Glu Gly Thr Val Thr Leu Ser Lys Asn Ile Ser Lys
                   165                 170                 175
   tct ggg gaa gtt tca gtt gaa ctt aat gac act gac agt agt gct gct     576
   Ser Gly Glu Val Ser Val Glu Leu Asn Asp Thr Asp Ser Ser Ala Ala
               180                 185                 190
   act aaa aaa act gca gct tgg aat tca ggc act tca act tta aca att     624
   Thr Lys Lys Thr Ala Ala Trp Asn Ser Gly Thr Ser Thr Leu Thr Ile
           195                 200                 205
   act gta aac agt aaa aaa act aaa gac ctt gtg ttt aca aaa gaa aac     672
   Thr Val Asn Ser Lys Lys Thr Lys Asp Leu Val Phe Thr Lys Glu Asn
       210                 215                 220
   aca att aca gta caa caa tac gac tca aat ggc acc aaa tta gag ggg     720
   Thr Ile Thr Val Gln Gln Tyr Asp Ser Asn Gly Thr Lys Leu Glu Gly
   225                 230                 235                 240
   tca gca gtt gaa att aca aaa ctt gat gaa att aaa aac gct tta aaa     768
   Ser Ala Val Glu Ile Thr Lys Leu Asp Glu Ile Lys Asn Ala Leu Lys
                   245                 250                 255
   ggt cac ccc atg gat gaa aag ctt tta aaa agt aaa gat gat aaa gca     816
   Gly His Pro Met Asp Glu Lys Leu Leu Lys Ser Lys Asp Asp Lys Ala
               260                 265                 270
   agt aaa gat ggt aaa gcc ttg gat ctt gat cga gaa tta aat tct aaa     864
   Ser Lys Asp Gly Lys Ala Leu Asp Leu Asp Arg Glu Leu Asn Ser Lys
           275                 280                 285
   gct tct agc aaa gaa aaa agt aaa gcc aag gaa gaa gaa ata acc aag     912
   Ala Ser Ser Lys Glu Lys Ser Lys Ala Lys Glu Glu Glu Ile Thr Lys
       290                 295                 300
   ggt aag tca cag aaa agc tta ggc gat ttg aat aat gat gaa aat ctt    960
   Gly Lys Ser Gln Lys Ser Leu Gly Asp Leu Asn Asn Asp Glu Asn Leu
   305                 310                 315                 320
   atg atg cca gaa gat caa aaa tta cct gag gtt aaa aaa tta gat agc   1008
   Met Met Pro Glu Asp Gln Lys Leu Pro Glu Val Lys Lys Leu Asp Ser
                   325                 330                 335
   aaa aaa gaa ttt aaa cct gtt tct gag gtt gag aaa tta gat aag att   1056
   Lys Lys Glu Phe Lys Pro Val Ser Glu Val Glu Lys Leu Asp Lys Ile
               340                 345                 350
   ttc aag tct aat aac aat gtt gga gaa tta tca ccg tta gat aaa tct   1104
   Phe Lys Ser Asn Asn Asn Val Gly Glu Leu Ser Pro Leu Asp Lys Ser
           355                 360                 365
   tct tat aaa gac att gat tca aaa gag gag aca gtt aat aaa gat gtt   1152
   Ser Tyr Lys Asp Ile Asp Ser Lys Glu Glu Thr Val Asn Lys Asp Val
       370                 375                 380
   aat ttg caa aag act aag cct cag gtt aaa gac caa gtt act tct ttg   1200
   Asn Leu Gln Lys Thr Lys Pro Gln Val Lys Asp Gln Val Thr Ser Leu
   385                 390                 395                 400
   aat gaa gat ttg act act atg tct ata gat tcc agt agt cct gta ttt   1248
   Asn Glu Asp Leu Thr Thr Met Ser Ile Asp Ser Ser Ser Pro Val Phe
                   405                 410                 415
   tta gag gtt att gat cca att aca aat tta gga act ctt caa ctt att   1296
   Leu Glu Val Ile Asp Pro Ile Thr Asn Leu Gly Thr Leu Gln Leu Ile
               420                 425                 430
   gat tta aat act ggt gtt agg ctt aaa gaa agc act cag caa ggc att   1344
   Asp Leu Asn Thr Gly Val Arg Leu Lys Glu Ser Thr Gln Gln Gly Ile
           435                 440                 445
   cag cgg tat gga att tat gaa cgt gaa aaa gat ttg gtt gtt att aaa   1392
   Gln Arg Tyr Gly Ile Tyr Glu Arg Glu Lys Asp Leu Val Val Ile Lys
       450                 455                 460
   atg gat tca gga aaa gct aag ctt cag ata ctt gat aaa ctt gaa aat   1440
   Met Asp Ser Gly Lys Ala Lys Leu Gln Ile Leu Asp Lys Leu Glu Asn
   465                 470                 475                 480
   tta aaa gtg gta tca gag tct aat ttt gag att aat aaa aat tca tct   1488
   Leu Lys Val Val Ser Glu Ser Asn Phe Glu Ile Asn Lys Asn Ser Ser
                   485                 490                 495
   ctt tat gtt gat tct aaa atg att tta gta gct gtt agg gat aaa gat   1536
   Leu Tyr Val Asp Ser Lys Met Ile Leu Val Ala Val Arg Asp Lys Asp
               500                 505                 510
   agt agt aat gat tgg aga ttg gcc aaa ttt tct cct aaa aat tta gat   1584
   Ser Ser Asn Asp Trp Arg Leu Ala Lys Phe Ser Pro Lys Asn Leu Asp
           515                 520                 525
   gag ttt att ctt tca gag aat aaa att atg cct ttt act agc ttt tct   1632
   Glu Phe Ile Leu Ser Glu Asn Lys Ile Met Pro Phe Thr Ser Phe Ser
       530                 535                 540
   gtg aga aaa aat ttt att tat ttg caa gat gag ttt aaa agt cta gtt    1680
   Val Arg Lys Asn Phe Ile Tyr Leu Gln Asp Glu Phe Lys Ser Leu Val
   545                 550                 555                 560
   att tta gat gta aat act tta aaa aaa gtt aag ggt cac c              1720
   Ile Leu Asp Val Asn Thr Leu Lys Lys Val Lys Gly His
                   565                 570
<210> 112
   <211> 573
   <212> PRT
   <213> Artificial Sequence
<220>
   <223> Chimeric Protein
<400> 112
   Lys Gln Asn Val Ser Ser Leu Asp Glu Lys Asn Ser Val Ser Val Asp
   1               5                   10                  15
   Leu Pro Gly Glu Met Lys Val Leu Val Ser Lys Glu Lys Asn Lys Asp
               20                  25                  30
   Gly Lys Tyr Asp Leu Ile Ala Thr Val Asp Lys Leu Glu Leu Lys Gly
           35                  40                  45
   Thr Ser Asp Lys Asn Asn Gly Ser Gly Val Leu Glu Gly Val Lys Ala
       50                  55                  60
   Asp Lys Ser Lys Val Lys Leu Thr Ile Ser Asp Asp Leu Gly Gln Thr
   65                  70                  75                  80
   Thr Leu Glu Val Phe Lys Glu Asp Gly Lys Thr Leu Val Ser Lys Lys
                   85                  90                  95
   Val Thr Ser Lys Asp Lys Ser Ser Thr Glu Glu Lys Phe Asn Glu Lys
               100                 105                 110
   Gly Glu Val Ser Glu Lys Ile Ile Thr Arg Ala Asp Gly Thr Arg Leu
           115                 120                 125
   Glu Tyr Thr Gly Ile Lys Ser Asp Gly Ser Gly Lys Ala Lys Glu Val
       130                 135                 140
   Leu Lys Gly Tyr Val Leu Glu Gly Thr Leu Thr Ala Glu Lys Thr Thr
   145                 150                 155                 160
   Leu Val Val Lys Glu Gly Thr Val Thr Leu Ser Lys Asn Ile Ser Lys
                   165                 170                 175
   Ser Gly Glu Val Ser Val Glu Leu Asn Asp Thr Asp Ser Ser Ala Ala
               180                 185                 190
   Thr Lys Lys Thr Ala Ala Trp Asn Ser Gly Thr Ser Thr Leu Thr Ile
           195                 200                 205
   Thr Val Asn Ser Lys Lys Thr Lys Asp Leu Val Phe Thr Lys Glu Asn
       210                 215                 220
   Thr Ile Thr Val Gln Gln Tyr Asp Ser Asn Gly Thr Lys Leu Glu Gly
   225                 230                 235                 240
   Ser Ala Val Glu Ile Thr Lys Leu Asp Glu Ile Lys Asn Ala Leu Lys
                   245                 250                 255
   Gly His Pro Met Asp Glu Lys Leu Leu Lys Ser Lys Asp Asp Lys Ala
               260                 265                 270
   Ser Lys Asp Gly Lys Ala Leu Asp Leu Asp Arg Glu Leu Asn Ser Lys
           275                 280                 285
   Ala Ser Ser Lys Glu Lys Ser Lys Ala Lys Glu Glu Glu Ile Thr Lys
       290                 295                 300
   Gly Lys Ser Gln Lys Ser Leu Gly Asp Leu Asn Asn Asp Glu Asn Leu
   305                 310                 315                 320
   Met Met Pro Glu Asp Gln Lys Leu Pro Glu Val Lys Lys Leu Asp Ser
                   325                 330                 335
   Lys Lys Glu Phe Lys Pro Val Ser Glu Val Glu Lys Leu Asp Lys Ile
               340                 345                 350
   Phe Lys Ser Asn Asn Asn Val Gly Glu Leu Ser Pro Leu Asp Lys Ser
           355                 360                 365
   Ser Tyr Lys Asp Ile Asp Ser Lys Glu Glu Thr Val Asn Lys Asp Val
       370                 375                 380
   Asn Leu Gln Lys Thr Lys Pro Gln Val Lys Asp Gln Val Thr Ser Leu
   385                 390                 395                 400
   Asn Glu Asp Leu Thr Thr Met Ser Ile Asp Ser Ser Ser Pro Val Phe
                   405                 410                 415
   Leu Glu Val Ile Asp Pro Ile Thr Asn Leu Gly Thr Leu Gln Leu Ile
               420                 425                 430
   Asp Leu Asn Thr Gly Val Arg Leu Lys Glu Ser Thr Gln Gln Gly Ile
           435                 440                 445
   Gln Arg Tyr Gly Ile Tyr Glu Arg Glu Lys Asp Leu Val Val Ile Lys
       450                 455                 460
   Met Asp Ser Gly Lys Ala Lys Leu Gln Ile Leu Asp Lys Leu Glu Asn
   465                 470                 475                 480
   Leu Lys Val Val Ser Glu Ser Asn Phe Glu Ile Asn Lys Asn Ser Ser
                   485                 490                 495
   Leu Tyr Val Asp Ser Lys Met Ile Leu Val Ala Val Arg Asp Lys Asp
               500                 505                 510
   Ser Ser Asn Asp Trp Arg Leu Ala Lys Phe Ser Pro Lys Asn Leu Asp
           515                 520                 525
   Glu Phe Ile Leu Ser Glu Asn Lys Ile Met Pro Phe Thr Ser Phe Ser
       530                 535                 540
   Val Arg Lys Asn Phe Ile Tyr Leu Gln Asp Glu Phe Lys Ser Leu Val
   545                 550                 555                 560
   Ile Leu Asp Val Asn Thr Leu Lys Lys Val Lys Gly His
                   565                 570
<210> 113
   <211> 1180
   <212> DNA
   <213> Artificial Sequence
<220>
   <221> CDS
   <222> (1) ... (1179)
   <223> Chimeric Nucleic Acid
<400> 113
   gca caa aaa ggt gct gag tca att ggt tct caa aaa gaa aat gat cta      48
   Ala Gln Lys Gly Ala Glu Ser Ile Gly Ser Gln Lys Glu Asn Asp Leu
    1               5                  10                  15
   aac ctt gaa gac tct agt aaa aaa tca cat caa aac gct aaa caa gac      96
   Asn Leu Glu Asp Ser Ser Lys Lys Ser His Gln Asn Ala Lys Gln Asp
               20                  25                  30
   ctt cct gcg gtg aca gaa gac tca gtg tct ttg ttt aat ggt aat aaa     144
   Leu Pro Ala Val Thr Glu Asp Ser Val Ser Leu Phe Asn Gly Asn Lys
           35                  40                  45
   att ttt gta agc aaa gaa aaa aat agc tcc ggc aaa tat gat tta aga     192
   Ile Phe Val Ser Lys Glu Lys Asn Ser Ser Gly Lys Tyr Asp Leu Arg
       50                  55                  60
   gca aca att gat cag gtt gaa ctt aaa gga act tcc gat aaa aac aat     240
   Ala Thr Ile Asp Gln Val Glu Leu Lys Gly Thr Ser Asp Lys Asn Asn
   65                  70                  75                  80
   ggt tct gga acc ctt gaa ggt tca aag cct gac aag agt aaa gta aaa     288
   Gly Ser Gly Thr Leu Glu Gly Ser Lys Pro Asp Lys Ser Lys Val Lys
                   85                  90                  95
   tta aca gtt tct gct gat tta aac aca gta acc tta gaa gca ttt gat     336
   Leu Thr Val Ser Ala Asp Leu Asn Thr Val Thr Leu Glu Ala Phe Asp
               100                 105                 110
   gcc agc aac caa aaa att tca agt aaa gtt act aaa aaa cag ggg tca     384
   Ala Ser Asn Gln Lys Ile Ser Ser Lys Val Thr Lys Lys Gln Gly Ser
           115                 120                 125
   ata aca gag gaa act ctc aaa gct aat aaa tta gac tca aag aaa tta     432
   Ile Thr Glu Glu Thr Leu Lys Ala Asn Lys Leu Asp Ser Lys Lys Leu
       130                 135                 140
   aca aga tca aac gga act aca ctt gaa tac tca caa ata aca gat gct     480
   Thr Arg Ser Asn Gly Thr Thr Leu Glu Tyr Ser Gln Ile Thr Asp Ala
   145                 150                 155                 160
   gac aat gct aca aaa gca gta gaa act cta aaa aat agc att aag ctt     528
   Asp Asn Ala Thr Lys Ala Val Glu Thr Leu Lys Asn Ser Ile Lys Leu
                   165                 170                 175
   gaa gga agt ctt gta gtc gga aaa aca aca gtg gaa att aaa gaa ggt     576
   Glu Gly Ser Leu Val Val Gly Lys Thr Thr Val Glu Ile Lys Glu Gly
               180                 185                 190
   act gtt act cta aaa aga gaa att gaa aaa gat gga aaa gta aaa gtc     624
   Thr Val Thr Leu Lys Arg Glu Ile Glu Lys Asp Gly Lys Val Lys Val
           195                 200                 205
   ttt ttg aat gac act gca ggt tct aac aaa aaa aca ggt aaa tgg gaa     672
   Phe Leu Asn Asp Thr Ala Gly Ser Asn Lys Lys Thr Gly Lys Trp Glu
       210                 215                 220
   gac agt act agc act tta aca att agt gct gac agc aaa aaa act aaa     720
   Asp Ser Thr Ser Thr Leu Thr Ile Ser Ala Asp Ser Lys Lys Thr Lys
   225                 230                 235                 240
   gat ttg gtg ttc tta aca gat ggt aca att aca gta caa caa tac aac     768
   Asp Leu Val Phe Leu Thr Asp Gly Thr Ile Thr Val Gln Gln Tyr Asn
                   245                 250                 255
   aca gct gga acc agc cta gaa gga tca gca agt gaa att aaa aat ctt     816
   Thr Ala Gly Thr Ser Leu Glu Gly Ser Ala Ser Glu Ile Lys Asn Leu
               260                 265                 270
   tca gag ctt aaa aac gct tta aaa ggt cac ccc atg gct caa tat aac     864
   Ser Glu Leu Lys Asn Ala Leu Lys Gly His Pro Met Ala Gln Tyr Asn
           275                 280                 285
   caa atg cac atg tta tca aac aaa tct gct tct caa aat gta aga aca     912
   Gln Met His Met Leu Ser Asn Lys Ser Ala Ser Gln Asn Val Arg Thr
       290                 295                 300
   gct gaa gag ctt gga atg cag cct gca aaa att aac aca cca gca tca     960
   Ala Glu Glu Leu Gly Met Gln Pro Ala Lys Ile Asn Thr Pro Ala Ser
   305                 310                 315                 320
   ctt tca ggg ctt caa gcg tct tgg act tta aga gtt cat gtt gga gca     1008
   Leu Ser Gly Leu Gln Ala Ser Trp Thr Leu Arg Val His Val Gly Ala
                   325                 330                 335
   acc caa gat gaa gct att gct gta aat att tat gca gct aat gtt gca     1056
   Thr Gln Asp Glu Ala Ile Ala Val Asn Ile Tyr Ala Ala Asn Val Ala
               340                 345                 350
   aat ctt ttc tct ggt gag gga gct caa act gct cag gct gca ccg gtt     1104
   Asn Leu Phe Ser Gly Glu Gly Ala Gln Thr Ala Gln Ala Ala Pro Val
           355                 360                 365
   caa gag ggt gtt caa cag gaa gga gct caa cag cca gca cct gct aca     1152
   Gln Glu Gly Val Gln Gln Glu Gly Ala Gln Gln Pro Ala Pro Ala Thr
       370                 375                 380
   gca cct tct caa ggc gga gtt ggt cac c                               1180
   Ala Pro Ser Gln Gly Gly Val Gly His
   385                 390
<210> 114
   <211> 393
   <212> PRT
   <213> Artificial Sequence
<220>
   <223> Chimeric Protein
<400> 114
   Ala Gln Lys Gly Ala Glu Ser Ile Gly Ser Gln Lys Glu Asn Asp Leu
   1               5                   10                  15
   Asn Leu Glu Asp Ser Ser Lys Lys Ser His Gln Asn Ala Lys Gln Asp
               20                  25                  30
   Leu Pro Ala Val Thr Glu Asp Ser Val Ser Leu Phe Asn Gly Asn Lys
           35                  40                  45
   Ile Phe Val Ser Lys Glu Lys Asn Ser Ser Gly Lys Tyr Asp Leu Arg
       50                  55                  60
   Ala Thr Ile Asp Gln Val Glu Leu Lys Gly Thr Ser Asp Lys Asn Asn
   65                  70                  75                  80
   Gly Ser Gly Thr Leu Glu Gly Ser Lys Pro Asp Lys Ser Lys Val Lys
                   85                  90                  95
   Leu Thr Val Ser Ala Asp Leu Asn Thr Val Thr Leu Glu Ala Phe Asp
               100                 105                 110
   Ala Ser Asn Gln Lys Ile Ser Ser Lys Val Thr Lys Lys Gln Gly Ser
           115                 120                 125
   Ile Thr Glu Glu Thr Leu Lys Ala Asn Lys Leu Asp Ser Lys Lys Leu
       130                 135                 140
   Thr Arg Ser Asn Gly Thr Thr Leu Glu Tyr Ser Gln Ile Thr Asp Ala
   145                 150                 155                 160
   Asp Asn Ala Thr Lys Ala Val Glu Thr Leu Lys Asn Ser Ile Lys Leu
                   165                 170                 175
   Glu Gly Ser Leu Val Val Gly Lys Thr Thr Val Glu Ile Lys Glu Gly
               180                 185                 190
   Thr Val Thr Leu Lys Arg Glu Ile Glu Lys Asp Gly Lys Val Lys Val
           195                 200                 205
   Phe Leu Asn Asp Thr Ala Gly Ser Asn Lys Lys Thr Gly Lys Trp Glu
       210                 215                 220
   Asp Ser Thr Ser Thr Leu Thr Ile Ser Ala Asp Ser Lys Lys Thr Lys
   225                 230                 235                 240
   Asp Leu Val Phe Leu Thr Asp Gly Thr Ile Thr Val Gln Gln Tyr Asn
                   245                 250                 255
   Thr Ala Gly Thr Ser Leu Glu Gly Ser Ala Ser Glu Ile Lys Asn Leu
               260                 265                 270
   Ser Glu Leu Lys Asn Ala Leu Lys Gly His Pro Met Ala Gln Tyr Asn
           275                 280                 285
   Gln Met His Met Leu Ser Asn Lys Ser Ala Ser Gln Asn Val Arg Thr
       290                 295                 300
   Ala Glu Glu Leu Gly Met Gln Pro Ala Lys Ile Asn Thr Pro Ala Ser
   305                 310                 315                 320
   Leu Ser Gly Leu Gln Ala Ser Trp Thr Leu Arg Val His Val Gly Ala
                   325                 330                 335
   Thr Gln Asp Glu Ala Ile Ala Val Asn Ile Tyr Ala Ala Asn Val Ala
               340                 345                 350
   Asn Leu Phe Ser Gly Glu Gly Ala Gln Thr Ala Gln Ala Ala Pro Val
           355                 360                 365
   Gln Glu Gly Val Gln Gln Glu Gly Ala Gln Gln Pro Ala Pro Ala Thr
       370                 375                 380
   Ala Pro Ser Gln Gly Gly Val Gly His
   385                 390
<210> 115
   <211> 1363
   <212> DNA
   <213> Artificial Sequence
<220>
   <221> CDS
   <222> (1) ... (1362)
   <223> Chimeric nucleic acid
<400> 115
   gca caa aaa ggt gct gag tca att ggt tct caa aaa gaa aat gat cta      48
   Ala Gln Lys Gly Ala Glu Ser Ile Gly Ser Gln Lys Glu Asn Asp Leu
    1               5                  10                  15
   aac ctt gaa gac tct agt aaa aaa tca cat caa aac gct aaa caa gac      96
   Asn Leu Glu Asp Ser Ser Lys Lys Ser His Gln Asn Ala Lys Gln Asp
               20                  25                  30
   ctt cct gcg gtg aca gaa gac tca gtg tct ttg ttt aat ggt aat aaa     144
   Leu Pro Ala Val Thr Glu Asp Ser Val Ser Leu Phe Asn Gly Asn Lys
           35                  40                  45
   att ttt gta agc aaa gaa aaa aat agc tcc ggc aaa tat gat tta aga     192
   Ile Phe Val Ser Lys Glu Lys Asn Ser Ser Gly Lys Tyr Asp Leu Arg
       50                  55                  60
   gca aca att gat cag gtt gaa ctt aaa gga act tcc gat aaa aac aat     240
   Ala Thr Ile Asp Gln Val Glu Leu Lys Gly Thr Ser Asp Lys Asn Asn
   65                  70                  75                  80
   ggt tct gga acc ctt gaa ggt tca aag cct gac aag agt aaa gta aaa     288
   Gly Ser Gly Thr Leu Glu Gly Ser Lys Pro Asp Lys Ser Lys Val Lys
                   85                  90                  95
   tta aca gtt tct gct gat tta aac aca gta acc tta gaa gca ttt gat     336
   Leu Thr Val Ser Ala Asp Leu Asn Thr Val Thr Leu Glu Ala Phe Asp
               100                 105                 110
   gcc agc aac caa aaa att tca agt aaa gtt act aaa aaa cag ggg tca     384
   Ala Ser Asn Gln Lys Ile Ser Ser Lys Val Thr Lys Lys Gln Gly Ser
           115                 120                 125
   ata aca gag gaa act ctc aaa gct aat aaa tta gac tca aag aaa tta     432
   Ile Thr Glu Glu Thr Leu Lys Ala Asn Lys Leu Asp Ser Lys Lys Leu
       130                 135                 140
   aca aga tca aac gga act aca ctt gaa tac tca caa ata aca gat gct     480
   Thr Arg Ser Asn Gly Thr Thr Leu Glu Tyr Ser Gln Ile Thr Asp Ala
   145                 150                 155                 160
   gac aat gct aca aaa gca gta gaa act cta aaa aat agc att aag ctt     528
   Asp Asn Ala Thr Lys Ala Val Glu Thr Leu Lys Asn Ser Ile Lys Leu
                   165                 170                 175
   gaa gga agt ctt gta gtc gga aaa aca aca gtg gaa att aaa gaa ggt     576
   Glu Gly Ser Leu Val Val Gly Lys Thr Thr Val Glu Ile Lys Glu Gly
               180                 185                 190
   act gtt act cta aaa aga gaa att gaa aaa gat gga aaa gta aaa gtc     624
   Thr Val Thr Leu Lys Arg Glu Ile Glu Lys Asp Gly Lys Val Lys Val
           195                 200                 205
   ttt ttg aat gac act gca ggt tct aac aaa aaa aca ggt aaa tgg gaa     672
   Phe Leu Asn Asp Thr Ala Gly Ser Asn Lys Lys Thr Gly Lys Trp Glu
       210                 215                 220
   gac agt act agc act tta aca att agt gct gac agc aaa aaa act aaa     720
   Asp Ser Thr Ser Thr Leu Thr Ile Ser Ala Asp Ser Lys Lys Thr Lys
   225                 230                 235                 240
   gat ttg gtg ttc tta aca gat ggt aca att aca gta caa caa tac aac     768
   Asp Leu Val Phe Leu Thr Asp Gly Thr Ile Thr Val Gln Gln Tyr Asn
                   245                 250                 255
   aca gct gga acc agc cta gaa gga tca gca agt gaa att aaa aat ctt     816
   Thr Ala Gly Thr Ser Leu Glu Gly Ser Ala Ser Glu Ile Lys Asn Leu
               260                 265                 270
   tca gag ctt aaa aac gct tta aaa ggt cac ccc atg gct caa tat aac     864
   Ser Glu Leu Lys Asn Ala Leu Lys Gly His Pro Met Ala Gln Tyr Asn
           275                 280                 285
   caa atg cac atg tta tca aac aaa tct gct tct caa aat gta aga aca     912
   Gln Met His Met Leu Ser Asn Lys Ser Ala Ser Gln Asn Val Arg Thr
       290                 295                 300
   gct gaa gag ctt gga atg cag cct gca aaa att aac aca cca gca tca     960
   Ala Glu Glu Leu Gly Met Gln Pro Ala Lys Ile Asn Thr Pro Ala Ser
   305                 310                 315                 320
   ctt tca ggg ctt caa gcg tct tgg act tta aga gtt cat gtt gga gca    1008
   Leu Ser Gly Leu Gln Ala Ser Trp Thr Leu Arg Val His Val Gly Ala
                   325                 330                 335
   acc caa gat gaa gct att gct gta aat att tat gca gct aat gtt gca    1056
   Thr Gln Asp Glu Ala Ile Ala Val Asn Ile Tyr Ala Ala Asn Val Ala
               340                 345                 350
   aat ctt ttc tct ggt gag gga gct caa act gct cag gct gca ccg gtt      1104
   Asn Leu Phe Ser Gly Glu Gly Ala Gln Thr Ala Gln Ala Ala Pro Val
           355                 360                 365
   caa gag ggt gtt caa cag gaa gga gct caa cag cca gca cct gct aca      1152
   Gln Glu Gly Val Gln Gln Glu Gly Ala Gln Gln Pro Ala Pro Ala Thr
       370                 375                 380
   gca cct tct caa ggc gga gtt aat tct cct gtt aat gtt aca act aca      1200
   Ala Pro Ser Gln Gly Gly Val Asn Ser Pro Val Asn Val Thr Thr Thr
   385                 390                 395                 400
   gtt gat gct aat aca tca ctt gct aaa att gaa aat gct att aga atg      1248
   Val Asp Ala Asn Thr Ser Leu Ala Lys Ile Glu Asn Ala Ile Arg Met
                   405                 410                 415
   ata agt gat caa agg gca aat tta ggt gct ttc caa aat aga ctt gaa      1296
   Ile Ser Asp Gln Arg Ala Asn Leu Gly Ala Phe Gln Asn Arg Leu Glu
               420                 425                 430
   tct ata aag aat agt act gag tat gca att gaa aat cta aaa gca tct      1344
   Ser Ile Lys Asn Ser Thr Glu Tyr Ala Ile Glu Asn Leu Lys Ala Ser
           435                 440                 445
   tat gct caa ata ggt cac c                                            1363
   Tyr Ala Gln Ile Gly His
       450
<210> 116
   <211> 454
   <212> PRT
   <213> Artificial Sequence
<220>
   <223> Chimeric Protein
<400> 116
   Ala Gln Lys Gly Ala Glu Ser Ile Gly Ser Gln Lys Glu Asn Asp Leu
   1               5                   10                  15
   Asn Leu Glu Asp Ser Ser Lys Lys Ser His Gln Asn Ala Lys Gln Asp
               20                  25                  30
   Leu Pro Ala Val Thr Glu Asp Ser Val Ser Leu Phe Asn Gly Asn Lys
           35                  40                  45
   Ile Phe Val Ser Lys Glu Lys Asn Ser Ser Gly Lys Tyr Asp Leu Arg
       50                  55                  60
   Ala Thr Ile Asp Gln Val Glu Leu Lys Gly Thr Ser Asp Lys Asn Asn
   65                  70                  75                  80
   Gly Ser Gly Thr Leu Glu Gly Ser Lys Pro Asp Lys Ser Lys Val Lys
                   85                  90                  95
   Leu Thr Val Ser Ala Asp Leu Asn Thr Val Thr Leu Glu Ala Phe Asp
               100                 105                 110
   Ala Ser Asn Gln Lys Ile Ser Ser Lys Val Thr Lys Lys Gln Gly Ser
           115                 120                 125
   Ile Thr Glu Glu Thr Leu Lys Ala Asn Lys Leu Asp Ser Lys Lys Leu
       130                 135                 140
   Thr Arg Ser Asn Gly Thr Thr Leu Glu Tyr Ser Gln Ile Thr Asp Ala
   145                 150                 155                 160
   Asp Asn Ala Thr Lys Ala Val Glu Thr Leu Lys Asn Ser Ile Lys Leu
                   165                 170                 175
   Glu Gly Ser Leu Val Val Gly Lys Thr Thr Val Glu Ile Lys Glu Gly
               180                 185                 190
   Thr Val Thr Leu Lys Arg Glu Ile Glu Lys Asp Gly Lys Val Lys Val
           195                 200                 205
   Phe Leu Asn Asp Thr Ala Gly Ser Asn Lys Lys Thr Gly Lys Trp Glu
       210                 215                 220
   Asp Ser Thr Ser Thr Leu Thr Ile Ser Ala Asp Ser Lys Lys Thr Lys
   225                 230                 235                 240
   Asp Leu Val Phe Leu Thr Asp Gly Thr Ile Thr Val Gln Gln Tyr Asn
                   245                 250                 255
   Thr Ala Gly Thr Ser Leu Glu Gly Ser Ala Ser Glu Ile Lys Asn Leu
               260                 265                 270
   Ser Glu Leu Lys Asn Ala Leu Lys Gly His Pro Met Ala Gln Tyr Asn
           275                 280                 285
   Gln Met His Met Leu Ser Asn Lys Ser Ala Ser Gln Asn Val Arg Thr
       290                 295                 300
   Ala Glu Glu Leu Gly Met Gln Pro Ala Lys Ile Asn Thr Pro Ala Ser
   305                 310                 315                 320
   Leu Ser Gly Leu Gln Ala Ser Trp Thr Leu Arg Val His Val Gly Ala
                   325                 330                 335
   Thr Gln Asp Glu Ala Ile Ala Val Asn Ile Tyr Ala Ala Asn Val Ala
               340                 345                 350
   Asn Leu Phe Ser Gly Glu Gly Ala Gln Thr Ala Gln Ala Ala Pro Val
           355                 360                 365
   Gln Glu Gly Val Gln Gln Glu Gly Ala Gln Gln Pro Ala Pro Ala Thr
       370                 375                 380
   Ala Pro Ser Gln Gly Gly Val Asn Ser Pro Val Asn Val Thr Thr Thr
   385                 390                 395                 400
   Val Asp Ala Asn Thr Ser Leu Ala Lys Ile Glu Asn Ala Ile Arg Met
                   405                 410                 415
   Ile Ser Asp Gln Arg Ala Asn Leu Gly Ala Phe Gln Asn Arg Leu Glu
               420                 425                 430
   Ser Ile Lys Asn Ser Thr Glu Tyr Ala Ile Glu Asn Leu Lys Ala Ser
           435                 440                 445
   Tyr Ala Gln Ile Gly His
       450
<210> 117
   <211> 1141
   <212> DNA
   <213> Artificial Sequence
<220>
   <221> CDS
   <222> (1)...(1140)
   <223> Chimeric nucleic acid
<400> 117
   gca caa aaa ggt gct gag tca att ggt tct caa aaa gaa aat gat cta      48
   Ala Gln Lys Gly Ala Glu Ser Ile Gly Ser Gln Lys Glu Asn Asp Leu
    1               5                  10                  15
   aac ctt gaa gac tct agt aaa aaa tca cat caa aac gct aaa caa gac      96
   Asn Leu Glu Asp Ser Ser Lys Lys Ser His Gln Asn Ala Lys Gln Asp
               20                  25                  30
   ctt cct gcg gtg aca gaa gac tca gtg tct ttg ttt aat ggt aat aaa     144
   Leu Pro Ala Val Thr Glu Asp Ser Val Ser Leu Phe Asn Gly Asn Lys
           35                  40                  45
   att ttt gta agc aaa gaa aaa aat agc tcc ggc aaa tat gat tta aga    192
   Ile Phe Val Ser Lys Glu Lys Asn Ser Ser Gly Lys Tyr Asp Leu Arg
        50                  55                  60
   gca aca att gat cag gtt gaa ctt aaa gga act tcc gat aaa aac aat    240
   Ala Thr Ile Asp Gln Val Glu Leu Lys Gly Thr Ser Asp Lys Asn Asn
    65                  70                  75                  80
   ggt tct gga acc ctt gaa ggt tca aag cct gac aag agt aaa gta aaa    288
   Gly Ser Gly Thr Leu Glu Gly Ser Lys Pro Asp Lys Ser Lys Val Lys
                    85                  90                  95
   tta aca gtt tct gct gat tta aac aca gta acc tta gaa gca ttt gat    336
   Leu Thr Val Ser Ala Asp Leu Asn Thr Val Thr Leu Glu Ala Phe Asp
               100                 105                 110
   gcc agc aac caa aaa att tca agt aaa gtt act aaa aaa cag ggg tca    384
   Ala Ser Asn Gln Lys Ile Ser Ser Lys Val Thr Lys Lys Gln Gly Ser
           115                 120                 125
   ata aca gag gaa act ctc aaa gct aat aaa tta gac tca aag aaa tta    432
   Ile Thr Glu Glu Thr Leu Lys Ala Asn Lys Leu Asp Ser Lys Lys Leu
       130                 135                 140
   aca aga tca aac gga act aca ctt gaa tac tca caa ata aca gat gct    480
   Thr Arg Ser Asn Gly Thr Thr Leu Glu Tyr Ser Gln Ile Thr Asp Ala
   145                 150                 155                 160
   gac aat gct aca aaa gca gta gaa act cta aaa aat agc att aag ctt    528
   Asp Asn Ala Thr Lys Ala Val Glu Thr Leu Lys Asn Ser Ile Lys Leu
                   165                 170                 175
   gaa gga agt ctt gta gtc gga aaa aca aca gtg gaa att aaa gaa ggt    576
   Glu Gly Ser Leu Val Val Gly Lys Thr Thr Val Glu Ile Lys Glu Gly
               180                 185                 190
   act gtt act cta aaa aga gaa att gaa aaa gat gga aaa gta aaa gtc    624
   Thr Val Thr Leu Lys Arg Glu Ile Glu Lys Asp Gly Lys Val Lys Val
           195                 200                 205
   ttt ttg aat gac act gca ggt tct aac aaa aaa aca ggt aaa tgg gaa    672
   Phe Leu Asn Asp Thr Ala Gly Ser Asn Lys Lys Thr Gly Lys Trp Glu
       210                 215                 220
   gac agt act agc act tta aca att agt gct gac agc aaa aaa act aaa    720
   Asp Ser Thr Ser Thr Leu Thr Ile Ser Ala Asp Ser Lys Lys Thr Lys
   225                 230                 235                 240
   gat ttg gtg ttc tta aca gat ggt aca att aca gta caa caa tac aac    768
   Asp Leu Val Phe Leu Thr Asp Gly Thr Ile Thr Val Gln Gln Tyr Asn
                   245                 250                 255
   aca gct gga acc agc cta gaa gga tca gca agt gaa att aaa aat ctt    816
   Thr Ala Gly Thr Ser Leu Glu Gly Ser Ala Ser Glu Ile Lys Asn Leu
               260                 265                 270
   tca gag ctt aaa aac gct tta aaa ggt cac ccc atg gct tct caa aat    864
   Ser Glu Leu Lys Asn Ala Leu Lys Gly His Pro Met Ala Ser Gln Asn
           275                 280                 285
   gta aga aca gct gaa gag ctt gga atg cag cct gca aaa att aac aca     912
   Val Arg Thr Ala Glu Glu Leu Gly Met Gln Pro Ala Lys Ile Asn Thr
       290                 295                 300
   cca gca tca ctt tca ggg ctt caa gcg tct tgg act tta aga gtt cat     960
   Pro Ala Ser Leu Ser Gly Leu Gln Ala Ser Trp Thr Leu Arg Val His
   305                 310                 315                 320
   gtt gga gca acc caa gat gaa gct att gct gta aat att tat gca gct    1008
   Val Gly Ala Thr Gln Asp Glu Ala Ile Ala Val Asn Ile Tyr Ala Ala
                   325                 330                 335
   aat gtt gca aat ctt ttc tct ggt gag gga gct caa act gct cag gct    1056
   Asn Val Ala Asn Leu Phe Ser Gly Glu Gly Ala Gln Thr Ala Gln Ala
               340                 345                 350
   gca ccg gtt caa gag ggt gtt caa cag gaa gga gct caa cag cca gca    1104
   Ala Pro Val Gln Glu Gly Val Gln Gln Glu Gly Ala Gln Gln Pro Ala
           355                 360                 365
   cct gct aca gca cct tct caa ggc gga gtt ggt cac c                  1141
   Pro Ala Thr Ala Pro Ser Gln Gly Gly Val Gly His
       370                 375                 380
<210> 118
   <211> 380
   <212> PRT
   <213> Artificial Sequence
<220>
   <223> Chimeric Protein
<400> 118
   Ala Gln Lys Gly Ala Glu Ser Ile Gly Ser Gln Lys Glu Asn Asp Leu
   1 5 10 15
   Asn Leu Glu Asp Ser Ser Lys Lys Ser His Gln Asn Ala Lys Gln Asp
   20 25 30
   Leu Pro Ala Val Thr Glu Asp Ser Val Ser Leu Phe Asn Gly Asn Lys
   35 40 45
   Ile Phe Val Ser Lys Glu Lys Asn Ser Ser Gly Lys Tyr Asp Leu Arg
   50 55 60
   Ala Thr Ile Asp Gln Val Glu Leu Lys Gly Thr Ser Asp Lys Asn Asn
   65 70 75 80
   Gly Ser Gly Thr Leu Glu Gly Ser Lys Pro Asp Lys Ser Lys Val Lys
   85 90 95
   Leu Thr Val Ser Ala Asp Leu Asn Thr Val Thr Leu Glu Ala Phe Asp
   100 105 110
   Ala Ser Asn Gln Lys Ile Ser Ser Lys Val Thr Lys Lys Gln Gly Ser
   115 120 125
   Ile Thr Glu Glu Thr Leu Lys Ala Asn Lys Leu Asp Ser Lys Lys Leu
   130 135 140
   Thr Arg Ser Asn Gly Thr Thr Leu Glu Tyr Ser Gln Ile Thr Asp Ala
   145 150 155 160
   Asp Asn Ala Thr Lys Ala Val Glu Thr Leu Lys Asn Ser Ile Lys Leu
   165 170 175
   Glu Gly Ser Leu Val Val Gly Lys Thr Thr Val Glu Ile Lys Glu Gly
   180 185 190
   Thr Val Thr Leu Lys Arg Glu Ile Glu Lys Asp Gly Lys Val Lys Val
   195 200 205
   Phe Leu Asn Asp Thr Ala Gly Ser Asn Lys Lys Thr Gly Lys Trp Glu
   210 215 220
   Asp Ser Thr Ser Thr Leu Thr Ile Ser Ala Asp Ser Lys Lys Thr Lys
   225 230 235 240
   Asp Leu Val Phe Leu Thr Asp Gly Thr Ile Thr Val Gln Gln Tyr Asn
   245 250 255
   Thr Ala Gly Thr Ser Leu Glu Gly Ser Ala Ser Glu Ile Lys Asn Leu
   260 265 270
   Ser Glu Leu Lys Asn Ala Leu Lys Gly His Pro Met Ala Ser Gln Asn
   275 280 285
   Val Arg Thr Ala Glu Glu Leu Gly Met Gln Pro Ala Lys Ile Asn Thr
   290 295 300
   Pro Ala Ser Leu Ser Gly Leu Gln Ala Ser Trp Thr Leu Arg Val His
   305 310 315 320
   Val Gly Ala Thr Gln Asp Glu Ala Ile Ala Val Asn Ile Tyr Ala Ala
   325 330 335
   Asn Val Ala Asn Leu Phe Ser Gly Glu Gly Ala Gln Thr Ala Gln Ala
   340 345 350
   Ala Pro Val Gln Glu Gly Val Gln Gln Glu Gly Ala Gln Gln Pro Ala
   355 360 365
   Pro Ala Thr Ala Pro Ser Gln Gly Gly Val Gly His
   370 375 380
<210> 119
   <211> 1324
   <212> DNA
   <213> Artificial sequence
<220>
   <221> CDS
   <222> (1)...(1323)
   <223> Chimeric nucleic acid
<400> 119
   gca caa aaa ggt gct gag tca att ggt tct caa aaa gaa aat gat cta      48
   Ala Gln Lys Gly Ala Glu Ser Ile Gly Ser Gln Lys Glu Asn Asp Leu
   1               5                   10                  15
   aac ctt gaa gac tct agt aaa aaa tca cat caa aac gct aaa caa gac      96
   Asn Leu Glu Asp Ser Ser Lys Lys Ser His Gln Asn Ala Lys Gln Asp
               20                  25                  30
   ctt cct gcg gtg aca gaa gac tca gtg tct ttg ttt aat ggt aat aaa     144
   Leu Pro Ala Val Thr Glu Asp Ser Val Ser Leu Phe Asn Gly Asn Lys
           35                  40                  45
   att ttt gta agc aaa gaa aaa aat agc tcc ggc aaa tat gat tta aga     192
   Ile Phe Val Ser Lys Glu Lys Asn Ser Ser Gly Lys Tyr Asp Leu Arg
       50                  55                  60
   gca aca att gat cag gtt gaa ctt aaa gga act tcc gat aaa aac aat     240
   Ala Thr Ile Asp Gln Val Glu Leu Lys Gly Thr Ser Asp Lys Asn Asn
   65                  70                  75                  80
   ggt tct gga acc ctt gaa ggt tca aag cct gac aag agt aaa gta aaa     288
   Gly Ser Gly Thr Leu Glu Gly Ser Lys Pro Asp Lys Ser Lys Val Lys
                   85                  90                  95
   tta aca gtt tct gct gat tta aac aca gta acc tta gaa gca ttt gat     336
   Leu Thr Val Ser Ala Asp Leu Asn Thr Val Thr Leu Glu Ala Phe Asp
               100                 105                 110
   gcc agc aac caa aaa att tca agt aaa gtt act aaa aaa cag ggg tca    384
   Ala Ser Asn Gln Lys Ile Ser Ser Lys Val Thr Lys Lys Gln Gly Ser
           115                 120                 125
   ata aca gag gaa act ctc aaa gct aat aaa tta gac tca aag aaa tta    432
   Ile Thr Glu Glu Thr Leu Lys Ala Asn Lys Leu Asp Ser Lys Lys Leu
       130                 135                 140
   aca aga tca aac gga act aca ctt gaa tac tca caa ata aca gat gct    480
   Thr Arg Ser Asn Gly Thr Thr Leu Glu Tyr Ser Gln Ile Thr Asp Ala
   145                 150                 155                 160
   gac aat gct aca aaa gca gta gaa act cta aaa aat agc att aag ctt    528
   Asp Asn Ala Thr Lys Ala Val Glu Thr Leu Lys Asn Ser Ile Lys Leu
                   165                 170                 175
   gaa gga agt ctt gta gtc gga aaa aca aca gtg gaa att aaa gaa ggt    576
   Glu Gly Ser Leu Val Val Gly Lys Thr Thr Val Glu Ile Lys Glu Gly
               180                 185                 190
   act gtt act cta aaa aga gaa att gaa aaa gat gga aaa gta aaa gtc    624
   Thr Val Thr Leu Lys Arg Glu Ile Glu Lys Asp Gly Lys Val Lys Val
           195                 200                 205
   ttt ttg aat gac act gca ggt tct aac aaa aaa aca ggt aaa tgg gaa    672
   Phe Leu Asn Asp Thr Ala Gly Ser Asn Lys Lys Thr Gly Lys Trp Glu
       210                 215                 220
   gac agt act agc act tta aca att agt gct gac agc aaa aaa act aaa    720
   Asp Ser Thr Ser Thr Leu Thr Ile Ser Ala Asp Ser Lys Lys Thr Lys
   225                 230                 235                 240
   gat ttg gtg ttc tta aca gat ggt aca att aca gta caa caa tac aac    768
   Asp Leu Val Phe Leu Thr Asp Gly Thr Ile Thr Val Gln Gln Tyr Asn
                   245                 250                 255
   aca gct gga acc agc cta gaa gga tca gca agt gaa att aaa aat ctt    816
   Thr Ala Gly Thr Ser Leu Glu Gly Ser Ala Ser Glu Ile Lys Asn Leu
               260                 265                 270
   tca gag ctt aaa aac gct tta aaa ggt cac ccc atg gct tct caa aat    864
   Ser Glu Leu Lys Asn Ala Leu Lys Gly His Pro Met Ala Ser Gln Asn
           275                 280                 285
   gta aga aca gct gaa gag ctt gga atg cag cct gca aaa att aac aca    912
   Val Arg Thr Ala Glu Glu Leu Gly Met Gln Pro Ala Lys Ile Asn Thr
       290                 295                 300
   cca gca tca ctt tca ggg ctt caa gcg tct tgg act tta aga gtt cat    960
   Pro Ala Ser Leu Ser Gly Leu Gln Ala Ser Trp Thr Leu Arg Val His
   305                 310                 315                 320
   gtt gga gca acc caa gat gaa gct att gct gta aat att tat gca gct    1008
   Val Gly Ala Thr Gln Asp Glu Ala Ile Ala Val Asn Ile Tyr Ala Ala
                   325                 330                 335
   aat gtt gca aat ctt ttc tct ggt gag gga gct caa act gct cag gct    1056
   Asn Val Ala Asn Leu Phe Ser Gly Glu Gly Ala Gln Thr Ala Gln Ala
               340                 345                 350
   gca ccg gtt caa gag ggt gtt caa cag gaa gga gct caa cag cca gca      1104
   Ala Pro Val Gln Glu Gly Val Gln Gln Glu Gly Ala Gln Gln Pro Ala
           355                 360                 365
   cct gct aca gca cct tct caa ggc gga gtt aat tct cct gtt aat gtt      1152
   Pro Ala Thr Ala Pro Ser Gln Gly Gly Val Asn Ser Pro Val Asn Val
       370                 375                 380
   aca act aca gtt gat gct aat aca tca ctt gct aaa att gaa aat gct      1200
   Thr Thr Thr Val Asp Ala Asn Thr Ser Leu Ala Lys Ile Glu Asn Ala
   385                 390                 395                 400
   att aga atg ata agt gat caa agg gca aat tta ggt gct ttc caa aat      1248
   Ile Arg Met Ile Ser Asp Gln Arg Ala Asn Leu Gly Ala Phe Gln Asn
                   405                 410                 415
   aga ctt gaa tct ata aag aat agt act gag tat gca att gaa aat cta      1296
   Arg Leu Glu Ser Ile Lys Asn Ser Thr Glu Tyr Ala Ile Glu Asn Leu
               420                 425                 430
   aaa gca tct tat gct caa ata ggt cac c                                1324
   Lys Ala Ser Tyr Ala Gln Ile Gly His
           435                 440
<210> 120
   <211> 441
   <212> PRT
   <213> Artificial Sequence
<220>
   <223> Chimeric Protein
<400> 120
   Ala Gln Lys Gly Ala Glu Ser Ile Gly Ser Gln Lys Glu Asn Asp Leu
    1               5                  10                  15
   Asn Leu Glu Asp Ser Ser Lys Lys Ser His Gln Asn Ala Lys Gln Asp
               20                  25                  30
   Leu Pro Ala Val Thr Glu Asp Ser Val Ser Leu Phe Asn Gly Asn Lys
           35                  40                  45
   Ile Phe Val Ser Lys Glu Lys Asn Ser Ser Gly Lys Tyr Asp Leu Arg
       50                  55                  60
   Ala Thr Ile Asp Gln Val Glu Leu Lys Gly Thr Ser Asp Lys Asn Asn
   65                  70                  75                  80
   Gly Ser Gly Thr Leu Glu Gly Ser Lys Pro Asp Lys Ser Lys Val Lys
                   85                  90                  95
   Leu Thr Val Ser Ala Asp Leu Asn Thr Val Thr Leu Glu Ala Phe Asp
               100                 105                 110
   Ala Ser Asn Gln Lys Ile Ser Ser Lys Val Thr Lys Lys Gln Gly Ser
           115                 120                 125
   Ile Thr Glu Glu Thr Leu Lys Ala Asn Lys Leu Asp Ser Lys Lys Leu
       130                 135                 140
   Thr Arg Ser Asn Gly Thr Thr Leu Glu Tyr Ser Gln Ile Thr Asp Ala
   145                 150                 155                 160
   Asp Asn Ala Thr Lys Ala Val Glu Thr Leu Lys Asn Ser Ile Lys Leu
                   165                 170                 175
   Glu Gly Ser Leu Val Val Gly Lys Thr Thr Val Glu Ile Lys Glu Gly
               180                 185                 190
   Thr Val Thr Leu Lys Arg Glu Ile Glu Lys Asp Gly Lys Val Lys Val
           195                 200                 205
   Phe Leu Asn Asp Thr Ala Gly Ser Asn Lys Lys Thr Gly Lys Trp Glu
       210                 215                 220
   Asp Ser Thr Ser Thr Leu Thr Ile Ser Ala Asp Ser Lys Lys Thr Lys
   225                 230                 235                 240
   Asp Leu Val Phe Leu Thr Asp Gly Thr Ile Thr Val Gln Gln Tyr Asn
                   245                 250                 255
   Thr Ala Gly Thr Ser Leu Glu Gly Ser Ala Ser Glu Ile Lys Asn Leu
               260                 265                 270
   Ser Glu Leu Lys Asn Ala Leu Lys Gly His Pro Met Ala Ser Gln Asn
           275                 280                 285
   Val Arg Thr Ala Glu Glu Leu Gly Met Gln Pro Ala Lys Ile Asn Thr
       290                 295                 300
   Pro Ala Ser Leu Ser Gly Leu Gln Ala Ser Trp Thr Leu Arg Val His
   305                 310                 315                 320
   Val Gly Ala Thr Gln Asp Glu Ala Ile Ala Val Asn Ile Tyr Ala Ala
                   325                 330                 335
   Asn Val Ala Asn Leu Phe Ser Gly Glu Gly Ala Gln Thr Ala Gln Ala
               340                 345                 350
   Ala Pro Val Gln Glu Gly Val Gln Gln Glu Gly Ala Gln Gln Pro Ala
           355                 360                 365
   Pro Ala Thr Ala Pro Ser Gln Gly Gly Val Asn Ser Pro Val Asn Val
       370                 375                 380
   Thr Thr Thr Val Asp Ala Asn Thr Ser Leu Ala Lys Ile Glu Asn Ala
   385                 390                 395                 400
   Ile Arg Met Ile Ser Asp Gln Arg Ala Asn Leu Gly Ala Phe Gln Asn
                   405                 410                 415
   Arg Leu Glu Ser Ile Lys Asn Ser Thr Glu Tyr Ala Ile Glu Asn Leu
               420                 425                 430
   Lys Ala Ser Tyr Ala Gln Ile Gly His
           435                 440
<210> 121
   <211> 1765
   <212> DNA
   <213> Artificial Sequence
<220>
   <221> CDS
   <222> (1)...(1764)
   <223> Chimeric nucleic acid
<400> 121
   gca caa aaa ggt gct gag tca att ggt tct caa aaa gaa aat gat cta     48
   Ala Gln Lys Gly Ala Glu Ser Ile Gly Ser Gln Lys Glu Asn Asp Leu
    1               5                  10                  15
   aac ctt gaa gac tct agt aaa aaa tca cat caa aac gct aaa caa gac     96
   Asn Leu Glu Asp Ser Ser Lys Lys Ser His Gln Asn Ala Lys Gln Asp
               20                  25                  30
   ctt cct gcg gtg aca gaa gac tca gtg tct ttg ttt aat ggt aat aaa    144
   Leu Pro Ala Val Thr Glu Asp Ser Val Ser Leu Phe Asn Gly Asn Lys
           35                  40                  45
   att ttt gta agc aaa gaa aaa aat agc tcc ggc aaa tat gat tta aga    192
   Ile Phe Val Ser Lys Glu Lys Asn Ser Ser Gly Lys Tyr Asp Leu Arg
       50                  55                  60
   gca aca att gat cag gtt gaa ctt aaa gga act tcc gat aaa aac aat    240
   Ala Thr Ile Asp Gln Val Glu Leu Lys Gly Thr Ser Asp Lys Asn Asn
   65                  70                  75                  80
   ggt tct gga acc ctt gaa ggt tca aag cct gac aag agt aaa gta aaa    288
   Gly Ser Gly Thr Leu Glu Gly Ser Lys Pro Asp Lys Ser Lys Val Lys
                   85                  90                  95
   tta aca gtt tct gct gat tta aac aca gta acc tta gaa gca ttt gat    336
   Leu Thr Val Ser Ala Asp Leu Asn Thr Val Thr Leu Glu Ala Phe Asp
               100                 105                 110
   gcc agc aac caa aaa att tca agt aaa gtt act aaa aaa cag ggg tca    384
   Ala Ser Asn Gln Lys Ile Ser Ser Lys Val Thr Lys Lys Gln Gly Ser
           115                 120                 125
   ata aca gag gaa act ctc aaa gct aat aaa tta gac tca aag aaa tta    432
   Ile Thr Glu Glu Thr Leu Lys Ala Asn Lys Leu Asp Ser Lys Lys Leu
       130                 135                 140
   aca aga tca aac gga act aca ctt gaa tac tca caa ata aca gat gct    480
   Thr Arg Ser Asn Gly Thr Thr Leu Glu Tyr Ser Gln Ile Thr Asp Ala
   145                 150                 155                 160
   gac aat gct aca aaa gca gta gaa act cta aaa aat agc att aag ctt    528
   Asp Asn Ala Thr Lys Ala Val Glu Thr Leu Lys Asn Ser Ile Lys Leu
                   165                 170                 175
   gaa gga agt ctt gta gtc gga aaa aca aca gtg gaa att aaa gaa ggt    576
   Glu Gly Ser Leu Val Val Gly Lys Thr Thr Val Glu Ile Lys Glu Gly
               180                 185                 190
   act gtt act cta aaa aga gaa att gaa aaa gat gga aaa gta aaa gtc    624
   Thr Val Thr Leu Lys Arg Glu Ile Glu Lys Asp Gly Lys Val Lys Val
           195                 200                 205
   ttt ttg aat gac act gca ggt tct aac aaa aaa aca ggt aaa tgg gaa    672
   Phe Leu Asn Asp Thr Ala Gly Ser Asn Lys Lys Thr Gly Lys Trp Glu
       210                 215                 220
   gac agt act agc act tta aca att agt gct gac agc aaa aaa act aaa    720
   Asp Ser Thr Ser Thr Leu Thr Ile Ser Ala Asp Ser Lys Lys Thr Lys
   225                 230                 235                 240
   gat ttg gtg ttc tta aca gat ggt aca att aca gta caa caa tac aac    768
   Asp Leu Val Phe Leu Thr Asp Gly Thr Ile Thr Val Gln Gln Tyr Asn
                   245                 250                 255
   aca gct gga acc agc cta gaa gga tca gca agt gaa att aaa aat ctt    816
   Thr Ala Gly Thr Ser Leu Glu Gly Ser Ala Ser Glu Ile Lys Asn Leu
               260                 265                 270
   tca gag ctt aaa aac gct tta aaa ggt cac ccc atg gga aat aat tca    864
   Ser Glu Leu Lys Asn Ala Leu Lys Gly His Pro Met Gly Asn Asn Ser
           275                 280                 285
   ggg aaa gat ggg aat aca tct gca aat tct gct gat gag tct gtt aaa    912
   Gly Lys Asp Gly Asn Thr Ser Ala Asn Ser Ala Asp Glu Ser Val Lys
       290                 295                 300
   ggg cct aat ctt aca gaa ata agt aaa aaa att acg gat tct aat gcg    960
   Gly Pro Asn Leu Thr Glu Ile Ser Lys Lys Ile Thr Asp Ser Asn Ala
   305                 310                 315                 320
   gtt tta ctt gct gtg aaa gag gtt gaa gcg ttg ctg tca tct ata gat     1008
   Val Leu Leu Ala Val Lys Glu Val Glu Ala Leu Leu Ser Ser Ile Asp
                   325                 330                 335
   gaa att gct gct aaa gct att ggt aaa aaa ata cac caa aat aat ggt     1056
   Glu Ile Ala Ala Lys Ala Ile Gly Lys Lys Ile His Gln Asn Asn Gly
               340                 345                 350
   ttg gat acc gaa tat aat cac aat gga tca ttg tta gcg gga cgt tat     1104
   Leu Asp Thr Glu Tyr Asn His Asn Gly Ser Leu Leu Ala Gly Arg Tyr
           355                 360                 365
   gca ata tca acc cta ata aaa caa aaa tta gat gga ttg aaa aat gaa     1152
   Ala Ile Ser Thr Leu Ile Lys Gln Lys Leu Asp Gly Leu Lys Asn Glu
       370                 375                 380
   gga tta aag gaa aaa att gat gcg gct aag aaa tgt tct gaa aca ttt     1200
   Gly Leu Lys Glu Lys Ile Asp Ala Ala Lys Lys Cys Ser Glu Thr Phe
   385                 390                 395                 400
   act aat aaa tta aaa gaa aaa cac aca gat ctt ggt aaa gaa ggt gtt     1248
   Thr Asn Lys Leu Lys Glu Lys His Thr Asp Leu Gly Lys Glu Gly Val
                   405                 410                 415
   act gat gct gat gca aaa gaa gcc att tta aaa aca aat ggt act aaa     1296
   Thr Asp Ala Asp Ala Lys Glu Ala Ile Leu Lys Thr Asn Gly Thr Lys
               420                 425                 430
   act aaa ggt gct gaa gaa ctt gga aaa tta ttt gaa tca gta gag gtc     1344
   Thr Lys Gly Ala Glu Glu Leu Gly Lys Leu Phe Glu Ser Val Glu Val
           435                 440                 445
   ttg tca aaa gca gct aaa gag atg ctt gct aat tca gtt aaa gag ctt     1392
   Leu Ser Lys Ala Ala Lys Glu Met Leu Ala Asn Ser Val Lys Glu Leu
       450                 455                 460
   aca agc cct gtt gtg gca gaa agt cca aaa aaa cct ggt acc atg gct     1440
   Thr Ser Pro Val Val Ala Glu Ser Pro Lys Lys Pro Gly Thr Met Ala
   465                 470                 475                 480
   caa tat aac caa atg cac atg tta tca aac aaa tct gct tct caa aat     1488
   Gln Tyr Asn Gln Met His Met Leu Ser Asn Lys Ser Ala Ser Gln Asn
                   485                 490                 495
   gta aga aca gct gaa gag ctt gga atg cag cct gca aaa att aac aca     1536
   Val Arg Thr Ala Glu Glu Leu Gly Met Gln Pro Ala Lys Ile Asn Thr
               500                 505                 510
   cca gca tca ctt tca ggg ctt caa gcg tct tgg act tta aga gtt cat     1584
   Pro Ala Ser Leu Ser Gly Leu Gln Ala Ser Trp Thr Leu Arg Val His
           515                 520                 525
   gtt gga gca acc caa gat gaa gct att gct gta aat att tat gca gct     1632
   Val Gly Ala Thr Gln Asp Glu Ala Ile Ala Val Asn Ile Tyr Ala Ala
       530                 535                 540
   aat gtt gca aat ctt ttc tct ggt gag gga gct caa act gct cag gct     1680
   Asn Val Ala Asn Leu Phe Ser Gly Glu Gly Ala Gln Thr Ala Gln Ala
   545                 550                 555                 560
   gca ccg gtt caa gag ggt gtt caa cag gaa gga gct caa cag cca gca      1728
   Ala Pro Val Gln Glu Gly Val Gln Gln Glu Gly Ala Gln Gln Pro Ala
                   565                 570                 575
   cct gct aca gca cct tct caa ggc gga gtt ggt cac c                    1765
   Pro Ala Thr Ala Pro Ser Gln Gly Gly Val Gly His
               580                 585
<210> 122
   <211> 588
   <212> PRT
   <213> Artificial Sequence
<220>
   <223> Chimeric Protein
<400> 122
   Ala Gln Lys Gly Ala Glu Ser Ile Gly Ser Gln Lys Glu Asn Asp Leu
   1               5                   10                  15
   Asn Leu Glu Asp Ser Ser Lys Lys Ser His Gln Asn Ala Lys Gln Asp
               20                  25                  30
   Leu Pro Ala Val Thr Glu Asp Ser Val Ser Leu Phe Asn Gly Asn Lys
           35                  40                  45
   Ile Phe Val Ser Lys Glu Lys Asn Ser Ser Gly Lys Tyr Asp Leu Arg
       50                  55                  60
   Ala Thr Ile Asp Gln Val Glu Leu Lys Gly Thr Ser Asp Lys Asn Asn
   65                  70                  75                  80
   Gly Ser Gly Thr Leu Glu Gly Ser Lys Pro Asp Lys Ser Lys Val Lys
                   85                  90                  95
   Leu Thr Val Ser Ala Asp Leu Asn Thr Val Thr Leu Glu Ala Phe Asp
               100                 105                 110
   Ala Ser Asn Gln Lys Ile Ser Ser Lys Val Thr Lys Lys Gln Gly Ser
           115                 120                 125
   Ile Thr Glu Glu Thr Leu Lys Ala Asn Lys Leu Asp Ser Lys Lys Leu
       130                 135                 140
   Thr Arg Ser Asn Gly Thr Thr Leu Glu Tyr Ser Gln Ile Thr Asp Ala
   145                 150                 155                 160
   Asp Asn Ala Thr Lys Ala Val Glu Thr Leu Lys Asn Ser Ile Lys Leu
                   165                 170                 175
   Glu Gly Ser Leu Val Val Gly Lys Thr Thr Val Glu Ile Lys Glu Gly
               180                 185                 190
   Thr Val Thr Leu Lys Arg Glu Ile Glu Lys Asp Gly Lys Val Lys Val
           195                 200                 205
   Phe Leu Asn Asp Thr Ala Gly Ser Asn Lys Lys Thr Gly Lys Trp Glu
       210                 215                 220
   Asp Ser Thr Ser Thr Leu Thr Ile Ser Ala Asp Ser Lys Lys Thr Lys
   225                 230                 235                 240
   Asp Leu Val Phe Leu Thr Asp Gly Thr Ile Thr Val Gln Gln Tyr Asn
                   245                 250                 255
   Thr Ala Gly Thr Ser Leu Glu Gly Ser Ala Ser Glu Ile Lys Asn Leu
               260                 265                 270
   Ser Glu Leu Lys Asn Ala Leu Lys Gly His Pro Met Gly Asn Asn Ser
           275                 280                 285
   Gly Lys Asp Gly Asn Thr Ser Ala Asn Ser Ala Asp Glu Ser Val Lys
       290                 295                 300
   Gly Pro Asn Leu Thr Glu Ile Ser Lys Lys Ile Thr Asp Ser Asn Ala
   305                 310                 315                 320
   Val Leu Leu Ala Val Lys Glu Val Glu Ala Leu Leu Ser Ser Ile Asp
                   325                 330                 335
   Glu Ile Ala Ala Lys Ala Ile Gly Lys Lys Ile His Gln Asn Asn Gly
               340                 345                 350
   Leu Asp Thr Glu Tyr Asn His Asn Gly Ser Leu Leu Ala Gly Arg Tyr
               355                 360                 365
   Ala Ile Ser Thr Leu Ile Lys Gln Lys Leu Asp Gly Leu Lys Asn Glu
       370                 375                 380
   Gly Leu Lys Glu Lys Ile Asp Ala Ala Lys Lys Cys Ser Glu Thr Phe
   385                 390                 395                 400
   Thr Asn Lys Leu Lys Glu Lys His Thr Asp Leu Gly Lys Glu Gly Val
                   405                 410                 415
   Thr Asp Ala Asp Ala Lys Glu Ala Ile Leu Lys Thr Asn Gly Thr Lys
               420                 425                 430
   Thr Lys Gly Ala Glu Glu Leu Gly Lys Leu Phe Glu Ser Val Glu Val
           435                 440                 445
   Leu Ser Lys Ala Ala Lys Glu Met Leu Ala Asn Ser Val Lys Glu Leu
       450                 455                 460
   Thr Ser Pro Val Val Ala Glu Ser Pro Lys Lys Pro Gly Thr Met Ala
   465                 470                 475                 480
   Gln Tyr Asn Gln Met His Met Leu Ser Asn Lys Ser Ala Ser Gln Asn
                   485                 490                 495
   Val Arg Thr Ala Glu Glu Leu Gly Met Gln Pro Ala Lys Ile Asn Thr
               500                 505                 510
   Pro Ala Ser Leu Ser Gly Leu Gln Ala Ser Trp Thr Leu Arg Val His
           515                 520                 525
   Val Gly Ala Thr Gln Asp Glu Ala Ile Ala Val Asn Ile Tyr Ala Ala
       530                 535                 540
   Asn Val Ala Asn Leu Phe Ser Gly Glu Gly Ala Gln Thr Ala Gln Ala
   545                 550                 555                 560
   Ala Pro Val Gln Glu Gly Val Gln Gln Glu Gly Ala Gln Gln Pro Ala
                   565                 570                 575
   Pro Ala Thr Ala Pro Ser Gln Gly Gly Val Gly His
               580                 585
<210> 123
   <211> 704
   <212> DNA
   <213> Borrelia burgdorferi
<400> 123
   ctactgttaa gtttattttt attgctctca atatcttgtt ctttagataa tgaaggtgta 60
   aactcaaaag attacgagtc aaaaaaacag agtatactag gtgaattaaa tcagctattg 120
   gggcaaacta caaattcact aaaagaagca aaaaatacaa cagataattt aaatgcatca 180
   aatgaggcaa ataaagttgt agaagcagtt ataagtgtgg ttaatttaat ttcatctgct 240
   gcagatcagg taaaaggtca acaacaaata tgcacgattt agctcaaatg gcagaaatag 300
   atttagaaaa aataaaggaa tctagtgata aagtaatagt tgcggctaat gttgcgaaag 360
   aagcatataa ccttactaaa gcagtagaac aaaatatgca aaaactgtac aaagagcaag 420
   aagagcaact aaaacactat ctgattctga tgaaacagaa cgagtttctg atgaaataaa 480
   acaagctaaa gaggctgtag aaatagcttg gaaagccaca gtaaaagtaa aagatgagtt 540
   aattgatgta gaaaatgcag tcaaagaggc attggataaa ataaagacag aaaccgcgaa 600
   caatacaaaa cttacagata tagaagaagt agcagagtta gtattacaga tagccaaaaa 660
   tgtagcggaa atagcgcaag aagttgtggc cttgttaaat actt                  704
<210> 124
   <211> 704
   <212> DNA
   <213> Borrelia burgdorferi
<400> 124
   ctactgttaa gtttattttt attgctctca atatcttgtt atttagataa tgaaggtgta 60
   aactcaaaag attacgagtc aaaaaaacag agtatactag gtgaattaaa tcagctattg 120
   gggcaaacta caaattcact aaaagaagca aaaaatacaa cagataattt aaatgcatca 180
   aatgaggcaa ataaagttgt agaagcagtt ataagtgtgg ttaatttaat ttcatctgct 240
   gcagatcagg taaaaggtca acaacaaata tgcacgattt agctcaaatg gcagaaatag 300
   atttagaaaa aataaaggaa tctagtgata aagtaatagt tgcggctaat gttgcgaaag 360
   aagcatataa ccttactaaa gcagtagaac aaaatatgca aaaactgtac aaagagcaag 420
   aagagcaact aaaacactat ctgattctga tgaaacagaa cgagtttctg atgaaataaa 480
   acaagctaaa gaggctgtag aaatagcttg gaaagccaca gtaaaagtaa aagatgagtt 540
   aattgatgta gaaaatgcag tcaaagaggc attggataaa ataaagacag aaaccgcgaa 600
   caatacaaaa cttacagata tagaagaagt agcagagtta gtattacaaa tagccaaaaa 660
   tgtagcggaa atagcgcaag aagttgtggc cttgttaaat actt 704
<210> 125
   <211> 704
   <212> DNA
   <213> Borrelia burgdorferi
<400> 125
   ctactgctaa gtttattttt attgctctca atatctggtt ctttagataa tgaaggtgta 60
   aactcaaaag attacgagtc aaaaaaacag agtatactag gtgaattaaa tcagctattg 120
   gggcaaacta caaattcact aaaagaagca aaaaatacaa cagataattt aaatgcatca 180
   aatgaggcaa ataaagttgt agaagcagtt ataagtgtgg ttaatttaat ttcatctgct 240
   gcagatcagg tgaaaggtca acaacaaata tgcacgattt agctcaaatg gcagaaatag 300
   atttagaaaa aataaaggaa tctagtgata aagtaatagt tgcggctaat gttgcgaaag 360
   aagcatataa ccttactaaa gcagtagaac aaaatatgca aaaactgtac aaagagcaag 420
   aagagcaact aaaacactat ctgattctga tgaagcagaa cgagtttctg atgaaataaa 480
   acaagctaaa gaggctgtag aaatagcttg gaaagccaca gtaaaagtaa aagatgagtt 540
   aattgatgta gaaaatgcag tcaaagaggc attggataaa ataaagacag aaaccgcgaa 600
   caatacaaaa cttacagata tagaagaagt agcagagtta gtattacaaa tagccaaaaa 660
   tgtagcggaa atagcgcaag aagttgtggc cttgttaaat actt 704
<210> 126
   <211> 704
   <212> DNA
   <213> Borrelia burgdorferi
<400> 126
   ctactgttaa gtttattttt attgctctca atatcttgtt ctttagataa tgaaggtgta 60
   agctcaaaag attacgagtc aaaaaaacag agtatactag gtgaattaaa tcagctattg 120
   gggcaaacta caaattcact aaaagaagca aaaaatacaa cagataattt aaatgcatca 180
   aatgaggcaa ataaagttgt agaagcagtt ataagtgtgg ttaatttaat ttcatctgct 240
   gcagatcagg tgaaaggtca acaacaaata tgcacgattt agctcaaatg gcagaaatag 300
   atttagaaaa aataaaggaa tctagtgata aagtaatagt tgcggctaat gttgcgaaag 360
   aagcatataa ccttactaaa gcagtagaac aaaatatgca aaaactgtac aaagagcaag 420
   aagagcaact aaaacactat ctgattctga tgaagcagaa cgagtttctg atgaaataaa 480
   acaagctaaa gaggctgtag aaatagcttg gaaagccaca gtaaaagtaa aagatgagtt 540
   aattgatgta gaaaatgcag tcaaagaggc attggataaa ataaagacag gaaccgcgaa 600
   caatacaaaa cttacagata tagaagaagt agcagagtta gtattacaaa tagccaaaaa 660
   tgtagcggaa atagcgcaag aagttgtggc cttgttaaat actt 704
<210> 127
   <211> 1011
   <212> DNA
   <213> Borrelia burgdorferi
<220>
   <221> CDS
   <222> (1)...(1011)
<400> 127
   atg att atc aat cat aat aca tca gct att aat gct tca aga aat aat    48
   Met Ile Ile Asn His Asn Thr Ser Ala Ile Asn Ala Ser Arg Asn Asn
    1               5                  10                  15
   ggc att aac gct gct aat ctt agt aaa act caa gaa aag ctt tct agt    96
   Gly Ile Asn Ala Ala Asn Leu Ser Lys Thr Gln Glu Lys Leu Ser Ser
               20                  25                  30
   ggc tac aga att aat cga gct tct gat gat gct gct ggc atg gga gtt   144
   Gly Tyr Arg Ile Asn Arg Ala Ser Asp Asp Ala Ala Gly Met Gly Val
           35                  40                  45
   tct ggt aag att aat gct caa ata aga ggt ttg tca caa gct tct aga   192
   Ser Gly Lys Ile Asn Ala Gln Ile Arg Gly Leu Ser Gln Ala Ser Arg
       50                  55                  60
   aat act tca aag gct att aat ttt att cag aca aca gaa ggg aat tta   240
   Asn Thr Ser Lys Ala Ile Asn Phe Ile Gln Thr Thr Glu Gly Asn Leu
   65                  70                  75                  80
   aat gaa gta gaa aaa gtc tta gta aga atg aag gaa ttg gca gtt caa   288
   Asn Glu Val Glu Lys Val Leu Val Arg Met Lys Glu Leu Ala Val Gln
                   85                  90                  95
   tca ggt aac ggc aca tat tca gat gca gac aga ggt tct ata caa att   336
   Ser Gly Asn Gly Thr Tyr Ser Asp Ala Asp Arg Gly Ser Ile Gln Ile
               100                 105                 110
   gaa ata gag caa ctt aca gac gaa att aat aga att gct gat caa gct   384
   Glu Ile Glu Gln Leu Thr Asp Glu Ile Asn Arg Ile Ala Asp Gln Ala
           115                 120                 125
   caa tat aac caa atg cac atg tta tca aac aaa tct gct tct caa aat   432
   Gln Tyr Asn Gln Met His Met Leu Ser Asn Lys Ser Ala Ser Gln Asn
       130                 135                 140
   gta aga aca gct gaa gag ctt gga atg cag cct gca aaa att aac aca   480
   Val Arg Thr Ala Glu Glu Leu Gly Met Gln Pro Ala Lys Ile Asn Thr
   145                 150                 155                 160
   cca gca tca ctt tca ggg ctt caa gcg tct tgg act tta aga gtt cat   528
   Pro Ala Ser Leu Ser Gly Leu Gln Ala Ser Trp Thr Leu Arg Val His
                   165                 170                 175
   gtt gga gca acc caa gat gaa gct att gct gta aat att tat gca gct   576
   Val Gly Ala Thr Gln Asp Glu Ala Ile Ala Val Asn Ile Tyr Ala Ala
               180                 185                 190
   aat gtt gca aat ctt ttc tct ggt gag gga gct caa act gct cag gct   624
   Asn Val Ala Asn Leu Phe Ser Gly Glu Gly Ala Gln Thr Ala Gln Ala
           195                 200                 205
   gca ccg gtt caa gag ggt gtt caa cag gaa gga gct caa cag cca gca     672
   Ala Pro Val Gln Glu Gly Val Gln Gln Glu Gly Ala Gln Gln Pro Ala
       210                 215                 220
   cct gct aca gca cct tct caa ggc gga gtt aat tct cct gtt aat gtt     720
   Pro Ala Thr Ala Pro Ser Gln Gly Gly Val Asn Ser Pro Val Asn Val
   225                 230                 235                 240
   aca act aca gtt gat gct aat aca tca ctt gct aaa att gaa aat gct     768
   Thr Thr Thr Val Asp Ala Asn Thr Ser Leu Ala Lys Ile Glu Asn Ala
                   245                 250                 255
   att aga atg ata agt gat caa agg gca aat tta ggt gct ttc caa aat     816
   Ile Arg Met Ile Ser Asp Gln Arg Ala Asn Leu Gly Ala Phe Gln Asn
               260                 265                 270
   aga ctt gaa tct ata aag aat agt act gag tat gca att gaa aat cta     864
   Arg Leu Glu Ser Ile Lys Asn Ser Thr Glu Tyr Ala Ile Glu Asn Leu
           275                 280                 285
   aaa gca tct tat gct caa ata aaa gat gct aca atg aca gat gag gtt     912
   Lys Ala Ser Tyr Ala Gln Ile Lys Asp Ala Thr Met Thr Asp Glu Val
       290                 295                 300
   gta gca gca aca act aat atg att tta aca caa tct gca atg gca atg     960
   Val Ala Ala Thr Thr Asn Met Ile Leu Thr Gln Ser Ala Met Ala Met
   305                 310                 315                 320
   att gcg cag gct aat caa gtt ccc caa tat gtt ttg tca ttg ctt aga     1008
   Ile Ala Gln Ala Asn Gln Val Pro Gln Tyr Val Leu Ser Leu Leu Arg
                   325                 330                 335
   taa                                                                 1011
    *
<210> 128
   <211> 336
   <212> PRT
   <213> Borrelia burgdorferi
<400> 128
   Met Ile Ile Asn His Asn Thr Ser Ala Ile Asn Ala Ser Arg Asn Asn
   1               5                   10                  15
   Gly Ile Asn Ala Ala Asn Leu Ser Lys Thr Gln Glu Lys Leu Ser Ser
               20                  25                  30
   Gly Tyr Arg Ile Asn Arg Ala Ser Asp Asp Ala Ala Gly Met Gly Val
           35                  40                  45
   Ser Gly Lys Ile Asn Ala Gln Ile Arg Gly Leu Ser Gln Ala Ser Arg
       50                  55                  60
   Asn Thr Ser Lys Ala Ile Asn Phe Ile Gln Thr Thr Glu Gly Asn Leu
   65                  70                  75                  80
   Asn Glu Val Glu Lys Val Leu Val Arg Met Lys Glu Leu Ala Val Gln
                   85                  90                  95
   Ser Gly Asn Gly Thr Tyr Ser Asp Ala Asp Arg Gly Ser Ile Gln Ile
               100                 105                 110
   Glu Ile Glu Gln Leu Thr Asp Glu Ile Asn Arg Ile Ala Asp Gln Ala
           115                 120                 125
   Gln Tyr Asn Gln Met His Met Leu Ser Asn Lys Ser Ala Ser Gln Asn
       130                 135                 140
   Val Arg Thr Ala Glu Glu Leu Gly Met Gln Pro Ala Lys Ile Asn Thr
   145                 150                 155                 160
   Pro Ala Ser Leu Ser Gly Leu Gln Ala Ser Trp Thr Leu Arg Val His
                   165                 170                 175
   Val Gly Ala Thr Gln Asp Glu Ala Ile Ala Val Asn Ile Tyr Ala Ala
               180                 185                 190
   Asn Val Ala Asn Leu Phe Ser Gly Glu Gly Ala Gln Thr Ala Gln Ala
           195                 200                 205
   Ala Pro Val Gln Glu Gly Val Gln Gln Glu Gly Ala Gln Gln Pro Ala
       210                 215                 220
   Pro Ala Thr Ala Pro Ser Gln Gly Gly Val Asn Ser Pro Val Asn Val
   225                 230                 235                 240
   Thr Thr Thr Val Asp Ala Asn Thr Ser Leu Ala Lys Ile Glu Asn Ala
                   245                 250                 255
   Ile Arg Met Ile Ser Asp Gln Arg Ala Asn Leu Gly Ala Phe Gln Asn
               260                 265                 270
   Arg Leu Glu Ser Ile Lys Asn Ser Thr Glu Tyr Ala Ile Glu Asn Leu
           275                 280                 285
   Lys Ala Ser Tyr Ala Gln Ile Lys Asp Ala Thr Met Thr Asp Glu Val
       290                 295                 300
   Val Ala Ala Thr Thr Asn Met Ile Leu Thr Gln Ser Ala Met Ala Met
   305                 310                 315                 320
   Ile Ala Gln Ala Asn Gln Val Pro Gln Tyr Val Leu Ser Leu Leu Arg
                   325                 330                 335
<210> 129
   <211> 1008
   <212> DNA
   <213> Borrelia burgdorferi
<400> 129
   atgattatca atcataatac atcagctatt aatgcttcaa gaaataatgg cattaacgct 60
   gctaatctta gtaaaactca agaaaagctt tctagtgggt acagaattaa tcgagcttct 120
   gatgatgctg ctggcatggg agtttctggt aagattaatg ctcaaataag aggtttgtca 180
   caagcttcta gaaatacttc aaaggctatt aattttattc agacaacaga agggaattta 240
   aatgaagtag aaaaagtctt agtaagaatg aaggaattgg cagttcaatc aggtaacggc 300
   acatattcag atgcagacag aggttctata caaattgaaa tagagcaact tacagacgaa 360
   attaatagaa ttgctgatca agctcaatat aaccaaatgc acatgttatc aaacaaatct 420
   gcttctcaaa atgtaagaac agctgaagag cttggaatgc agcctgcaaa aattaacaca 480
   ccagcatcac tttcagggtc tcaagcgtct tggactttaa gagttcatgt tggagcaaac 540
   caagatgaag ctattgctgt aaatatttat gcagctaatg ttgcaaatct tttctctggt 600
   gagggagctc aaactgctca ggctgcaccg gttcaagagg gtgttcaaca ggaaggagct 660
   caacagccag cacctgctac agcaccttct caaggcggag ttaattctcc tgttaatgtt 720
   acaactacag ttgatgctaa tacatcactt gctaaaattg aaaatgctat tagaatgata 780
   agtgatcaaa gggcaaattt aggtgctttc caaaatagac ttgaatctat aaagaatagt 840
   actgagtatg caattgaaaa tctaaaagca tcttatgctc aaataaaaga tgctacaatg 900
   acagatgagg ttgtagcagc aacaactaat agtattttaa cacaatctgc aatggcaatg 960
   attgcgcagg ctaatcaagt tccccaatat gttttgtcat tgcttaga              1008
<210> 130
   <211> 1008
   <212> DNA
   <213> Borrelia burgdorferi
<400> 130
   atgattatca atcataatac atcagctatt aatgcttcaa gaaataatgc cattaatgct 60
   gctaatctta gtaaaaccca agagaagctt tctagtggtt acagaattaa tcgagcttct 120
   gatgatgctg ctggtatggg ggtttctggc aagattaatg ctcaaataag aggcttatca 180
   caagcttcta gaaacacttc aaaagctatc aattttattc agacaacaga aggaaattta 240
   aatgaagtag aaaaagtttt agtaagaatg aaagaattag cagttcaatc aggtaacgga 300
   acgtattcag actcagacag aggttctata cagattgaaa tagagcaact tacagacgaa 360
   attaatagaa ttgctgatca ggctcaatat aaccaaatgc acatgttgtc aaacaaatct 420
   gcttcccaaa atgtaaaaac agctgaagag cttggaatgc agcctgcaaa aattaacaca 480
   ccagcatcac tttcaggatc tcaagcttct tggactttaa gagttcatgt gggagcaaat 540
   caagatgaag caattgctgt aaatatttat tcagctaatg ttgcaaatct ttttgctggt 600
   gagggagctc aagctgctca ggctgcacct gttcaagagg gtgctcaaga agaaggagct 660
   cagcaaccaa cacctgctac agcacctact caaggtggag ttaattctcc tgttaatgtt 720
   acaaccacag ttgatgctaa tacatcactt gctaaaatag aaaatgctat tagaatgata 780
   agtgatcaaa gagcaaattt aggtgctttc caaaatagac ttgaatctat aaagaatagc 840
   actgagtatg ctattgaaaa tctaaaagca tcttatgctc aaataaaaga tgctacaatg 900
   acagatgagg ttgtagcagc tacaactaat agtattttaa ctcaatctgc aatggcaatg 960
   attgcacagg ctaatcaagt tcctcaatat gttttgtcat tgcttaga            1008
<210> 131
   <211> 1008
   <212> DNA
   <213> Borrelia burgdorferi
<400> 131
   atgattatca atcataatac atcagctatt aatgcttcaa gaaataatgc cattaatgct 60
   gctaatctta gtaaaaccca agagaagcct tctagtggtt acagaattaa tcgagcttct 120
   gatgatgctg ctggtatggg ggtttctggc aagattaatg ctcaaataag aggcttatca 180
   caagcttcta gaaacacttc aaaagctatc aattttattc agacaacaga aggaaattta 240
   aatgaagtag aaaaagtttt agtaagaatg aaagaattag cagttcaatc aggtaacgga 300
   acgtattcag actcagacag aggttctata cagattgaaa tagagcaact tacagacgaa 360
   attaatagaa ttgctgatca ggctcaatat aaccaaatgc acatgttgtc aaacaaatct 420
   gcttcccaaa atgtaaaaac agctgaagag cttggaatgc agcctgcaaa aattaacaca 480
   ccagcatcac tttcaggatc tcaagcttct tggactttaa gagttcatgt gggagcaaat 540
   caagatgaag caattgctgt aaatatttat tcagctaatg ttgcaaatct ttttgctggt 600
   gagggagctc aagctgctca ggctgcacct gttcaagagg gtgctcaaga agaaggagct 660
   cagcaaccaa cacctgctac agcacctact caaggtggag ttaattctcc tgttaatgtt 720
   acaaccacag ttgatgctaa tacatcactt gctaaaatag aaaatgctat tagaatgata 780
   agtgatcaaa gagcaaattt aggtgctttc caaaatagac ttgaatctat aaagaatagc 840
   actgagtatg ctattgaaaa tctaaaagca tcttatgctc aaataaaaga tgctacaatg 900
   acagatgagg ttgtagcagc tacaactaaa agtattttaa ctcaatctgc aatggcaatg 960
   attgcacagg ctaatcaagt tcctcaatat gttttgtcat tgcttaga            1008
<210> 132
   <211> 822
   <212> DNA
   <213> Borrelia burgdorferi
<400> 132
   atgaaaaaat atttattggg aataggtcta atattagcct taatagcatg taagcaaaat 60
   gttagcagcc ttgacgagaa aaacagcgtt tcagtagatt tgcctggtga aatgaaagtt 120
   cttgtaagca aagaaaaaaa caaagacggc aagtacgatc taattgcaac agtagacaag 180
   cttgagctta aaggaacttc tgataaaaac aatggatctg gagtacttga aggcgtaaaa 240
   gctgacaaaa gtaaagtaaa attaacaatt tctgacgatc taggtcaaac cacacttgaa 300
   gttttcaaag aagatggcaa aacactagta tcaaaaaaag taacttccaa agacaagtca 360
   tcaacagaag aaaaattcaa tgaaaaaggt gaagtatctg aaaaaataat aacaagagca 420
   gacggaacca gacttgaata cacaggaatt aaaagcgatg gatctggaaa agctaaagag 480
   gttttaaaag gctatgttct tgaaggaact ctaactgctg aaaaaacaac attggtggtt 540
   aaagaaggaa ctgttacttt aagcaaaaat atttcaaaat ctggggaagt ttcagttgaa 600
   cttaatgaca ctgacagtag tgctgctact aaaaaaactg cagcttggaa ttcaggcact 660
   tcaactttaa caattactgt aaacagtaaa aaaactaaag accttgtgtt tacaaaagaa 720
   aacacaatta cagtacaaca atacgactca aatggcacca aattagaggg gtcagcagtt 780
   gaaattacaa aacttgatga aattaaaaac gctttaaaat aa                    822
<210> 133
   <211> 822
   <212> DNA
   <213> Borrelia burgdorferi
<400> 133
   atgaaaaaat atttattggg aataggtcta atattagcct taatagcatg taagcaaaat 60
   gttagcagcc ttgacgagaa aaacagcgtt tcagtagatt tgcctggtga aatgaacgtt 120
   cttgtaagca aagaaaaaaa caaagacggc aagtacgatc taattgcaac agtagacaag 180
   cttgagctta aaggaacttc tgataaaaac aatggatctg gagtacttga aggcgtaaaa 240
   gctgacaaaa gtaaagtaaa attaacaatt tctgacgatc taggtcaaac cacacttgaa 300
   gttttcaaag aagatggcaa aacactagta tcaaaaaaag taacttccaa agacaagtca 360
   tcaacagaag aaaaattcaa tgaaaaaggt gaagtatctg aaaaaataat aacaagagca 420
   gacggaacca gacttgaata cacagaaatt aaaagcgatg gatctggaaa agctaaagag 480
   gttttaaaag gctatgttct tgaaggaact ctaactgctg aaaaaacaac attggtggtt 540
   aaagaaggaa ctgttacttt aagcaaaaat atttcaaaat ctggggaagt ttcagttgaa 600
   cttaatgaca ctgacagtag tgctgctact aaaaaaactg cagcttggaa ttcaggcact 660
   tcaactttaa caattactgt aaacagtaaa aaaactaaag accttgtgtt tacaaaagaa 720
   aacacaatta cagtacaaca atacgactca aatggcacca aattagaggg gtcagcagtt 780
   gaaattacaa aacttgatga aattaaaaac gctttaaaat aa                    822
<210> 134
   <211> 822
   <212> DNA
   <213> Borrelia burgdorferi
<400> 134
   atgaaaaaat atttattggg aataggtcta atattagcct taatagcatg taagcaaaat 60
   gttagcagcc ttgacgagaa aaacagcgtt tcagtagatt tgcctggtga aatgaacgtt 120
   cttgtaagca aagaaaaaaa caaagacggc aagtacgatc taattgcaac agtagacaag 180
   cttgagctta aaggaacttc tgataaaaac aatggatctg gagtacttga aggcgtaaaa 240
   gctgacaaaa gtaaagtaaa attaacaatt tctgacgatc taggtcaaac cacacttgaa 300
   gttttcaaag aagatggcaa aacactagta tcaaaaaaag taacttccaa agacaagtca 360
   tcaacagaag aaaaattcaa tgaaaaaggt gaagtatctg aaaaaataat aacaagagca 420
   gacggaacca gacttgaata cacagaaatt aaaagcgatg gatctggaaa agctaaagag 480
   gttttaaaaa gctatgttct tgaaggaact ttaactgctg aaaaaacaac attggtggtt 540
   aaagaaggaa ctgttacttt aagcaaaaat atttcaaaat ctggggaagt ttcagttgaa 600
   cttaatgaca ctgacagtag tgctgctact aaaaaaactg cagcttggaa ttcaggcact 660
   tcaactttaa caattactgt aaacagtaaa aaaactaaag accttgtgtt tacaaaagaa 720
   aacacaatta cagtacaaca atacgactca aatggcacca aattagaggg gtcagcagtt 780
   gaaattacaa aacttgatga aattaaaaac gctttaaaat aa                    822
<210> 135
   <211> 821
   <212> DNA
   <213> Borrelia burgdorferi
<400> 135
   atgaaaaaat atttattggg aataggtcta atattagcct taatagcatg taagcaaaat 60
   gttagcagcc ttgatgagaa aaacagcgtt tcagtagatt tacctggtga aatgaaagtt 120
   cttgtaagca aagaaaaaga caaagatggt aaatacagtc taatggcaac agtagacaag 180
   ctagagctta aaggaacttc tgataaaagc aacggttctg gaacacttga aggtgaaaaa 240
   tctgacaaaa gtaaagcaaa attaacaatt tctgaagatc taagtaaaac cacatttgaa 300
   attttcaaag aagatggcaa aacattagta tcaaaaaaag taaattctaa agataagtca 360
   tcaatagaag aaaaattcaa cgcaaaaggt gaattatctg aaaaaacaat actaagagca 420
   aacggaacca ggcttgaata cacagaaata aaaagcgatg gaaccggaaa agctaaagaa 480
   gctttaaaag actttgctct tgaaggaact ctagctgccg acaaaacaac attgaaagtt 540
   acagaaggca ctgttgtttt aagcaaacac attccaaact ctggagaaat aacagttgag 600
   cttaatgact ctaactctac tcaggctact aaaaaaactg gaaaatggga ttcaaatact 660
   tccactttaa caattagtgt gaatagcaaa aaaactaaaa acattgtatt tacaaaagaa 720
   gacacaataa cagtacaaaa atacgactca gcaggcacca atctagaagg caacgcagtc 780
   gaaattaaaa cacttgatga acttaaaaac gctttaaaat a                     821
<210> 136
   <211> 821
   <212> DNA
   <213> Borrelia burgdorferi
<400> 136
   atgaaaaaat atttattggg aataggtcta atattagcct taatagcatg taagcaaaat 60
   gttagcagcc ttgatgaaaa aaatagcgtt tcagtagatt tacctggtgg aatgaaagtt 120
   cttgtaagta aagaaaaaga caaagatggt aaatacagtc taatggcaac agtagaaaag 180
   cttgagctta aaggaacttc tgataaaaac aacggttctg gaacacttga aggtgaaaaa 240
   actgacaaaa gtaaagtaaa attaacaatt gctgaggatc taagtaaaac cacatttgaa 300
   atcttcaaag aagatggcaa aacattagta tcgaaaaaag taacccttaa agacaagtca 360
   tcaacagaag aaaaattcaa cgaaaagggt gaaatatctg aaaaaacaat agtaagagca 420
   aatggaacca gacttgaata cacagacata aaaagcgata aaaccggaaa agctaaagaa 480
   gttttaaaag actttactct tgaaggaact ctagctgctg acggcaaaac aacattgaaa 540
   gttacagagg gcactgttac tttaagcaag aacatttcaa aatccggaga aataacagtt 600
   gcacttgatg acactgactc tagcggcaat aaaaaatccg gaacatggga ttcaggtact 660
   tctactttaa caattagtaa aaacagacaa aaaactaaac aacttgtatt cacaaaagaa 720
   gacacaataa cagtacaaaa ctacgactca gcaggcacca atctagaagg caaagcagtc 780
   gaaattacaa cacttaaaga acttaaaaac gctttaaaat a                     821
<210> 137
   <211> 825
   <212> DNA
   <213> Artificial Sequence
<220>
   <221> CDS
   <222> (1)...(825)
   <223> Chimeric Nucleic Acid
<400> 137
   atg aaa aaa tat tta ttg gga ata ggt cta ata tta gcc tta ata gca    48
   Met Lys Lys Tyr Leu Leu Gly Ile Gly Leu Ile Leu Ala Leu Ile Ala
    1               5                  10                  15
   tgt aag caa aat gtt agc agc ctt gat gaa aaa aat agc gtt tca gta    96
   Cys Lys Gln Asn Val Ser Ser Leu Asp Glu Lys Asn Ser Val Ser Val
               20                  25                  30
   gat tta cct ggt gga atg aca gtt ctt gta agt aaa gaa aaa gac aaa   144
   Asp Leu Pro Gly Gly Met Thr Val Leu Val Ser Lys Glu Lys Asp Lys
           35                  40                  45
   gac ggt aaa tac agt cta gag gca aca gta gac aag ctt gag ctt aaa   192
   Asp Gly Lys Tyr Ser Leu Glu Ala Thr Val Asp Lys Leu Glu Leu Lys
       50                  55                  60
   gga act tct gat aaa aac aac ggt tct gga aca ctt gaa ggt gaa aaa   240
   Gly Thr Ser Asp Lys Asn Asn Gly Ser Gly Thr Leu Glu Gly Glu Lys
   65                  70                  75                  80
   act gac aaa agt aaa gta aaa tta aca att gct gat gac cta agt caa   288
   Thr Asp Lys Ser Lys Val Lys Leu Thr Ile Ala Asp Asp Leu Ser Gln
                   85                  90                  95
   act aaa ttt gaa att ttc aaa gaa gat gcc aaa aca tta gta tca aaa   336
   Thr Lys Phe Glu Ile Phe Lys Glu Asp Ala Lys Thr Leu Val Ser Lys
               100                 105                 110
   aaa gta acc ctt aaa gac aag tca tca aca gaa gaa aaa ttc aac gaa    384
   Lys Val Thr Leu Lys Asp Lys Ser Ser Thr Glu Glu Lys Phe Asn Glu
           115                 120                 125
   aag ggt gaa aca tct gaa aaa aca ata gta aga gca aat gga acc aga    432
   Lys Gly Glu Thr Ser Glu Lys Thr Ile Val Arg Ala Asn Gly Thr Arg
       130                 135                 140
   ctt gaa tac aca gac ata aaa agc gat gga tcc gga aaa gct aaa gaa    480
   Leu Glu Tyr Thr Asp Ile Lys Ser Asp Gly Ser Gly Lys Ala Lys Glu
   145                 150                 155                 160
   gtt tta aaa gac ttt act ctt gaa gga act cta gct gct gac ggc aaa    528
   Val Leu Lys Asp Phe Thr Leu Glu Gly Thr Leu Ala Ala Asp Gly Lys
                   165                 170                 175
   aca aca ttg aaa gtt aca gaa ggc act gtt gtt tta agc aag aac att    576
   Thr Thr Leu Lys Val Thr Glu Gly Thr Val Val Leu Ser Lys Asn Ile
               180                 185                 190
   tta aaa tcc gga gaa ata aca gtt gca ctt gat gac tct gac act act    624
   Leu Lys Ser Gly Glu Ile Thr Val Ala Leu Asp Asp Ser Asp Thr Thr
           195                 200                 205
   cag gct act aaa aaa act gga aaa tgg gat tca aat act tcc act tta    672
   Gln Ala Thr Lys Lys Thr Gly Lys Trp Asp Ser Asn Thr Ser Thr Leu
       210                 215                 220
   aca att agt gtg aat agc aaa aaa act aaa aac att gta ttt aca aaa    720
   Thr Ile Ser Val Asn Ser Lys Lys Thr Lys Asn Ile Val Phe Thr Lys
   225                 230                 235                 240
   gaa gac aca ata aca gta caa aaa tac gac tca gca ggc acc aat cta    768
   Glu Asp Thr Ile Thr Val Gln Lys Tyr Asp Ser Ala Gly Thr Asn Leu
                   245                 250                 255
   gaa ggc aac gca gtc gaa att aaa aca ctt gat gaa ctt aaa aac gct    816
   Glu Gly Asn Ala Val Glu Ile Lys Thr Leu Asp Glu Leu Lys Asn Ala
               260                 265                 270
   tta aaa taa                                                        825
   Leu Lys  *
<210> 138
   <211> 274
   <212> PRT
   <213> Artificial Sequence
<220>
   <223> Chimeric Protein
<400> 138
   Met Lys Lys Tyr Leu Leu Gly Ile Gly Leu Ile Leu Ala Leu Ile Ala
   1               5                   10                  15
   Cys Lys Gln Asn Val Ser Ser Leu Asp Glu Lys Asn Ser Val Ser Val
               20                  25                  30
   Asp Leu Pro Gly Gly Met Thr Val Leu Val Ser Lys Glu Lys Asp Lys
           35                  40                  45
   Asp Gly Lys Tyr Ser Leu Glu Ala Thr Val Asp Lys Leu Glu Leu Lys
       50                  55                  60
   Gly Thr Ser Asp Lys Asn Asn Gly Ser Gly Thr Leu Glu Gly Glu Lys
   65                  70                  75                  80
   Thr Asp Lys Ser Lys Val Lys Leu Thr Ile Ala Asp Asp Leu Ser Gln
                   85                  90                  95
   Thr Lys Phe Glu Ile Phe Lys Glu Asp Ala Lys Thr Leu Val Ser Lys
               100                 105                 110
   Lys Val Thr Leu Lys Asp Lys Ser Ser Thr Glu Glu Lys Phe Asn Glu
           115                 120                 125
   Lys Gly Glu Thr Ser Glu Lys Thr Ile Val Arg Ala Asn Gly Thr Arg
       130                 135                 140
   Leu Glu Tyr Thr Asp Ile Lys Ser Asp Gly Ser Gly Lys Ala Lys Glu
   145                 150                 155                 160
   Val Leu Lys Asp Phe Thr Leu Glu Gly Thr Leu Ala Ala Asp Gly Lys
                   165                 170                 175
   Thr Thr Leu Lys Val Thr Glu Gly Thr Val Val Leu Ser Lys Asn Ile
               180                 185                 190
   Leu Lys Ser Gly Glu Ile Thr Val Ala Leu Asp Asp Ser Asp Thr Thr
           195                 200                 205
   Gln Ala Thr Lys Lys Thr Gly Lys Trp Asp Ser Asn Thr Ser Thr Leu
       210                 215                 220
   Thr Ile Ser Val Asn Ser Lys Lys Thr Lys Asn Ile Val Phe Thr Lys
   225                 230                 235                 240
   Glu Asp Thr Ile Thr Val Gln Lys Tyr Asp Ser Ala Gly Thr Asn Leu
                   245                 250                 255
   Glu Gly Asn Ala Val Glu Ile Lys Thr Leu Asp Glu Leu Lys Asn Ala
               260                 265                 270
   Leu Lys
<210> 139
   <211> 822
   <212> DNA
   <213> Artificial Sequence
<220>
   <221> CDS
   <222> (1)...(822)
   <223> Chimeric Nucleic Acid
<400> 139
   atg aaa aaa tat tta ttg gga ata ggt cta ata tta gcc tta ata gca      48
   Met Lys Lys Tyr Leu Leu Gly Ile Gly Leu Ile Leu Ala Leu Ile Ala
    1               5                  10                  15
   tgc aag caa aat gtt agc agc ctt gat gaa aaa aac agc gct tca gta      96
   Cys Lys Gln Asn Val Ser Ser Leu Asp Glu Lys Asn Ser Ala Ser Val
               20                  25                  30
   gat ttg cct ggt gag atg aaa gtt ctt gta agt aaa gaa aaa gac aaa      144
   Asp Leu Pro Gly Glu Met Lys Val Leu Val Ser Lys Glu Lys Asp Lys
           35                  40                  45
   gac ggt aag tac agt cta aag gca aca gta gac aag att gag cta aaa      192
   Asp Gly Lys Tyr Ser Leu Lys Ala Thr Val Asp Lys Ile Glu Leu Lys
       50                  55                  60
   gga act tct gat aaa gac aat ggt tct gga gtg ctt gaa ggt aca aaa      240
   Gly Thr Ser Asp Lys Asp Asn Gly Ser Gly Val Leu Glu Gly Thr Lys
   65                  70                  75                  80
   gat gac aaa agt aaa gca aaa tta aca att gct gac gat cta agt aaa      288
   Asp Asp Lys Ser Lys Ala Lys Leu Thr Ile Ala Asp Asp Leu Ser Lys
                   85                  90                  95
   acc aca ttc gaa ctt tta aaa gaa gat ggc aaa aca tta gtg tca aga      336
   Thr Thr Phe Glu Leu Leu Lys Glu Asp Gly Lys Thr Leu Val Ser Arg
               100                 105                 110
   aaa gta agt tct aga gac aaa aca tca aca gat gaa atg ttc aat gaa      384
   Lys Val Ser Ser Arg Asp Lys Thr Ser Thr Asp Glu Met Phe Asn Glu
           115                 120                 125
   aaa ggt gaa ttg tct gca aaa acc atg aca aga gaa aat gga acc aaa      432
   Lys Gly Glu Leu Ser Ala Lys Thr Met Thr Arg Glu Asn Gly Thr Lys
       130                 135                 140
   ctt gaa tat aca gaa atg aaa agc gat gga acc gga aaa gct aaa gaa      480
   Leu Glu Tyr Thr Glu Met Lys Ser Asp Gly Thr Gly Lys Ala Lys Glu
   145                 150                 155                 160
   gtt tta aaa aag ttt act ctt gaa gga aaa gta gct aat gat aaa gta      528
   Val Leu Lys Lys Phe Thr Leu Glu Gly Lys Val Ala Asn Asp Lys Val
                   165                 170                 175
   aca ttg gaa gta aaa gaa gga acc gtt act tta agt aag gaa att gca      576
   Thr Leu Glu Val Lys Glu Gly Thr Val Thr Leu Ser Lys Glu Ile Ala
               180                 185                 190
   aaa tct gga gaa gta aca gtt gct ctt aat gac act aac act act cag      624
   Lys Ser Gly Glu Val Thr Val Ala Leu Asn Asp Thr Asn Thr Thr Gln
           195                 200                 205
   gct act aaa aaa act ggc gca tgg gat tca aaa act tct act tta aca      672
   Ala Thr Lys Lys Thr Gly Ala Trp Asp Ser Lys Thr Ser Thr Leu Thr
       210                 215                 220
   att agt gtt aac agc aaa aaa act aca caa ctt gtg ttt act aaa caa      720
   Ile Ser Val Asn Ser Lys Lys Thr Thr Gln Leu Val Phe Thr Lys Gln
   225                 230                 235                 240
   gac aca ata act gta caa aaa tac gac tcc gca ggt acc aat tta gaa      768
   Asp Thr Ile Thr Val Gln Lys Tyr Asp Ser Ala Gly Thr Asn Leu Glu
                   245                 250                 255
   ggc aca gca gtc gaa att aaa aca ctt gat gaa ctt aaa aac gct tta      816
   Gly Thr Ala Val Glu Ile Lys Thr Leu Asp Glu Leu Lys Asn Ala Leu
               260                 265                 270
   aaa taa                                                              822
   Lys  *
<210> 140
   <211> 273
   <212> PRT
   <213> Artificial Sequence
<220>
   <223> Chimeric Protein
<400> 140
   Met Lys Lys Tyr Leu Leu Gly Ile Gly Leu Ile Leu Ala Leu Ile Ala
    1               5                  10                  15
   Cys Lys Gln Asn Val Ser Ser Leu Asp Glu Lys Asn Ser Ala Ser Val
               20                  25                  30
   Asp Leu Pro Gly Glu Met Lys Val Leu Val Ser Lys Glu Lys Asp Lys
           35                  40                  45
   Asp Gly Lys Tyr Ser Leu Lys Ala Thr Val Asp Lys Ile Glu Leu Lys
       50                  55                  60
   Gly Thr Ser Asp Lys Asp Asn Gly Ser Gly Val Leu Glu Gly Thr Lys
   65                  70                  75                  80
   Asp Asp Lys Ser Lys Ala Lys Leu Thr Ile Ala Asp Asp Leu Ser Lys
                   85                  90                  95
   Thr Thr Phe Glu Leu Leu Lys Glu Asp Gly Lys Thr Leu Val Ser Arg
               100                 105                 110
   Lys Val Ser Ser Arg Asp Lys Thr Ser Thr Asp Glu Met Phe Asn Glu
           115                 120                 125
   Lys Gly Glu Leu Ser Ala Lys Thr Met Thr Arg Glu Asn Gly Thr Lys
       130                 135                 140
   Leu Glu Tyr Thr Glu Met Lys Ser Asp Gly Thr Gly Lys Ala Lys Glu
   145                 150                 155                 160
   Val Leu Lys Lys Phe Thr Leu Glu Gly Lys Val Ala Asn Asp Lys Val
                   165                 170                 175
   Thr Leu Glu Val Lys Glu Gly Thr Val Thr Leu Ser Lys Glu Ile Ala
               180                 185                 190
   Lys Ser Gly Glu Val Thr Val Ala Leu Asn Asp Thr Asn Thr Thr Gln
           195                 200                 205
   Ala Thr Lys Lys Thr Gly Ala Trp Asp Ser Lys Thr Ser Thr Leu Thr
       210                 215                 220
   Ile Ser Val Asn Ser Lys Lys Thr Thr Gln Leu Val Phe Thr Lys Gln
   225                 230                 235                 240
   Asp Thr Ile Thr Val Gln Lys Tyr Asp Ser Ala Gly Thr Asn Leu Glu
                   245                 250                 255
   Gly Thr Ala Val Glu Ile Lys Thr Leu Asp Glu Leu Lys Asn Ala Leu
               260                 265                 270
   Lys
<210> 141
   <211> 822
   <212> DNA
   <213> Artificial Sequence
<220>
   <221> CDS
   <222> (1)...(822)
   <223> Chimeric Nucleic Acid
<400> 141
   atg aaa aaa tat tta ttg gga ata ggt cta ata tta gcc tta ata gca    48
   Met Lys Lys Tyr Leu Leu Gly Ile Gly Leu Ile Leu Ala Leu Ile Ala
    1               5                  10                  15
   tgc aag caa aat gtt agc agc ctt gat gaa aaa aac agc gct tca gta      96
   Cys Lys Gln Asn Val Ser Ser Leu Asp Glu Lys Asn Ser Ala Ser Val
                   20                  25                  30
   gat ttg cct ggt gag atg aaa gtt ctt gta agt aaa gaa aaa gac aaa      144
   Asp Leu Pro Gly Glu Met Lys Val Leu Val Ser Lys Glu Lys Asp Lys
               35                  40                  45
   gac ggt aag tac agt cta aag gca aca gta gac aag att gag cta aaa      192
   Asp Gly Lys Tyr Ser Leu Lys Ala Thr Val Asp Lys Ile Glu Leu Lys
           50                  55                  60
   gga act tct gat aaa gac aat ggt tct gga gtg ctt gaa ggt aca aaa      240
   Gly Thr Ser Asp Lys Asp Asn Gly Ser Gly Val Leu Glu Gly Thr Lys
   65                  70                  75                  80
   gat gac aaa agt aaa gca aaa tta aca att gct gac gat cta agt aaa      288
   Asp Asp Lys Ser Lys Ala Lys Leu Thr Ile Ala Asp Asp Leu Ser Lys
                   85                  90                  95
   acc aca ttc gaa ctt tta aaa gaa gat ggc aaa aca tta gtg tca aga      336
   Thr Thr Phe Glu Leu Leu Lys Glu Asp Gly Lys Thr Leu Val Ser Arg
               100                 105                 110
   aaa gta agt tct aga gac aaa aca tca aca gat gaa atg ttc aat gaa      384
   Lys Val Ser Ser Arg Asp Lys Thr Ser Thr Asp Glu Met Phe Asn Glu
           115                 120                 125
   aaa ggt gaa ttg tct gca aaa acc atg aca aga gaa aat gga acc aaa      432
   Lys Gly Glu Leu Ser Ala Lys Thr Met Thr Arg Glu Asn Gly Thr Lys
       130                 135                 140
   ctt gaa tat aca gaa atg aaa agc gat gga acc gga aaa gct aaa gaa      480
   Leu Glu Tyr Thr Glu Met Lys Ser Asp Gly Thr Gly Lys Ala Lys Glu
   145                 150                 155                 160
   gtt tta aaa aag ttt act ctt gaa gga aaa gta gct aat gat aaa gta      528
   Val Leu Lys Lys Phe Thr Leu Glu Gly Lys Val Ala Asn Asp Lys Val
                   165                 170                 175
   aca ttg gaa gta aaa gaa gga acc gtt act tta agt aag gaa att tca      576
   Thr Leu Glu Val Lys Glu Gly Thr Val Thr Leu Ser Lys Glu Ile Ser
               180                 185                 190
   aaa tct ggg gaa gtt tca gtt gaa ctt aat gac act gac agt agt gct      624
   Lys Ser Gly Glu Val Ser Val Glu Leu Asn Asp Thr Asp Ser Ser Ala
           195                 200                 205
   gct act aaa aaa act gca gct tgg aat tca aaa act tcc act tta aca      672
   Ala Thr Lys Lys Thr Ala Ala Trp Asn Ser Lys Thr Ser Thr Leu Thr
       210                 215                 220
   att agt gtg aat agc caa aaa acc aaa aac ctt gta ttc aca aaa gaa      720
   Ile Ser Val Asn Ser Gln Lys Thr Lys Asn Leu Val Phe Thr Lys Glu
   225                 230                 235                 240
   gac aca ata aca gta caa aaa tac gac tca gca ggc acc aat cta gaa      768
   Asp Thr Ile Thr Val Gln Lys Tyr Asp Ser Ala Gly Thr Asn Leu Glu
                   245                 250                 255
   ggc aaa gca gtc gaa att aca aca ctt aaa gaa ctt aaa aac gct tta      816
   Gly Lys Ala Val Glu Ile Thr Thr Leu Lys Glu Leu Lys Asn Ala Leu
               260                 265                 270
   aaa taa                                                              822
   Lys  *
<210> 142
   <211> 273
   <212> PRT
   <213> Artificial Sequence
<220>
   <223> Chimeric Protein
<400> 142
   Met Lys Lys Tyr Leu Leu Gly Ile Gly Leu Ile Leu Ala Leu Ile Ala
    1               5                  10                  15
   Cys Lys Gln Asn Val Ser Ser Leu Asp Glu Lys Asn Ser Ala Ser Val
               20                  25                  30
   Asp Leu Pro Gly Glu Met Lys Val Leu Val Ser Lys Glu Lys Asp Lys
           35                  40                  45
   Asp Gly Lys Tyr Ser Leu Lys Ala Thr Val Asp Lys Ile Glu Leu Lys
       50                  55                  60
   Gly Thr Ser Asp Lys Asp Asn Gly Ser Gly Val Leu Glu Gly Thr Lys
   65                  70                  75                  80
   Asp Asp Lys Ser Lys Ala Lys Leu Thr Ile Ala Asp Asp Leu Ser Lys
                   85                  90                  95
   Thr Thr Phe Glu Leu Leu Lys Glu Asp Gly Lys Thr Leu Val Ser Arg
               100                 105                 110
   Lys Val Ser Ser Arg Asp Lys Thr Ser Thr Asp Glu Met Phe Asn Glu
           115                 120                 125
   Lys Gly Glu Leu Ser Ala Lys Thr Met Thr Arg Glu Asn Gly Thr Lys
       130                 135                 140
   Leu Glu Tyr Thr Glu Met Lys Ser Asp Gly Thr Gly Lys Ala Lys Glu
   145                 150                 155                 160
   Val Leu Lys Lys Phe Thr Leu Glu Gly Lys Val Ala Asn Asp Lys Val
                   165                 170                 175
   Thr Leu Glu Val Lys Glu Gly Thr Val Thr Leu Ser Lys Glu Ile Ser
               180                 185                 190
   Lys Ser Gly Glu Val Ser Val Glu Leu Asn Asp Thr Asp Ser Ser Ala
           195                 200                 205
   Ala Thr Lys Lys Thr Ala Ala Trp Asn Ser Lys Thr Ser Thr Leu Thr
       210                 215                 220
   Ile Ser Val Asn Ser Gln Lys Thr Lys Asn Leu Val Phe Thr Lys Glu
   225                 230                 235                 240
   Asp Thr Ile Thr Val Gln Lys Tyr Asp Ser Ala Gly Thr Asn Leu Glu
                   245                 250                 255
   Gly Lys Ala Val Glu Ile Thr Thr Leu Lys Glu Leu Lys Asn Ala Leu
               260                 265                 270
   Lys
<210> 143
   <211> 822
   <212> DNA
   <213> Artificial Sequence
<220>
   <221> CDS
   <222> (1)...(822)
   <223> Chimeric Nucleic Acid
<400> 143
   atg aaa aaa tat tta ttg gga ata ggt cta ata tta gcc tta ata gca      48
   Met Lys Lys Tyr Leu Leu Gly Ile Gly Leu Ile Leu Ala Leu Ile Ala
   1               5                   10                  15
   tgt aag caa aat gtt agc agc ctt gac gag aaa aac agc gtt tca gta      96
   Cys Lys Gln Asn Val Ser Ser Leu Asp Glu Lys Asn Ser Val Ser Val
               20                  25                  30
   gat ttg cct ggt gaa atg aaa gtt ctt gta agc aaa gaa aaa aac aaa     144
   Asp Leu Pro Gly Glu Met Lys Val Leu Val Ser Lys Glu Lys Asn Lys
           35                  40                  45
   gac ggc aag tac gat cta att gca aca gta gac aag ctt gag ctt aaa     192
   Asp Gly Lys Tyr Asp Leu Ile Ala Thr Val Asp Lys Leu Glu Leu Lys
       50                  55                  60
   gga act tct gat aaa aac aat gga tct gga gta ctt gaa ggc gta aaa     240
   Gly Thr Ser Asp Lys Asn Asn Gly Ser Gly Val Leu Glu Gly Val Lys
   65                  70                  75                  80
   gct gac aaa agt aaa gta aaa tta aca att tct gac gat cta ggt caa     288
   Ala Asp Lys Ser Lys Val Lys Leu Thr Ile Ser Asp Asp Leu Gly Gln
                   85                  90                  95
   acc aca ctt gaa gtt ttc aaa gaa gat ggc aaa aca cta gta tca aaa     336
   Thr Thr Leu Glu Val Phe Lys Glu Asp Gly Lys Thr Leu Val Ser Lys
               100                 105                 110
   aaa gta act tcc aaa gac aag tca tca aca gaa gaa aaa ttc aat gaa     384
   Lys Val Thr Ser Lys Asp Lys Ser Ser Thr Glu Glu Lys Phe Asn Glu
           115                 120                 125
   aaa ggt gaa gta tct gaa aaa ata ata aca aga gca aat gga acc aaa     432
   Lys Gly Glu Val Ser Glu Lys Ile Ile Thr Arg Ala Asn Gly Thr Lys
       130                 135                 140
   ctt gaa tat aca gaa atg aaa agc gat gga acc gga aaa gct aaa gaa     480
   Leu Glu Tyr Thr Glu Met Lys Ser Asp Gly Thr Gly Lys Ala Lys Glu
   145                 150                 155                 160
   gtt tta aaa aag ttt act ctt gaa gga aaa gta gct aat gat aaa gta     528
   Val Leu Lys Lys Phe Thr Leu Glu Gly Lys Val Ala Asn Asp Lys Val
                   165                 170                 175
   aca ttg gaa gta aaa gaa gga acc gtt act tta agt aag gaa att tca     576
   Thr Leu Glu Val Lys Glu Gly Thr Val Thr Leu Ser Lys Glu Ile Ser
               180                 185                 190
   aaa tct ggg gaa gtt tca gtt gaa ctt aat gac act gac agt agt gct    624
   Lys Ser Gly Glu Val Ser Val Glu Leu Asn Asp Thr Asp Ser Ser Ala
           195                 200                 205
   gct act aaa aaa act gca gct tgg aat tca aaa act tcc act tta aca    672
   Ala Thr Lys Lys Thr Ala Ala Trp Asn Ser Lys Thr Ser Thr Leu Thr
       210                 215                 220
   att agt gtg aat agc caa aaa acc aaa aac ctt gta ttc aca aaa gaa    720
   Ile Ser Val Asn Ser Gln Lys Thr Lys Asn Leu Val Phe Thr Lys Glu
   225                 230                 235                 240
   gac aca ata aca gta caa aaa tac gac tca gca ggc acc aat cta gaa    768
   Asp Thr Ile Thr Val Gln Lys Tyr Asp Ser Ala Gly Thr Asn Leu Glu
                   245                 250                 255
   ggc aaa gca gtc gaa att aca aca ctt aaa gaa ctt aaa aac gct tta    816
   Gly Lys Ala Val Glu Ile Thr Thr Leu Lys Glu Leu Lys Asn Ala Leu
               260                 265                 270
   aaa taa                                                            822
   Lys  *
<210> 144
   <211> 273
   <212> PRT
   <213> Artificial Sequence
<220>
   <223> Chimeric Protein
<400> 144
   Met Lys Lys Tyr Leu Leu Gly Ile Gly Leu Ile Leu Ala Leu Ile Ala
    1               5                  10                  15
   Cys Lys Gln Asn Val Ser Ser Leu Asp Glu Lys Asn Ser Val Ser Val
               20                  25                  30
   Asp Leu Pro Gly Glu Met Lys Val Leu Val Ser Lys Glu Lys Asn Lys
           35                  40                  45
   Asp Gly Lys Tyr Asp Leu Ile Ala Thr Val Asp Lys Leu Glu Leu Lys
       50                  55                  60
   Gly Thr Ser Asp Lys Asn Asn Gly Ser Gly Val Leu Glu Gly Val Lys
   65                  70                  75                  80
   Ala Asp Lys Ser Lys Val Lys Leu Thr Ile Ser Asp Asp Leu Gly Gln
                   85                  90                  95
   Thr Thr Leu Glu Val Phe Lys Glu Asp Gly Lys Thr Leu Val Ser Lys
               100                 105                 110
   Lys Val Thr Ser Lys Asp Lys Ser Ser Thr Glu Glu Lys Phe Asn Glu
           115                 120                 125
   Lys Gly Glu Val Ser Glu Lys Ile Ile Thr Arg Ala Asn Gly Thr Lys
       130                 135                 140
   Leu Glu Tyr Thr Glu Met Lys Ser Asp Gly Thr Gly Lys Ala Lys Glu
   145                 150                 155                 160
   Val Leu Lys Lys Phe Thr Leu Glu Gly Lys Val Ala Asn Asp Lys Val
                   165                 170                 175
   Thr Leu Glu Val Lys Glu Gly Thr Val Thr Leu Ser Lys Glu Ile Ser
               180                 185                 190
   Lys Ser Gly Glu Val Ser Val Glu Leu Asn Asp Thr Asp Ser Ser Ala
           195                 200                 205
   Ala Thr Lys Lys Thr Ala Ala Trp Asn Ser Lys Thr Ser Thr Leu Thr
       210                 215                 220
   Ile Ser Val Asn Ser Gln Lys Thr Lys Asn Leu Val Phe Thr Lys Glu
   225                 230                 235                 240
   Asp Thr Ile Thr Val Gln Lys Tyr Asp Ser Ala Gly Thr Asn Leu Glu
                   245                 250                 255
   Gly Lys Ala Val Glu Ile Thr Thr Leu Lys Glu Leu Lys Asn Ala Leu
               260                 265                 270
   Lys
<210> 145
   <211> 1362
   <212> DNA
   <213> Artificial Sequence
<220>
   <221> CDS
   <222> (1)...(1362)
   <223> Chimeric nucleic acid
<400> 145
   atg gct tgt aat aat tca ggg aaa gat ggg aat aca tct gca aat tct      48
   Met Ala Cys Asn Asn Ser Gly Lys Asp Gly Asn Thr Ser Ala Asn Ser
   1               5                   10                  15
   gct gat gag tct gtt aaa ggg cct aat ctt aca gaa ata aat aaa aaa      96
   Ala Asp Glu Ser Val Lys Gly Pro Asn Leu Thr Glu Ile Asn Lys Lys
               20                  25                  30
   att acg gat tct aat gcg gtt tta ctt gct gtg aaa gag gtt gaa gcg     144
   Ile Thr Asp Ser Asn Ala Val Leu Leu Ala Val Lys Glu Val Glu Ala
           35                  40                  45
   ttg ctg tca tct ata gat gaa att gct gct aaa gct att ggt aaa aaa     192
   Leu Leu Ser Ser Ile Asp Glu Ile Ala Ala Lys Ala Ile Gly Lys Lys
       50                  55                  60
   ata cac caa aat aat ggt ttg gat acc gaa aat aat cac aat gga tca     240
   Ile His Gln Asn Asn Gly Leu Asp Thr Glu Asn Asn His Asn Gly Ser
   65                  70                  75                  80
   ttg tta gcg gga gct tat gca ata tca acc cta ata aaa caa aaa tta     288
   Leu Leu Ala Gly Ala Tyr Ala Ile Ser Thr Leu Ile Lys Gln Lys Leu
                   85                  90                  95
   gat gga ttg aaa aat gaa gga tta aag gaa aaa att gat gcg gct aag     336
   Asp Gly Leu Lys Asn Glu Gly Leu Lys Glu Lys Ile Asp Ala Ala Lys
               100                 105                 110
   aaa tgt tct gaa aca ttt act aat aaa tta aaa gaa aaa cac aca gat     384
   Lys Cys Ser Glu Thr Phe Thr Asn Lys Leu Lys Glu Lys His Thr Asp
           115                 120                 125
   ctt ggt aaa gaa ggt gtt act gat gct gat gca aaa gaa gcc att tta     432
   Leu Gly Lys Glu Gly Val Thr Asp Ala Asp Ala Lys Glu Ala Ile Leu
       130                 135                 140
   aaa gca aat ggt act aaa act aaa ggt gct gaa gaa ctt gga aaa tta     480
   Lys Ala Asn Gly Thr Lys Thr Lys Gly Ala Glu Glu Leu Gly Lys Leu
   145                 150                 155                 160
   ttt gaa tca gta gag gtc ttg tca aaa gca gct aaa gag atg ctt gct      528
   Phe Glu Ser Val Glu Val Leu Ser Lys Ala Ala Lys Glu Met Leu Ala
                   165                 170                 175
   aat tca gtt aaa gag ctt aca agc cct gtt gtg gca gaa agt cca aaa      576
   Asn Ser Val Lys Glu Leu Thr Ser Pro Val Val Ala Glu Ser Pro Lys
               180                 185                 190
   aaa cct tcc atg gcc aag caa aat gtt agc agc ctt gac gag aaa aac      624
   Lys Pro Ser Met Ala Lys Gln Asn Val Ser Ser Leu Asp Glu Lys Asn
           195                 200                 205
   agc gtt tca gta gat ttg cct ggt gaa atg aaa gtt ctt gta agc aaa      672
   Ser Val Ser Val Asp Leu Pro Gly Glu Met Lys Val Leu Val Ser Lys
       210                 215                 220
   gaa aaa aac aaa gac ggc aag tac gat cta att gca aca gta gac aag      720
   Glu Lys Asn Lys Asp Gly Lys Tyr Asp Leu Ile Ala Thr Val Asp Lys
   225                 230                 235                 240
   ctt gag ctt aaa gga act tct gat aaa aac aat gga tct gga gta ctt      768
   Leu Glu Leu Lys Gly Thr Ser Asp Lys Asn Asn Gly Ser Gly Val Leu
                   245                 250                 255
   gaa ggc gta aaa gct gac aaa agt aaa gta aaa tta aca att tct gac      816
   Glu Gly Val Lys Ala Asp Lys Ser Lys Val Lys Leu Thr Ile Ser Asp
               260                 265                 270
   gat cta ggt caa acc aca ctt gaa gtt ttc aaa gaa gat ggc aaa aca      864
   Asp Leu Gly Gln Thr Thr Leu Glu Val Phe Lys Glu Asp Gly Lys Thr
           275                 280                 285
   cta gta tca aaa aaa gta act tcc aaa gac aag tca tca aca gaa gaa      912
   Leu Val Ser Lys Lys Val Thr Ser Lys Asp Lys Ser Ser Thr Glu Glu
       290                 295                 300
   aaa ttc aat gaa aaa ggt gaa gta tct gaa aaa ata ata aca aga gca      960
   Lys Phe Asn Glu Lys Gly Glu Val Ser Glu Lys Ile Ile Thr Arg Ala
   305                 310                 315                 320
   gac gga acc aga ctt gaa tac aca gga att aaa agc gat gga tct gga     1008
   Asp Gly Thr Arg Leu Glu Tyr Thr Gly Ile Lys Ser Asp Gly Ser Gly
                   325                 330                 335
   aaa gct aaa gag gtt tta aaa ggc tat gtt ctt gaa gga act cta act     1056
   Lys Ala Lys Glu Val Leu Lys Gly Tyr Val Leu Glu Gly Thr Leu Thr
               340                 345                 350
   gct gaa aaa aca aca ttg gtg gtt aaa gaa gga act gtt act tta agc     1104
   Ala Glu Lys Thr Thr Leu Val Val Lys Glu Gly Thr Val Thr Leu Ser
           355                 360                 365
   aaa aat att tca aaa tct ggg gaa gtt tca gtt gaa ctt aat gac act     1152
   Lys Asn Ile Ser Lys Ser Gly Glu Val Ser Val Glu Leu Asn Asp Thr
       370                 375                 380
   gac agt agt gct gct act aaa aaa act gca gct tgg aat tca ggc act     1200
   Asp Ser Ser Ala Ala Thr Lys Lys Thr Ala Ala Trp Asn Ser Gly Thr
   385                 390                 395                 400
   tca act tta aca att act gta aac agt aaa aaa act aaa gac ctt gtg    1248
   Ser Thr Leu Thr Ile Thr Val Asn Ser Lys Lys Thr Lys Asp Leu Val
                   405                 410                 415
   ttt aca aaa gaa aac aca att aca gta caa caa tac gac tca aat ggc    1296
   Phe Thr Lys Glu Asn Thr Ile Thr Val Gln Gln Tyr Asp Ser Asn Gly
               420                 425                 430
   acc aaa tta gag ggg tca gca gtt gaa att aca aaa ctt gat gaa att    1344
   Thr Lys Leu Glu Gly Ser Ala Val Glu Ile Thr Lys Leu Asp Glu Ile
           435                 440                 445
   aaa aac gct tta aaa taa                                            1362
   Lys Asn Ala Leu Lys  *
       450
<210> 146
   <211> 453
   <212> PRT
   <213> Artificial Sequence
<220>
   <223> Chimeric Protein
<400> 146
   Met Ala Cys Asn Asn Ser Gly Lys Asp Gly Asn Thr Ser Ala Asn Ser
   1               5                   10                  15
   Ala Asp Glu Ser Val Lys Gly Pro Asn Leu Thr Glu Ile Asn Lys Lys
               20                  25                  30
   Ile Thr Asp Ser Asn Ala Val Leu Leu Ala Val Lys Glu Val Glu Ala
           35                  40                  45
   Leu Leu Ser Ser Ile Asp Glu Ile Ala Ala Lys Ala Ile Gly Lys Lys
       50                  55                  60
   Ile His Gln Asn Asn Gly Leu Asp Thr Glu Asn Asn His Asn Gly Ser
   65                  70                  75                  80
   Leu Leu Ala Gly Ala Tyr Ala Ile Ser Thr Leu Ile Lys Gln Lys Leu
                   85                  90                  95
   Asp Gly Leu Lys Asn Glu Gly Leu Lys Glu Lys Ile Asp Ala Ala Lys
               100                 105                 110
   Lys Cys Ser Glu Thr Phe Thr Asn Lys Leu Lys Glu Lys His Thr Asp
           115                 120                 125
   Leu Gly Lys Glu Gly Val Thr Asp Ala Asp Ala Lys Glu Ala Ile Leu
       130                 135                 140
   Lys Ala Asn Gly Thr Lys Thr Lys Gly Ala Glu Glu Leu Gly Lys Leu
   145                 150                 155                 160
   Phe Glu Ser Val Glu Val Leu Ser Lys Ala Ala Lys Glu Met Leu Ala
                   165                 170                 175
   Asn Ser Val Lys Glu Leu Thr Ser Pro Val Val Ala Glu Ser Pro Lys
               180                 185                 190
   Lys Pro Ser Met Ala Lys Gln Asn Val Ser Ser Leu Asp Glu Lys Asn
           195                 200                 205
   Ser Val Ser Val Asp Leu Pro Gly Glu Met Lys Val Leu Val Ser Lys
       210                 215                 220
   Glu Lys Asn Lys Asp Gly Lys Tyr Asp Leu Ile Ala Thr Val Asp Lys
   225                 230                 235                 240
   Leu Glu Leu Lys Gly Thr Ser Asp Lys Asn Asn Gly Ser Gly Val Leu
                   245                 250                 255
   Glu Gly Val Lys Ala Asp Lys Ser Lys Val Lys Leu Thr Ile Ser Asp
               260                 265                 270
   Asp Leu Gly Gln Thr Thr Leu Glu Val Phe Lys Glu Asp Gly Lys Thr
           275                 280                 285
   Leu Val Ser Lys Lys Val Thr Ser Lys Asp Lys Ser Ser Thr Glu Glu
       290                 295                 300
   Lys Phe Asn Glu Lys Gly Glu Val Ser Glu Lys Ile Ile Thr Arg Ala
   305                 310                 315                 320
   Asp Gly Thr Arg Leu Glu Tyr Thr Gly Ile Lys Ser Asp Gly Ser Gly
                   325                 330                 335
   Lys Ala Lys Glu Val Leu Lys Gly Tyr Val Leu Glu Gly Thr Leu Thr
               340                 345                 350
   Ala Glu Lys Thr Thr Leu Val Val Lys Glu Gly Thr Val Thr Leu Ser
           355                 360                 365
   Lys Asn Ile Ser Lys Ser Gly Glu Val Ser Val Glu Leu Asn Asp Thr
       370                 375                 380
   Asp Ser Ser Ala Ala Thr Lys Lys Thr Ala Ala Trp Asn Ser Gly Thr
   385                 390                 395                 400
   Ser Thr Leu Thr Ile Thr Val Asn Ser Lys Lys Thr Lys Asp Leu Val
                   405                 410                 415
   Phe Thr Lys Glu Asn Thr Ile Thr Val Gln Gln Tyr Asp Ser Asn Gly
               420                 425                 430
   Thr Lys Leu Glu Gly Ser Ala Val Glu Ile Thr Lys Leu Asp Glu Ile
           435                 440                 445
   Lys Asn Ala Leu Lys
       450
<210> 147
   <211> 1353
   <212> DNA
   <213> Artificial Sequence
<220>
   <221> CDS
   <222> (1)...(1353)
   <223> Chimeric nucleic acid
<400> 147
   atg gct tgt aat aat tca ggg aaa gat ggg aat aca tct gca aat tct     48
   Met Ala Cys Asn Asn Ser Gly Lys Asp Gly Asn Thr Ser Ala Asn Ser
    1               5                  10                  15
   gct gat gag tct gtt aaa ggg cct aat ctt aca gaa ata aat aaa aaa     96
   Ala Asp Glu Ser Val Lys Gly Pro Asn Leu Thr Glu Ile Asn Lys Lys
               20                  25                  30
   att acg gat tct aat gcg gtt tta ctt gct gtg aaa gag gtt gaa gcg    144
   Ile Thr Asp Ser Asn Ala Val Leu Leu Ala Val Lys Glu Val Glu Ala
           35                  40                  45
   ttg ctg tca tct ata gat gaa att gct gct aaa gct att ggt aaa aaa    192
   Leu Leu Ser Ser Ile Asp Glu Ile Ala Ala Lys Ala Ile Gly Lys Lys
       50                  55                  60
   ata cac caa aat aat ggt ttg gat acc gaa aat aat cac aat gga tca    240
   Ile His Gln Asn Asn Gly Leu Asp Thr Glu Asn Asn His Asn Gly Ser
   65                  70                  75                  80
   ttg tta gcg gga gct tat gca ata tca acc cta ata aaa caa aaa tta    288
   Leu Leu Ala Gly Ala Tyr Ala Ile Ser Thr Leu Ile Lys Gln Lys Leu
                   85                  90                  95
   gat gga ttg aaa aat gaa gga tta aag gaa aaa att gat gcg gct aag     336
   Asp Gly Leu Lys Asn Glu Gly Leu Lys Glu Lys Ile Asp Ala Ala Lys
               100                 105                 110
   aaa tgt tct gaa aca ttt act aat aaa tta aaa gaa aaa cac aca gat     384
   Lys Cys Ser Glu Thr Phe Thr Asn Lys Leu Lys Glu Lys His Thr Asp
           115                 120                 125
   ctt ggt aaa gaa ggt gtt act gat gct gat gca aaa gaa gcc att tta     432
   Leu Gly Lys Glu Gly Val Thr Asp Ala Asp Ala Lys Glu Ala Ile Leu
       130                 135                 140
   aaa gca aat ggt act aaa act aaa ggt gct gaa gaa ctt gga aaa tta     480
   Lys Ala Asn Gly Thr Lys Thr Lys Gly Ala Glu Glu Leu Gly Lys Leu
   145                 150                 155                 160
   ttt gaa tca gta gag gtc ttg tca aaa gca gct aaa gag atg ctt gct     528
   Phe Glu Ser Val Glu Val Leu Ser Lys Ala Ala Lys Glu Met Leu Ala
                   165                 170                 175
   aat tca gtt aaa gag ctt aca agc cct gtt gtg gca gaa agt cca gcc     576
   Asn Ser Val Lys Glu Leu Thr Ser Pro Val Val Ala Glu Ser Pro Ala
               180                 185                 190
   atg gcc aag caa aat gtt agc agc ctt gac gag aaa aac agc gtt tca     624
   Met Ala Lys Gln Asn Val Ser Ser Leu Asp Glu Lys Asn Ser Val Ser
           195                 200                 205
   gta gat ttg cct ggt gaa atg aaa gtt ctt gta agc aaa gaa aaa aac     672
   Val Asp Leu Pro Gly Glu Met Lys Val Leu Val Ser Lys Glu Lys Asn
       210                 215                 220
   aaa gac ggc aag tac gat cta att gca aca gta gac aag ctt gag ctt     720
   Lys Asp Gly Lys Tyr Asp Leu Ile Ala Thr Val Asp Lys Leu Glu Leu
   225                 230                 235                 240
   aaa gga act tct gat aaa aac aat gga tct gga gta ctt gaa ggc gta     768
   Lys Gly Thr Ser Asp Lys Asn Asn Gly Ser Gly Val Leu Glu Gly Val
                   245                 250                 255
   aaa gct gac aaa agt aaa gta aaa tta aca att tct gac gat cta ggt     816
   Lys Ala Asp Lys Ser Lys Val Lys Leu Thr Ile Ser Asp Asp Leu Gly
               260                 265                 270
   caa acc aca ctt gaa gtt ttc aaa gaa gat ggc aaa aca cta gta tca     864
   Gln Thr Thr Leu Glu Val Phe Lys Glu Asp Gly Lys Thr Leu Val Ser
           275                 280                 285
   aaa aaa gta act tcc aaa gac aag tca tca aca gaa gaa aaa ttc aat     912
   Lys Lys Val Thr Ser Lys Asp Lys Ser Ser Thr Glu Glu Lys Phe Asn
       290                 295                 300
   gaa aaa ggt gaa gta tct gaa aaa ata ata aca aga gca gac gga acc     960
   Glu Lys Gly Glu Val Ser Glu Lys Ile Ile Thr Arg Ala Asp Gly Thr
   305                 310                 315                 320
   aga ctt gaa tac aca gga att aaa agc gat gga tct gga aaa gct aaa    1008
   Arg Leu Glu Tyr Thr Gly Ile Lys Ser Asp Gly Ser Gly Lys Ala Lys
                   325                 330                 335
   gag gtt tta aaa ggc tat gtt ctt gaa gga act cta act gct gaa aaa      1056
   Glu Val Leu Lys Gly Tyr Val Leu Glu Gly Thr Leu Thr Ala Glu Lys
               340                 345                 350
   aca aca ttg gtg gtt aaa gaa gga act gtt act tta agc aaa aat att      1104
   Thr Thr Leu Val Val Lys Glu Gly Thr Val Thr Leu Ser Lys Asn Ile
           355                 360                 365
   tca aaa tct ggg gaa gtt tca gtt gaa ctt aat gac act gac agt agt      1152
   Ser Lys Ser Gly Glu Val Ser Val Glu Leu Asn Asp Thr Asp Ser Ser
       370                 375                 380
   gct gct act aaa aaa act gca gct tgg aat tca ggc act tca act tta      1200
   Ala Ala Thr Lys Lys Thr Ala Ala Trp Asn Ser Gly Thr Ser Thr Leu
   385                 390                 395                     400
   aca att act gta aac agt aaa aaa act aaa gac ctt gtg ttt aca aaa      1248
   Thr Ile Thr Val Asn Ser Lys Lys Thr Lys Asp Leu Val Phe Thr Lys
                   405                 410                 415
   gaa aac aca att aca gta caa caa tac gac tca aat ggc acc aaa tta      1296
   Glu Asn Thr Ile Thr Val Gln Gln Tyr Asp Ser Asn Gly Thr Lys Leu
               420                 425                 430
   gag ggg tca gca gtt gaa att aca aaa ctt gat gaa att aaa aac gct      1344
   Glu Gly Ser Ala Val Glu Ile Thr Lys Leu Asp Glu Ile Lys Asn Ala
           435                 440                 445
   tta aaa taa                                                          1353
   Leu Lys  *
       450
<210> 148
   <211> 450
   <212> PRT
   <213> Artificial Sequence
<220>
   <223> Chimeric Protein
<400> 148
   Met Ala Cys Asn Asn Ser Gly Lys Asp Gly Asn Thr Ser Ala Asn Ser
   1               5                   10                  15
   Ala Asp Glu Ser Val Lys Gly Pro Asn Leu Thr Glu Ile Asn Lys Lys
               20                  25                  30
   Ile Thr Asp Ser Asn Ala Val Leu Leu Ala Val Lys Glu Val Glu Ala
           35                  40                  45
   Leu Leu Ser Ser Ile Asp Glu Ile Ala Ala Lys Ala Ile Gly Lys Lys
       50                  55                  60
   Ile His Gln Asn Asn Gly Leu Asp Thr Glu Asn Asn His Asn Gly Ser
   65                  70                  75                  80
   Leu Leu Ala Gly Ala Tyr Ala Ile Ser Thr Leu Ile Lys Gln Lys Leu
                   85                  90                  95
   Asp Gly Leu Lys Asn Glu Gly Leu Lys Glu Lys Ile Asp Ala Ala Lys
               100                 105                 110
   Lys Cys Ser Glu Thr Phe Thr Asn Lys Leu Lys Glu Lys His Thr Asp
           115                 120                 125
   Leu Gly Lys Glu Gly Val Thr Asp Ala Asp Ala Lys Glu Ala Ile Leu
       130                 135                 140
   Lys Ala Asn Gly Thr Lys Thr Lys Gly Ala Glu Glu Leu Gly Lys Leu
   145                 150                 155                 160
   Phe Glu Ser Val Glu Val Leu Ser Lys Ala Ala Lys Glu Met Leu Ala
               165                 170                 175
   Asn Ser Val Lys Glu Leu Thr Ser Pro Val Val Ala Glu Ser Pro Ala
               180                 185                 190
   Met Ala Lys Gln Asn Val Ser Ser Leu Asp Glu Lys Asn Ser Val Ser
           195                 200                 205
   Val Asp Leu Pro Gly Glu Met Lys Val Leu Val Ser Lys Glu Lys Asn
       210                 215                 220
   Lys Asp Gly Lys Tyr Asp Leu Ile Ala Thr Val Asp Lys Leu Glu Leu
   225                 230                 235                 240
   Lys Gly Thr Ser Asp Lys Asn Asn Gly Ser Gly Val Leu Glu Gly Val
                   245                 250                 255
   Lys Ala Asp Lys Ser Lys Val Lys Leu Thr Ile Ser Asp Asp Leu Gly
               260                 265                 270
   Gln Thr Thr Leu Glu Val Phe Lys Glu Asp Gly Lys Thr Leu Val Ser
           275                 280                 285
   Lys Lys Val Thr Ser Lys Asp Lys Ser Ser Thr Glu Glu Lys Phe Asn
       290                 295                 300
   Glu Lys Gly Glu Val Ser Glu Lys Ile Ile Thr Arg Ala Asp Gly Thr
   305                 310                 315                 320
   Arg Leu Glu Tyr Thr Gly Ile Lys Ser Asp Gly Ser Gly Lys Ala Lys
                   325                 330                 335
   Glu Val Leu Lys Gly Tyr Val Leu Glu Gly Thr Leu Thr Ala Glu Lys
               340                 345                 350
   Thr Thr Leu Val Val Lys Glu Gly Thr Val Thr Leu Ser Lys Asn Ile
           355                 360                 365
   Ser Lys Ser Gly Glu Val Ser Val Glu Leu Asn Asp Thr Asp Ser Ser
       370                 375                 380
   Ala Ala Thr Lys Lys Thr Ala Ala Trp Asn Ser Gly Thr Ser Thr Leu
   385                 390                 395                 400
   Thr Ile Thr Val Asn Ser Lys Lys Thr Lys Asp Leu Val Phe Thr Lys
                   405                 410                 415
   Glu Asn Thr Ile Thr Val Gln Gln Tyr Asp Ser Asn Gly Thr Lys Leu
               420                 425                 430
   Glu Gly Ser Ala Val Glu Ile Thr Lys Leu Asp Glu Ile Lys Asn Ala
           435                 440                 445
   Leu Lys
       450
<210> 149
   <211> 1341
   <212> DNA
   <213> Artificial Sequence
<220>
   <221> CDS
   <222> (1)...(1341)
   <223> Chimeric nucleic acid
<400> 149
   atg gct tgt aat aat tca ggg aaa gat ggg aat aca tct gca aat tct    48
   Met Ala Cys Asn Asn Ser Gly Lys Asp Gly Asn Thr Ser Ala Asn Ser
    1               5                  10                  15
   gct gat gag tct gtt aaa ggg cct aat ctt aca gaa ata agt aaa aaa    96
   Ala Asp Glu Ser Val Lys Gly Pro Asn Leu Thr Glu Ile Ser Lys Lys
               20                  25                  30
   att acg gat tct aat gcg gtt tta ctt gct gtg aaa gag gtt gaa gcg     144
   Ile Thr Asp Ser Asn Ala Val Leu Leu Ala Val Lys Glu Val Glu Ala
               35                  40                  45
   ttg ctg tca tct ata gat gag ctt gct aaa gct att ggt aaa aaa ata     192
   Leu Leu Ser Ser Ile Asp Glu Leu Ala Lys Ala Ile Gly Lys Lys Ile
           50                  55                  60
   aaa aac gat ggt agt tta gat aat gaa gca aat cgc aac gag tca ttg     240
   Lys Asn Asp Gly Ser Leu Asp Asn Glu Ala Asn Arg Asn Glu Ser Leu
   65                  70                  75                  80
   tta gca gga gct tat aca ata tca acc tta ata aca caa aaa tta agt     288
   Leu Ala Gly Ala Tyr Thr Ile Ser Thr Leu Ile Thr Gln Lys Leu Ser
                   85                  90                  95
   aaa tta aac gga tca gaa ggt tta aag gaa aag att gcc gca gct aag     336
   Lys Leu Asn Gly Ser Glu Gly Leu Lys Glu Lys Ile Ala Ala Ala Lys
               100                 105                 110
   aaa tgc tct gaa gag ttt agt act aaa cta aaa gat aat cat gca cag     384
   Lys Cys Ser Glu Glu Phe Ser Thr Lys Leu Lys Asp Asn His Ala Gln
           115                 120                 125
   ctt ggt ata cag ggc gtt act gat gaa aat gca aaa aaa gct att tta     432
   Leu Gly Ile Gln Gly Val Thr Asp Glu Asn Ala Lys Lys Ala Ile Leu
       130                 135                 140
   aaa gca aat gca gcg ggt aaa gat aag ggc gtt gaa gaa ctt gaa aag     480
   Lys Ala Asn Ala Ala Gly Lys Asp Lys Gly Val Glu Glu Leu Glu Lys
   145                 150                 155                 160
   ttg tcc gga tca tta gaa agc tta tca aaa gca gct aaa gag atg ctt     528
   Leu Ser Gly Ser Leu Glu Ser Leu Ser Lys Ala Ala Lys Glu Met Leu
                   165                 170                 175
   gct aat tca gtt aaa gag ctt aca agc cct gtt gtc cat ggc aag caa     576
   Ala Asn Ser Val Lys Glu Leu Thr Ser Pro Val Val His Gly Lys Gln
               180                 185                 190
   aat gtt agc agc ctt gac gag aaa aac agc gtt tca gta gat ttg cct     624
   Asn Val Ser Ser Leu Asp Glu Lys Asn Ser Val Ser Val Asp Leu Pro
           195                 200                 205
   ggt gaa atg aaa gtt ctt gta agc aaa gaa aaa aac aaa gac ggc aag     672
   Gly Glu Met Lys Val Leu Val Ser Lys Glu Lys Asn Lys Asp Gly Lys
       210                 215                 220
   tac gat cta att gca aca gta gac aag ctt gag ctt aaa gga act tct     720
   Tyr Asp Leu Ile Ala Thr Val Asp Lys Leu Glu Leu Lys Gly Thr Ser
   225                 230                 235                 240
   gat aaa aac aat gga tct gga gta ctt gaa ggc gta aaa gct gac aaa     768
   Asp Lys Asn Asn Gly Ser Gly Val Leu Glu Gly Val Lys Ala Asp Lys
                   245                 250                 255
   agt aaa gta aaa tta aca att tct gac gat cta ggt caa acc aca ctt     816
   Ser Lys Val Lys Leu Thr Ile Ser Asp Asp Leu Gly Gln Thr Thr Leu
               260                 265                 270
   gaa gtt ttc aaa gaa gat ggc aaa aca cta gta tca aaa aaa gta act    864
   Glu Val Phe Lys Glu Asp Gly Lys Thr Leu Val Ser Lys Lys Val Thr
           275                 280                 285
   tcc aaa gac aag tca tca aca gaa gaa aaa ttc aat gaa aaa ggt gaa    912
   Ser Lys Asp Lys Ser Ser Thr Glu Glu Lys Phe Asn Glu Lys Gly Glu
       290                 295                 300
   gta tct gaa aaa ata ata aca aga gca gac gga acc aga ctt gaa tac    960
   Val Ser Glu Lys Ile Ile Thr Arg Ala Asp Gly Thr Arg Leu Glu Tyr
   305                 310                 315                 320
   aca gga att aaa agc gat gga tct gga aaa gct aaa gag gtt tta aaa    1008
   Thr Gly Ile Lys Ser Asp Gly Ser Gly Lys Ala Lys Glu Val Leu Lys
                   325                 330                 335
   ggc tat gtt ctt gaa gga act cta act gct gaa aaa aca aca ttg gtg    1056
   Gly Tyr Val Leu Glu Gly Thr Leu Thr Ala Glu Lys Thr Thr Leu Val
               340                 345                 350
   gtt aaa gaa gga act gtt act tta agc aaa aat att tca aaa tct ggg    1104
   Val Lys Glu Gly Thr Val Thr Leu Ser Lys Asn Ile Ser Lys Ser Gly
           355                 360                 365
   gaa gtt tca gtt gaa ctt aat gac act gac agt agt gct gct act aaa    1152
   Glu Val Ser Val Glu Leu Asn Asp Thr Asp Ser Ser Ala Ala Thr Lys
       370                 375                 380
   aaa act gca gct tgg aat tca ggc act tca act tta aca att act gta    1200
   Lys Thr Ala Ala Trp Asn Ser Gly Thr Ser Thr Leu Thr Ile Thr Val
   385                 390                 395                 400
   aac agt aaa aaa act aaa gac ctt gtg ttt aca aaa gaa aac aca att    1248
   Asn Ser Lys Lys Thr Lys Asp Leu Val Phe Thr Lys Glu Asn Thr Ile
                   405                 410                 415
   aca gta caa caa tac gac tca aat ggc acc aaa tta gag ggg tca gca    1296
   Thr Val Gln Gln Tyr Asp Ser Asn Gly Thr Lys Leu Glu Gly Ser Ala
               420                 425                 430
   gtt gaa att aca aaa ctt gat gaa att aaa aac gct tta aaa taa        1341
   Val Glu Ile Thr Lys Leu Asp Glu Ile Lys Asn Ala Leu Lys  *
           435                 440                 445
<210> 150
   <211> 446
   <212> PRT
   <213> Artificial Sequence
<220>
   <223> Chimeric Protein
<400> 150
   Met Ala Cys Asn Asn Ser Gly Lys Asp Gly Asn Thr Ser Ala Asn Ser
    1               5                  10                  15
   Ala Asp Glu Ser Val Lys Gly Pro Asn Leu Thr Glu Ile Ser Lys Lys
               20                  25                  30
   Ile Thr Asp Ser Asn Ala Val Leu Leu Ala Val Lys Glu Val Glu Ala
           35                  40                  45
   Leu Leu Ser Ser Ile Asp Glu Leu Ala Lys Ala Ile Gly Lys Lys Ile
       50                  55                  60
   Lys Asn Asp Gly Ser Leu Asp Asn Glu Ala Asn Arg Asn Glu Ser Leu
   65                  70                  75                  80
   Leu Ala Gly Ala Tyr Thr Ile Ser Thr Leu Ile Thr Gln Lys Leu Ser
                   85                  90                  95
   Lys Leu Asn Gly Ser Glu Gly Leu Lys Glu Lys Ile Ala Ala Ala Lys
               100                 105                 110
   Lys Cys Ser Glu Glu Phe Ser Thr Lys Leu Lys Asp Asn His Ala Gln
           115                 120                 125
   Leu Gly Ile Gln Gly Val Thr Asp Glu Asn Ala Lys Lys Ala Ile Leu
       130                 135                 140
   Lys Ala Asn Ala Ala Gly Lys Asp Lys Gly Val Glu Glu Leu Glu Lys
   145                 150                 155                 160
   Leu Ser Gly Ser Leu Glu Ser Leu Ser Lys Ala Ala Lys Glu Met Leu
                   165                 170                 175
   Ala Asn Ser Val Lys Glu Leu Thr Ser Pro Val Val His Gly Lys Gln
               180                 185                 190
   Asn Val Ser Ser Leu Asp Glu Lys Asn Ser Val Ser Val Asp Leu Pro
           195                 200                 205
   Gly Glu Met Lys Val Leu Val Ser Lys Glu Lys Asn Lys Asp Gly Lys
       210                 215                 220
   Tyr Asp Leu Ile Ala Thr Val Asp Lys Leu Glu Leu Lys Gly Thr Ser
   225                 230                 235                 240
   Asp Lys Asn Asn Gly Ser Gly Val Leu Glu Gly Val Lys Ala Asp Lys
                   245                 250                 255
   Ser Lys Val Lys Leu Thr Ile Ser Asp Asp Leu Gly Gln Thr Thr Leu
               260                 265                 270
   Glu Val Phe Lys Glu Asp Gly Lys Thr Leu Val Ser Lys Lys Val Thr
           275                 280                 285
   Ser Lys Asp Lys Ser Ser Thr Glu Glu Lys Phe Asn Glu Lys Gly Glu
       290                 295                 300
   Val Ser Glu Lys Ile Ile Thr Arg Ala Asp Gly Thr Arg Leu Glu Tyr
   305                 310                 315                 320
   Thr Gly Ile Lys Ser Asp Gly Ser Gly Lys Ala Lys Glu Val Leu Lys
                   325                 330                 335
   Gly Tyr Val Leu Glu Gly Thr Leu Thr Ala Glu Lys Thr Thr Leu Val
               340                 345                 350
   Val Lys Glu Gly Thr Val Thr Leu Ser Lys Asn Ile Ser Lys Ser Gly
           355                 360                 365
   Glu Val Ser Val Glu Leu Asn Asp Thr Asp Ser Ser Ala Ala Thr Lys
       370                 375                 380
   Lys Thr Ala Ala Trp Asn Ser Gly Thr Ser Thr Leu Thr Ile Thr Val
   385                 390                 395                 400
   Asn Ser Lys Lys Thr Lys Asp Leu Val Phe Thr Lys Glu Asn Thr Ile
                   405                 410                 415
   Thr Val Gln Gln Tyr Asp Ser Asn Gly Thr Lys Leu Glu Gly Ser Ala
               420                 425                 430
   Val Glu Ile Thr Lys Leu Asp Glu Ile Lys Asn Ala Leu Lys
           435                 440                 445
<210> 151
   <211> 1362
   <212> DNA
   <213> Artificial Sequence
<220>
   <221> CDS
   <222> (1)...(1362)
   <223> Chimeric nucleic acid
<400> 151
   atg gct tgt aat aat tca ggg aaa gat ggg aat aca tct gca aat tct      48
   Met Ala Cys Asn Asn Ser Gly Lys Asp Gly Asn Thr Ser Ala Asn Ser
    1               5                  10                  15
   gct gat gag tct gtt aaa ggg cct aat ctt aca gaa ata aat aaa aaa      96
   Ala Asp Glu Ser Val Lys Gly Pro Asn Leu Thr Glu Ile Asn Lys Lys
               20                  25                  30
   att acg gat tct aat gcg gtt tta ctt gct gtg aaa gag gtt gaa gcg     144
   Ile Thr Asp Ser Asn Ala Val Leu Leu Ala Val Lys Glu Val Glu Ala
           35                  40                  45
   ttg ctg tca tct ata gat gaa att gct gct aaa gct att ggt aaa aaa     192
   Leu Leu Ser Ser Ile Asp Glu Ile Ala Ala Lys Ala Ile Gly Lys Lys
       50                  55                  60
   ata cac caa aat aat ggt ttg gat acc gaa aat aat cac aat gga tca     240
   Ile His Gln Asn Asn Gly Leu Asp Thr Glu Asn Asn His Asn Gly Ser
   65                  70                  75                  80
   ttg tta gcg gga gct tat gca ata tca acc cta ata aaa caa aaa tta     288
   Leu Leu Ala Gly Ala Tyr Ala Ile Ser Thr Leu Ile Lys Gln Lys Leu
                   85                  90                  95
   gat gga ttg aaa aat gaa gga tta aag gaa aaa att gat gcg gct aag     336
   Asp Gly Leu Lys Asn Glu Gly Leu Lys Glu Lys Ile Asp Ala Ala Lys
               100                 105                 110
   aaa tgt tct gaa aca ttt act aat aaa tta aaa gaa aaa cac aca gat     384
   Lys Cys Ser Glu Thr Phe Thr Asn Lys Leu Lys Glu Lys His Thr Asp
           115                 120                 125
   ctt ggt aaa gaa ggt gtt act gat gct gat gca aaa gaa gcc att tta     432
   Leu Gly Lys Glu Gly Val Thr Asp Ala Asp Ala Lys Glu Ala Ile Leu
       130                 135                 140
   aaa gca aat ggt act aaa act aaa ggt gct gaa gaa ctt gga aaa tta     480
   Lys Ala Asn Gly Thr Lys Thr Lys Gly Ala Glu Glu Leu Gly Lys Leu
   145                 150                 155                 160
   ttt gaa tca gta gag gtc ttg tca aaa gca gct aaa gag atg ctt gct     528
   Phe Glu Ser Val Glu Val Leu Ser Lys Ala Ala Lys Glu Met Leu Ala
                   165                 170                 175
   aat tca gtt aaa gag ctt aca agc cct gtt gtg gca gaa agt cca aaa     576
   Asn Ser Val Lys Glu Leu Thr Ser Pro Val Val Ala Glu Ser Pro Lys
               180                 185                 190
   aaa cct tcc atg gcc aag caa aat gtt agc agc ctt gac gag aaa aac     624
   Lys Pro Ser Met Ala Lys Gln Asn Val Ser Ser Leu Asp Glu Lys Asn
           195                 200                 205
   agc gtt tca gta gat ttg cct ggt gaa atg aaa gtt ctt gta agc aaa     672
   Ser Val Ser Val Asp Leu Pro Gly Glu Met Lys Val Leu Val Ser Lys
       210                 215                 220
   gaa aaa aac aaa gac ggc aag tac gat cta att gca aca gta gac aag     720
   Glu Lys Asn Lys Asp Gly Lys Tyr Asp Leu Ile Ala Thr Val Asp Lys
   225                 230                 235                 240
   ctt gag ctt aaa gga act tct gat aaa aac aat gga tct gga gta ctt     768
   Leu Glu Leu Lys Gly Thr Ser Asp Lys Asn Asn Gly Ser Gly Val Leu
                   245                 250                 255
   gaa ggc gta aaa gct gac aaa agt aaa gta aaa tta aca att tct gac     816
   Glu Gly Val Lys Ala Asp Lys Ser Lys Val Lys Leu Thr Ile Ser Asp
               260                 265                 270
   gat cta ggt caa acc aca ctt gaa gtt ttc aaa gaa gat ggc aaa aca     864
   Asp Leu Gly Gln Thr Thr Leu Glu Val Phe Lys Glu Asp Gly Lys Thr
           275                 280                 285
   cta gta tca aaa aaa gta act tcc aaa gac aag tca tca aca gaa gaa     912
   Leu Val Ser Lys Lys Val Thr Ser Lys Asp Lys Ser Ser Thr Glu Glu
       290                 295                 300
   aaa ttc aat gaa aaa ggt gaa gta tct gaa aaa ata ata aca aga gca     960
   Lys Phe Asn Glu Lys Gly Glu Val Ser Glu Lys Ile Ile Thr Arg Ala
   305                 310                 315                 320
   gac gga acc aga ctt gaa tac aca gga att aaa agc gat gga tct gga    1008
   Asp Gly Thr Arg Leu Glu Tyr Thr Gly Ile Lys Ser Asp Gly Ser Gly
                   325                 330                 335
   aaa gct aaa gag gtt tta aaa ggc tat gtt ctt gaa gga act cta act    1056
   Lys Ala Lys Glu Val Leu Lys Gly Tyr Val Leu Glu Gly Thr Leu Thr
               340                 345                 350
   gct gaa aaa aca aca ttg gtg gtt aaa gaa gga act gtt act tta agc    1104
   Ala Glu Lys Thr Thr Leu Val Val Lys Glu Gly Thr Val Thr Leu Ser
           355                 360                 365
   aaa aat att tca aaa tct ggg gaa gtt tca gtt gaa ctt aat gac act    1152
   Lys Asn Ile Ser Lys Ser Gly Glu Val Ser Val Glu Leu Asn Asp Thr
       370                 375                 380
   gac agt agt gct gct act aaa aaa act gca gct tgg aat tca aaa act    1200
   Asp Ser Ser Ala Ala Thr Lys Lys Thr Ala Ala Trp Asn Ser Lys Thr
   385                 390                 395                 400
   tcc act tta aca att agt gtg aat agc caa aaa acc aaa aac ctt gta    1248
   Ser Thr Leu Thr Ile Ser Val Asn Ser Gln Lys Thr Lys Asn Leu Val
                   405                 410                 415
   ttc aca aaa gaa gac aca ata aca gta caa aaa tac gac tca gca ggc    1296
   Phe Thr Lys Glu Asp Thr Ile Thr Val Gln Lys Tyr Asp Ser Ala Gly
               420                 425                 430
   acc aat cta gaa ggc aaa gca gtc gaa att aca aca ctt aaa gaa ctt      1344
   Thr Asn Leu Glu Gly Lys Ala Val Glu Ile Thr Thr Leu Lys Glu Leu
           435                 440                 445
   aaa aac gct tta aaa taa                                                1362
   Lys Asn Ala Leu Lys  *
       450
<210> 152
   <211> 453
   <212> PRT
   <213> Artificial Sequence
<220>
   <223> Chimeric Protein
<400> 152
   Met Ala Cys Asn Asn Ser Gly Lys Asp Gly Asn Thr Ser Ala Asn Ser
   1               5                   10                  15
   Ala Asp Glu Ser Val Lys Gly Pro Asn Leu Thr Glu Ile Asn Lys Lys
               20                  25                  30
   Ile Thr Asp Ser Asn Ala Val Leu Leu Ala Val Lys Glu Val Glu Ala
           35                  40                  45
   Leu Leu Ser Ser Ile Asp Glu Ile Ala Ala Lys Ala Ile Gly Lys Lys
       50                  55                  60
   Ile His Gln Asn Asn Gly Leu Asp Thr Glu Asn Asn His Asn Gly Ser
   65                  70                  75                  80
   Leu Leu Ala Gly Ala Tyr Ala Ile Ser Thr Leu Ile Lys Gln Lys Leu
                   85                  90                  95
   Asp Gly Leu Lys Asn Glu Gly Leu Lys Glu Lys Ile Asp Ala Ala Lys
               100                 105                 110
   Lys Cys Ser Glu Thr Phe Thr Asn Lys Leu Lys Glu Lys His Thr Asp
           115                 120                 125
   Leu Gly Lys Glu Gly Val Thr Asp Ala Asp Ala Lys Glu Ala Ile Leu
       130                 135                 140
   Lys Ala Asn Gly Thr Lys Thr Lys Gly Ala Glu Glu Leu Gly Lys Leu
   145                 150                 155                 160
   Phe Glu Ser Val Glu Val Leu Ser Lys Ala Ala Lys Glu Met Leu Ala
                   165                 170                 175
   Asn Ser Val Lys Glu Leu Thr Ser Pro Val Val Ala Glu Ser Pro Lys
               180                 185                 190
   Lys Pro Ser Met Ala Lys Gln Asn Val Ser Ser Leu Asp Glu Lys Asn
           195                 200                 205
   Ser Val Ser Val Asp Leu Pro Gly Glu Met Lys Val Leu Val Ser Lys
       210                 215                 220
   Glu Lys Asn Lys Asp Gly Lys Tyr Asp Leu Ile Ala Thr Val Asp Lys
   225                 230                 235                 240
   Leu Glu Leu Lys Gly Thr Ser Asp Lys Asn Asn Gly Ser Gly Val Leu
                   245                 250                 255
   Glu Gly Val Lys Ala Asp Lys Ser Lys Val Lys Leu Thr Ile Ser Asp
               260                 265                 270
   Asp Leu Gly Gln Thr Thr Leu Glu Val Phe Lys Glu Asp Gly Lys Thr
           275                 280                 285
   Leu Val Ser Lys Lys Val Thr Ser Lys Asp Lys Ser Ser Thr Glu Glu
       290                 295                 300
   Lys Phe Asn Glu Lys Gly Glu Val Ser Glu Lys Ile Ile Thr Arg Ala
   305                 310                 315                 320
   Asp Gly Thr Arg Leu Glu Tyr Thr Gly Ile Lys Ser Asp Gly Ser Gly
                   325                 330                 335
   Lys Ala Lys Glu Val Leu Lys Gly Tyr Val Leu Glu Gly Thr Leu Thr
               340                 345                 350
   Ala Glu Lys Thr Thr Leu Val Val Lys Glu Gly Thr Val Thr Leu Ser
           355                 360                 365
   Lys Asn Ile Ser Lys Ser Gly Glu Val Ser Val Glu Leu Asn Asp Thr
       370                 375                 380
   Asp Ser Ser Ala Ala Thr Lys Lys Thr Ala Ala Trp Asn Ser Lys Thr
   385                 390                 395                 400
   Ser Thr Leu Thr Ile Ser Val Asn Ser Gln Lys Thr Lys Asn Leu Val
                   405                 410                 415
   Phe Thr Lys Glu Asp Thr Ile Thr Val Gln Lys Tyr Asp Ser Ala Gly
               420                 425                 430
   Thr Asn Leu Glu Gly Lys Ala Val Glu Ile Thr Thr Leu Lys Glu Leu
           435                 440                 445
   Lys Asn Ala Leu Lys
       450
<210> 153
   <211> 1341
   <212> DNA
   <213> Artificial Sequence
<220>
   <221> CDS
   <222> (1)...(1341)
   <223> Chimeric nucleic acid
<400> 153
   atg gct tgt aat aat tca ggg aaa gat ggg aat aca tct gca aat tct     48
   Met Ala Cys Asn Asn Ser Gly Lys Asp Gly Asn Thr Ser Ala Asn Ser
    1               5                  10                  15
   gct gat gag tct gtt aaa ggg cct aat ctt aca gaa ata agt aaa aaa     96
   Ala Asp Glu Ser Val Lys Gly Pro Asn Leu Thr Glu Ile Ser Lys Lys
               20                  25                  30
   att acg gat tct aat gcg gtt tta ctt gct gtg aaa gag gtt gaa gcg    144
   Ile Thr Asp Ser Asn Ala Val Leu Leu Ala Val Lys Glu Val Glu Ala
           35                  40                  45
   ttg ctg tca tct ata gat gag ctt gct aaa gct att ggt aaa aaa ata    192
   Leu Leu Ser Ser Ile Asp Glu Leu Ala Lys Ala Ile Gly Lys Lys Ile
       50                  55                  60
   aaa aac gat ggt agt tta gat aat gaa gca aat cgc aac gag tca ttg    240
   Lys Asn Asp Gly Ser Leu Asp Asn Glu Ala Asn Arg Asn Glu Ser Leu
   65                  70                  75                  80
   tta gca gga gct tat aca ata tca acc tta ata aca caa aaa tta agt    288
   Leu Ala Gly Ala Tyr Thr Ile Ser Thr Leu Ile Thr Gln Lys Leu Ser
                   85                  90                  95
   aaa tta aac gga tca gaa ggt tta aag gaa aag att gcc gca gct aag    336
   Lys Leu Asn Gly Ser Glu Gly Leu Lys Glu Lys Ile Ala Ala Ala Lys
               100                 105                 110
   aaa tgc tct gaa gag ttt agt act aaa cta aaa gat aat cat gca cag    384
   Lys Cys Ser Glu Glu Phe Ser Thr Lys Leu Lys Asp Asn His Ala Gln
           115                 120                 125
   ctt ggt ata cag ggc gtt act gat gaa aat gca aaa aaa gct att tta      432
   Leu Gly Ile Gln Gly Val Thr Asp Glu Asn Ala Lys Lys Ala Ile Leu
       130                 135                 140
   aaa gca aat gca gcg ggt aaa gat aag ggc gtt gaa gaa ctt gaa aag      480
   Lys Ala Asn Ala Ala Gly Lys Asp Lys Gly Val Glu Glu Leu Glu Lys
   145                 150                 155                 160
   ttg tcc gga tca tta gaa agc tta tca aaa gca gct aaa gag atg ctt      528
   Leu Ser Gly Ser Leu Glu Ser Leu Ser Lys Ala Ala Lys Glu Met Leu
                   165                 170                 175
   gct aat tca gtt aaa gag ctt aca agc cct gtt gtc cat ggc aag caa      576
   Ala Asn Ser Val Lys Glu Leu Thr Ser Pro Val Val His Gly Lys Gln
               180                 185                 190
   aat gtt agc agc ctt gac gag aaa aac agc gtt tca gta gat ttg cct      624
   Asn Val Ser Ser Leu Asp Glu Lys Asn Ser Val Ser Val Asp Leu Pro
           195                 200                 205
   ggt gaa atg aaa gtt ctt gta agc aaa gaa aaa aac aaa gac ggc aag      672
   Gly Glu Met Lys Val Leu Val Ser Lys Glu Lys Asn Lys Asp Gly Lys
       210                 215                 220
   tac gat cta att gca aca gta gac aag ctt gag ctt aaa gga act tct      720
   Tyr Asp Leu Ile Ala Thr Val Asp Lys Leu Glu Leu Lys Gly Thr Ser
   225                 230                 235                 240
   gat aaa aac aat gga tct gga gta ctt gaa ggc gta aaa gct gac aaa      768
   Asp Lys Asn Asn Gly Ser Gly Val Leu Glu Gly Val Lys Ala Asp Lys
                   245                 250                 255
   agt aaa gta aaa tta aca att tct gac gat cta ggt caa acc aca ctt      816
   Ser Lys Val Lys Leu Thr Ile Ser Asp Asp Leu Gly Gln Thr Thr Leu
               260                 265                 270
   gaa gtt ttc aaa gaa gat ggc aaa aca cta gta tca aaa aaa gta act      864
   Glu Val Phe Lys Glu Asp Gly Lys Thr Leu Val Ser Lys Lys Val Thr
           275                 280                 285
   tcc aaa gac aag tca tca aca gaa gaa aaa ttc aat gaa aaa ggt gaa      912
   Ser Lys Asp Lys Ser Ser Thr Glu Glu Lys Phe Asn Glu Lys Gly Glu
       290                 295                 300
   gta tct gaa aaa ata ata aca aga gca gac gga acc aga ctt gaa tac      960
   Val Ser Glu Lys Ile Ile Thr Arg Ala Asp Gly Thr Arg Leu Glu Tyr
   305                 310                 315                 320
   aca gga att aaa agc gat gga tct gga aaa gct aaa gag gtt tta aaa     1008
   Thr Gly Ile Lys Ser Asp Gly Ser Gly Lys Ala Lys Glu Val Leu Lys
                   325                 330                 335
   ggc tat gtt ctt gaa gga act cta act gct gaa aaa aca aca ttg gtg     1056
   Gly Tyr Val Leu Glu Gly Thr Leu Thr Ala Glu Lys Thr Thr Leu Val
               340                 345                 350
   gtt aaa gaa gga act gtt act tta agc aaa aat att tca aaa tct ggg     1104
   Val Lys Glu Gly Thr Val Thr Leu Ser Lys Asn Ile Ser Lys Ser Gly
           355                 360                 365
   gaa gtt tca gtt gaa ctt aat gac act gac agt agt gct gct act aaa     1152
   Glu Val Ser Val Glu Leu Asn Asp Thr Asp Ser Ser Ala Ala Thr Lys
       370                 375                 380
   aaa act gca gct tgg aat tca aaa act tcc act tta aca att agt gtg     1200
   Lys Thr Ala Ala Trp Asn Ser Lys Thr Ser Thr Leu Thr Ile Ser Val
   385                 390                 395                 400
   aat agc caa aaa acc aaa aac ctt gta ttc aca aaa gaa gac aca ata     1248
   Asn Ser Gln Lys Thr Lys Asn Leu Val Phe Thr Lys Glu Asp Thr Ile
                   405                 410                 415
   aca gta caa aaa tac gac tca gca ggc acc aat cta gaa ggc aaa gca     1296
   Thr Val Gln Lys Tyr Asp Ser Ala Gly Thr Asn Leu Glu Gly Lys Ala
               420                 425                 430
   gtc gaa att aca aca ctt aaa gaa ctt aaa aac gct tta aaa taa         1341
   Val Glu Ile Thr Thr Leu Lys Glu Leu Lys Asn Ala Leu Lys  *
           435                 440                 445
<210> 154
   <211> 446
   <212> PRT
   <213> Artificial Sequence
<220>
   <223> Chimeric protein
<400> 154
   Met Ala Cys Asn Asn Ser Gly Lys Asp Gly Asn Thr Ser Ala Asn Ser
    1               5                  10                  15
   Ala Asp Glu Ser Val Lys Gly Pro Asn Leu Thr Glu Ile Ser Lys Lys
               20                  25                  30
   Ile Thr Asp Ser Asn Ala Val Leu Leu Ala Val Lys Glu Val Glu Ala
           35                  40                  45
   Leu Leu Ser Ser Ile Asp Glu Leu Ala Lys Ala Ile Gly Lys Lys Ile
       50                  55                  60
   Lys Asn Asp Gly Ser Leu Asp Asn Glu Ala Asn Arg Asn Glu Ser Leu
   65                  70                  75                  80
   Leu Ala Gly Ala Tyr Thr Ile Ser Thr Leu Ile Thr Gln Lys Leu Ser
                   85                  90                  95
   Lys Leu Asn Gly Ser Glu Gly Leu Lys Glu Lys Ile Ala Ala Ala Lys
               100                 105                 110
   Lys Cys Ser Glu Glu Phe Ser Thr Lys Leu Lys Asp Asn His Ala Gln
           115                 120                 125
   Leu Gly Ile Gln Gly Val Thr Asp Glu Asn Ala Lys Lys Ala Ile Leu
       130                 135                 140
   Lys Ala Asn Ala Ala Gly Lys Asp Lys Gly Val Glu Glu Leu Glu Lys
   145                 150                 155                 160
   Leu Ser Gly Ser Leu Glu Ser Leu Ser Lys Ala Ala Lys Glu Met Leu
                   165                 170                 175
   Ala Asn Ser Val Lys Glu Leu Thr Ser Pro Val Val His Gly Lys Gln
               180                 185                 190
   Asn Val Ser Ser Leu Asp Glu Lys Asn Ser Val Ser Val Asp Leu Pro
           195                 200                 205
   Gly Glu Met Lys Val Leu Val Ser Lys Glu Lys Asn Lys Asp Gly Lys
       210                 215                 220
   Tyr Asp Leu Ile Ala Thr Val Asp Lys Leu Glu Leu Lys Gly Thr Ser
   225                 230                 235                 240
   Asp Lys Asn Asn Gly Ser Gly Val Leu Glu Gly Val Lys Ala Asp Lys
                   245                 250                 255
   Ser Lys Val Lys Leu Thr Ile Ser Asp Asp Leu Gly Gln Thr Thr Leu
               260                 265                 270
   Glu Val Phe Lys Glu Asp Gly Lys Thr Leu Val Ser Lys Lys Val Thr
           275                 280                 285
   Ser Lys Asp Lys Ser Ser Thr Glu Glu Lys Phe Asn Glu Lys Gly Glu
       290                 295                 300
   Val Ser Glu Lys Ile Ile Thr Arg Ala Asp Gly Thr Arg Leu Glu Tyr
   305                 310                 315                 320
   Thr Gly Ile Lys Ser Asp Gly Ser Gly Lys Ala Lys Glu Val Leu Lys
                   325                 330                 335
   Gly Tyr Val Leu Glu Gly Thr Leu Thr Ala Glu Lys Thr Thr Leu Val
               340                 345                 350
   Val Lys Glu Gly Thr Val Thr Leu Ser Lys Asn Ile Ser Lys Ser Gly
           355                 360                 365
   Glu Val Ser Val Glu Leu Asn Asp Thr Asp Ser Ser Ala Ala Thr Lys
       370                 375                 380
   Lys Thr Ala Ala Trp Asn Ser Lys Thr Ser Thr Leu Thr Ile Ser Val
   385                 390                 395                 400
   Asn Ser Gln Lys Thr Lys Asn Leu Val Phe Thr Lys Glu Asp Thr Ile
                   405                 410                 415
   Thr Val Gln Lys Tyr Asp Ser Ala Gly Thr Asn Leu Glu Gly Lys Ala
               420                 425                 430
   Val Glu Ile Thr Thr Leu Lys Glu Leu Lys Asn Ala Leu Lys
           435                 440                 445
<210> 155
   <211> 1362
   <212> DNA
   <213> Artificial Sequence
<220>
   <221> CDS
   <222> (1)... (1362)
   <223> Chimeric nucleic acid
<400> 155
   atg gct tgt aat aat tca ggg aaa gat ggg aat aca tct gca aat tct      48
   Met Ala Cys Asn Asn Ser Gly Lys Asp Gly Asn Thr Ser Ala Asn Ser
    1               5                  10                  15
   gct gat gag tct gtt aaa ggg cct aat ctt aca gaa ata aat aaa aaa      96
   Ala Asp Glu Ser Val Lys Gly Pro Asn Leu Thr Glu Ile Asn Lys Lys
               20                  25                  30
   att acg gat tct aat gcg gtt tta ctt gct gtg aaa gag gtt gaa gcg     144
   Ile Thr Asp Ser Asn Ala Val Leu Leu Ala Val Lys Glu Val Glu Ala
           35                  40                  45
   ttg ctg tca tct ata gat gaa att gct gct aaa gct att ggt aaa aaa     192
   Leu Leu Ser Ser Ile Asp Glu Ile Ala Ala Lys Ala Ile Gly Lys Lys
       50                  55                  60
   ata cac caa aat aat ggt ttg gat acc gaa aat aat cac aat gga tca     240
   Ile His Gln Asn Asn Gly Leu Asp Thr Glu Asn Asn His Asn Gly Ser
   65                  70                  75                  80
   ttg tta gcg gga gct tat gca ata tca acc cta ata aaa caa aaa tta    288
   Leu Leu Ala Gly Ala Tyr Ala Ile Ser Thr Leu Ile Lys Gln Lys Leu
                   85                  90                  95
   gat gga ttg aaa aat gaa gga tta aag gaa aaa att gat gcg gct aag    336
   Asp Gly Leu Lys Asn Glu Gly Leu Lys Glu Lys Ile Asp Ala Ala Lys
               100                 105                 110
   aaa tgt tct gaa aca ttt act aat aaa tta aaa gaa aaa cac aca gat    384
   Lys Cys Ser Glu Thr Phe Thr Asn Lys Leu Lys Glu Lys His Thr Asp
           115                 120                 125
   ctt ggt aaa gaa ggt gtt act gat gct gat gca aaa gaa gcc att tta    432
   Leu Gly Lys Glu Gly Val Thr Asp Ala Asp Ala Lys Glu Ala Ile Leu
       130                 135                 140
   aaa gca aat ggt act aaa act aaa ggt gct gaa gaa ctt gga aaa tta    480
   Lys Ala Asn Gly Thr Lys Thr Lys Gly Ala Glu Glu Leu Gly Lys Leu
   145                 150                 155                 160
   ttt gaa tca gta gag gtc ttg tca aaa gca gct aaa gag atg ctt gct    528
   Phe Glu Ser Val Glu Val Leu Ser Lys Ala Ala Lys Glu Met Leu Ala
                   165                 170                 175
   aat tca gtt aaa gag ctt aca agc cct gtt gtg gca gaa agt cca aaa    576
   Asn Ser Val Lys Glu Leu Thr Ser Pro Val Val Ala Glu Ser Pro Lys
               180                 185                 190
   aaa cct tcc atg gcc aag caa aat gtt agc agc ctt gac gag aaa aac    624
   Lys Pro Ser Met Ala Lys Gln Asn Val Ser Ser Leu Asp Glu Lys Asn
           195                 200                 205
   agc gtt tca gta gat ttg cct ggt gaa atg aaa gtt ctt gta agc aaa    672
   Ser Val Ser Val Asp Leu Pro Gly Glu Met Lys Val Leu Val Ser Lys
       210                 215                 220
   gaa aaa aac aaa gac ggc aag tac gat cta att gca aca gta gac aag    720
   Glu Lys Asn Lys Asp Gly Lys Tyr Asp Leu Ile Ala Thr Val Asp Lys
   225                 230                 235                 240
   ctt gag ctt aaa gga act tct gat aaa aac aat gga tct gga gta ctt    768
   Leu Glu Leu Lys Gly Thr Ser Asp Lys Asn Asn Gly Ser Gly Val Leu
                   245                 250                 255
   gaa ggc gta aaa gct gac aaa agt aaa gta aaa tta aca att tct gac    816
   Glu Gly Val Lys Ala Asp Lys Ser Lys Val Lys Leu Thr Ile Ser Asp
               260                 265                 270
   gat cta ggt caa acc aca ctt gaa gtt ttc aaa gaa gat ggc aaa aca    864
   Asp Leu Gly Gln Thr Thr Leu Glu Val Phe Lys Glu Asp Gly Lys Thr
           275                 280                 285
   cta gta tca aaa aaa gta act tcc aaa gac aag tca tca aca gaa gaa    912
   Leu Val Ser Lys Lys Val Thr Ser Lys Asp Lys Ser Ser Thr Glu Glu
       290                 295                 300
   aaa ttc aat gaa aaa ggt gaa gta tct gaa aaa ata ata aca aga gca    960
   Lys Phe Asn Glu Lys Gly Glu Val Ser Glu Lys Ile Ile Thr Arg Ala
   305                 310                 315                 320
   gac gga acc aga ctt gaa tac aca gga att aaa agc gat gga tct gga     1008
   Asp Gly Thr Arg Leu Glu Tyr Thr Gly Ile Lys Ser Asp Gly Ser Gly
                   325                 330                 335
   aaa gct aaa gag gtt tta aaa ggc tat gtt ctt gaa gga act cta act     1056
   Lys Ala Lys Glu Val Leu Lys Gly Tyr Val Leu Glu Gly Thr Leu Thr
               340                 345                 350
   gct gaa aaa aca aca ttg gtg gtt aaa gaa gga act gtt act tta agc     1104
   Ala Glu Lys Thr Thr Leu Val Val Lys Glu Gly Thr Val Thr Leu Ser
           355                 360                 365
   aaa aat att tca aaa tct ggg gaa gtt tca gtt gaa ctt aat gac act     1152
   Lys Asn Ile Ser Lys Ser Gly Glu Val Ser Val Glu Leu Asn Asp Thr
       370                 375                 380
   gac agt agt gct gct act aaa aaa act gca gct tgg aat tca aaa act     1200
   Asp Ser Ser Ala Ala Thr Lys Lys Thr Ala Ala Trp Asn Ser Lys Thr
   385                 390                 395                 400
   tct act tta aca att agt gtt aac agc aaa aaa act aca caa ctt gtg     1248
   Ser Thr Leu Thr Ile Ser Val Asn Ser Lys Lys Thr Thr Gln Leu Val
                   405                 410                 415
   ttt act aaa caa gac aca ata act gta caa aaa tac gac tcc gca ggt     1296
   Phe Thr Lys Gln Asp Thr Ile Thr Val Gln Lys Tyr Asp Ser Ala Gly
               420                 425                 430
   acc aat tta gaa ggc aca gca gtc gaa att aaa aca ctt gat gaa ctt     1344
   Thr Asn Leu Glu Gly Thr Ala Val Glu Ile Lys Thr Leu Asp Glu Leu
           435                 440                 445
   aaa aac gct tta aaa taa                                             1362
   Lys Asn Ala Leu Lys  *
       450
<210> 156
   <211> 453
   <212> PRT
   <213> Artificial Sequence
<220>
   <223> Chimeric Protein
<400> 156
   Met Ala Cys Asn Asn Ser Gly Lys Asp Gly Asn Thr Ser Ala Asn Ser
   1               5                   10                  15
   Ala Asp Glu Ser Val Lys Gly Pro Asn Leu Thr Glu Ile Asn Lys Lys
               20                  25                  30
   Ile Thr Asp Ser Asn Ala Val Leu Leu Ala Val Lys Glu Val Glu Ala
           35                  40                  45
   Leu Leu Ser Ser Ile Asp Glu Ile Ala Ala Lys Ala Ile Gly Lys Lys
       50                  55                  60
   Ile His Gln Asn Asn Gly Leu Asp Thr Glu Asn Asn His Asn Gly Ser
   65                  70                  75                  80
   Leu Leu Ala Gly Ala Tyr Ala Ile Ser Thr Leu Ile Lys Gln Lys Leu
                   85                  90                  95
   Asp Gly Leu Lys Asn Glu Gly Leu Lys Glu Lys Ile Asp Ala Ala Lys
               100                 105                 110
   Lys Cys Ser Glu Thr Phe Thr Asn Lys Leu Lys Glu Lys His Thr Asp
           115                 120                 125
   Leu Gly Lys Glu Gly Val Thr Asp Ala Asp Ala Lys Glu Ala Ile Leu
       130                 135                 140
   Lys Ala Asn Gly Thr Lys Thr Lys Gly Ala Glu Glu Leu Gly Lys Leu
   145                 150                 155                 160
   Phe Glu Ser Val Glu Val Leu Ser Lys Ala Ala Lys Glu Met Leu Ala
                   165                 170                 175
   Asn Ser Val Lys Glu Leu Thr Ser Pro Val Val Ala Glu Ser Pro Lys
               180                 185                 190
   Lys Pro Ser Met Ala Lys Gln Asn Val Ser Ser Leu Asp Glu Lys Asn
           195                 200                 205
   Ser Val Ser Val Asp Leu Pro Gly Glu Met Lys Val Leu Val Ser Lys
       210                 215                 220
   Glu Lys Asn Lys Asp Gly Lys Tyr Asp Leu Ile Ala Thr Val Asp Lys
   225                 230                 235                 240
   Leu Glu Leu Lys Gly Thr Ser Asp Lys Asn Asn Gly Ser Gly Val Leu
                   245                 250                 255
   Glu Gly Val Lys Ala Asp Lys Ser Lys Val Lys Leu Thr Ile Ser Asp
               260                 265                 270
   Asp Leu Gly Gln Thr Thr Leu Glu Val Phe Lys Glu Asp Gly Lys Thr
           275                 280                 285
   Leu Val Ser Lys Lys Val Thr Ser Lys Asp Lys Ser Ser Thr Glu Glu
       290                 295                 300
   Lys Phe Asn Glu Lys Gly Glu Val Ser Glu Lys Ile Ile Thr Arg Ala
   305                 310                 315                 320
   Asp Gly Thr Arg Leu Glu Tyr Thr Gly Ile Lys Ser Asp Gly Ser Gly
                   325                 330                 335
   Lys Ala Lys Glu Val Leu Lys Gly Tyr Val Leu Glu Gly Thr Leu Thr
               340                 345                 350
   Ala Glu Lys Thr Thr Leu Val Val Lys Glu Gly Thr Val Thr Leu Ser
           355                 360                 365
   Lys Asn Ile Ser Lys Ser Gly Glu Val Ser Val Glu Leu Asn Asp Thr
       370                 375                 380
   Asp Ser Ser Ala Ala Thr Lys Lys Thr Ala Ala Trp Asn Ser Lys Thr
   385                 390                 395                 400
   Ser Thr Leu Thr Ile Ser Val Asn Ser Lys Lys Thr Thr Gln Leu Val
                   405                 410                 415
   Phe Thr Lys Gln Asp Thr Ile Thr Val Gln Lys Tyr Asp Ser Ala Gly
               420                 425                 430
   Thr Asn Leu Glu Gly Thr Ala Val Glu Ile Lys Thr Leu Asp Glu Leu
           435                 440                 445
   Lys Asn Ala Leu Lys
       450
<210> 157
   <211> 1341
   <212> DNA
   <213> Artificial Sequence
<220>
   <221> CDS
   <222> (1)...(1341)
   <223> Chimeric nucleic acid
<400> 157
   atg gct tgt aat aat tca ggg aaa gat ggg aat aca tct gca aat tct    48
   Met Ala Cys Asn Asn Ser Gly Lys Asp Gly Asn Thr Ser Ala Asn Ser
    1               5                  10                  15
   gct gat gag tct gtt aaa ggg cct aat ctt aca gaa ata agt aaa aaa      96
   Ala Asp Glu Ser Val Lys Gly Pro Asn Leu Thr Glu Ile Ser Lys Lys
               20                  25                  30
   att acg gat tct aat gcg gtt tta ctt gct gtg aaa gag gtt gaa gcg     144
   Ile Thr Asp Ser Asn Ala Val Leu Leu Ala Val Lys Glu Val Glu Ala
           35                  40                  45
   ttg ctg tca tct ata gat gag ctt gct aaa gct att ggt aaa aaa ata     192
   Leu Leu Ser Ser Ile Asp Glu Leu Ala Lys Ala Ile Gly Lys Lys Ile
       50                  55                  60
   aaa aac gat ggt agt tta gat aat gaa gca aat cgc aac gag tca ttg     240
   Lys Asn Asp Gly Ser Leu Asp Asn Glu Ala Asn Arg Asn Glu Ser Leu
   65                  70                  75                  80
   tta gca gga gct tat aca ata tca acc tta ata aca caa aaa tta agt     288
   Leu Ala Gly Ala Tyr Thr Ile Ser Thr Leu Ile Thr Gln Lys Leu Ser
                   85                  90                  95
   aaa tta aac gga tca gaa ggt tta aag gaa aag att gcc gca gct aag     336
   Lys Leu Asn Gly Ser Glu Gly Leu Lys Glu Lys Ile Ala Ala Ala Lys
               100                 105                 110
   aaa tgc tct gaa gag ttt agt act aaa cta aaa gat aat cat gca cag     384
   Lys Cys Ser Glu Glu Phe Ser Thr Lys Leu Lys Asp Asn His Ala Gln
           115                 120                 125
   ctt ggt ata cag ggc gtt act gat gaa aat gca aaa aaa gct att tta     432
   Leu Gly Ile Gln Gly Val Thr Asp Glu Asn Ala Lys Lys Ala Ile Leu
       130                 135                 140
   aaa gca aat gca gcg ggt aaa gat aag ggc gtt gaa gaa ctt gaa aag     480
   Lys Ala Asn Ala Ala Gly Lys Asp Lys Gly Val Glu Glu Leu Glu Lys
   145                 150                 155                 160
   ttg tcc gga tca tta gaa agc tta tca aaa gca gct aaa gag atg ctt     528
   Leu Ser Gly Ser Leu Glu Ser Leu Ser Lys Ala Ala Lys Glu Met Leu
                   165                 170                 175
   gct aat tca gtt aaa gag ctt aca agc cct gtt gtc cat ggc aag caa     576
   Ala Asn Ser Val Lys Glu Leu Thr Ser Pro Val Val His Gly Lys Gln
               180                 185                 190
   aat gtt agc agc ctt gac gag aaa aac agc gtt tca gta gat ttg cct     624
   Asn Val Ser Ser Leu Asp Glu Lys Asn Ser Val Ser Val Asp Leu Pro
           195                 200                 205
   ggt gaa atg aaa gtt ctt gta agc aaa gaa aaa aac aaa gac ggc aag     672
   Gly Glu Met Lys Val Leu Val Ser Lys Glu Lys Asn Lys Asp Gly Lys
       210                 215                 220
   tac gat cta att gca aca gta gac aag ctt gag ctt aaa gga act tct     720
   Tyr Asp Leu Ile Ala Thr Val Asp Lys Leu Glu Leu Lys Gly Thr Ser
   225                 230                 235                 240
   gat aaa aac aat gga tct gga gta ctt gaa ggc gta aaa gct gac aaa     768
   Asp Lys Asn Asn Gly Ser Gly Val Leu Glu Gly Val Lys Ala Asp Lys
                   245                 250                 255
   agt aaa gta aaa tta aca att tct gac gat cta ggt caa acc aca ctt    816
   Ser Lys Val Lys Leu Thr Ile Ser Asp Asp Leu Gly Gln Thr Thr Leu
               260                 265                 270
   gaa gtt ttc aaa gaa gat ggc aaa aca cta gta tca aaa aaa gta act    864
   Glu Val Phe Lys Glu Asp Gly Lys Thr Leu Val Ser Lys Lys Val Thr
           275                 280                 285
   tcc aaa gac aag tca tca aca gaa gaa aaa ttc aat gaa aaa ggt gaa    912
   Ser Lys Asp Lys Ser Ser Thr Glu Glu Lys Phe Asn Glu Lys Gly Glu
       290                 295                 300
   gta tct gaa aaa ata ata aca aga gca gac gga acc aga ctt gaa tac    960
   Val Ser Glu Lys Ile Ile Thr Arg Ala Asp Gly Thr Arg Leu Glu Tyr
   305                 310                 315                 320
   aca gga att aaa agc gat gga tct gga aaa gct aaa gag gtt tta aaa   1008
   Thr Gly Ile Lys Ser Asp Gly Ser Gly Lys Ala Lys Glu Val Leu Lys
                   325                 330                 335
   ggc tat gtt ctt gaa gga act cta act gct gaa aaa aca aca ttg gtg   1056
   Gly Tyr Val Leu Glu Gly Thr Leu Thr Ala Glu Lys Thr Thr Leu Val
               340                 345                 350
   gtt aaa gaa gga act gtt act tta agc aaa aat att tca aaa tct ggg   1104
   Val Lys Glu Gly Thr Val Thr Leu Ser Lys Asn Ile Ser Lys Ser Gly
           355                 360                 365
   gaa gtt tca gtt gaa ctt aat gac act gac agt agt gct gct act aaa   1152
   Glu Val Ser Val Glu Leu Asn Asp Thr Asp Ser Ser Ala Ala Thr Lys
       370                 375                 380
   aaa act gca gct tgg aat tca aaa act tct act tta aca att agt gtt   1200
   Lys Thr Ala Ala Trp Asn Ser Lys Thr Ser Thr Leu Thr Ile Ser Val
   385                 390                 395                 400
   aac agc aaa aaa act aca caa ctt gtg ttt act aaa caa tac aca ata   1248
   Asn Ser Lys Lys Thr Thr Gln Leu Val Phe Thr Lys Gln Tyr Thr Ile
                   405                 410                 415
   act gta aaa caa tac gac tcc gca ggt acc aat tta gaa ggc aca gca   1296
   Thr Val Lys Gln Tyr Asp Ser Ala Gly Thr Asn Leu Glu Gly Thr Ala
               420                 425                 430
   gtc gaa att aaa aca ctt gat gaa ctt aaa aac gct tta aaa taa       1341
   Val Glu Ile Lys Thr Leu Asp Glu Leu Lys Asn Ala Leu Lys  *
           435                 440                 445
<210> 158
   <211> 446
   <212> PRT
   <213> Artificial Sequence
<220>
   <223> Chimeric protein
<400> 158
   Met Ala Cys Asn Asn Ser Gly Lys Asp Gly Asn Thr Ser Ala Asn Ser
   1               5                   10                  15
   Ala Asp Glu Ser Val Lys Gly Pro Asn Leu Thr Glu Ile Ser Lys Lys
               20                  25                  30
   Ile Thr Asp Ser Asn Ala Val Leu Leu Ala Val Lys Glu Val Glu Ala
           35                  40                  45
   Leu Leu Ser Ser Ile Asp Glu Leu Ala Lys Ala Ile Gly Lys Lys Ile
       50                  55                  60
   Lys Asn Asp Gly Ser Leu Asp Asn Glu Ala Asn Arg Asn Glu Ser Leu
   65                  70                  75                  80
   Leu Ala Gly Ala Tyr Thr Ile Ser Thr Leu Ile Thr Gln Lys Leu Ser
                   85                  90                  95
   Lys Leu Asn Gly Ser Glu Gly Leu Lys Glu Lys Ile Ala Ala Ala Lys
               100                 105                 110
   Lys Cys Ser Glu Glu Phe Ser Thr Lys Leu Lys Asp Asn His Ala Gln
           115                 120                 125
   Leu Gly Ile Gln Gly Val Thr Asp Glu Asn Ala Lys Lys Ala Ile Leu
       130                 135                 140
   Lys Ala Asn Ala Ala Gly Lys Asp Lys Gly Val Glu Glu Leu Glu Lys
   145                 150                 155                 160
   Leu Ser Gly Ser Leu Glu Ser Leu Ser Lys Ala Ala Lys Glu Met Leu
                   165                 170                 175
   Ala Asn Ser Val Lys Glu Leu Thr Ser Pro Val Val His Gly Lys Gln
               180                 185                 190
   Asn Val Ser Ser Leu Asp Glu Lys Asn Ser Val Ser Val Asp Leu Pro
           195                 200                 205
   Gly Glu Met Lys Val Leu Val Ser Lys Glu Lys Asn Lys Asp Gly Lys
       210                 215                 220
   Tyr Asp Leu Ile Ala Thr Val Asp Lys Leu Glu Leu Lys Gly Thr Ser
   225                 230                 235                 240
   Asp Lys Asn Asn Gly Ser Gly Val Leu Glu Gly Val Lys Ala Asp Lys
                   245                 250                 255
   Ser Lys Val Lys Leu Thr Ile Ser Asp Asp Leu Gly Gln Thr Thr Leu
               260                 265                 270
   Glu Val Phe Lys Glu Asp Gly Lys Thr Leu Val Ser Lys Lys Val Thr
           275                 280                 285
   Ser Lys Asp Lys Ser Ser Thr Glu Glu Lys Phe Asn Glu Lys Gly Glu
       290                 295                 300
   Val Ser Glu Lys Ile Ile Thr Arg Ala Asp Gly Thr Arg Leu Glu Tyr
   305                 310                 315                 320
   Thr Gly Ile Lys Ser Asp Gly Ser Gly Lys Ala Lys Glu Val Leu Lys
                   325                 330                 335
   Gly Tyr Val Leu Glu Gly Thr Leu Thr Ala Glu Lys Thr Thr Leu Val
               340                 345                 350
   Val Lys Glu Gly Thr Val Thr Leu Ser Lys Asn Ile Ser Lys Ser Gly
           355                 360                 365
   Glu Val Ser Val Glu Leu Asn Asp Thr Asp Ser Ser Ala Ala Thr Lys
       370                 375                 380
   Lys Thr Ala Ala Trp Asn Ser Lys Thr Ser Thr Leu Thr Ile Ser Val
   385                 390                 395                 400
   Asn Ser Lys Lys Thr Thr Gln Leu Val Phe Thr Lys Gln Tyr Thr Ile
                   405                 410                 415
   Thr Val Lys Gln Tyr Asp Ser Ala Gly Thr Asn Leu Glu Gly Thr Ala
               420                 425                 430
   Val Glu Ile Lys Thr Leu Asp Glu Leu Lys Asn Ala Leu Lys
           435                 440                 445
<210> 159
   <211> 1365
   <212> DNA
   <213> Artificial Sequence
<220>
   <221> CDS
   <222> (1)...(1365)
   <223> Chimeric nucleic acid
<400> 159
   atg gct tgt aat aat tca ggg aaa gat ggg aat aca tct gca aat tct      48
   Met Ala Cys Asn Asn Ser Gly Lys Asp Gly Asn Thr Ser Ala Asn Ser
    1               5                  10                  15
   gct gat gag tct gtt aaa ggg cct aat ctt aca gaa ata agt aaa aaa      96
   Ala Asp Glu Ser Val Lys Gly Pro Asn Leu Thr Glu Ile Ser Lys Lys
               20                  25                  30
   att acg gat tct aat gcg gtt tta ctt gct gtg aaa gag gtt gaa gcg     144
   Ile Thr Asp Ser Asn Ala Val Leu Leu Ala Val Lys Glu Val Glu Ala
           35                  40                  45
   ttg ctg tca tct ata gat gaa att gct gct aaa gct att ggt aaa aaa     192
   Leu Leu Ser Ser Ile Asp Glu Ile Ala Ala Lys Ala Ile Gly Lys Lys
       50                  55                  60
   ata cac caa aat aat ggt ttg gat acc gaa tat aat cac aat gga tca     240
   Ile His Gln Asn Asn Gly Leu Asp Thr Glu Tyr Asn His Asn Gly Ser
   65                  70                  75                  80
   ttg tta gcg gga gct tat gca ata tca acc cta ata aaa caa aaa tta     288
   Leu Leu Ala Gly Ala Tyr Ala Ile Ser Thr Leu Ile Lys Gln Lys Leu
                   85                  90                  95
   gat gga ttg aaa aat gaa gga tta aag gaa aaa att gat gcg gct aag     336
   Asp Gly Leu Lys Asn Glu Gly Leu Lys Glu Lys Ile Asp Ala Ala Lys
               100                 105                 110
   aaa tgt tct gaa aca ttt act aat aaa tta aaa gaa aaa cac aca gat     384
   Lys Cys Ser Glu Thr Phe Thr Asn Lys Leu Lys Glu Lys His Thr Asp
           115                 120                 125
   ctt ggt aaa gaa ggt gtt act gat gct gat gca aaa gaa gcc att tta     432
   Leu Gly Lys Glu Gly Val Thr Asp Ala Asp Ala Lys Glu Ala Ile Leu
       130                 135                 140
   aaa aca aat ggt act aaa act aaa ggt gct gaa gaa ctt gga aaa tta     480
   Lys Thr Asn Gly Thr Lys Thr Lys Gly Ala Glu Glu Leu Gly Lys Leu
   145                 150                 155                 160
   ttt gaa tca gta gag gtc ttg tca aaa gca gct aaa gag atg ctt gct     528
   Phe Glu Ser Val Glu Val Leu Ser Lys Ala Ala Lys Glu Met Leu Ala
                   165                 170                 175
   aat tca gtt aaa gag ctt aca agc cct gtt gtg gca gaa agt cca aaa     576
   Asn Ser Val Lys Glu Leu Thr Ser Pro Val Val Ala Glu Ser Pro Lys
               180                 185                 190
   aaa cct tcc atg gcc aag caa aat gtt agc agc ctt gat gaa aaa aat     624
   Lys Pro Ser Met Ala Lys Gln Asn Val Ser Ser Leu Asp Glu Lys Asn
           195                 200                 205
   agc gtt tca gta gat tta cct ggt gga atg aca gtt ctt gta agt aaa     672
   Ser Val Ser Val Asp Leu Pro Gly Gly Met Thr Val Leu Val Ser Lys
       210                 215                 220
   gaa aaa gac aaa gac ggt aaa tac agt cta gag gca aca gta gac aag     720
   Glu Lys Asp Lys Asp Gly Lys Tyr Ser Leu Glu Ala Thr Val Asp Lys
   225                 230                 235                     240
   ctt gag ctt aaa gga act tct gat aaa aac aac ggt tct gga aca ctt     768
   Leu Glu Leu Lys Gly Thr Ser Asp Lys Asn Asn Gly Ser Gly Thr Leu
                   245                 250                 255
   gaa ggt gaa aaa act gac aaa agt aaa gta aaa tta aca att gct gat     816
   Glu Gly Glu Lys Thr Asp Lys Ser Lys Val Lys Leu Thr Ile Ala Asp
               260                 265                 270
   gac cta agt caa act aaa ttt gaa att ttc aaa gaa gat gcc aaa aca     864
   Asp Leu Ser Gln Thr Lys Phe Glu Ile Phe Lys Glu Asp Ala Lys Thr
           275                 280                 285
   tta gta tca aaa aaa gta acc ctt aaa gac aag tca tca aca gaa gaa     912
   Leu Val Ser Lys Lys Val Thr Leu Lys Asp Lys Ser Ser Thr Glu Glu
       290                 295                 300
   aaa ttc aac gaa aag ggt gaa aca tct gaa aaa aca ata gta aga gca     960
   Lys Phe Asn Glu Lys Gly Glu Thr Ser Glu Lys Thr Ile Val Arg Ala
   305                 310                 315                 320
   aat gga acc aga ctt gaa tac aca gac ata aaa agc gat gga tcc gga    1008
   Asn Gly Thr Arg Leu Glu Tyr Thr Asp Ile Lys Ser Asp Gly Ser Gly
                   325                 330                 335
   aaa gct aaa gaa gtt tta aaa gac ttt act ctt gaa gga act cta gct    1056
   Lys Ala Lys Glu Val Leu Lys Asp Phe Thr Leu Glu Gly Thr Leu Ala
               340                 345                 350
   gct gac ggc aaa aca aca ttg aaa gtt aca gaa ggc act gtt gtt tta    1104
   Ala Asp Gly Lys Thr Thr Leu Lys Val Thr Glu Gly Thr Val Val Leu
           355                 360                 365
   agc aag aac att tta aaa tcc gga gaa ata aca gtt gca ctt gat gac    1152
   Ser Lys Asn Ile Leu Lys Ser Gly Glu Ile Thr Val Ala Leu Asp Asp
       370                 375                 380
   tct gac act act cag gct act aaa aaa act gga aaa tgg gat tca aat    1200
   Ser Asp Thr Thr Gln Ala Thr Lys Lys Thr Gly Lys Trp Asp Ser Asn
   385                 390                 395                 400
   act tcc act tta aca att agt gtg aat agc aaa aaa act aaa aac att    1248
   Thr Ser Thr Leu Thr Ile Ser Val Asn Ser Lys Lys Thr Lys Asn Ile
                   405                 410                 415
   gta ttt aca aaa gaa gac aca ata aca gta caa aaa tac gac tca gca    1296
   Val Phe Thr Lys Glu Asp Thr Ile Thr Val Gln Lys Tyr Asp Ser Ala
               420                 425                 430
   ggc acc aat cta gaa ggc aac gca gtc gaa att aaa aca ctt gat gaa    1344
   Gly Thr Asn Leu Glu Gly Asn Ala Val Glu Ile Lys Thr Leu Asp Glu
           435                 440                 445
   ctt aaa aac gct tta aaa tag                                         1365
   Leu Lys Asn Ala Leu Lys  *
       450
<210> 160
   <211> 454
   <212> PRT
   <213> Artificial Sequence
<220>
   <223> Chimeric protein
<400> 160
   Met Ala Cys Asn Asn Ser Gly Lys Asp Gly Asn Thr Ser Ala Asn Ser
   1               5                   10                  15
   Ala Asp Glu Ser Val Lys Gly Pro Asn Leu Thr Glu Ile Ser Lys Lys
               20                  25                  30
   Ile Thr Asp Ser Asn Ala Val Leu Leu Ala Val Lys Glu Val Glu Ala
           35                  40                  45
   Leu Leu Ser Ser Ile Asp Glu Ile Ala Ala Lys Ala Ile Gly Lys Lys
       50                  55                  60
   Ile His Gln Asn Asn Gly Leu Asp Thr Glu Tyr Asn His Asn Gly Ser
   65                  70                  75                  80
   Leu Leu Ala Gly Ala Tyr Ala Ile Ser Thr Leu Ile Lys Gln Lys Leu
                   85                  90                  95
   Asp Gly Leu Lys Asn Glu Gly Leu Lys Glu Lys Ile Asp Ala Ala Lys
               100                 105                 110
   Lys Cys Ser Glu Thr Phe Thr Asn Lys Leu Lys Glu Lys His Thr Asp
           115                 120                 125
   Leu Gly Lys Glu Gly Val Thr Asp Ala Asp Ala Lys Glu Ala Ile Leu
       130                 135                 140
   Lys Thr Asn Gly Thr Lys Thr Lys Gly Ala Glu Glu Leu Gly Lys Leu
   145                 150                 155                 160
   Phe Glu Ser Val Glu Val Leu Ser Lys Ala Ala Lys Glu Met Leu Ala
                   165                 170                 175
   Asn Ser Val Lys Glu Leu Thr Ser Pro Val Val Ala Glu Ser Pro Lys
               180                 185                 190
   Lys Pro Ser Met Ala Lys Gln Asn Val Ser Ser Leu Asp Glu Lys Asn
           195                 200                 205
   Ser Val Ser Val Asp Leu Pro Gly Gly Met Thr Val Leu Val Ser Lys
       210                 215                 220
   Glu Lys Asp Lys Asp Gly Lys Tyr Ser Leu Glu Ala Thr Val Asp Lys
   225                 230                 235                 240
   Leu Glu Leu Lys Gly Thr Ser Asp Lys Asn Asn Gly Ser Gly Thr Leu
                   245                 250                 255
   Glu Gly Glu Lys Thr Asp Lys Ser Lys Val Lys Leu Thr Ile Ala Asp
               260                 265                 270
   Asp Leu Ser Gln Thr Lys Phe Glu Ile Phe Lys Glu Asp Ala Lys Thr
           275                 280                 285
   Leu Val Ser Lys Lys Val Thr Leu Lys Asp Lys Ser Ser Thr Glu Glu
       290                 295                 300
   Lys Phe Asn Glu Lys Gly Glu Thr Ser Glu Lys Thr Ile Val Arg Ala
   305                 310                 315                 320
   Asn Gly Thr Arg Leu Glu Tyr Thr Asp Ile Lys Ser Asp Gly Ser Gly
                   325                 330                 335
   Lys Ala Lys Glu Val Leu Lys Asp Phe Thr Leu Glu Gly Thr Leu Ala
               340                 345                 350
   Ala Asp Gly Lys Thr Thr Leu Lys Val Thr Glu Gly Thr Val Val Leu
           355                 360                 365
   Ser Lys Asn Ile Leu Lys Ser Gly Glu Ile Thr Val Ala Leu Asp Asp
       370                 375                 380
   Ser Asp Thr Thr Gln Ala Thr Lys Lys Thr Gly Lys Trp Asp Ser Asn
   385                 390                 395                 400
   Thr Ser Thr Leu Thr Ile Ser Val Asn Ser Lys Lys Thr Lys Asn Ile
                   405                 410                 415
   Val Phe Thr Lys Glu Asp Thr Ile Thr Val Gln Lys Tyr Asp Ser Ala
               420                 425                 430
   Gly Thr Asn Leu Glu Gly Asn Ala Val Glu Ile Lys Thr Leu Asp Glu
           435                 440                 445
   Leu Lys Asn Ala Leu Lys
       450
<210> 161
   <211> 1344
   <212> DNA
   <213> Artificial Sequence
<220>
   <221> CDS
   <222> (1)... (1344)
   <223> Chimeric nucleic acid
<400> 161
   atg gct tgt aat aat tca ggg aaa gat ggg aat aca tct gca aat tct      48
   Met Ala Cys Asn Asn Ser Gly Lys Asp Gly Asn Thr Ser Ala Asn Ser
    1               5                  10                  15
   gct gat gag tct gtt aaa ggg cct aat ctt aca gaa ata agt aaa aaa      96
   Ala Asp Glu Ser Val Lys Gly Pro Asn Leu Thr Glu Ile Ser Lys Lys
               20                  25                  30
   att acg gat tct aat gcg gtt tta ctt gct gtg aaa gag gtt gaa gcg     144
   Ile Thr Asp Ser Asn Ala Val Leu Leu Ala Val Lys Glu Val Glu Ala
           35                  40                  45
   ttg ctg tca tct ata gat gag ctt gct aaa gct att ggt aaa aaa ata     192
   Leu Leu Ser Ser Ile Asp Glu Leu Ala Lys Ala Ile Gly Lys Lys Ile
       50                  55                  60
   aaa aac gat ggt agt tta gat aat gaa gca aat cgc aac gag tca ttg     240
   Lys Asn Asp Gly Ser Leu Asp Asn Glu Ala Asn Arg Asn Glu Ser Leu
   65                  70                  75                  80
   tta gca gga gct tat aca ata tca acc tta ata aca caa aaa tta agt     288
   Leu Ala Gly Ala Tyr Thr Ile Ser Thr Leu Ile Thr Gln Lys Leu Ser
                   85                  90                  95
   aaa tta aac gga tca gaa ggt tta aag gaa aag att gcc gca gct aag     336
   Lys Leu Asn Gly Ser Glu Gly Leu Lys Glu Lys Ile Ala Ala Ala Lys
               100                 105                 110
   aaa tgc tct gaa gag ttt agt act aaa cta aaa gat aat cat gca cag     384
   Lys Cys Ser Glu Glu Phe Ser Thr Lys Leu Lys Asp Asn His Ala Gln
           115                 120                 125
   ctt ggt ata cag ggc gtt act gat gaa aat gca aaa aaa gct att tta      432
   Leu Gly Ile Gln Gly Val Thr Asp Glu Asn Ala Lys Lys Ala Ile Leu
       130                 135                 140
   aaa gca aat gca gcg ggt aaa gat aag ggc gtt gaa gaa ctt gaa aag      480
   Lys Ala Asn Ala Ala Gly Lys Asp Lys Gly Val Glu Glu Leu Glu Lys
   145                 150                 155                 160
   ttg tcc gga tca tta gaa agc tta tca aaa gca gct aaa gag atg ctt      528
   Leu Ser Gly Ser Leu Glu Ser Leu Ser Lys Ala Ala Lys Glu Met Leu
                   165                 170                 175
   gct aat tca gtt aaa gag ctt aca agc cct gtt gtc cat ggc aag caa      576
   Ala Asn Ser Val Lys Glu Leu Thr Ser Pro Val Val His Gly Lys Gln
               180                 185                 190
   aat gtt agc agc ctt gat gaa aaa aat agc gtt tca gta gat tta cct      624
   Asn Val Ser Ser Leu Asp Glu Lys Asn Ser Val Ser Val Asp Leu Pro
           195                 200                 205
   ggt gga atg aca gtt ctt gta agt aaa gaa aaa gac aaa gac ggt aaa      672
   Gly Gly Met Thr Val Leu Val Ser Lys Glu Lys Asp Lys Asp Gly Lys
       210                 215                 220
   tac agt cta gag gca aca gta gac aag ctt gag ctt aaa gga act tct      720
   Tyr Ser Leu Glu Ala Thr Val Asp Lys Leu Glu Leu Lys Gly Thr Ser
   225                 230                 235                 240
   gat aaa aac aac ggt tct gga aca ctt gaa ggt gaa aaa act gac aaa      768
   Asp Lys Asn Asn Gly Ser Gly Thr Leu Glu Gly Glu Lys Thr Asp Lys
                   245                 250                 255
   agt aaa gta aaa tta aca att gct gat gac cta agt caa act aaa ttt      816
   Ser Lys Val Lys Leu Thr Ile Ala Asp Asp Leu Ser Gln Thr Lys Phe
               260                 265                 270
   gaa att ttc aaa gaa gat gcc aaa aca tta gta tca aaa aaa gta acc      864
   Glu Ile Phe Lys Glu Asp Ala Lys Thr Leu Val Ser Lys Lys Val Thr
           275                 280                 285
   ctt aaa gac aag tca tca aca gaa gaa aaa ttc aac gaa aag ggt gaa      912
   Leu Lys Asp Lys Ser Ser Thr Glu Glu Lys Phe Asn Glu Lys Gly Glu
       290                 295                 300
   aca tct gaa aaa aca ata gta aga gca aat gga acc aga ctt gaa tac      960
   Thr Ser Glu Lys Thr Ile Val Arg Ala Asn Gly Thr Arg Leu Glu Tyr
   305                 310                 315                 320
   aca gac ata aaa agc gat gga tcc gga aaa gct aaa gaa gtt tta aaa     1008
   Thr Asp Ile Lys Ser Asp Gly Ser Gly Lys Ala Lys Glu Val Leu Lys
                   325                 330                 335
   gac ttt act ctt gaa gga act cta gct gct gac ggc aaa aca aca ttg     1056
   Asp Phe Thr Leu Glu Gly Thr Leu Ala Ala Asp Gly Lys Thr Thr Leu
               340                 345                 350
   aaa gtt aca gaa ggc act gtt gtt tta agc aag aac att tta aaa tcc     1104
   Lys Val Thr Glu Gly Thr Val Val Leu Ser Lys Asn Ile Leu Lys Ser
           355                 360                 365
   gga gaa ata aca gtt gca ctt gat gac tct gac act act cag gct act      1152
   Gly Glu Ile Thr Val Ala Leu Asp Asp Ser Asp Thr Thr Gln Ala Thr
       370                 375                 380
   aaa aaa act gga aaa tgg gat tca aat act tcc act tta aca att agt      1200
   Lys Lys Thr Gly Lys Trp Asp Ser Asn Thr Ser Thr Leu Thr Ile Ser
   385                 390                 395                 400
   gtg aat agc aaa aaa act aaa aac att gta ttt aca aaa gaa gac aca      1248
   Val Asn Ser Lys Lys Thr Lys Asn Ile Val Phe Thr Lys Glu Asp Thr
                   405                 410                 415
   ata aca gta caa aaa tac gac tca gca ggc acc aat cta gaa ggc aac      1296
   Ile Thr Val Gln Lys Tyr Asp Ser Ala Gly Thr Asn Leu Glu Gly Asn
               420                 425                 430
   gca gtc gaa att aaa aca ctt gat gaa ctt aaa aac gct tta aaa tag      1344
   Ala Val Glu Ile Lys Thr Leu Asp Glu Leu Lys Asn Ala Leu Lys  *
           435                 440                 445
<210> 162
   <211> 447
   <212> PRT
   <213> Artificial Sequence
<220>
   <223> Chimeric protein
<400> 162
   Met Ala Cys Asn Asn Ser Gly Lys Asp Gly Asn Thr Ser Ala Asn Ser
    1               5                  10                  15
   Ala Asp Glu Ser Val Lys Gly Pro Asn Leu Thr Glu Ile Ser Lys Lys
               20                  25                  30
   Ile Thr Asp Ser Asn Ala Val Leu Leu Ala Val Lys Glu Val Glu Ala
           35                  40                  45
   Leu Leu Ser Ser Ile Asp Glu Leu Ala Lys Ala Ile Gly Lys Lys Ile
       50                  55                  60
   Lys Asn Asp Gly Ser Leu Asp Asn Glu Ala Asn Arg Asn Glu Ser Leu
   65                  70                  75                  80
   Leu Ala Gly Ala Tyr Thr Ile Ser Thr Leu Ile Thr Gln Lys Leu Ser
                   85                  90                  95
   Lys Leu Asn Gly Ser Glu Gly Leu Lys Glu Lys Ile Ala Ala Ala Lys
               100                 105                 110
   Lys Cys Ser Glu Glu Phe Ser Thr Lys Leu Lys Asp Asn His Ala Gln
           115                 120                 125
   Leu Gly Ile Gln Gly Val Thr Asp Glu Asn Ala Lys Lys Ala Ile Leu
       130                 135                 140
   Lys Ala Asn Ala Ala Gly Lys Asp Lys Gly Val Glu Glu Leu Glu Lys
   145                 150                 155                 160
   Leu Ser Gly Ser Leu Glu Ser Leu Ser Lys Ala Ala Lys Glu Met Leu
                   165                 170                 175
   Ala Asn Ser Val Lys Glu Leu Thr Ser Pro Val Val His Gly Lys Gln
               180                 185                 190
   Asn Val Ser Ser Leu Asp Glu Lys Asn Ser Val Ser Val Asp Leu Pro
           195                 200                 205
   Gly Gly Met Thr Val Leu Val Ser Lys Glu Lys Asp Lys Asp Gly Lys
       210                 215                 220
   Tyr Ser Leu Glu Ala Thr Val Asp Lys Leu Glu Leu Lys Gly Thr Ser
   225                 230                 235                 240
   Asp Lys Asn Asn Gly Ser Gly Thr Leu Glu Gly Glu Lys Thr Asp Lys
                   245                 250                 255
   Ser Lys Val Lys Leu Thr Ile Ala Asp Asp Leu Ser Gln Thr Lys Phe
               260                 265                 270
   Glu Ile Phe Lys Glu Asp Ala Lys Thr Leu Val Ser Lys Lys Val Thr
           275                 280                 285
   Leu Lys Asp Lys Ser Ser Thr Glu Glu Lys Phe Asn Glu Lys Gly Glu
       290                 295                 300
   Thr Ser Glu Lys Thr Ile Val Arg Ala Asn Gly Thr Arg Leu Glu Tyr
   305                 310                 315                 320
   Thr Asp Ile Lys Ser Asp Gly Ser Gly Lys Ala Lys Glu Val Leu Lys
                   325                 330                 335
   Asp Phe Thr Leu Glu Gly Thr Leu Ala Ala Asp Gly Lys Thr Thr Leu
               340                 345                 350
   Lys Val Thr Glu Gly Thr Val Val Leu Ser Lys Asn Ile Leu Lys Ser
           355                 360                 365
   Gly Glu Ile Thr Val Ala Leu Asp Asp Ser Asp Thr Thr Gln Ala Thr
       370                 375                 380
   Lys Lys Thr Gly Lys Trp Asp Ser Asn Thr Ser Thr Leu Thr Ile Ser
   385                 390                 395                 400
   Val Asn Ser Lys Lys Thr Lys Asn Ile Val Phe Thr Lys Glu Asp Thr
                   405                 410                 415
   Ile Thr Val Gln Lys Tyr Asp Ser Ala Gly Thr Asn Leu Glu Gly Asn
               420                 425                 430
   Ala Val Glu Ile Lys Thr Leu Asp Glu Leu Lys Asn Ala Leu Lys
           435                 440                 445
<210> 163
   <211> 1305
   <212> DNA
   <213> Artificial Sequence
<220>
   <221> CDS
   <222> (1)...(1305)
   <223> Chimeric nucleic acid
<400> 163
   atg gct tgt aat aat tca gga aaa gat ggg aat aca tct gca aat tct      48
   Met Ala Cys Asn Asn Ser Gly Lys Asp Gly Asn Thr Ser Ala Asn Ser
    1               5                  10                  15
   gct gat gag tct gtt aaa ggg cct aat ctt aca gaa ata agt aaa aaa      96
   Ala Asp Glu Ser Val Lys Gly Pro Asn Leu Thr Glu Ile Ser Lys Lys
               20                  25                  30
   att aca gaa tct aac gca gtt gtt ctg gct gtg aaa gaa att gaa act     144
   Ile Thr Glu Ser Asn Ala Val Val Leu Ala Val Lys Glu Ile Glu Thr
           35                  40                  45
   ttg ctt gca tct ata gat gaa ctt gct act aaa gct att ggt aaa aaa     192
   Leu Leu Ala Ser Ile Asp Glu Leu Ala Thr Lys Ala Ile Gly Lys Lys
       50                  55                  60
   ata caa caa aat ggt ggt tta gct gtc gaa gcg ggg cat aat gga aca     240
   Ile Gln Gln Asn Gly Gly Leu Ala Val Glu Ala Gly His Asn Gly Thr
   65                  70                  75                  80
   ttg tta gca ggt gct tat aca ata tca aaa cta ata aca caa aaa tta    288
   Leu Leu Ala Gly Ala Tyr Thr Ile Ser Lys Leu Ile Thr Gln Lys Leu
                   85                  90                  95
   gat gga ttg aaa aat tca gaa aaa tta aag gaa aaa att gaa aat gct    336
   Asp Gly Leu Lys Asn Ser Glu Lys Leu Lys Glu Lys Ile Glu Asn Ala
               100                 105                 110
   aag aaa tgt tct gaa gat ttt act aaa aaa cta gaa gga gaa cat gcg    384
   Lys Lys Cys Ser Glu Asp Phe Thr Lys Lys Leu Glu Gly Glu His Ala
           115                 120                 125
   caa ctt gga att gaa aat gtt act gat gag aat gca aaa aaa gct att    432
   Gln Leu Gly Ile Glu Asn Val Thr Asp Glu Asn Ala Lys Lys Ala Ile
       130                 135                 140
   tta ata aca gat gca gct aaa gat aag ggc gct gca gag ctt gaa aag    480
   Leu Ile Thr Asp Ala Ala Lys Asp Lys Gly Ala Ala Glu Leu Glu Lys
   145                 150                 155                 160
   cta ttt aaa gca gta gaa aac ttg gca aaa gca gct aaa gag atg ctt    528
   Leu Phe Lys Ala Val Glu Asn Leu Ala Lys Ala Ala Lys Glu Met Leu
                   165                 170                 175
   gct aat tca gtt aaa gag ctt aca agt cct att gtg cat ggc gtt tca    576
   Ala Asn Ser Val Lys Glu Leu Thr Ser Pro Ile Val His Gly Val Ser
               180                 185                 190
   gta gat ttg cct ggt gaa atg aaa gtt ctt gta agc aaa gaa aaa aac    624
   Val Asp Leu Pro Gly Glu Met Lys Val Leu Val Ser Lys Glu Lys Asn
           195                 200                 205
   aaa gac ggc aag tac gat cta att gca aca gta gac aag ctt gag ctt    672
   Lys Asp Gly Lys Tyr Asp Leu Ile Ala Thr Val Asp Lys Leu Glu Leu
       210                 215                 220
   aaa gga act tct gat aaa aac aat gga tct gga gta ctt gaa ggc gta    720
   Lys Gly Thr Ser Asp Lys Asn Asn Gly Ser Gly Val Leu Glu Gly Val
   225                 230                 235                 240
   aaa gct gac aaa agt aaa gta aaa tta aca att tct gac gat cta ggt    768
   Lys Ala Asp Lys Ser Lys Val Lys Leu Thr Ile Ser Asp Asp Leu Gly
                   245                 250                 255
   caa acc aca ctt gaa gtt ttc aaa gaa gat ggc aaa aca cta gta tca    816
   Gln Thr Thr Leu Glu Val Phe Lys Glu Asp Gly Lys Thr Leu Val Ser
               260                 265                 270
   aaa aaa gta act tcc aaa gac aag tca tca aca gaa gaa aaa ttc aat    864
   Lys Lys Val Thr Ser Lys Asp Lys Ser Ser Thr Glu Glu Lys Phe Asn
           275                 280                 285
   gaa aaa ggt gaa gta tct gaa aaa ata ata aca aga gca gac gga acc    912
   Glu Lys Gly Glu Val Ser Glu Lys Ile Ile Thr Arg Ala Asp Gly Thr
       290                 295                 300
   aga ctt gaa tac aca gga att aaa agc gat gga tct gga aaa gct aaa    960
   Arg Leu Glu Tyr Thr Gly Ile Lys Ser Asp Gly Ser Gly Lys Ala Lys
   305                 310                 315                 320
   gag gtt tta aaa aaa ttt act ctt gaa gga aaa gta gct aat gat aaa      1008
   Glu Val Leu Lys Lys Phe Thr Leu Glu Gly Lys Val Ala Asn Asp Lys
                   325                 330                 335
   gta aca ttg gaa gta aaa gaa gga acc gtt act tta agt aag aac att      1056
   Val Thr Leu Glu Val Lys Glu Gly Thr Val Thr Leu Ser Lys Asn Ile
               340                 345                 350
   tca aaa tct ggg gaa gtt tca gtt gaa ctt aat gac act gac agt agt      1104
   Ser Lys Ser Gly Glu Val Ser Val Glu Leu Asn Asp Thr Asp Ser Ser
           355                 360                 365
   gct gct act aaa aaa act gca gct tgg aat tca ggc act tca act tta      1152
   Ala Ala Thr Lys Lys Thr Ala Ala Trp Asn Ser Gly Thr Ser Thr Leu
       370                 375                 380
   aca att act gta aac agt aaa aaa act aaa gac ctt gtg ttt aca aaa      1200
   Thr Ile Thr Val Asn Ser Lys Lys Thr Lys Asp Leu Val Phe Thr Lys
   385                 390                 395                 400
   gaa aac aca att aca gta caa caa tac gac tca aat ggc acc aaa tta      1248
   Glu Asn Thr Ile Thr Val Gln Gln Tyr Asp Ser Asn Gly Thr Lys Leu
                   405                 410                 415
   gag ggg tca gca gtt gaa att aca aaa ctt gat gaa att aaa aac gct      1296
   Glu Gly Ser Ala Val Glu Ile Thr Lys Leu Asp Glu Ile Lys Asn Ala
               420                 425                 430
   tta aaa taa                                                          1305
   Leu Lys  *
<210> 164
   <211> 434
   <212> PRT
   <213> Artificial Sequence
<220>
   <223> Chimeric protein
<400> 164
   Met Ala Cys Asn Asn Ser Gly Lys Asp Gly Asn Thr Ser Ala Asn Ser
    1               5                  10                  15
   Ala Asp Glu Ser Val Lys Gly Pro Asn Leu Thr Glu Ile Ser Lys Lys
               20                  25                  30
   Ile Thr Glu Ser Asn Ala Val Val Leu Ala Val Lys Glu Ile Glu Thr
           35                  40                  45
   Leu Leu Ala Ser Ile Asp Glu Leu Ala Thr Lys Ala Ile Gly Lys Lys
       50                  55                  60
   Ile Gln Gln Asn Gly Gly Leu Ala Val Glu Ala Gly His Asn Gly Thr
   65                  70                  75                  80
   Leu Leu Ala Gly Ala Tyr Thr Ile Ser Lys Leu Ile Thr Gln Lys Leu
                   85                  90                  95
   Asp Gly Leu Lys Asn Ser Glu Lys Leu Lys Glu Lys Ile Glu Asn Ala
               100                 105                 110
   Lys Lys Cys Ser Glu Asp Phe Thr Lys Lys Leu Glu Gly Glu His Ala
           115                 120                 125
   Gln Leu Gly Ile Glu Asn Val Thr Asp Glu Asn Ala Lys Lys Ala Ile
       130                 135                 140
   Leu Ile Thr Asp Ala Ala Lys Asp Lys Gly Ala Ala Glu Leu Glu Lys
   145                 150                 155                 160
   Leu Phe Lys Ala Val Glu Asn Leu Ala Lys Ala Ala Lys Glu Met Leu
                   165                 170                 175
   Ala Asn Ser Val Lys Glu Leu Thr Ser Pro Ile Val His Gly Val Ser
               180                 185                 190
   Val Asp Leu Pro Gly Glu Met Lys Val Leu Val Ser Lys Glu Lys Asn
           195                 200                 205
   Lys Asp Gly Lys Tyr Asp Leu Ile Ala Thr Val Asp Lys Leu Glu Leu
       210                 215                 220
   Lys Gly Thr Ser Asp Lys Asn Asn Gly Ser Gly Val Leu Glu Gly Val
   225                 230                 235                 240
   Lys Ala Asp Lys Ser Lys Val Lys Leu Thr Ile Ser Asp Asp Leu Gly
                   245                 250                 255
   Gln Thr Thr Leu Glu Val Phe Lys Glu Asp Gly Lys Thr Leu Val Ser
               260                 265                 270
   Lys Lys Val Thr Ser Lys Asp Lys Ser Ser Thr Glu Glu Lys Phe Asn
           275                 280                 285
   Glu Lys Gly Glu Val Ser Glu Lys Ile Ile Thr Arg Ala Asp Gly Thr
       290                 295                 300
   Arg Leu Glu Tyr Thr Gly Ile Lys Ser Asp Gly Ser Gly Lys Ala Lys
   305                 310                 315                 320
   Glu Val Leu Lys Lys Phe Thr Leu Glu Gly Lys Val Ala Asn Asp Lys
                   325                 330                 335
   Val Thr Leu Glu Val Lys Glu Gly Thr Val Thr Leu Ser Lys Asn Ile
               340                 345                 350
   Ser Lys Ser Gly Glu Val Ser Val Glu Leu Asn Asp Thr Asp Ser Ser
           355                 360                 365
   Ala Ala Thr Lys Lys Thr Ala Ala Trp Asn Ser Gly Thr Ser Thr Leu
       370                 375                 380
   Thr Ile Thr Val Asn Ser Lys Lys Thr Lys Asp Leu Val Phe Thr Lys
   385                 390                 395                 400
   Glu Asn Thr Ile Thr Val Gln Gln Tyr Asp Ser Asn Gly Thr Lys Leu
                   405                 410                 415
   Glu Gly Ser Ala Val Glu Ile Thr Lys Leu Asp Glu Ile Lys Asn Ala
               420                 425                 430
   Leu Lys
<210> 165
   <211> 1332
   <212> DNA
   <213> Artificial Sequence
<220>
   <221> CDS
   <222> (1)...(1332)
   <223> Chimeric nucleic acid
<400> 165
   atg gct tgt agt aat tca ggg aaa ggt ggg gat tct gca tct act aat      48
   Met Ala Cys Ser Asn Ser Gly Lys Gly Gly Asp Ser Ala Ser Thr Asn
    1               5                  10                  15
   cct gct gac gag tct gcg aaa ggg cct aat ctt aca gaa ata agc aaa      96
   Pro Ala Asp Glu Ser Ala Lys Gly Pro Asn Leu Thr Glu Ile Ser Lys
               20                  25                  30
   aaa att aca gat tct aat gca ttt gta ctt gct gtt aaa gaa gtt gag     144
   Lys Ile Thr Asp Ser Asn Ala Phe Val Leu Ala Val Lys Glu Val Glu
           35                  40                  45
   act ttg gtt tta tct ata gat gaa ctt gct aag aaa gct att ggt caa   192
   Thr Leu Val Leu Ser Ile Asp Glu Leu Ala Lys Lys Ala Ile Gly Gln
        50                  55                  60
   aaa ata gac aat aat aat ggt tta gct gct tta aat aat cag aat gga   240
   Lys Ile Asp Asn Asn Asn Gly Leu Ala Ala Leu Asn Asn Gln Asn Gly
    65                  70                  75                  80
   tcg ttg tta gca gga gcc tat gca ata tca acc cta ata aca gaa aaa   288
   Ser Leu Leu Ala Gly Ala Tyr Ala Ile Ser Thr Leu Ile Thr Glu Lys
                    85                  90                  95
   ttg agt aaa ttg aaa aat tta gaa gaa tta aag aca gaa att gca aag   336
   Leu Ser Lys Leu Lys Asn Leu Glu Glu Leu Lys Thr Glu Ile Ala Lys
               100                 105                 110
   gct aag aaa tgt tcc gaa gaa ttt act aat aaa cta aaa agt ggt cat   384
   Ala Lys Lys Cys Ser Glu Glu Phe Thr Asn Lys Leu Lys Ser Gly His
           115                 120                 125
   gca gat ctt ggc aaa cag gat gct acc gat gat cat gca aaa gca gct   432
   Ala Asp Leu Gly Lys Gln Asp Ala Thr Asp Asp His Ala Lys Ala Ala
       130                 135                 140
   att tta aaa aca cat gca act acc gat aaa ggt gct aaa gaa ttt aaa   480
   Ile Leu Lys Thr His Ala Thr Thr Asp Lys Gly Ala Lys Glu Phe Lys
   145                 150                 155                 160
   gat tta ttt gaa tca gta gaa ggt ttg tta aaa gca gct caa gta gca   528
   Asp Leu Phe Glu Ser Val Glu Gly Leu Leu Lys Ala Ala Gln Val Ala
                   165                 170                 175
   cta act aat tca gtt aaa gaa ctt aca agt cct gtt gta gca gaa agt   576
   Leu Thr Asn Ser Val Lys Glu Leu Thr Ser Pro Val Val Ala Glu Ser
               180                 185                 190
   cca aaa aaa cct tcc atg gcc gtt tca gta gat ttg cct ggt gaa atg   624
   Pro Lys Lys Pro Ser Met Ala Val Ser Val Asp Leu Pro Gly Glu Met
           195                 200                 205
   aaa gtt ctt gta agc aaa gaa aaa aac aaa gac ggc aag tac gat cta   672
   Lys Val Leu Val Ser Lys Glu Lys Asn Lys Asp Gly Lys Tyr Asp Leu
       210                 215                 220
   att gca aca gta gac aag ctt gag ctt aaa gga act tct gat aaa aac   720
   Ile Ala Thr Val Asp Lys Leu Glu Leu Lys Gly Thr Ser Asp Lys Asn
   225                 230                 235                 240
   aat gga tct gga gta ctt gaa ggc gta aaa gct gac aaa agt aaa gta   768
   Asn Gly Ser Gly Val Leu Glu Gly Val Lys Ala Asp Lys Ser Lys Val
                   245                 250                 255
   aaa tta aca att tct gac gat cta ggt caa acc aca ctt gaa gtt ttc   816
   Lys Leu Thr Ile Ser Asp Asp Leu Gly Gln Thr Thr Leu Glu Val Phe
               260                 265                 270
   aaa gaa gat ggc aaa aca cta gta tca aaa aaa gta act tcc aaa gac   864
   Lys Glu Asp Gly Lys Thr Leu Val Ser Lys Lys Val Thr Ser Lys Asp
           275                 280                 285
   aag tca tca aca gaa gaa aaa ttc aat gaa aaa ggt gaa gta tct gaa     912
   Lys Ser Ser Thr Glu Glu Lys Phe Asn Glu Lys Gly Glu Val Ser Glu
       290                 295                 300
   aaa ata ata aca aga gca gac gga acc aga ctt gaa tac aca gga att     960
   Lys Ile Ile Thr Arg Ala Asp Gly Thr Arg Leu Glu Tyr Thr Gly Ile
   305                 310                 315                 320
   aaa agc gat gga tct gga aaa gct aaa gag gtt tta aaa aaa ttt act     1008
   Lys Ser Asp Gly Ser Gly Lys Ala Lys Glu Val Leu Lys Lys Phe Thr
                   325                 330                 335
   ctt gaa gga aaa gta gct aat gat aaa gta aca ttg gaa gta aaa gaa     1056
   Leu Glu Gly Lys Val Ala Asn Asp Lys Val Thr Leu Glu Val Lys Glu
               340                 345                 350
   gga acc gtt act tta agt aag aat att tca aaa tct ggg gaa gtt tca     1104
   Gly Thr Val Thr Leu Ser Lys Asn Ile Ser Lys Ser Gly Glu Val Ser
           355                 360                 365
   gtt gaa ctt aat gac act gac agt agt gct gct act aaa aaa act gca     1152
   Val Glu Leu Asn Asp Thr Asp Ser Ser Ala Ala Thr Lys Lys Thr Ala
       370                 375                 380
   gct tgg aat tca aaa act tcc act tta aca att agt gtg aat agc caa     1200
   Ala Trp Asn Ser Lys Thr Ser Thr Leu Thr Ile Ser Val Asn Ser Gln
   385                 390                 395                 400
   aaa acc aaa aac ctt gta ttc aca aaa gaa gac aca ata aca gta caa     1248
   Lys Thr Lys Asn Leu Val Phe Thr Lys Glu Asp Thr Ile Thr Val Gln
                   405                 410                 415
   aaa tac gac tca gca ggc acc aat cta gaa ggc aaa gca gtc gaa att     1296
   Lys Tyr Asp Ser Ala Gly Thr Asn Leu Glu Gly Lys Ala Val Glu Ile
               420                 425                 430
   aca aca ctt aaa gaa ctt aaa aac gct tta aaa taa                     1332
   Thr Thr Leu Lys Glu Leu Lys Asn Ala Leu Lys  *
           435                 440
<210> 166
   <211> 443
   <212> PRT
   <213> Artificial Sequence
<220>
   <223> Chimeric protein
<400> 166
   Met Ala Cys Ser Asn Ser Gly Lys Gly Gly Asp Ser Ala Ser Thr Asn
   1               5                   10                  15
   Pro Ala Asp Glu Ser Ala Lys Gly Pro Asn Leu Thr Glu Ile Ser Lys
               20                  25                  30
   Lys Ile Thr Asp Ser Asn Ala Phe Val Leu Ala Val Lys Glu Val Glu
           35                  40                  45
   Thr Leu Val Leu Ser Ile Asp Glu Leu Ala Lys Lys Ala Ile Gly Gln
       50                  55                  60
   Lys Ile Asp Asn Asn Asn Gly Leu Ala Ala Leu Asn Asn Gln Asn Gly
   65                  70                  75                  80
   Ser Leu Leu Ala Gly Ala Tyr Ala Ile Ser Thr Leu Ile Thr Glu Lys
                   85                  90                  95
   Leu Ser Lys Leu Lys Asn Leu Glu Glu Leu Lys Thr Glu Ile Ala Lys
               100                 105                 110
   Ala Lys Lys Cys Ser Glu Glu Phe Thr Asn Lys Leu Lys Ser Gly His
           115                 120                 125
   Ala Asp Leu Gly Lys Gln Asp Ala Thr Asp Asp His Ala Lys Ala Ala
       130                 135                 140
   Ile Leu Lys Thr His Ala Thr Thr Asp Lys Gly Ala Lys Glu Phe Lys
   145                 150                 155                 160
   Asp Leu Phe Glu Ser Val Glu Gly Leu Leu Lys Ala Ala Gln Val Ala
                   165                 170                 175
   Leu Thr Asn Ser Val Lys Glu Leu Thr Ser Pro Val Val Ala Glu Ser
               180                 185                 190
   Pro Lys Lys Pro Ser Met Ala Val Ser Val Asp Leu Pro Gly Glu Met
           195                 200                 205
   Lys Val Leu Val Ser Lys Glu Lys Asn Lys Asp Gly Lys Tyr Asp Leu
       210                 215                 220
   Ile Ala Thr Val Asp Lys Leu Glu Leu Lys Gly Thr Ser Asp Lys Asn
   225                 230                 235                 240
   Asn Gly Ser Gly Val Leu Glu Gly Val Lys Ala Asp Lys Ser Lys Val
                   245                 250                 255
   Lys Leu Thr Ile Ser Asp Asp Leu Gly Gln Thr Thr Leu Glu Val Phe
               260                 265                 270
   Lys Glu Asp Gly Lys Thr Leu Val Ser Lys Lys Val Thr Ser Lys Asp
           275                 280                 285
   Lys Ser Ser Thr Glu Glu Lys Phe Asn Glu Lys Gly Glu Val Ser Glu
       290                 295                 300
   Lys Ile Ile Thr Arg Ala Asp Gly Thr Arg Leu Glu Tyr Thr Gly Ile
   305                 310                 315                 320
   Lys Ser Asp Gly Ser Gly Lys Ala Lys Glu Val Leu Lys Lys Phe Thr
                   325                 330                 335
   Leu Glu Gly Lys Val Ala Asn Asp Lys Val Thr Leu Glu Val Lys Glu
               340                 345                 350
   Gly Thr Val Thr Leu Ser Lys Asn Ile Ser Lys Ser Gly Glu Val Ser
           355                 360                 365
   Val Glu Leu Asn Asp Thr Asp Ser Ser Ala Ala Thr Lys Lys Thr Ala
       370                 375                 380
   Ala Trp Asn Ser Lys Thr Ser Thr Leu Thr Ile Ser Val Asn Ser Gln
   385                 390                 395                 400
   Lys Thr Lys Asn Leu Val Phe Thr Lys Glu Asp Thr Ile Thr Val Gln
                   405                 410                 415
   Lys Tyr Asp Ser Ala Gly Thr Asn Leu Glu Gly Lys Ala Val Glu Ile
               420                 425                 430
   Thr Thr Leu Lys Glu Leu Lys Asn Ala Leu Lys
           435                 440
<210> 167
   <211> 1317
   <212> DNA
   <213> Artificial Sequence
<220>
   <221> CDS
   <222> (1)...(1317)
   <223> Chimeric nucleic acid
<400> 167
   atg gct tgt aat aat tca ggt ggg gat tct gca tct act aat cct gat      48
   Met Ala Cys Asn Asn Ser Gly Gly Asp Ser Ala Ser Thr Asn Pro Asp
    1               5                  10                  15
   gag tct gca aaa gga cct aat ctt acc gta ata agc aaa aaa att aca      96
   Glu Ser Ala Lys Gly Pro Asn Leu Thr Val Ile Ser Lys Lys Ile Thr
               20                  25                  30
   gat tct aat gca ttt tta ctg gct gtg aaa gaa gtt gag gct ttg ctt     144
   Asp Ser Asn Ala Phe Leu Leu Ala Val Lys Glu Val Glu Ala Leu Leu
           35                  40                  45
   tca tct ata gat gaa ctt tct aaa gct att ggt aaa aaa ata aaa aat     192
   Ser Ser Ile Asp Glu Leu Ser Lys Ala Ile Gly Lys Lys Ile Lys Asn
       50                  55                  60
   gat ggt act tta gat aac gaa gca aat cga aac gaa tca ttg ata gca     240
   Asp Gly Thr Leu Asp Asn Glu Ala Asn Arg Asn Glu Ser Leu Ile Ala
   65                  70                  75                  80
   gga gct tat gaa ata tca aaa cta ata aca caa aaa tta agt gta ttg     288
   Gly Ala Tyr Glu Ile Ser Lys Leu Ile Thr Gln Lys Leu Ser Val Leu
                   85                  90                  95
   aat tca gaa gaa tta aag gaa aaa att aaa gag gct aag gat tgt tcc     336
   Asn Ser Glu Glu Leu Lys Glu Lys Ile Lys Glu Ala Lys Asp Cys Ser
               100                 105                 110
   gaa aaa ttt act act aag cta aaa gat agt cat gca gag ctt ggt ata     384
   Glu Lys Phe Thr Thr Lys Leu Lys Asp Ser His Ala Glu Leu Gly Ile
           115                 120                 125
   caa agc gtt cag gat gat aat gca aaa aaa gct att tta aaa aca cat     432
   Gln Ser Val Gln Asp Asp Asn Ala Lys Lys Ala Ile Leu Lys Thr His
       130                 135                 140
   gga act aaa gac aag ggt gct aaa gaa ctt gaa gag tta ttt aaa tca     480
   Gly Thr Lys Asp Lys Gly Ala Lys Glu Leu Glu Glu Leu Phe Lys Ser
   145                 150                 155                 160
   cta gaa agc ttg tca aaa gca gcg caa gca gca tta act aat tca gtt     528
   Leu Glu Ser Leu Ser Lys Ala Ala Gln Ala Ala Leu Thr Asn Ser Val
                   165                 170                 175
   aaa gag ctt aca aat cct gtt gtg gca gaa agt cca aaa aaa cct tcc     576
   Lys Glu Leu Thr Asn Pro Val Val Ala Glu Ser Pro Lys Lys Pro Ser
               180                 185                 190
   atg gcc gtt tca gta gat ttg cct ggt gaa atg aaa gtt ctt gta agc     624
   Met Ala Val Ser Val Asp Leu Pro Gly Glu Met Lys Val Leu Val Ser
           195                 200                 205
   aaa gaa aaa aac aaa gac ggc aag tac gat cta att gca aca gta gac     672
   Lys Glu Lys Asn Lys Asp Gly Lys Tyr Asp Leu Ile Ala Thr Val Asp
       210                 215                 220
   aag ctt gag ctt aaa gga act tct gat aaa aac aat gga tct gga gta     720
   Lys Leu Glu Leu Lys Gly Thr Ser Asp Lys Asn Asn Gly Ser Gly Val
   225                 230                 235                 240
   ctt gaa ggc gta aaa gct gac aaa agt aaa gta aaa tta aca att tct     768
   Leu Glu Gly Val Lys Ala Asp Lys Ser Lys Val Lys Leu Thr Ile Ser
                   245                 250                 255
   gac gat cta ggt caa acc aca ctt gaa gtt ttc aaa gaa gat ggc aaa     816
   Asp Asp Leu Gly Gln Thr Thr Leu Glu Val Phe Lys Glu Asp Gly Lys
               260                 265                 270
   aca cta gta tca aaa aaa gta act tcc aaa gac aag tca tca aca gaa     864
   Thr Leu Val Ser Lys Lys Val Thr Ser Lys Asp Lys Ser Ser Thr Glu
           275                 280                 285
   gaa aaa ttc aat gaa aaa ggt gaa gta tct gaa aaa ata ata aca aga     912
   Glu Lys Phe Asn Glu Lys Gly Glu Val Ser Glu Lys Ile Ile Thr Arg
       290                 295                 300
   gca gac gga acc aga ctt gaa tac aca gga att aaa agc gat gga tct     960
   Ala Asp Gly Thr Arg Leu Glu Tyr Thr Gly Ile Lys Ser Asp Gly Ser
   305                 310                 315                 320
   gga aaa gct aaa gag gtt tta aaa aaa ttt act ctt gaa gga aaa gta    1008
   Gly Lys Ala Lys Glu Val Leu Lys Lys Phe Thr Leu Glu Gly Lys Val
                   325                 330                 335
   gct aat gat aaa gta aca ttg gaa gta aaa gaa gga acc gtt act tta    1056
   Ala Asn Asp Lys Val Thr Leu Glu Val Lys Glu Gly Thr Val Thr Leu
               340                 345                 350
   agt aag aac att tca aaa tct ggg gaa gtt tca gtt gaa ctt aat gac    1104
   Ser Lys Asn Ile Ser Lys Ser Gly Glu Val Ser Val Glu Leu Asn Asp
           355                 360                 365
   act gac agt agt gct gct act aaa aaa act gca gct tgg aat tca aaa    1152
   Thr Asp Ser Ser Ala Ala Thr Lys Lys Thr Ala Ala Trp Asn Ser Lys
       370                 375                 380
   act tct act tta aca att agt gtt aac agc aaa aaa act aca caa ctt    1200
   Thr Ser Thr Leu Thr Ile Ser Val Asn Ser Lys Lys Thr Thr Gln Leu
   385                 390                 395                 400
   gtg ttt act aaa caa gac aca ata act gta caa aaa tac gac tcc gca    1248
   Val Phe Thr Lys Gln Asp Thr Ile Thr Val Gln Lys Tyr Asp Ser Ala
                   405                 410                 415
   ggt acc aat tta gaa ggc aca gca gtc gaa att aaa aca ctt gat gaa    1296
   Gly Thr Asn Leu Glu Gly Thr Ala Val Glu Ile Lys Thr Leu Asp Glu
               420                 425                 430
   ctt aaa aac gct tta aaa taa                                        1317
   Leu Lys Asn Ala Leu Lys  *
           435
<210> 168
   <211> 438
   <212> PRT
   <213> Artificial Sequence
<220>
   <223> Chimeric protein
<400> 168
   Met Ala Cys Asn Asn Ser Gly Gly Asp Ser Ala Ser Thr Asn Pro Asp
   1               5                   10                  15
   Glu Ser Ala Lys Gly Pro Asn Leu Thr Val Ile Ser Lys Lys Ile Thr
               20                  25                  30
   Asp Ser Asn Ala Phe Leu Leu Ala Val Lys Glu Val Glu Ala Leu Leu
           35                  40                  45
   Ser Ser Ile Asp Glu Leu Ser Lys Ala Ile Gly Lys Lys Ile Lys Asn
       50                  55                  60
   Asp Gly Thr Leu Asp Asn Glu Ala Asn Arg Asn Glu Ser Leu Ile Ala
   65                  70                  75                  80
   Gly Ala Tyr Glu Ile Ser Lys Leu Ile Thr Gln Lys Leu Ser Val Leu
                   85                  90                  95
   Asn Ser Glu Glu Leu Lys Glu Lys Ile Lys Glu Ala Lys Asp Cys Ser
               100                 105                 110
   Glu Lys Phe Thr Thr Lys Leu Lys Asp Ser His Ala Glu Leu Gly Ile
           115                 120                 125
   Gln Ser Val Gln Asp Asp Asn Ala Lys Lys Ala Ile Leu Lys Thr His
       130                 135                 140
   Gly Thr Lys Asp Lys Gly Ala Lys Glu Leu Glu Glu Leu Phe Lys Ser
   145                 150                 155                 160
   Leu Glu Ser Leu Ser Lys Ala Ala Gln Ala Ala Leu Thr Asn Ser Val
                   165                 170                 175
   Lys Glu Leu Thr Asn Pro Val Val Ala Glu Ser Pro Lys Lys Pro Ser
               180                 185                 190
   Met Ala Val Ser Val Asp Leu Pro Gly Glu Met Lys Val Leu Val Ser
           195                 200                 205
   Lys Glu Lys Asn Lys Asp Gly Lys Tyr Asp Leu Ile Ala Thr Val Asp
       210                 215                 220
   Lys Leu Glu Leu Lys Gly Thr Ser Asp Lys Asn Asn Gly Ser Gly Val
   225                 230                 235                 240
   Leu Glu Gly Val Lys Ala Asp Lys Ser Lys Val Lys Leu Thr Ile Ser
                   245                 250                 255
   Asp Asp Leu Gly Gln Thr Thr Leu Glu Val Phe Lys Glu Asp Gly Lys
               260                 265                 270
   Thr Leu Val Ser Lys Lys Val Thr Ser Lys Asp Lys Ser Ser Thr Glu
           275                 280                 285
   Glu Lys Phe Asn Glu Lys Gly Glu Val Ser Glu Lys Ile Ile Thr Arg
       290                 295                 300
   Ala Asp Gly Thr Arg Leu Glu Tyr Thr Gly Ile Lys Ser Asp Gly Ser
   305                 310                 315                 320
   Gly Lys Ala Lys Glu Val Leu Lys Lys Phe Thr Leu Glu Gly Lys Val
                   325                 330                 335
   Ala Asn Asp Lys Val Thr Leu Glu Val Lys Glu Gly Thr Val Thr Leu
               340                 345                 350
   Ser Lys Asn Ile Ser Lys Ser Gly Glu Val Ser Val Glu Leu Asn Asp
           355                 360                 365
   Thr Asp Ser Ser Ala Ala Thr Lys Lys Thr Ala Ala Trp Asn Ser Lys
       370                 375                 380
   Thr Ser Thr Leu Thr Ile Ser Val Asn Ser Lys Lys Thr Thr Gln Leu
   385                 390                 395                 400
   Val Phe Thr Lys Gln Asp Thr Ile Thr Val Gln Lys Tyr Asp Ser Ala
                   405                 410                 415
   Gly Thr Asn Leu Glu Gly Thr Ala Val Glu Ile Lys Thr Leu Asp Glu
               420                 425                 430
   Leu Lys Asn Ala Leu Lys
           435
<210> 169
   <211> 1029
   <212> DNA
   <213> Artificial Sequence
<220>
   <221> CDS
   <222> (1)...(1029)
   <223> Chimeric nucleic acid
<400> 169
   atg gct tgt aat aat tca ggg aaa gat ggg aat aca tct gca aat tct      48
   Met Ala Cys Asn Asn Ser Gly Lys Asp Gly Asn Thr Ser Ala Asn Ser
    1               5                  10                  15
   gct gat gag tct gtt aaa ggg cct aat ctt aca gaa ata aat aaa aaa      96
   Ala Asp Glu Ser Val Lys Gly Pro Asn Leu Thr Glu Ile Asn Lys Lys
               20                  25                  30
   att acg gat tct aat gcg gtt tta ctt gct gtg aaa gag gtt gaa gcg     144
   Ile Thr Asp Ser Asn Ala Val Leu Leu Ala Val Lys Glu Val Glu Ala
           35                  40                  45
   ttg ctg tca tct ata gat gaa att gct gct aaa gct att ggt aaa aaa     192
   Leu Leu Ser Ser Ile Asp Glu Ile Ala Ala Lys Ala Ile Gly Lys Lys
       50                  55                  60
   ata cac caa aat aat ggt ttg gat acc gaa aat aat cac aat gga tca     240
   Ile His Gln Asn Asn Gly Leu Asp Thr Glu Asn Asn His Asn Gly Ser
   65                  70                  75                  80
   ttg tta gcg gga gct tat gca ata tca acc cta ata aaa caa aaa tta     288
   Leu Leu Ala Gly Ala Tyr Ala Ile Ser Thr Leu Ile Lys Gln Lys Leu
                   85                  90                  95
   gat gga ttg aaa aat gaa gga tta aag gaa aaa att gat gcg gct aag     336
   Asp Gly Leu Lys Asn Glu Gly Leu Lys Glu Lys Ile Asp Ala Ala Lys
               100                 105                 110
   aaa tgt tct gaa aca ttt act aat aaa tta aaa gaa aaa cac aca gat     384
   Lys Cys Ser Glu Thr Phe Thr Asn Lys Leu Lys Glu Lys His Thr Asp
           115                 120                 125
   ctt ggt aaa gaa ggt gtt act gat gct gat gca aaa gaa gcc att tta     432
   Leu Gly Lys Glu Gly Val Thr Asp Ala Asp Ala Lys Glu Ala Ile Leu
       130                 135                 140
   aaa gca aat ggt act aaa act aaa ggt gct gaa gaa ctt gga aaa tta     480
   Lys Ala Asn Gly Thr Lys Thr Lys Gly Ala Glu Glu Leu Gly Lys Leu
   145                 150                 155                 160
   ttt gaa tca gta gag gtc ttg tca aaa gca gct aaa gag atg ctt gct      528
   Phe Glu Ser Val Glu Val Leu Ser Lys Ala Ala Lys Glu Met Leu Ala
                   165                 170                 175
   aat tca gtt aaa gag ctt aca agc cct gtt gtg gca gaa agt cca aaa      576
   Asn Ser Val Lys Glu Leu Thr Ser Pro Val Val Ala Glu Ser Pro Lys
               180                 185                 190
   aaa cct tcc atg gcc aag caa aat gtt tct gaa aaa ata ata aca aga      624
   Lys Pro Ser Met Ala Lys Gln Asn Val Ser Glu Lys Ile Ile Thr Arg
           195                 200                 205
   gca gac gga acc aga ctt gaa tac aca gga att aaa agc gat gga tct      672
   Ala Asp Gly Thr Arg Leu Glu Tyr Thr Gly Ile Lys Ser Asp Gly Ser
       210                 215                 220
   gga aaa gct aaa gag gtt tta aaa ggc tat gtt ctt gaa gga act cta      720
   Gly Lys Ala Lys Glu Val Leu Lys Gly Tyr Val Leu Glu Gly Thr Leu
   225                 230                 235                 240
   act gct gaa aaa aca aca ttg gtg gtt aaa gaa gga act gtt act tta      768
   Thr Ala Glu Lys Thr Thr Leu Val Val Lys Glu Gly Thr Val Thr Leu
                   245                 250                 255
   agc aaa aat att tca aaa tct ggg gaa gtt tca gtt gaa ctt aat gac      816
   Ser Lys Asn Ile Ser Lys Ser Gly Glu Val Ser Val Glu Leu Asn Asp
               260                 265                 270
   act gac agt agt gct gct act aaa aaa act gca gct tgg aat tca ggc      864
   Thr Asp Ser Ser Ala Ala Thr Lys Lys Thr Ala Ala Trp Asn Ser Gly
           275                 280                 285
   act tca act tta aca att act gta aac agt aaa aaa act aaa gac ctt      912
   Thr Ser Thr Leu Thr Ile Thr Val Asn Ser Lys Lys Thr Lys Asp Leu
       290                 295                 300
   gtg ttt aca aaa gaa aac aca att aca gta caa caa tac gac tca aat      960
   Val Phe Thr Lys Glu Asn Thr Ile Thr Val Gln Gln Tyr Asp Ser Asn
   305                 310                 315                 320
   ggc acc aaa tta gag ggg tca gca gtt gaa att aca aaa ctt gat gaa     1008
   Gly Thr Lys Leu Glu Gly Ser Ala Val Glu Ile Thr Lys Leu Asp Glu
                   325                 330                 335
   att aaa aac gct tta aaa taa                                         1029
   Ile Lys Asn Ala Leu Lys  *
               340
<210> 170
   <211> 342
   <212> PRT
   <213> Artificial Sequence
<220>
   <223> Chimeric protein
<400> 170
   Met Ala Cys Asn Asn Ser Gly Lys Asp Gly Asn Thr Ser Ala Asn Ser
   1               5                   10                  15
   Ala Asp Glu Ser Val Lys Gly Pro Asn Leu Thr Glu Ile Asn Lys Lys
               20                  25                  30
   Ile Thr Asp Ser Asn Ala Val Leu Leu Ala Val Lys Glu Val Glu Ala
           35                  40                  45
   Leu Leu Ser Ser Ile Asp Glu Ile Ala Ala Lys Ala Ile Gly Lys Lys
       50                  55                  60
   Ile His Gln Asn Asn Gly Leu Asp Thr Glu Asn Asn His Asn Gly Ser
   65                  70                  75                  80
   Leu Leu Ala Gly Ala Tyr Ala Ile Ser Thr Leu Ile Lys Gln Lys Leu
                   85                  90                  95
   Asp Gly Leu Lys Asn Glu Gly Leu Lys Glu Lys Ile Asp Ala Ala Lys
               100                 105                 110
   Lys Cys Ser Glu Thr Phe Thr Asn Lys Leu Lys Glu Lys His Thr Asp
           115                 120                 125
   Leu Gly Lys Glu Gly Val Thr Asp Ala Asp Ala Lys Glu Ala Ile Leu
       130                 135                 140
   Lys Ala Asn Gly Thr Lys Thr Lys Gly Ala Glu Glu Leu Gly Lys Leu
   145                 150                 155                 160
   Phe Glu Ser Val Glu Val Leu Ser Lys Ala Ala Lys Glu Met Leu Ala
                   165                 170                 175
   Asn Ser Val Lys Glu Leu Thr Ser Pro Val Val Ala Glu Ser Pro Lys
               180                 185                 190
   Lys Pro Ser Met Ala Lys Gln Asn Val Ser Glu Lys Ile Ile Thr Arg
           195                 200                 205
   Ala Asp Gly Thr Arg Leu Glu Tyr Thr Gly Ile Lys Ser Asp Gly Ser
       210                 215                 220
   Gly Lys Ala Lys Glu Val Leu Lys Gly Tyr Val Leu Glu Gly Thr Leu
   225                 230                 235                 240
   Thr Ala Glu Lys Thr Thr Leu Val Val Lys Glu Gly Thr Val Thr Leu
                   245                 250                 255
   Ser Lys Asn Ile Ser Lys Ser Gly Glu Val Ser Val Glu Leu Asn Asp
               260                 265                 270
   Thr Asp Ser Ser Ala Ala Thr Lys Lys Thr Ala Ala Trp Asn Ser Gly
           275                 280                 285
   Thr Ser Thr Leu Thr Ile Thr Val Asn Ser Lys Lys Thr Lys Asp Leu
       290                 295                 300
   Val Phe Thr Lys Glu Asn Thr Ile Thr Val Gln Gln Tyr Asp Ser Asn
   305                 310                 315                 320
   Gly Thr Lys Leu Glu Gly Ser Ala Val Glu Ile Thr Lys Leu Asp Glu
                   325                 330                 335
   Ile Lys Asn Ala Leu Lys
               340
<210> 171
   <211> 1029
   <212> DNA
   <213> Artificial Sequence
<220>
   <221> CDS
   <222> (1)...(1029)
   <223> Chimeric nucleic acid
<400> 171
   atg gct tgt aat aat tca ggg aaa gat ggg aat aca tct gca aat tct    48
   Met Ala Cys Asn Asn Ser Gly Lys Asp Gly Asn Thr Ser Ala Asn Ser
    1               5                  10                  15
   gct gat gag tct gtt aaa ggg cct aat ctt aca gaa ata aat aaa aaa    96
   Ala Asp Glu Ser Val Lys Gly Pro Asn Leu Thr Glu Ile Asn Lys Lys
               20                  25                  30
   att acg gat tct aat gcg gtt tta ctt gct gtg aaa gag gtt gaa gcg    144
   Ile Thr Asp Ser Asn Ala Val Leu Leu Ala Val Lys Glu Val Glu Ala
           35                  40                  45
   ttg ctg tca tct ata gat gaa att gct gct aaa gct att ggt aaa aaa    192
   Leu Leu Ser Ser Ile Asp Glu Ile Ala Ala Lys Ala Ile Gly Lys Lys
       50                  55                  60
   ata cac caa aat aat ggt ttg gat acc gaa aat aat cac aat gga tca    240
   Ile His Gln Asn Asn Gly Leu Asp Thr Glu Asn Asn His Asn Gly Ser
   65                  70                  75                  80
   ttg tta gcg gga gct tat gca ata tca acc cta ata aaa caa aaa tta    288
   Leu Leu Ala Gly Ala Tyr Ala Ile Ser Thr Leu Ile Lys Gln Lys Leu
                   85                  90                  95
   gat gga ttg aaa aat gaa gga tta aag gaa aaa att gat gcg gct aag    336
   Asp Gly Leu Lys Asn Glu Gly Leu Lys Glu Lys Ile Asp Ala Ala Lys
               100                 105                 110
   aaa tgt tct gaa aca ttt act aat aaa tta aaa gaa aaa cac aca gat    384
   Lys Cys Ser Glu Thr Phe Thr Asn Lys Leu Lys Glu Lys His Thr Asp
           115                 120                 125
   ctt ggt aaa gaa ggt gtt act gat gct gat gca aaa gaa gcc att tta    432
   Leu Gly Lys Glu Gly Val Thr Asp Ala Asp Ala Lys Glu Ala Ile Leu
       130                 135                 140
   aaa gca aat ggt act aaa act aaa ggt gct gaa gaa ctt gga aaa tta    480
   Lys Ala Asn Gly Thr Lys Thr Lys Gly Ala Glu Glu Leu Gly Lys Leu
   145                 150                 155                 160
   ttt gaa tca gta gag gtc ttg tca aaa gca gct aaa gag atg ctt gct    528
   Phe Glu Ser Val Glu Val Leu Ser Lys Ala Ala Lys Glu Met Leu Ala
                   165                 170                 175
   aat tca gtt aaa gag ctt aca agc cct gtt gtg gca gaa agt cca aaa    576
   Asn Ser Val Lys Glu Leu Thr Ser Pro Val Val Ala Glu Ser Pro Lys
               180                 185                 190
   aaa cct tcc atg gcc aag caa aat gtt tct gaa aaa ata ata aca aga    624
   Lys Pro Ser Met Ala Lys Gln Asn Val Ser Glu Lys Ile Ile Thr Arg
           195                 200                 205
   gca gac gga acc aga ctt gaa tac aca gga att aaa agc gat gga tct    672
   Ala Asp Gly Thr Arg Leu Glu Tyr Thr Gly Ile Lys Ser Asp Gly Ser
       210                 215                 220
   gga aaa gct aaa gag gtt tta aaa ggc tat gtt ctt gaa gga act cta    720
   Gly Lys Ala Lys Glu Val Leu Lys Gly Tyr Val Leu Glu Gly Thr Leu
   225                 230                 235                 240
   act gct gaa aaa aca aca ttg gtg gtt aaa gaa gga act gtt act tta    768
   Thr Ala Glu Lys Thr Thr Leu Val Val Lys Glu Gly Thr Val Thr Leu
                   245                 250                 255
   agc aaa aat att tca aaa tct ggg gaa gtt tca gtt gaa ctt aat gac    816
   Ser Lys Asn Ile Ser Lys Ser Gly Glu Val Ser Val Glu Leu Asn Asp
               260                 265                 270
   act gac agt agt gct gct act aaa aaa act gca gct tgg aat tca aaa     864
   Thr Asp Ser Ser Ala Ala Thr Lys Lys Thr Ala Ala Trp Asn Ser Lys
           275                 280                 285
   act tcc act tta aca att agt gtg aat agc caa aaa acc aaa aac ctt     912
   Thr Ser Thr Leu Thr Ile Ser Val Asn Ser Gln Lys Thr Lys Asn Leu
       290                 295                 300
   gta ttc aca aaa gaa gac aca ata aca gta caa aaa tac gac tca gca     960
   Val Phe Thr Lys Glu Asp Thr Ile Thr Val Gln Lys Tyr Asp Ser Ala
   305                 310                 315                 320
   ggc acc aat cta gaa ggc aaa gca gtc gaa att aca aca ctt aaa gaa    1008
   Gly Thr Asn Leu Glu Gly Lys Ala Val Glu Ile Thr Thr Leu Lys Glu
                   325                 330                 335
   ctt aaa aac gct tta aaa taa                                        1029
   Leu Lys Asn Ala Leu Lys  *
               340
<210> 172
   <211> 342
   <212> PRT
   <213> Artificial Sequence
<220>
   <223> Chimeric protein
<400> 172
   Met Ala Cys Asn Asn Ser Gly Lys Asp Gly Asn Thr Ser Ala Asn Ser
   1               5                   10                  15
   Ala Asp Glu Ser Val Lys Gly Pro Asn Leu Thr Glu Ile Asn Lys Lys
               20                  25                  30
   Ile Thr Asp Ser Asn Ala Val Leu Leu Ala Val Lys Glu Val Glu Ala
           35                  40                  45
   Leu Leu Ser Ser Ile Asp Glu Ile Ala Ala Lys Ala Ile Gly Lys Lys
       50                  55                  60
   Ile His Gln Asn Asn Gly Leu Asp Thr Glu Asn Asn His Asn Gly Ser
   65                  70                  75                  80
   Leu Leu Ala Gly Ala Tyr Ala Ile Ser Thr Leu Ile Lys Gln Lys Leu
                   85                  90                  95
   Asp Gly Leu Lys Asn Glu Gly Leu Lys Glu Lys Ile Asp Ala Ala Lys
               100                 105                 110
   Lys Cys Ser Glu Thr Phe Thr Asn Lys Leu Lys Glu Lys His Thr Asp
           115                 120                 125
   Leu Gly Lys Glu Gly Val Thr Asp Ala Asp Ala Lys Glu Ala Ile Leu
       130                 135                 140
   Lys Ala Asn Gly Thr Lys Thr Lys Gly Ala Glu Glu Leu Gly Lys Leu
   145                 150                 155                 160
   Phe Glu Ser Val Glu Val Leu Ser Lys Ala Ala Lys Glu Met Leu Ala
                   165                 170                 175
   Asn Ser Val Lys Glu Leu Thr Ser Pro Val Val Ala Glu Ser Pro Lys
               180                 185                 190
   Lys Pro Ser Met Ala Lys Gln Asn Val Ser Glu Lys Ile Ile Thr Arg
           195                 200                 205
   Ala Asp Gly Thr Arg Leu Glu Tyr Thr Gly Ile Lys Ser Asp Gly Ser
       210                 215                 220
   Gly Lys Ala Lys Glu Val Leu Lys Gly Tyr Val Leu Glu Gly Thr Leu
   225                 230                 235                 240
   Thr Ala Glu Lys Thr Thr Leu Val Val Lys Glu Gly Thr Val Thr Leu
                   245                 250                 255
   Ser Lys Asn Ile Ser Lys Ser Gly Glu Val Ser Val Glu Leu Asn Asp
               260                 265                 270
   Thr Asp Ser Ser Ala Ala Thr Lys Lys Thr Ala Ala Trp Asn Ser Lys
           275                 280                 285
   Thr Ser Thr Leu Thr Ile Ser Val Asn Ser Gln Lys Thr Lys Asn Leu
       290                 295                 300
   Val Phe Thr Lys Glu Asp Thr Ile Thr Val Gln Lys Tyr Asp Ser Ala
   305                 310                 315                 320
   Gly Thr Asn Leu Glu Gly Lys Ala Val Glu Ile Thr Thr Leu Lys Glu
                   325                 330                 335
   Leu Lys Asn Ala Leu Lys
               340
<210> 173
   <211> 1029
   <212> DNA
   <213> Artificial Sequence
<220>
   <221> CDS
   <222> (1)...(1029)
   <223> Chimeric nucleic acid
<400> 173
   atg gct tgt aat aat tca ggg aaa gat ggg aat aca tct gca aat tct      48
   Met Ala Cys Asn Asn Ser Gly Lys Asp Gly Asn Thr Ser Ala Asn Ser
    1               5                  10                  15
   gct gat gag tct gtt aaa ggg cct aat ctt aca gaa ata aat aaa aaa      96
   Ala Asp Glu Ser Val Lys Gly Pro Asn Leu Thr Glu Ile Asn Lys Lys
               20                  25                  30
   att acg gat tct aat gcg gtt tta ctt gct gtg aaa gag gtt gaa gcg     144
   Ile Thr Asp Ser Asn Ala Val Leu Leu Ala Val Lys Glu Val Glu Ala
           35                  40                  45
   ttg ctg tca tct ata gat gaa att gct gct aaa gct att ggt aaa aaa     192
   Leu Leu Ser Ser Ile Asp Glu Ile Ala Ala Lys Ala Ile Gly Lys Lys
       50                  55                  60
   ata cac caa aat aat ggt ttg gat acc gaa aat aat cac aat gga tca     240
   Ile His Gln Asn Asn Gly Leu Asp Thr Glu Asn Asn His Asn Gly Ser
   65                  70                  75                  80
   ttg tta gcg gga gct tat gca ata tca acc cta ata aaa caa aaa tta     288
   Leu Leu Ala Gly Ala Tyr Ala Ile Ser Thr Leu Ile Lys Gln Lys Leu
                   85                  90                  95
   gat gga ttg aaa aat gaa gga tta aag gaa aaa att gat gcg gct aag     336
   Asp Gly Leu Lys Asn Glu Gly Leu Lys Glu Lys Ile Asp Ala Ala Lys
               100                 105                 110
   aaa tgt tct gaa aca ttt act aat aaa tta aaa gaa aaa cac aca gat     384
   Lys Cys Ser Glu Thr Phe Thr Asn Lys Leu Lys Glu Lys His Thr Asp
           115                 120                 125
   ctt ggt aaa gaa ggt gtt act gat gct gat gca aaa gaa gcc att tta     432
   Leu Gly Lys Glu Gly Val Thr Asp Ala Asp Ala Lys Glu Ala Ile Leu
       130                 135                 140
   aaa gca aat ggt act aaa act aaa ggt gct gaa gaa ctt gga aaa tta     480
   Lys Ala Asn Gly Thr Lys Thr Lys Gly Ala Glu Glu Leu Gly Lys Leu
   145                 150                 155                 160
   ttt gaa tca gta gag gtc ttg tca aaa gca gct aaa gag atg ctt gct     528
   Phe Glu Ser Val Glu Val Leu Ser Lys Ala Ala Lys Glu Met Leu Ala
                   165                 170                 175
   aat tca gtt aaa gag ctt aca agc cct gtt gtg gca gaa agt cca aaa     576
   Asn Ser Val Lys Glu Leu Thr Ser Pro Val Val Ala Glu Ser Pro Lys
               180                 185                 190
   aaa cct tcc atg gcc aag caa aat gtt tct gaa aaa ata ata aca aga     624
   Lys Pro Ser Met Ala Lys Gln Asn Val Ser Glu Lys Ile Ile Thr Arg
           195                 200                 205
   gca gac gga acc aga ctt gaa tac aca gga att aaa agc gat gga tct     672
   Ala Asp Gly Thr Arg Leu Glu Tyr Thr Gly Ile Lys Ser Asp Gly Ser
       210                 215                 220
   gga aaa gct aaa gag gtt tta aaa ggc tat gtt ctt gaa gga act cta     720
   Gly Lys Ala Lys Glu Val Leu Lys Gly Tyr Val Leu Glu Gly Thr Leu
   225                 230                 235                 240
   act gct gaa aaa aca aca ttg gtg gtt aaa gaa gga act gtt act tta     768
   Thr Ala Glu Lys Thr Thr Leu Val Val Lys Glu Gly Thr Val Thr Leu
                   245                 250                 255
   agc aaa aat att tca aaa tct ggg gaa gtt tca gtt gaa ctt aat gac     816
   Ser Lys Asn Ile Ser Lys Ser Gly Glu Val Ser Val Glu Leu Asn Asp
               260                 265                 270
   act gac agt agt gct gct act aaa aaa act gca gct tgg aat tca aaa     864
   Thr Asp Ser Ser Ala Ala Thr Lys Lys Thr Ala Ala Trp Asn Ser Lys
           275                 280                 285
   act tct act tta aca att agt gtt aac agc aaa aaa act aca caa ctt     912
   Thr Ser Thr Leu Thr Ile Ser Val Asn Ser Lys Lys Thr Thr Gln Leu
       290                 295                 300
   gtg ttt act aaa caa gac aca ata act gta caa aaa tac gac tcc gca     960
   Val Phe Thr Lys Gln Asp Thr Ile Thr Val Gln Lys Tyr Asp Ser Ala
   305                 310                 315                 320
   ggt acc aat tta gaa ggc aca gca gtc gaa att aaa aca ctt gat gaa     1008
   Gly Thr Asn Leu Glu Gly Thr Ala Val Glu Ile Lys Thr Leu Asp Glu
                   325                 330                 335
   ctt aaa aac gct tta aaa taa                                         1029
   Leu Lys Asn Ala Leu Lys  *
               340
<210> 174
   <211> 342
   <212> PRT
   <213> Artificial Sequence
<220>
   <223> Chimeric protein
<400> 174
   Met Ala Cys Asn Asn Ser Gly Lys Asp Gly Asn Thr Ser Ala Asn Ser
    1               5                  10                  15
   Ala Asp Glu Ser Val Lys Gly Pro Asn Leu Thr Glu Ile Asn Lys Lys
               20                  25                  30
   Ile Thr Asp Ser Asn Ala Val Leu Leu Ala Val Lys Glu Val Glu Ala
           35                  40                  45
   Leu Leu Ser Ser Ile Asp Glu Ile Ala Ala Lys Ala Ile Gly Lys Lys
       50                  55                  60
   Ile His Gln Asn Asn Gly Leu Asp Thr Glu Asn Asn His Asn Gly Ser
   65                  70                  75                  80
   Leu Leu Ala Gly Ala Tyr Ala Ile Ser Thr Leu Ile Lys Gln Lys Leu
                   85                  90                  95
   Asp Gly Leu Lys Asn Glu Gly Leu Lys Glu Lys Ile Asp Ala Ala Lys
               100                 105                 110
   Lys Cys Ser Glu Thr Phe Thr Asn Lys Leu Lys Glu Lys His Thr Asp
           115                 120                 125
   Leu Gly Lys Glu Gly Val Thr Asp Ala Asp Ala Lys Glu Ala Ile Leu
       130                 135                 140
   Lys Ala Asn Gly Thr Lys Thr Lys Gly Ala Glu Glu Leu Gly Lys Leu
   145                 150                 155                 160
   Phe Glu Ser Val Glu Val Leu Ser Lys Ala Ala Lys Glu Met Leu Ala
                   165                 170                 175
   Asn Ser Val Lys Glu Leu Thr Ser Pro Val Val Ala Glu Ser Pro Lys
               180                 185                 190
   Lys Pro Ser Met Ala Lys Gln Asn Val Ser Glu Lys Ile Ile Thr Arg
           195                 200                 205
   Ala Asp Gly Thr Arg Leu Glu Tyr Thr Gly Ile Lys Ser Asp Gly Ser
       210                 215                 220
   Gly Lys Ala Lys Glu Val Leu Lys Gly Tyr Val Leu Glu Gly Thr Leu
   225                 230                 235                 240
   Thr Ala Glu Lys Thr Thr Leu Val Val Lys Glu Gly Thr Val Thr Leu
                   245                 250                 255
   Ser Lys Asn Ile Ser Lys Ser Gly Glu Val Ser Val Glu Leu Asn Asp
               260                 265                 270
   Thr Asp Ser Ser Ala Ala Thr Lys Lys Thr Ala Ala Trp Asn Ser Lys
           275                 280                 285
   Thr Ser Thr Leu Thr Ile Ser Val Asn Ser Lys Lys Thr Thr Gln Leu
       290                 295                 300
   Val Phe Thr Lys Gln Asp Thr Ile Thr Val Gln Lys Tyr Asp Ser Ala
   305                 310                 315                 320
   Gly Thr Asn Leu Glu Gly Thr Ala Val Glu Ile Lys Thr Leu Asp Glu
                   325                 330                 335
   Leu Lys Asn Ala Leu Lys
               340
<210> 175
   <211> 1035
   <212> DNA
   <213> Artificial Sequence
<220>
   <221> CDS
   <222> (1)...(1035)
   <223> Chimeric nucleic acid
<400> 175
   atg gct tgt aat aat tca ggg aaa gat ggg aat aca tct gca aat tct     48
   Met Ala Cys Asn Asn Ser Gly Lys Asp Gly Asn Thr Ser Ala Asn Ser
    1               5                  10                  15
   gct gat gag tct gtt aaa ggg cct aat ctt aca gaa ata agt aaa aaa     96
   Ala Asp Glu Ser Val Lys Gly Pro Asn Leu Thr Glu Ile Ser Lys Lys
               20                  25                  30
   att acg gat tct aat gcg gtt tta ctt gct gtg aaa gag gtt gaa gcg    144
   Ile Thr Asp Ser Asn Ala Val Leu Leu Ala Val Lys Glu Val Glu Ala
           35                  40                  45
   ttg ctg tca tct ata gat gaa att gct gct aaa gct att ggt aaa aaa    192
   Leu Leu Ser Ser Ile Asp Glu Ile Ala Ala Lys Ala Ile Gly Lys Lys
       50                  55                  60
   ata cac caa aat aat ggt ttg gat acc gaa tat aat cac aat gga tca    240
   Ile His Gln Asn Asn Gly Leu Asp Thr Glu Tyr Asn His Asn Gly Ser
   65                  70                  75                  80
   ttg tta gcg gga gct tat gca ata tca acc cta ata aaa caa aaa tta    288
   Leu Leu Ala Gly Ala Tyr Ala Ile Ser Thr Leu Ile Lys Gln Lys Leu
                   85                  90                  95
   gat gga ttg aaa aat gaa gga tta aag gaa aaa att gat gcg gct aag    336
   Asp Gly Leu Lys Asn Glu Gly Leu Lys Glu Lys Ile Asp Ala Ala Lys
               100                 105                 110
   aaa tgt tct gaa aca ttt act aat aaa tta aaa gaa aaa cac aca gat    384
   Lys Cys Ser Glu Thr Phe Thr Asn Lys Leu Lys Glu Lys His Thr Asp
           115                 120                 125
   ctt ggt aaa gaa ggt gtt act gat gct gat gca aaa gaa gcc att tta    432
   Leu Gly Lys Glu Gly Val Thr Asp Ala Asp Ala Lys Glu Ala Ile Leu
       130                 135                 140
   aaa aca aat ggt act aaa act aaa ggt gct gaa gaa ctt gga aaa tta    480
   Lys Thr Asn Gly Thr Lys Thr Lys Gly Ala Glu Glu Leu Gly Lys Leu
   145                 150                 155                 160
   ttt gaa tca gta gag gtc ttg tca aaa gca gct aaa gag atg ctt gct    528
   Phe Glu Ser Val Glu Val Leu Ser Lys Ala Ala Lys Glu Met Leu Ala
                   165                 170                 175
   aat tca gtt aaa gag ctt aca agc cct gtt gtg gca gaa agt cca aaa    576
   Asn Ser Val Lys Glu Leu Thr Ser Pro Val Val Ala Glu Ser Pro Lys
               180                 185                 190
   aaa cct tcc atg gcc aag caa aat gtt aca tct gaa aaa aca ata gta    624
   Lys Pro Ser Met Ala Lys Gln Asn Val Thr Ser Glu Lys Thr Ile Val
           195                 200                 205
   aga gca aat gga acc aga ctt gaa tac aca gac ata aaa agc gat gga    672
   Arg Ala Asn Gly Thr Arg Leu Glu Tyr Thr Asp Ile Lys Ser Asp Gly
       210                 215                 220
   tcc gga aaa gct aaa gaa gtt tta aaa gac ttt act ctt gaa gga act    720
   Ser Gly Lys Ala Lys Glu Val Leu Lys Asp Phe Thr Leu Glu Gly Thr
   225                 230                 235                 240
   cta gct gct gac ggc aaa aca aca ttg aaa gtt aca gaa ggc act gtt    768
   Leu Ala Ala Asp Gly Lys Thr Thr Leu Lys Val Thr Glu Gly Thr Val
                   245                 250                 255
   gtt tta agc aag aac att tta aaa tcc gga gaa ata aca gtt gca ctt    816
   Val Leu Ser Lys Asn Ile Leu Lys Ser Gly Glu Ile Thr Val Ala Leu
               260                 265                 270
   gat gac tct gac act act cag gct act aaa aaa act gga aaa tgg gat    864
   Asp Asp Ser Asp Thr Thr Gln Ala Thr Lys Lys Thr Gly Lys Trp Asp
           275                 280                 285
   tca aat act tcc act tta aca att agt gtg aat agc aaa aaa act aaa    912
   Ser Asn Thr Ser Thr Leu Thr Ile Ser Val Asn Ser Lys Lys Thr Lys
       290                 295                 300
   aac att gta ttt aca aaa gaa gac aca ata aca gta caa aaa tac gac    960
   Asn Ile Val Phe Thr Lys Glu Asp Thr Ile Thr Val Gln Lys Tyr Asp
   305                 310                 315                 320
   tca gca ggc acc aat cta gaa ggc aac gca gtc gaa att aaa aca ctt    1008
   Ser Ala Gly Thr Asn Leu Glu Gly Asn Ala Val Glu Ile Lys Thr Leu
                   325                 330                 335
   gat gaa ctt aaa aac gct tta aaa tag                                1035
   Asp Glu Leu Lys Asn Ala Leu Lys  *
               340
<210> 176
   <211> 344
   <212> PRT
   <213> Artificial Sequence
<220>
   <223> Chimeric protein
<400> 176
   Met Ala Cys Asn Asn Ser Gly Lys Asp Gly Asn Thr Ser Ala Asn Ser
   1               5                   10                  15
   Ala Asp Glu Ser Val Lys Gly Pro Asn Leu Thr Glu Ile Ser Lys Lys
               20                  25                  30
   Ile Thr Asp Ser Asn Ala Val Leu Leu Ala Val Lys Glu Val Glu Ala
           35                  40                  45
   Leu Leu Ser Ser Ile Asp Glu Ile Ala Ala Lys Ala Ile Gly Lys Lys
       50                  55                  60
   Ile His Gln Asn Asn Gly Leu Asp Thr Glu Tyr Asn His Asn Gly Ser
   65                  70                  75                  80
   Leu Leu Ala Gly Ala Tyr Ala Ile Ser Thr Leu Ile Lys Gln Lys Leu
                   85                  90                  95
   Asp Gly Leu Lys Asn Glu Gly Leu Lys Glu Lys Ile Asp Ala Ala Lys
               100                 105                 110
   Lys Cys Ser Glu Thr Phe Thr Asn Lys Leu Lys Glu Lys His Thr Asp
           115                 120                 125
   Leu Gly Lys Glu Gly Val Thr Asp Ala Asp Ala Lys Glu Ala Ile Leu
       130                 135                 140
   Lys Thr Asn Gly Thr Lys Thr Lys Gly Ala Glu Glu Leu Gly Lys Leu
   145                 150                 155                 160
   Phe Glu Ser Val Glu Val Leu Ser Lys Ala Ala Lys Glu Met Leu Ala
                   165                 170                 175
   Asn Ser Val Lys Glu Leu Thr Ser Pro Val Val Ala Glu Ser Pro Lys
               180                 185                 190
   Lys Pro Ser Met Ala Lys Gln Asn Val Thr Ser Glu Lys Thr Ile Val
           195                 200                 205
   Arg Ala Asn Gly Thr Arg Leu Glu Tyr Thr Asp Ile Lys Ser Asp Gly
       210                 215                 220
   Ser Gly Lys Ala Lys Glu Val Leu Lys Asp Phe Thr Leu Glu Gly Thr
   225                 230                 235                 240
   Leu Ala Ala Asp Gly Lys Thr Thr Leu Lys Val Thr Glu Gly Thr Val
                   245                 250                 255
   Val Leu Ser Lys Asn Ile Leu Lys Ser Gly Glu Ile Thr Val Ala Leu
               260                 265                 270
   Asp Asp Ser Asp Thr Thr Gln Ala Thr Lys Lys Thr Gly Lys Trp Asp
           275                 280                 285
   Ser Asn Thr Ser Thr Leu Thr Ile Ser Val Asn Ser Lys Lys Thr Lys
       290                 295                 300
   Asn Ile Val Phe Thr Lys Glu Asp Thr Ile Thr Val Gln Lys Tyr Asp
   305                 310                 315                 320
   Ser Ala Gly Thr Asn Leu Glu Gly Asn Ala Val Glu Ile Lys Thr Leu
                   325                 330                 335
   Asp Glu Leu Lys Asn Ala Leu Lys
               340
<210> 177
   <211> 1323
   <212> DNA
   <213> Artificial Sequence
<220>
   <221> CDS
   <222> (1)...(1323)
   <223> Chimeric nucleic acid
<400> 177
   atg gct tgt aat aat tca ggg aaa gat ggg aat aca tct gca aat tct      48
   Met Ala Cys Asn Asn Ser Gly Lys Asp Gly Asn Thr Ser Ala Asn Ser
    1               5                  10                  15
   gct gat gag tct gtt aaa ggg cct aat ctt aca gaa ata aat aaa aaa      96
   Ala Asp Glu Ser Val Lys Gly Pro Asn Leu Thr Glu Ile Asn Lys Lys
               20                  25                  30
   att acg gat tct aat gcg gtt tta ctt gct gtg aaa gag gtt gaa gcg     144
   Ile Thr Asp Ser Asn Ala Val Leu Leu Ala Val Lys Glu Val Glu Ala
           35                  40                  45
   ttg ctg tca tct ata gat gaa att gct gct aaa gct att ggt aaa aaa     192
   Leu Leu Ser Ser Ile Asp Glu Ile Ala Ala Lys Ala Ile Gly Lys Lys
       50                  55                  60
   ata cac caa aat aat ggt ttg gat acc gaa aat aat cac aat gga tca    240
   Ile His Gln Asn Asn Gly Leu Asp Thr Glu Asn Asn His Asn Gly Ser
    65                 70                  75                  80
   ttg tta gcg gga gct tat gca ata tca acc cta ata aaa caa aaa tta    288
   Leu Leu Ala Gly Ala Tyr Ala Ile Ser Thr Leu Ile Lys Gln Lys Leu
                    85                  90                  95
   gat gga ttg aaa aat gaa gga tta aag gaa aaa att gat gcg gct aag    336
   Asp Gly Leu Lys Asn Glu Gly Leu Lys Glu Lys Ile Asp Ala Ala Lys
               100                 105                 110
   aaa tgt tct gaa aca ttt act aat aaa tta aaa gaa aaa cac aca gat    384
   Lys Cys Ser Glu Thr Phe Thr Asn Lys Leu Lys Glu Lys His Thr Asp
           115                 120                 125
   ctt ggt aaa gaa ggt gtt act gat gct gat gca aaa gaa gcc att tta    432
   Leu Gly Lys Glu Gly Val Thr Asp Ala Asp Ala Lys Glu Ala Ile Leu
       130                 135                 140
   aaa gca aat ggt act aaa act aaa ggt gct gaa gaa ctt gga aaa tta    480
   Lys Ala Asn Gly Thr Lys Thr Lys Gly Ala Glu Glu Leu Gly Lys Leu
   145                 150                 155                 160
   ttt gaa tca gta gag gtc ttg tca aaa gca gct aaa gag atg ctt gct    528
   Phe Glu Ser Val Glu Val Leu Ser Lys Ala Ala Lys Glu Met Leu Ala
                   165                 170                 175
   aat tca gtt aaa gag ctt aca agc cct gtt gtg gca gaa agt cca aaa    576
   Asn Ser Val Lys Glu Leu Thr Ser Pro Val Val Ala Glu Ser Pro Lys
               180                 185                 190
   aaa cct tcc atg gcc gtt tca gta gat ttg cct ggt gaa atg aaa gtt    624
   Lys Pro Ser Met Ala Val Ser Val Asp Leu Pro Gly Glu Met Lys Val
           195                 200                 205
   ctt gta agc aaa gaa aaa aac aaa gac ggc aag tac gat cta att gca    672
   Leu Val Ser Lys Glu Lys Asn Lys Asp Gly Lys Tyr Asp Leu Ile Ala
       210                 215                 220
   aca gta gac aag ctt gag ctt aaa gga act tct gat aaa aac aat gga    720
   Thr Val Asp Lys Leu Glu Leu Lys Gly Thr Ser Asp Lys Asn Asn Gly
   225                 230                 235                 240
   tct gga gta ctt gaa ggc gta aaa gct gac aaa agt aaa gta aaa tta    768
   Ser Gly Val Leu Glu Gly Val Lys Ala Asp Lys Ser Lys Val Lys Leu
                   245                 250                 255
   aca att tct gac gat cta ggt caa acc aca ctt gaa gtt ttc aaa gaa    816
   Thr Ile Ser Asp Asp Leu Gly Gln Thr Thr Leu Glu Val Phe Lys Glu
               260                 265                 270
   gat ggc aaa aca cta gta tca aaa aaa gta act tcc aaa gac aag tca    864
   Asp Gly Lys Thr Leu Val Ser Lys Lys Val Thr Ser Lys Asp Lys Ser
           275                 280                 285
   tca aca gaa gaa aaa ttc aat gaa aaa ggt gaa gta tct gaa aaa ata    912
   Ser Thr Glu Glu Lys Phe Asn Glu Lys Gly Glu Val Ser Glu Lys Ile
       290                 295                 300
   ata aca aga gca gac gga acc aga ctt gaa tac aca gga att aaa agc    960
   Ile Thr Arg Ala Asp Gly Thr Arg Leu Glu Tyr Thr Gly Ile Lys Ser
   305                 310                 315                 320
   gat gga tct gga aaa gct aaa gag gtt tta aaa ggc ttt act ctt gaa    1008
   Asp Gly Ser Gly Lys Ala Lys Glu Val Leu Lys Gly Phe Thr Leu Glu
                   325                 330                 335
   gga aaa gta gct aat gat aaa gta aca ttg gaa gta aaa gaa gga acc    1056
   Gly Lys Val Ala Asn Asp Lys Val Thr Leu Glu Val Lys Glu Gly Thr
               340                 345                 350
   gtt act tta agt aag att tca aaa tct ggg gaa gtt tca gtt gaa ctt    1104
   Val Thr Leu Ser Lys Ile Ser Lys Ser Gly Glu Val Ser Val Glu Leu
           355                 360                 365
   aat gac act gac agt agt gct gct act aaa aaa act gca gct tgg aat    1152
   Asn Asp Thr Asp Ser Ser Ala Ala Thr Lys Lys Thr Ala Ala Trp Asn
       370                 375                 380
   tca aaa act tct act tta aca att agt gtt aac agc aaa aaa act aca    1200
   Ser Lys Thr Ser Thr Leu Thr Ile Ser Val Asn Ser Lys Lys Thr Thr
   385                 390                 395                 400
   caa ctt gtg ttt act aaa caa gac aca ata act gta caa aaa tac gac    1248
   Gln Leu Val Phe Thr Lys Gln Asp Thr Ile Thr Val Gln Lys Tyr Asp
                   405                 410                 415
   tcc gca ggt acc aat tta gaa ggc aca gca gtc gaa att aaa aca ctt    1296
   Ser Ala Gly Thr Asn Leu Glu Gly Thr Ala Val Glu Ile Lys Thr Leu
               420                 425                 430
   gat gaa ctt aaa aac gct tta aaa taa                                1323
   Asp Glu Leu Lys Asn Ala Leu Lys  *
           435                 440
<210> 178
   <211> 440
   <212> PRT
   <213> Artificial Sequence
<220>
   <223> Chimeric protein
<400> 178
   Met Ala Cys Asn Asn Ser Gly Lys Asp Gly Asn Thr Ser Ala Asn Ser
    1               5                  10                  15
   Ala Asp Glu Ser Val Lys Gly Pro Asn Leu Thr Glu Ile Asn Lys Lys
               20                  25                  30
   Ile Thr Asp Ser Asn Ala Val Leu Leu Ala Val Lys Glu Val Glu Ala
           35                  40                  45
   Leu Leu Ser Ser Ile Asp Glu Ile Ala Ala Lys Ala Ile Gly Lys Lys
       50                  55                  60
   Ile His Gln Asn Asn Gly Leu Asp Thr Glu Asn Asn His Asn Gly Ser
   65                  70                  75                  80
   Leu Leu Ala Gly Ala Tyr Ala Ile Ser Thr Leu Ile Lys Gln Lys Leu
                   85                  90                  95
   Asp Gly Leu Lys Asn Glu Gly Leu Lys Glu Lys Ile Asp Ala Ala Lys
               100                 105                 110
   Lys Cys Ser Glu Thr Phe Thr Asn Lys Leu Lys Glu Lys His Thr Asp
           115                 120                 125
   Leu Gly Lys Glu Gly Val Thr Asp Ala Asp Ala Lys Glu Ala Ile Leu
       130                 135                 140
   Lys Ala Asn Gly Thr Lys Thr Lys Gly Ala Glu Glu Leu Gly Lys Leu
   145                 150                 155                 160
   Phe Glu Ser Val Glu Val Leu Ser Lys Ala Ala Lys Glu Met Leu Ala
                   165                 170                 175
   Asn Ser Val Lys Glu Leu Thr Ser Pro Val Val Ala Glu Ser Pro Lys
               180                 185                 190
   Lys Pro Ser Met Ala Val Ser Val Asp Leu Pro Gly Glu Met Lys Val
           195                 200                 205
   Leu Val Ser Lys Glu Lys Asn Lys Asp Gly Lys Tyr Asp Leu Ile Ala
       210                 215                 220
   Thr Val Asp Lys Leu Glu Leu Lys Gly Thr Ser Asp Lys Asn Asn Gly
   225                 230                 235                 240
   Ser Gly Val Leu Glu Gly Val Lys Ala Asp Lys Ser Lys Val Lys Leu
                   245                 250                 255
   Thr Ile Ser Asp Asp Leu Gly Gln Thr Thr Leu Glu Val Phe Lys Glu
               260                 265                 270
   Asp Gly Lys Thr Leu Val Ser Lys Lys Val Thr Ser Lys Asp Lys Ser
           275                 280                 285
   Ser Thr Glu Glu Lys Phe Asn Glu Lys Gly Glu Val Ser Glu Lys Ile
       290                 295                 300
   Ile Thr Arg Ala Asp Gly Thr Arg Leu Glu Tyr Thr Gly Ile Lys Ser
   305                 310                 315                 320
   Asp Gly Ser Gly Lys Ala Lys Glu Val Leu Lys Gly Phe Thr Leu Glu
                   325                 330                 335
   Gly Lys Val Ala Asn Asp Lys Val Thr Leu Glu Val Lys Glu Gly Thr
               340                 345                 350
   Val Thr Leu Ser Lys Ile Ser Lys Ser Gly Glu Val Ser Val Glu Leu
           355                 360                 365
   Asn Asp Thr Asp Ser Ser Ala Ala Thr Lys Lys Thr Ala Ala Trp Asn
       370                 375                 380
   Ser Lys Thr Ser Thr Leu Thr Ile Ser Val Asn Ser Lys Lys Thr Thr
   385                 390                 395                 400
   Gln Leu Val Phe Thr Lys Gln Asp Thr Ile Thr Val Gln Lys Tyr Asp
                   405                 410                 415
   Ser Ala Gly Thr Asn Leu Glu Gly Thr Ala Val Glu Ile Lys Thr Leu
               420                 425                 430
   Asp Glu Leu Lys Asn Ala Leu Lys
           435                 440
<210> 179
   <211> 1302
   <212> DNA
   <213> Artificial Sequence
<220>
   <221> CDS
   <222> (1)...(1302)
   <223> Chimeric nucleic acid
<400> 179
   atg gct tgt aat aat tca ggg aaa gat ggg aat aca tct gca aat tct      48
   Met Ala Cys Asn Asn Ser Gly Lys Asp Gly Asn Thr Ser Ala Asn Ser
    1               5                  10                  15
   gct gat gag tct gtt aaa ggg cct aat ctt aca gaa ata agt aaa aaa      96
   Ala Asp Glu Ser Val Lys Gly Pro Asn Leu Thr Glu Ile Ser Lys Lys
               20                  25                  30
   att acg gat tct aat gcg gtt tta ctt gct gtg aaa gag gtt gaa gcg    144
   Ile Thr Asp Ser Asn Ala Val Leu Leu Ala Val Lys Glu Val Glu Ala
            35                  40                  45
   ttg ctg tca tct ata gat gag ctt gct aaa gct att ggt aaa aaa ata    192
   Leu Leu Ser Ser Ile Asp Glu Leu Ala Lys Ala Ile Gly Lys Lys Ile
        50                  55                  60
   aaa aac gat ggt agt tta gat aat gaa gca aat cgc aac gag tca ttg    240
   Lys Asn Asp Gly Ser Leu Asp Asn Glu Ala Asn Arg Asn Glu Ser Leu
    65                  70                  75                  80
   tta gca gga gct tat aca ata tca acc tta ata aca caa aaa tta agt    288
   Leu Ala Gly Ala Tyr Thr Ile Ser Thr Leu Ile Thr Gln Lys Leu Ser
                    85                  90                  95
   aaa tta aac gga tca gaa ggt tta aag gaa aag att gcc gca gct aag    336
   Lys Leu Asn Gly Ser Glu Gly Leu Lys Glu Lys Ile Ala Ala Ala Lys
               100                 105                 110
   aaa tgc tct gaa gag ttt agt act aaa cta aaa gat aat cat gca cag    384
   Lys Cys Ser Glu Glu Phe Ser Thr Lys Leu Lys Asp Asn His Ala Gln
           115                 120                 125
   ctt ggt ata cag ggc gtt act gat gaa aat gca aaa aaa gct att tta    432
   Leu Gly Ile Gln Gly Val Thr Asp Glu Asn Ala Lys Lys Ala Ile Leu
       130                 135                 140
   aaa gca aat gca gcg ggt aaa gat aag ggc gtt gaa gaa ctt gaa aag    480
   Lys Ala Asn Ala Ala Gly Lys Asp Lys Gly Val Glu Glu Leu Glu Lys
   145                 150                 155                 160
   ttg tcc gga tca tta gaa agc tta tca aaa gca gct aaa gag atg ctt    528
   Leu Ser Gly Ser Leu Glu Ser Leu Ser Lys Ala Ala Lys Glu Met Leu
                   165                 170                 175
   gct aat tca gtt aaa gag ctt aca agc cct gtt gtc cat ggc gtt tca    576
   Ala Asn Ser Val Lys Glu Leu Thr Ser Pro Val Val His Gly Val Ser
               180                 185                 190
   gta gat ttg cct ggt gaa atg aaa gtt ctt gta agc aaa gaa aaa aac    624
   Val Asp Leu Pro Gly Glu Met Lys Val Leu Val Ser Lys Glu Lys Asn
           195                 200                 205
   aaa gac ggc aag tac gat cta att gca aca gta gac aag ctt gag ctt    672
   Lys Asp Gly Lys Tyr Asp Leu Ile Ala Thr Val Asp Lys Leu Glu Leu
       210                 215                 220
   aaa gga act tct gat aaa aac aat gga tct gga gta ctt gaa ggc gta    720
   Lys Gly Thr Ser Asp Lys Asn Asn Gly Ser Gly Val Leu Glu Gly Val
   225                 230                 235                 240
   aaa gct gac aaa agt aaa gta aaa tta aca att tct gac gat cta ggt    768
   Lys Ala Asp Lys Ser Lys Val Lys Leu Thr Ile Ser Asp Asp Leu Gly
                   245                 250                 255
   caa acc aca ctt gaa gtt ttc aaa gaa gat ggc aaa aca cta gta tca    816
   Gln Thr Thr Leu Glu Val Phe Lys Glu Asp Gly Lys Thr Leu Val Ser
               260                 265                 270
   aaa aaa gta act tcc aaa gac aag tca tca aca gaa gaa aaa ttc aat    864
   Lys Lys Val Thr Ser Lys Asp Lys Ser Ser Thr Glu Glu Lys Phe Asn
           275                 280                 285
   gaa aaa ggt gaa gta tct gaa aaa ata ata aca aga gca gac gga acc    912
   Glu Lys Gly Glu Val Ser Glu Lys Ile Ile Thr Arg Ala Asp Gly Thr
       290                 295                 300
   aga ctt gaa tac aca gga att aaa agc gat gga tct gga aaa gct aaa    960
   Arg Leu Glu Tyr Thr Gly Ile Lys Ser Asp Gly Ser Gly Lys Ala Lys
   305                 310                 315                 320
   gag gtt tta aaa ggc ttt act ctt gaa gga aaa gta gct aat gat aaa    1008
   Glu Val Leu Lys Gly Phe Thr Leu Glu Gly Lys Val Ala Asn Asp Lys
                   325                 330                 335
   gta aca ttg gaa gta aaa gaa gga acc gtt act tta agt aag att tca    1056
   Val Thr Leu Glu Val Lys Glu Gly Thr Val Thr Leu Ser Lys Ile Ser
               340                 345                 350
   aaa tct ggg gaa gtt tca gtt gaa ctt aat gac act gac agt agt gct    1104
   Lys Ser Gly Glu Val Ser Val Glu Leu Asn Asp Thr Asp Ser Ser Ala
           355                 360                 365
   gct act aaa aaa act gca gct tgg aat tca aaa act tct act tta aca    1152
   Ala Thr Lys Lys Thr Ala Ala Trp Asn Ser Lys Thr Ser Thr Leu Thr
       370                 375                 380
   att agt gtt aac agc aaa aaa act aca caa ctt gtg ttt act aaa caa    1200
   Ile Ser Val Asn Ser Lys Lys Thr Thr Gln Leu Val Phe Thr Lys Gln
   385                 390                 395                 400
   gac aca ata act gta caa aaa tac gac tcc gca ggt acc aat tta gaa    1248
   Asp Thr Ile Thr Val Gln Lys Tyr Asp Ser Ala Gly Thr Asn Leu Glu
                   405                 410                 415
   ggc aca gca gtc gaa att aaa aca ctt gat gaa ctt aaa aac gct tta    1296
   Gly Thr Ala Val Glu Ile Lys Thr Leu Asp Glu Leu Lys Asn Ala Leu
               420                 425                 430
   aaa taa                                                            1302
   Lys  *
<210> 180
   <211> 433
   <212> PRT
   <213> Artificial Sequence
<220>
   <223> Chimeric protein
<400> 180
   Met Ala Cys Asn Asn Ser Gly Lys Asp Gly Asn Thr Ser Ala Asn Ser
   1               5                   10                  15
   Ala Asp Glu Ser Val Lys Gly Pro Asn Leu Thr Glu Ile Ser Lys Lys
               20                  25                  30
   Ile Thr Asp Ser Asn Ala Val Leu Leu Ala Val Lys Glu Val Glu Ala
           35                  40                  45
   Leu Leu Ser Ser Ile Asp Glu Leu Ala Lys Ala Ile Gly Lys Lys Ile
       50                  55                  60
   Lys Asn Asp Gly Ser Leu Asp Asn Glu Ala Asn Arg Asn Glu Ser Leu
   65 70 75 80
   Leu Ala Gly Ala Tyr Thr Ile Ser Thr Leu Ile Thr Gln Lys Leu Ser
   85 90 95
   Lys Leu Asn Gly Ser Glu Gly Leu Lys Glu Lys Ile Ala Ala Ala Lys
   100 105 110
   Lys Cys Ser Glu Glu Phe Ser Thr Lys Leu Lys Asp Asn His Ala Gln
   115 120 125
   Leu Gly Ile Gln Gly Val Thr Asp Glu Asn Ala Lys Lys Ala Ile Leu
   130 135 140
   Lys Ala Asn Ala Ala Gly Lys Asp Lys Gly Val Glu Glu Leu Glu Lys
   145 150 155 160
   Leu Ser Gly Ser Leu Glu Ser Leu Ser Lys Ala Ala Lys Glu Met Leu
   165 170 175
   Ala Asn Ser Val Lys Glu Leu Thr Ser Pro Val Val His Gly Val Ser
   180 185 190
   Val Asp Leu Pro Gly Glu Met Lys Val Leu Val Ser Lys Glu Lys Asn
   195 200 205
   Lys Asp Gly Lys Tyr Asp Leu Ile Ala Thr Val Asp Lys Leu Glu Leu
   210 215 220
   Lys Gly Thr Ser Asp Lys Asn Asn Gly Ser Gly Val Leu Glu Gly Val
   225 230 235 240
   Lys Ala Asp Lys Ser Lys Val Lys Leu Thr Ile Ser Asp Asp Leu Gly
   245 250 255
   Gln Thr Thr Leu Glu Val Phe Lys Glu Asp Gly Lys Thr Leu Val Ser
   260 265 270
   Lys Lys Val Thr Ser Lys Asp Lys Ser Ser Thr Glu Glu Lys Phe Asn
   275 280 285
   Glu Lys Gly Glu Val Ser Glu Lys Ile Ile Thr Arg Ala Asp Gly Thr
   290 295 300
   Arg Leu Glu Tyr Thr Gly Ile Lys Ser Asp Gly Ser Gly Lys Ala Lys
   305 310 315 320
   Glu Val Leu Lys Gly Phe Thr Leu Glu Gly Lys Val Ala Asn Asp Lys
   325 330 335
   Val Thr Leu Glu Val Lys Glu Gly Thr Val Thr Leu Ser Lys Ile Ser
   340 345 350
   Lys Ser Gly Glu Val Ser Val Glu Leu Asn Asp Thr Asp Ser Ser Ala
   355 360 365
   Ala Thr Lys Lys Thr Ala Ala Trp Asn Ser Lys Thr Ser Thr Leu Thr
   370 375 380
   Ile Ser Val Asn Ser Lys Lys Thr Thr Gln Leu Val Phe Thr Lys Gln
   385 390 395 400
   Asp Thr Ile Thr Val Gln Lys Tyr Asp Ser Ala Gly Thr Asn Leu Glu
   405 410 415
   Gly Thr Ala Val Glu Ile Lys Thr Leu Asp Glu Leu Lys Asn Ala Leu
   420 425 430
   Lys
<210> 181
   <211> 31
   <212> DNA
   <213> Artificial Sequence
<220>
   <223> Oligonucleotide Primer
<400> 181
   gtcatatggc ttgtaataat tcagggaaag a 31
<210> 182
   <211> 30
   <212> DNA
   <213> Artificial Sequence
<220>
   <223> Oligonucleotide Primer
<400> 182
   tttccatgga aggttttttt ggactttctg 30
<210> 183
   <211> 30
   <212> DNA
   <213> Artificial Sequence
<220>
   <223> Oligonucleotide Primer
<400> 183
   tttccatggc caagcaaaat gttagcagcc 30
<210> 184
   <211> 27
   <212> DNA
   <213> Artificial Sequence
<220>
   <223> Oligonucleotide Primer
<400> 184
   taaggatcct tattttaaag cgttttt 27
<210> 185
   <211> 819
   <212> DNA
   <213> Borrelia burgdorferi
<220>
   <221> CDS
   <222> (1)...(819)
<400> 185
   atg aaa aaa tat tta ttg gga ata ggt cta ata tta gcc tta ata gca    48
   Met Lys Lys Tyr Leu Leu Gly Ile Gly Leu Ile Leu Ala Leu Ile Ala
    1               5                  10                  15
   tgt aag caa aat gtt agc agc ctt gac gag aaa aac agc gtt tca gta    96
   Cys Lys Gln Asn Val Ser Ser Leu Asp Glu Lys Asn Ser Val Ser Val
               20                  25                  30
   gat ttg cct ggt gaa atg aaa gtt ctt gta agc aaa gaa aaa aac aaa     144
   Asp Leu Pro Gly Glu Met Lys Val Leu Val Ser Lys Glu Lys Asn Lys
           35                  40                  45
   gac ggc aag tac gat cta att gca aca gta gac aag ctt gag ctt aaa     192
   Asp Gly Lys Tyr Asp Leu Ile Ala Thr Val Asp Lys Leu Glu Leu Lys
       50                  55                  60
   gga act tct gat aaa aac aat gga tct gga gta ctt gaa ggc gta aaa     240
   Gly Thr Ser Asp Lys Asn Asn Gly Ser Gly Val Leu Glu Gly Val Lys
   65                  70                  75                  80
   gct gac aaa agt aaa gta aaa tta aca att tct gac gat cta ggt caa     288
   Ala Asp Lys Ser Lys Val Lys Leu Thr Ile Ser Asp Asp Leu Gly Gln
                   85                  90                  95
   acc aca ctt gaa gtt ttc aaa gaa gat ggc aaa aca cta gta tca aaa     336
   Thr Thr Leu Glu Val Phe Lys Glu Asp Gly Lys Thr Leu Val Ser Lys
               100                 105                 110
   aaa gta act tcc aaa gac aag tca tca aca gaa gaa aaa ttc aat gaa     384
   Lys Val Thr Ser Lys Asp Lys Ser Ser Thr Glu Glu Lys Phe Asn Glu
           115                 120                 125
   aaa ggt gaa gta tct gaa aaa ata ata aca aga gca gac gga acc aga     432
   Lys Gly Glu Val Ser Glu Lys Ile Ile Thr Arg Ala Asp Gly Thr Arg
       130                 135                 140
   ctt gaa tac aca gga att aaa agc gat gga tct gga aaa gct aaa gag     480
   Leu Glu Tyr Thr Gly Ile Lys Ser Asp Gly Ser Gly Lys Ala Lys Glu
   145                 150                 155                 160
   gtt tta aaa ggc tat gtt ctt gaa gga act cta act gct gaa aaa aca     528
   Val Leu Lys Gly Tyr Val Leu Glu Gly Thr Leu Thr Ala Glu Lys Thr
                   165                 170                 175
   aca ttg gtg gtt aaa gaa gga act gtt act tta agc aaa aat att tca     576
   Thr Leu Val Val Lys Glu Gly Thr Val Thr Leu Ser Lys Asn Ile Ser
               180                 185                 190
   aaa tct ggg gaa gtt tca gtt gaa ctt aat gac act gac agt agt gct     624
   Lys Ser Gly Glu Val Ser Val Glu Leu Asn Asp Thr Asp Ser Ser Ala
           195                 200                 205
   gct act aaa aaa act gca gct tgg aat gca ggc act tca act tta aca     672
   Ala Thr Lys Lys Thr Ala Ala Trp Asn Ala Gly Thr Ser Thr Leu Thr
       210                 215                 220
   att act gta aac aac aaa aaa act aaa gcc ctt gta ttt aca aaa caa     720
   Ile Thr Val Asn Asn Lys Lys Thr Lys Ala Leu Val Phe Thr Lys Gln
   225                 230                 235                 240
   gac aca att aca tca caa aaa tac gac tca gca gga acc aac ttg gaa     768
   Asp Thr Ile Thr Ser Gln Lys Tyr Asp Ser Ala Gly Thr Asn Leu Glu
                   245                 250                 255
   ggc aca gca gtc gaa att aaa aca ctt gat gaa ctt aaa aac gct tta     816
   Gly Thr Ala Val Glu Ile Lys Thr Leu Asp Glu Leu Lys Asn Ala Leu
               260                 265                 270
   aga                                                                 819
   Arg
<210> 186
   <211> 273
   <212> PRT
   <213> Borrelia burgdorferi
<400> 186
   Met Lys Lys Tyr Leu Leu Gly Ile Gly Leu Ile Leu Ala Leu Ile Ala
   1               5                   10                  15
   Cys Lys Gln Asn Val Ser Ser Leu Asp Glu Lys Asn Ser Val Ser Val
               20                  25                  30
   Asp Leu Pro Gly Glu Met Lys Val Leu Val Ser Lys Glu Lys Asn Lys
           35                  40                  45
   Asp Gly Lys Tyr Asp Leu Ile Ala Thr Val Asp Lys Leu Glu Leu Lys
       50                  55                  60
   Gly Thr Ser Asp Lys Asn Asn Gly Ser Gly Val Leu Glu Gly Val Lys
   65                  70                  75                  80
   Ala Asp Lys Ser Lys Val Lys Leu Thr Ile Ser Asp Asp Leu Gly Gln
                   85                  90                  95
   Thr Thr Leu Glu Val Phe Lys Glu Asp Gly Lys Thr Leu Val Ser Lys
               100                 105                 110
   Lys Val Thr Ser Lys Asp Lys Ser Ser Thr Glu Glu Lys Phe Asn Glu
           115                 120                 125
   Lys Gly Glu Val Ser Glu Lys Ile Ile Thr Arg Ala Asp Gly Thr Arg
       130                 135                 140
   Leu Glu Tyr Thr Gly Ile Lys Ser Asp Gly Ser Gly Lys Ala Lys Glu
   145                 150                 155                 160
   Val Leu Lys Gly Tyr Val Leu Glu Gly Thr Leu Thr Ala Glu Lys Thr
                   165                 170                 175
   Thr Leu Val Val Lys Glu Gly Thr Val Thr Leu Ser Lys Asn Ile Ser
               180                 185                 190
   Lys Ser Gly Glu Val Ser Val Glu Leu Asn Asp Thr Asp Ser Ser Ala
           195                 200                 205
   Ala Thr Lys Lys Thr Ala Ala Trp Asn Ala Gly Thr Ser Thr Leu Thr
       210                 215                 220
   Ile Thr Val Asn Asn Lys Lys Thr Lys Ala Leu Val Phe Thr Lys Gln
   225                 230                 235                 240
   Asp Thr Ile Thr Ser Gln Lys Tyr Asp Ser Ala Gly Thr Asn Leu Glu
                   245                 250                 255
   Gly Thr Ala Val Glu Ile Lys Thr Leu Asp Glu Leu Lys Asn Ala Leu
               260                 265                 270
   Arg

## Description

### BACKGROUND OF THE INVENTION

Lyme disease (Lyme borreliosis) is the most common tick-borne infectious disease in North America and Europe, and has been found in Russia, Japan, China and Australia. Lyme disease begins at the site of a tick bite, producing a primary infection with spread of the organism to secondary sites occurring during the course of infection. The causative bacterial agent of this disease is the spirochete *Borrelia burgdorferi,* which was first isolated and cultivated in 1982 (Burgdorferi, W.A. et al., Science 216: 1317-1319 (1982); Steere, A.R. et al., N. Engl. J. Med. 308: 733-740 (1983)). With that discovery, a wide array of clinical syndromes, described in both the European and American literature since the early 20th century, could be attributed to infection by *B. burgdorferi* (Afzelius, A., Acta Derm. Venereol. 2: 120-125 (1921); Bannwarth, A., Arch. Psychiatr. Nervenkrankh. 117: 161-185 (1944); Garin, C. and A. Bujadouz, J. Med. Lyon 71: 765-767 (1922); Herxheimer, K. and K. Hartmann, Arch. Dermatol. Syphilol. 61: 57-76, 255-300 (1902)).

Three pathogenic genospecies of *Borrelia, B. burgdorferi* sensu stricto *(B. burgdorferi* or *B.b.s.s.), B. afzelii* and *B. garinii* have been described (Baranton, G., et al., Int. J. Syst. Bacteriol. 42:378-383 (1992)). These are members of a species complex, *B. burgdorferi* sensu lato, which consists of at least 10 different genospecies (Piken, R.N., et al., J. Invest. Dermatol., 110:211-214 (1998); Postic, D., et al., Int. J. Syst. Bacteriol. 44:743-752 (1994); Valsangiacomo, C.T., et al., Int. J. Syst. Bacteriol. 47:1-10 (1997)). The three genospecies, *B. burgdorferi* sensu stricto, *B. afzelii* and *B. garinii,* all are thought to be pathogenic and all are found in Europe. However, in North America, *B. burgdorferi* sensu stricto is the only identified pathogenic genospecies. Each of these three genospecies is associated with distinct clinical manifestations (Van Dam, A. P. et al., Clin. Infect. Dis. 17:708-717 (1993)). This implies that differences in genospecies may play an important role in the wide array of clinical manifestations observed in Lyme Disease.

OspA is a basic lipoprotein of approximately 31 kd, which is encoded on a large linear plasmid along with OspB, a basic lipoprotein of approximately 34 kd (Szczepanski, A., and J.L. Benach, Microbiol. Rev. 55:21 (1991)). Analysis of isolates of *B. burgdorferi* obtained from North America and Europe has demonstrated that OspA has antigenic variability, and that several distinct groups can be serologically and genotypically defined (Wilske, B., et al., World J. Microbiol. 7: 130 (1991)). Other *Borrelia* proteins demonstrate similar antigenic variability. Surprisingly, the immune response to these outer surface proteins tends to occur late in the disease, if at all (Craft, J. E. et al., J. Clin Invest. 78: 934-939 (1986); Dattwyler, R.J. and B.J. Luft, Rheum. Clin. North Am. 15: 727-734 (1989)). Furthermore, patients acutely and chronically infected with *B. burgdorferi* respond variably to the different antigens, including OspA, OspB, OspC, OspD, p39, p41 and p93.

As an infected tick begins to feed on a mammal, the synthesis of another outer surface protein, outer surface protein C or OspC, is induced (Schwan, T.G., et al., Proc. Natl. Acad. Sci. 2:2909-2913 (1995)). Thus, in early infection, OspC is the major outer membrane protein expressed by the spirochete (Fung, B.P., et al., Infect. Immun. 62:3213-3221 (1994); Padula, S.J., et al., J. Clin. Microbiol., 32:1733-1738 (1994)). Even through OspC has been demonstrated to have limited surface exposure (Cox, D.L., et al., Proc. Natl. Acad. Sci., 93:7973-7978 (1996); Mathiesen, M. M., et al., Infect. Immun. 66:4073-4079 (1998)), OspC is a potent immunogen. Immunization with OspC is protective against tick-transmitted *Borrelia* infection (Gilmore Jr., R.D., Infect. Immun. 64:2234-2239 (1999)). However, because OspC is highly variable in its sequence, the protection is limited to the *Borrelia burgdorferi* strain expressing the same allele of OspC. Challenge with heterologous isolates, expressing other *ospC* alleles results in infection (Probert, W.S., et al., J. Infect. D., 175:400-405 (1997)). OspC is a very diverse genetic locus (Jauris-Heipke, S., et al., Med. Microbiol. Immunol. 182:37-50 (1993)) as evidenced by the fact that Livey *et al.* found thirty-four alleles of OspC in seventy-six *B. burgdorferi sensu lato* isolates (Livey, I., et al., Mol. Microbiol. 18:257-269 (1995)).

Currently, Lyme Disease is treated with a range of antibiotics, e.g., tetracyclines, penicillin and cephalosporins. However, such treatment is not always successful in clearing the infection. Treatment is often delayed due to improper diagnosis with the deleterious effect that the infection proceeds to a chronic condition, where treatment with antibiotics is often not useful. One of the factors contributing to delayed treatment is the lack of effective diagnostic tools.

Vaccines against Lyme borreliosis have been attempted. Mice immunized with a recombinant form of OspA are protected from challenge with the same strain of *B. burgdorferi* from which the protein was obtained (Fikrig, E., et al., Science 250: 553-556 (1990)). Furthermore, passively transferred anti-OspA monoclonal antibodies (MAbs) have been shown to be protective in mice, and vaccination with a recombinant protein induced protective immunity against subsequent infection with the homologous strain of *B. burgdorferi* (Simon, M.M., et al., J. Infect. Dis. 164: 123 (1991)). In addition, there have been two independent trials of first generation vaccines for the prevention of Lyme disease that have studied the efficacy and safety of a vaccine based on recombinant outer surface protein A (OspA) (Sigal, L.H. et al., N. Engl. J. Med 339:216-222, 1998; Steere, A.C. et al., N. Engl. J. Med. 339:209-215, (1998)). However, a vaccine that consists of recombinant OspA may require frequent booster immunizations. An additional concern of OspA-based vaccines is the recent identification of a putative autoreactive OspA domain with a high degree of similarity to a region of human leukocyte function-associated antigen-1 (hLFA-1) (Gross, D.M. et al., Science, 281: 703-706 (1998)).

It has been noted that immunization with a single protein from a particular strain of *Borrelia* often does not confer resistance to that strain in all individuals (Fikrig, E. et al., J. Immunol. 7: 2256-1160 (1992)). There is considerable variation displayed in OspA, OspB and OspC, as well as p93, including the regions conferring antigenicity. Therefore, the degree and frequency of protection from vaccination with a protein from a single strain depend upon the response of the immune system to the particular variation, as well as the frequency of genetic variation in *B. burgdorferi.* In the case of vaccines directed against OspA, the vaccine is typically only effective against strains of *Borrelia* that express OspA that is homologous to OspA from which the vaccine was derived.

Another limitation of current OspA Lyme Disease vaccines is that they are directed against an antigen that is expressed predominantly in the tick vector. Indeed, recent reports have indicated that *Borrelia burgdorferi* in infected ticks alter their surface expression by increasing expression of OspC during ingestion of a blood meal (Schwan, T.G. et al., Proc. Natl. Acad Sci. USA, 92: 2909-2913 (1995)). Thus, it seems that natural infection with *B. burgdorferi* does not elicit an antibody response to OspA, as it does against OspC.

Given the heterogeneity of antigenic determinants present in *Borrelia* proteins, a need exists for a vaccine and diagnostic tool which can provide immunogenicity to various strains and/or genospecies of *Borrelia burgdorferi,* as well as to more epitopes within a strain or genospecies. There is also a need for vaccines and diagnostic tools which detect antibody responses against immunoprotective targets that are expressed at different stages of the life cycle of *Borrelia burgdorferi.* This would allow for diagnosis and/or vaccination against all, or most forms, *of Borrelia* that cause systemic disease.

WO-A-9512676 is by the same inventors as this invention and discloses earlier chimeric proteins of Borrelia polypeptides.

### SUMMARY OF THE INVENTION

The current invention pertains to chimeric *Borrelia* proteins which include two or more antigenic *Borrelia* polypeptides which do not occur naturally (in nature) in the same protein in *Borrelia,* as well as the nucleic acids encoding such chimeric proteins. The proteins from which the antigenic polypeptides are derived can be from the same strain or genospecies of *Borrelia,* from different strains or genospecies, or from combinations of proteins from the same and from different strains or genospecies. Particular chimeric proteins, and the nucleotide sequences encoding them, are set forth in Figs, 30-37 and 55-72..

The chimeric proteins of the current invention provide antigenic polypeptides of a variety of *Borrelia* strains and/or proteins within a single protein. Such proteins are particularly useful in immunodiagnostic assays to detect the presence of antibodies to native *Borrelia* in potentially infected individuals as well as to measure T-coll reactivity, and can therefore be used as immunodiagnostic reagents. These chimeric proteins are also useful in the generation of immune responses (such as antibody production) against proteins expressed by *Borrelia burgdorferi.* The chimeric proteins of the current invention are additionally useful as vaccine immunogens against *Borrelia* infection.

In one embodiment of the present invention, the chimeric proteins are made up of polypeptide fragments from Lyme Disease-causing strains of *Borrelia.* The polypeptide fragments that make up the chimeric protein are from outer surface protein A (OspA) and outer surface protein C (OspC), which have the general structure of OspC linked via a peptide bond to the N-terminus of OspA. The present invention encompasses unlipidated chimeric proteins. The OspC polypeptide portion does not include a lipidation signal.

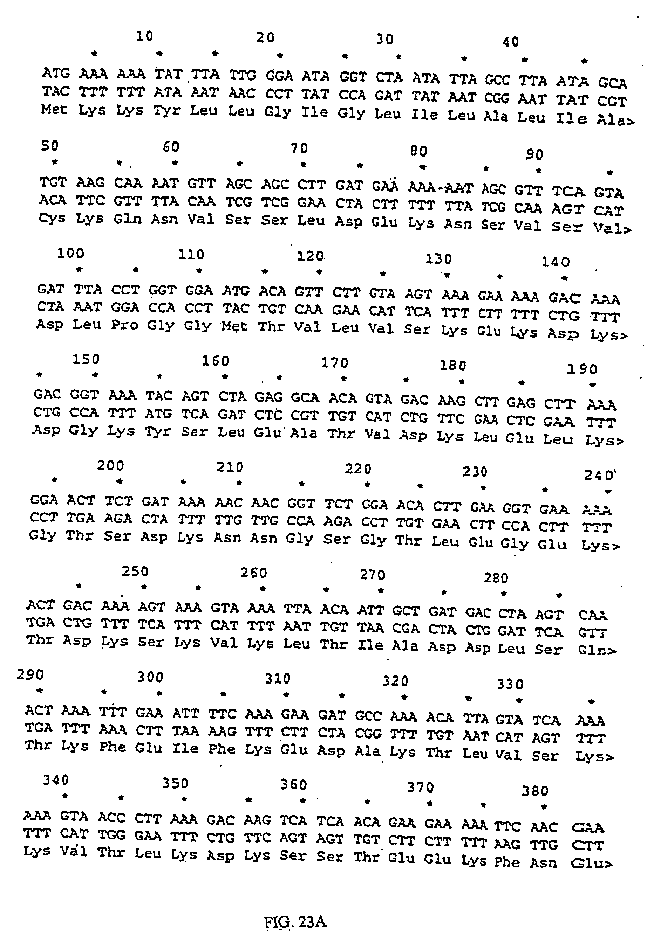

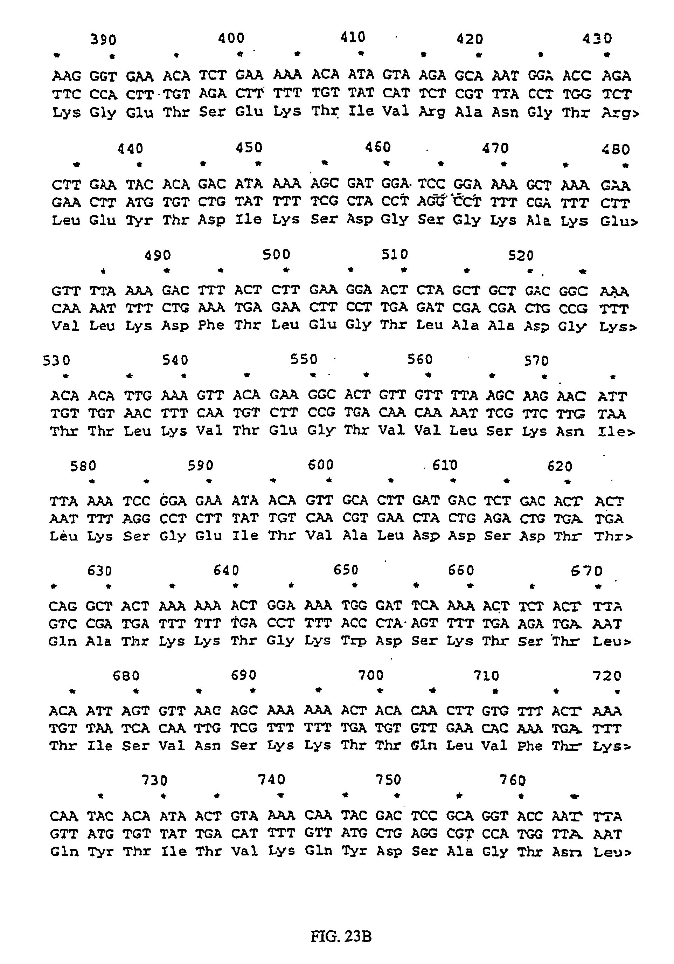

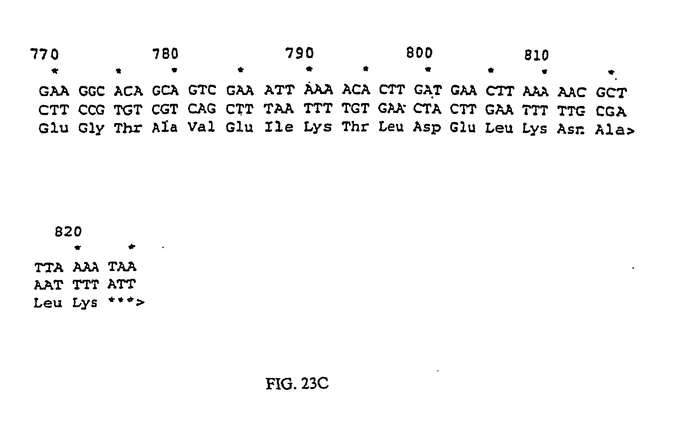

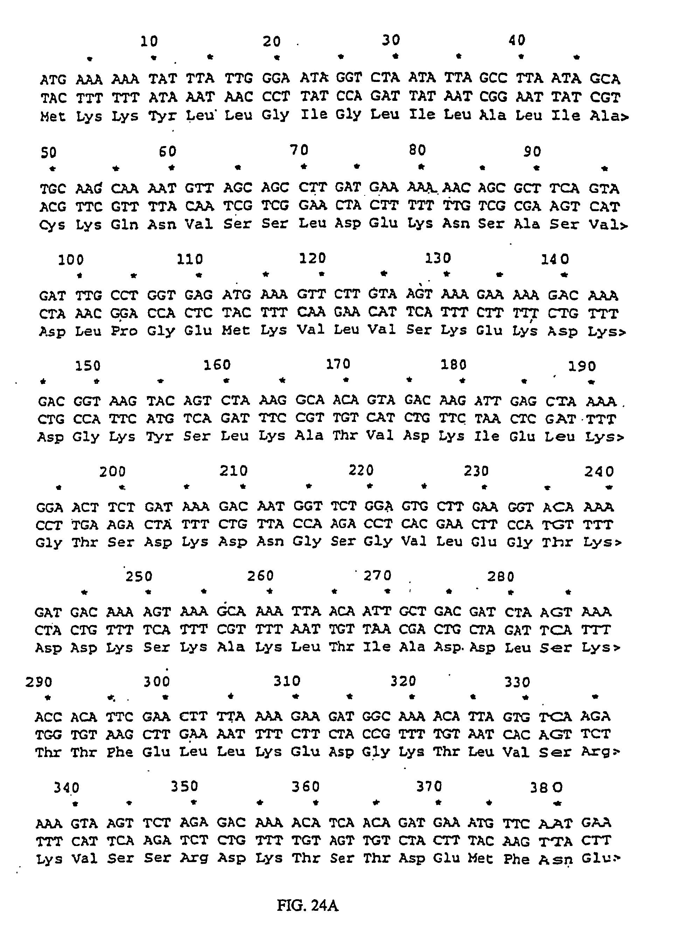

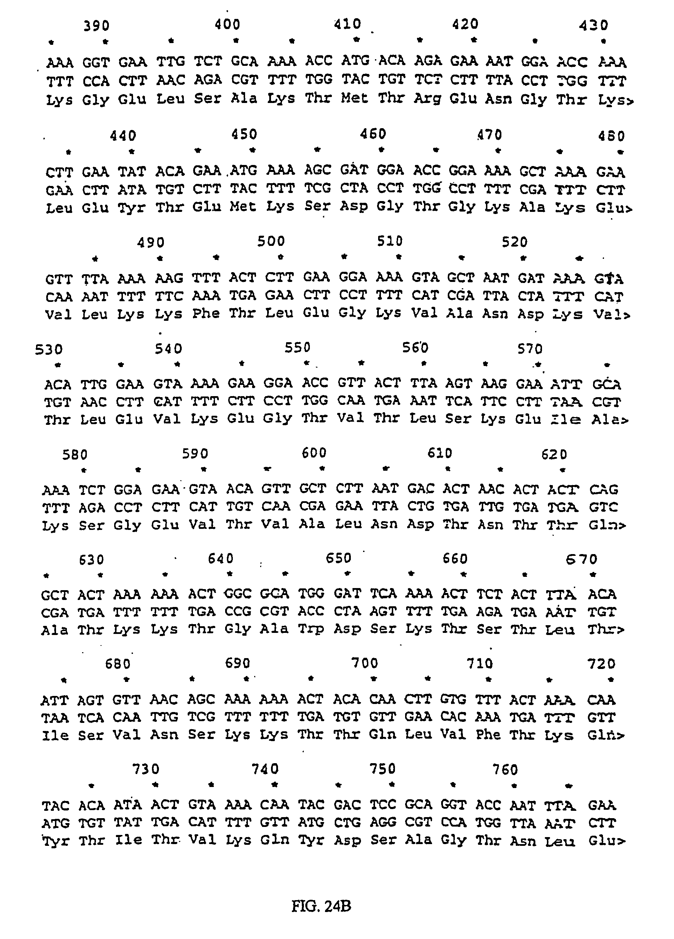

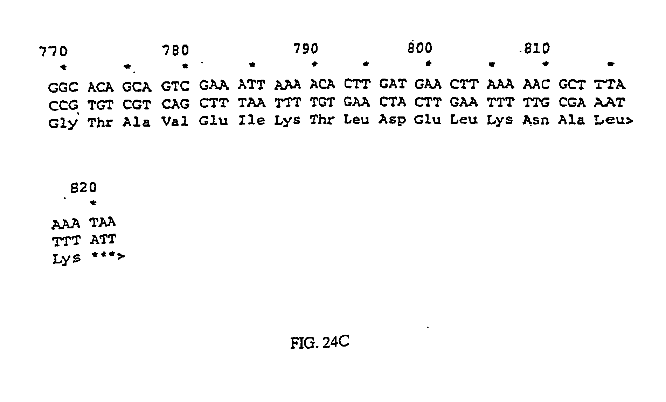

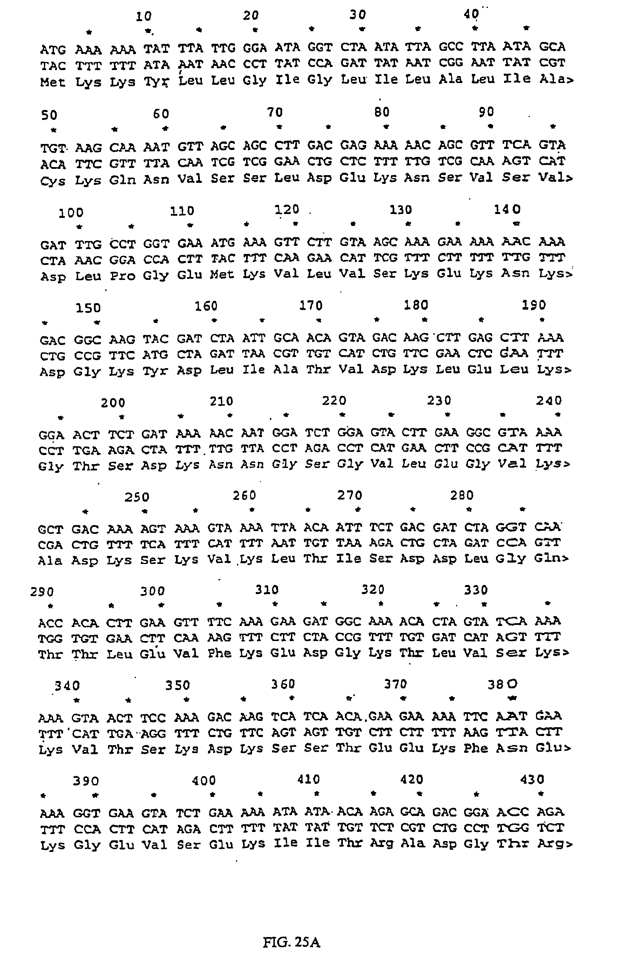

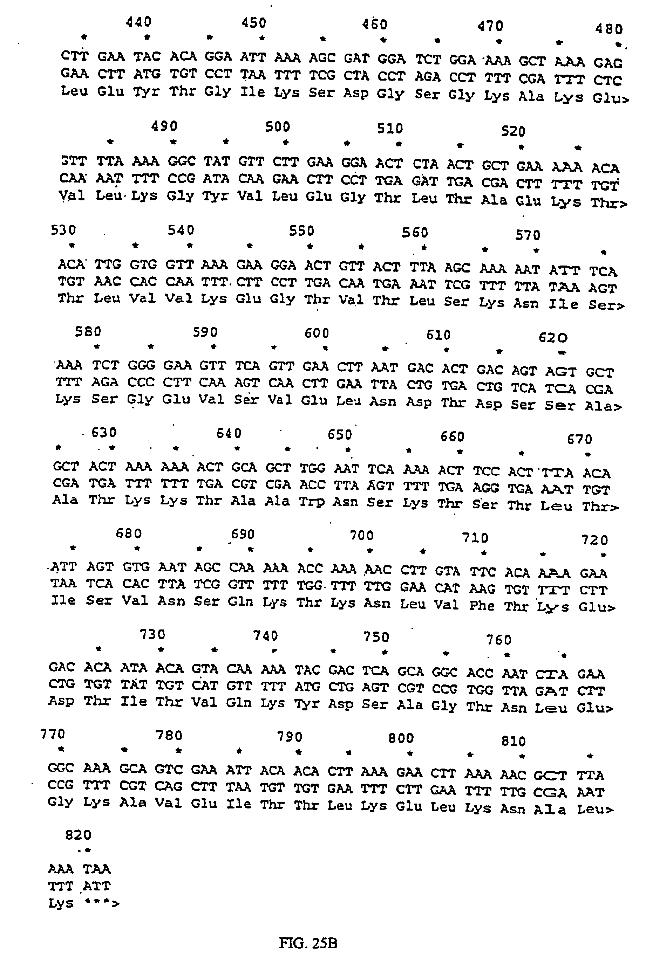

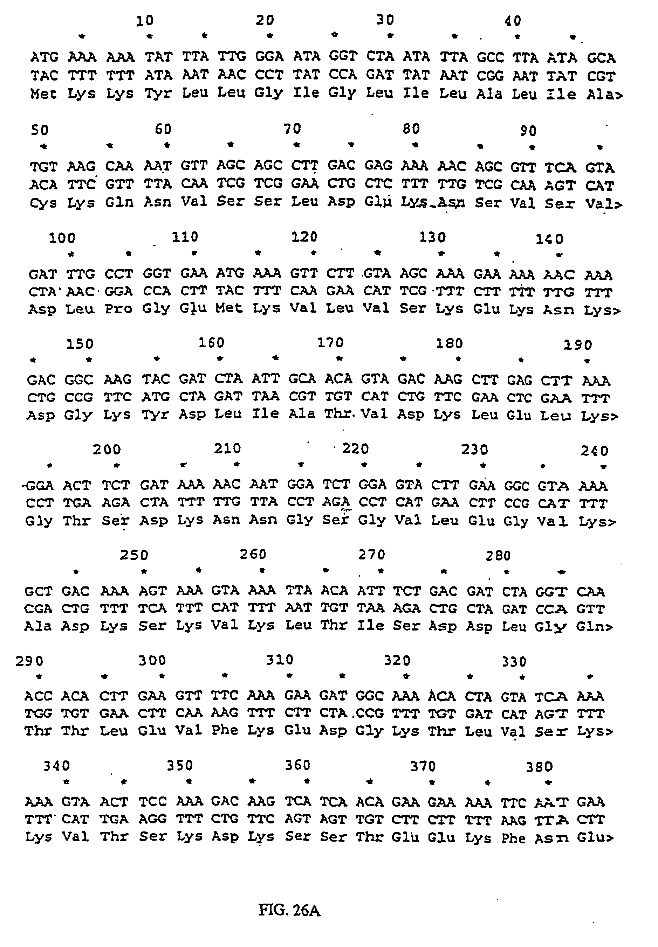

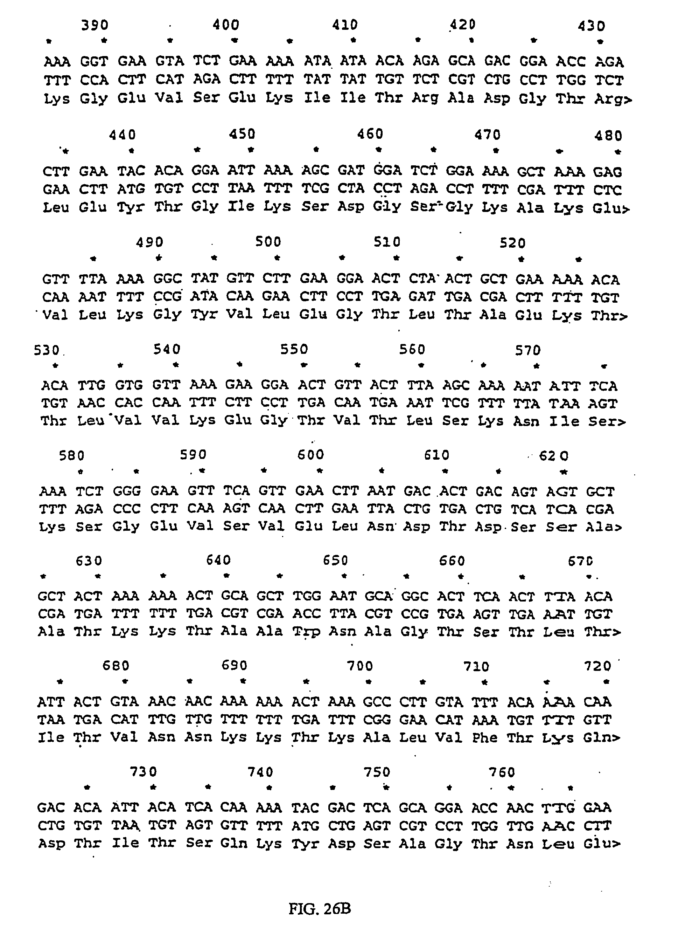

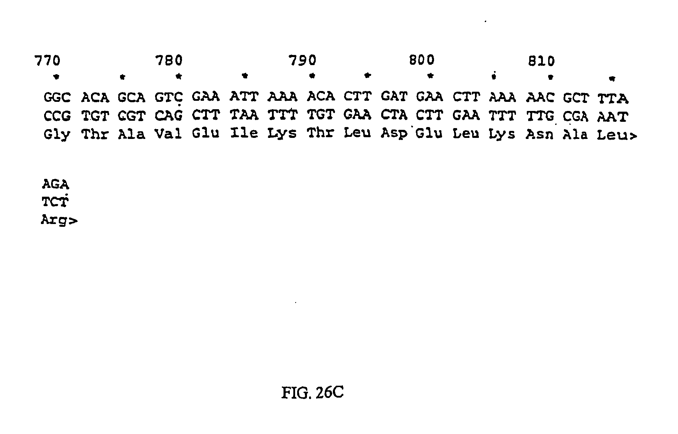

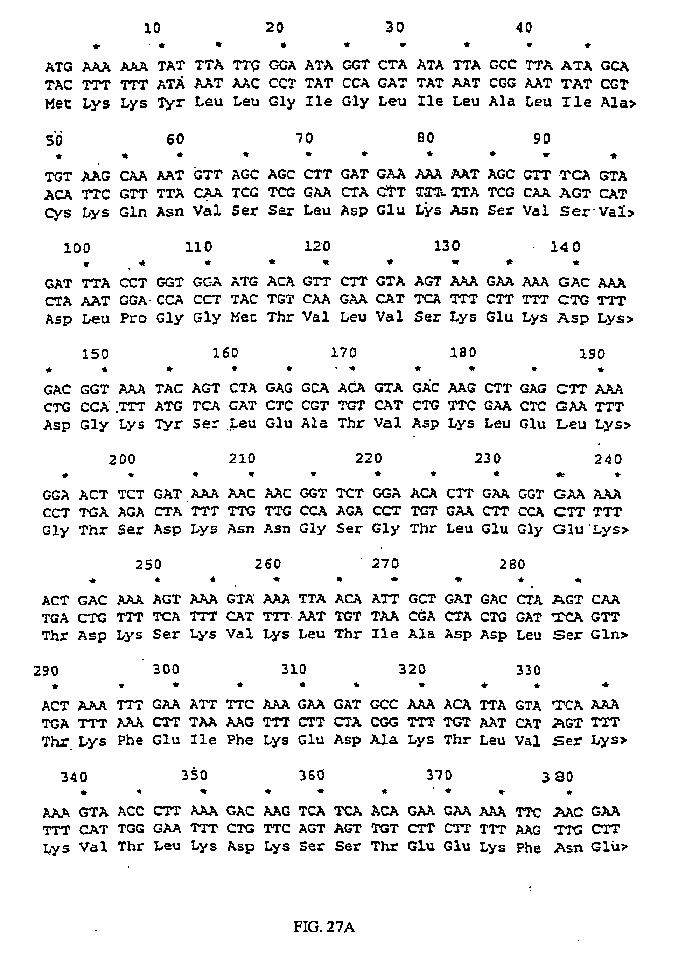

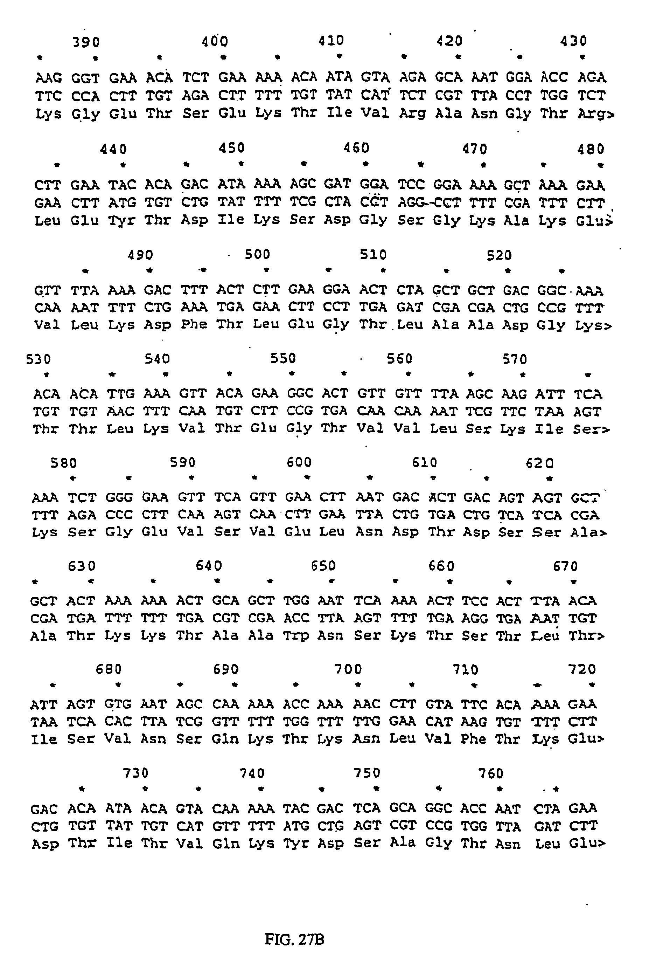

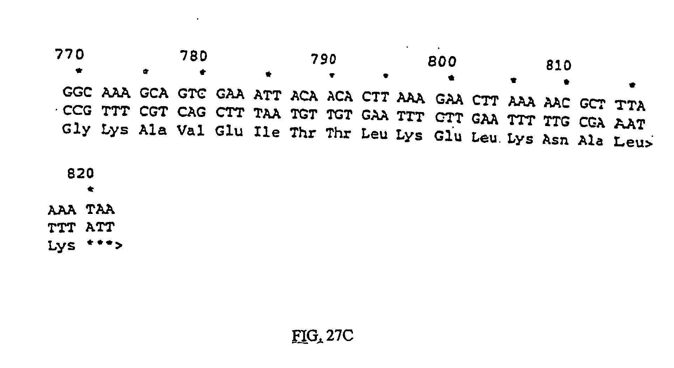

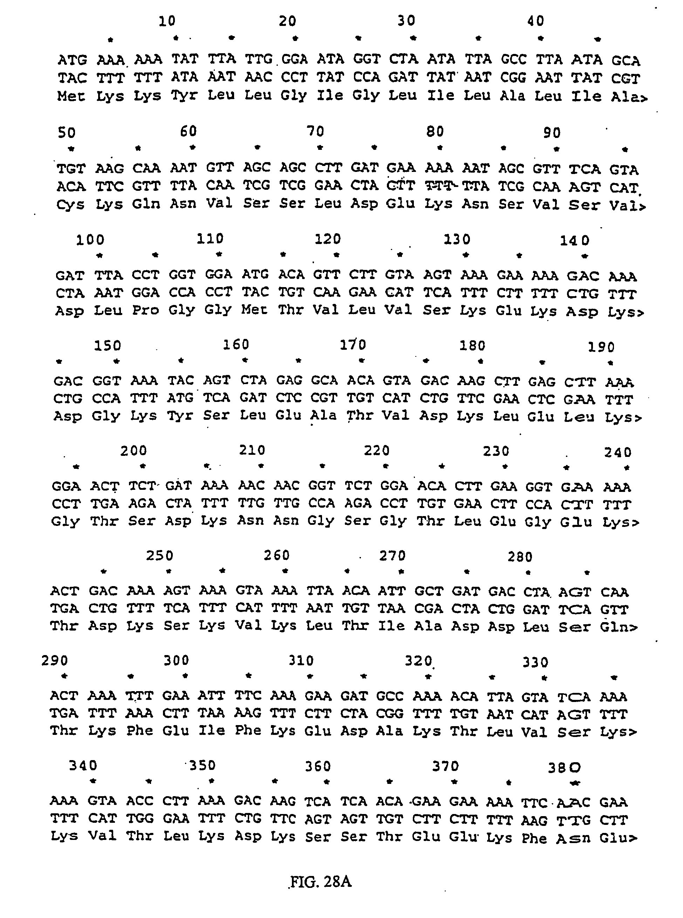

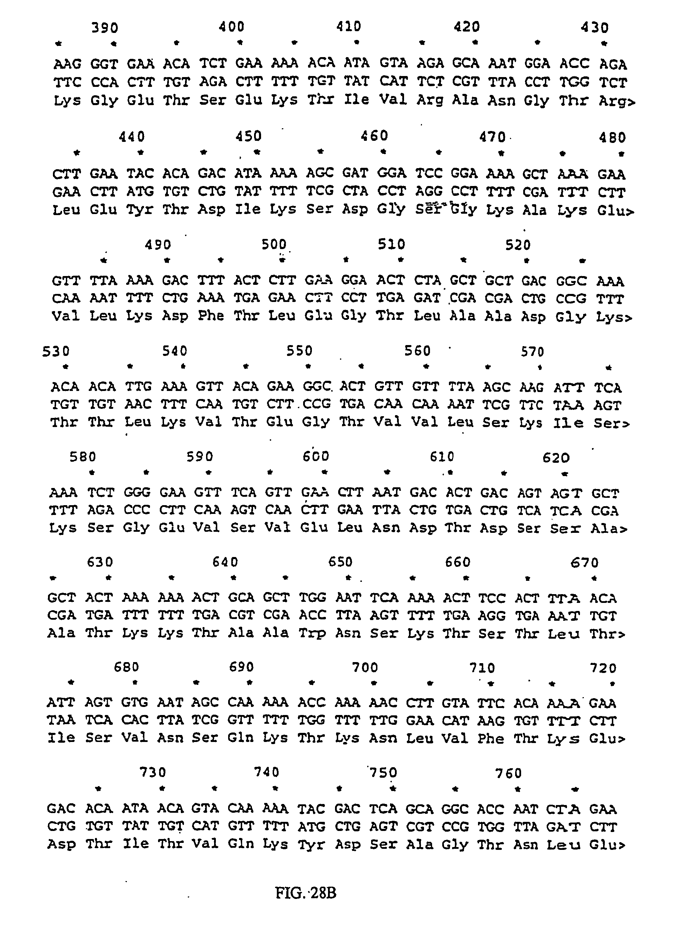

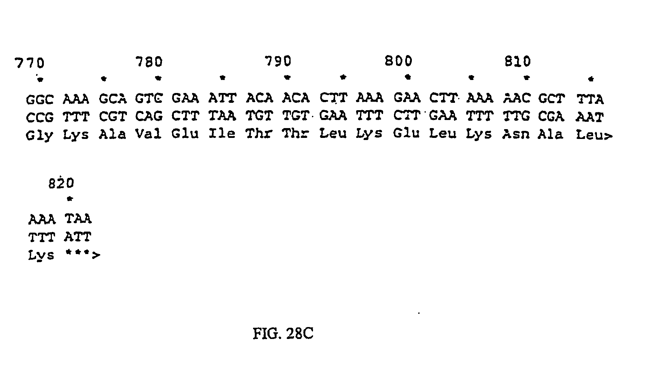

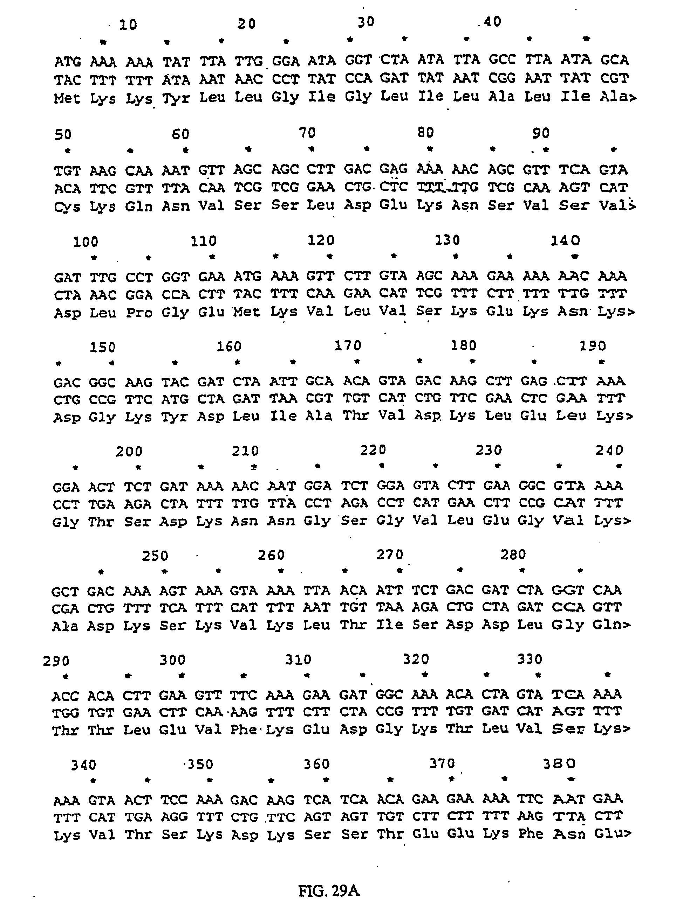

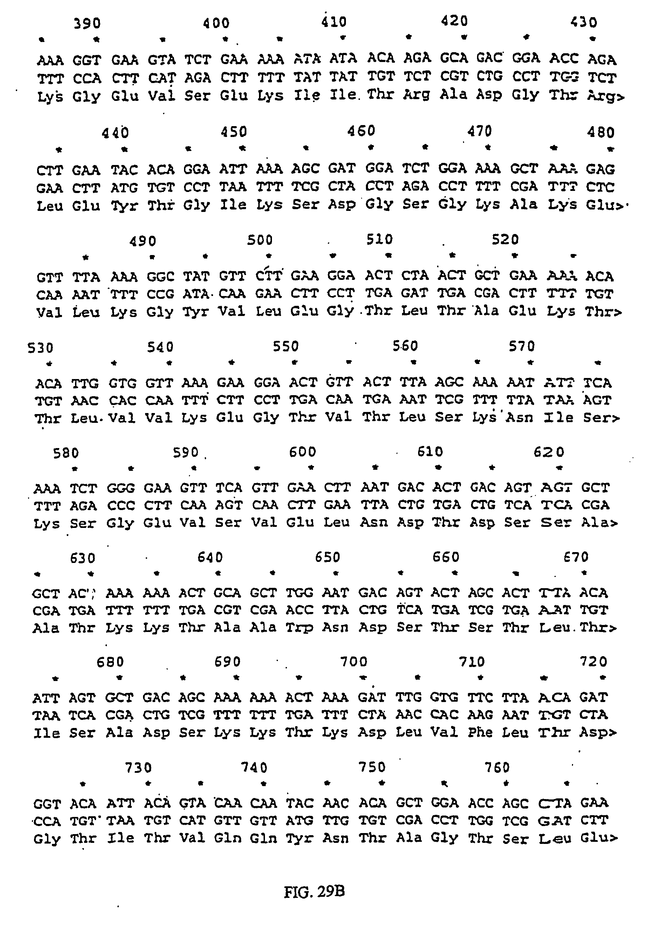

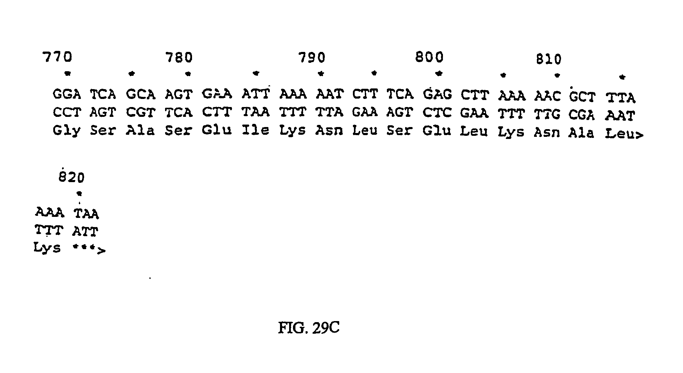

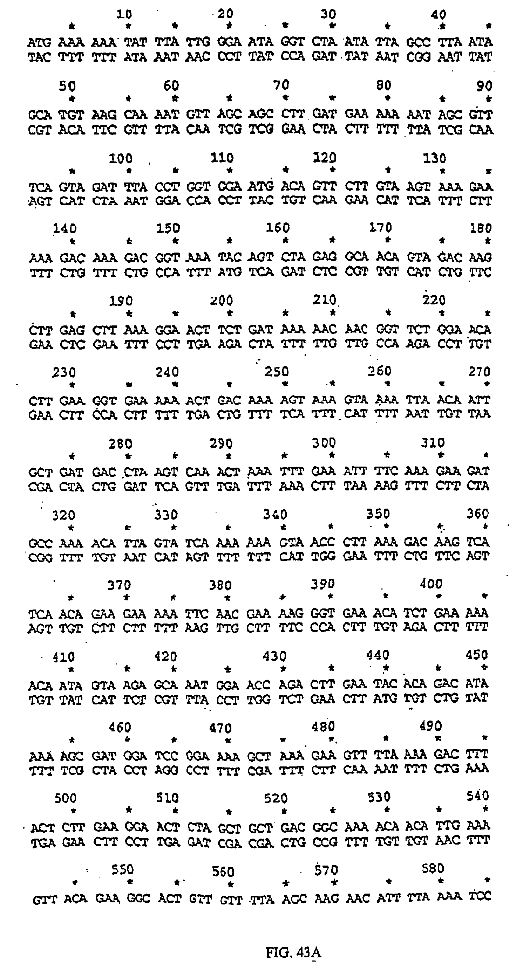

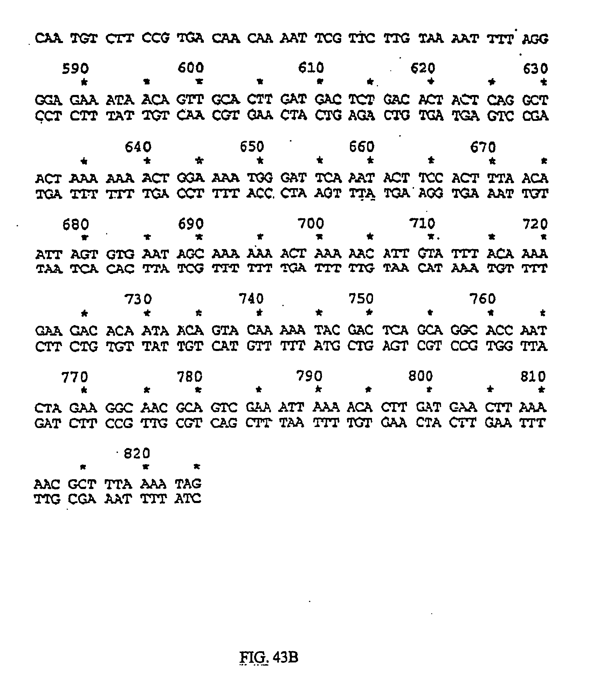

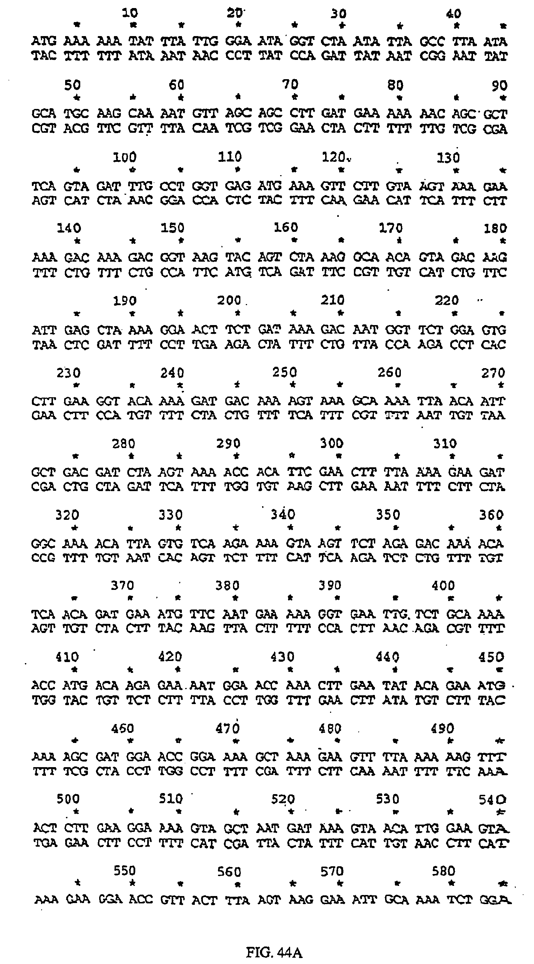

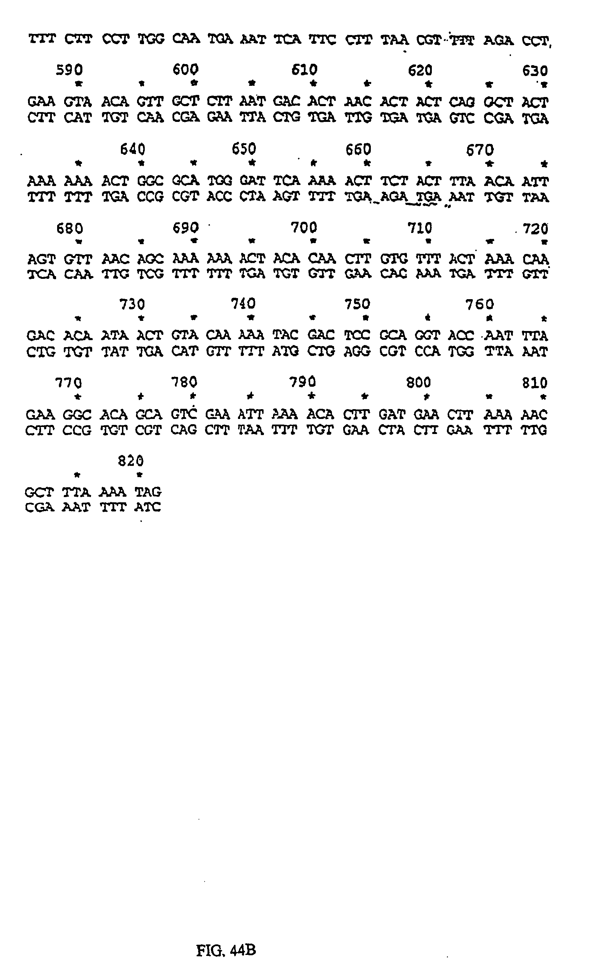

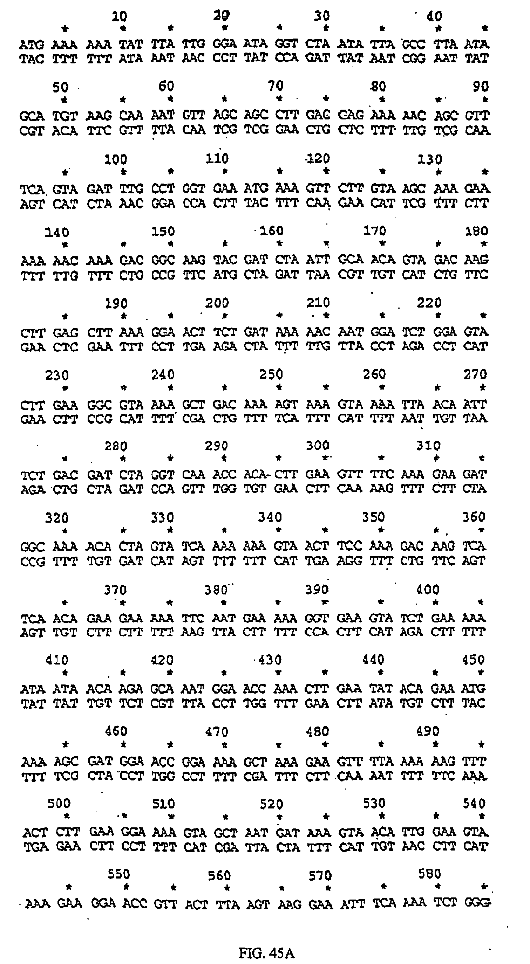

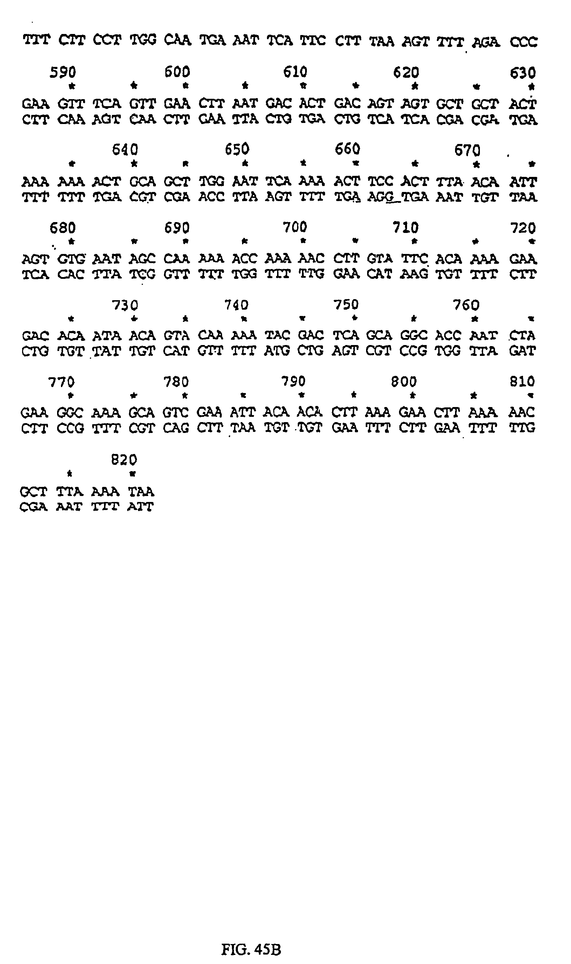

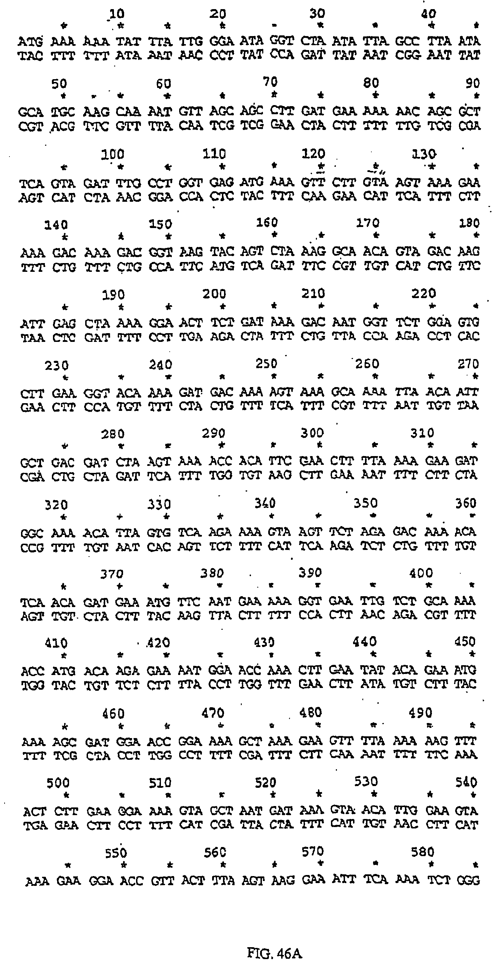

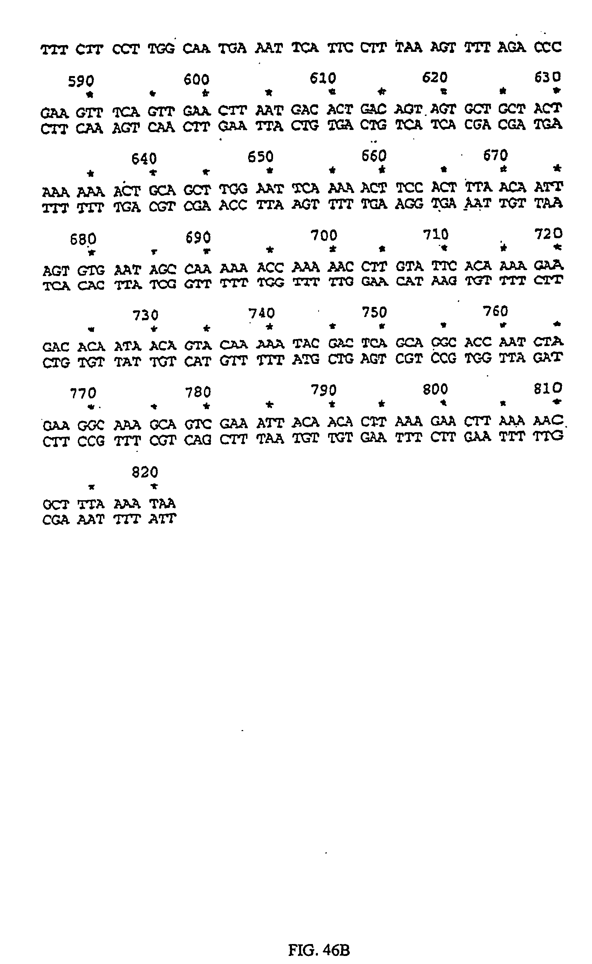

The OspA portion of the chimeric polypeptide can itself comprise OspA portions from two or more strains of Lyme Disease-causing *Borrelia* as described herein and provided, for example, in Figs. 23-29 and 43-46. In another embodiment, the OspA polypeptide comprises OspA portions from two or more genospecies of Lyme Disease-causing *Borrelia,* for example, wherein the genospecies are defined as *Borrelia burgdorferi sensu stricto, Borrelia afzelii* and *Borrelia garinii.* In this manner, the OspC and OspA polypeptide fragments that make up the chimeric protein can be from the same strain or genospecies of *Borrelia,* from different strains or genospecies of *Borrelia,* or from combinations of Proteins from the same and from different strains or genospecies of *Borrelia.*

The present invention is also drawn to nucleic acids which encode a *Borrelia* chimeric protein. The composition comprises a nucleic acid that encodes a chimeric protein of at least two polypeptides, wherein the first polypeptide comprises *Borrelia burgdorferi* OspC, and the second polypeptide comprises *Borrelia burgdorferi* OspA, such that OspC is upstream of OspA. The OspC and OspA nucleic acid fragments that make up the chimeric protein can be from the same strain or genospecies of *Borrelia,* from different strains or genospecies of *Borrelia,* or from combinations of proteins that are from the same and/or different strains or genospecies of *Borrelia.*

The present invention is also drawn to expression vectors which comprise an isolated DNA encoding a *Borrelia* chimeric protein. In one embodiment, the composition includes an expression vector comprising an isolated DNA which encodes an OspC/OspA chimeric protein as described herein. The present invention also encompasses host cells which comprise a recombinant nucleic acid encoding an OspC/OspA chimeric protein as described herein.

The present invention is also drawn to methods of making the *Borrelia* chimeric polypeptides described herein. In one embodiment, the method of making a chimeric *Borrelia* protein comprises selecting a polynucleotide sequence encoding OspC, or an antigenic portion thereof, selecting a polynucleotide sequence encoding OspA, or an antigenic portion thereof, and ligating these polynucleotide sequences together.

The present invention may also be used in methods of delivering the *Borrelia* chimeric polypeptides described herein. The method may comprise administering the chimeric protein in a physiologically-acceptable carrier to an individual. As a result of the administration of the chimeric protein, the individual develops at least some immune response to the chimeric protein, e.g., the individual generates a humoral immune response, wherein antibodies are produced by the individual that recognize at least a portion of said chimeric polypeptide.

The present invention may also be used in methods of delivering nucleic acids which encode the chimeric polypeptides described herein. The method comprises administering the nucleic acid in a physiologically-acceptable carrier to an individual. As a result of the administration of the nucleic acid, the individual expresses the chimeric protein at least transiently and develops at least some immune response to the chimeric protein encoded by the nucleic acid, e.g, the individual generates a humoral immune response, wherein antibodies that recognize at least a portion of the chimeric polypeptide produced from the nucleic acid, are produced by the individual.

The invention also encompasses methods of using the chimeric proteins described herein in a diagnostic assay. As described herein, the method can be used to detect the presence of OspA- and/or OspC-specific antibodies in a sample, e.g., a host sample of interest. The method comprises contacting a sample, e.g., a host sample of interest, with the chimeric protein, under conditions, wherein antibodies, if present in the host sample, bind to the chimeric protein thereby forming antigen-antibody complexes. The antigen-antibody complexes are then detected. In this manner, the chimeric proteins of the present invention can be used to detect an immune response to Lyme Disease causing *Borrelia.*

The present invention is also drawn to diagnostic kits which comprise the chimeric polypeptides described herein. The kit comprises a *Borrelia burgdorferi* OspC/OspA chimeric protein. The kit also includes reagents for detecting anybody-antigen complexes that are formed between the OspC/OspA chimeric protein and antibodies that are present in a sample, e.g., a user-supplied host sample.

The present invention is also drawn to pharmaceutical compositions which can be used to vaccinate and/or treat *Borrelia* infection in an animal or human. The pharmaceutical composition can be administered together with a physiologically-acceptable carrier and/or with suitable excipients and/or adjuvants.

The present invention may also be used in methods of immunizing an animal or human against Lyme disease. In a particular embodiment, the method comprises administering a *Borrelia* chimeric OspC/OspA protein. The chimeric protein can be administered together with a physiologically-acceptable carrier, a suitable excipient and/or a suitable adjuvant, to an animal or human such that the animal or human develops an immune response to at least one of the OspC and/or OspA polypeptides of the composition.

By incorporating polypeptide fragments from multiple *Borrelia burgdorferi* proteins, the present invention provides a composition that has great utility for vaccines and diagnostic kits. As a result of the present invention, there exist diagnostic tools and-vaccines that comprise both OspA and OspC antigens from various *Borrelia burgdorferi* strains and/or genospecies in a single protein. Since OspA is primarily expressed in the tick vector, and OspC is upregulated in response to the feeding of an infected tick on a mammal, this allows for a diagnostic tool or vaccine that can recognize antigens that are expressed at different stages of the life cycle of *Borrelia burgdorferi.* Thus, the chimeric proteins of the present invention can act at the level of the tick as well as the level of the host, in preventing infection and/or disease caused by *Borrelia.* Moreover, by incorporating unique polypeptide fragments from pathogenic families of *Borrelia,* such as *Borrelia burgdorferi sensu stricto, Borrelia afzelii* and *Borrelia garinii,* an improved diagnostic tool or vaccine is produced which can detect clinically important exposure to a wider variety of pathogenic *Borrelia,* while overlooking the remainder of non-pathogenic families of *Borrelia.* Furthermore, OspC polypeptides can be selected from strains of *Barrelia* that are associated with disseminated disease, as described in WO 00/78966, the teachings of which are incorporated herein in their entirety.

The present invention also provides a combination of *Borrelia* antigens in a single polypeptide that, when used as a vaccine, are expected to prevent Lyme disease from becoming systemic. The chimeric proteins of the present invention can be effective in preventing Lyme disease, as well as having a therapeutic affect on established infection, for example after the tick bite is noticed by the patient.

The present invention is drawn to unlipidated chimeric proteins, Unlipidated chimeric proteins, such as the OspC/OspA chimeric proteins described herein, have certain advantages over their lipidated counterparts. These advantages include simpler production methods, improved yields of protein and simpler purification methods. While the lack of a lipidation signal provides several advantages, it had been thought that the lipidation signal was required for immunogenicity. However, as described herein, the non-lipidated OspC/OspA chimeric proteins of the present invention elicit an immune response that is at least as broadly reactive as that of lipidated OspA and lipidated OspC control proteins. Moreover, the unlipidated OspC/OspA chimeric proteins of the present invention unexpectedly elicit an immune response to more than one genospecies and/or strain of Lyme disease-causing *Borrelia,* including genospecies and/or strains that were not used to generate the particular chimeric OspC/OspA immunogen.

For a better understanding of the present invention together with other and further objects, reference is made to the following description, taken together with the accompanying drawings.

### BRIEF DESCRIPTION OF THE DRAWINGS

Fig. 1 summarizes peptides and antigenic domains localized by proteolytic and chemical fragmentation of OspA.
Fig. 2 is a comparison of the antigenic domains depicted in Fig. 1, for OspA in nine strains of *B. burgdorferi.*
Fig. 3 is a graph depicting a plot of weighted polymorphism versus amino acid position among 14 OspA variants. The marked peaks are: a) amino acids 132-145; b) amino acids 163-177; c) amino acids 208-221. The lower line at polymorphism value 1.395 demarcates statistically significant excesses of polymorphism at p = 0.05. The upper line at polymorphism value 1.520 is the same, except that the first 29 amino acids at the monomorphic N-terminus have been removed from the original analysis.
Fig. 4 depicts the amino acid alignment of residues 200 through 220 for OspAs from strains B31 and K48 as well as for the site-directed mutants 613, 625, 640, 613/625, and 613/640. The arrow indicates Trp216. Amino acid changes are underlined.
Fig. 5 is a helical wheel projection of residues 204-217 of B31 OspA. Capital letters indicate hydrophobic residues; lower case letters indicate hydrophilic residues; + and - indicate positively and negatively charged residues, respectively. The dashed line indicates division of the alpha-helix into a hydrophobic arc (above the line) and a polar arc (below the line). Adapted from France et al. (Biochem. Biophys. Acta 1120: 59 (1992)).

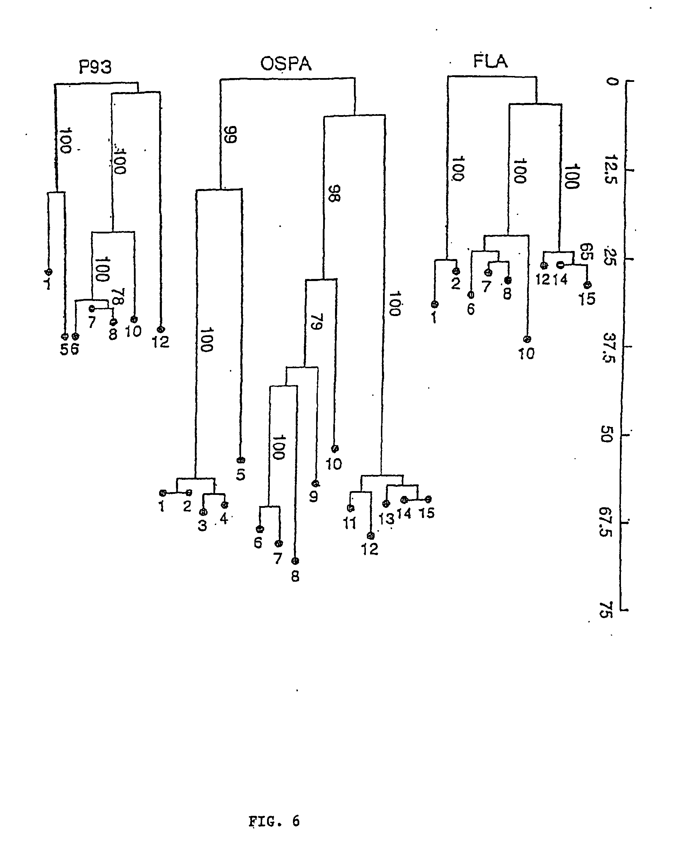

Fig. 6 depicts a phylogenic tree for strains *of Borrelia* described in Table I. The strains are as follows: 1 = B31; 2 = PKa1; 3 = ZS7; 4 = N40; 5 = 25015; 6 = K48; 7 = DK29; 8 = PHei; 9 = Ip90; 10 = PTrob; 11 = ACAI; 12 = PGau; 13 = Ip3; 14 = PBo; 15 = PKo.
Figs. 7A and 7B depict the nucleic acid sequence of OspA-B31 (SEQ ID NO. 6), and the encoded protein sequence (SEQ ID NO. 7).

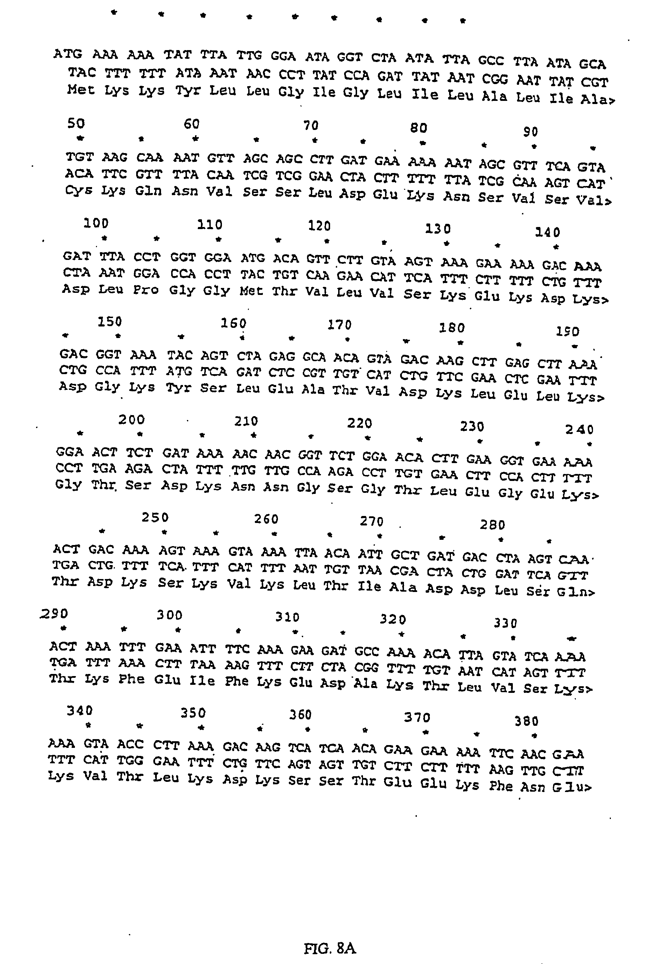

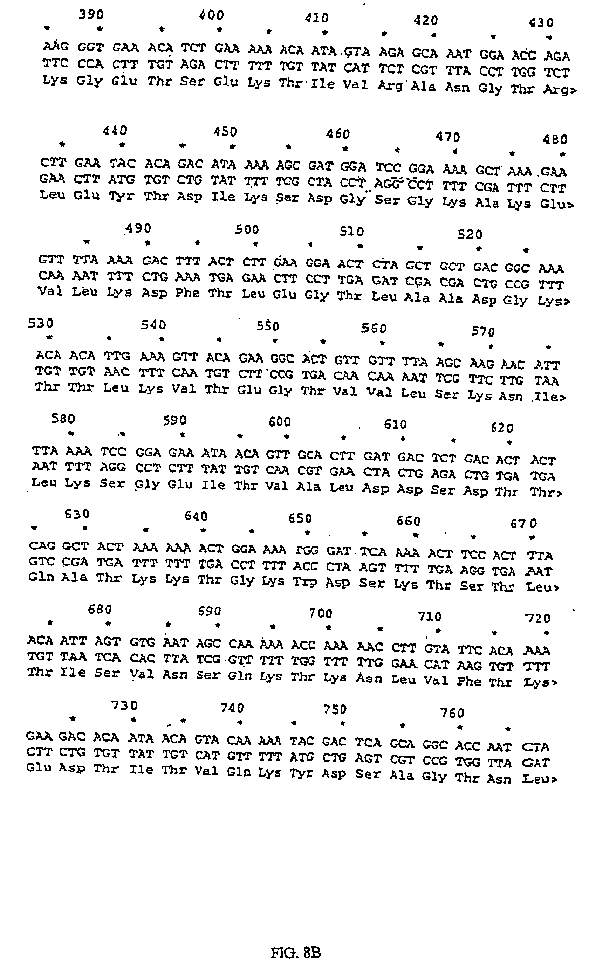

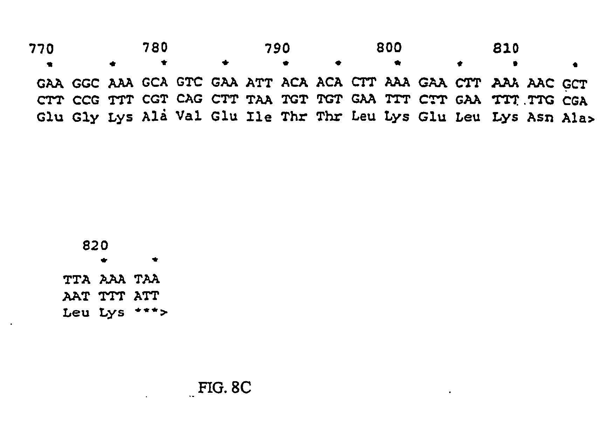

Figs. 8A, 8B and 8C depict the nucleic acid sequence of OspA-K48 (SEQ ID NO. 8), and the encoded protein sequence (SEQ ID NO. 9).

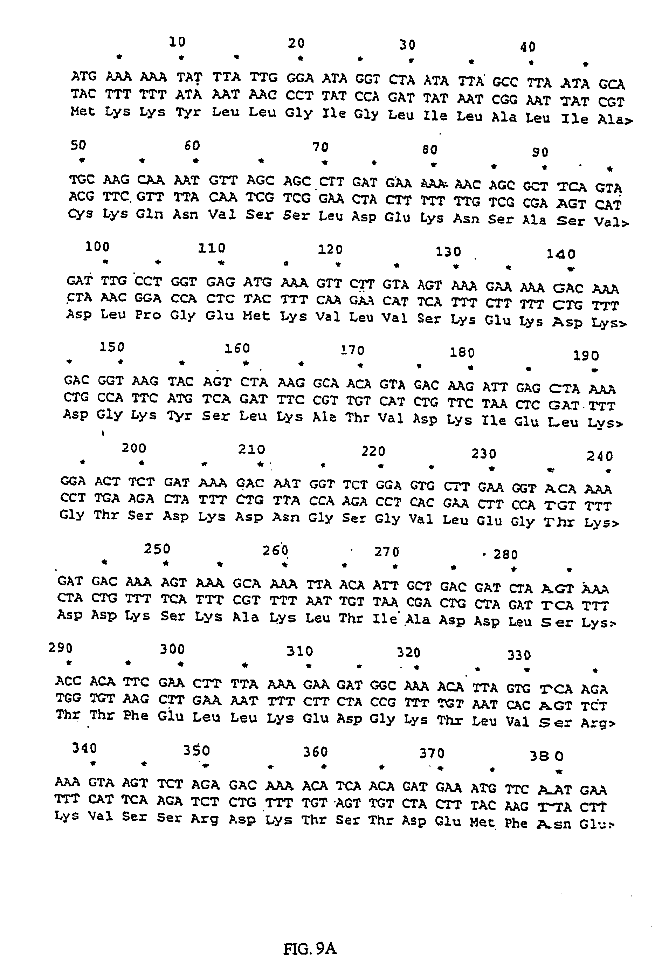

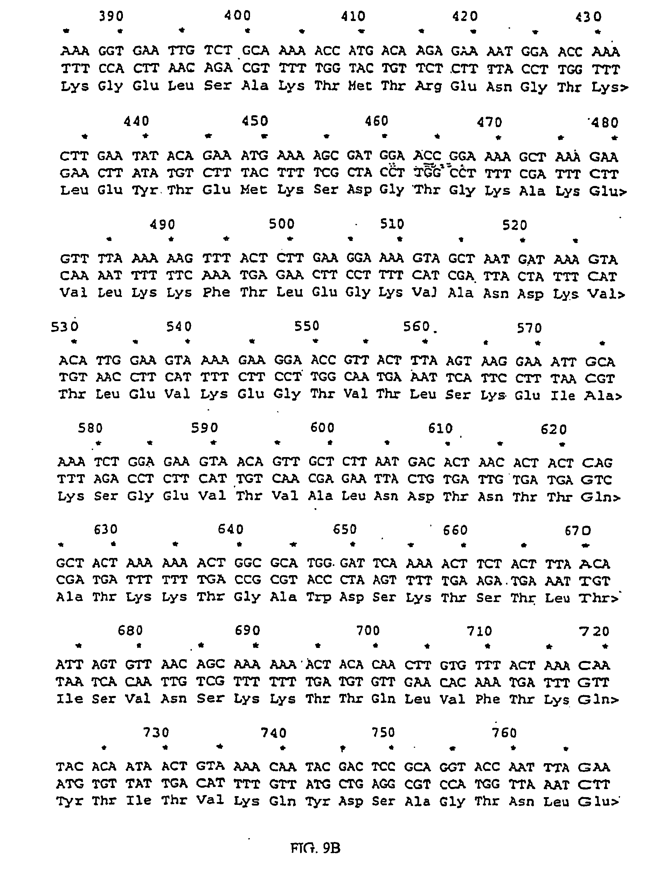

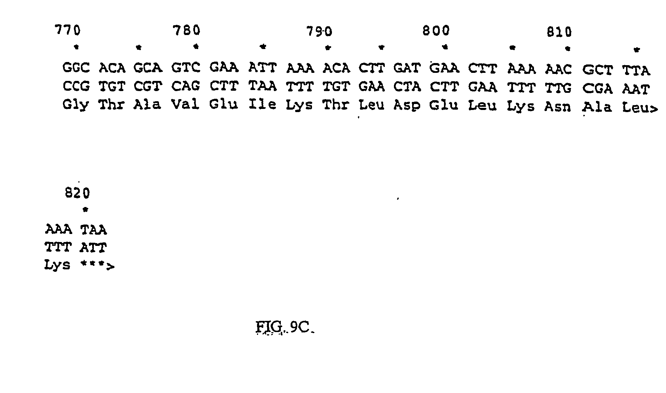

Figs. 9A, 9B and 9C depict the nucleic acid sequence of OspA-PGau (SEQ ID NO. 10), and the encoded protein sequence (SEQ ID NO. 11).

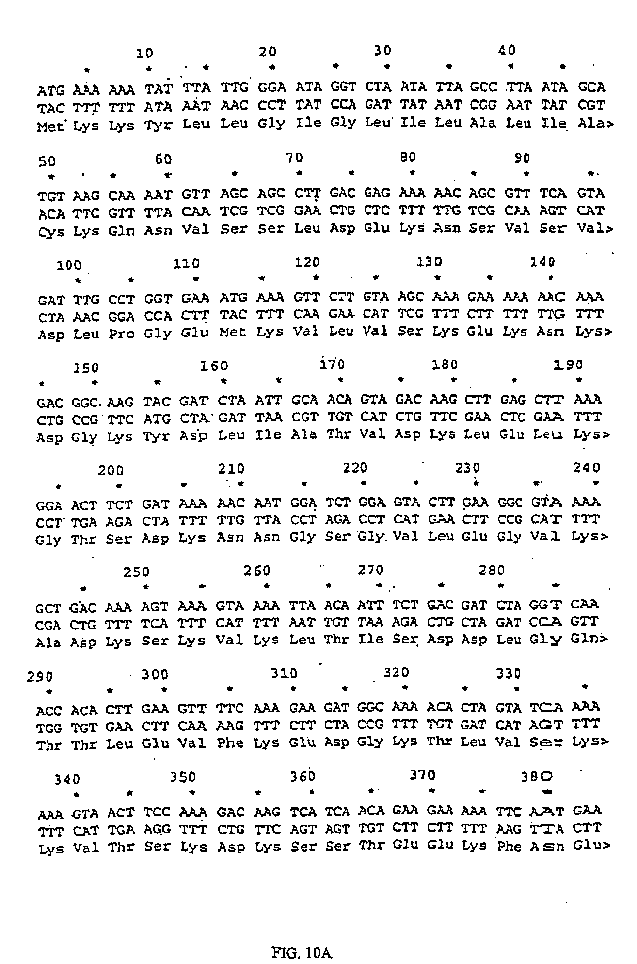

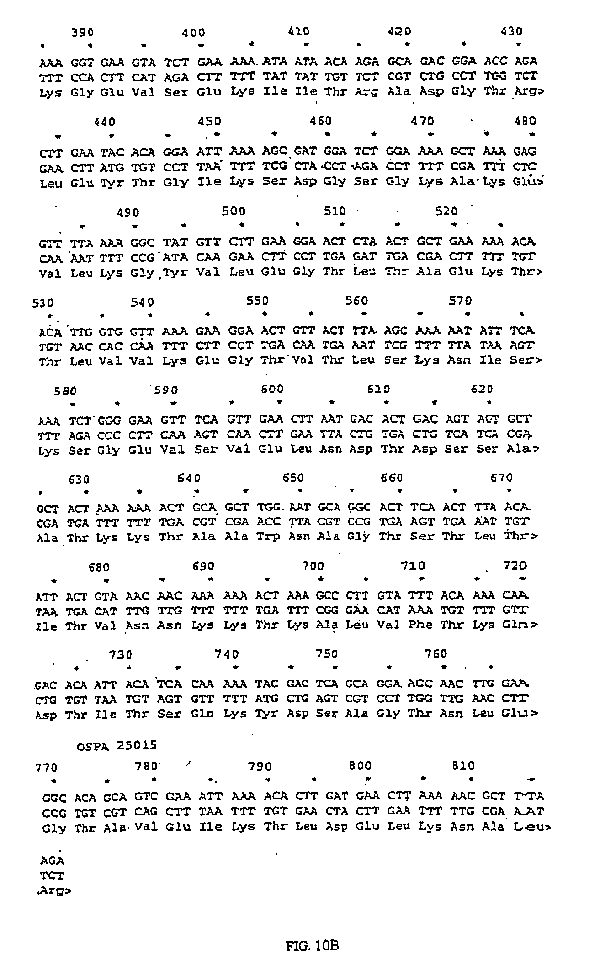

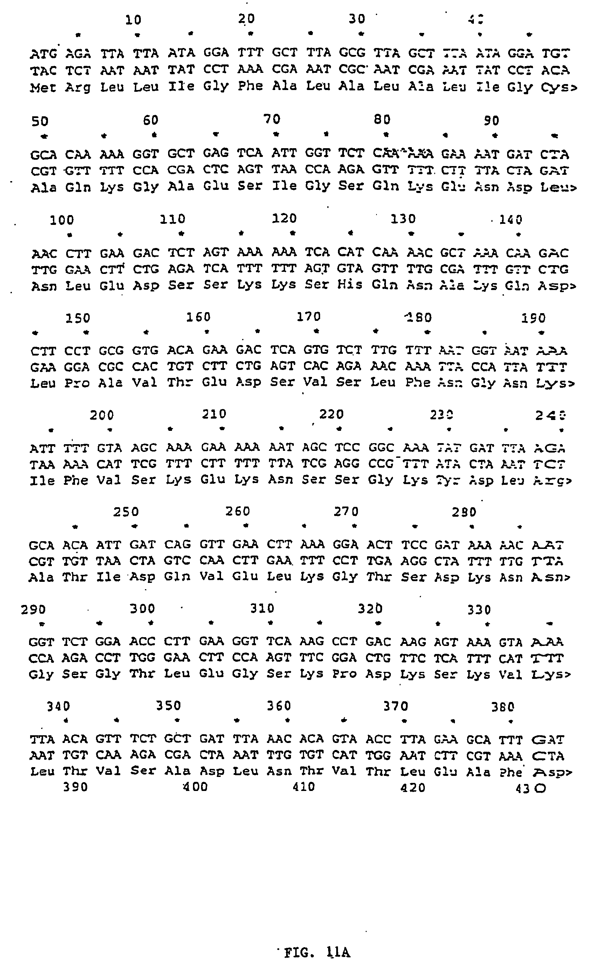

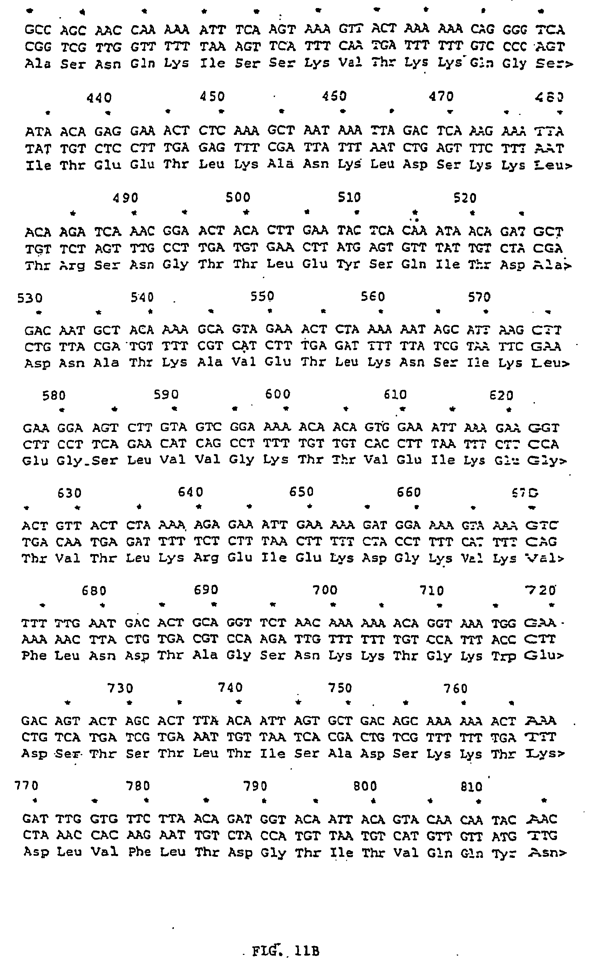

Figs. 10A and 10B depict the nucleic acid sequence of a portion of an OspA gene (SEQ ID NO. 185) and its encoded protein sequence (SEQ ID NO. 186).

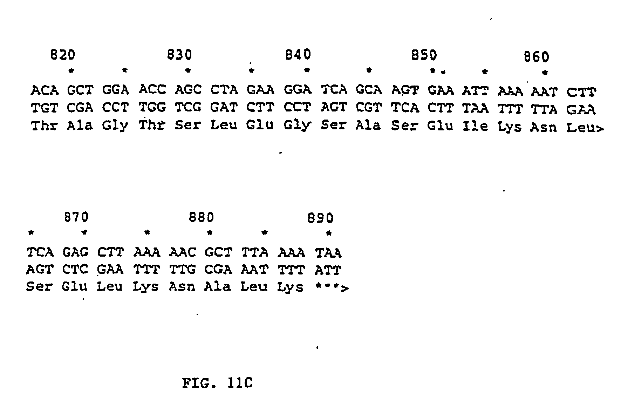

Figs. 11A, 11B and 11C depict the nucleic acid sequence of OspB-B31 (SEQ ID NO. 21), and the encoded protein sequence (SEQ ID NO. 22).
Figs. 12A and 12B depict the nucleic acid sequence of OspC-B31 (SEQ ID NO. 29), and the encoded protein sequence (SEQ ID NO. 30).

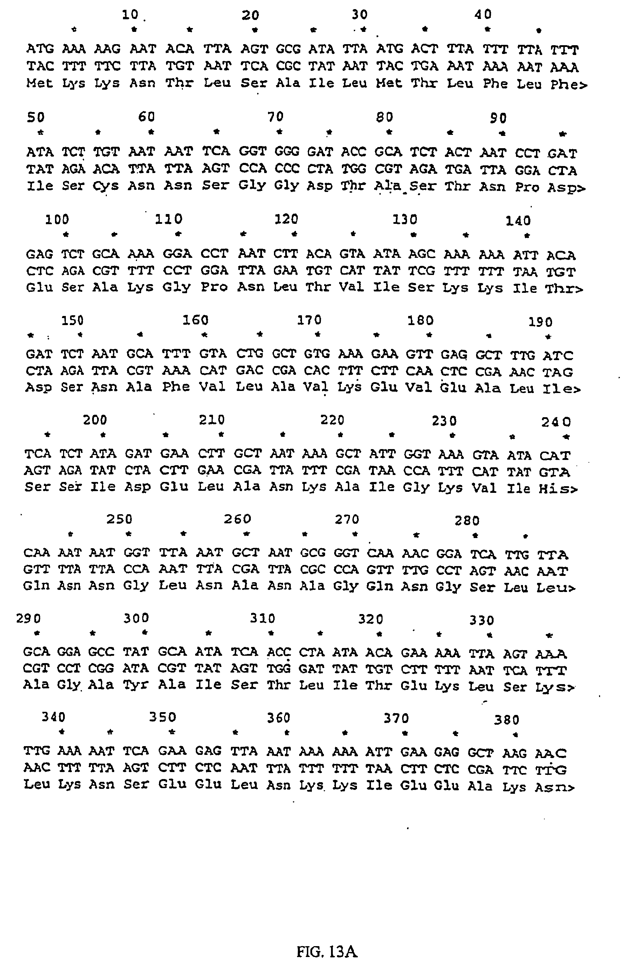

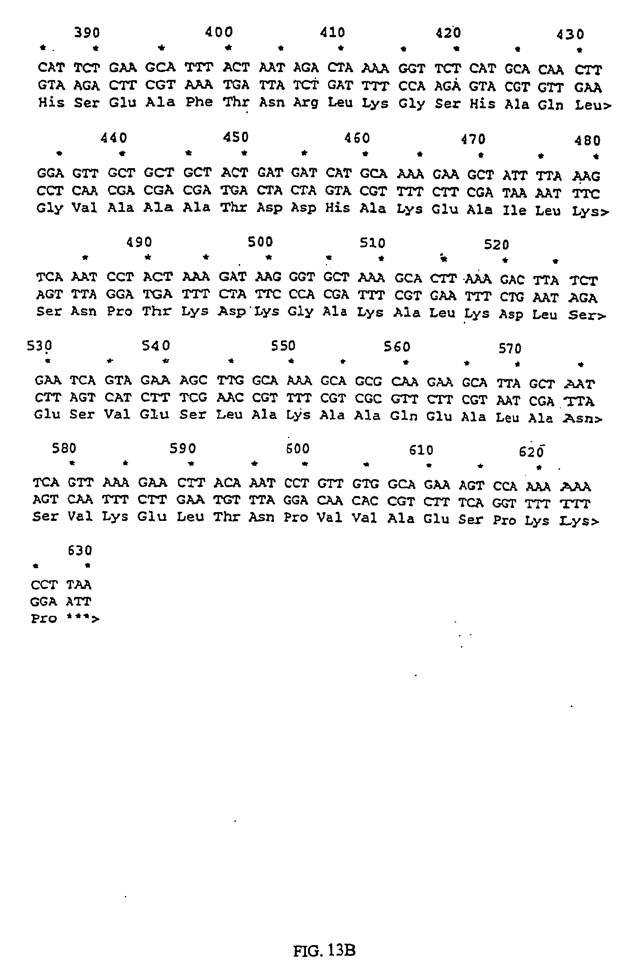

Figs. 13A and 13B depict the nucleic acid sequence of OspC-K48 (SEQ ID NO. 31), and the encoded protein sequence (SEQ ID NO. 32).

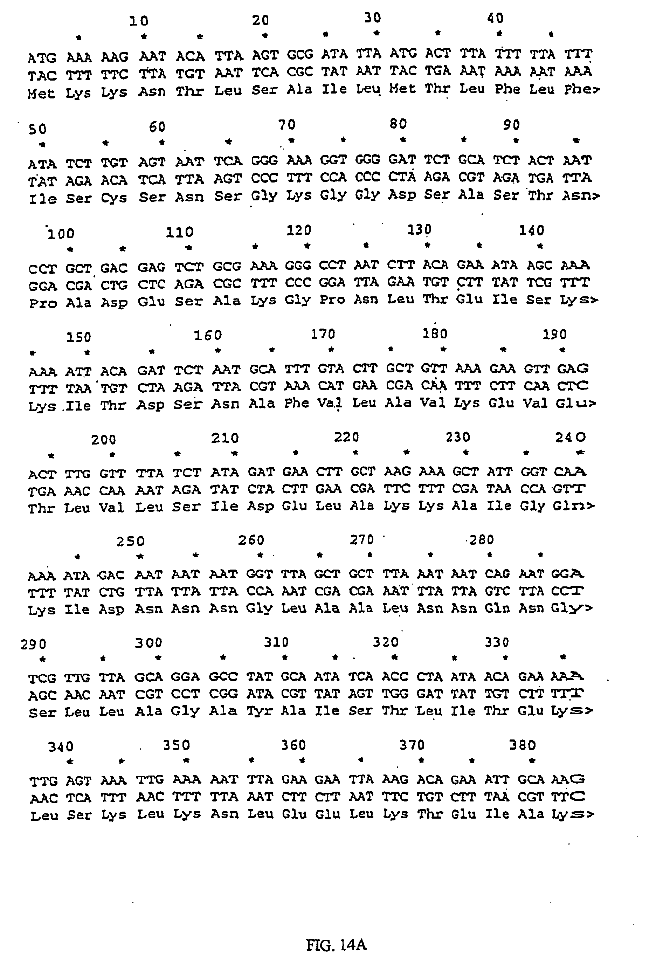

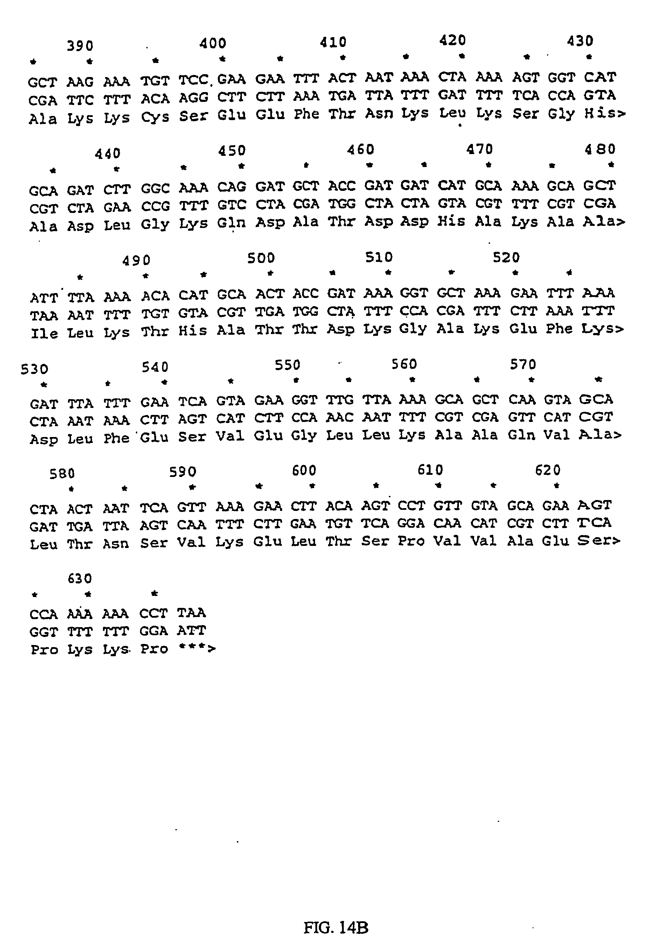

Figs. 14A and 14B depict the nucleic acid sequence of OspC-PKo (SEQ ID NO. 33), and the encoded protein sequence (SEQ ID NO. 34).

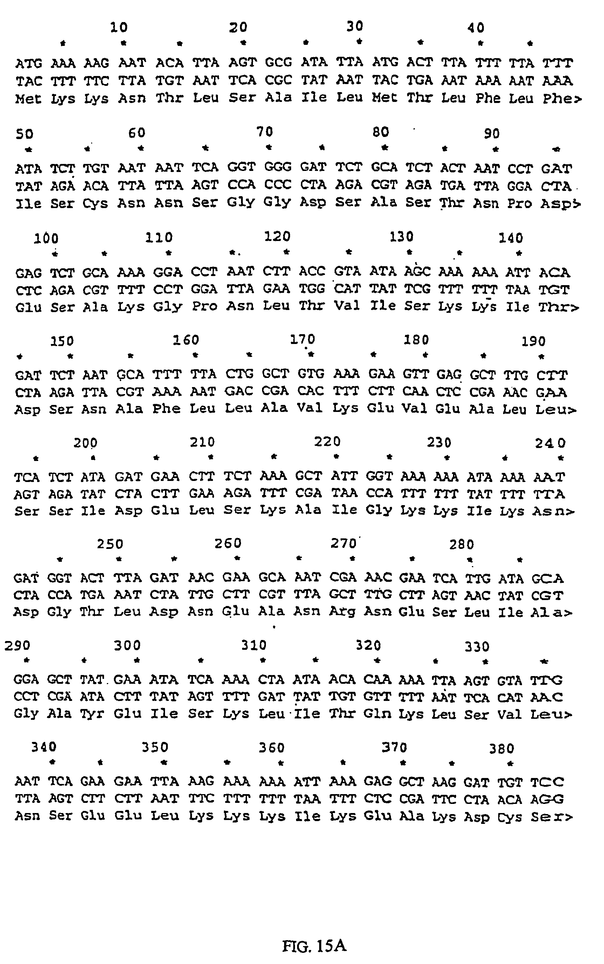

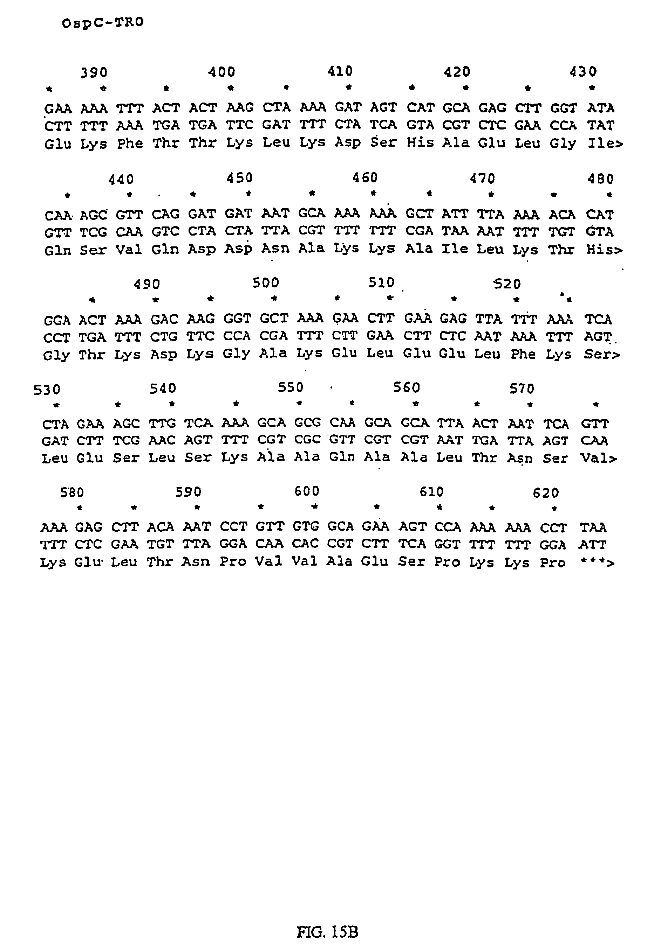

Figs. 15A and 15B depict the nucleic acid sequence of OspC-PTrob (SEQ ID NO. 35) and the encoded protein sequence (SEQ ID NO. 36).

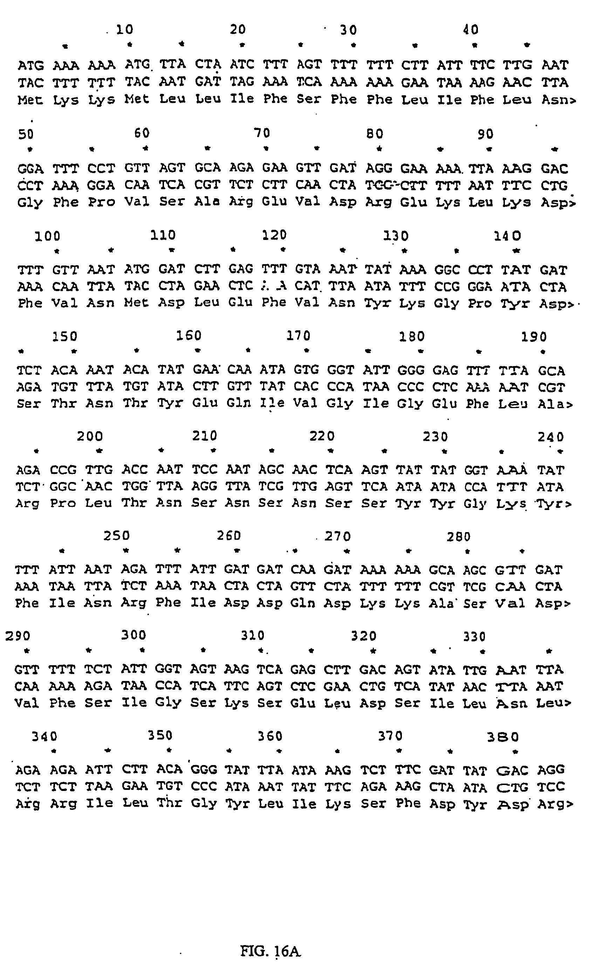

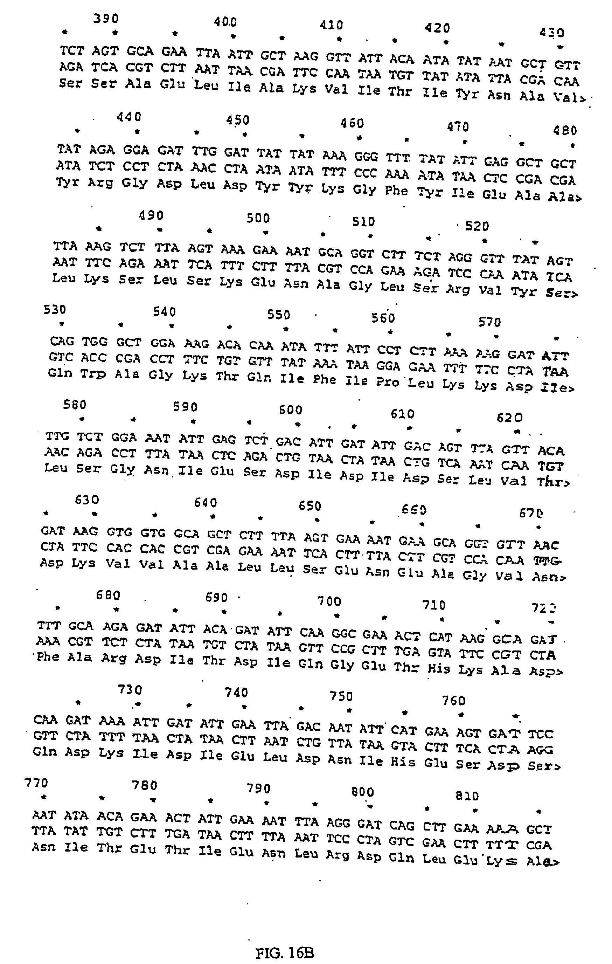

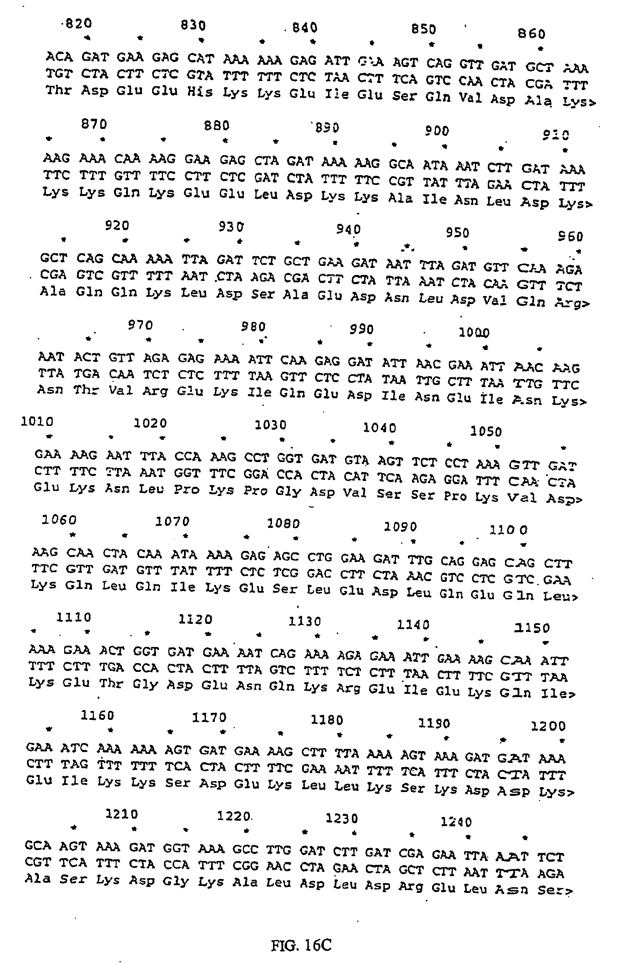

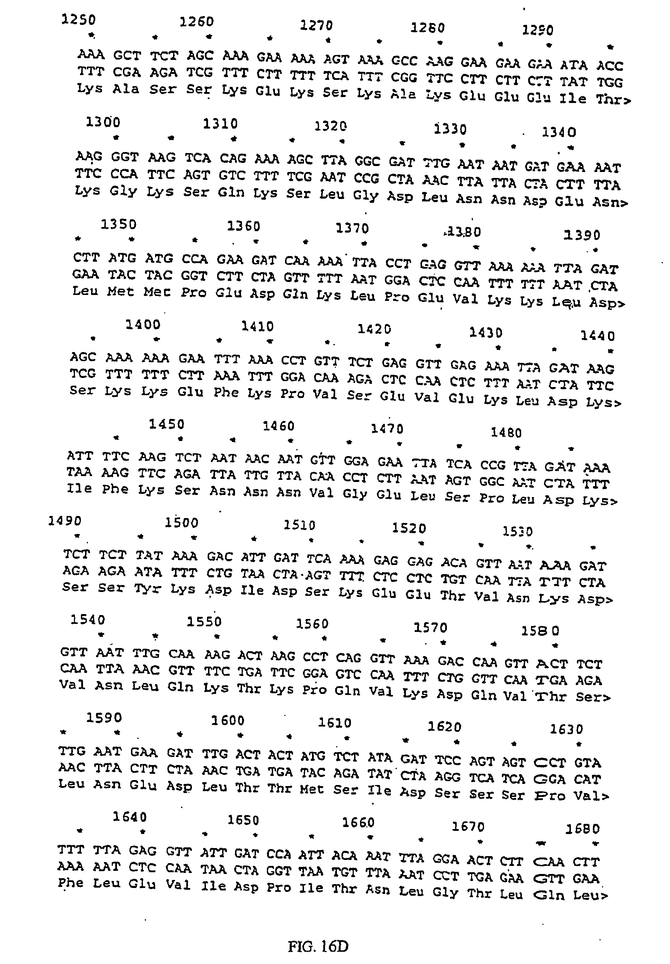

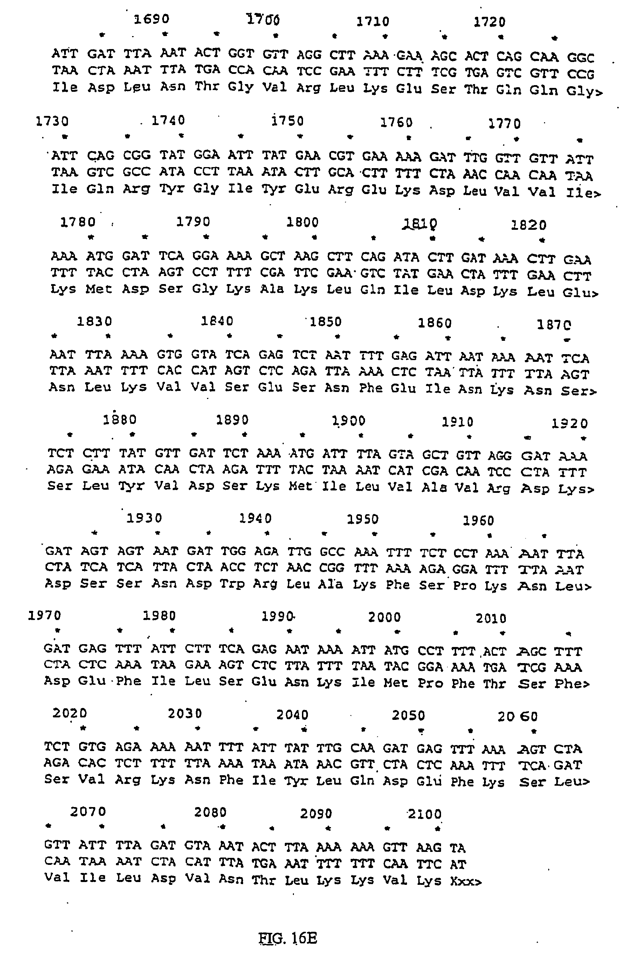

Figs. 16A, 16B, 16C, 16D and 16E depict the nucleic acid sequence of p93-B31 (SEQ ID NO. 65) and the encoded protein sequence (SEQ ID NO. 66).

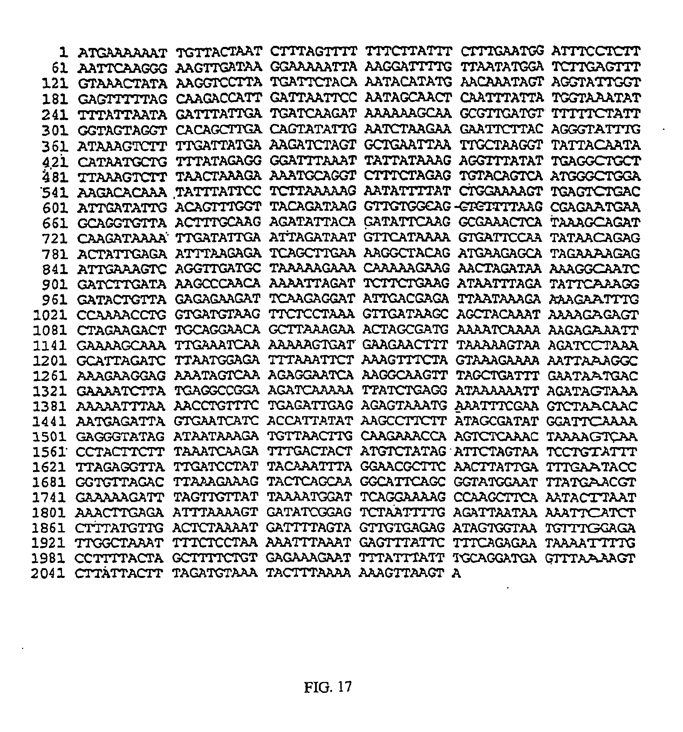

Fig. 17 depicts the nucleic acid sequence of p93-K48 (SEQ ID NO. 67).

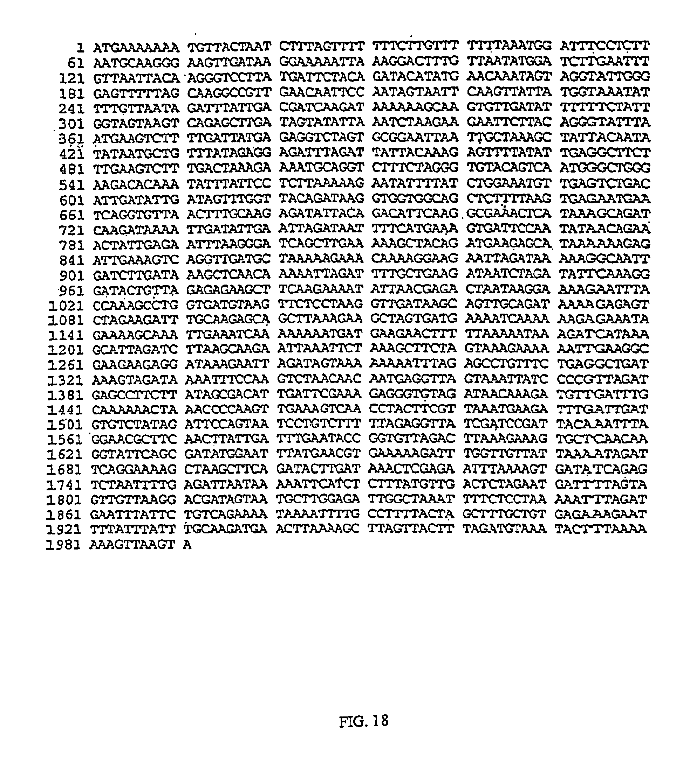

Fig. 18 depicts the nucleic acid sequence of p93-PBo (SEQ ID NO. 69).

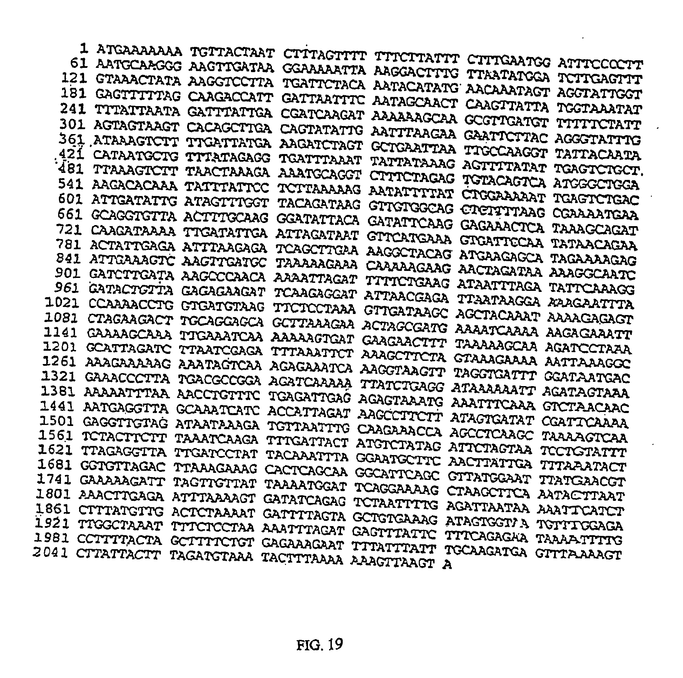

Fig. 19 depicts the nucleic acid sequence of p93-PTrob (SEQ ID NO. 71).

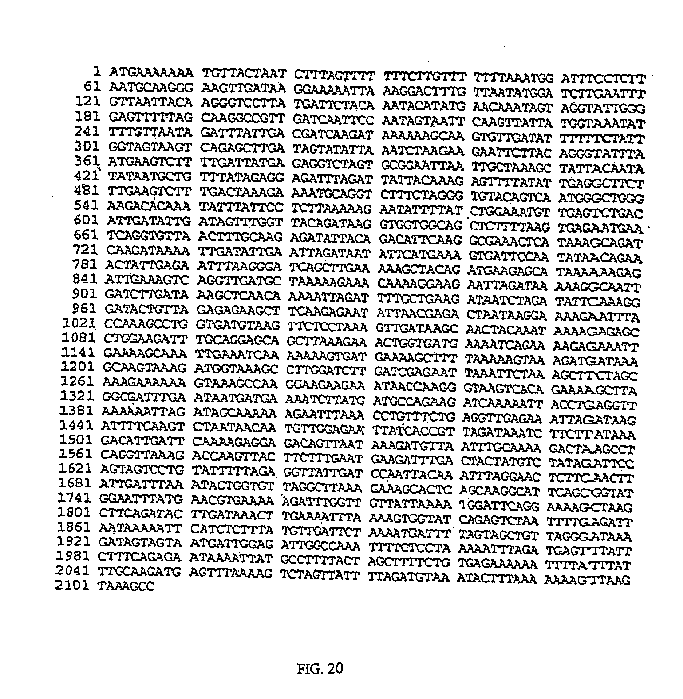

Fig. 20 depicts the nucleic acid sequence of p93-PGAU (SEQ ID NO. 73).

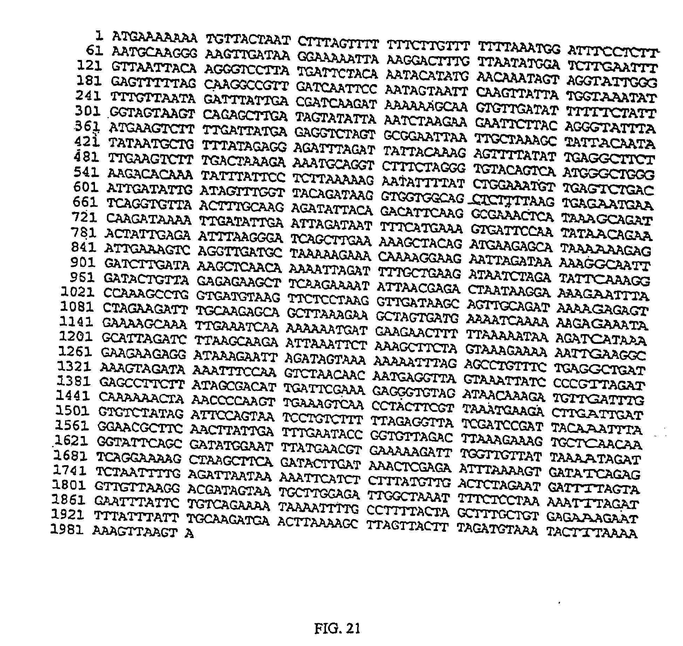

Fig. 21 depicts the nucleic acid sequence of p93-25015 (SEQ ID NO. 77).

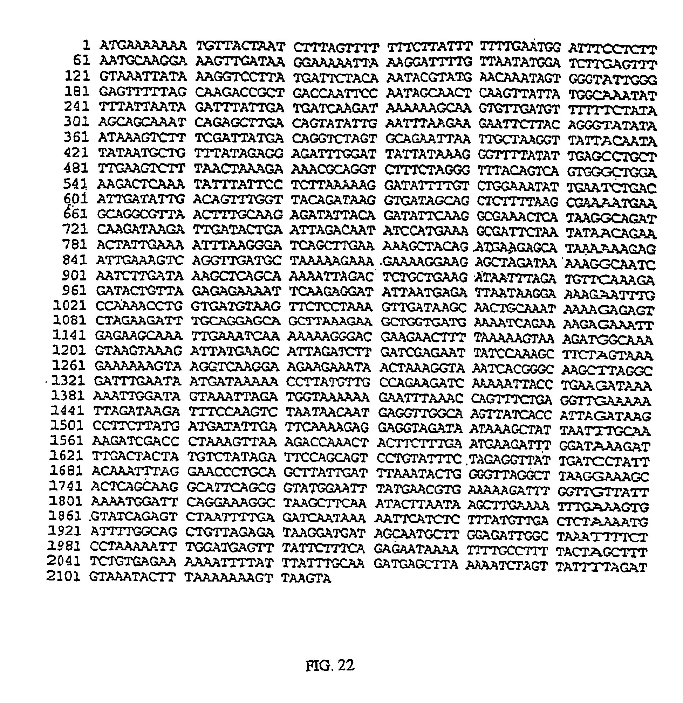

Fig. 22 depicts the nucleic acid sequence of p93-PKo (SEQ ID NO. 75).
Figs. 23A, 23B and 23C depict the nucleic acid sequence of the OspA-K48/OspA-PGAU chimer (SEQ ID NO. 85) and the encoded chimeric protein sequence (SEQ ID NO. 86).
Figs. 24A, 24B and 24C depict the nucleic acid sequence of the OspA-B31/OspA-PGAU chimer (SEQ ID NO. 88) and the encoded chimeric protein sequence (SEQ ID NO. 89).
Figs. 25A and 25B depict the nucleic acid sequence of the OspA-B31/OspA-K48 chimer (SEQ ID NO. 91) and the encoded chimeric protein sequence (SEQ ID NO. 92).
Figs. 26A, 26B and 26C depict the nucleic acid sequence of the OspA-B31/OspA-2501.5 chimer (SEQ ID NO. 94) and the encoded chimeric protein sequence (SEQ ID NO. 95).
Figs. 27A, 27B and 27C depict the nucleic acid sequence of the OspA-K48/OspA-B31/OspA-K48 chimer (SEQ ID NO. 97) and the encoded chimeric protein sequence (SEQ ID NO. 98).
Figs. 28A, 28B and 28C depict the nucleic acid sequence of the OspA-B31/OspA-K48/OspA-B31/OspA-K48 chimer (SEQ ID NO. 100) and the encoded chimeric protein sequence (SEQ ID NO. 101).
Figs. 29A, 29B and 29C depict the nucleic acid sequence of the OspA B31/OspB-B31 chimer (SEQ ID NO. 103) and the encoded chimeric protein sequence (SEQ ID NO. 104).

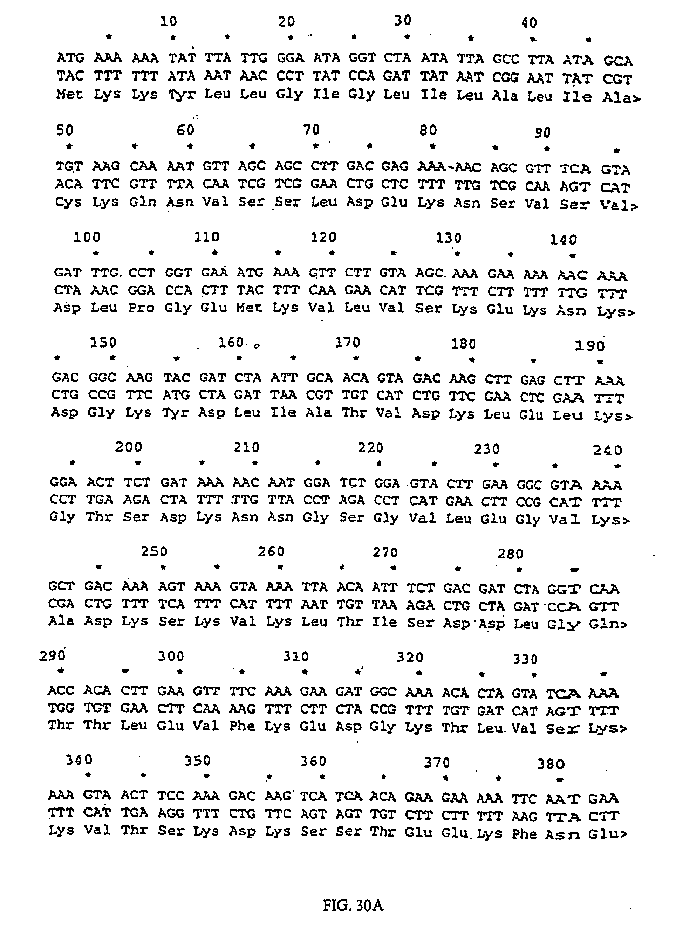

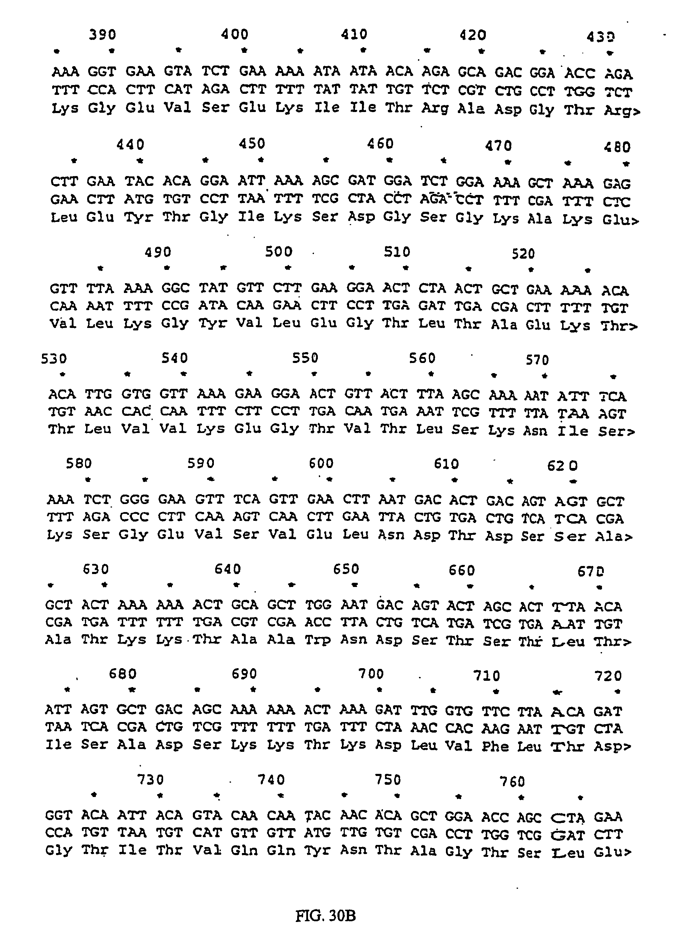

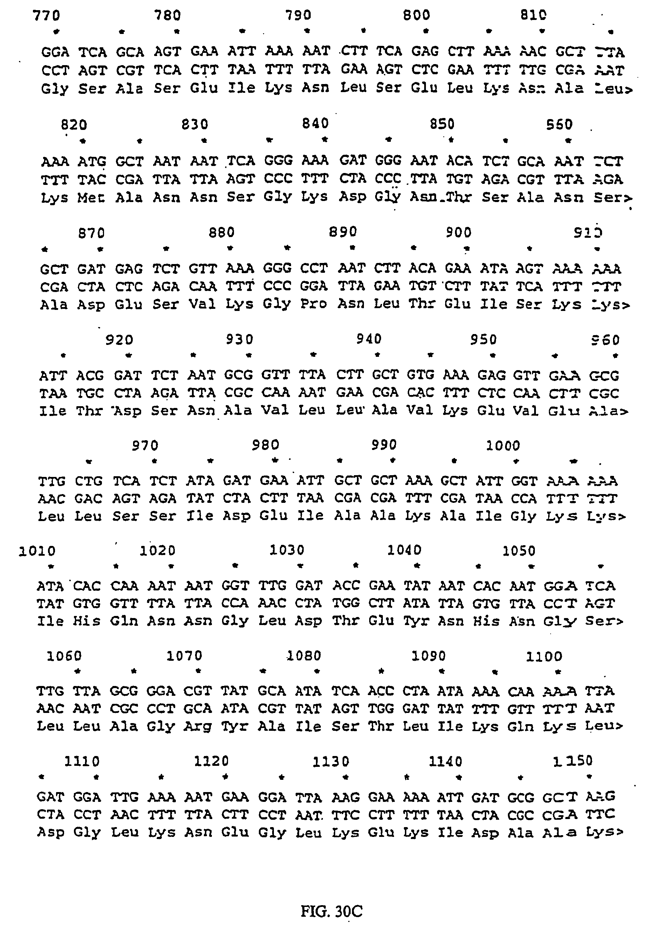

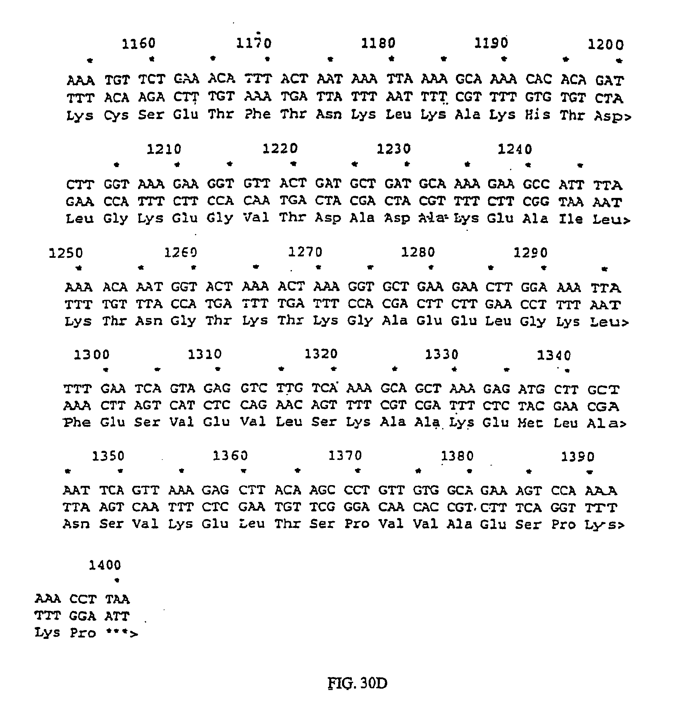

Figs. 30A, 30B, 30C and 30D depict the nucleic acid sequence of the OspA-B31/OspB-B31/OspC-B31 chimer (SEQ ID NO. 106) and the encoded chimeric protein sequence (SEQ ID NO. 107).

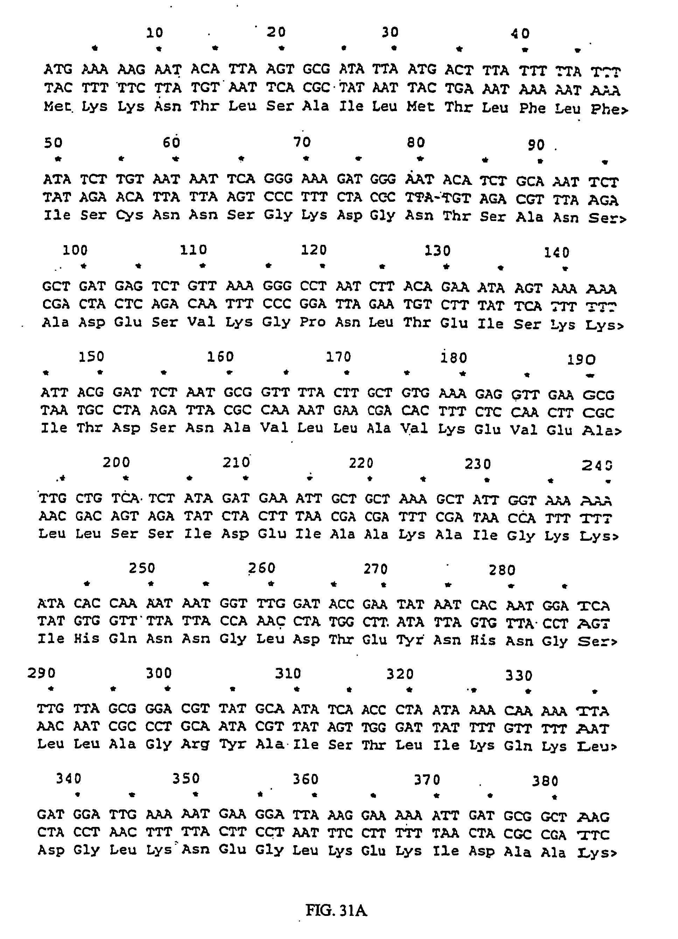

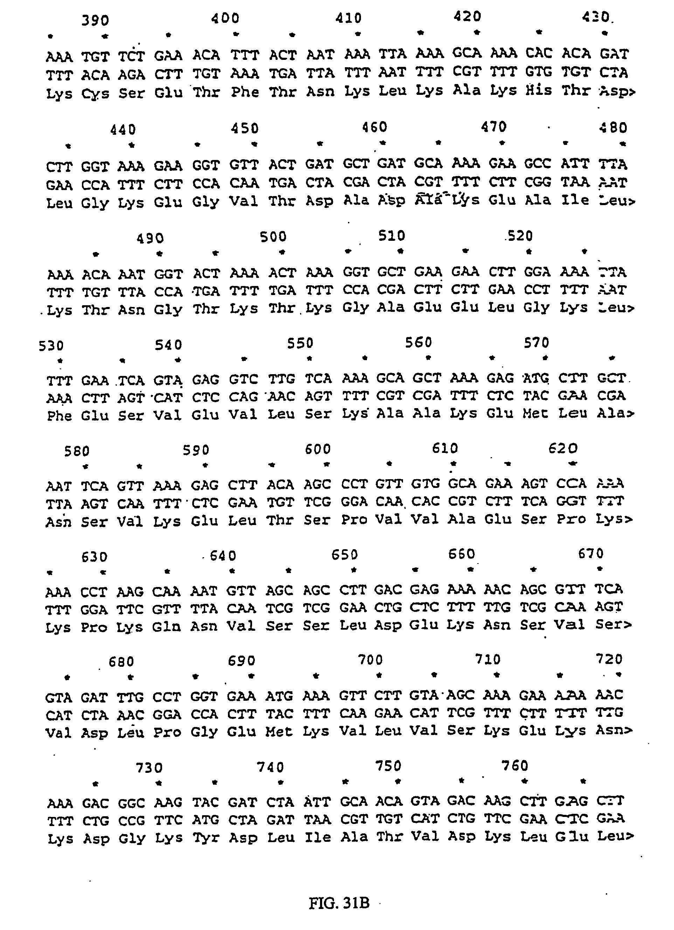

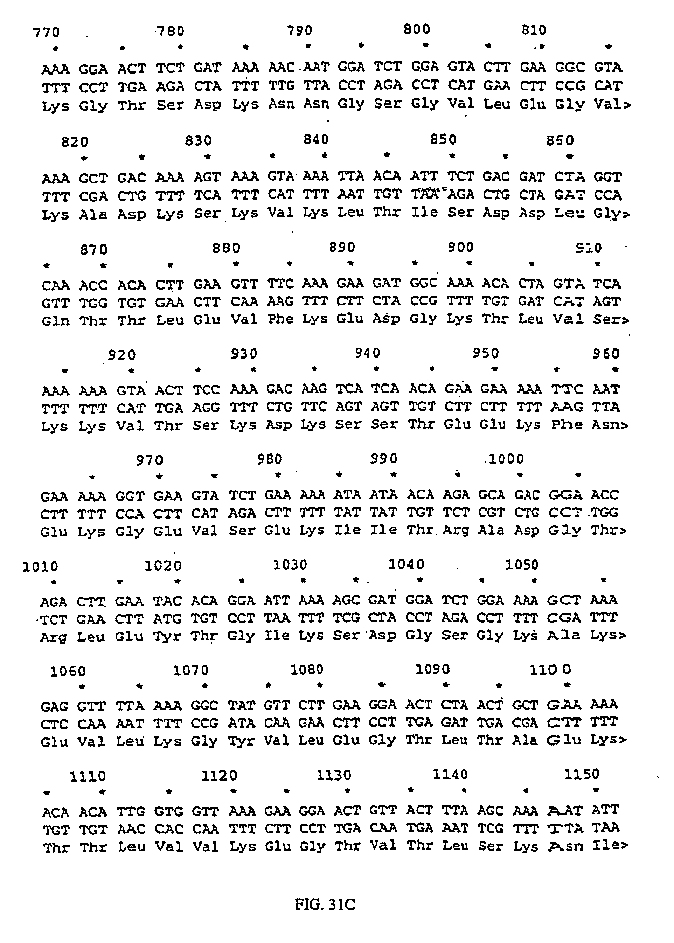

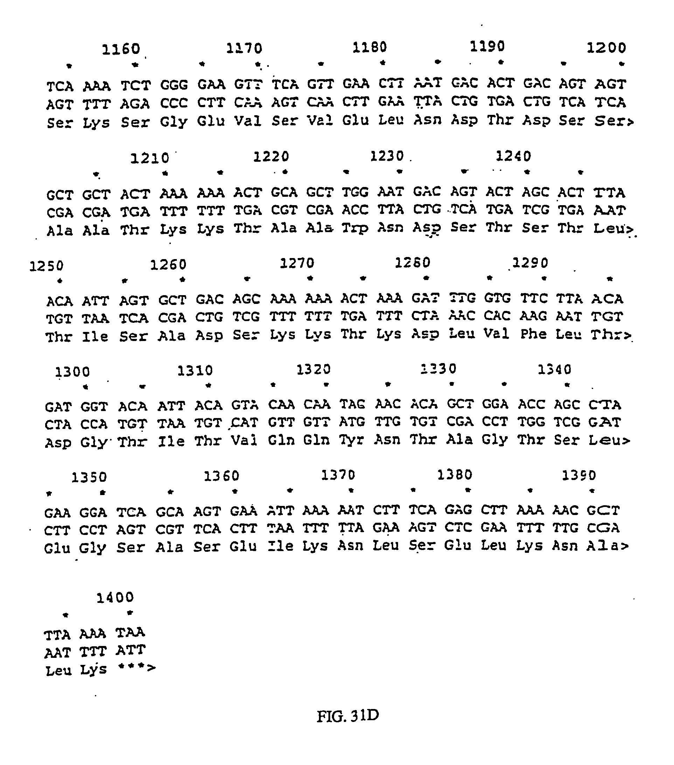

Figs. 31A, 31B, 31C and 31D depict the nucleic acid sequence of the OspC-B31/OspA-B31/OspB-B31 chimer (SEQ ID NO. 109) and the encoded chimeric protein sequence (SEQ ID NO. 110).

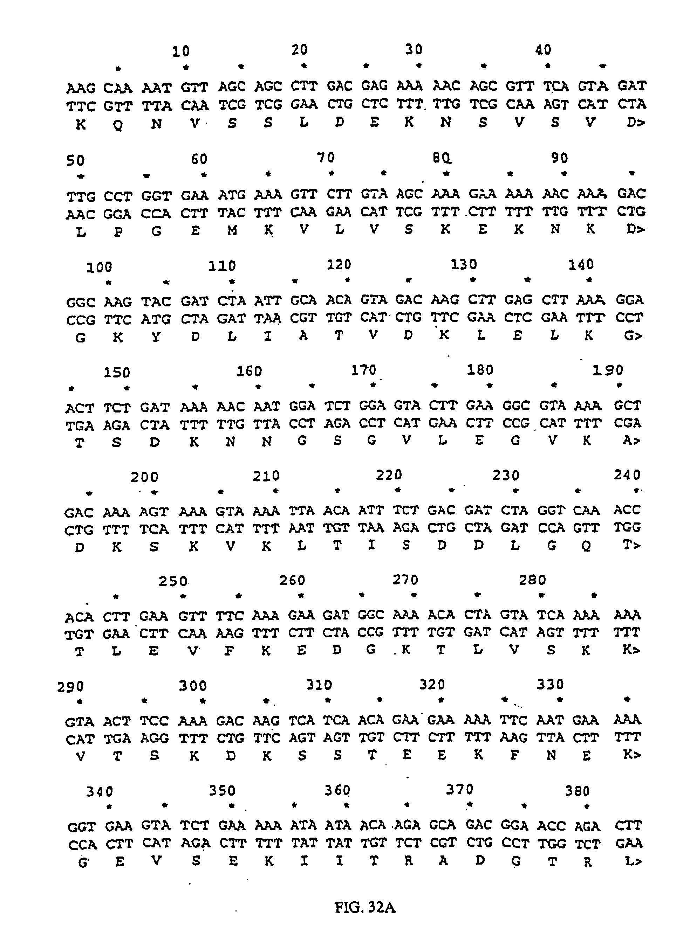

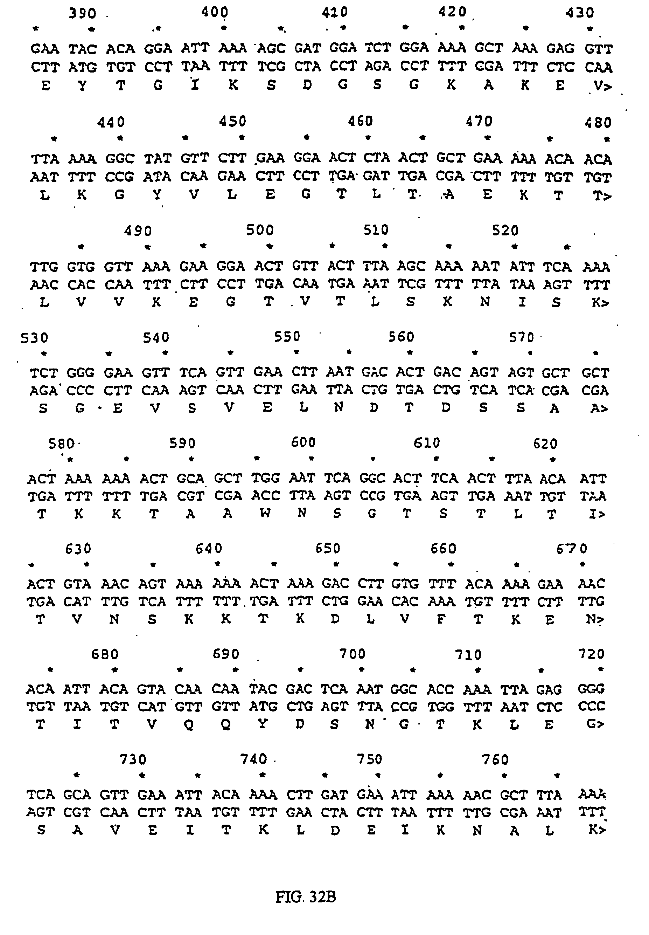

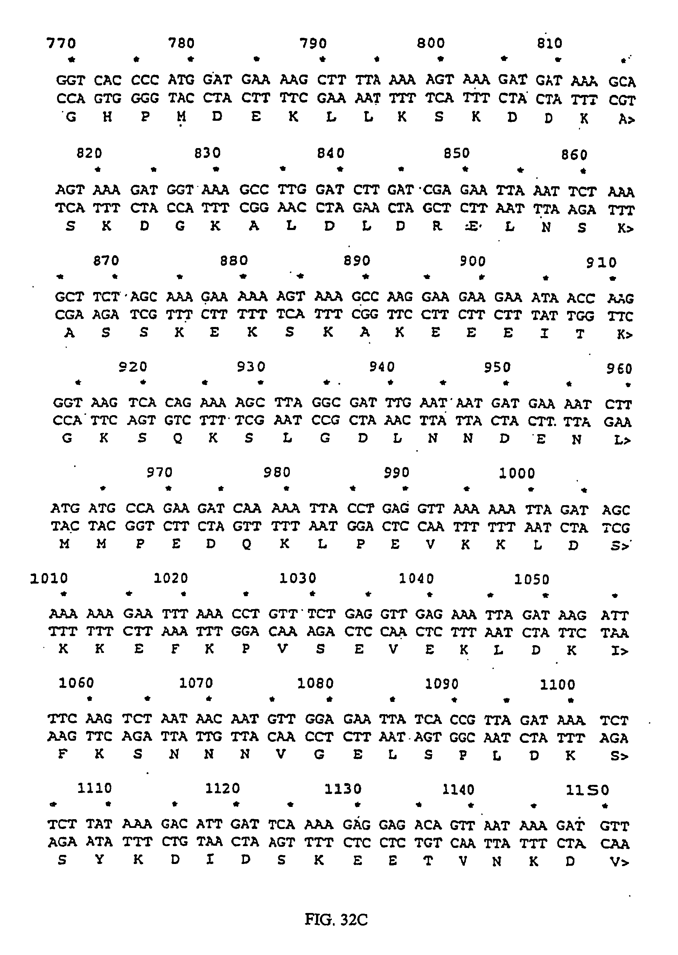

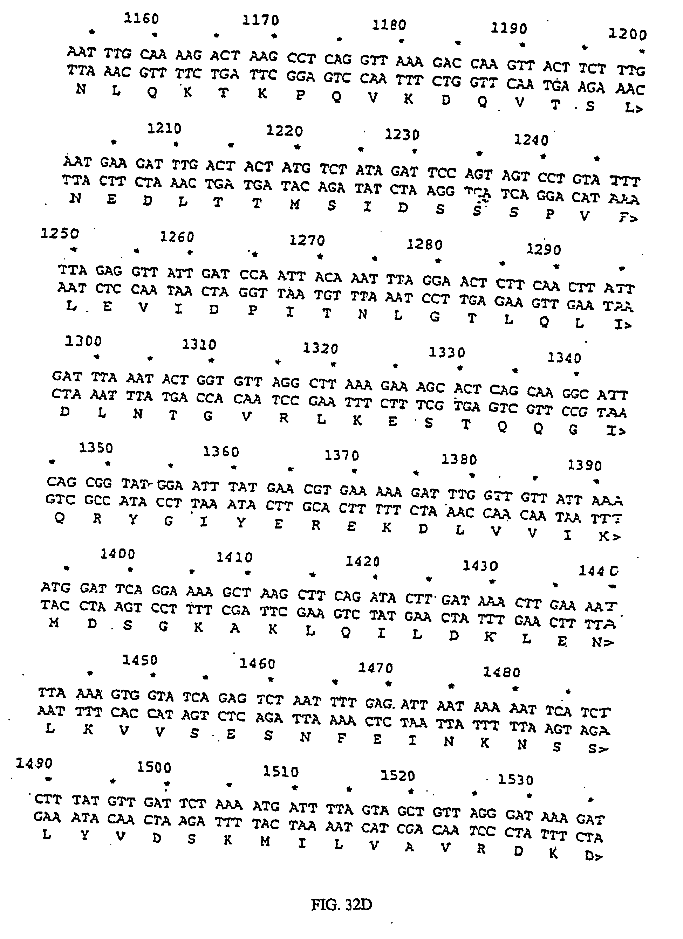

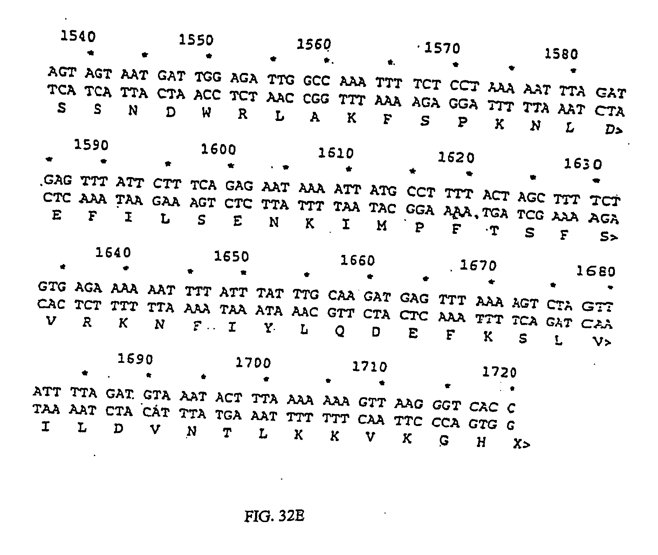

Figs. 32A, 32B, 32C, 32D and 32E depict the nucleic acid sequence of the OspA-B311p93-B31 chimer (SEQ ID NO. 111) and the encoded chimeric protein sequence (SEQ ID NO. 112).

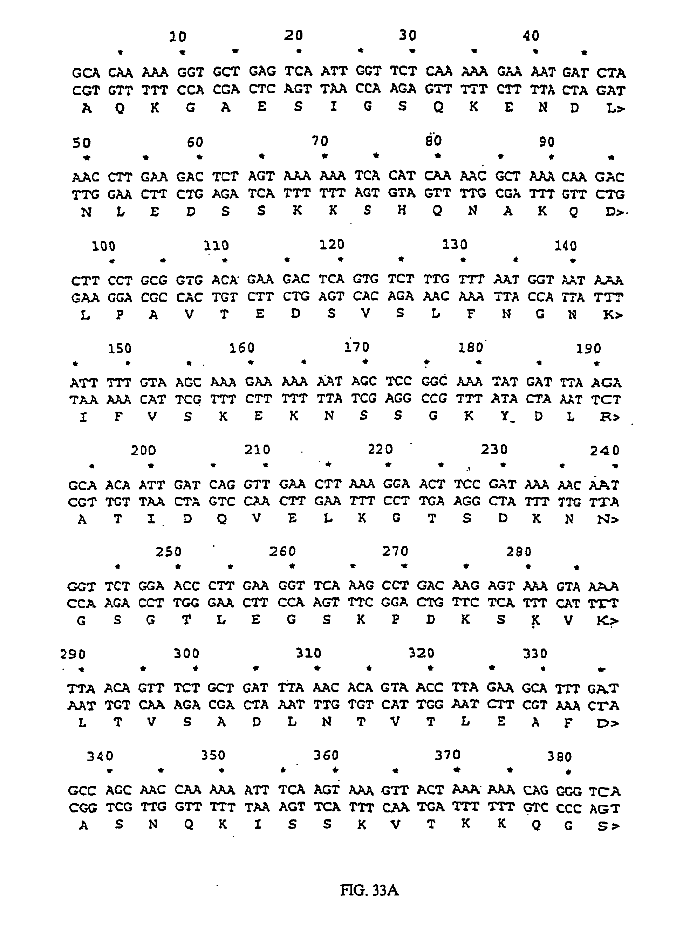

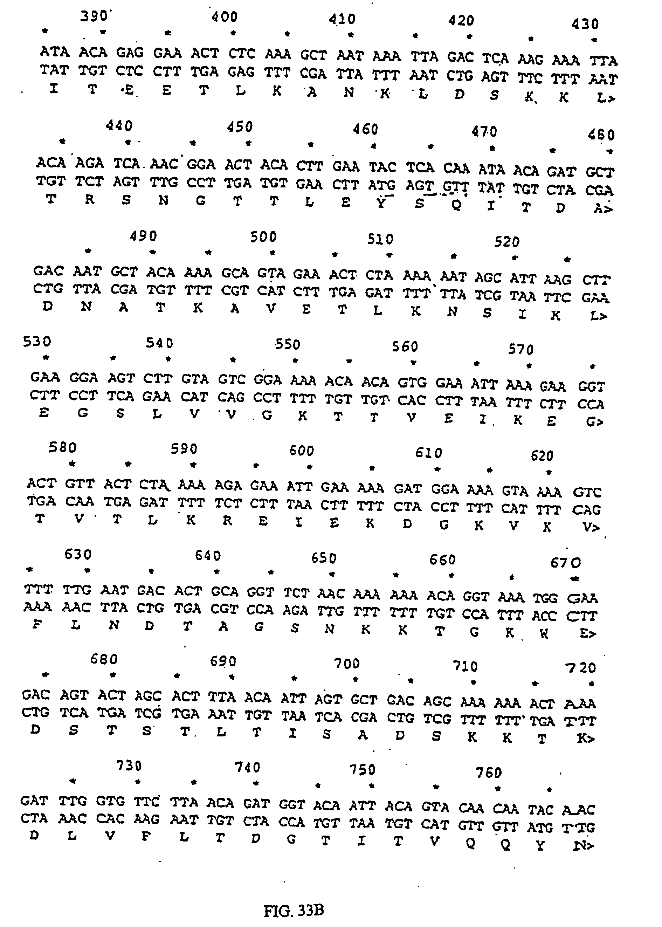

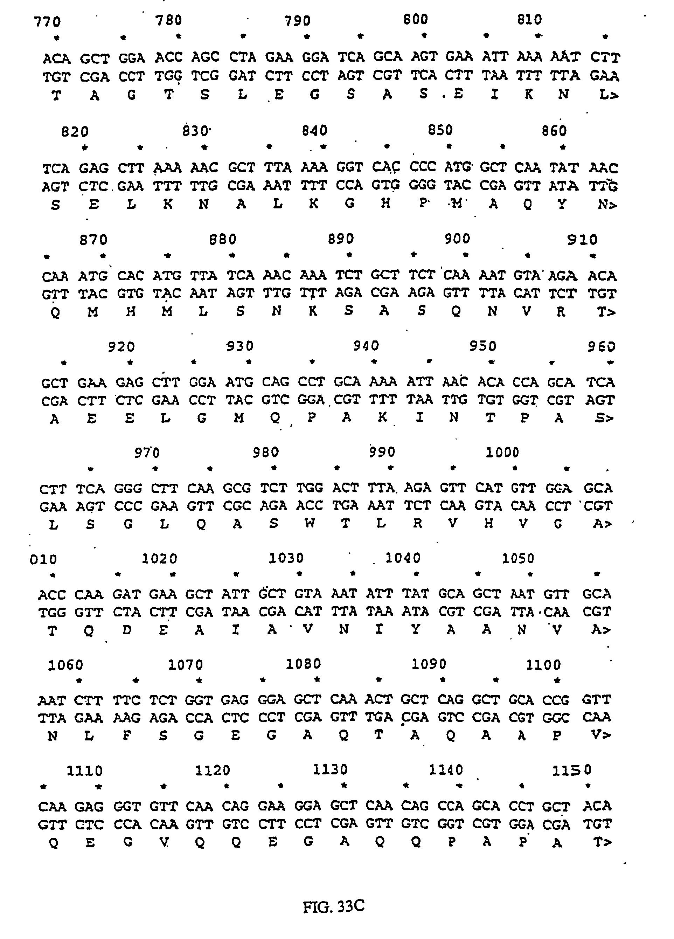

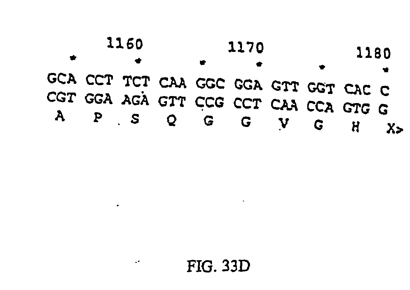

Figs. 33A, 33B, 33C and 33D depict the nucleic acid sequence of the OspB-B31/p41-B31 (122-234) chimer (SEQ ID NO. 113) and the encoded chimeric protein sequence (SEQ ID NO. 114).

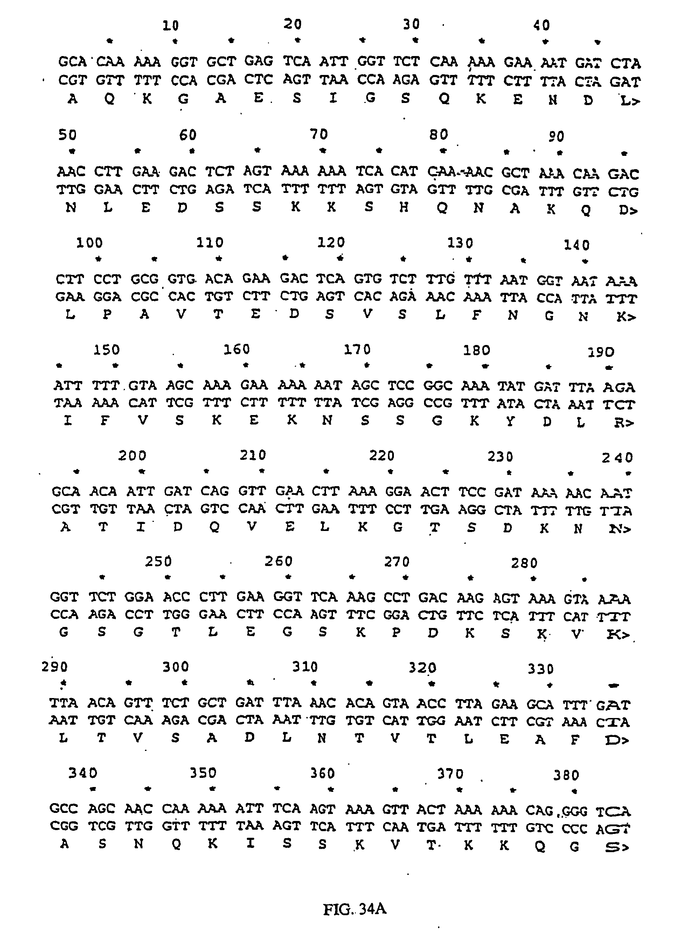

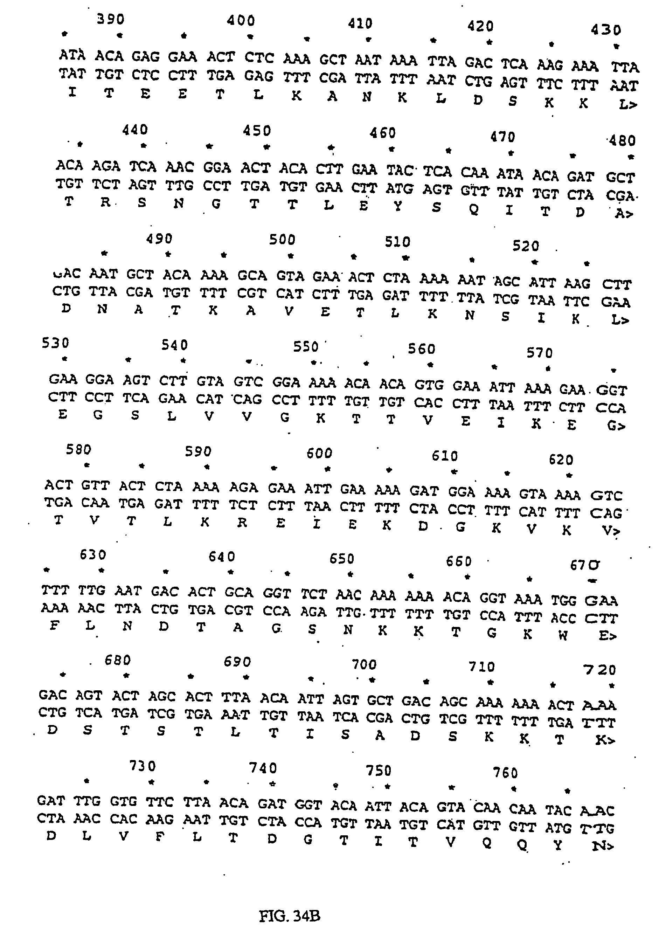

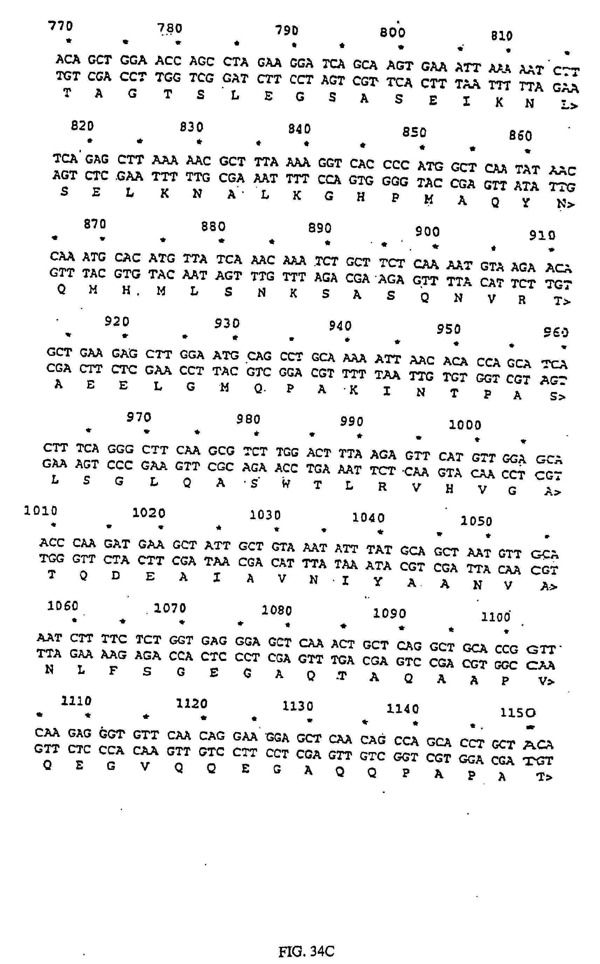

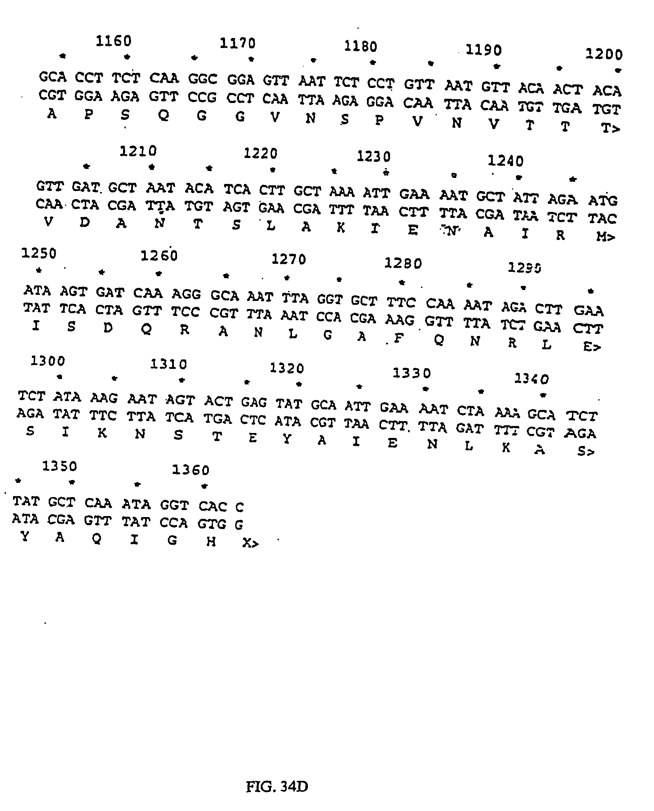

Figs. 34A, 34B, 34C and 34D depict the nucleic acid sequence of the OspB-B31/p41-B31 (122-295) chimer (SEQ ID NO. 115) and the encoded chimeric protein sequence (SEQ ID NO. 116).

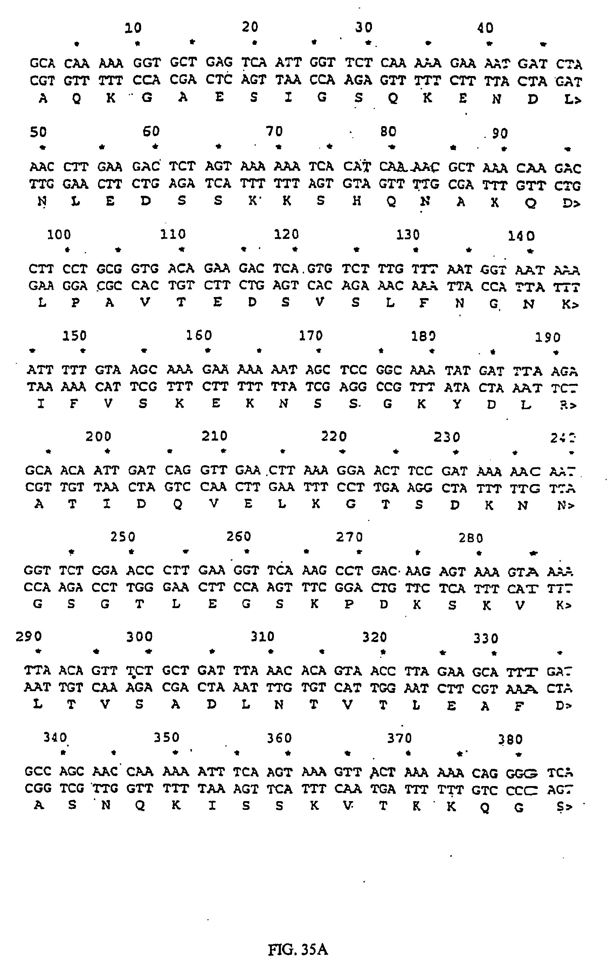

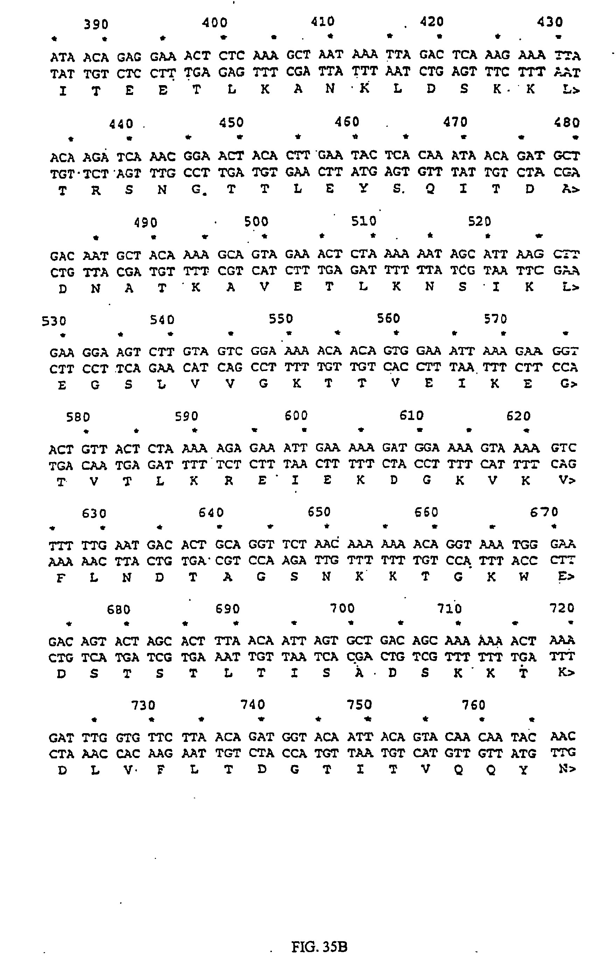

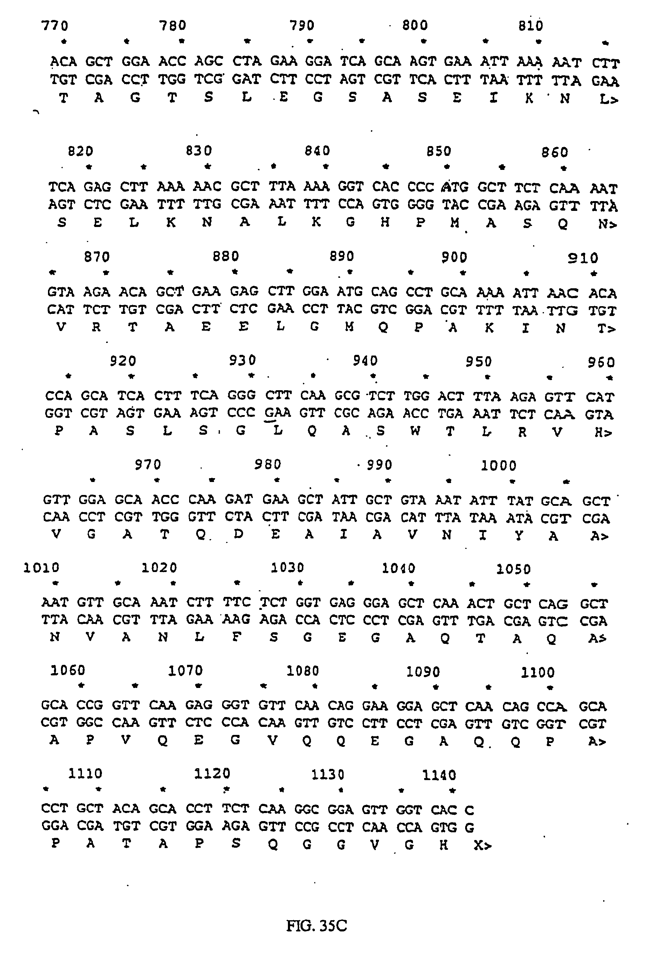

Figs. 35A, 35B and 35C depict the nucleic acid sequence of the OspB-B31/p41-B31 (140-234) chimer (SEQ ID NO. 117) and the encoded chimeric protein sequence (SEQ ID NO. 118).

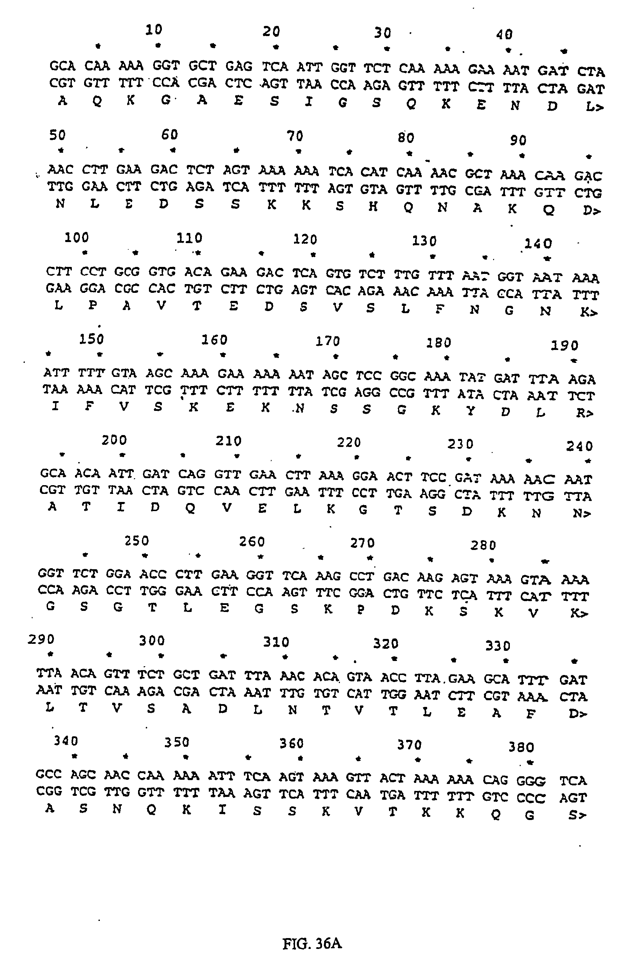

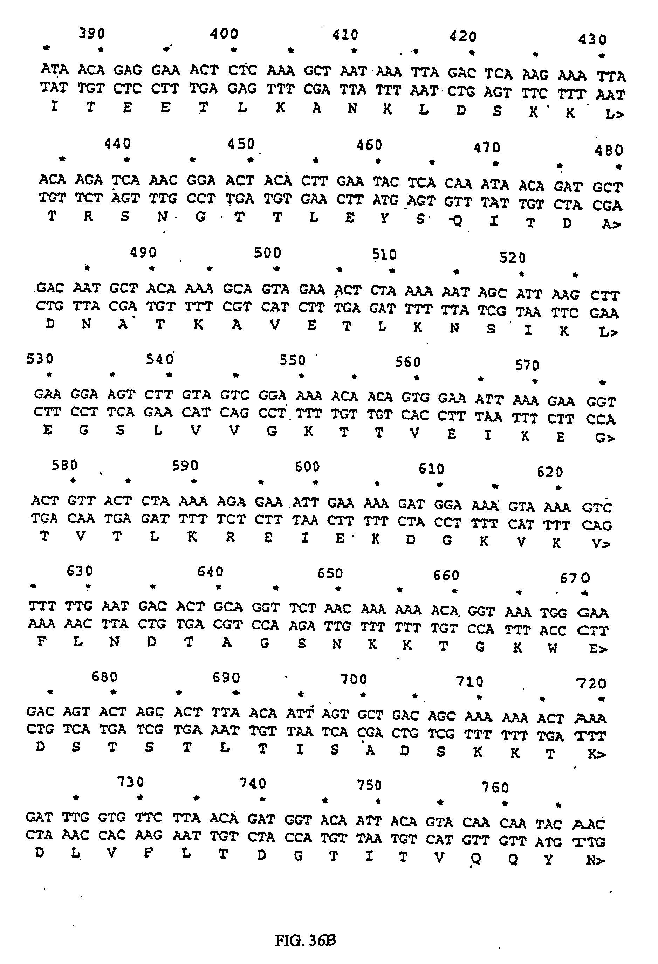

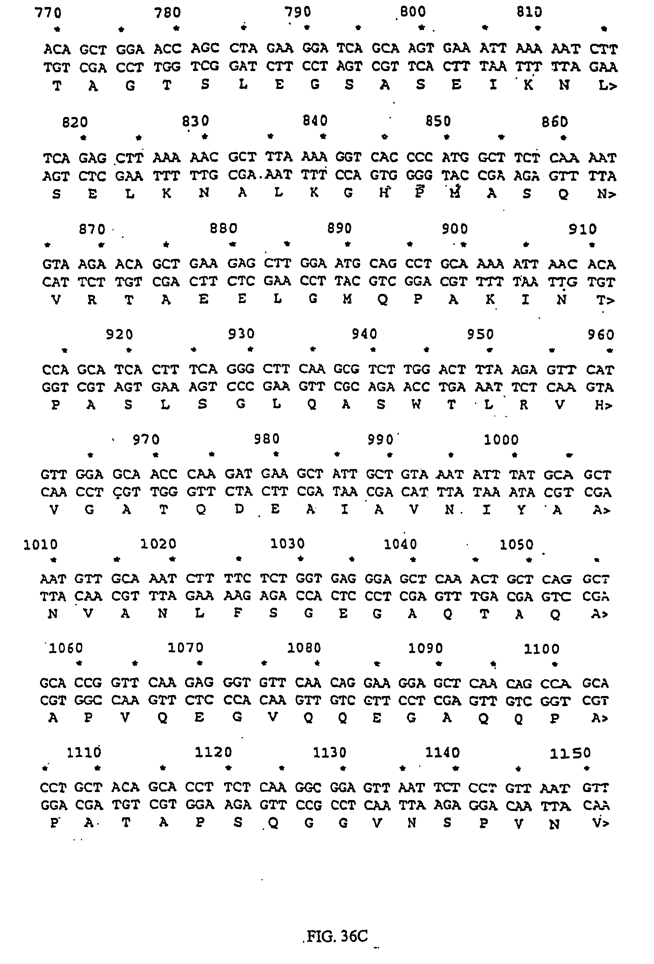

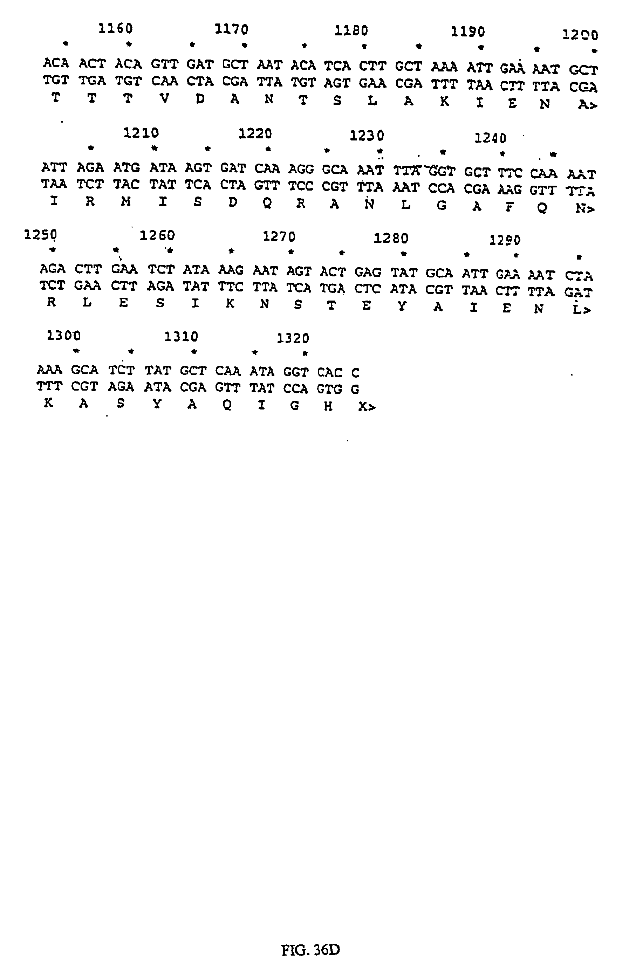

Figs. 36A, 36B, 36C and 36D depict the nucleic acid sequence of the OspB-B31/p41-B31 (140-295) chimer (SEQ ID NO. 119) and the encoded chimeric protein sequence (SEQ ID NO. 120).

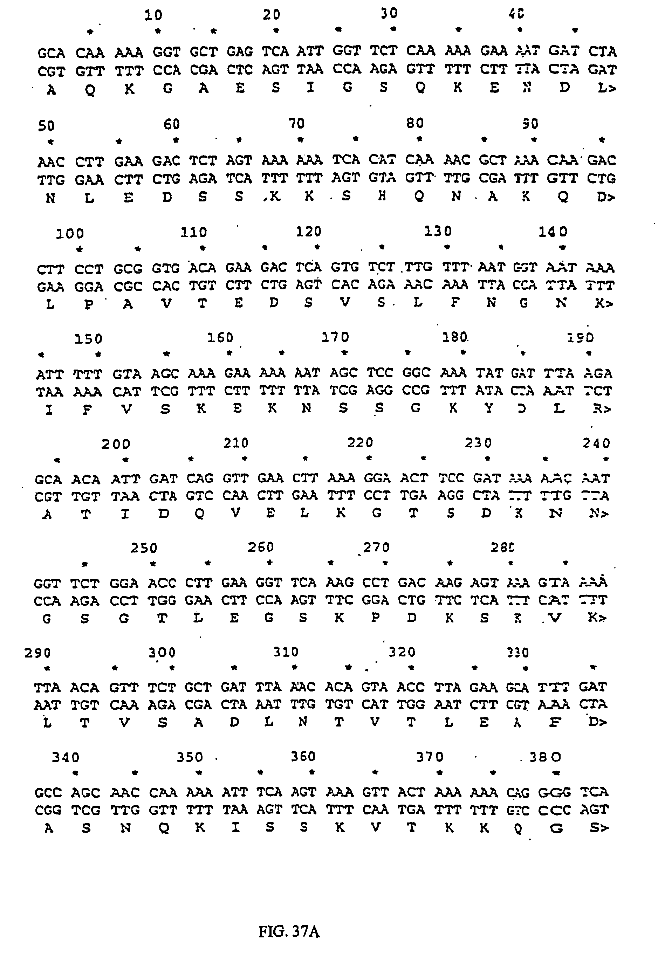

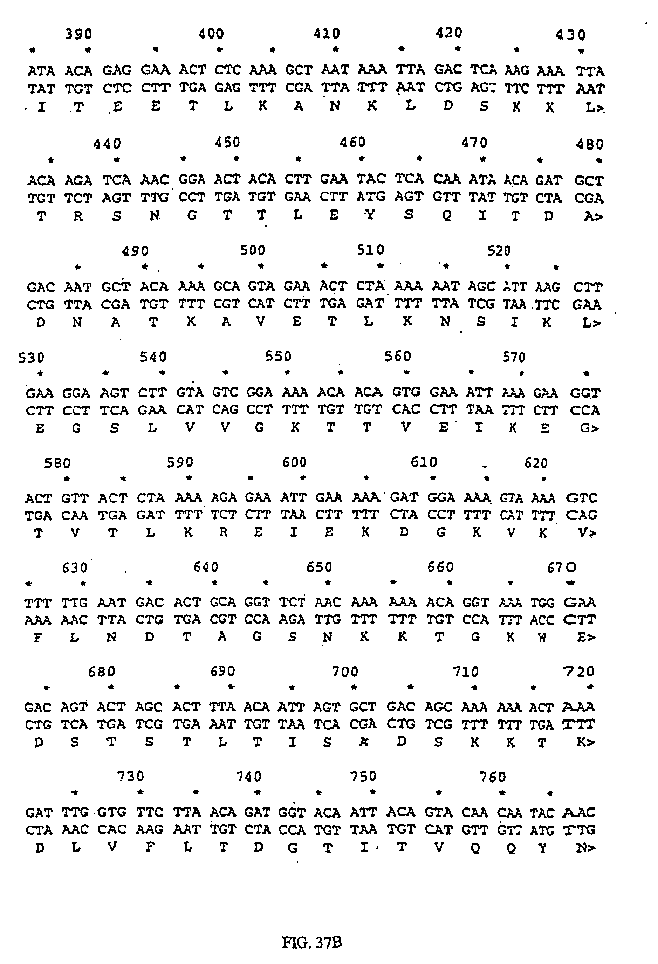

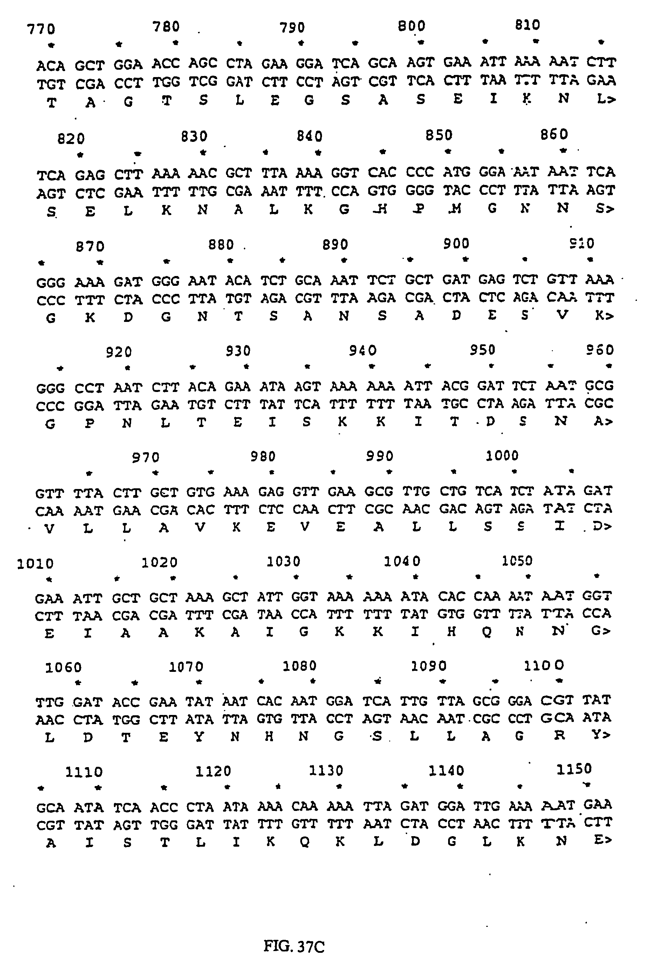

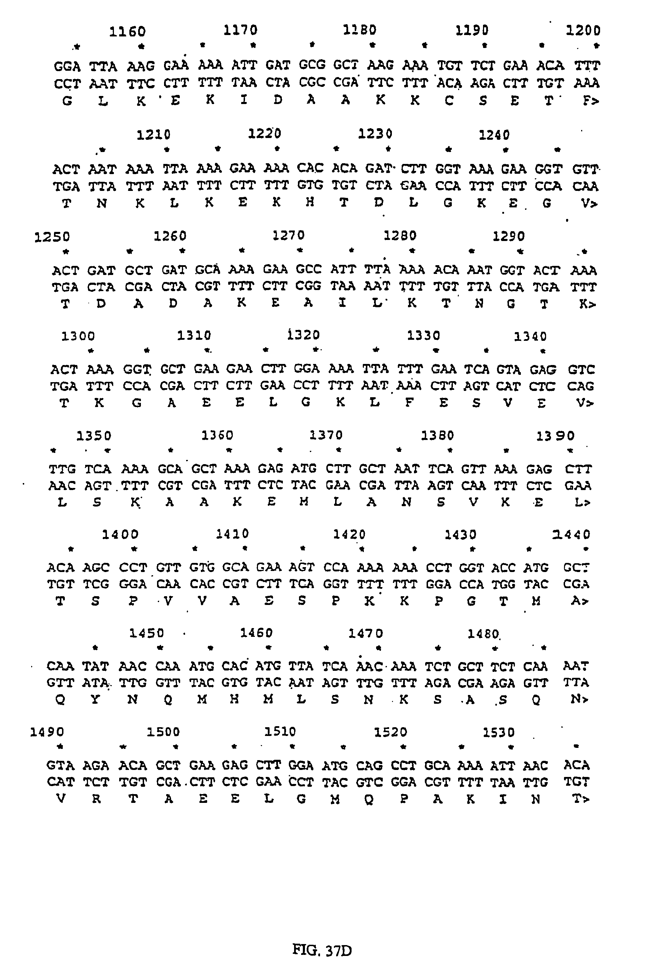

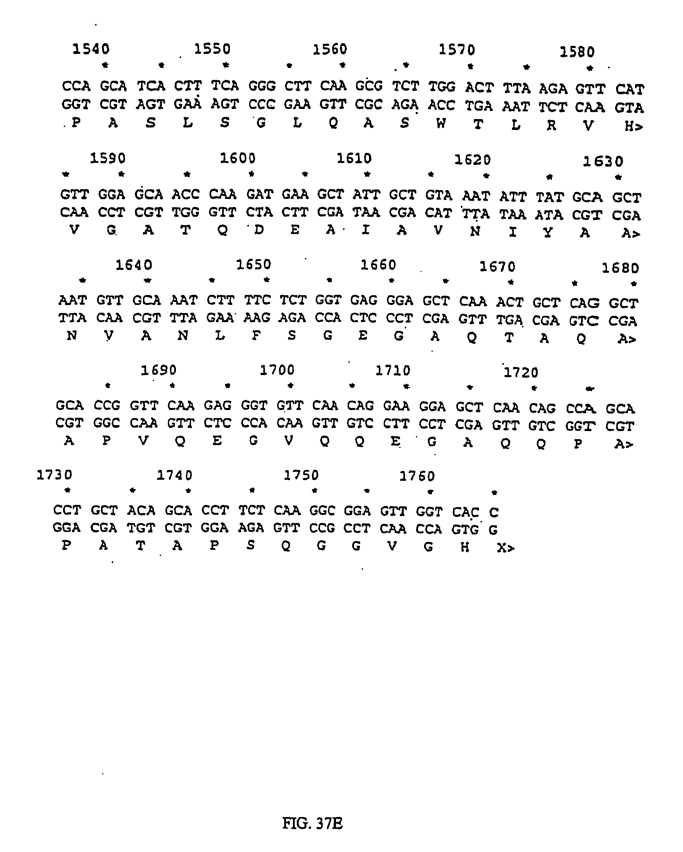

Figs. 37A, 37B, 37C, 37D and 37E depict the nucleic acid sequence of the OspB-B31/p41-B31 (122-234)/OspC-B31 chimer (SEQ ID NO. 121) and the encoded chimeric protein sequence (SEQ ID NO. 122).
Figs. 38A, 38B, 38C and 38D depict an alignment of the nucleic acid sequences for OspC-B31 (SEQ ID NO. 29), OspC-PKo (SEQ ID NO. 33), OspC-PTrob (SEQ ID NO. 35), and OspC-K48 (SEQ ID NO. 31). Nucleic acids which are identical to those in the lead nucleic acid sequence (here, OspC-B31) are represented by a period (.); differing nucleic acids are shown in lower case letters.
Figs. 39A, 39B, 39C and39D depict an alignment of the nucleic acid sequences for OspD-PBo (SEQ ID NO. 123), OspD-PGAU (SEQ ID NO. 124), OspD-DK29 (SEQ ID NO. 125), and OspD-K48 (SEQ ID NO. 126). Nucleic acids which are identical to those in the lead nucleic acid sequence (here, OspD-PBo) are represented by a period (.); differing nucleic acids are shown in lower case letters.

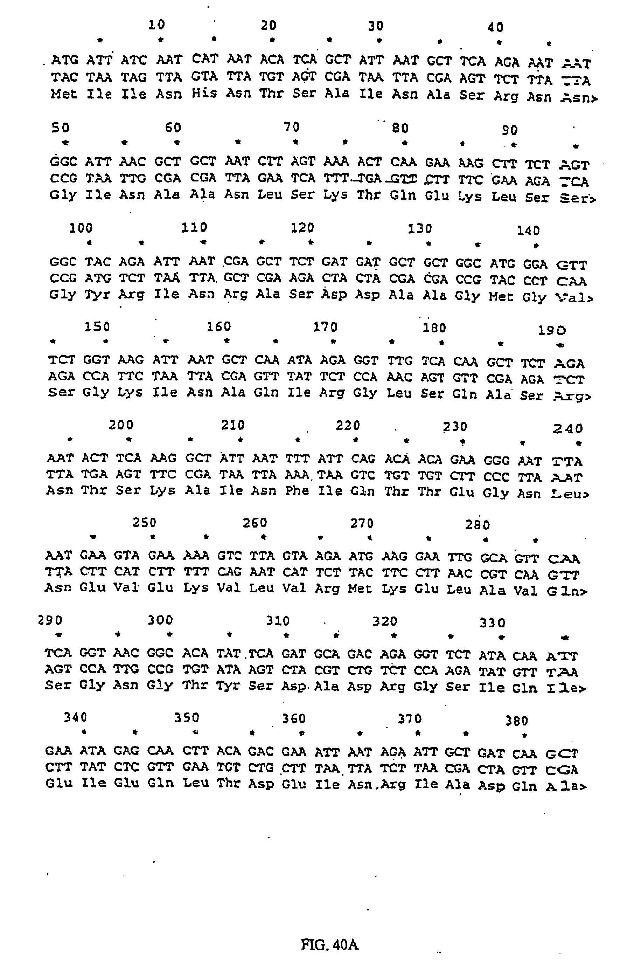

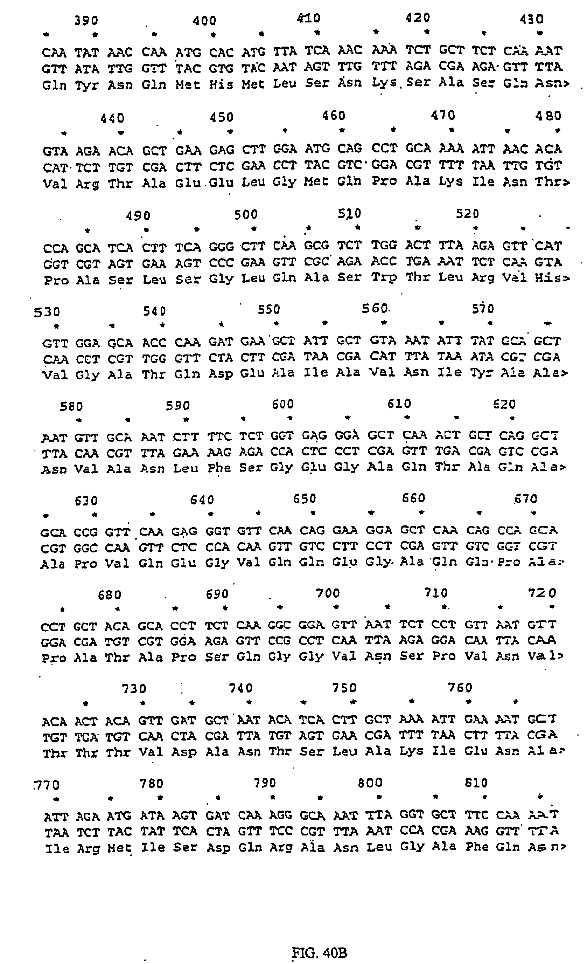

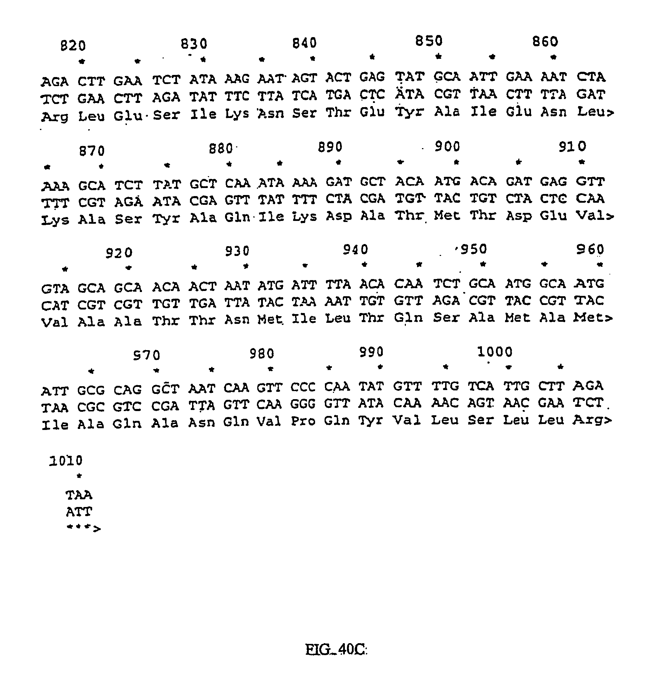

Figs. 40A, 40B and 40C depict the nucleic acid sequence of p41-B31 (SEQ ID NO. 127) and then encoded protein sequence (SEQ ID NO. 128).
Figs. 41A, 41B, 41C, 41D, 41E, 41F, 41G and 41H depict an alignment of the nucleic acid sequences for p41-B31 (SEQ ID NO. 127), p41-PKa1 (SEQ ID NO. 129), p41-PGAU (SEQ ID NO. 51), p41-PBo (SEQ ID NO. 130), p41-DK29 (SEQ ID NO. 53), and p41-PKo (SEQ ID NO. 131). Nucleic acids which are identical to those in the lead nucleic acid sequence (here, p41-B31) are represented by a period (.); differing nucleic acids are shown in lower case letters.

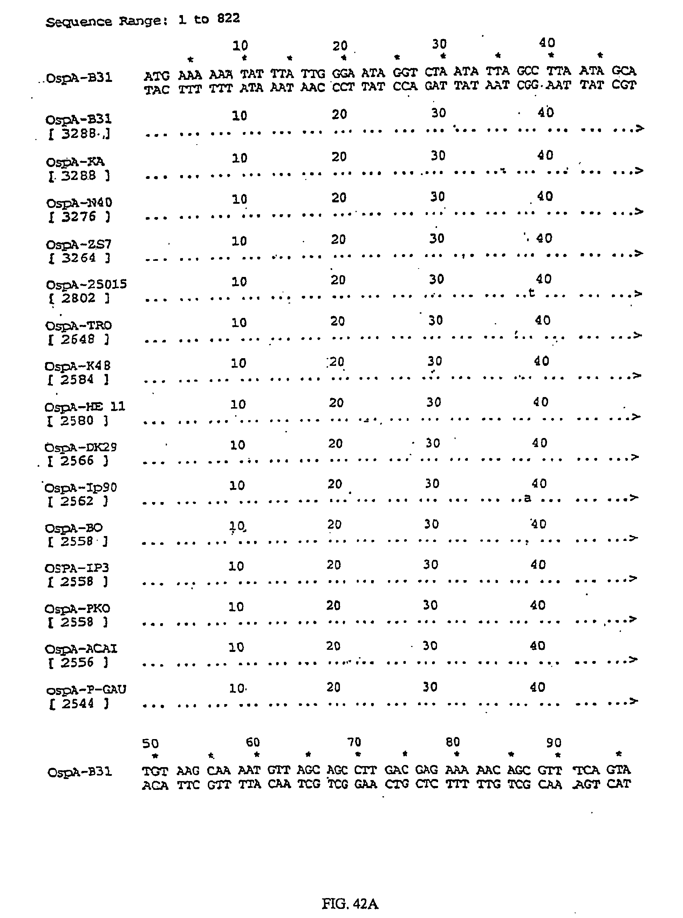

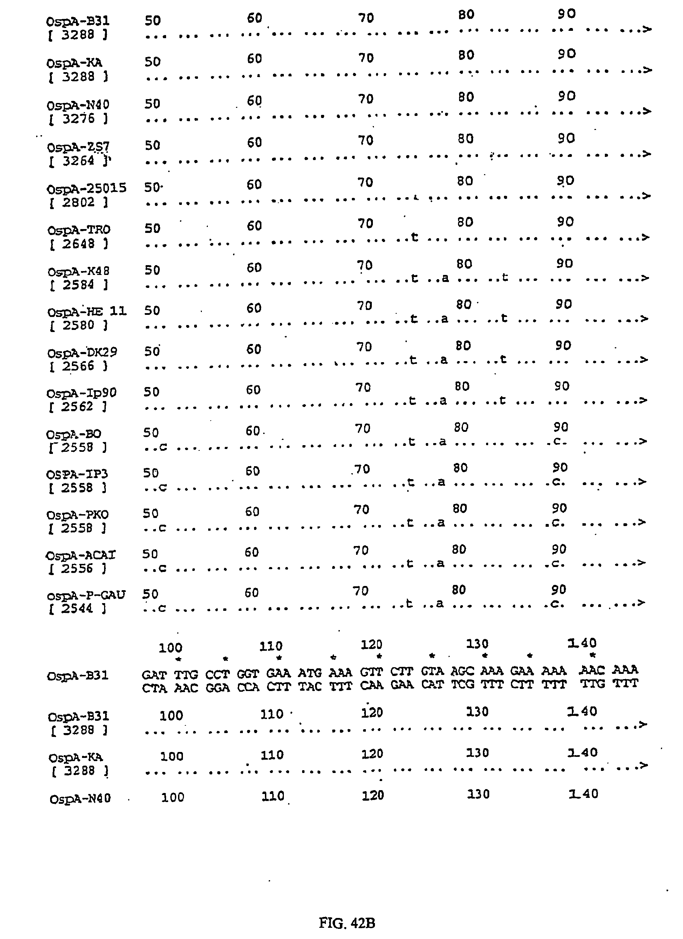

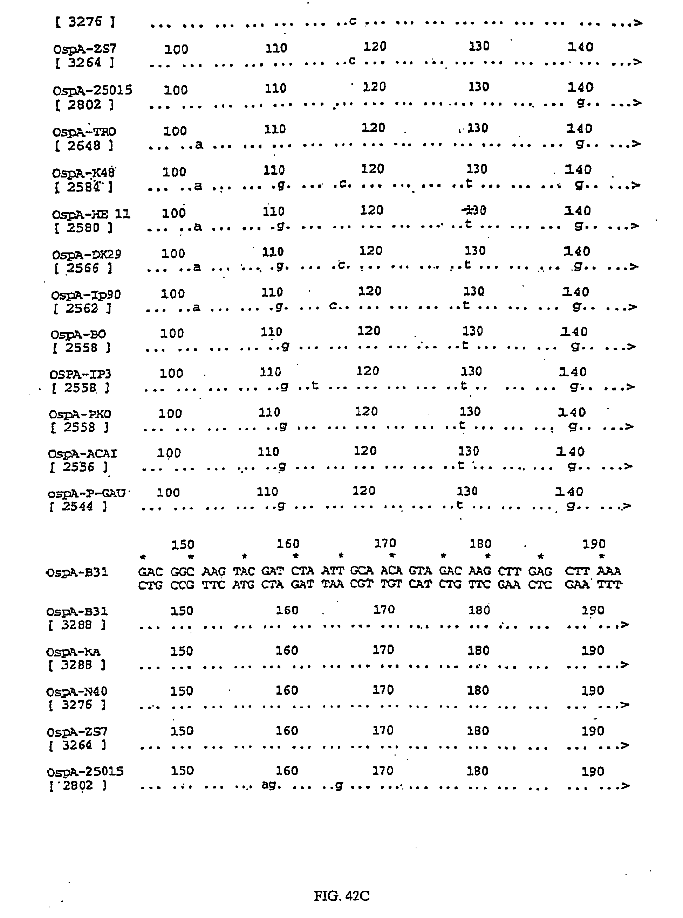

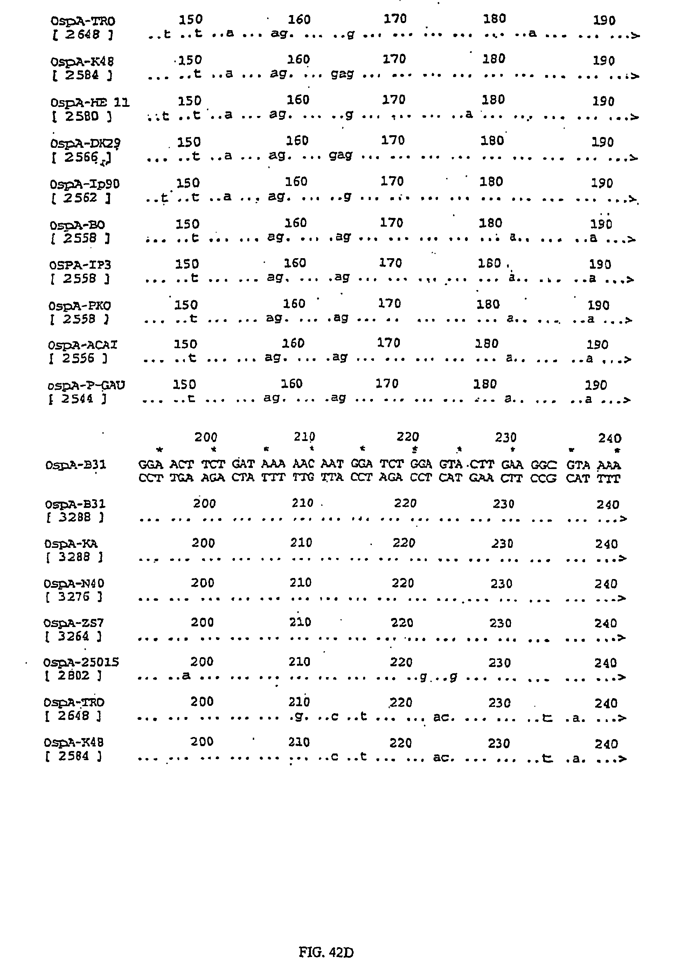

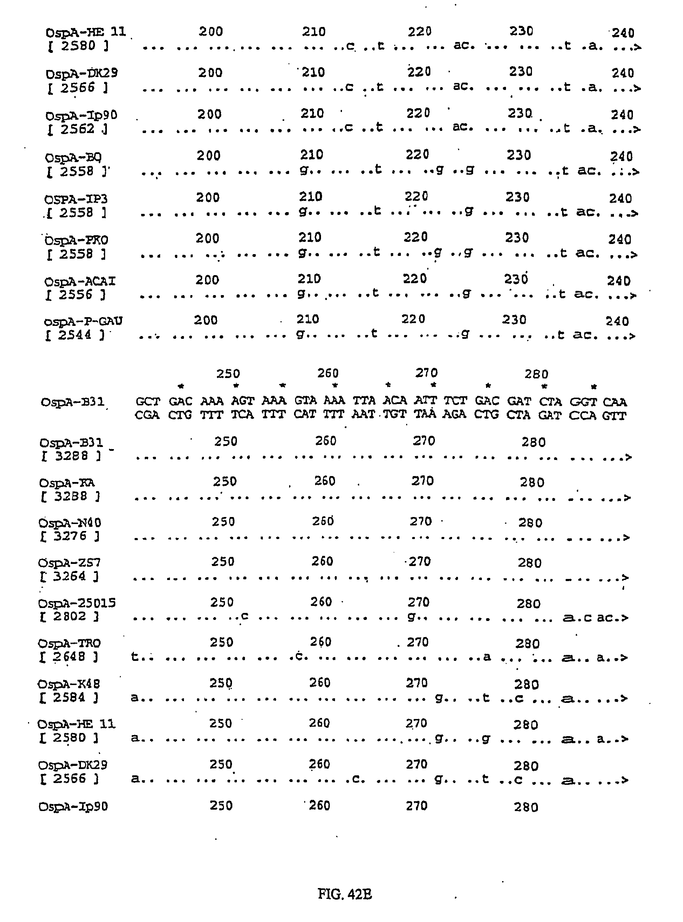

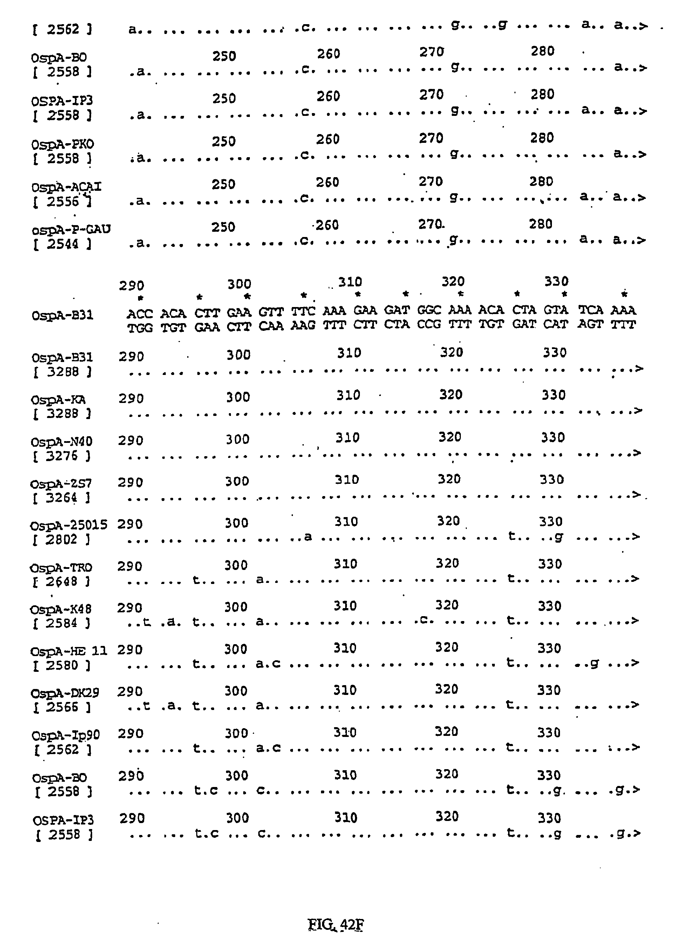

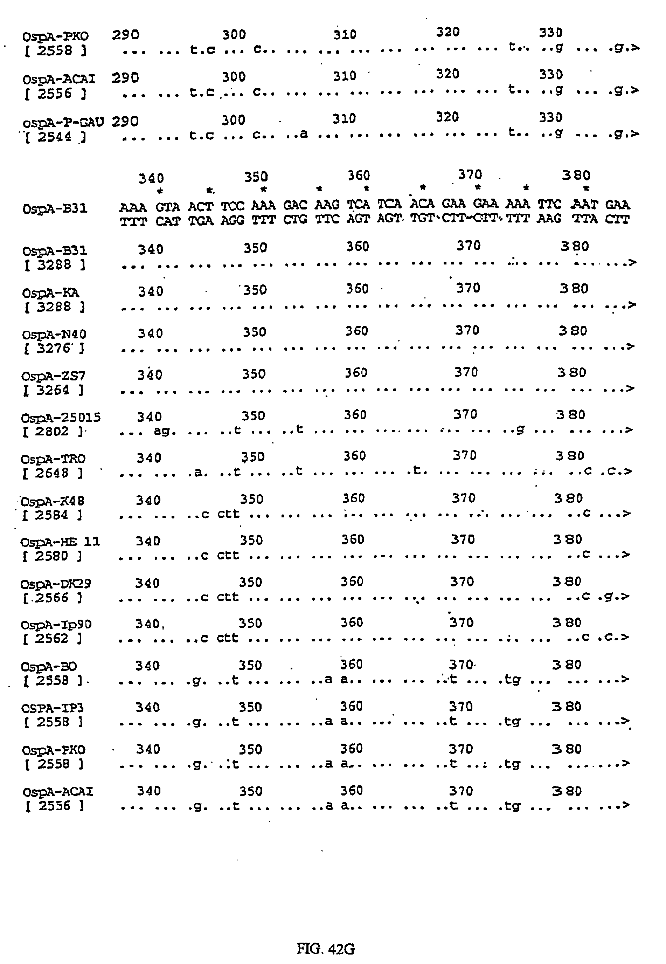

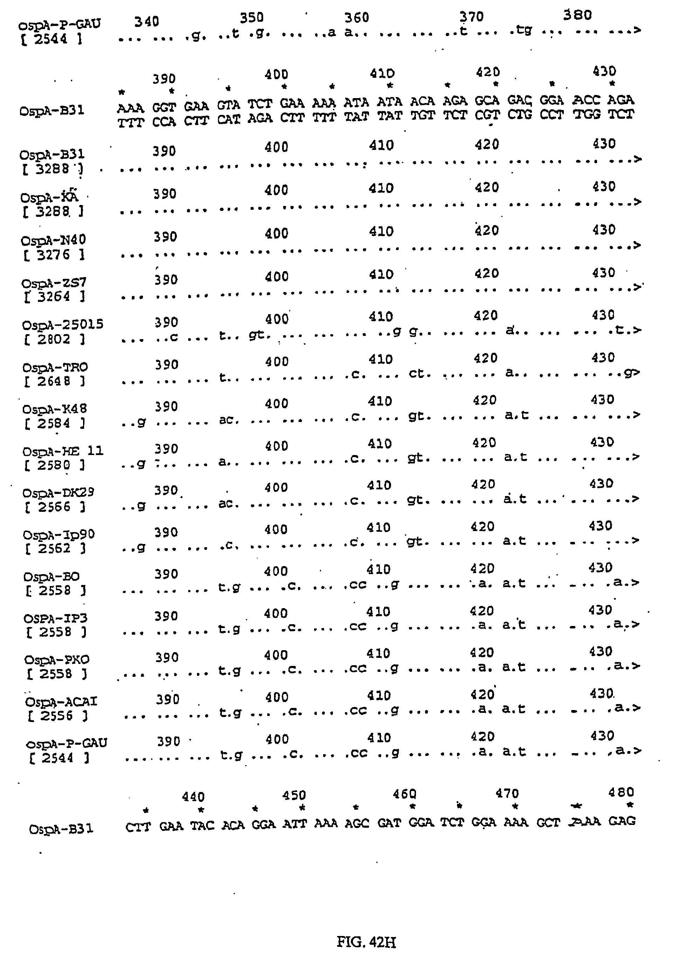

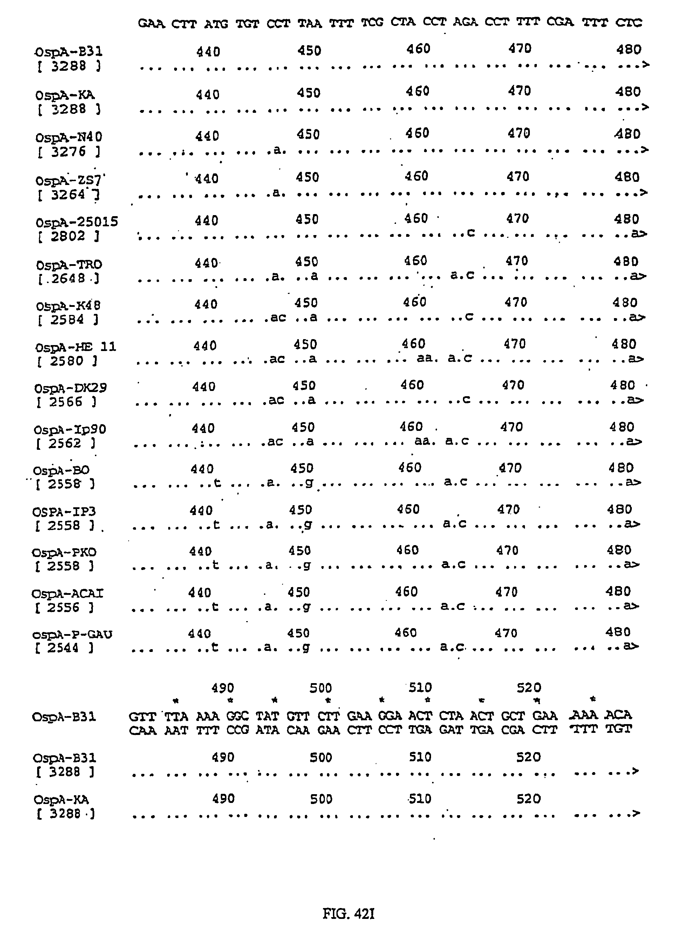

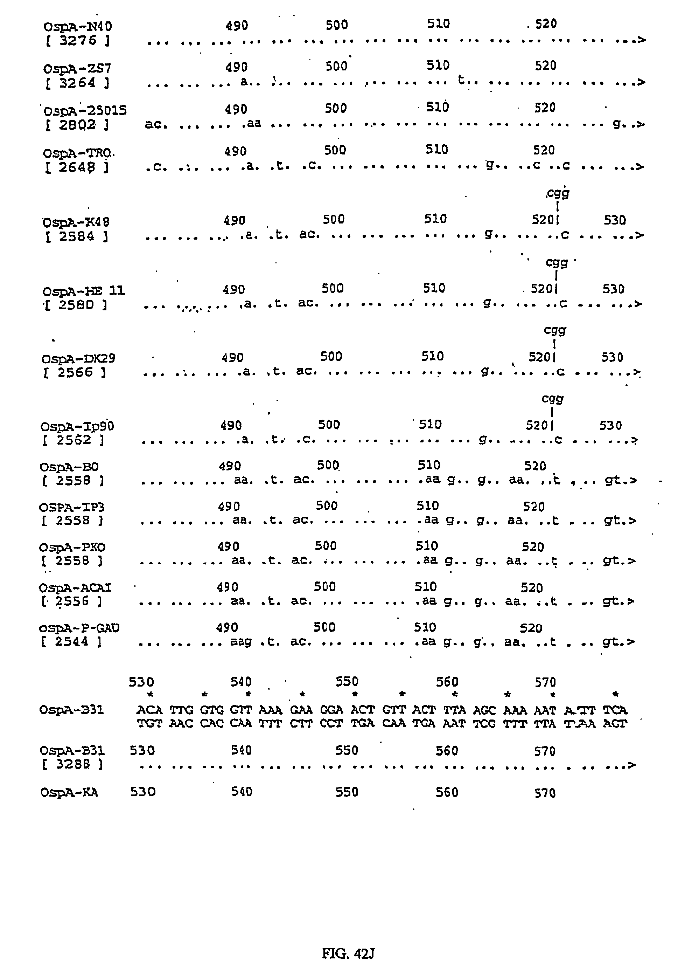

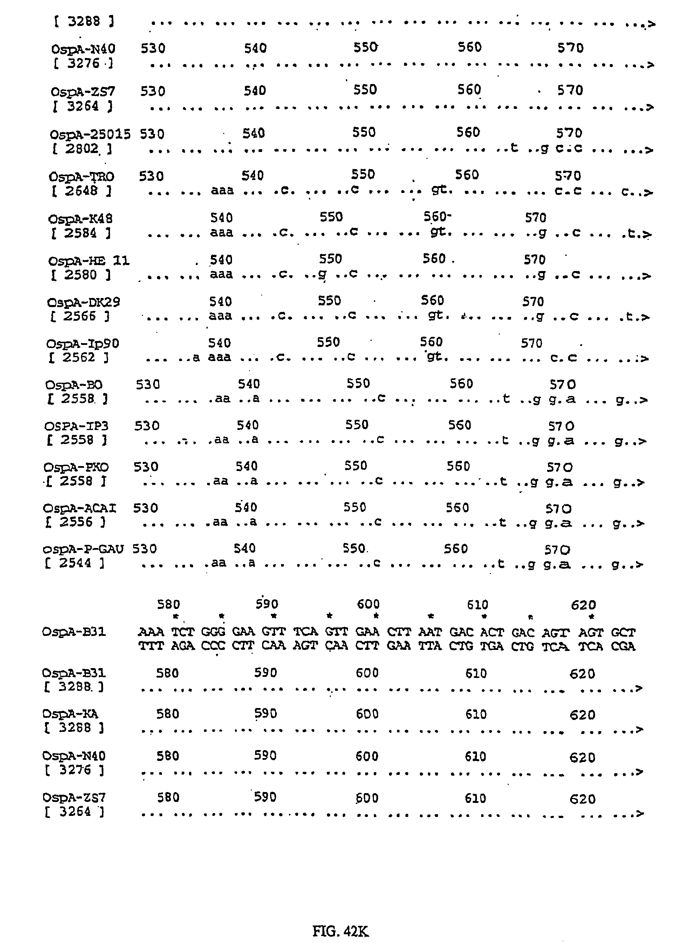

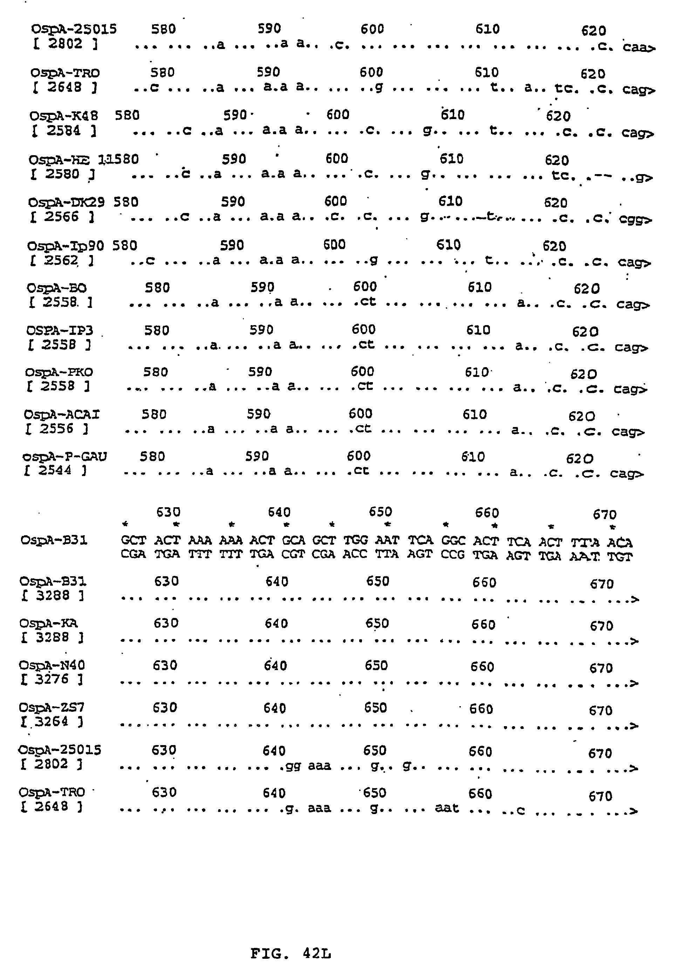

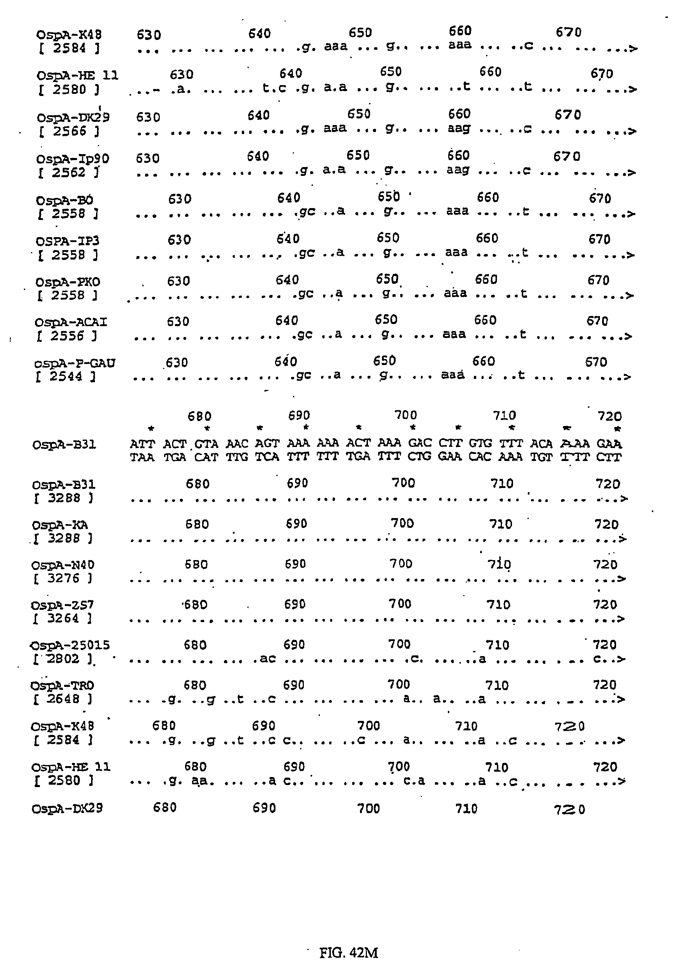

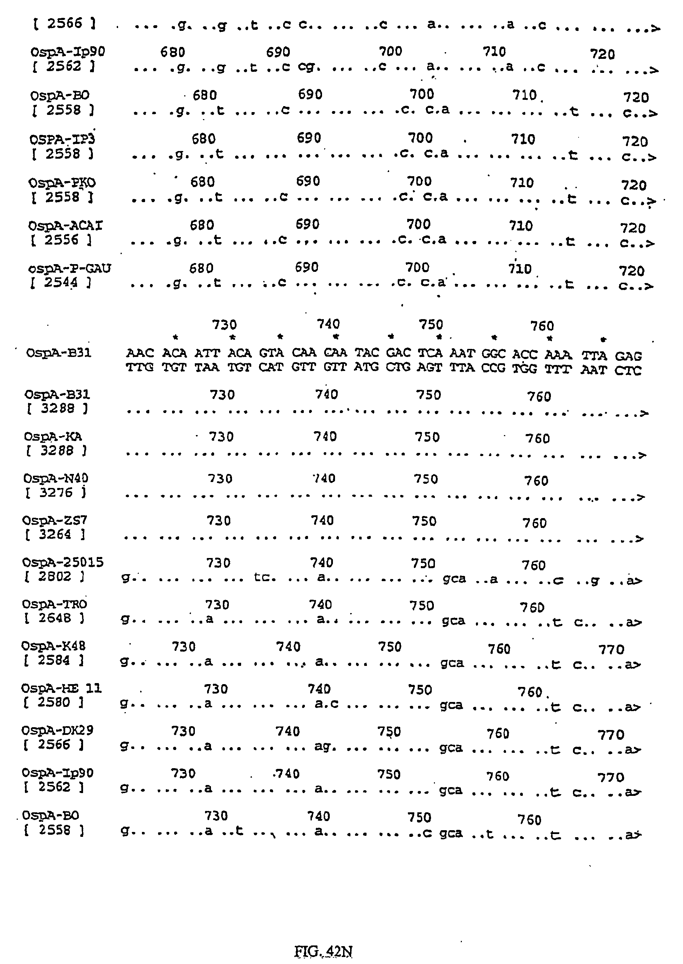

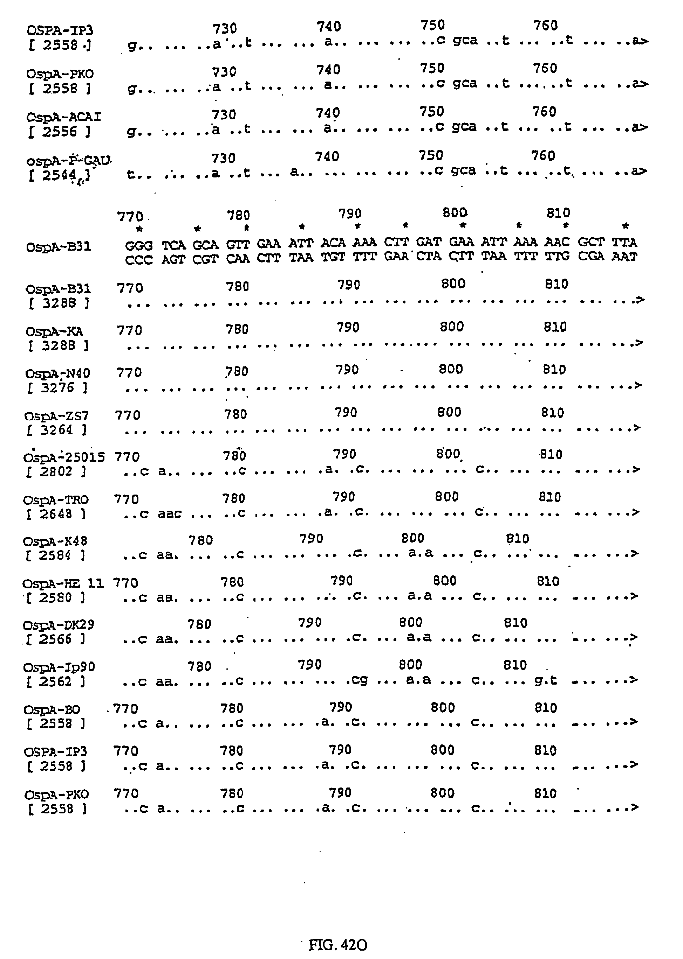

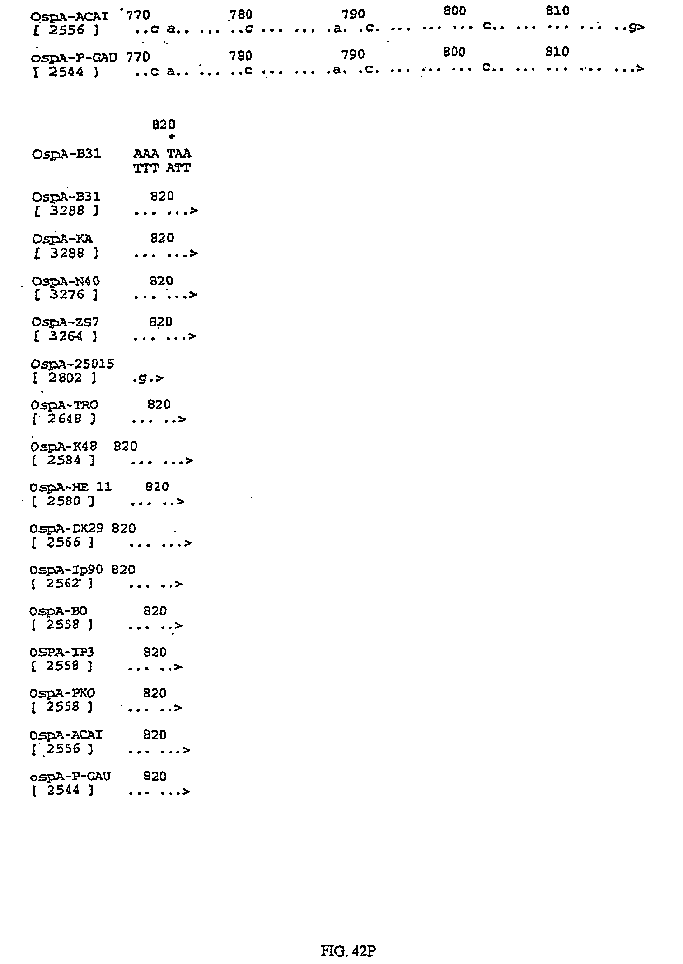

Figs. 42A, 42B, 42C, 42D, 42E, 42F, 42G, 42H, 42I, 42J, 42K, 42L, 42M, 42N, 420 and 42P depict an alignment of the nucleic acid sequences for OspA-B31 (SEQ ID NO. 6), OspA-PKal (SEQ ID NO. 132), OspA-N40 (SEQ ID NO. 133), OspA-ZS7 (SEQ ID NO. 134), OspA-25015 (SEQ ID NO. 12), OspA-PTrob (SEQ ID NO. 135), OspA-K48 (SEQ ID NO. 8), OspA-Hei (SEQ ID NO. 136), OspA-DK29 (SEQ ID NO. 49), OspA-Ip90 (SEQ ID NO. 50), OspA-PBo (Seq ID NO. 55), OspA-Ip3 (SEQ ID NO. 56), OspA-PKo (SEQ ID NO. 57), OspA-ACAI (SEQ ID NO. 58), and OspA-PGAU (SEQ ID NO. 10). Nucleic acids which are identical to those in the lead nucleic acid sequence (here, OspA-B31) are represented by a period (.); differing nucleic acids are shown in lower case letters.
Figs. 43A and 43B depict the nucleic acid sequence of the OspA-Tro/OspA-Bo chimer (SEQ ID NO. 137) which encodes the chimeric protein sequence SEQ ID NO. 138.
Figs. 44A and 44B depict the nucleic acid sequence of the OspA-PGAU/OspA-Bo chimer (SEQ ID NO. 139) which encodes the chimeric protein sequence SEQ ID NO. 140.
Figs. 45A and 45B depict the nucleic acid sequence of the OspA-B31/OspA-PGAU/OspA-B31/OspA-K48 chimer (SEQ ID NO. 143) which encodes the chimeric protein sequence SEQ ID NO. 144.
Figs. 46A and 46B depict the nucleic acid sequence of the OspA-PGAU/OspA-B31/OspA-K48 chimer (SEQ ID NO. 141) which encodes the chimeric protein sequence SEQ ID NO. 142.
Fig. 47 is a bar graph showing the reactivity (as measured by ELISA) of sera from mice immunized with the indicated *Borrelia* protein (OspA or OspC) or recombinant chimeric protein (OspC2-OspA) (X-axis) against OspA B31 or OspC B31 antigens (legend).
Fig. 48 is a bar graph showing the reactivity (as measured by ELISA) of sera from mice immunized with the indicated *Borrelia* protein (OspA or OspC) or recombinant chimeric protein (OspC2-OspA) (X-axis) against OspA B31 or OspC B31 antigens (legend). For the ELISA results to the B31 OspA antigen, a purified fragment of B31 OspA (amino acids 18-139) was added in excess to the sera so that the detected immune response was specific for the C-terminal region of OspA.
Fig. 49 is a bar graph showing the reactivity of sera from mice immunized with the indicated *Borrelia* chimeric protein (lipOspA/Bo, lipOspAB/P or OspC-OspAB/P) (X-axis) against the indicated OspA antigens (legend) from strains B31 *(Borrelia burgdorferi* sensu stricto), K48 *(Borrelia garinii)* and PGau *(Borrelia afzelli).*
Fig. 50 is a bar graph showing the reactivity of sera from mice immunized with the indicated *Borrelia* chimeric protein (lipOspAP/Bo, lipOspAB/P) or OspC-OspAB/P) (X-axis) against the indicated OspA (legend) from strains B31 *(Borrelia burgdorferi* sensu stricto), K48 *(Borrelia garinii)* and PGau *(Borrelia afzelli).* In all cases, a purified fragment of B31 OspA (amino acids 18-139) was added in excess to the sera so that the detected immune response is specific for the C-terminal region of OspA.
Fig. 51 is a bar graph showing the reactivity of sera from mice immunized with the indicated *Borrelia* chimeric protein (OspCB31-OspAB31, OspC2-OspAB31 or lip OspC-B31) (X-axis) against the indicated OspC antigen (legend) from the strain B31 *(Borrelia burgdorferi* sensu stricto).
Fig. 52 is a bar graph showing the reactivity of sera from mice immunized with the indicated *Borrelia* chimeric protein (OspCB31-OspAB31, OspC2-OspAB31 or Lip OspA K/T) (X-axis) against the indicated OspA antigens (legend) from strains B31 *(Borrelia burgdorferi* sensu stricto), K48 *(Borrelia garinii)* and PGau *(Borrelia afzelli).*
Fig. 53 is a bar graph showing the reactivity of sera from mice immunized with the indicated *Borrelia* chimeric protein (OspCB31-OspAB/P, OspCB31-OspABPBP or OspCB31-OspAB31) (X-axis) against the indicated OspA antigens (legend) from strains B31 *(Borrelia burgdorferi* sensu stricto), K48 *(Borrelia garinii)* and PGau *(Borrelia afzelli).*
Fig. 54 is a bar graph showing the reactivity of sera from mice immunized with the indicated *Borrelia* chimeric protein (OspCB31-OspAB/P, OspCB31-OspABPBP or OspCB31-OspAB31) (X-axis) against the indicated OspA (legend) from strains B31 *(Borrelia burgdorferi* sensu stricto), K48 *(Borrelia garinii)* and PGau *(Borrelia afzelli).* In all cases, a purified fragment of B31 OspA (amino acids 18-139) was added in excess to the sera so that the detected immune response is specific for the C-terminal region of OspA.

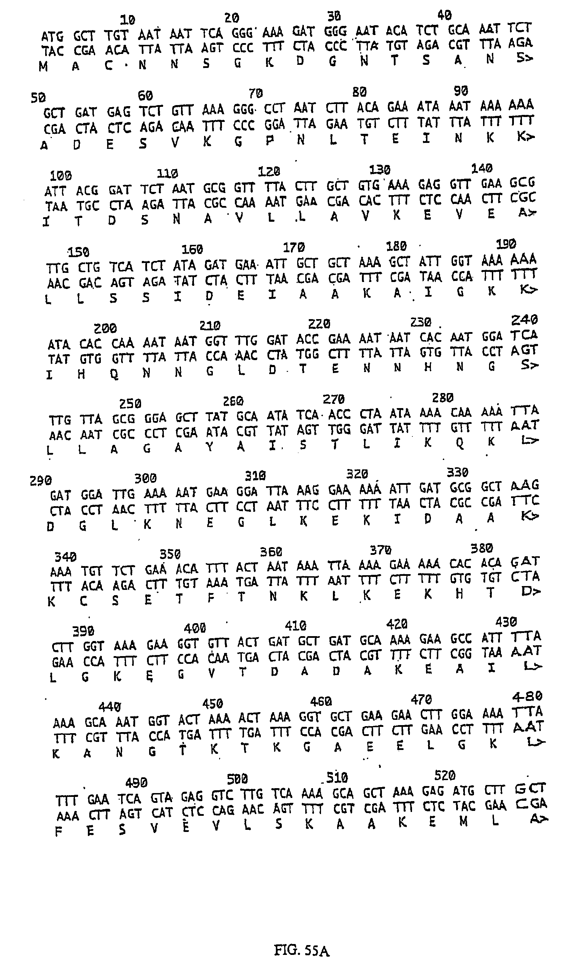

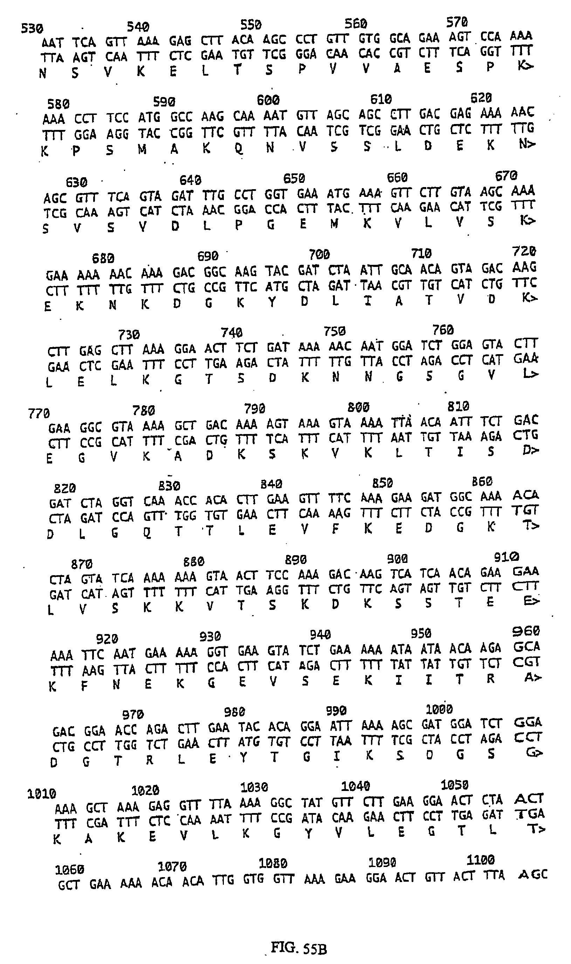

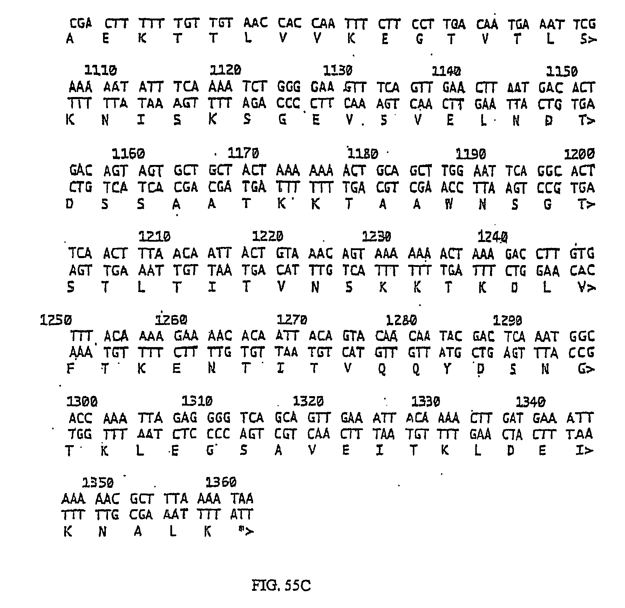

Figs. 55A, 55B and 55C depict the nucleic acid sequence of the OspC-B31 (bp 55-633)/OspA-B31 (bp 52-822) chimer (SEQ ID NO. 145) and the encoded chimeric protein sequence (SEQ ID NO. 146).

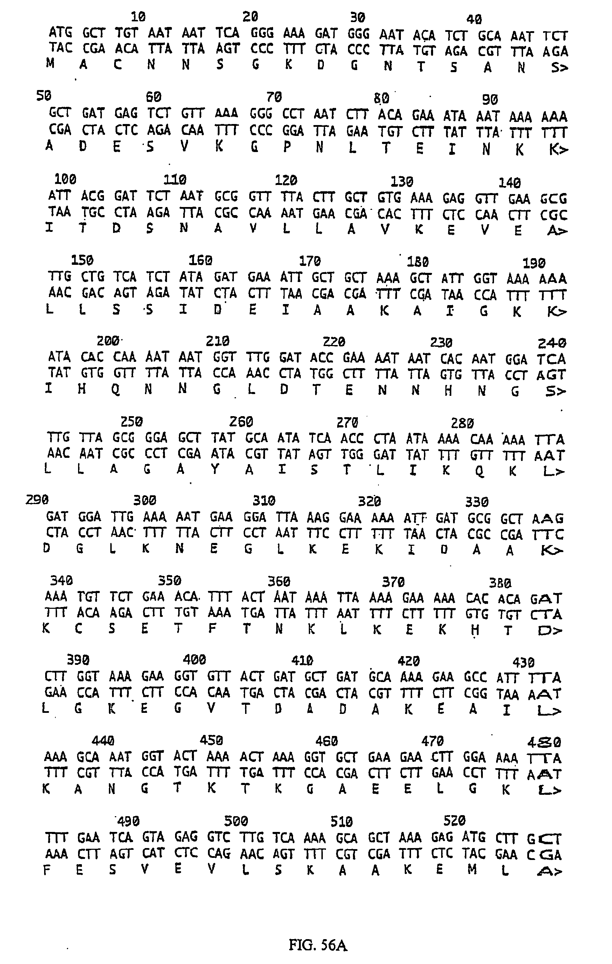

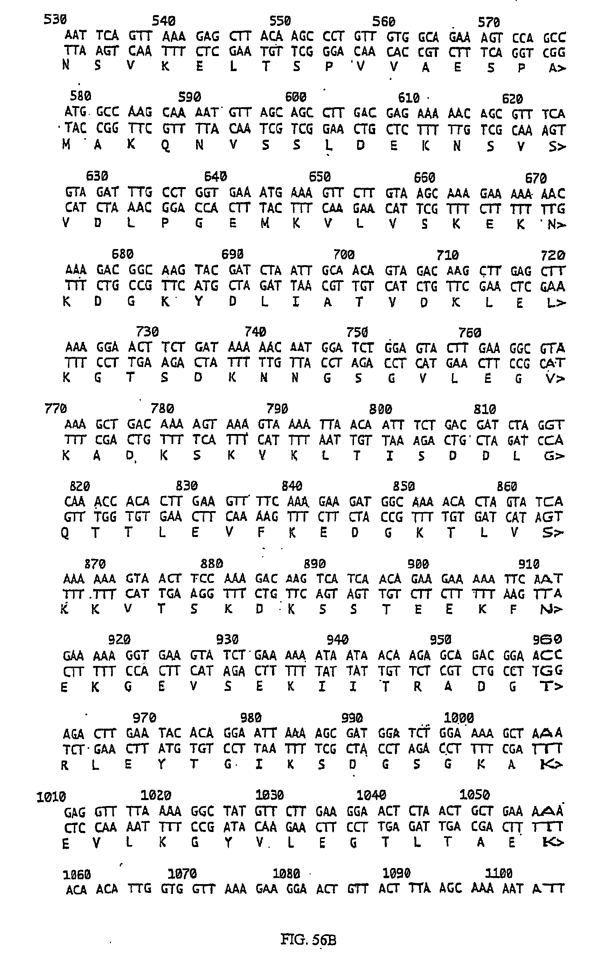

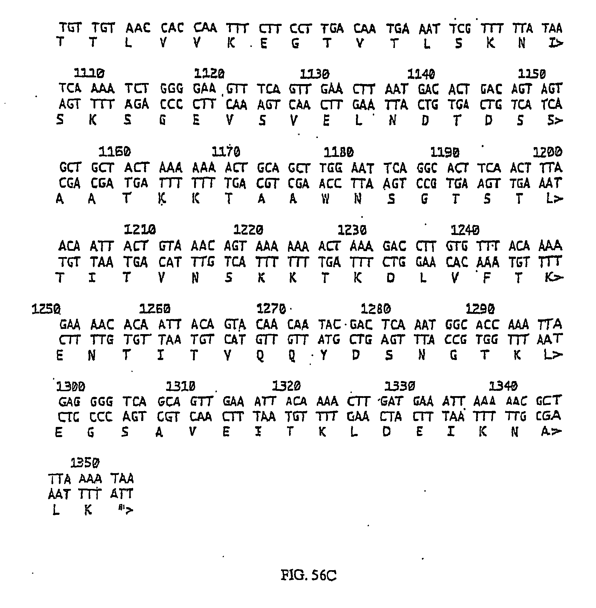

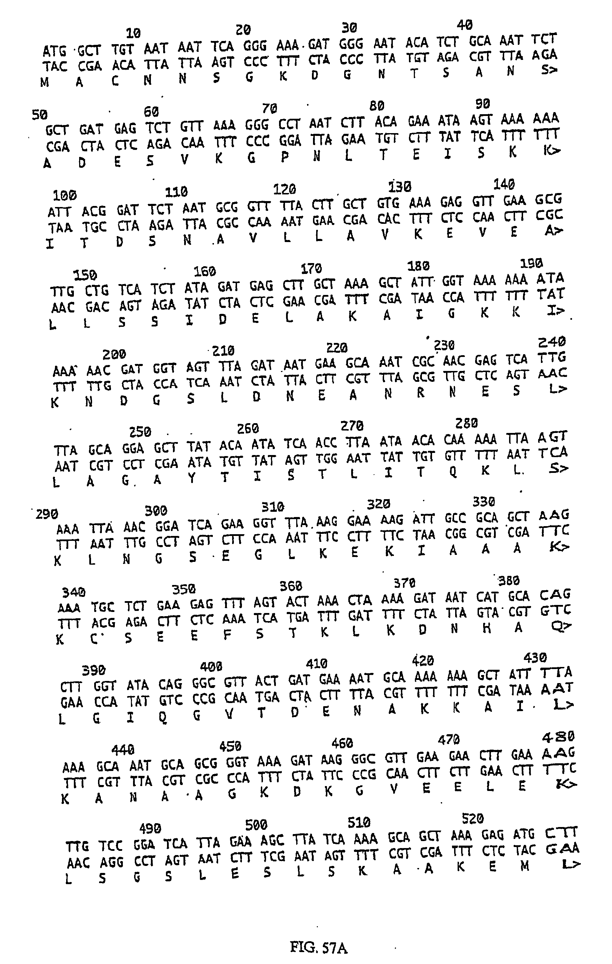

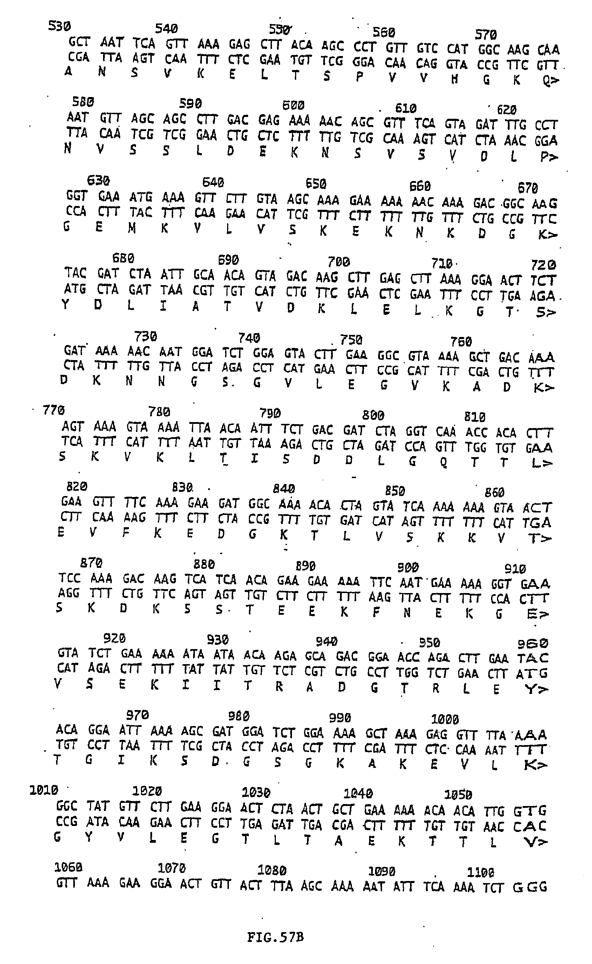

Figs. 56A, 56B and 56C depict the nucleic acid sequence of the OspC-B31 (bp 55-624)/OspA-B31 (bp 52-822) chimer (SEQ ID NO. 147) and the encoded chimeric protein sequence (SEQ ID NO. 148).

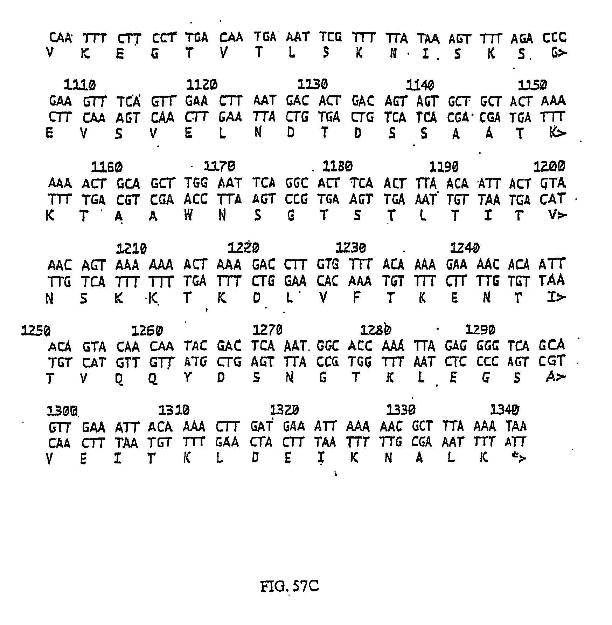

Figs. 57A, 57B and 57C depict the nucleic acid sequence of the OspC-C2 (bp 55-612)/OspA-B31 (bp 52-822) chimer (SEQ ID NO. 149) and the encoded chimeric protein sequence (SEQ ID NO. 150).

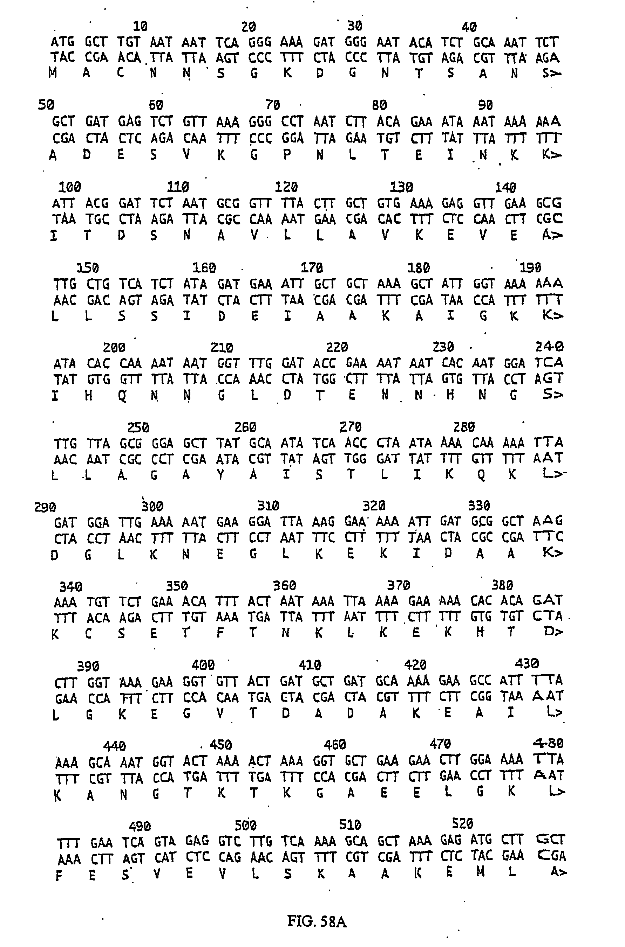

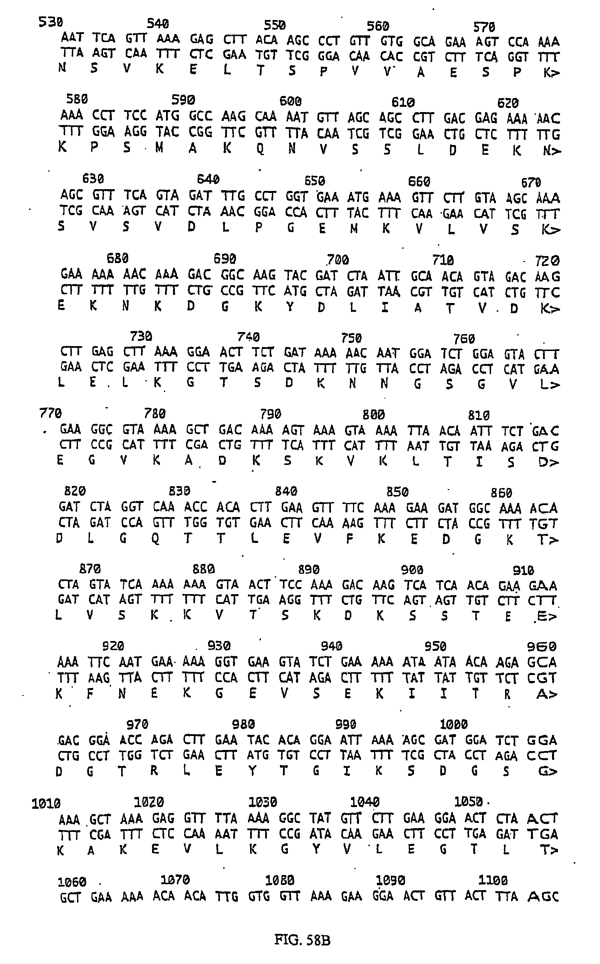

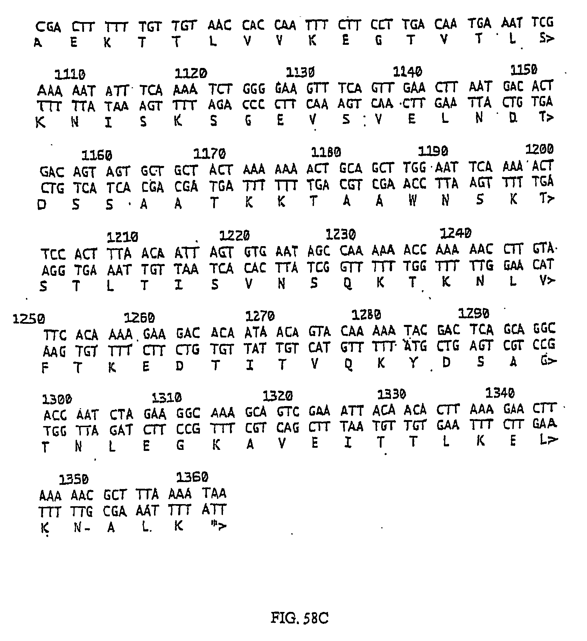

Figs. 58A, 58B and 58C depict the nucleic acid sequence of the OspC-B31 (bp 55-633)/OspA-B31 (bp 52-651)/OspA-K48 (bp 652-820) chimer (SEQ ID NO. 151) and the encoded chimeric protein sequence (SEQ ID NO. 152).

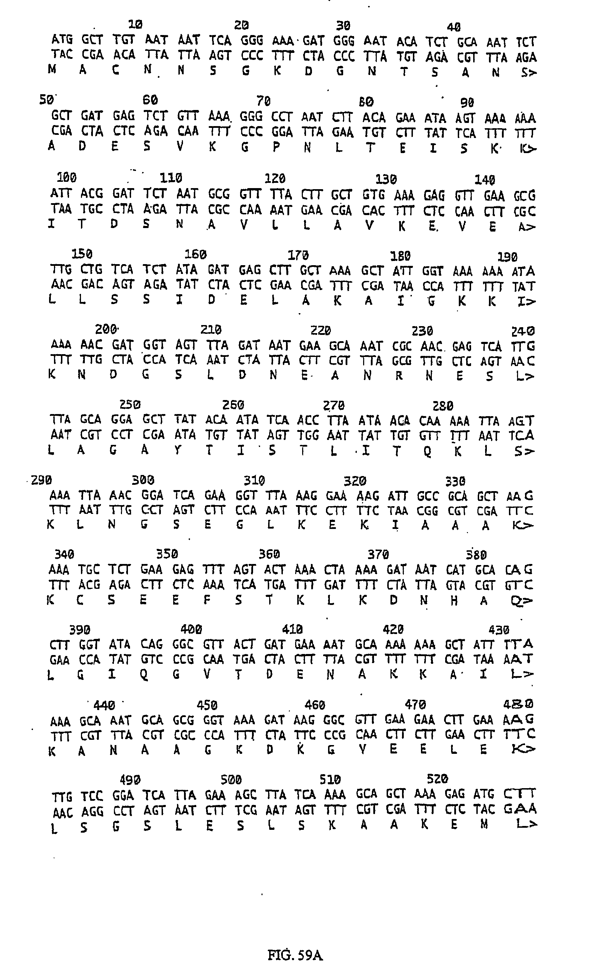

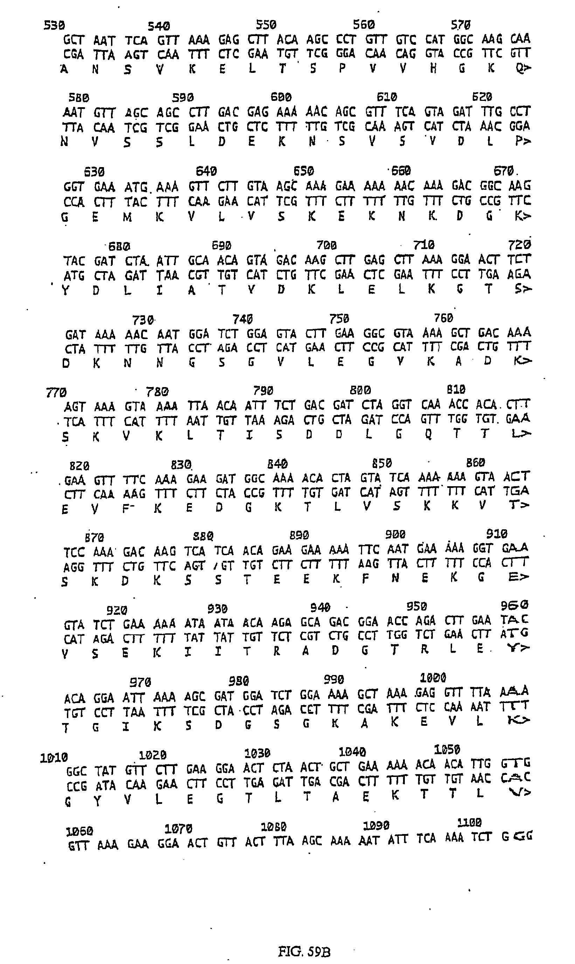

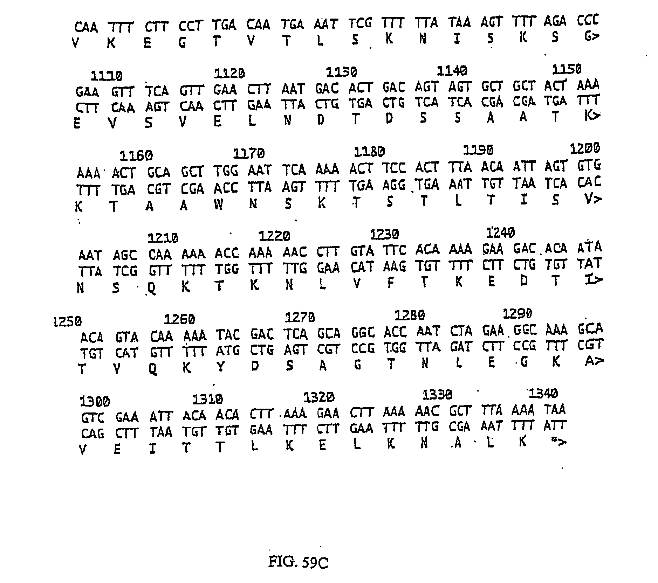

Figs. 59A, 59B and 59C depict the nucleic acid sequence of the OspC-C2 (bp 55-612)/OspA-B31 (bp 52-651)/OspA-K48 (bp 652-820) chimer (SEQ ID NO. 153) and the encoded chimeric protein sequence (SEQ ID NO. 154).

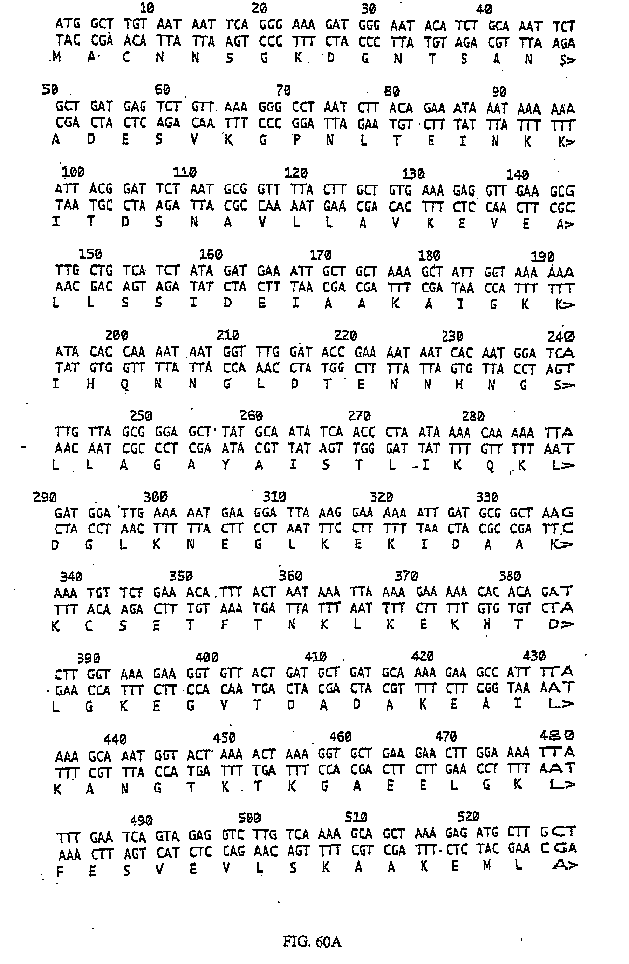

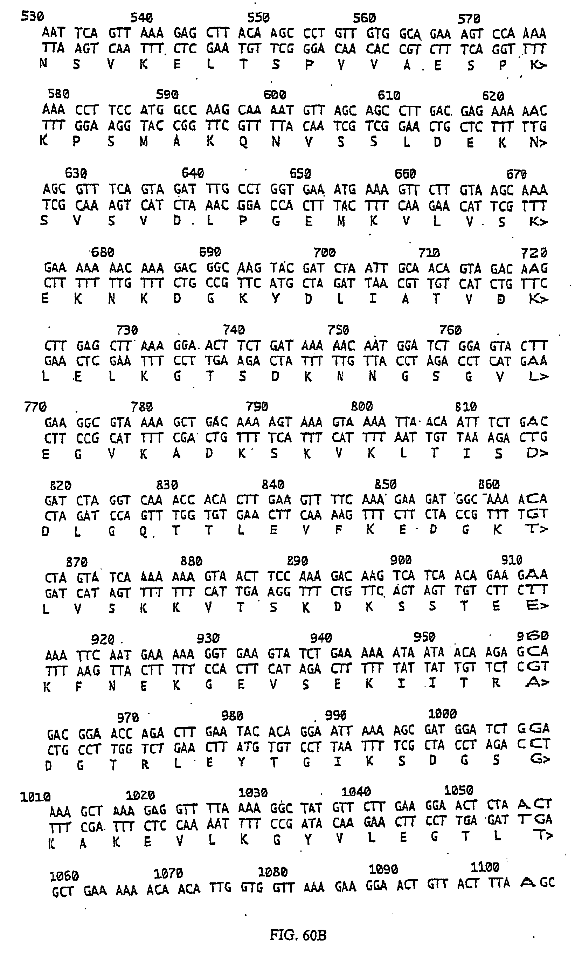

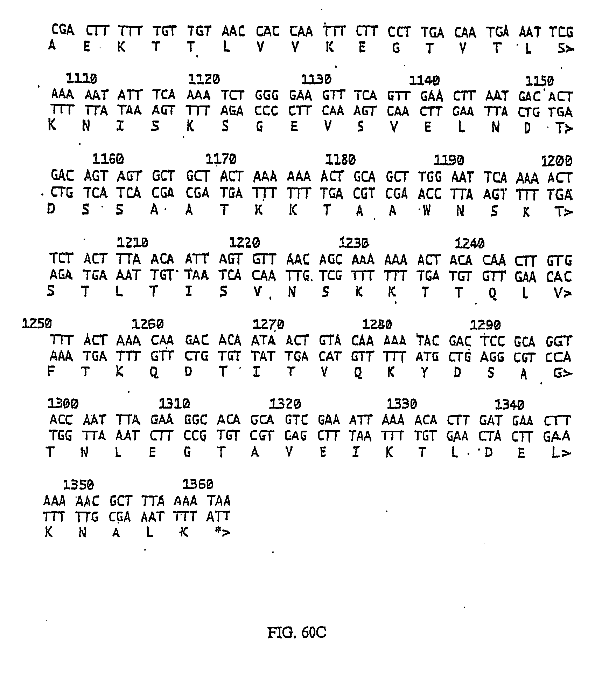

Figs. 60A, 60B and 60C depict the nucleic acid sequence of the OspC-B31 (bp 55-633)/OspA-B31 (bp 52-651)/OspA-PKo (bp 652-820) chimer (SEQ ID NO. 155) and the encoded chimeric protein sequence (SEQ ID NO. 156).

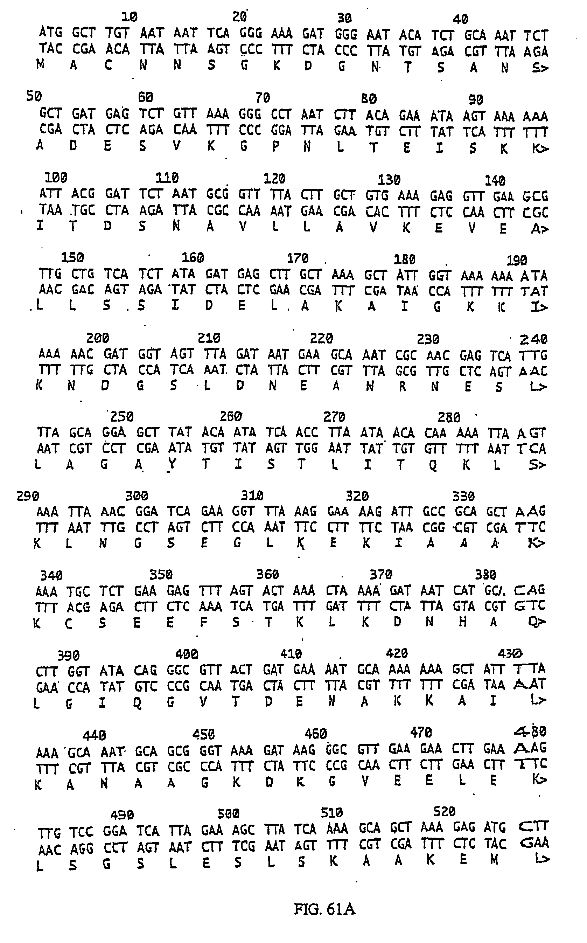

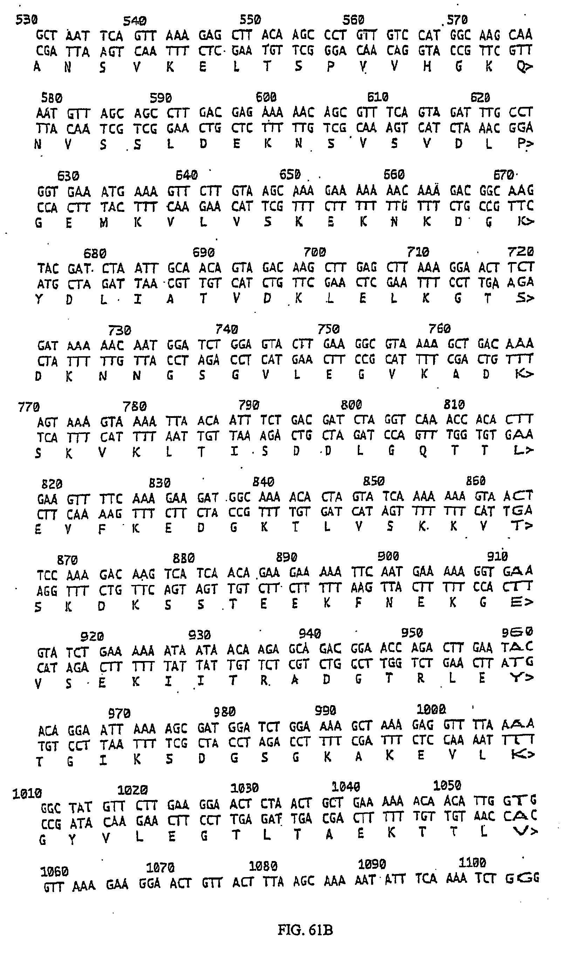

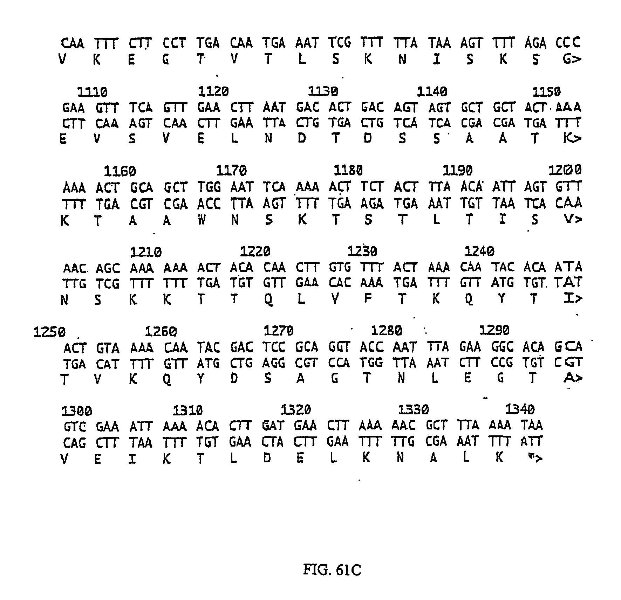

Figs. 61A, 61B and 61C depict the nucleic acid sequence of the OspC-C2 (bp 55-612)/OspA-B31 (bp 52-651)/OspA-PKo (bp 652-820) chimer (SEQ ID NO. 157) and the encoded chimeric protein sequence (SEQ ID NO. 158).

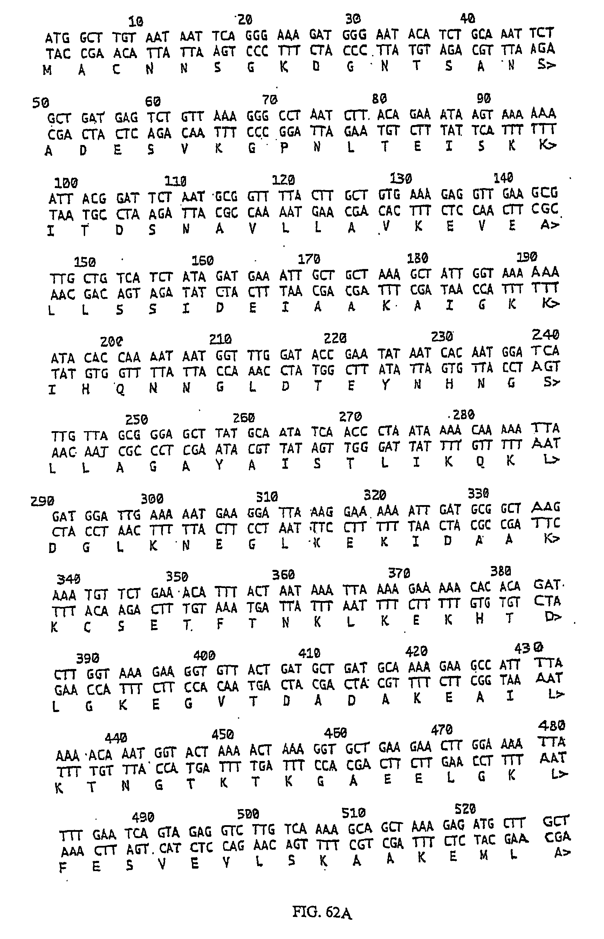

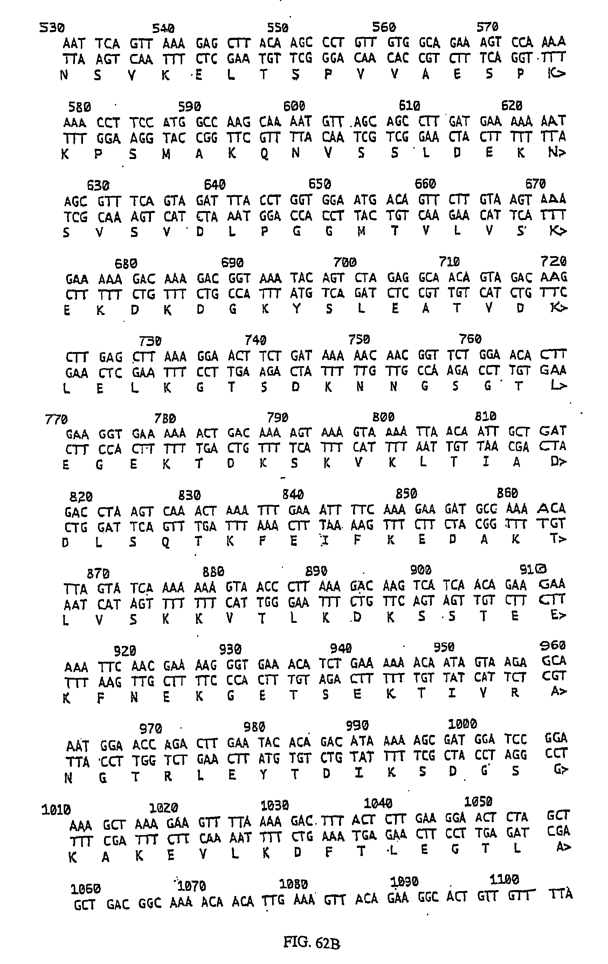

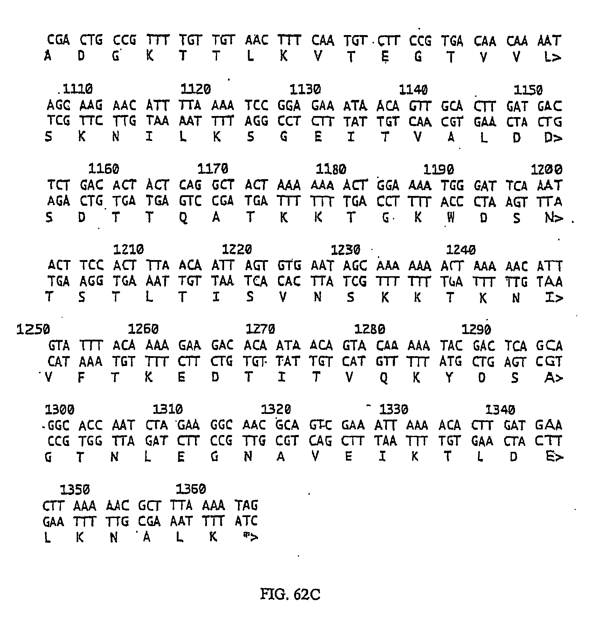

Figs. 62A, 62B and 62C depict the nucleic acid sequence of the OspC-B31 (bp 55-633)/OspA-K48 (bp 52-654)/OspA-Tro (bp 655-819) chimer (SEQ ID NO. 159) and the encoded chimeric protein sequence (SEQ ID NO. 160).

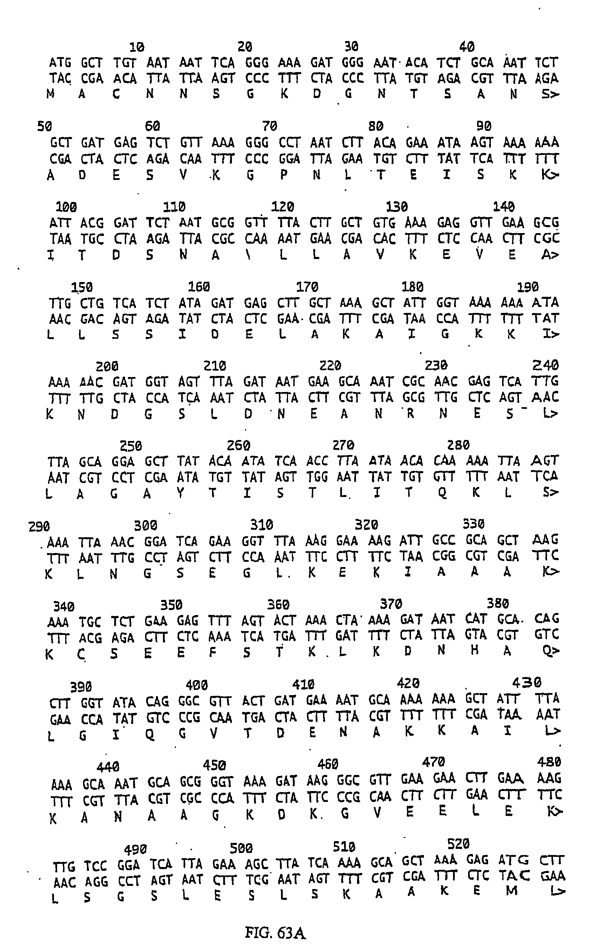

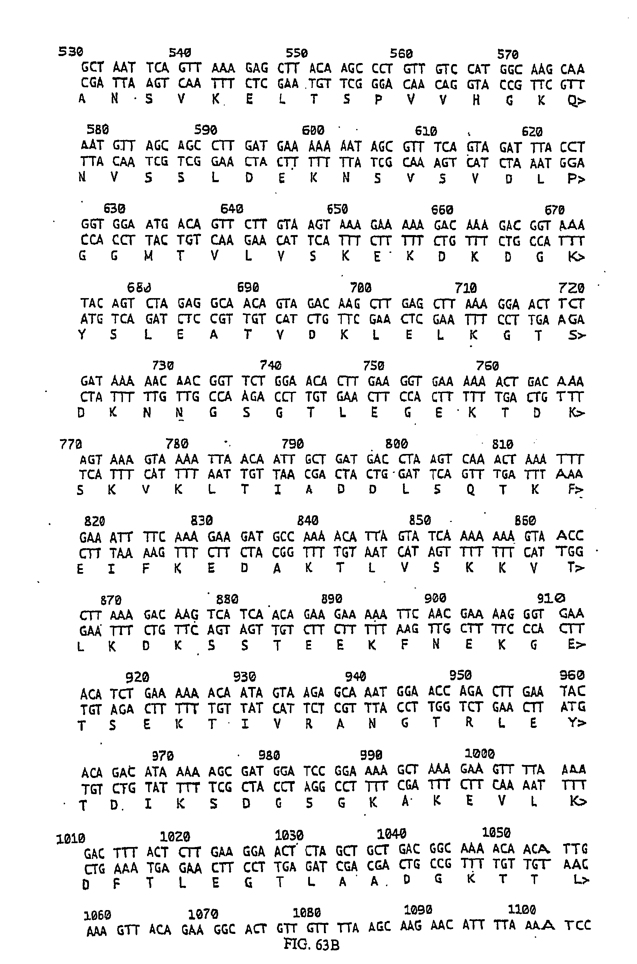

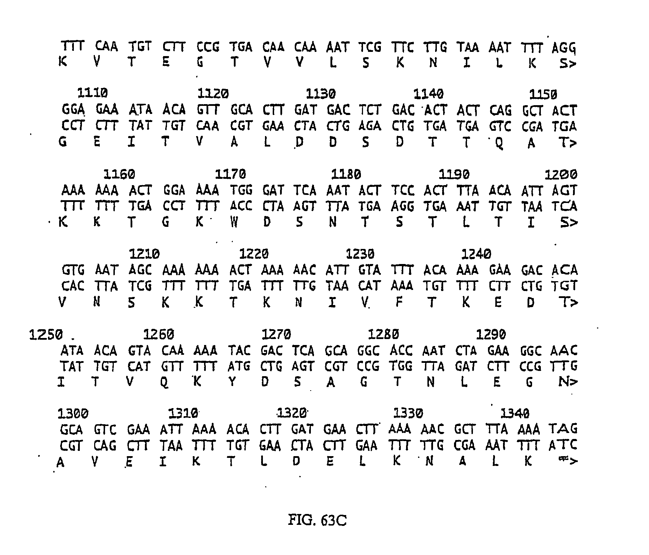

Figs. 63A, 63B and 63C depict the nucleic acid sequence of the OspC-C2 (bp 55-612)/OspA-K48 (bp 52-654)/OspA-Tro (bp 655-819) chimer (SEQ ID NO. 161) and the encoded chimeric protein sequence (SEQ ID NO. 162).

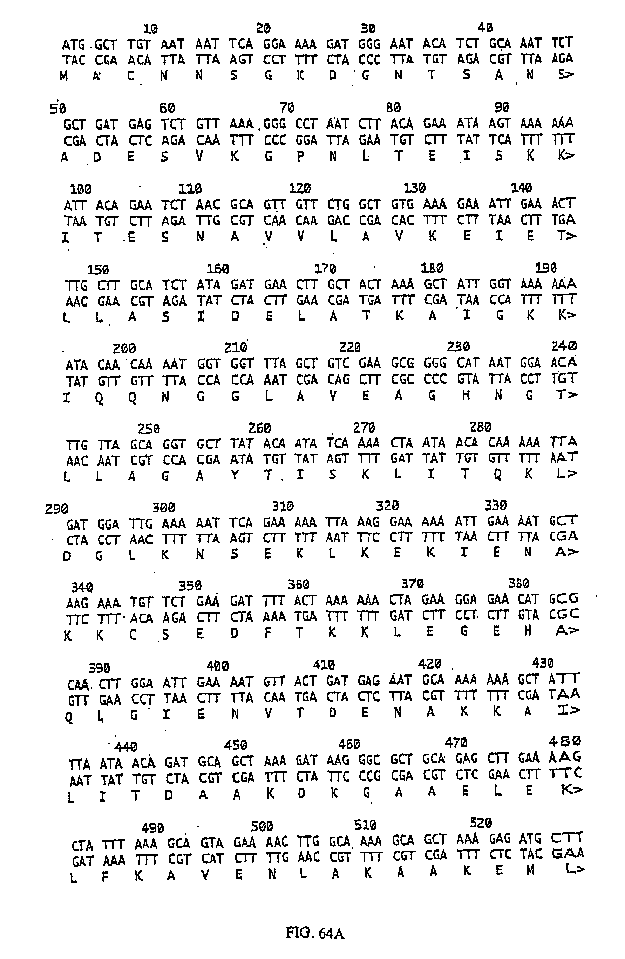

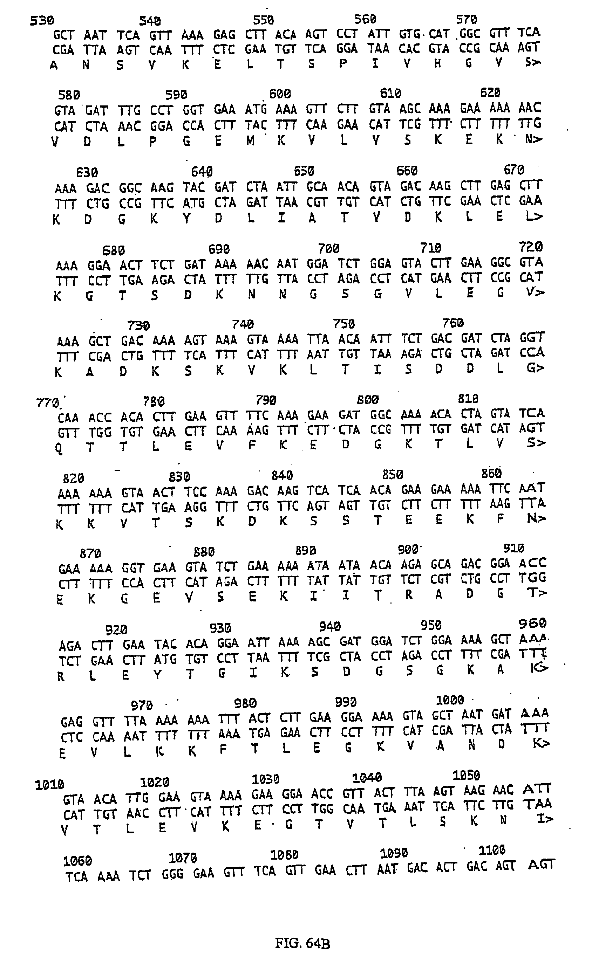

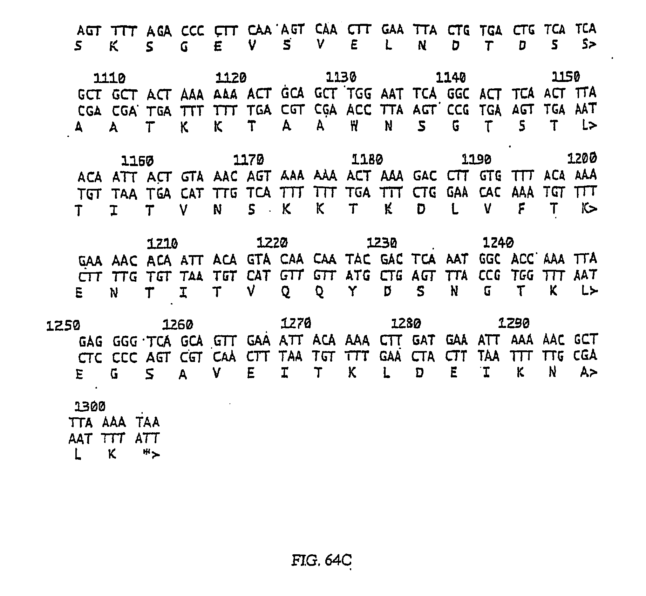

Figs. 64A, 64B and 64C depict the nucleic acid sequence of the OspC-C12 (bp 55-612)/OspA-B31 (bp 88-492)/OspA-PKo (bp 493-537)/OspA-B31 (bp 538-822) chimer (SEQ ID NO. 163) and the encoded chimeric protein sequence (SEQ ID NO. 164).

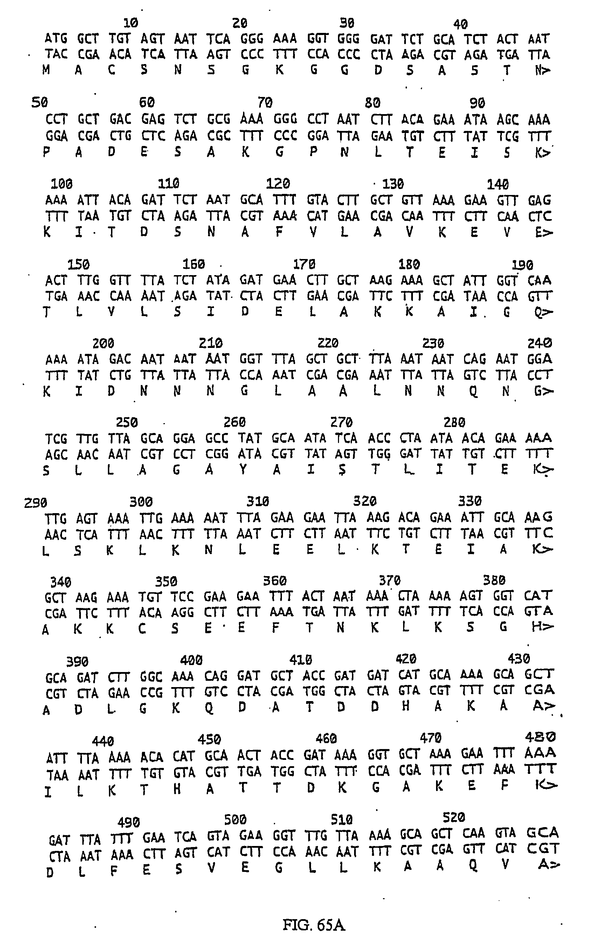

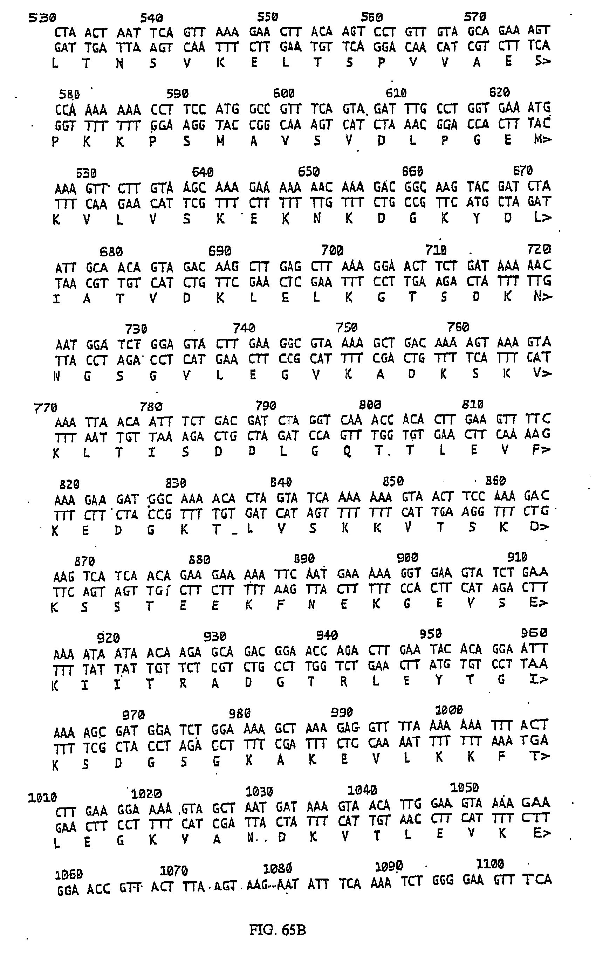

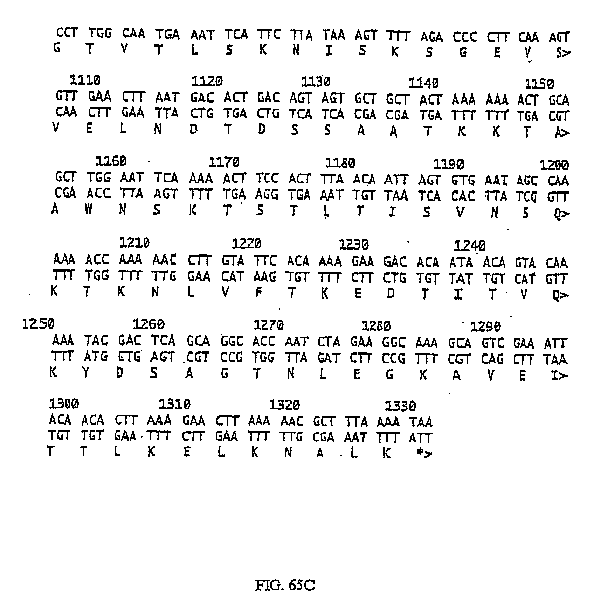

Figs. 65A, 65B and 65C depict the nucleic acid sequence of the OspC-PKo (bp 55-639)/OspA-B31 (bp 88-492)/OspA-PKo (bp 493-537)/OspA-B31 (bp 538-651)/OspA-K48 (bp 652-825) chimer (SEQ ID NO. 165) and the encoded chimeric protein sequence (SEQ ID NO. 166).

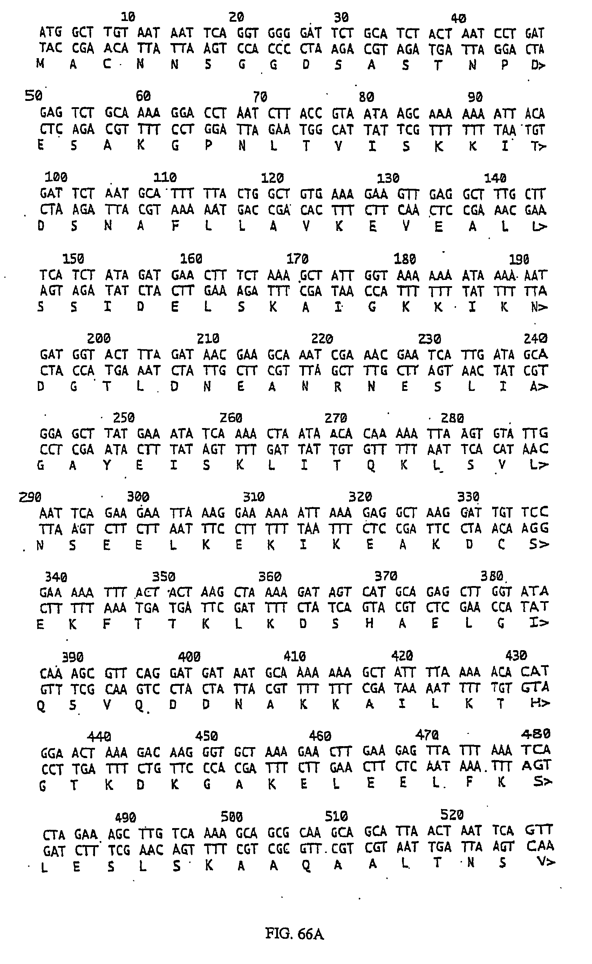

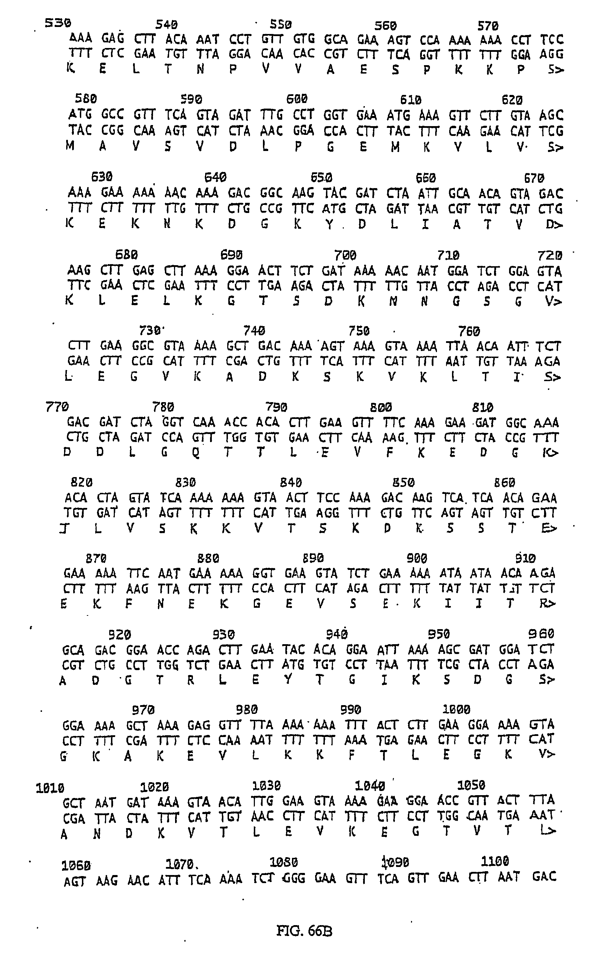

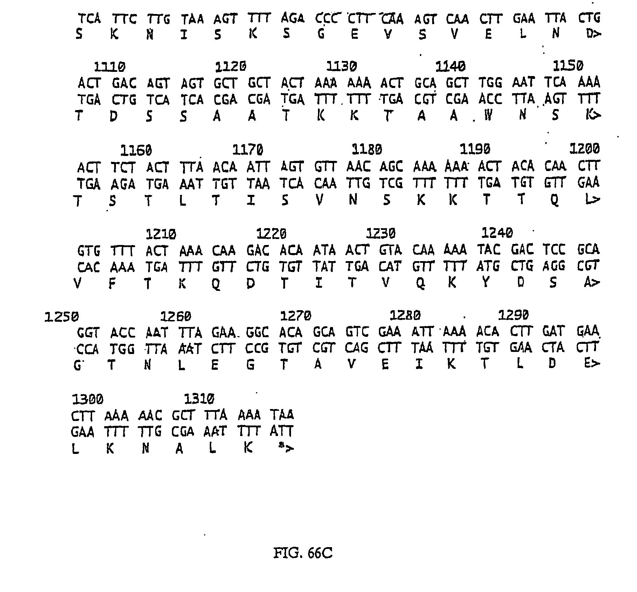

Figs. 66A, 66B and 66C depict the nucleic acid sequence of the OspC-Tro (bp 55-624)/OspA-B31 (bp 88-492)/OspA-PKo (bp 493-537)/OspA-B31 (bp 538-651)/OspA-PKo (bp 652-822) chimer (SEQ ID NO. 167) and the encoded chimeric protein sequence (SEQ ID NO. 168).

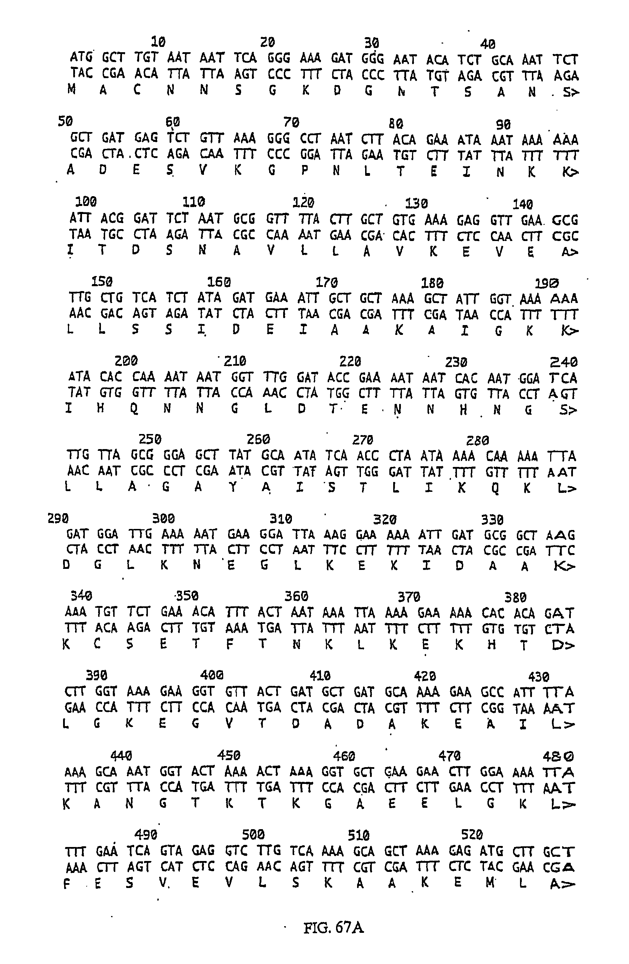

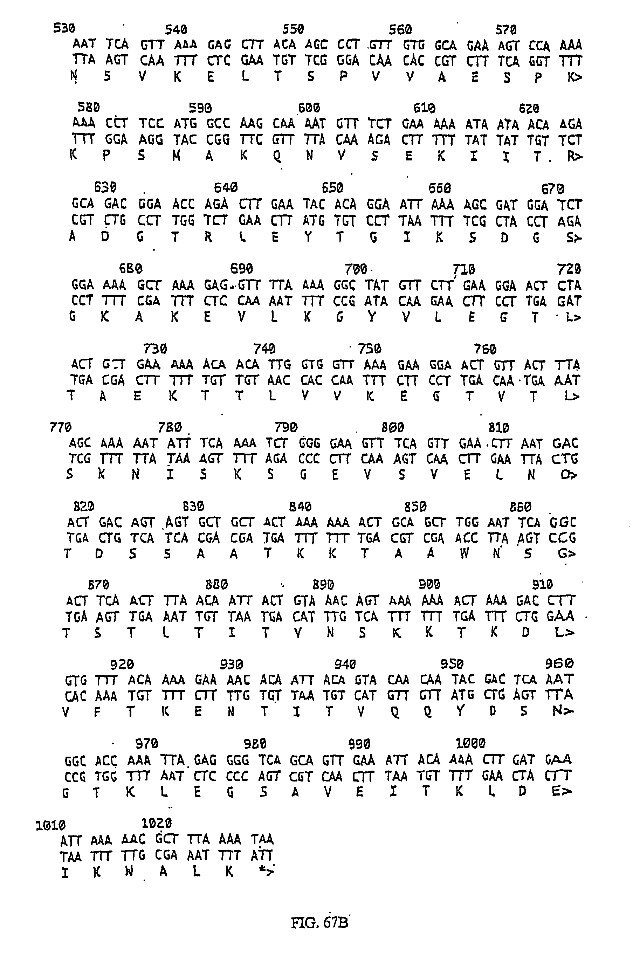

Figs. 67A and 67B depict the nucleic acid sequence of the OspC-B31 (bp 55-633)/OspA-B31 (bp 394-820) chimer (SEQ ID NO. 169) and the encoded chimeric protein sequence (SEQ ID NO. 170).

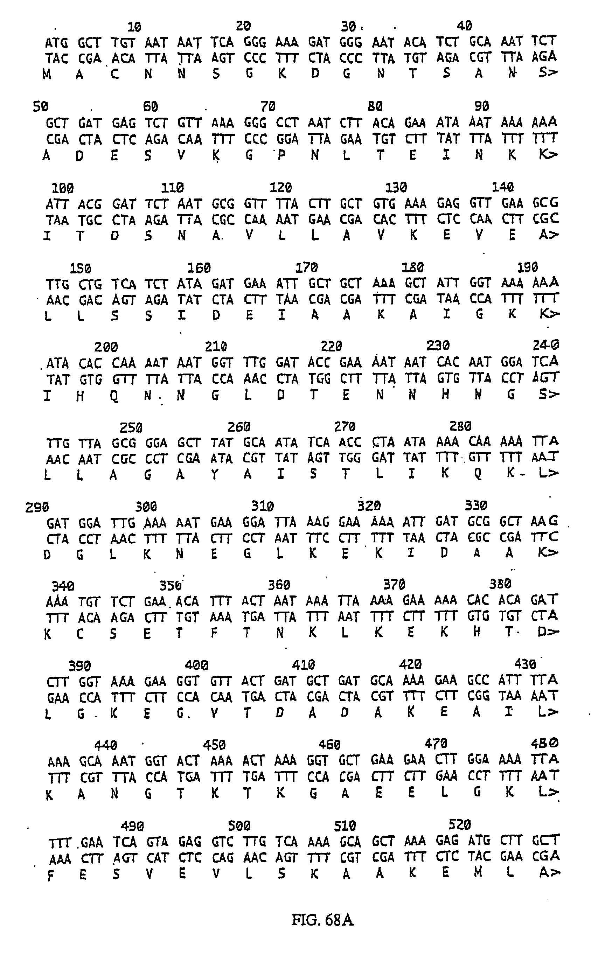

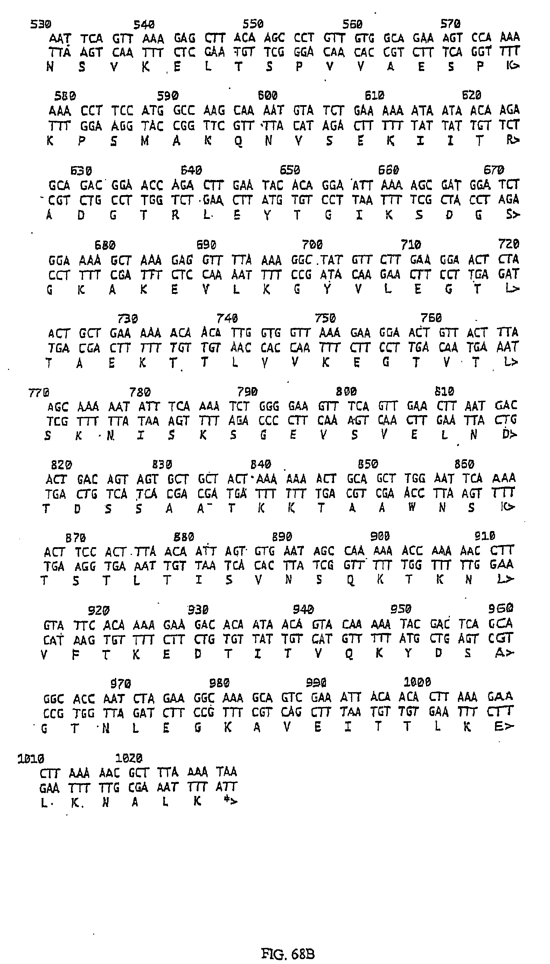

Figs. 68A and 68B depict the nucleic acid sequence of the OspC-B31 (bp 55-631)/OspA-B31 (bp 394-651)/OspA-K48 (bp 652-820) chimer (SEQ ID NO. 171) and the encoded chimeric protein sequence (SEQ ID NO. 172).

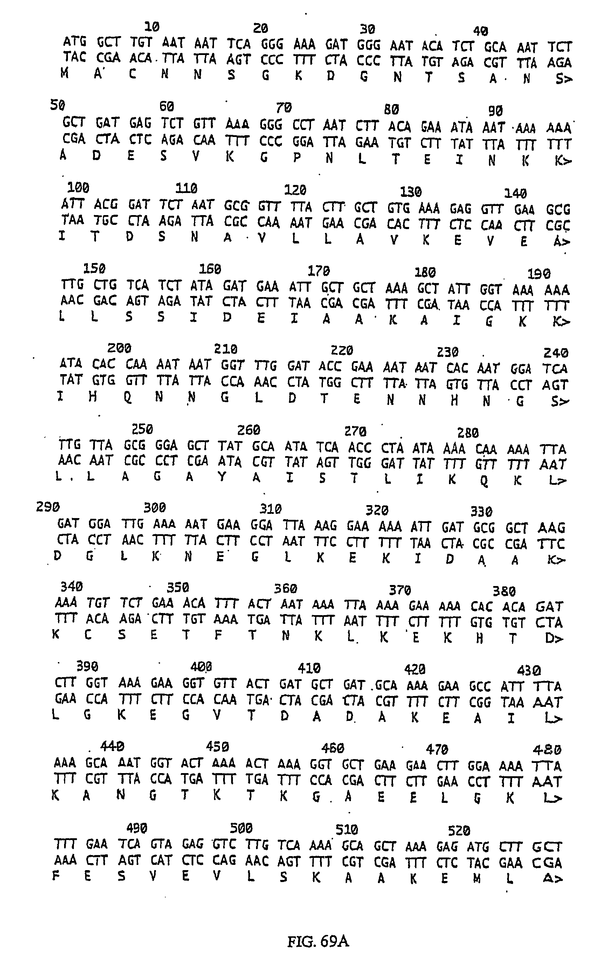

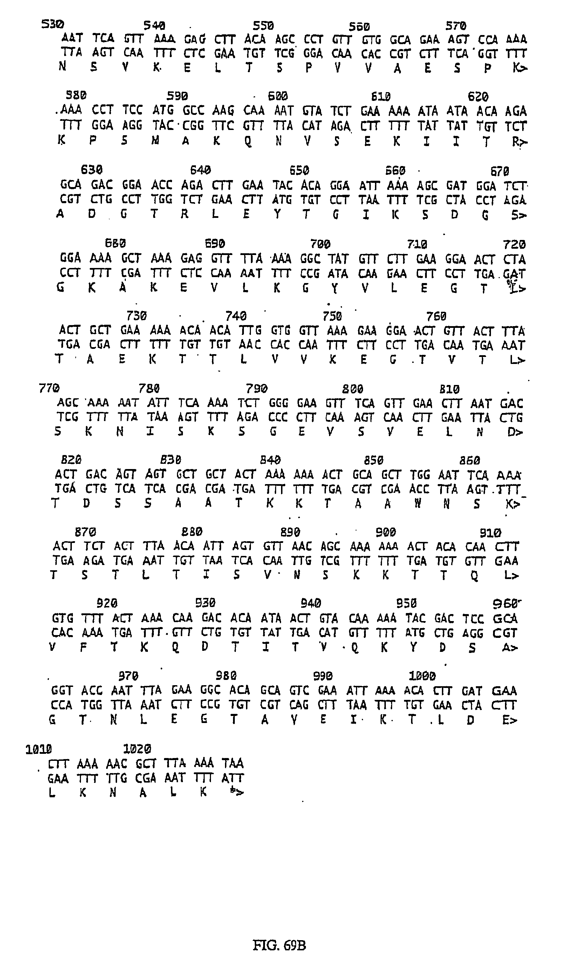

Figs. 69A and 69B depict the nucleic acid sequence of the OspC-B31 (bp 55-633)/OspA-B31 (bp 394-651)/OspA-PKo (bp 652-820) chimer (SEQ ID NO. 173) and the encoded chimeric protein sequence (SEQ ID NO. 174).

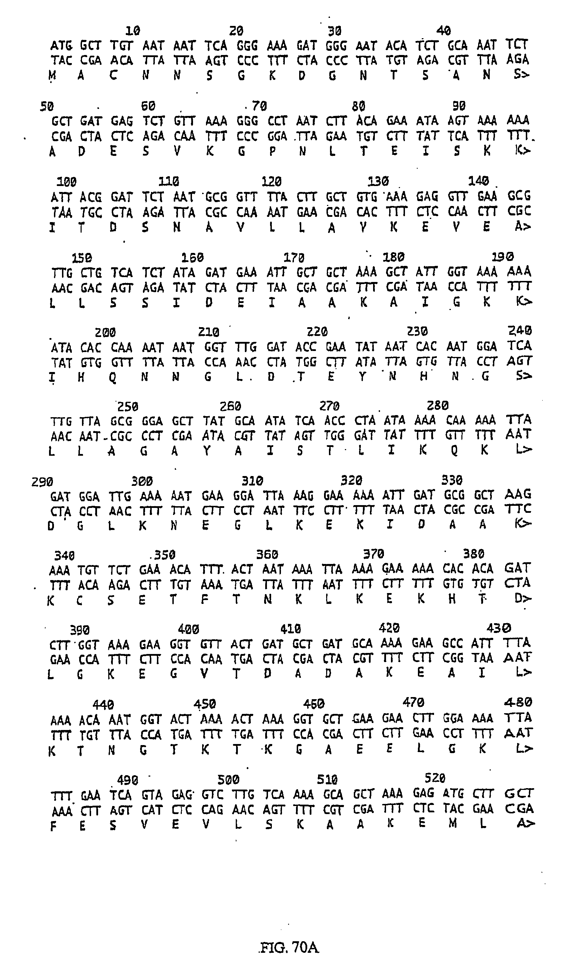

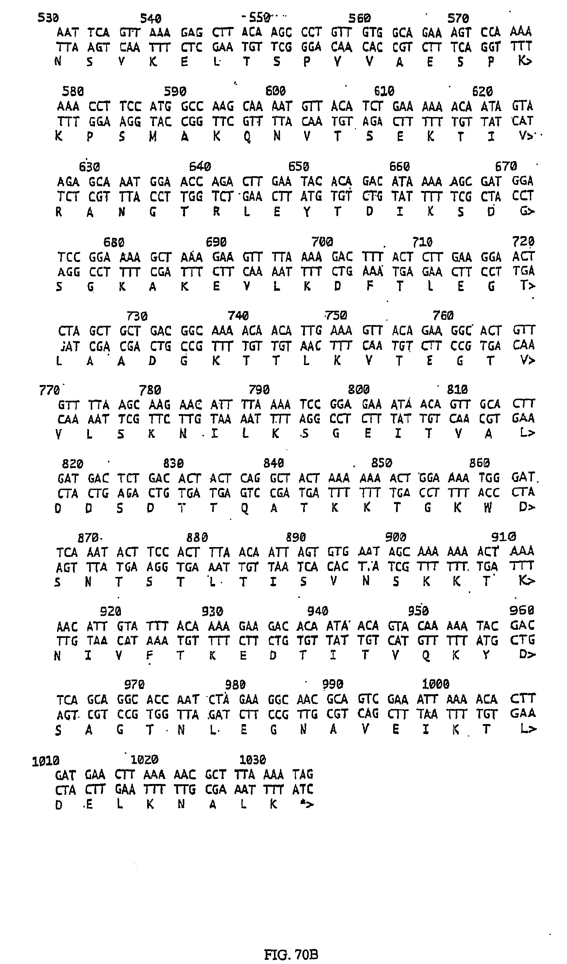

Figs. 70A and 70B depict the nucleic acid sequence of the OspC-B31 (bp 55-633)/OspA-K48 (bp 394-654)/OspA-Tro (bp 655-819) chimer (SEQ ID NO. 175) and the encoded chimeric protein sequence (SEQ ID NO. 176).

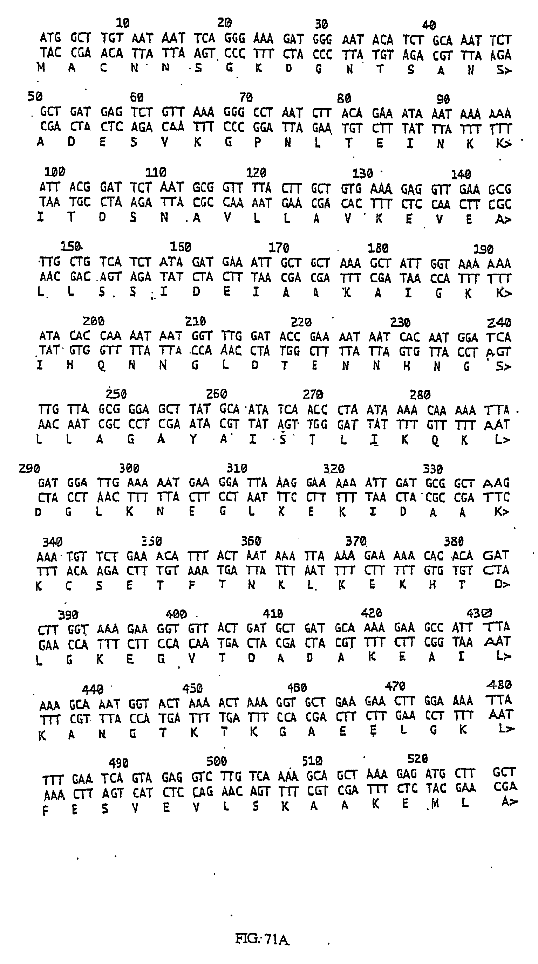

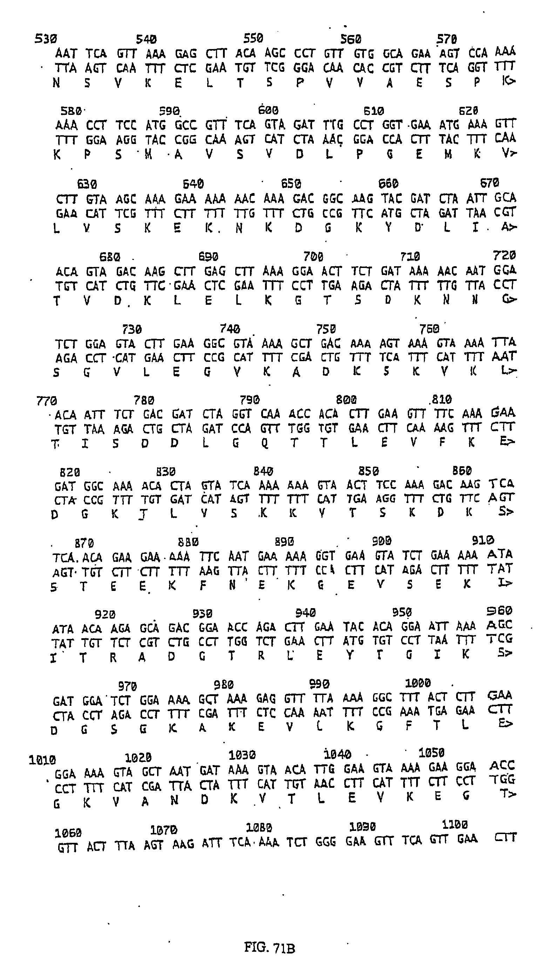

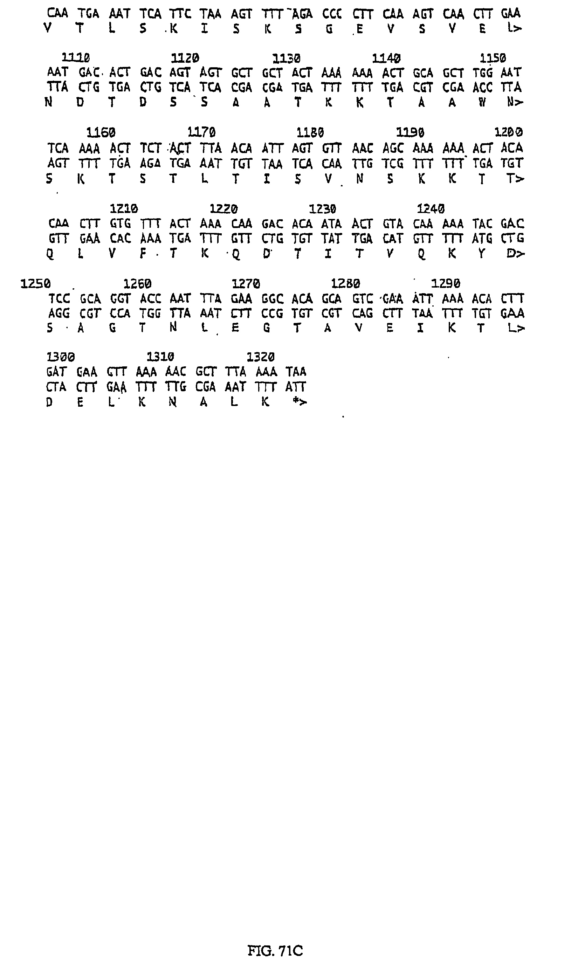

Figs. 71A, 71B and 71C depict the nucleic acid sequence of the OspC-B31 (bp 55-633)/OspA-B31 (bp 88-492)/OspA-PKo (bp 493-537)/OspA-B31 (bp 541-651)/OspA-PKo (bp 652-822) chimer (SEQ ID NO. 177) and the encoded chimeric protein sequence (SEQ ID NO. 178); a variant of this sequence was also generated, where the N at position 190 of B31 OspA was deleted.

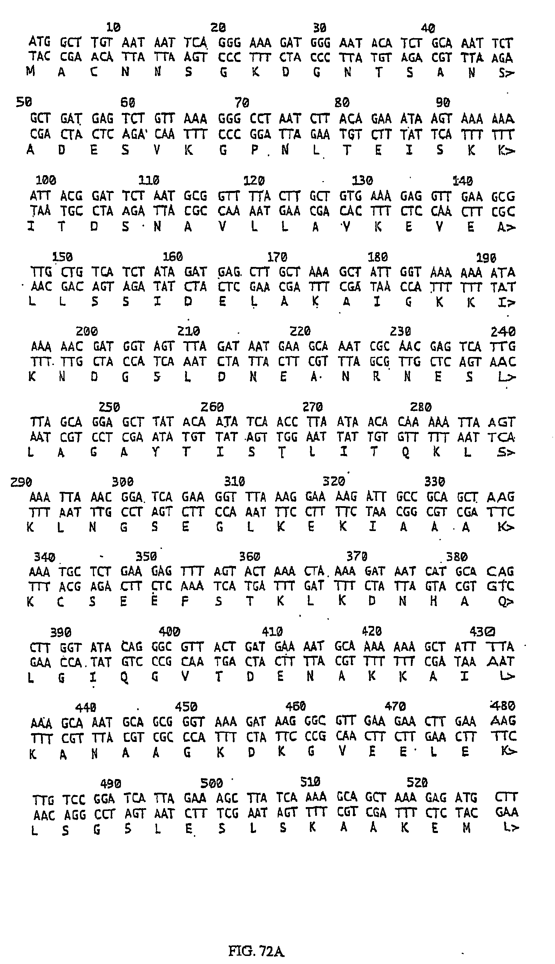

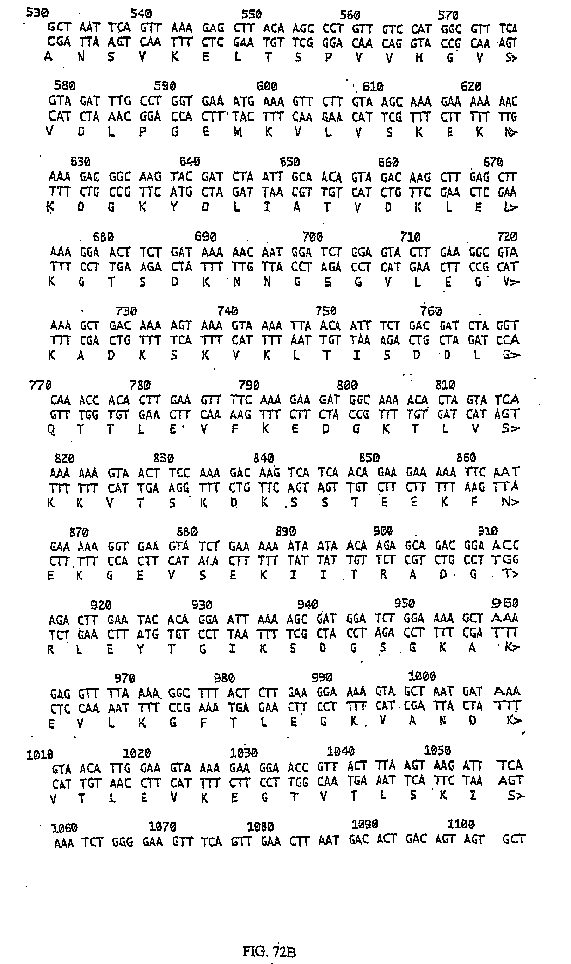

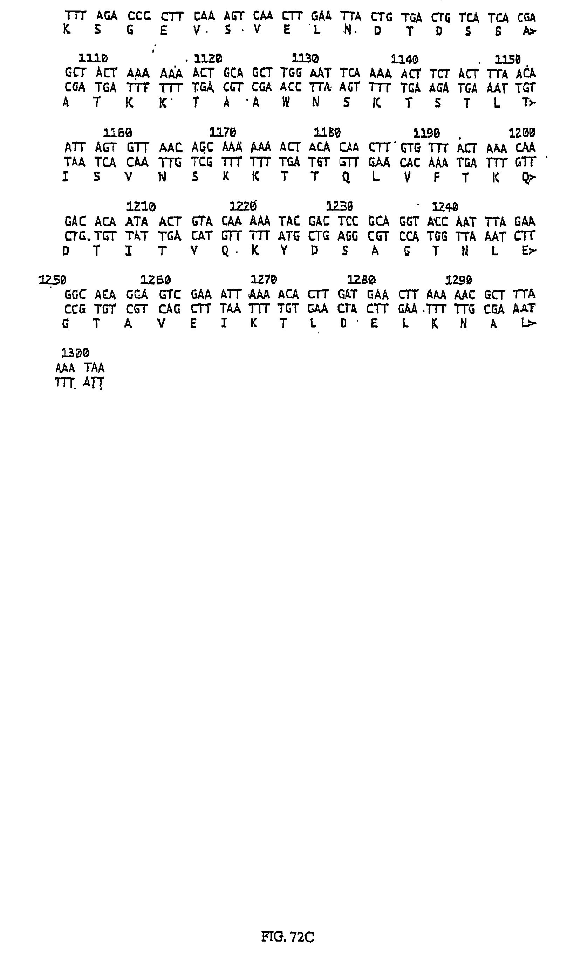

Figs. 72A, 72B and 72C depict the nucleic acid sequence of the OspC-C2 (bp 55-612)/OspA-B31 (bp 88-492)/OspA-PKo (bp 493-537)/OspA-B31 (bp 541-651)/OspA-PKo (bp 652-822) chimer (SEQ ID NO. 179) and the encoded chimeric protein sequence (SEQ ID NO. 180); a variant of this sequence was also generated, where the N at position 190 of B31 OspA was deleted.

### DETAILED DESCRIPTION OF THE INVENTION

The present invention pertains to chimeric proteins comprising various antigenic *Borrelia* polypeptides. The chimeric protein comprises *Borrelia* outer surface protein C (OspC) and outer surface protein A (OspA). These chimeric proteins have the general structure of OspC linked to OspA via a peptide bond. Each of the OspA and OspC portions of the chimeric OspC/OspA protein can be lipidated or unlipidated. The OspC/OspA chimer comprises OspC and OspA polypeptide fragments that do not possess their lipidation signals.

The chimeric forms of the OspA and OspC proteins described herein were bioengineered such that the resultant chimeric protein maintained at least some antigenicity of one or both of the parent molecules. As described herein, antigenic refers to the ability of a compound to bind products of an immune response, such as antibodies, T-cell receptors or both. Such responses can be measured using standard antibody detection assays, such as ELISA or standard T-cell activation assays. In a particular embodiment, the chimeric OspC/OspA proteins comprise OspA polypeptides which lack the putative autoreactive domain that has similarity to a region of human leukocyte function-associated antigen-1 (hLFA-1) (Gross, D.M. et al., Science, 281: 703-706 (1998)).

The current invention pertains to chimeric proteins comprising antigenic *Borrelia* polypeptides which do not occur in nature in the same *Borrelia* protein. The chimeric proteins are a combination of two or more antigenic polypeptides derived from *Borrelia* proteins. The antigenic polypeptides can be derived from different proteins from the same species of *Borrelia,* or different proteins from different *Borrelia* species, as well as from corresponding proteins from different species. As used herein, the term "chimeric protein" describes a protein comprising two or more polypeptides which are derived from corresponding and/or non-corresponding native *Borrelia* protein. A polypeptide "derived from" a native *Borrelia* protein is a polypeptide which has an amino acid sequence the same as an amino acid sequence present in a *Borrelia* protein, an amino acid sequence equivalent to the amino acid sequence of a naturally occurring *Borrelia* protein, or an amino acid sequence substantially similar to the amino acid sequence of a naturally occurring *Borrelia* protein (e.g., differing by a few amino acids), such as when a nucleic acid encoding a protein is subjected to site-directed mutagenesis. "Corresponding" proteins are equivalent proteins from different species or strains of *Borrelia,* such as outer surface protein A (OspA) from strain B31 and OspA from strain K48. The invention additionally pertains to nucleic acids encoding these chimeric proteins.

In one embodiment, the present invention is drawn to chimeric proteins comprising antigenic polypeptides from Lyme Disease-causing strains of *Borrelia.* In another embodiment, the chimeric proteins described herein comprise antigenic polypeptides from different pathogenic genospecies of *Borrelia,* such as *Borrelia burgdorferi sensu stricto, Borrelia afzelii* and *Borrelia garinii.* In a preferred embodiment, the chimeric proteins comprise antigenic polypeptides from each of the pathogenic genospecies of *Borrelia,* including *Borrelia burgdorferi sensu stricto, Borrelia afzelii* and *Borrelia garinii.*

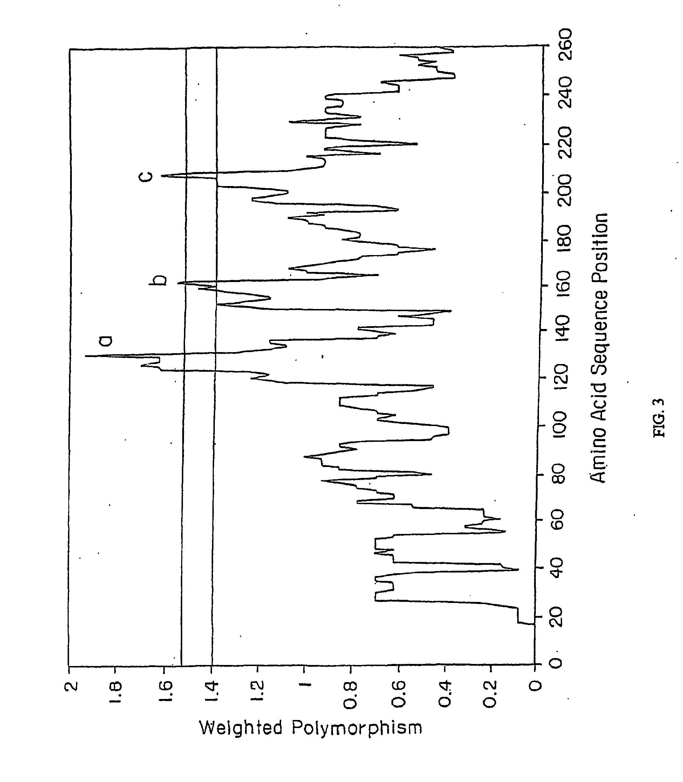

The OspA portion of the chimeric molecules of the present invention can themselves be chimeric combinations of more than one OspA polypeptide. Similarly, the OspC portion of the chimeric molecules of the present invention can themselves be chimeric combinations of more than one OspC polypeptide. As described below, Applicants have identified two separate antigenic domains of OspA and OspB which flank the sole conserved tryptophan present in OspA and in OspB. These domains share cross-reactivity with different genospecies *of Borrelia.* The precise amino acids responsible for antigenic variability were determined through site-directed mutagenesis, so that proteins with specific amino acid substitutions are available for the development of chimeric versions of OspA which can be included in the OspC/OspA chimeric proteins of the present invention. Furthermore, Applicants have identified immunologically important hypervariable domains in OspA proteins, as described below in Example 2. The first hypervariable domain of interest for chimeric proteins, Domain A, includes amino acid residues 120-140 of OspA, the second hypervariable domain, Domain B, includes residues 150-180 and the third hypervariable domain, Domain C, includes residues 200-216 or 217 (depending on the position of the sole conserved tryptophan residue in the OspA of that particular species of *Borrelia)* (see Fig. 3). In addition, Applicants have sequenced the genes for several *Borrelia* proteins.

These discoveries have aided in the development of novel recombinant *Borrelia* proteins which include two or more amino acid regions or sequences which do not occur in the same *Borrelia* protein in nature. The recombinant proteins comprise polypeptides from a variety *of Borrelia* proteins, including, but not limited to, OspA, OspB, OspC, OspD, p12, p39, p41, p66, and p93. Preferred combinations include all or a portion of OspC linked to all or a portion of OspA. Antigenically relevant polypeptides from each of a number of proteins are combined into a single chimeric protein.

In one embodiment of the current invention, chimeras are now available which include antigenic OspA polypeptides flanking a tryptophan residue. OspB has a similar primary structure as OspA and is included in the following discussion. The antigenic polypeptides are derived from either the proximal portion from the tryptophan (the portion of the OspA protein present between the amino terminus and the conserved tryptophan of the protein), or the distal portion from the tryptophan (the portion of the OspA protein present between the conserved tryptophan of the protein and the carboxy terminus) in OspA. The resultant chimeras can be OspA-OspA chimeras (e.g., chimeras incorporating polypeptides derived from OspA from different strains of Borrelia), OspA-OspB chimeras, or OspB-OspB chimeras, and are constructed such that amino acid residues amino-proximal to an invariant tryptophan are from one protein and residues carboxy-proximal to the invariant tryptophan are from the other protein. For example, one available chimer consists of a polypeptide derived from the amino-proximal region of OspA from strain B31, followed by the tryptophan residue, followed by a polypeptide derived from the carboxy-proximal region of OspA from strain K48 (SEQ ID NO. 92). Another available chimer includes a polypeptide derived from the amino-proximal region of OspA from strain B31, and a polypeptide derived from the carboxy-proximal region of OspB from strain B31 (SEQ ID NO. 104). If the polypeptide proximal to the tryptophan of these chimeric proteins is derived from OspA, the proximal polypeptide can be further subdivided into the three hypervariable domains (Domains A, B, and C), each of which can be derived from OspA from a different strain *of Borrelia.* These chimeric proteins further comprise antigenic polypeptides from OspC, in addition to the antigenic polypeptides flanking the tryptophan residue.

The chimeric OspC/OspA proteins of the present invention comprise at least a first and a second polypeptide, wherein the first polypeptide comprises *Borrelia burgdorferi* OspC and wherein the second polypeptide comprises *Borrelia burgdorferi* OspA, such that OspC comprises the N-terminus of the protein.

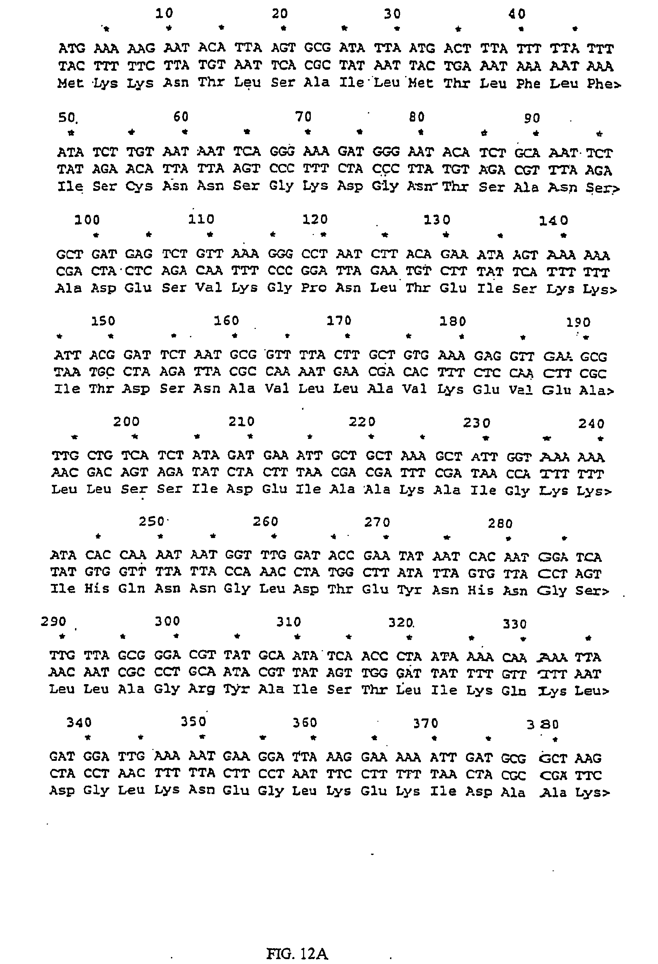

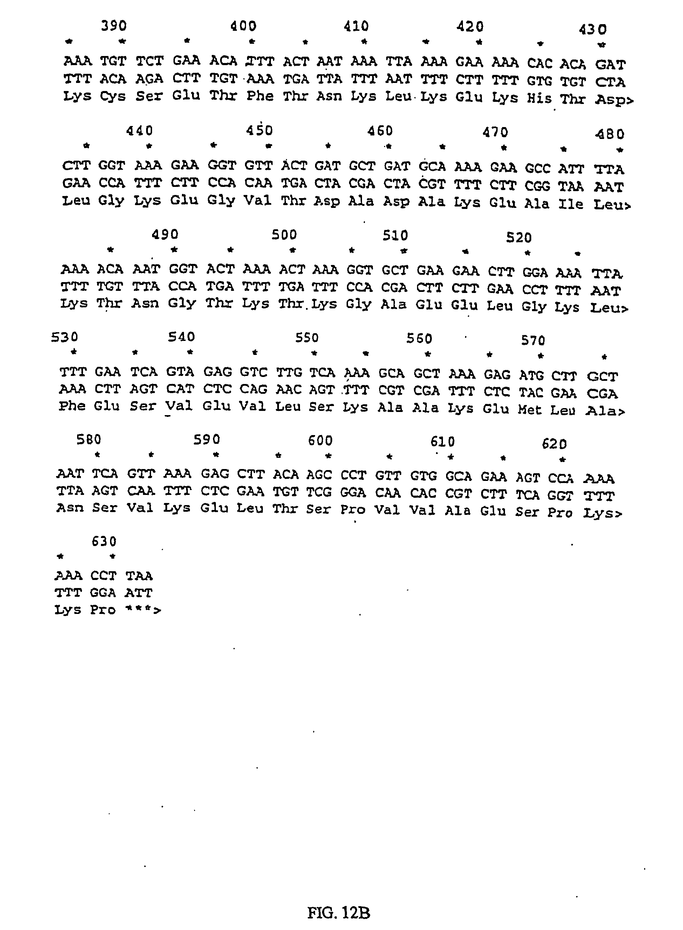

The first polypeptide comprises a *Borrelia burgdorferi* OspC polypeptide from about amino acid residue 19 to about amino acid residue 213, and the second polypeptide comprises a *Borrelia burgdorferi* OspA polypeptide. In another embodiment, the first polypeptide comprises a *Borrelia burgdorferi* OspC polypeptide from about amino acid residue 19 to about amino acid residue 211. In another embodiment, the first polypeptide comprises a *Borrelia burgdorferi* OspC polypeptide from about amino acid residue 19 to about amino acid residue 208. In another embodiment, the first polypeptide comprises a *Borrelia burgdorferi* OspC polypeptide from about amino acid residue 19 to about amino acid residue 204. The numbering of the OspC residues is according to the numbering of SEQ ID NO: 30 (Figs. 12 A and 12B). It is evident that the person of skill in the art recognizes that OspC genes from different strains and/or genospecies may differ in their primary sequence and that based on homology, similar regions of such OspC proteins could be identified and used in the present invention with no or only routine experimentation.

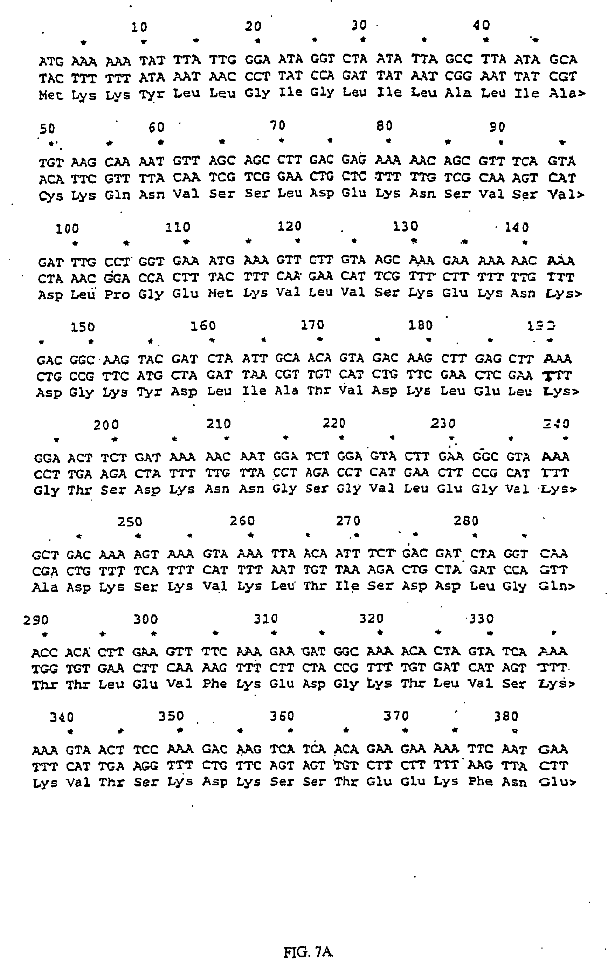

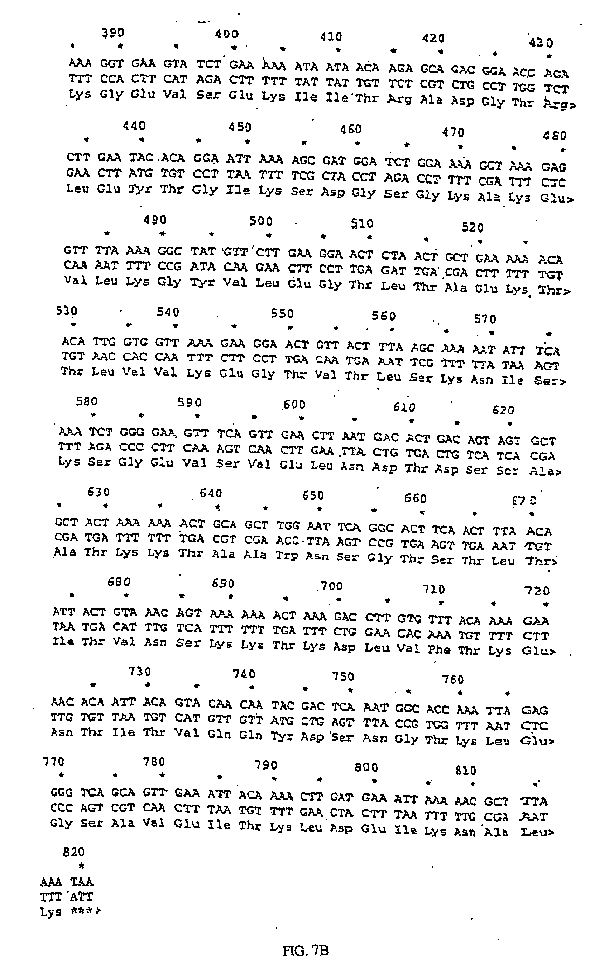

In one embodiment, the invention is drawn to chimeric OspC/OspA proteins wherein the first polypeptide comprises a *Borrelia burgdorferi* OspC polypeptide and the second polypeptide comprises a *Borrelia burgdorferi* OspA polypeptide from about amino acid residue 18 to about amino acid residue 273. In other embodiments, the chimeric OspC/OspA protein comprises a first polypeptide which is a *Borrelia burgdorferi* OspC polypeptide and a second polypeptide which is a *Borrelia burgdorferi* QspA polypeptide selected from the group consisting of an OspA polypeptide from about amino acid residue 132 to about amino acid residue 273, an OspA polypeptide from about amino acid residue 18 to about amino acid residue 273. The numbering of the OspA residues is according to the numbering of SEQ ID NO: 7 (Fig. 7A and 7B). It is evident that the person of skill in the art recognizes that OspA genes from different strains and/or genospecies may differ in their primary sequence and that based on homology, similar regions of such OspA proteins could be identified and used in the present invention with no or only routine experimentation.

The present invention is also drawn to OspC/OspA chimeric proteins wherein the first polypeptide comprises a *Borrelia burgdorferi* OspC polypeptide and the second polypeptide comprises a *Borrelia burgdorferi* OspA polypeptide, wherein the OspA polypeptide comprises two or more OspA polypeptide fragments as described above. The OspA polypeptide comprises portions of OspA from two or more strains of *Borrelia.* In another preferred embodiment, the OspA polypeptide comprises portions of OspA from two or more Lyme Disease-causing genospecies of *Borrelia,* e.g., wherein the genospecies are *Borrelia burgdorferi sensu stricto, Borrelia afzelii* and/or *Borrelia garinii.* In still another preferred embodiment, the OspC/OspA chimeric protein comprises one or more polypeptides from each of the pathogenic genospecies, *Borrelia burgdorferi sensu stricto, Borrelia afzelii* and *Borrelia garinii.*

The chimeras described herein can be produced so that they are highly soluble, hyper-produced in *E. coli,* and non-lipidated. Lipidated chimeric proteins can also be produced. In addition, the chimeric proteins can be designed to end in an affinity tag (His-tag) to facilitate purification. The recombinant proteins described herein have been constructed to maintain antigenicity of at least one of the parent polypeptides. In addition, recombinant proteins specific for the various genospecies of *Borrelia* that cause Lyme disease are now available, because the genes from each of the major genospecies have been sequenced. These recombinant proteins with their novel biophysical and antigenic properties will be important diagnostic reagent and vaccine candidates.

The chimeric proteins of the current invention are advantageous in that they retain at least some specific reactivity to monoclonal or polyclonal antibodies that recognize wild-type *Borrelia* proteins. The proteins are immunogenic, and elicit antibodies that inhibit growth and/or induce lysis of *Borrelia in vitro.* Furthermore, in some embodiments, the proteins provide antigenic domains of two or more *Borrelia* strains and/or proteins within a single protein. Such proteins are particularly useful in immunodiagostic assays. For example, proteins of the present invention can be used as reagents in assays to detect the presence of antibodies to native *Borrelia* in potentially infected individuals. These proteins can also be used as immunodiagnostic reagents, such as in dot blots, Western blots, enzyme-linked immunosorbent assays (ELISA), or agglutination assays. The chimeric proteins of the present invention can be produced by known techniques, such as by recombinant methodology, polymerase chain reaction, or mutagenesis.

Furthermore, the proteins of the current invention are useful as vaccine immunogens against *Borrelia* infection. Because *Borrelia* has been shown to be clonal, a protein comprising antigenic polypeptides from a variety of *Borrelia* proteins and/or species, will provide immunoprotection for a considerable time when used in a vaccine. The lack of significant intragenic recombination, a process which might rapidly generate novel epitopes with changed antigenic properties, ensures that *Borrelia* can only change antigenic type by accumulating mutational change, which is slow when compared with recombination in generating different antigenic types. The chimeric protein can be combined with a physiologically-acceptable carrier and administered to a vertebrate animal through standard methods (e.g., intravenously or intramuscularly, for example).

In addition to the chimeric proteins described herein, the present invention is also drawn to nucleic acids which encode the *Borrelia* chimeric protein described herein. In one embodiment of the present invention, the composition comprises a nucleic acid that encodes a chimeric protein of at least two polypeptides, wherein the first polypeptide comprises *Borrelia burgdorferi* OspC, and the second polypeptide comprises *Borrelia burgdorferi* OspA, such that OspC is upstream of OspA. The OspC and OspA nucleic acid fragments that make up the chimeric protein can be from the same strain or genospecies *of Borrelia,* from different strains or genospecies *of Borrelia,* or from combinations of nucleic acids that are from the same and/or different strains or genospecies *of Borrelia.*

It is understood that the nucleic acids that encode the polypeptides that comprise the chimeric protein can include extra nucleotides or fewer nucleotides in order to simplify the construction of the gene encoding the chimeric polypeptide, e.g., to allow for the use of convenient restriction endonuclease sites or to allow the ligation of the gene fragments such that a contiguous coding region is created. Based on the guidance provided herein, one of ordinary skill in the art would readily be able to add or remove nucleotides from the termini of the gene fragments encoding the polypeptides of the chimeric OspC/OspA protein in order to generate the chimeric proteins of the present invention with no or only routine experimentation. Furthermore, there can be an extra about 1 to about 10 amino acids on the N- and/or C-terminus of the polypeptides and chimeric proteins of the present invention and still retain the properties of the present invention. It is also understood that those of skill in the art, using art-known methods and/or the methods described herein, could generate additional OspC-OspA chimeric proteins, and that these chimeric proteins are encompassed by the invention.

The present invention is also drawn to expression vectors which comprise an isolated DNA encoding the *Borrelia* chimeric protein described herein. The composition includes an expression vector comprising an isolated DNA which encodes an OspC/OspA chimeric protein, wherein the OspC portion of the protein is upstream of the OspA portion. The present invention also encompasses host cells which comprise a recombinant nucleic acid that encodes an OspC/OspA chimeric protein, as described herein.

The present invention is also drawn to methods of making the *Borrelia* chimeric polypeptides described herein. The method of making a chimeric *Borrelia* protein comprises selecting a polynucleotide sequence encoding OspC, or an antigenic portion thereof, selecting a polynucleotide sequence encoding OspA, or an antigenic portion thereof, and ligating these polynucleotide sequences together, such that OspC comprises the N-terminus of the protein. The polypeptides of the present invention can also be recombinantly expressed in suitable microbial hosts, wherein said hosts include, but are not limited to, bacterial hosts, such as E. *coli,* fungal hosts, such as *S. cerevisiae* or cell culture hosts, such as those of mammalian cell culture or insect cell culture.

The present invention may also be used in methods of delivering the *Borrelia* chimeric polypeptides described herein. The method comprises administering the chimeric protein in a physiologically-acceptable carrier to an individual. The individual develops at least some immune response to the chimeric protein. As an example, the individual could generate a humoral immune response, wherein antibodies that recognize at least a portion of said chimeric polypeptide are produced by the individual. The antibodies that recognize the chimeric polypeptide can be of any class of immunoglobulin, such as IgM, IgD, IgA and IgG or combinations thereof.

The present invention may also be used in methods of delivering a nucleic acid which encodes a chimeric polypeptide described herein. The method comprises administering the nucleic acid in a physiologically-acceptable carrier to an individual using art-accepted methods of DNA delivery, including but not limited to, biolistic delivery and lipid encapsulation. The chimeric polypeptide is at least transiently expressed and the individual develops at least some immune response to the chimeric protein encoded by the nucleic acid.

The invention also encompasses methods of using the chimeric proteins described herein in diagnostic assays. In one embodiment, the method can be used to detect the presence of OspA- and/or OspC-specific antibodies in a sample, e.g., a host sample of interest. In one embodiment, the method comprises contacting a host sample of interest with the chimeric OspC/OspA protein, under conditions, wherein antibodies, ifpresent in the host sample, bind to the chimeric protein thereby forming antigen-antibody complexes. The antigen-antibody complexes are then detected. In this manner, an immune response to Lyme-Disease causing *Borrelia* can be detected.

As described herein, the chimeric proteins of the present invention incorporate antigenic domains from different *Borrelia* proteins, as well as from different *Borrelia* strains and/or genospecies. As such, they are useful in the detection or diagnosis of the presence of Lyme disease-causing *Borrelia,* especially *Borrelia* from groups capable of causing disseminated symptoms of Lyme disease. Disseminated symptoms refer to infection outside of the erythema migrans skin lesion, e.g., infection in blood, CNS or synovia.

The chimeric polypeptides of the present invention elicit specific immune responses to OspC and OspA. In one embodiment, the chimeric polypeptides elicit immune responses to stains of Lyme disease-causing *Borrelia* of the same genospecies as that represented by the OspC/OspA chimeric protein. In another embodiment, the chimeric polypeptides elicit immune responses to strains of Lyme disease-causing *Borrelia* of different genospecies than that represented by the OspC/ospA chimeric protein, as well as to Lyme disease-causing *Borrelia* of the same genospecies as that represented by the OspC/OspA chimeric protein. The immune response includes, but is not limited to, a humoral response, a secretory response, a cell-mediated response, or any combination thereof.

The immunogenic compositions of the present invention can also be used to immunize animals, e.g., mammals, including humans. Immunization is understood to elicit specific immunogenic responses as described herein. In one embodiment, administration of an immunogenic composition, namely an Osp/OspA chimeric protein or an OspC/OspA chimeric nucleic acid, to an animal results in the animal developing immunity to infection by Lyme disease-causing *Borrelia,* e.g., *Borrelia burgdorferi, Borrelia afzelii* and/or *Borrelia garinii.*

Immunity, as described herein, is understood to mean the ability of the treated animal to resist infection (e.g., systemic infection), to overcome infection (e.g., systemic infection) or to overcome infection (e.g., systemic infection) more easily and/or more quickly when compared to non-immunized and/or non-treated individuals. Immunity can also include an improved ability of the treated individual to sustain an infection with reduced or no clinical symptoms of systemic infection. The individual may be treated with the chimeric proteins of the present invention either proactively, e.g., once a year or alternatively after sustaining a tick bite.

In one embodiment, the OspC/OspA chimeric protein of the present invention, together with suitable excipients and/or adjuvants, is administered to an animal such that the animal develops an immune response to at least one of the OspC and/or OspA polypeptides of the composition. The pharmaceutical composition can also be administered with other components suitable for *in vitro* and/or *in vivo* use. These additional components include buffers, carrier proteins, adjuvants, excipients, preservatives and combinations thereof.

The present invention is also drawn to pharmaceutical compositions which can be used to vaccinate and/or treat *Borrelia* infection in an animal or human. In a particular embodiment, the pharmaceutical composition comprises a *Borrelia burgdorferi* OspC/OspA chimeric protein. The pharmaceutical composition can also be administered together with a physiologically-acceptable carrier, an excipient and/or an adjuvant. Suitable adjuvants are well known in the art (see for example PCT Publication WO 96/40290, the entire teachings of which are incorporated herein by reference), and can be used, for example, to enhance immunogenicity, potency or half-life of the chimeric proteins in the treated animal.

The pharmaceutical compositions used to vaccinate and/or treat *Borrelia* infection can be prepared using methods for preparing vaccines which are well known in the art. For example, the OspC/OspA chimeric proteins described herein can be isolated and/or purified by known techniques, such as by size exclusion chromatography, affinity chromatography, ion exchange chromatography, preparative electrophoresis, selective precipitation or combinations thereof. The prepared chimeric proteins can be mixed with suitable other reagents as described herein, such that the chimeric protein is at a suitable concentration. The dosage of the chimeric protein will vary and depends upon the age, weight and/or physical condition of the animal, e.g., mammal, human, to be treated. The optimal dosage can be determined by routine optimization techniques, using suitable animal models.

Administration of the pharmaceutical composition to be used as a vaccine can be by any suitable technique. Suitable techniques for administration of the pharmaceutical composition include, but are not limited to, injection, e.g., subcutaneous injection, intramuscular injection, intravenous injection, intra peritoneal injection; mucosal administration, e.g., exposing nasal mucosa to nose drops containing the proteins or chimeric proteins of the present invention; oral administration; and DNA immunization.

The incorporation of polypeptide fragments from different strains and/or genospecies of *Borrelia burgdorferi* allows for a greater detection range and a more effective vaccination tool. The present invention provides a chimeric combination of proteins that, when used as a vaccine, can prevent Lyme disease from becoming systemic. The chimeric proteins of the present invention can be effective in preventing Lyme disease, as well as having a therapeutic effect on established infection, for example, after the tick bite is noticed by the patient. Since the chimeric proteins of the present invention comprise both OspC and OspA polypeptides, they are expected to act at the level of the tick as well as the level of the host in preventing both infection and disease due to *Borrelia burgdorferi, Borrelia afzelii* and/or *Borrelia garinii.*

The present invention is also drawn to diagnostic kits which comprise the chimeric polypeptides described herein. The kit comprises a chimeric protein comprising at least a first and a second polypeptide, wherein the first polypeptide comprises *Borrelia burgdorferi* OspC and wherein the second polypeptide comprises *Borrelia burgdorferi* OspA, such that OspC comprises the N-terminus of the protein. The kit also includes reagents for detecting antibody-antigen complexes that are formed between the OspC/OspA chimeric protein and antibodies that are present in a sample, e,g., a user-supplied host sample.

As a result of the present invention, it is now possible to prepare improved diagnostic tools comprising both OspA and OspC antigens from various *Borrelia burgdorferi* strains and/or genospecies. Since OspA is primarily expressed in the tick vector, and OspC is upregulated in response to the feeding of an infected tick on a mammal, the diagnostic compositions of the invention can recognize antigens that are expressed at different stages of the life cycle of *borrelia burgdorferi.* Moreover, by incorporating unique polypeptide fragments from pathogenic families of *Borrelia,* the present invention allows for improved diagnostic compositions which can detect clinically important exposure to pathogenic *Borrelia* while overlooking the remainder of non-pathogenic families *of Borrelia.*

As described herein, the OspC/OspA chimeric proteins were bioengineered such that the protective domains of each protein were maintained. In experiments described herein, mice were either immunized with OspA, OspC or OspC/OspA chimeric proteins in aluminum hydroxide. Mice were then bled and tested for antibody responses against OspC and OspA derived from various strains *of Borrelia.* In additional experiments, these immunized mice were challenged with ticks infected with *Borrelia burgdorferi* and transmission of infection was assessed.

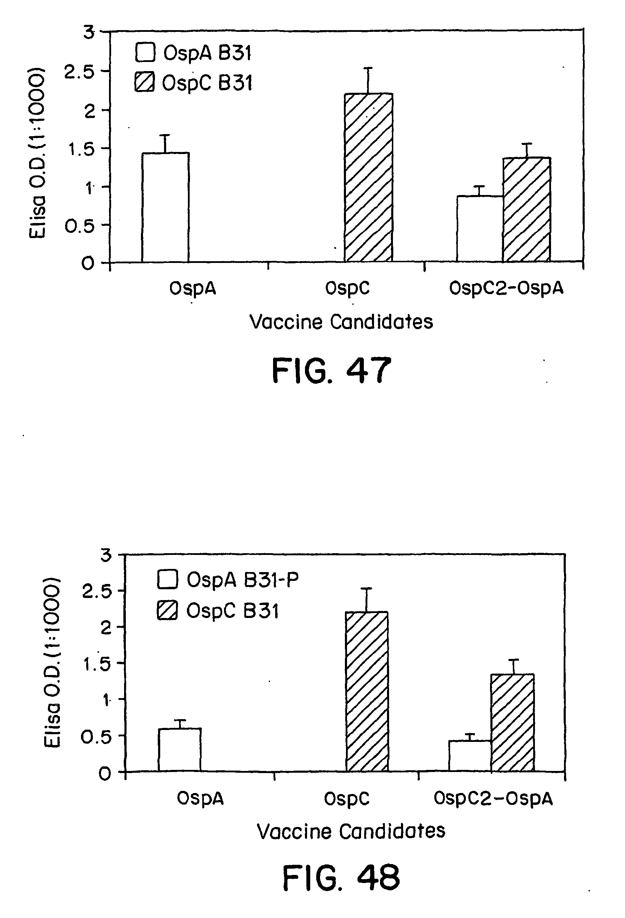

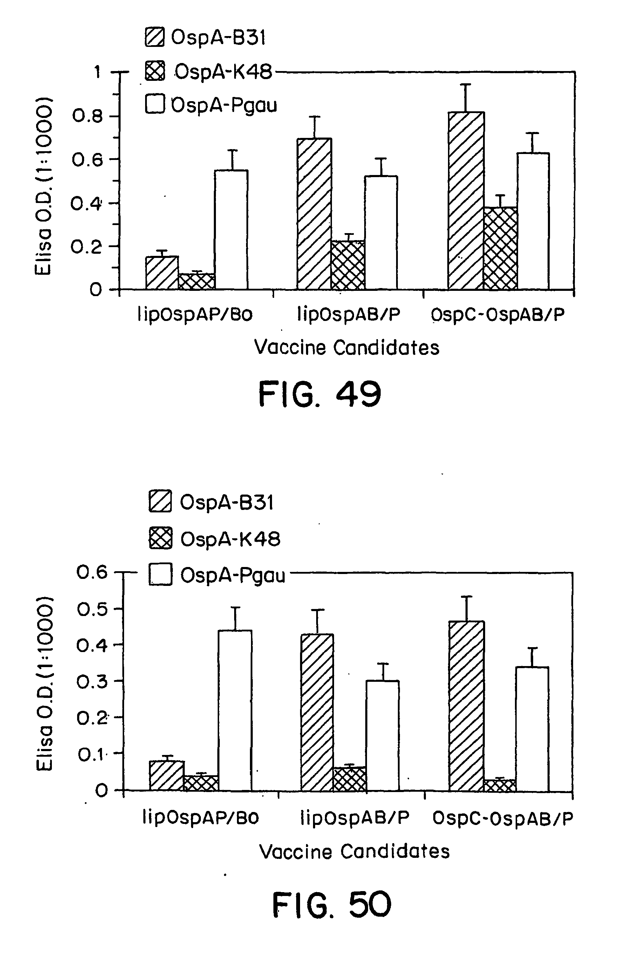

Mice immunized with the OspC/OspA chimeric protein gave a remarkable and equivalent antibody response to both OspA and OspC, as compared to mice immunized with OspA and OspC control proteins (Figs. 47 and 48). In addition, antibodies in the sera of mice immunized with the OspC/OspA chimeric protein were also reactive against antigens derived from different strains of *Borrelia burgdorferi* (Figs. 49-50 and 52-54). Chimer-inimunized mice were fully protected against challenge with ticks infected with *Borrelia burgdorferi,* as compared to sham-vaccinated controls (infection rates of 100%) (Table VI).

In other experiments described herein, mice were either immunized with a lipidated OspA chimeric protein, a lipidated OspC chimeric protein, or a non-lipidated OspC/OspA chimeric protein, once again in the presence of aluminum hydroxide. Mice were then bled and tested for antibody responses against OspA and OspC derived from various strains of *Borrelia.* Surprisingly, the results of these studies indicate that mice immunized with the non-lipidated OspC/OspA chimeric protein have antibody responses to OspA and OspC that are equivalent or greater than those generated by mice immunized with the corresponding lipidated OspA or lipidated OspC chimeric proteins (Figs. 49-51).

The results of the studies presented herein indicate that mice immunized with OspC-OspA chimeric proteins generate a potent antibody response against two immunoprotective targets that are expressed at different stages of the life cycle of *Borrelia burgdorferi.*

The current invention is illustrated by the following Examples, which are not to be construed to be limiting in any way.

### EXEMPLIFICATION

### Example 1. Purification of Borrelia burgdorferi Outer Surface Protein A and Analysis of Antibody Binding Domains

This example details a method for the purification of large amounts of native outer surface protein A (OspA) to homogeneity, and describes mapping of the antigenic specificities of several anti-OspA MAbs. OspA was purified to homogeneity by exploiting its resistance to trypsin digestion. Intrinsic labeling with ¹⁴C-palmitic acid confirmed that OspA was lipidated, and partial digestion established lipidation at the amino-terminal cysteine of the molecule.

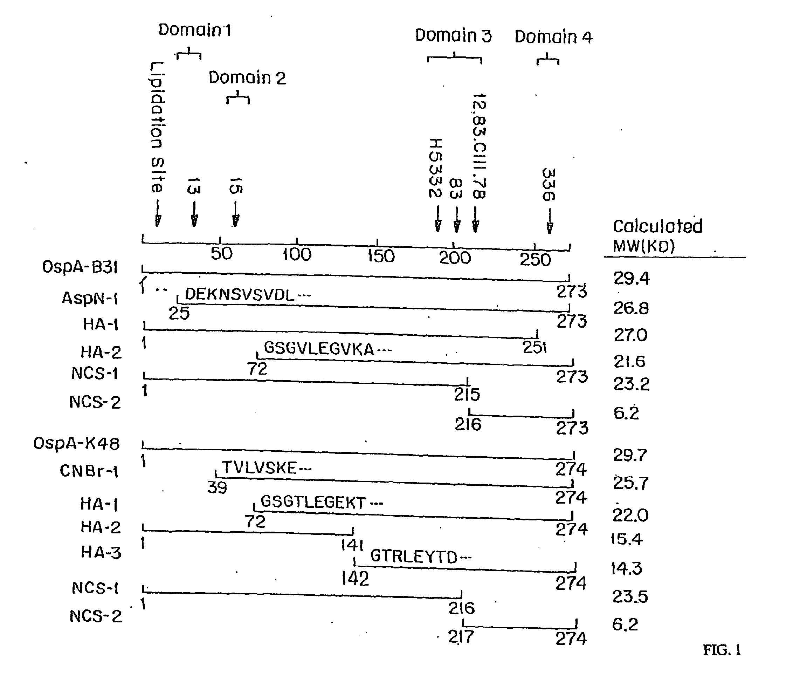

The reactivity of seven anti-OspA murine monoclonal antibodies to nine different *Borrelia* isolates was ascertained by Western blot analysis. Purified OspA was fragmented by enzymatic or chemical cleavage, and the monoclonal antibodies were able to define four distinct immunogenic domains (see Fig. 1). Domain 3, which included residues 190-220 of OspA, was reactive with protective antibodies known to agglutinate the organism *in vitro,* and included distinct specificities, some of which were not restricted to a genotype of *B. burgdorferi.*

### A. Purification of Native OspA

Detergent solubilization of *B. burgdorferi* strips the outer surface proteins and yields partially-purified preparations containing both OspA and outer surface protein B (OspB) (Barbour, A.G. et al., Infect. Immun. 52 (5): 549-554 (1986); Coleman, J.L. and J.L. Benach, J Infect. Dis. 155 (4): 756-765 (1987); Cunningham, T.M. et al., Ann. Ny Acad. Sci. 539: 376-378 (1988); Brandt, M.E. et al., Infect. Immun. 58: 983-991 (1990); Sambri, V. and R. Cevenini, Microbiol. 14: 307-314 (1991)). Although both OspA and OspB are sensitive to proteinase K digestion, in contrast to OspB, OspA is resistant to cleavage by trypsin (Dunn, J. et al., Prot. Exp. Purif. 1: 159-168 (1990); Barbour, A.G. et al, Infect. Immun. 45: 94-100 (1984)). The relative insensitivity to trypsin is surprising in view of the fact that OspB A has a high (16% for B31) lysine content, and may relate to the relative configuration of OspB A and B in the outer membrane.

### Intrinsic Radiolabeling of Borrelia

Labeling for lipoproteins was performed as described by Brandt *et al.* (Brandt et al., Infect. Immun. 58: 983-991 (1990)). ¹⁴C-palmitic acid (ICN, Irvine, California) was added to the BSK II media to a final concentration of 0.5 µCi per milliliter (ml). Organisms were cultured at 34°C in this medium until a density of 10⁸ cells per ml was achieved. Purification of OspA Protein from *Borrelia* Strain B31

*Borrelia burgdorferi,* either ¹⁴C-palmitic acid-labeled or unlabeled, were harvested and washed as described (Brandt, M.E. et al., Infect. Immun. 58: 983-991 (1990)). Whole organisms were trypsinized according to the protocol of Barbour et al. (Infect. Immun. 45: 94-100 (1984)) with some modifications. The pellet was suspended in phosphate buffered saline (PBS, 10mM, pH 7.2), containing 0.8% tosyl-L-phenylalanine chloromethyl ketone (TPCK)-treated trypsin (Sigma, St. Louis, Missouri), the latter at a ratio of 1 µg per 10⁸ cells. Reaction was carried out at 25°C for 1 hour, following which the cells were centrifuged. The pellet was washed in PBS with 100 µg/ml phenylmethylsulfonyl fluoride (PMSF). Triton X-114 partitioning of the pellet was carried out as described by Brandt *et al.* (Brandt et al., Infect. Immun. 58: 983-991 (1990)). Following trypsin treatment, cells were resuspended in ice-cold 2% (v/v) Triton X-114 in PBS at 10⁹ cells per ml. The suspension was rotated overnight at 4°C, and the insoluble fraction removed as a pellet after centrifugation at 10,000 X g for 15 minutes at 4°C. The supernatant (soluble fraction) was incubated at 37°C for 15 minutes and centrifuged at room temperature at 1000 X g for 15 minutes to separate the aqueous and detergent phases. The aqueous phase was decanted, and ice cold PBS added to the lower Triton phase, mixed, warmed to 37°C, and again centrifuged at 1000 X g for 15 minutes. Washing was repeated twice more. Finally, detergent was removed from the preparation using a spin column ofBio-beads SM2 (BioRad, Melville, New York) as described (Holloway, P.W., Anal. Biochem. 53: 304-308 (1973)).

Ion exchange chromatography was carried out as described by Dunn *et al.* (Dunn et al., Prot. Exp. Purif. 1: 159-168 (1990)) with minor modifications. Crude OspA was dissolved in buffer A (1% Triton X-100, 10mM phosphate buffer (pH 5.0)) and loaded onto a SP Sepharose resin (Pharmacia, Piscataway, New Jersey), pre-equilibrated with buffer A at 25°C. After washing the column with 10 bed-volumes of buffer A, the bound OspA was eluted with buffer B (1% Triton X-100, 10mM phosphate buffer (pH 8.0)). OspA fractions were detected by protein assay using the BCA method (Pierce, Rockford, Illinois), or as radioactivity when intrinsically labeled material was fractionated. Triton X-100 was removed using a spin column of Bio-beads SM2.

This method purifies OspA from an outer surface membrane preparation. In the absence of trypsin-treatment, OspA and B were the major components of the soluble fraction obtained after Triton partitioning of strain B31. In contrast, when Triton extraction was carried out after trypsin-treatment, the OspB band is not seen. Further purification of OspA-B31 on a SP Sepharose column resulted in a single band by SDS-PAGE. The yield following removal of detergent was approximately 2 mg per liter of culture. This method of purification of OspA, as described herein for strain B31, can be used for other isolates *of Borrelia* as well. For strains such as strain K48, which lack OspB, trypsin treatment can be omitted. Lipidation site of OspA-B31

¹⁴C-palmitic acid labeled OspA from strain B31 was purified as described above and partially digested with endoproteinase Asp-N (data not shown). Following digestion, a new band of lower molecular weight was apparent by SDS-PAGE, found by direct amino-terminal sequencing to begin at Asp₂₅. This band had no trace of radioactivity by autoradiography (data not shown). OspA and B contain a signal sequence (L-X-Y-C) similar to the consensus described for lipoproteins of *E. coli,* and it has been predicted that the lipidation site of OspA and B should be the amino-terminal cysteine (Brandt, M.E. et al., Infect. Immun 58: 983-991 (1990)). The results presented herein support this prediction.

### B. Comparison of OspA Antibody Binding Regions in Nine Strains of Borrelia burgdorferi

The availability of the amino acid sequenced for OspA from a number of different isolates, combined with peptide mapping and Western blot analysis, permitted the identification of the antigenic domains recognized by monoclonal antibodies (MAbs) and allowed inference of the key amino acid residues responsible for specific antibody reactivity.

### Strains of Borrelia burgdorferi

Nine strains of *Borrelia,* including seven European strains and two North American strains, were used in this study of antibody binding domains of several proteins. Information concerning the strains is summarized in Table I, below.

**Table I. Representative Borrelia Strains**

| Strain | Location and Source | Reference for Strain |
|---|---|---|
| K48 | Czechoslovakia, *Ixodes ricinus* | none |
| PGAU | Germany, human ACA | Wilske, B. et al., J. Clin. Microbiol. 32: 340-350 (1993) |
| DK29 | Denmark, human EM | Wilske, B. *et al.* |
| PKo | Germany, human EM | Wilske, B*. et al.* |
| PTrob | Germany, human skin | Wilske, B*. et al.* |
| Ip3 | Khabarovsk, Russia, *I. persulcatus* | Asbrink, E. et al., Acta Derm.Venereol. 64: 506-512 (1984) |
| Ip90 | Khabarovsk, Russia, *I. persulcatus* | Asbrink, E. *et al.* |
| 25015 | Millbrook, NY, *I. persulcatus* | Barbour, A.G. et al., Curr.Microbiol. 8:123-126 (1983) |
| B31 | Shelter Island, NY, *I. scapularis* | Luft, B.J. et al., Infect. Immun.60: 4309-4321 (1992); ATCC 35210 |
| PKa1 | Germany, human CSF | Wilske, B. *et al.* |
| ZS7 | Freiburg, Germany, *I. ricinus* | Wallich, R et al., Nucl. Acids Res. 17: 8864 (1989) |
| N40 | Westchester Co., NY | Fikrig, E. et al., Science 250: 553-556 (1990) |
| PHei | Germany, human CSF | Wilske, B*. et al.* |
| ACAI | Sweden, human ACA | Luft, B. J. et al., FEMS Microbiol. Lett. 93: 73-68 (1992) |
| PBo | Germany, human CSF | Wilske, B. *et al.* |

| | | |
|---|---|---|
| ACA = patient with acrodermatitis chronica atrophicans; EM = patient with erythema migrans; CSF = cerebrospinal fluid of patient with Lyme disease | | |

Strains K48, PGAU and DK29 were supplied by R. Johnson, University of Minnesota; PKo and PTrob were provided by B. Wilske and V. Preac-Mursic of the Pettenkhofer Institute, Munich, Germany; and Ip3 and Ip90 were supplied by L. Mayer of the Center for Disease Control, Atlanta, Georgia. The North American strains included strain 25015, provided by J. Anderson of the Connecticut Department of Agriculture; and strain B31 (ATCC 35210).

### Monoclonal Antibodies

Seven monoclonal antibodies (MAbs) were utilized in this study. Five of the MAbs (12, 13, 15, 83 and 336) were produced from hybridomas cloned and subcloned as previously described (Schubach, W.H., et al., Infect. Immun. 59(6):1911-1915 (1991.)). MAb H5332 (Barbour, A.G. et al., Infect. Immun. 41: 795-804 (1983)) was a gift from Drs. Alan Barbour, University of Texas, and MAb CIII.78 (Sears, J.E. et al., J. Immunol. 147(6):1995-2000 (1991)) was a gift from Richard A. Flavell, Yale University. MAbs 12 and 15 were raised against whole sonicated B3; MAb 336 was produced against whole PGAU; and MAbs 13 and 83 were raised to a truncated form of OspA cloned from the K48 strain and expressed in *E. coli* using the T7 RNA polymerase system (McGrath, B.C. et al., Vaccines, Cold Spring Harbor Laboratory Press, Plainview, New York, pp. 365-370 (1993)). All MAbs were typed as being Immunoglobulin G (IgG).

### Methods of Protein Cleavage, Western Blotting and Ammo-Terminal Sequencing

Prediction of the various cleavage sites was achieved by knowledge of the primary amino acid sequence derived from the full nucleotide sequences of OspA, many of which are currently available (see Table II, below). Cleavage sites can also be predicted based on the peptide sequence of OspA, which can be determined by standard techniques after isolation and purification of OspA by the method described above. Cleavage of several OspA isolates was conducted to determine the localization of monoclonal antibody binding of the proteins.

Hydroxylamine-HCl (HA), N-chlorosuccinimide (NCS), and cyanogen bromide cleavage of OspA followed the methods described by Bornstein (Biochem. 9(12):2408-2421 (1970)), Shechter et al., (Biochem. 15 (23):5071-5075 (1976)), and Gross (in Hirs, C.H.W. (ed): Methods in Enzymology, (N.Y. Acad. Press), 11:238-255 (1967)) respectively. Protease cleavage by endoproteinase, Asp-N (Boehringer Mannheim, Indianapolis, Indiana), was performed as described by Cleveland D.W. et al., (J. Biol. Chem. 252: 1102-1106 (1977)). Ten micrograms of OspA were used for each reaction. The ratio of enzyme to OspA was approximately 1 to 10 (w/w).

Proteins and peptides generated by cleavage were separated by SDS-polyacrylamide gel electrophoresis (SDS-PAGE) (Laemmli, U.K., Nature (London) 227:680-685 (1970)), and electroblotted onto immobilon Polyvinylidine Difluoride (PVDF) membranes (Ploskal, M.G. et al., Biotechniques 4: 272-283 (1986)). They were detected by amido black staining or by immunostaining with murine MAbs, followed by alkaline phosphatase-conjugated goat antimouse IgG. Specific binding was detected using a 5-bromo-4-chloro-3-indolylphosphate (BCIP)/nitroblue tetrazolium (NBT) developer system (KPL Inc., Gathersburg, Maryland).

In addition, amino-terminal amino acid sequence analysis was carried out on several cleavage products, as described by Luft et al. (Infect. Immun. 57: 3637-3645 (1989)). Amido black stained bands were excised from PVDF blots and sequenced by Edman degradation using a Biosystems model 475A sequenator with model 120A PTH analyzer and model 900A control/data analyzer.

### Cleavage Products of Outer Surface Protein A Isolates

Purified OspA-B31, labeled with ¹⁴C-palmitic acid, was fragmented with hydroxylamine-HCl (HA) into two peptides, designated HA1 and HA2 (data not shown). The HA1 band migrated at 27 KD and retained its radioactivity, indicating that the peptide included the lipidation site at the N-terminus of the molecule (data not shown). From the predicted cleavage point, HA1 should correspond to residues 1 to 251 of OspA-B31. HA2 had a MW of 21.6 KD by SDS-PAGE, with amino-terminal sequence analysis showing it to begin at Gly72, i.e. residues 72 to 273 of OspA-B31. By contrast, HA cleaved OspA-K48 into three peptides, designated HA1, HA2, and HA3 with apparent MWs of 22KD, 16 KD and 12 KD, respectively. Amino-terminal sequencing showed HA1 to start at Gly72, and HA3 at Gly142. HA2 was found to have a blocked amino-terminus, as was observed for the full-length OspA protein. HA1,2 and 3 of OspA-K48 were predicted to be residues 72-274, 1 to 141 and 142 to 274, respectively.

N-Chlorosuccinimide (NCS) cleaves tryptophan (W), which is at residue 216 of OspA-B31 or residue 217 of OspA-K48 (data not shown). NCS cleaved OspA-B31 into 2 fragments, NCS1, with MW of 23 KD, residues 1-216 of the protein, and NCS2 with a MW of 6.2 KD, residues 217 to 273 (data not shown). Similarly, K48 OspA was divided into 2 pieces, NCS1 residues 1-217, and NCS2 residues 218 to 274 (data not shown).

Cleavage of OspA by cyanogen bromide (CNBr) occurs at the carboxy side of methionine, residue 39. The major fragment, CNBr1, has a MW of 25.7 KD, residues 39-274 by amino-terminal amino acid sequence analysis (data not shown). CNBr2 (about 4 KD) could not be visualized by amido black staining; instead, lightly stained bands of about 20 KD MW were seen. These bands reacted with anti-OspA MAbs, and most likely were degradation products due to cleavage by formic acid.

### Determination of Antibody Binding Domains for Anti-OspA Monoclonal Antibodies

The cleavage products of OspA-B31 and OspA-K48 were analyzed by Western blot to assess their ability to bind to the six different MAbs. Preliminary Western blot analysis of the cleavage products demonstrated that strains K48 and DK29 have similar patterns of reactivity, as do Ip3, PGAU and PKo. The OspA of strain PTrob was immunologically distinct from the others, being recognized only by MAb 336. MAb 12 recognized only the two North American strains, B31 and 25015. When the isolates were separated into genogroups, it was remarkable that all the MAbs, except MAb 12, crossed over to react with multiple genogroups.

MAb12, specific for OspA-B31, bound to both HA1 and HA2 ofOspA-B31. However, cleavage of OspA-B31 by NCS at residue Trp216 created fragments which did not react with MAb12, suggesting that the relevant domain is near or is structurally dependent upon the integrity of this residue (data not shown). MAb 13 bound only to OspA-K48, and to peptides containing the amino-terminus of that molecule (e.g. HA2; NCS1). It did not bind to CNBr1 residues 39 to 274. Thus the domain recognized by MAb13 is in the amino-terminal end of OspA-K48, near Met38.

MAb15 reacts with the OspA of both the B31 and K48 strains, and to peptides containing the N-terminus of OspA, such as HA1 of OspA-B31 and NCS1, but not to peptides HA2 of OspA-B31 and HA1 of OspA-K48 (data not shown). Both peptides include residue 72 to the C-terminus of the molecules. MAb15 bound to CNBr1 of OspA-K48, indicating the domain for this antibody to be residues 39 to 72, specifically near Gly72 (data not shown).

MAb83 binds to OspA-K48, and to peptides containing the C-terminal portion of the molecule, such as HA1. They do not bind to HA2 of OspA-K48, most likely because the C-terminus of HA2 of OspA-K48 ends at 141. Similar to MAb12 and OspA-B31, binding of MAbs 83 and CIII.78 is eliminated by cleavage of OspA at the tryptophan residue. Thus binding of MAbs 12, 83 and CIII.78 to OspA depends on the structural integrity of the Trp₂₁₆ residue, which appears to be critical for antigenicity. Also apparent is that, although these MAbs bind to a common antigenic domain, the precise epitopes which they recognize are distinct from one another given the varying degrees of cross-reactivity to these MAbs among strains.

Although there is similar loss of binding activity of MAb336 with cleavage at Trp₂₁₆, this MAb does not bind to HA1 of OspA-B31, suggesting the domain for this antibody includes the carboxy-terminal end of the molecule, inclusive of residues 251 to 273. Low MW peptides, such as HA3 (10 KD) and NCS2 (6KD), of OspA-K48 do not bind this MAb on Western blots. In order to confirm this observation, we tested binding of the 6 MAbs with a recombinant fusion construct p3A/EC that contains a trpE leader protein fused with residues 217 to 273 of OspA-B31 (Schubach, W.H. et al., Infect. Immun. 59(6): 1911-1915 (1991)). Only MAb336 reacted with this construct (data not shown). Peptides and antigenic domains localized by fragmentation of OspA are summarized in Fig. 1.

### Mapping of Domains to Define the Molecular Basis for the Serotype Analysis

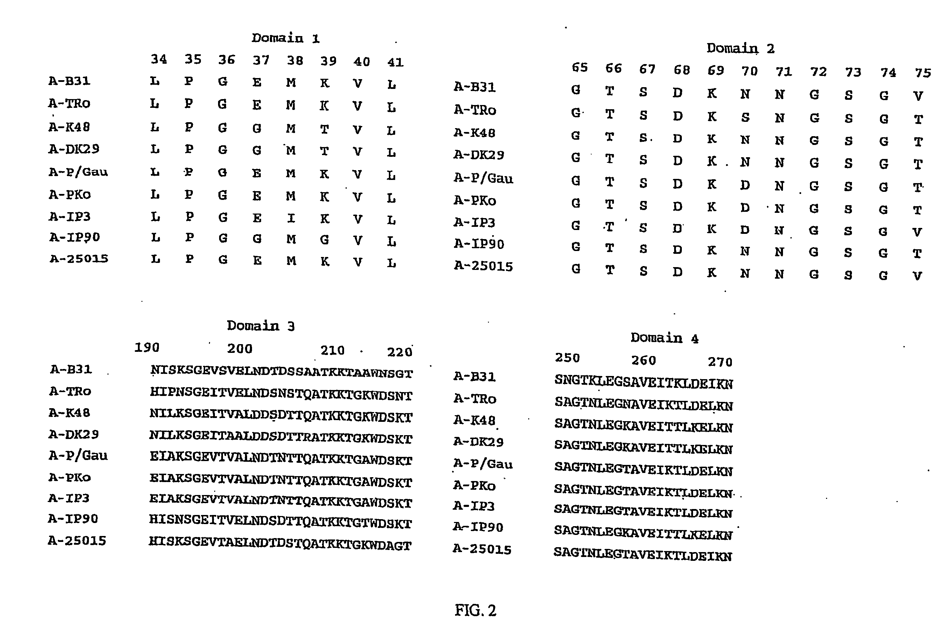

To define the molecular basis for the serotype analysis of OspA, we compared the derived amino acid sequences of OspA for the nine isolates (Fig. 2). At the amino terminus of the protein, these predictions can be more precise given the relatively small number of amino acid substitutions in this region compared to the carboxy terminus. Domain 1, which is recognized by MAb 13, includes residues Leu34 to Leu41. MAb13 only binds to the OspA of species K48, DK29 and IP90. Within this region, residue 37 is variable, however Gly37 is conserved amongst the three reactive strains. When Gly37 is changed to Glu37, as it is in OspA of strains B31, PTrob, PGAU, and PKo, MAb13 does not recognize the protein (data not shown). By similar analysis, it can be seen that Asp70 is a crucial residue for Domain 2, which includes residues 65 to 75 and is recognized by MAb15. Domain 3 is reactive with MAbs H5332, 12 and 83, and includes residues 190-220. It is clear that significant heterogeneity exists between MAbs reactive with this domain, and that more than one conformational epitope must be contained within the sequence. Domain 4 binds MAb336, and includes residues 250 to 270. In this region, residue 266 is variable and therefore may be an important determinant. It is apparent, however, that other determinants of the reactivity of this monoclonal antibody reside in the region comprising amino acids 217-250. Furthermore, the structural integrity of Trp216 is essential for antibody reactivity in the intact protein. Finally, it is important to stress that Fig. 2 indicates only the locations of the domains, and does not necessarily encompass the entire domain. Exact epitopes are being analyzed by site-directed mutagenesis of specific residues.

Overall, evidence suggests that the N-terminal portion is not the immunodominant domain of OspA, possibly by virtue of its lipidation, and the putative function of the lipid moiety in anchoring the protein to the outer envelope. The C-terminal end is immunodominant and includes domains that account in part for structural heterogeneity (Wilske, B. et al., Med. Microbiol. Immunol. 181: 191-207 (1992)), and may provide epitopes for antibody neutralization (Sears, J.E. et al., J. Immunol. 147(6): 1995-2000 (1991)), and relate to other activities, such as the induction of T-cell proliferation (Shanafel, M.M., et al., J. Immunol. 148: 218-224 (1992)). There are common epitopes in the carboxy-end of the protein that are shared among genospecies which may have immunoprotective potential (Wilske, B., et al., Med. Microbiol. Immunol. 181: 191-207 (1992)).

Prediction of secondary structure on the basis of hydropathy analysis and circular dichroism and fluorescence spectroscopy measurements (McGrath, B.C., et al., Vaccines, Cold Spring Harbor Laboratory Press, Plainview, New York; pp. 365-370 (1993)) suggest domains 3 and 4 to be in a region of the molecule with a propensity to form alpha-helix, whereas domains 1 and 2 occur in regions predicted to be beta-sheets (see Fig. 1). These differences may distinguish domains in accessibility to antibody or to reactive T-cells (Shanafel, M.M. et al., J. Immunol. 148: 218-224 (1992)). Site-directed mutagenesis of specific epitopes, as described below in Example 2, aids in identifying exact epitopes.

### Example 2. Identification of an Immunologically Important Hypervariable Domain of the Major Outer Surface Protein A of Borrelia.

This Example describes epitope mapping studies using chemically cleaved OspA and TrpE-OspA fusion proteins. The studies indicate a hypervariable region surrounding the single conserved tryptophan residue of OspA (at residue 216, or in some cases 217), as determined by a moving window population analysis of OspA from fifteen European and North American isolates of *Borrelia.* The hypervariable region is important for immune recognition.

Site-directed mutagenesis was also conducted to examine the hypervariable regions more closely. Fluorescence and circular dichroism spectroscopy have indicated that the conserved tryptophan is part of an alpha-helical region in which the tryptophan is buried in a hydrophobic environment (McGrath, B.C., et al., Vaccines, Cold Spring Harbor Laboratory Press, Plainview, New York; pp. 365-370 (1993)). More polar amino acid side-chains flanking the tryptophan are likely to be exposed to the hydrophilic solvent. The hypervariability of these solvent-exposed residues among the various strains of *Borrelia* suggested that these amino acid residues may contribute to the antigenic variation in OspA. Therefore, site-directed mutagenesis was performed to replace some of the potentially exposed amino acid side chains in the protein from one strain with the analogous residues of a second strain. The altered proteins were then analyzed by Western Blot using monoclonal antibodies which bind OspA on the surface of the intact, non-mutated spirochete. The results indicated that certain specific amino acid changes near the tryptophan can abolish reactivity of OspA to these monoclonal antibodies.

### A. Verification of Clustered Polymorphisms in Outer Surface Protein A Sequences

Cloning and sequencing of the OspA protein from fifteen European and North American isolates (described above in Table I) demonstrated that amino acid polymorphism is not randomly distributed throughout the protein; rather, polymorphism tended to be clustered in three regions of OspA. The analysis was carried out by plotting the moving, weighted average polymorphism of a window (a fixed length subsection of the total sequence) as it is slid along the sequence. The window size in this analysis was thirteen amino acids, based upon the determination of the largest number of significantly deviating points as established by the method of Tajima (J. Mol. Evol. 33: 470-473 (1991)). The average weighted polymorphism was calculated by summing the number of variant alleles for each site. Polymorphism calculations were weighted by the severity of amino acid replacement (Dayhoff, M.O. et al., in: Dayhoff, M.O. (ed.) Atlas of Protein Sequence and Structure NBRF, Washington, Vol. 5, Suppl. 3: 345 (1978)). The sum was normalized by the window size and plotted. The amino acid sequence position corresponds to a window that encompasses amino acids 1 through 13. Bootstrap resampling was used to generate 95% confidence intervals on the sliding window analysis. Since *Borrelia* has been shown to be clonal, the bootstrap analysis should give a reliable estimate of the expected variance from polymorphism calculations. The bootstrap was iterated five hundred times at each position, and the mean was calculated from the sum of all positions. The clonal nature of *Borrelia* ensures that the stochastic variance that results from differing genealogical histories of the sequence positions (as would be expected if recombination were prevalent) will be minimized.

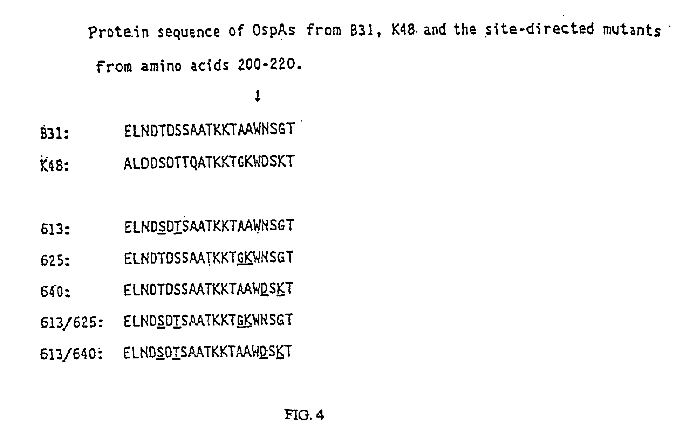

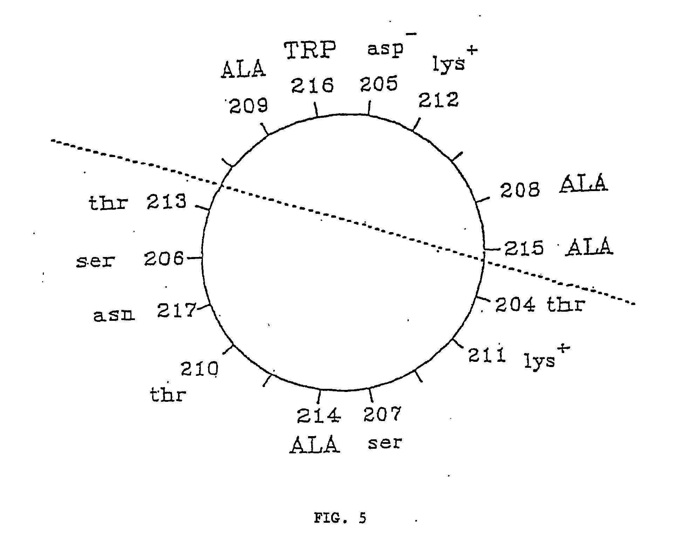

This test verified that the three regions around the observed peaks all have significant excesses of polymorphism. Excesses of polymorphism were observed in the regions including amino acid residues 132-145, residues 163-177, and residues 208-221 (Fig. 3). An amino acid alignment between residues 200 and 220 for B31, K48 and the four site-directed mutants is shown in Fig. 4. The amino acid 208-221 region includes the region of OspA which has been modeled as an oriented alpha-helix in which the single tryptophan residue at amino acid 216 is buried in a hydrophobic pocket, thereby exposing more polar amino acids to the solvent (Fig. 5) (France, L.L., et al., Biochem. Biophys. Acta 1120: 59 (1992)). These potentially solvent-exposed residues showed considerable variability among the OspAs from various strains and may be an important component of OspA antigenic variation. For the purposes of generating chimeric proteins, the hypervariable domains of interest are Domain A, which includes amino acid residues 120-140 of OspA; Domain B, which includes residues 150-180; and Domain C, which includes residues 200-216 or 217.

### B. Site-Directed Mutagenesis of the Hypervariable Region

Site-directed mutagenesis was performed to convert residues within the 204-219 domain of the recombinant B31 OspA to the analogous residues of a European OspA variant, K48. In the region of OspA between residues 204 and 219, which includes the helical domain (amino acids 204-217), there are seven amino acid differences between OspA-B31 and OspA-K48. Three oligonucleotides were generated, each containing nucleotide changes which would incorporate K48 amino acids at their analogous positions in the B31 OspA protein. The oligos used to create the site-directed mutants were:
5'-CTTAATGACTCTGACACTAGTGC-3' (#613, which converts serine at position 204 to threonine, and serine at 206 to threonine (Ser204-Thr, Ser206-Thr)) (SEQ ID NO. 1);
5'-GCTACTAAAAAAACCGGGAAATGGAATTCA-3' (#625, which converts alanine at 214 to glycine, and alanine at 215 to lysine (Ala214-Gly, Ala215-Lys)) (SEQ ID NO. 2); and
5'-GCAGCTTGGGATTCAAAAACATCCACTTTAACA-3' (#640, which converts asparagine at 217 to aspartate, and glycine at 219 to lysine (Asn217-Asp, Gly219-Lys)) (SEQ ID NO. 3).

Site-directed mutagenesis was carried out by performing mutagenesis with pairs of the above oligos. Three site-directed mutants were created, each with two changes: OspA 613 (Ser204-Thr, Ser206-Thr), OspA 625 (Ala214-Gly, Ala215-Lys), and 640 (Asn217-Asp, Gly219-Lys). There were also two proteins with four changes: OspA 613/625 (Ser204-Thr, Ser206-Thr, Ala214-Gly, Ala215-Lys) and OspA 613/640 (Ser204-Thr, Ser206-Thr, Asn217-Asp, Gly219-Lys).

### Specificity of Antibody Binding to Epitopes of the Non-mutated Hypervariable Region

Monoclonal antibodies that agglutinate spirochetes, including several which are neutralizing *in vitro,* recognize epitopes that map to the hypervariable region around Trp216 (Barbour, A.G. et al., Infect. and Immun. 41: 759 (1983); Schubach, W.H. et al., Infect. and Immun. 59: 1911 (1991)). Western Blot analysis demonstrated that chemical cleavage of OspA from the B31 strain at Trp 216 abolishes reactivity of the protein with the agglutinating MAb 105, a monoclonal raised against B31 spirochetes (data not shown). The reagent, n-chlorosuccinimide (NCS), cleaves OspA at the Trp 216, forming a 23.2kd fragment and a 6.2kd peptide which is not retained on the Imobilon-P membrane after transfer. The uncleaved material binds MAb 105; however, the 23.2kd fragment is unreactive. Similar Western blots with a TrpE-OspA fusion protein containing the carboxy-terminal portion of the OspA protein demonstrated that the small 6.2kd piece also fails to bind MAb 105 (Schubach, W.H. et al., Infect. and Immun. 59: 1911 (1991)).

Monoclonal antibodies H5332 and H3TS (Barbour, A.G. et al., Infect. and Immun. 41: 759 (1983)) have been shown by immunofluorescence to decorate the surface of fixed spirochetes (Wilske, B. et al., World J. Microbiol. 7: 130 (1991)). These monoclonals also inhibit the growth of the organism in culture. Epitope mapping with fusion proteins has confirmed that the epitopes which bind these MAbs are conformationally determined and reside in the carboxy half of the protein. MAb H5332 is cross-reactive among all of the known phylogenetic groups, whereas MAb H3TS and MAb 105 seem to be specific to the B31 strain to which they were raised. Like MAb 105, the reactivities of H5332 and H3TS to OspA are abrogated by fragmentation of the protein at Trp216 (data not shown). MAb 336 was raised to whole spirochetes of the strain PGau. It cross-reacts to OspA from group 1 (the group to which B31 belongs) but not to group 2 (of which K48 is a member). Previous studies using fusion proteins and chemical cleavage have indicated that this antibody recognizes a domain of OspA in the region between residues 217 and 273 (data not shown). All of these MAbs will agglutinate the B31 spirochete.

### Western Blot Analysis of Antibody Binding to Mutated Hypervariable Regions

MAbs were used for Western Blot analysis of the site-directed OspA mutants induced in *E*. *coli* using the T7 expression system (Dunn, J.J. et al., Protein Expression and Purification 1: 159 (1990)). *E. coli* cells carrying pET9c plasmids having a site-directed OspA mutant insert were induced at mid-log phase growth with IPTG for four hours at 37°C. Cell lysates were made by boiling an aliquot of the induced cultures in SDS gel loading dye, and this material was then loaded onto a 12% SDS gel (BioRad mini-Protean II), and electrophoresed. The proteins were then transferred to Imobilon-P membranes (Millipore) 70V, 2 hour at 4°C using the BioRad mini transfer system. Western analysis was carried out as described by Schubach et al. (Infect. Immun. 59: 1911 (1991)).

Western Blot analysis indicated that only the 625 mutant (A1a214-Gly and Ala215-Lys) retained binding to the agglutinating monoclonal H3TS antibody (data not shown). However, the 613/625 mutant which has additional alterations to the amino terminus of Trp216 (Ser204-Thr and Ser206-Thr) did not bind this monoclonal antibody. Both 640 and 613/640 OspAs which have the Asn217-Asp and Gly219-Lys changes on the carboxy-terminal side of Trp216 also failed to bind MAb H3TS. This indicated that the epitope of the B31 OspA which binds MAb H3TS is comprised of amino acid side-chains on both sides of Trp216.

The 613/625 mutant failed to bind MAbs 105 and H5332, while the other mutants retained their ability to bind these MAbs. This is important in light of the data using fusion proteins that indicate that MAb 105 behaves more like MAb H3TS in terms of its serotype specificity and binding to OspA (Wilske, B. et al., Med. Microbiol. Immunol. 181: 191 (1992)). The 613/625 protein has, in addition to the differences at residues Ser204 and Ser206, changes immediately amino-terminal to Trp216 (Ala214-Gly and Ala215-Lys). The abrogation of reactivity of MAbs 105 and H5332 to this protein indicated that the epitopes of OspA which bind these monoclonals are comprised of residues on the amino-terminal side of Trp216.

The two proteins carrying the Asn217-Asp and Gly219-Lys replacements on the carboxy-terminal side of Trp216 (OspAs 640 and 613/640) retained binding to MAbs 105 and H5332; however, they failed to react with MAb 336, a monoclonal which has been mapped with TrpE-OspA fusion proteins and by chemical cleavage to a more carboxy-terminal domain. This result may explain why MAb 336 failed to recognize the K48-type of OspA (Group 2).

It is clear that amino acids Ser204 and Ser206 play an important part in the agglutinating epitopes in the region of the B31 OspA flanking Trp216. Replacement of these two residues altered the epitopes of OspA that bind MAbs 105, H3TS and H5332. The ability of the 640 changes alone to abolish reactivity of MAb 336 indicated that Ser204 and Ser206 are not involved in direct interaction with MAb 336.

The results indicated that the epitopes of OspA which are available to MAbs that agglutinate spirochetes are comprised at least in part by amino acids in the immediate vicinity of Trp216. Since recent circular dichroism analysis indicated that the structures of B31 and K48 OspA differ very little within this domain, it is unlikely that the changes made by mutation have radically altered the overall structure of the OspA protein (France, L.L. et al., Biochem. Biophys. Acta 1120: 59 (1992); and France et al., Biochem. Biophys Acta, submitted (1993)). This hypothesis is supported by the finding that the recombinant, mutant OspAs exhibit the same high solubility and purification properties as the parent B31 protein (data not shown).

In summary, amino acid side-chains at Ser204 and Ser206 are important for many of the agglutinating epitopes. However, a limited set of conservative changes at these sites were not sufficient to abolish binding of all of the agglutinating MAbs. These results suggested that the agglutinating epitopes of OspA are distinct, yet may have some overlap. The results also supported the hypothesis that the surface-exposed epitope around Trp216 which is thought to be important for immune recognition and neutralization is a conformationally-determined and complex domain of OspA.

### EXAMPLE 3. Borrelia Strains and Proteins

Proteins and genes from any strain of *Borrelia* can be utilized in the current invention. Representative strains are summarized in Table I, above.

### A. Genes Encoding Borrelia Proteins

The chimeric peptides of the current invention can comprise peptides derived from any *Borrelia* proteins. Representative proteins include OspA, OspB, OspC, OspD, p12, p39, p41 (fla), p66, and p93. Nucleic acid sequences encoding several *Borrelia* proteins are presently available (see Table II, below); alternatively, nucleic acid sequences encoding *Borrelia* proteins can be isolated and characterized using methods such as those described below.

**Table II. References for Nucleic Acid Sequences for Several Proteins of Various Borrelia Strains**

| Strain | p93 | OspA | p41 (fla) |
|---|---|---|---|
| K48 | X69602 (SID 67) | X62624 (SID 8) | X69610 |
| PGau | SID 73 | X62387 (SID 10) | X69612 (SID 51) |
| DK29 | ND | X63412 (SID 49) | X69608 (SID 53) |
| PKo | X69803 (SID 77) | X65599 (SID 57) | X69613 (SID 131) |
| PTrob | X69604 (SID 71) | X65598 (SID 135) | X69614 |
| Ip3 | ND | X70365 (SID 56) | ND |
| Ip90 | ND | Kryuchechnikov, V.N. et al., J.Microbiol Epid. Immunobiol 12:41-44 (1988)(SID 50) | ND |
| 25015 | X70365 (SID 75) | Fikrig, E.S. et al., J.Immunol. 7:2256-2260 (1992) (SID 12) | ND |
| B31 | Pemg, G.C. et al., Infect.Immun. 59:2070-74 (1992); Luft, B.J. et al., Infect. Immun. 60:4309-4321 (1992) (SID 65) | Bergstrom, S. et al., Mol.Microbiol. 3: 479-486 (1989) (SID 6) | Gassmann, G.S. et al., Nucl. Acids Res. 17: 3590 (1989) (SID 127) |
| PKa1 | ND | X69606 (SID 132) | X69611 (SID 129) |
| ZS7 | ND | Jonsson, M. et al., Infect.Immun. 60:1845-1853 (1992) (SID 134) | ND |
| N40 | ND | Kryuchechnikov, V.N. *et al.* (SID 133) | ND |
| PHei | ND | X65600 (SID 136) | ND |
| ACAI | ND | Kryuchechnikov, V.N. *et al.* (SID 58) | ND |
| PBo | X69601 (SID 69) | X65605 (SID 55) | X69610 (SID 130) |

| | | | |
|---|---|---|---|
| Numbers with an "X" prefix are GenBank data base accession numbers. SID = SEQ ID NO. | | | |

### B. Isolation of Borrelia Genes

Nucleic acid sequences encoding full length, lipidated proteins from known *Borrelia* strains were isolated using the polymerase chain reaction (PCR) as described below. In addition, nucleic acid sequences were generated which encoded truncated proteins (proteins in which the lipidation signal has been removed, such as by eliminating the nucleic acid sequence encoding the first 18 amino acids, resulting in non-lipidated proteins). Other proteins were generated which encoded polypeptides of a particular gene (i.e., encoding a segment of the protein which has a different number of amino acids than the protein does in nature). Using similar methods as those described below, primers can be generated from known nucleic acid sequences encoding *Borrelia* proteins and used to isolate other genes encoding *Borrelia* proteins. Primers can be designed to amplify all of a gene, as well as to amplify a nucleic acid sequence encoding truncated protein sequences, such as described below for OspC, or nucleic acid sequences encoding a polypeptide derived from a *Borrelia* protein. Primers can also be designed to incorporate unique restriction enzyme cleavage sites into the amplified nucleic acid sequences. Sequence analysis of the amplified nucleic acid sequences can then be performed using standard techniques.

### Cloning and Sequencing of OspA Genes and Relevant Nucleic Acid Sequences

*Borrelia* OspA sequences were isolated in the following manner: 100 µl reaction mixtures containing 50 mM KCl, 10 mM TRIS-HCl (pH 8,3), 1.5 mM MgCl₂, 200 µM each NTP, 2.5 units ofTaqI DNA polymerase (Amplitaq, Perkin-Elmer/Cetus) and 100 pmol each of the 5' and 3' primers (described below) were used. Amplification was performed in a Perkin-Elmer/Cetus thermal cycler as described (Schubach, W.H. et al., Infect. Immun. 59: 1811-1915 (1991)). The amplicon was visualized on an agarose gel by ethidium bromide staining. Twenty nanograms of the chloroform-extracted PCR product were cloned directly into the PC-TA vector (Invitrogen) by following the manufacturer's instructions. Recombinant colonies containing the amplified fragment were selected, the plasmids were prepared, and the nucleic acid sequence of each OspA was determined by the dideoxy chain-termination technique using the Sequenase kit (United States Biochemical). Directed sequencing was performed with M13 primers followed by OspA-specific primers derived from sequences, previously obtained with M13 primers.

Because the 5' and 3' ends of the OspA gene are highly conserved (Fikrig, E.S. et al., J. Immunol. 7:2256-2260 (1992); Bergstrom, S. et al., Mol. Microbiol. 3: 479-486 (1989); Zumstein, G. et al., Med. Microbiol. Immunol. 181: 57-70 (1992)), the 5' and 3' primers for cloning can be based upon any known OspA sequences. For example, the following primers based upon the OspA nucleic acid sequence from strain B31 were used:
5'-GGAGAATATATTATGAAA-3' (-12 to +6) (SEQ ID NO. 4); and
5'-CTCCTTATTTTAAAGCG-3' (+826 to +809) (SEQ ID NO. 5). (Schubach, W.H. et al., Infect. Immun 59: 1811-1915 (1991)).

OspA genes isolated in this manner include those for strains B31, K48, PGau, and 25015; the nucleic acid sequences are depicted in the sequence listing as SEQ ID NO. 6 (OspA-B31), SEQ ID NO. 8 (OspA-K48), SEQ ID NO. 10 (OspA-PGau), and SEQ ID NO. 12 (OspA-25015). An alignment of these and other OspA nucleic acid sequences is shown in Fig. 42. The amino acid sequences of the proteins encoded by these nucleic acid sequences are represented as SEQ ID NO. 7 (OspA-B31), SEQ ID NO. 9 (OspA-K48), SEQ ID NO. 11 (OspA-PGau), and SEQ ID NO. 13 (OspA-25015).

The following primers were used to generate specific nucleic acid sequences of the OspA gene, to be used to generate chimeric nucleic acid sequences (as described in Example 4):
5'-GTCTGCAAAAACCATGACAAG-3' (plus strand primer #369) (SEQ ID NO. 14);
5'-GTCATCAACAGAAGAAAAATTC-3' (plus strand primer #357) (SEQ ID NO 15);
5'-CCGGATCCATATGAAAAAATATTTATTGGG-3' AAAAATATTTATTGG-3' (plus strand primer #607) (SEQ ID NO. 16);
5'-CCGGGATCCATATGGCTAAGCAAAATGTTAGC-3' (plus strand primer #584) (SEQ ID NO. 17);
5'-GCGTTCAAGTACTCCAGA-3' (minus strand primer #200) (SEQ ID NO. 18);
5'-GATATCTAGATCTTATTTTAAAGCGTT-3' (minus strand primer #586) (SEQ ID NO. 19); and,
5'-GGATCCGGTGACCTTTTAAAGCGTTTTTAAT-3' (minus strand primer #1169) (SEQ ID NO. 20).

### Cloning and Sequencing of OspB

Similar methods were also used to isolate OspB genes. One OspB genes isolated is represented as SEQ ID NO. 21 (OspB-B31); its encoded amino acid sequence is SEQ ID NO. 22.

The following primers were used to generate specific nucleic acid sequences of the OspB gene, to be used in generation of chimeric nucleic acid sequences (see Example 4):
5'-GGTACAATTACAGTACAA-3' (plus strand primer #721) (SEQ ID NO. 23);
5'-CCGAGAATCTCATATGGCACAAAAAGGTGCTGAGTCAATTGG-3'(plus strand primer #1105) (SEQ ID NO. 24);
5'-CCGATATCGGATCCTATTTTAAAGCGTTTTTAAGC-3' (minus strand primer # 1106) (SEQ ID NO. 25); and
5'-GGATCCGGTGACCTTTTAAAGCGTTTTTAAG-3' (minus strand primer #1170) (SEQ ID NO. 26).

### Cloning and Sequencing of OspC

Similar methods were also used to isolate OspC genes. The following primers were used to isolate entire OspC genes from *Borrelia* strains B31, K48, PKo, and PTrob:
5'-GTGCGCGACCATATGAAAAAGAATACATTAAGTGCG-3' (plus strand primer having Nde1 site combined with start codon) (SEQ ID NO. 27), and
5'-GTCGGCGGATCCTTAAGGTTTTTTTGGACTTTCTGC-3' (minus strand primer having BamH1 site followed by stop codon) (SEQ ID NO. 28).

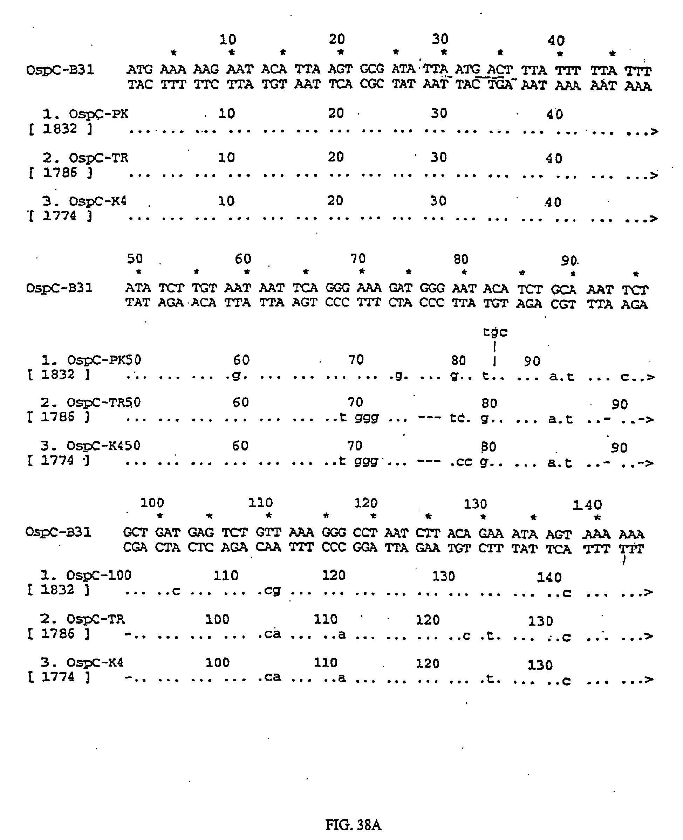

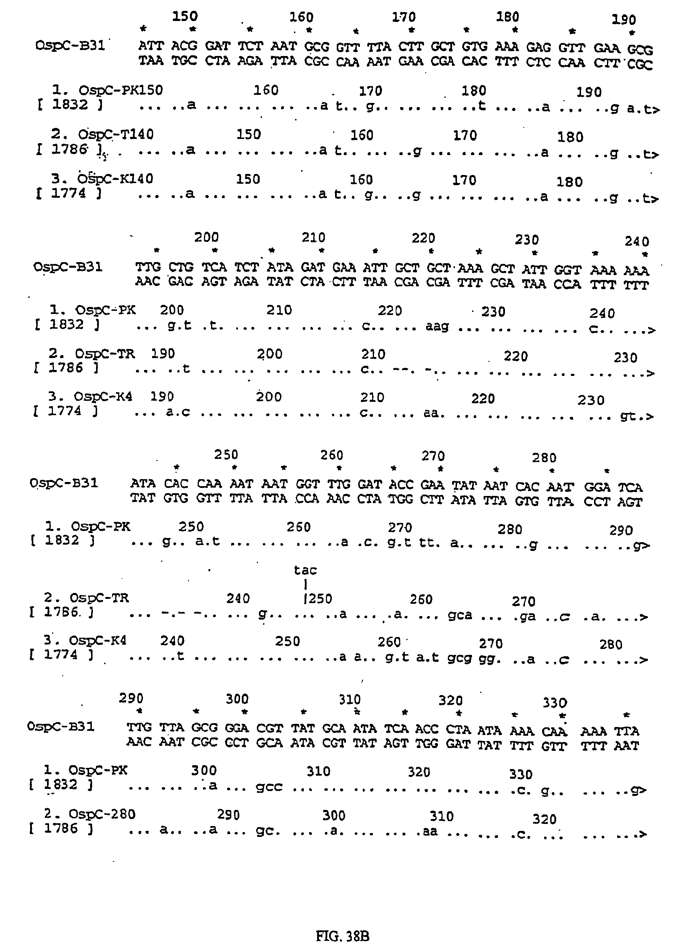

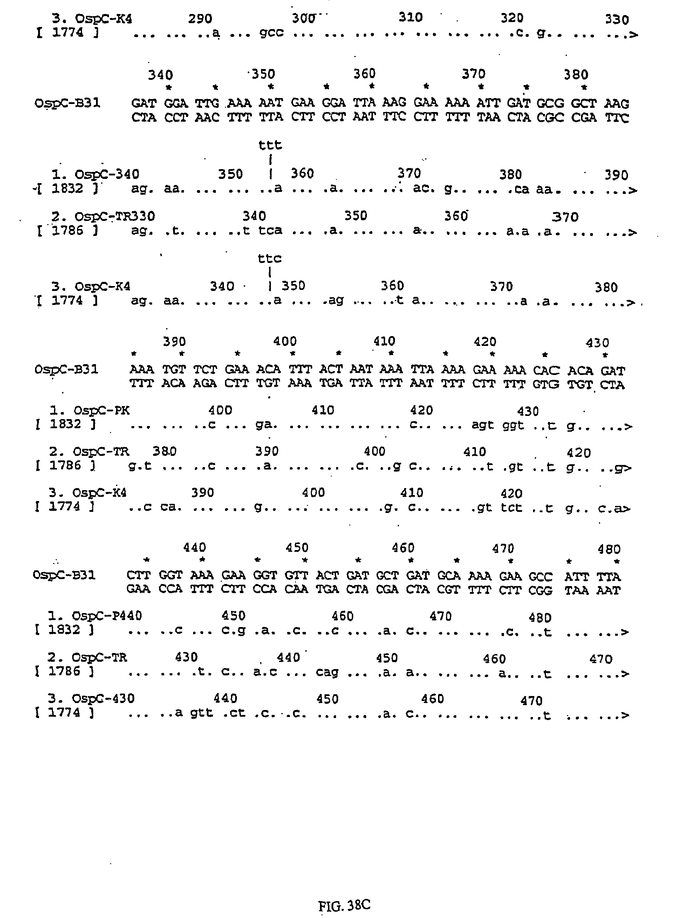

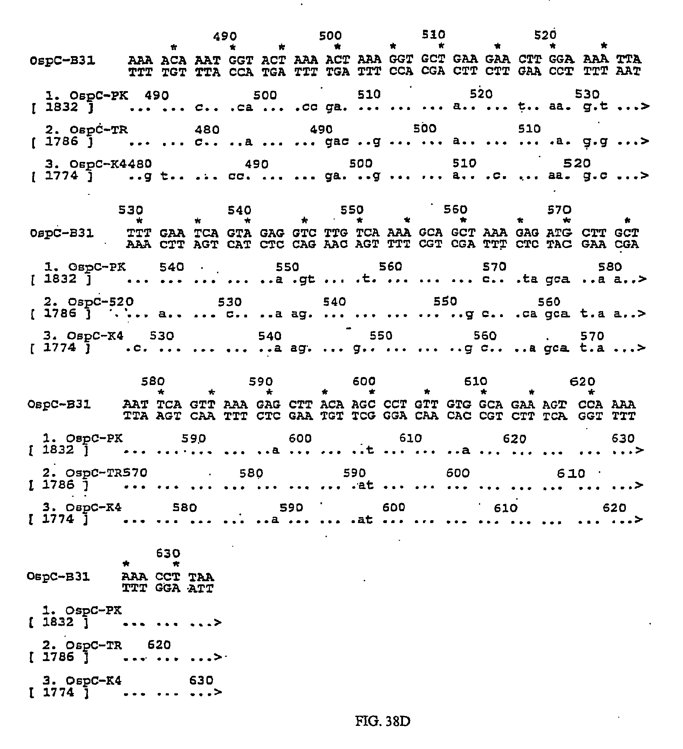

The nucleic acid sequences of the OspC genes were then determined by the dideoxy chain-termination technique using the Sequenase kit (United States Biochemical). OspC genes isolated and sequenced in this manner include those for strains B31, K48, PKo, and Tro; the nucleic acid sequences are depicted in the sequence listing as SEQ ID NO. 29 (OspC-B31), SEQ ID NO. 31 (OspC-K48), SEQ ID NO. 33 (OspC-PKo), and SEQ ID NO. 35 (OspC-Tro). An alignment of these sequences is shown in Fig. 38. The amino acid sequences of the proteins encoded by these nucleic acid sequences are represented as SEQ ID NO. 30 (OspC-B31), SEQ ID NO. 32 (OspC-K48), SEQ ID NO. 34 (OspC-PKo), and SEQ ID NO. 36 (OspC-Tro).

Truncated OspC genes were generated using other primers. These primers were designed to amplify nucleic acid sequences, derived from the OspC gene, that lacked the nucleic acids encoding the signal peptidase sequence of the full-length protein. The primers corresponded to bp 58-75 of the natural protein, with codons for Met-Ala attached ahead. For strain B31, the following primer was used:
5'-GTGCGCGACCATATGGCTAATAATTCAGGGAAAGAT-3' (SEQ ID NO. 37).

For strain PKo,
5'-GTGCGCGACCATATGGCTAGTAATTCAGGGAAAGGT-3' (SEQ ID NO. 38) was used.

For strains PTrob and K48,
5'-GTGCGCGACCATATGGCTAATAATTCAGGTGGGGAT-3' (SEQ ID NO. 39) was used.

Additional primers were also designed to amplify nucleic acids encoding particular polypeptides, for use in creation of chimeric nucleic acid sequences (see Example 4). These primers included:
5'-CTTGGAAAATTATTTGAA-3' (plus strand primer #520) (SEQ ID NO. 40);
5'-CACGGTCACCCCATGGGAAATAATTCAGGGAAAGG-3' (plus strand primer #58) (SEQ ID NO. 41);
5'-TATAGATGACAGCAACGC-3' (minus strand primer #207) (SEQ ID NO. 42); and
5'-CCGGTGACCCCATGGTACCAGGTTTTTTTGGACTTTCTGC-3' (minus strand primer #636) (SEQ ID NO. 43).

### Cloning and Sequencing of OspD

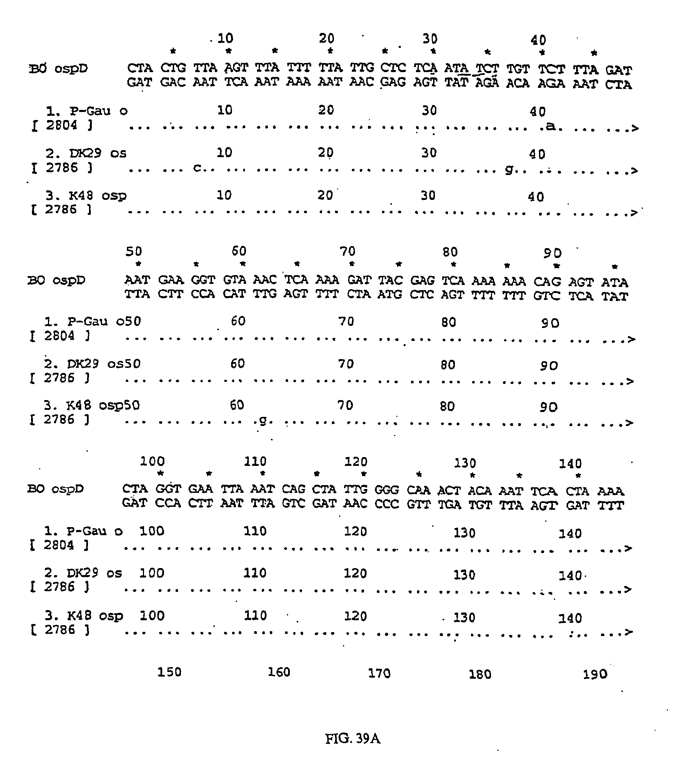

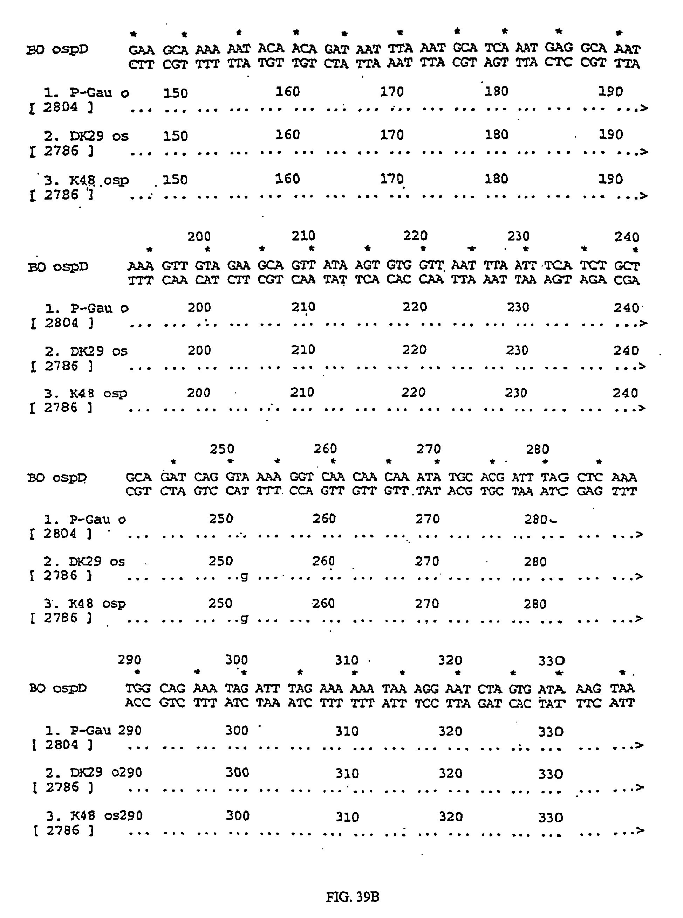

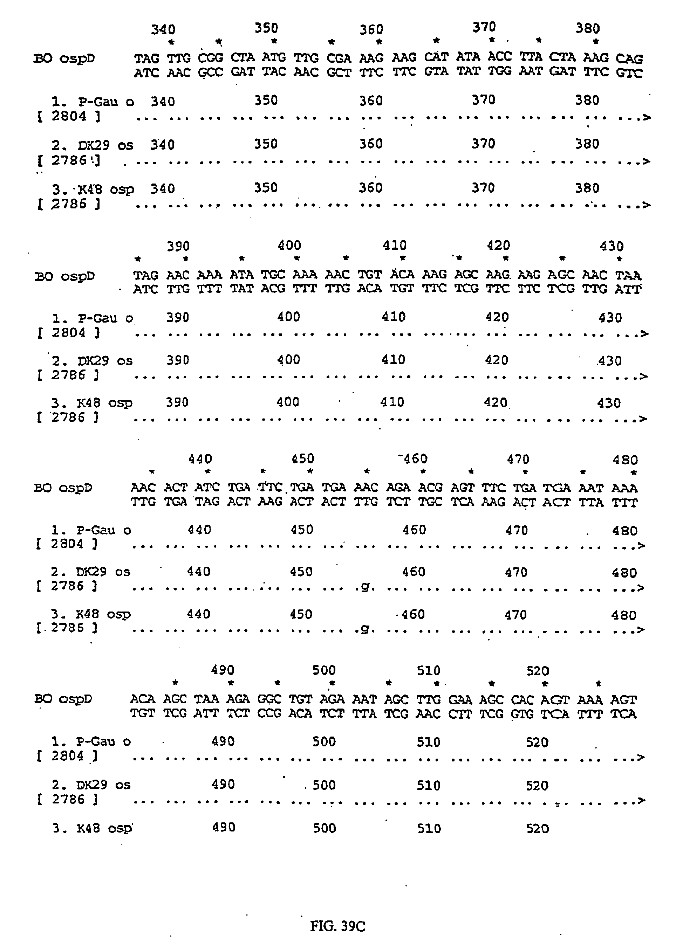

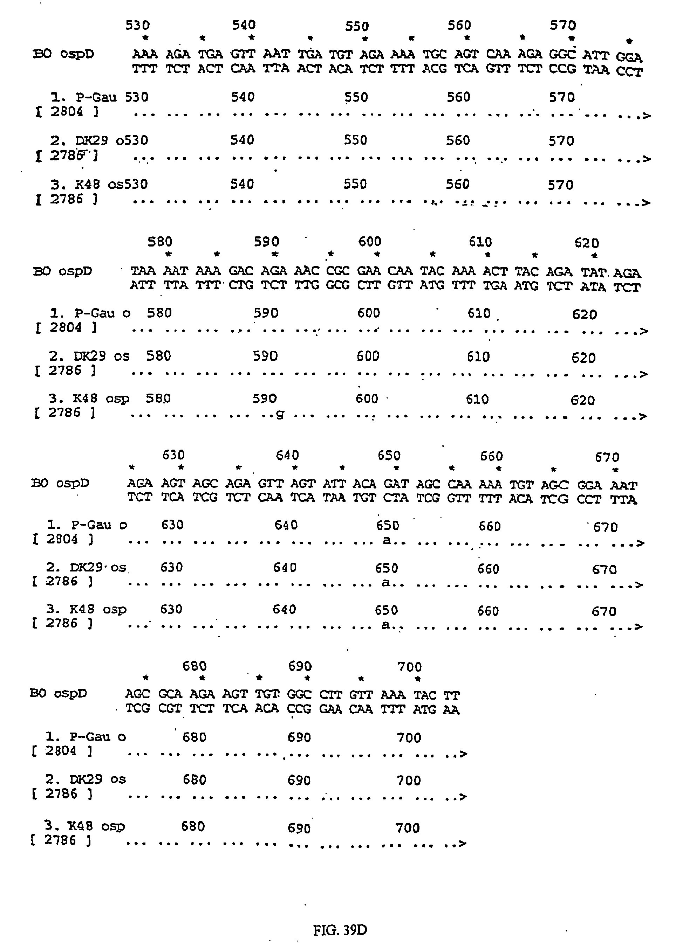

Similar methods can be used to isolate OspD genes. An alignment of four OspD nucleic acid sequences (from strains PBo, PGau, DK29, and K48) is shown in Fig. 39.

### Cloning and Sequencing of p12

The p12 gene was similarly identified. Primers used to clone the entire p12 gene included: 5'-CCGGATCCATATGGTTAAAAAAATAATATTTATTTC-3' (forward primer # 757) (SEQ ID NO. 44); and 5'-GATATCTAGATCTTTAATTGCTCTGCTCACTCTCTTC-3' (reverse primer #758) (SEQ ID NO. 45).

To amplify a truncated p12 gene (one in which the transcribed protein is non-lipidated, and begins at amino acid 18 of the native sequence), the following primers were used: 5'-CCGGGATCCATATGGCTAGTGCAATTGGTCGTGG-3' (forward primer # 759) (SEQ ID NO. 46); and primer #758 (SEQ ID NO. 45).

### Cloning and Sequencing of p41 (fla)

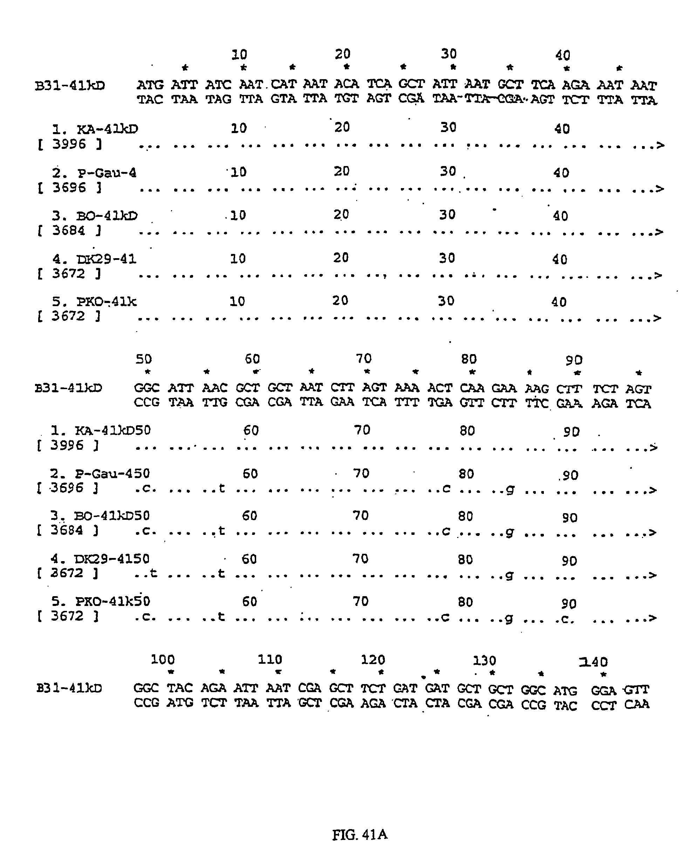

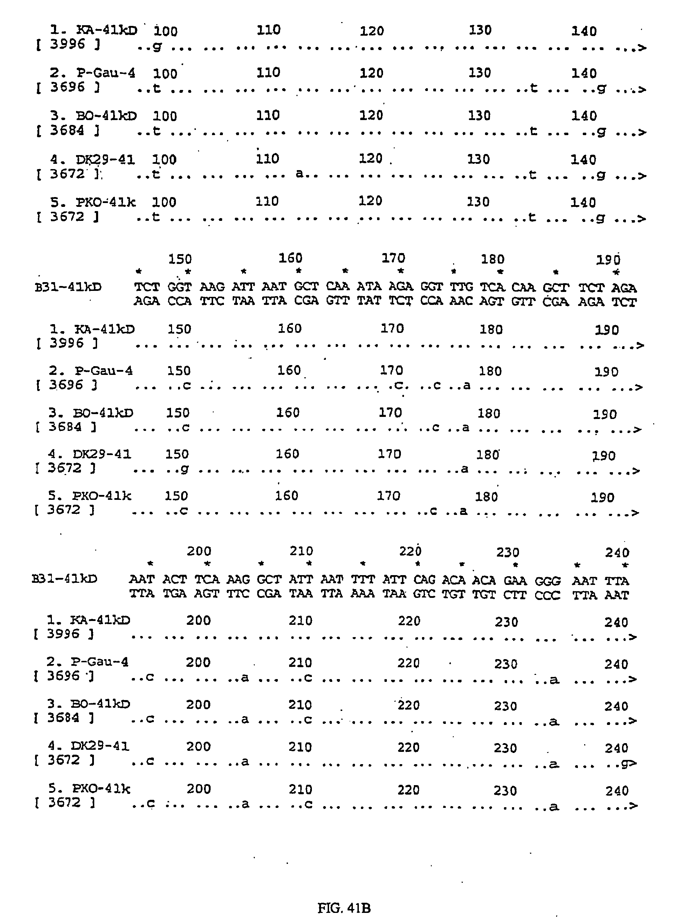

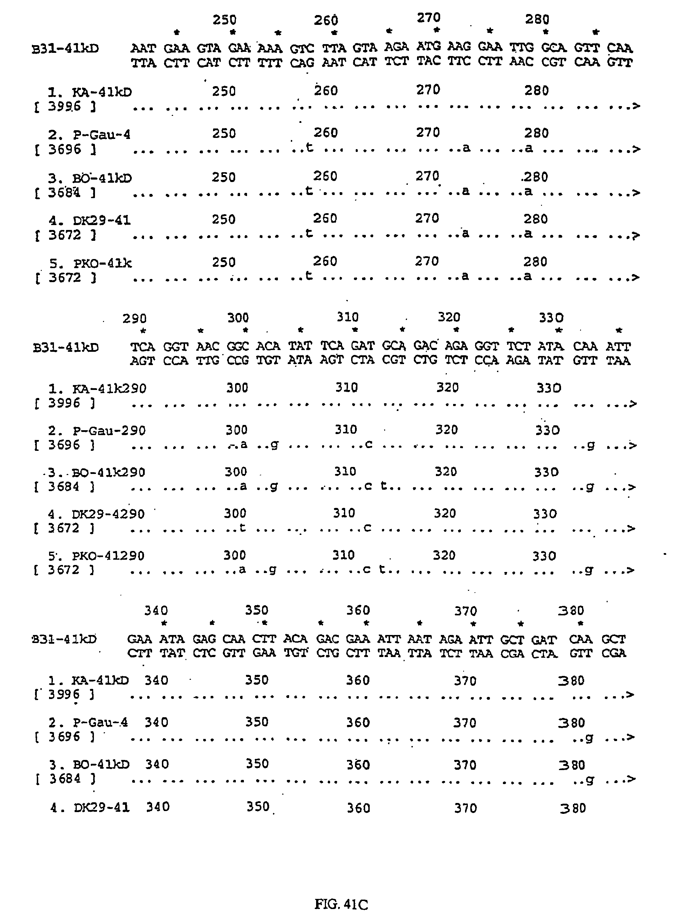

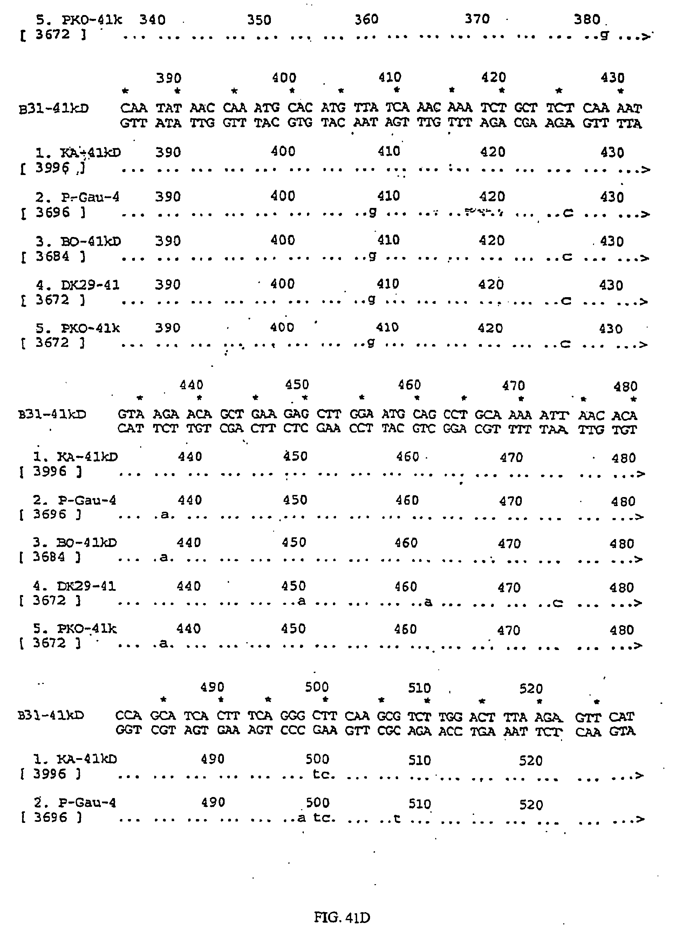

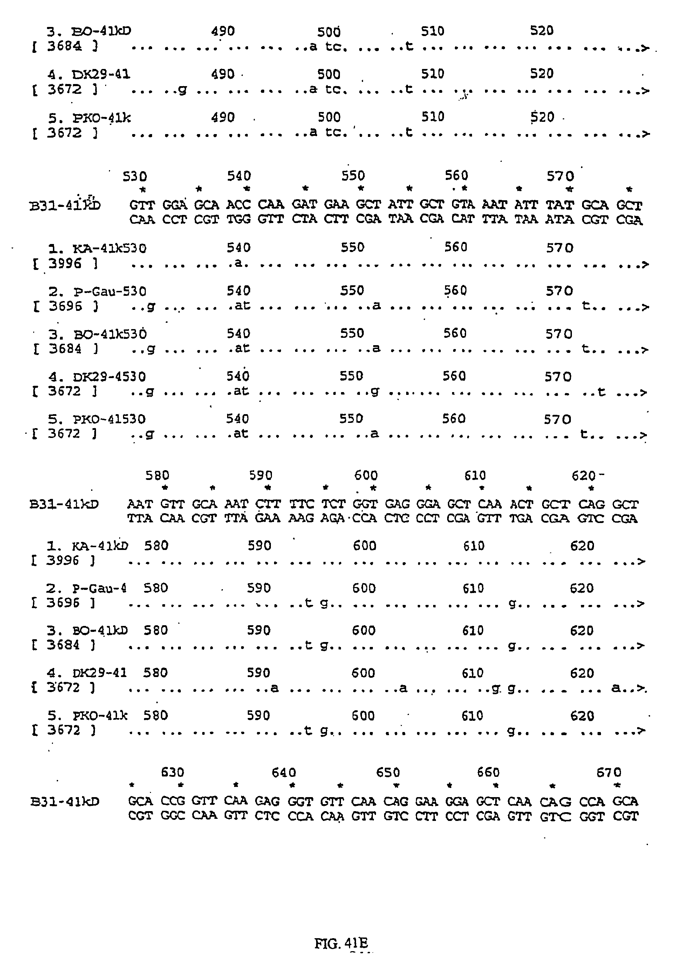

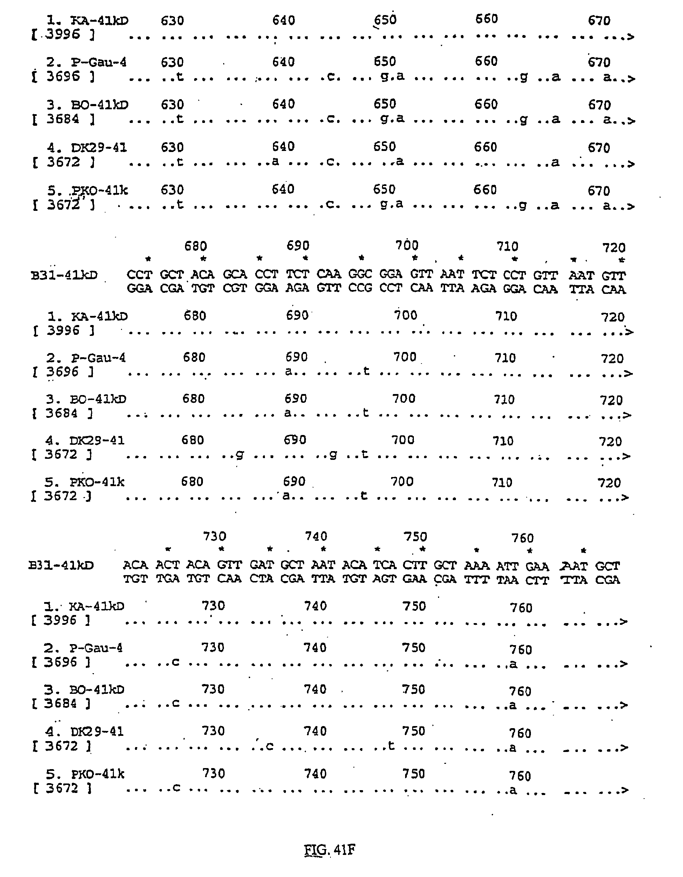

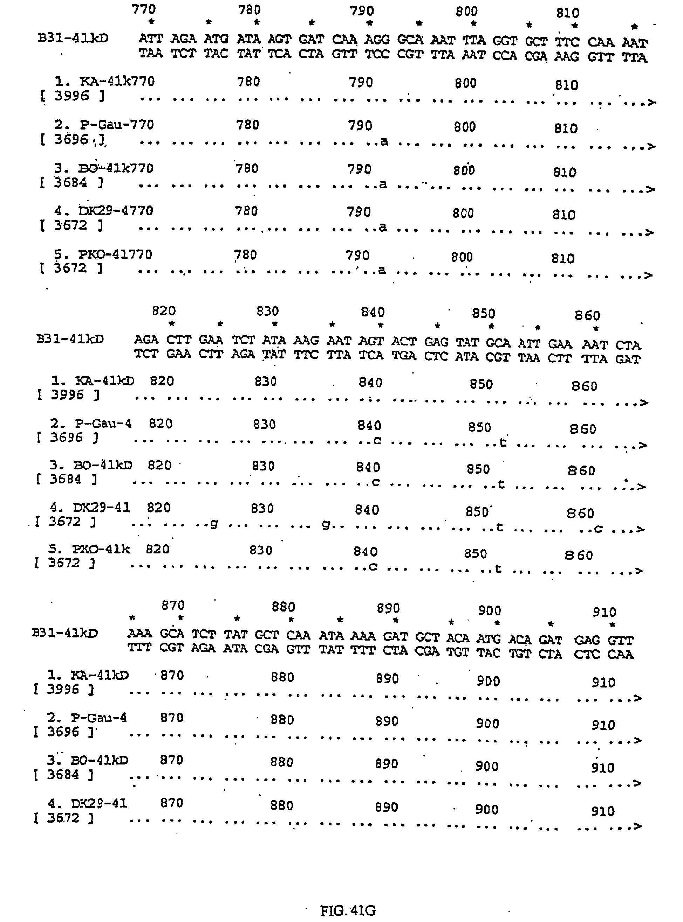

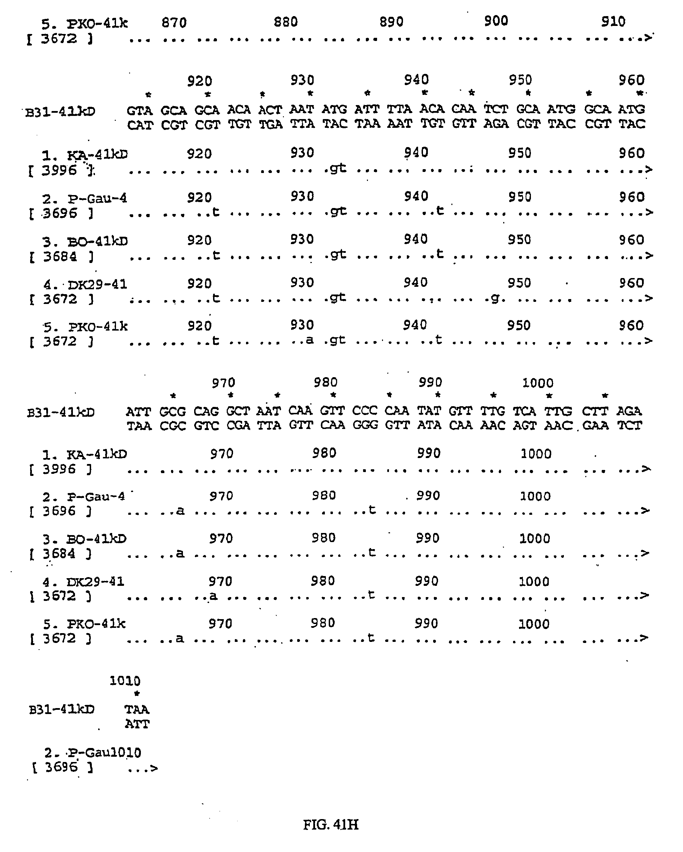

A similar approach was used to clone and sequence genes encoding the p41 (fla) protein. The p41 sequences listed in Table II with GenBank accession numbers were isolated using the following primers from strain B31:
5'-ATGATTATCAATCATAAT-3' (+1 to +18) (SEQ ID NO. 47); and 5'-TCTGAACAATGACAAAAC-3' (+1008 to +991) (SEQ ID NO. 48). The nucleic acid sequences of p41 isolated in this manner are depicted in the sequence listing as SEQ ID NO. 51 (p41-PGau), and SEQ ID NO. 53 (p41-DK29). An alignment of several p41 nucleic acid sequences, including those for strains B31, PKal, PGau, PBo, DK29, and PKo, is shown in Fig. 41. The amino acid sequences of the proteins encoded by these nucleic acid sequences are represented as SEQ ID NO. 52 (p41-PGau) and SEQ ID NO. 54 (p41-DK29).

Other primers were designed to amplify nucleic acid sequences encoding polypeptides of p41, to be used in chimeric nucleic acid sequences. These primers included:
5'-TTGGATCCGGTCACCCCATGGCTCAATATAACCAATG-3' (minus strand primer #122) (SEQ ID NO. 59);
5'-TTGGATCCGGTCACCCCATGGCTTCTCAAAATGTAAG-3' (plus strand primer # 140) (SEQ ID NO. 60);
5'-TTGGATCCGGTGACCAACTCCGCCTTGAGAAGG-3' (minus strand primer # 234) (SEQ ID NO. 61); and
5'-TTGGATCCGGTGACCTATTTGAGCATAAGATGC-3' (minus strand primer #141) (SEQ ID NO. 62).

### Cloning and Sequencing of p93

The same approach was also used to clone and sequence p93 proteins. Genes encoding p93, as listed in Table II with GenBank accession numbers, were isolated by this method with the following primers from strain B31:
5'-GGTGAATTTAGTTGGTAAGG-3' (-54 to -35) (SEQ ID NO. 63); and
5'-CACCAGTTTCTTTAAGCTGCTCCTGC-3' (+1117 to +1092) (SEQ ID NO. 64).

The nucleic acid sequences of p93 isolated in this manner are depicted in the sequence listing as SEQ ID NO. 65 (p93-B31), SEQ ID NO. 67 (p93-K48) SEQ ID NO. 69 (p93-PBo), SEQ ID NO. 71 (p93-PTrob), SEQ ID NO. 73 (p93-PGau), SEQ ID NO. 77 (p93-25015), and SEQ ID NO. 75 (p93-PKo). The amino acid sequences of the proteins encoded by these nucleic acid sequences are represented as SEQ ID NO. 66 (p93-B31), SEQ ID NO. 68 (p93-K48) SEQ ID NO. 70 (p93-PBo), SEQ ID NO. 72 (p93-PTrob), SEQ ID NO. 74 (p93-PGau), SEQ ID NO. 78 (p93-25015), and SEQ ID NO. 76 (p93-PKo).

Other primers were used to amplify nucleic acid sequences encoding polypeptides of p93 to be used in generating chimeric nucleic acid sequences. These primers included:
5'-CCGGTCACCCCATGGCTGCTTTAAAGTCTTTA-3' (plus strand primer #475) (SEQ ID NO. 79);
5'-CCGGTCACCCCATGAATCTTGATAAAGCTCAG-3' (plus strand primer #900) (SEQ ID NO. 80);
5'-CCGGTCACCCCATGGATGAAAAGCTTTTAAAAAGT-3' (plus strand primer #1168) (SEQ ID NO. 81);
5'-CCGGTCACCCCCATGGTTGAGAAATTAGATAAG-3' (plus strand primer #1423) (SEQ ID NO. 82); and
5'-TTGGATCCGGTGACCCTTAACTTTTTTTAAAG-3' (minus strand primer # 2100) (SEQ ID NO. 83).

### C. Expression of Proteins from Borrelia Genes

The nucleic acid sequences described above can be incorporated into expression plasmids, using standard techniques, and transfected into compatible host cells in order to express the proteins encoded by the nucleic acid sequences. As an example, the expression of the p12 gene and the isolation of p12 protein is set forth.

Amplification of the p12 nucleic acid sequence was conducted with primers that included a NdeI restriction site into the nucleic acid sequence. The PCR product was extracted with phenol/chloroform and precipitated with ethanol. The precipitated product was digested and ligated into an expression plasmid as follows: 15 µl (approximately 1 µg) of PCR DNA was combined with 2 µl 10X restriction buffer for NdeI (Gibco/BRL), 1 µl NdeI (Gibco/BRL), and 2 µl distilled water, and incubated overnight at 37°C. This mixture was subsequently combined with 3 µl 10X buffer (buffer 3, New England BioLabs), 1 µl BamHI (NEB), and 6 µl distilled water, and incubated at 37° for two hours. The resultant material was purified by preparative gel electrophoresis using low melting point agarose, and the band was visualized under long wave ultraviolet light and excised from the gel. The gel slice was treated with Gelase using conditions recommended by the manufacturer (Epicentre Technologies). The resulting DNA pellet was resuspended in 25-50 µl of 10 mM TRIS-CL (pH 8.0) and 1 mM EDTA (TE). An aliquot of this material was ligated into the pET9c expression vector (Dunn, J. J. *et al., Protein Expression and Purification 1:* 159 (1990)).

To ligate the material into the pET9c expression vector, 20-50 ng of p12 nucleic acid sequences cut and purified as described above was combined with 5 µl 10 One-Phor-All (OPA) buffer (Pharmacia), 30-60 ng pET9c cut with NdeI and BamHI, 2.5 µl 20 mM ATP, 2 µl T4 DNA ligase (Pharmacia) diluted 1:5 in 1X OPA buffer, and sufficient distilled water to bring the final volume to 50 µl. The mixture was incubated at 12°C overnight.

The resultant ligations were transformed into competent DH5-alpha cells and plated on nutrient agar plates containing 50 µg/ml kanamycin and incubated overnight at 37 °C. DH5-alpha is used as a "storage strain" for T7 expression clones, because it is RecA deficient, so that recombination and concatenation are not problematic, and because it lacks the T7 RNA polymerase gene necessary to express the cloned gene. The use of this strain allows for cloning of potentially toxic gene products while minimizing the chance of deletion and/or rearrangement of the desired genes. Other cell lines having similar properties may also be used.

Kanamycin resistant colonies were single-colony purified on nutrient agar plates supplemented with kanamycin at 50 µg/ml. A colony from each isolate was inoculated into 3-5 ml of liquid medium containing 50 µg/ml kanamycin, and incubated at 37°C without agitation. Plasmid DNA was obtained from 1 ml of each isolate using a hot alkaline lysis procedure (Mantiatis, T. et al., Molecular Cloning: A Laboratory Manual, Cold Spring Harbor Laboratory, Cold Spring Harbor, NY (1982)).

Plasmid DNA was digested with EcoRI and Bg1II in the following manner: 15 µl plasmid DNA was combined with 2 µl 10X buffer 3 (NEB), 1 µl EcoRI (NEB), 1 µl BglII (NEB) and 1 µl distilled water, and incubated for two hours at 37°C. The entire reaction mixture was electrophoresed on an analytical agarose gel. Plasmids carrying the p 12 insert were identified by the presence of a band corresponding to 925 base-pairs (full length p12) or 875 base-pairs (nonlipidated p12). One or two plasmid DNAs from the full length and nonlipidated p12 clones in pET9c were used to transform BL21 DE3 pLysS to kanamycin resistance as described by Studier et al. (Methods in Enzymology, Goeddel, D. (Ed.), Academic Press, 185: 60-89 (1990)). One or two transformants of the full length and nonlipidated clones were single-colony purified on nutrient plates containing 25 µg/ml chloramphenicol (to maintain pLysS) and 50 µg/ml kanamycin at 37 °C. One colony of each isolate was inoculated into liquid medium supplemented with chloramphenicol and kanamycin and incubated overnight at 37°C. The overnight culture was subcultured the following morning into 500 ml of liquid broth with chloramphenicol (25 µg/ml) and kanamycin (50 µg/ml) and grown with aeration at 37°C in an orbital air-shaker until the absorbance at 600 nm reached 0.4-0.7. Isopropyl-thio-galactoside (IPTG) was added to a final concentration of 0.5 mM, for induction, and the culture was incubated for 3-4 hours at 37° as before. The induced cells were pelleted by centrifugation and resuspended in 25 ml of 20 mM NaPO₄ (pH 7.7). A small aliquot was removed for analysis by gel electrophoresis. Expressing clones produced proteins which migrated at the 12 kDa position.

A crude cell lysate was prepared from the culture as described for recombinant OspA by Dunn, J.J. et al., (Protein Expression and Purification 1: 159 (1990)). The crude lysate was first passed over a Q-sepharose column (Pharmacia) which had been pre-equilibrated in Buffer A: 10 mM NaPO₄ (pH 7.7), 10 mM NaCl, 0.5 mM PMSF. The column was washed with 10 mM NaPO₄, 50 mM NaCl and 0.5 mM PMSF and then p 12 was eluted in 10 mM NaPO₄, 0.5 mM PMSF with a NaCl gradient from 50-400 mM. p12 eluted approximately halfway through the gradient between 100 and 200 mM NaCl. The peak fractions were pooled and dialyzed against 10 mM NaPo4 (pH 7.7), 10 mM NaCl, 0.5 mM PMSF. The protein was then concentrated and applied to a Sephadex G50 gel filtration column of approximately 50 ml bed volume (Pharmacia), in 10 mM NaPO₄, 200 mM NaCl, 0.5 mM PMSF. p12 would typically elute shortly after the excluded volume marker. Peak fractions were determined by running small aliquots of all fractions on an SDS gel. The p12 peak was pooled and stored in small aliquots at -20°C.

### Example 4. Generation of Chimeric Nucleic Acid Sequences and Chimeric Proteins

### A. General Protocol for Creation of Chimeric Nucleic Acid Sequences

The megaprimer method of site directed mutagenesis and its modification were used to generate chimeric nucleic acid sequences (Sarkar and Sommer, Biotechniques 8(4): 404-407 (1990); Aiyar, A. and J. Leis, Biotechniques 14(3): 366-369 (1993)). A 5' primer for the first genomic template and a 3' fusion oligo are used to amplify the desired region. The fusion primer consists of a 3' end of the first template (DNA that encodes the amino-proximal polypeptide of the fusion protein), coupled to a 5' end of the second template (DNA that encodes the carboxy-proximal polypeptide of the fusion protein).

The PCR amplifications are performed using Taq DNA polymerase, 10X PCR buffer, and MgCl₂ (Promega Corp., Madison, WI), and Ultrapure dNTPs (Pharmacia, Piscataway, NJ). One µg of genomic template 1, 5 µl of 10 µM 5' oligo and 5 µl of 10 µM fusion oligo are combined with the following reagents at indicated final concentrations: 10X Buffer-Mg FREE (1X), MgCl₂ (2 mM), dNTP mix (200 µM each dNTP), Taq DNA polymerase (2.5 units), water to bring final volume to 100 µl. A Thermal Cycler (Perkin Elmer Cetus, Norwalk, CT) is used to amplify under the following conditions: 35 cycles at 95°C for one minute, 55°C for two minutes, and 72° for three minutes. This procedure results in a "megaprimer".

The resulting megaprimer is run on a 1X TAE, 4% low-melt agarose gel. The megaprimer band is cut from the gel and purified using the Promega Magic PCR Preps DNA purification system. Purified megaprimer is then used in a second PCR step. One µg of genomic template 2, approximately 0.5 µg of the megaprimer, and 5 µl of 10 µM 3' oligo are added to a cocktail of 10X buffer, MgCl₂, dNTPs and Taq at the same final concentrations as noted above, and brought to 100 µl with water. PCR conditions are the same as above. The fusion product resulting from this amplification is also purified using the Promega Magic PCR Preps DNA purification system.

The fusion product is then ligated into TA vector and transformed into E. *coli* using the Invitrogen (San Diego, CA) TA Cloning Kit. Approximately 50 ng of PCR fusion product is ligated to 50 ng of pCRII vector with 1X Ligation Buffer, 4 units of T4 ligase, and brought to 10 µl with water. This ligated product mixture is incubated at 12°C overnight (approximately 14 hours). Two µl of the ligation product mixture is added to 50 µl competent INC F' cells and 2 µl beta mercaptoethanol. The cells are then incubated for 30 minutes, followed by heat shock treatment at 42°C for 60 seconds, and an ice quenching for two minutes. 450 µl of warmed SOC media is then added to the cells, resulting in a transformed cell culture which is incubated at 37°C for one hour with slight shaking. 50 µl of the transformed cell culture is plated on LB + 50 µg/µl ampicillin plates and incubated overnight at 37°C. Single white colonies are picked and added to individual overnight cultures containing 3 ml LB with ampicillin (50 µg/µl).

The individual overnight cultures are prepared using Promega's Magic Miniprep DNA purification system. A small amount of the resulting DNA is cut using a restriction digest as a check. DNA sequencing is then performed to check the sequence of the fusion nucleic acid sequence, using the United States Biochemical (Cleveland, OH) Sequenase Version 2.0 DNA sequencing kit. Three to five µg of plasmid DNA is used per reaction. 2 µl 2M NaOH/2mM EDTA are added to the DNA, and the volume is brought to 20 µl with water. The mixture is then incubated at room temperature for five minutes. 7 µl water, 3µl 3M NaAc, 75 µl EtOH are added. The resultant mixture is mixed by vortex and incubated for ten minutes at -70°C, and then subjected to microcentrifugation. After microcentrifugation for ten minutes, the supernatant is aspirated off, and the pellet is dried in the speed vac for 30 second. 6 µl water, 2 µl annealing buffer, and 2 µl of 10 µM of the appropriate oligo is then added. This mixture is incubated for 10 minutes at 37°C and then allowed to stand at room temperature for 10 minutes. Subsequently, 5.5 µl of label cocktail (described above) is added to each sample of the mixture, which are incubated at room temperature for an additional five minutes. 3.5 µl labeled DNA is then added to each sample which is then incubated for five minutes at 37°C. 4 µl stop solution is added to each well. The DNA is denatured at 95° for two minutes, and then placed on ice.

Clones with the desired fusion nucleic acid sequences are then reckoned in frame in the pET expression system in the lipidated (full length) and non-lipidated (truncated, i.e., without first 17 amino acids) forms. The product is amplified using restriction sites contained in the PCR primers. The vector and product are cut with the same enzymes and ligated together with T4 ligase. The resultant plasmid is transformed into competent E. *coli* using standard transformation techniques. Colonies are screened as described earlier and positive clones are transformed into expression cells, such as E. *coli* BL21, for protein expression with IPTG for induction. The expressed protein in its bacterial culture lysate form and/or purified form is then injected in mice for antibody production. The mice are bled, and the sera collected for agglutination, *in vitro* growth inhibition, and complement-dependent and -independent lysis tests.

### B. Specific Chimeric Nucleic. Acid Sequences

Various chimeric nucleic acid sequences were generated. The nucleic acid sequences are described as encoding polypeptides from *Borrelia* proteins. The chimeric nucleic acid sequences are produced such that the nucleic acid sequence encoding one polypeptide is in the same reading frame as the nucleic acid sequence encoding the next polypeptide in the chimeric protein sequence encoded by the chimeric nucleic acid sequence. The proteins are listed sequentially (in order of presence of the encoding sequence) in the description of the chimeric nucleic acid sequence. For example, if a chimeric nucleic acid sequence consists of bp 1-650 from OspA-1 and bp 651-820 from OspA-2 were sequenced, the sequence of the chimer would include the first 650 base pairs from OspA-1 followed immediately by base pairs 651-820 of OspA-2.

### OspA-K48/OspA-PGau

A chimer of OspA from strain K48 (OspA-K48) and OspA from strain PGau (OspA-PGau) was generated using the method described above. This chimeric nucleic acid sequence included bp 1-654 from OspA-K48, followed by bp 655-820 from OspA-PGau. Primers used included: the amino-terminal sequence of OspA primer #607 (SEQ ID NO. 16); the fusion primer,
5'-AAAGTAGAAGTTTTTGAATCCCATTTTCCAGTTTTTTT-3' (minus strand primer #668-654) (SEQ ID NO. 84); the carboxy-terminal sequence of OspA primer #586 (SEQ ID NO. 19); and the sequence primers #369 (SEQ ID NO. 14) and #357 (SEQ ID NO. 15). The chimeric nucleic acid sequence is presented as SEQ ID NO. 85; the chimeric protein encoded by this chimeric nucleic acid sequence is presented as SEQ ID NO. 86.

### OspA-B31/OspA-PGau

A chimer of OspA from strain B31 (OspA-B31) and OspA from strain PGau (OspA-PGau) was generated using the method described above. This chimeric nucleic acid sequence included bp 1-651 from OspA-B31, followed by bp 652-820 from OspA-PGau. Primers used included: the fusion primer,
5'-AAAGTAGAAGTTTTTGAATTCCAAGCTGCAGTTTT-3' (minus strand primer #668-651) (SEQ ID NO. 87); and the sequence primer, #369 (SEQ ID NO. 14). The chimeric nucleic acid sequence is presented as SEQ ID NO. 88; the chimeric protein encoded by this chimeric nucleic acid sequence is presented as SEQ ID NO. 89.

### OspA-B31/OspA-K48

A chimer of OspA from strain B31 (OspA-B31) and OspA from strain K48 (OspA-K48) was generated using the method described above. This chimeric nucleic acid sequence included bp 1-651 from OspA-B31, followed by bp 652-820 from OspA-K48. Primers used included: the fusion primer,
5'-AAAGTGGAAGTTTTTGAATTCCAAGCTGCAGTTTTTTT-3' (minus strand primer #671-651) (SEQ ID NO. 90); and the sequence primer, #369 (SEQ ID NO. 14). The chimeric nucleic acid sequence is presented as SEQ ID NO. 91; the chimeric protein encoded by this chimeric nucleic acid sequence is presented as SEQ ID NO. 92.

### OspA-B31/OspA-25015

A chimer of OspA from strain B31 (OspA-B31) and OspA from strain 25015 (OspA-25015) was generated using the method described above. This chimeric nucleic acid sequence included bp 1-651 from OspA-B31, followed by bp 652-820 from OspA-25015. Primers used included: the fusion primer, 5'-TAAAGTTGAAGTGCCTGCATTCCAAGCTGCAGTTT-3' (SEQ ID NO. 93). The chimeric nucleic acid sequence is presented as SEQ ID NO. 94; the chimeric protein encoded by this chimeric nucleic acid sequence is presented as SEQ ID NO. 95.

### OspA-K48/OspA-B31/OspA-K48

A chimer of OspA from strain B31 (OspA-B31) and OspA from strain K48 (OspA-K48) was generated using the method described above. This chimeric nucleic acid sequence included bp 1-570 from OspA-K48, followed by bp 570-651 from OspA-B31, followed by bp 650-820 from OspA-K48. Primers used included: the fusion primer, 5'-CCCCAGATTTTGAAATCTTGCTTAAAACAAC-3' (SEQ ID NO. 96); and the sequence primer, #357 (SEQ ID NO. 15). The chimeric nucleic acid sequence is presented as SEQ ID NO. 97; the chimeric protein encoded by this chimeric nucleic acid sequence is presented as SEQ ID NO. 98.

### OspA-B31/OspA-K48/OspA-B31/OspA-K48

A chimer of OspA from strain B31 (OspA-B31) and OspA from strain K48 (OspA-K48) was generated using the method described above. This chimeric nucleic acid sequence included bp 1-420 from OspA-B31, followed by 420-570 from OspA-K48, followed by bp 570-650 from OspA-B31, followed by bp 651-820 from OspA-K48. Primers used included: the fusion primer, 5'-CAAGTCTGGTTCCAATTTGCTCTTGTTATTAT-3' (minus strand primer #436-420) (SEQ ID NO. 99); and the sequence primer, #357 (SEQ ID NO. 15). The chimeric nucleic acid sequence is presented as SEQ ID NO. 100; the chimeric protein encoded by this chimeric nucleic acid sequence is presented as SEQ ID NO. 101.

### OspA-B31/OspB-B31

A chimer of OspA and OspB from strain B31 (OspA-B31, OspB-B31) was generated using the method described above. The chimeric nucleic acid sequence included bp 1-651 from OspA-B31, followed by bp 652-820 from OspB-B31. Primers used included: the fusion primer, 5'-GTTAAAGTGCTAGTACTGTCATTCCAAGCTGCAGTTTTTTT-3' (minus strand primer #740-651) (SEQ ID NO. 102); the carboxy-terminal sequence of OspB primer #1106 (SEQ ID NO. 25); and the sequence primer #357 (SEQ ID NO. 15). The chimeric nucleic acid sequence is presented as SEQ ID NO. 103; the chimeric protein encoded by this chimeric nucleic acid sequence is presented as SEQ ID NO. 104.

### OspA-B31/OspB-B31/OspC-B31

A chimer of OspA, OspB and OspC from strain B31 (OspA-B31, OspB-B31, and OspC-B31) was generated using the method described above. The chimeric nucleic acid sequence included bp 1-650 from OspA-B31, followed by bp 652-820 from OspB-B31, followed by bp 74-630 of OspC-B31. Primers used included: the fusion primer, 5'-TGCAGATGTAATCCCATCCGCCATTTTTAAAGCGTTTTT-3' (SEQ ID NO. 105); and the carboxy-terminal sequence of OspC primer (SEQ ID NO. 28). The chimeric nucleic acid sequence is presented as SEQ ID NO. 106; the chimeric protein encoded by this chimeric nucleic acid sequence is presented as SEQ ID NO. 107.

### OspC-B31/OspA-B31/OspB-B31

A chimer of OspA, OspB and OspC from strain B31 (OspA-B31, OspB-B31, and OspC-B31) was generated using the method described above. The chimeric nucleic acid sequence included bp 1-630 from OspC-B31, followed by bp 52-650 from OspA-B31, followed by bp 650-820 of OspB-B31. Primers used included: the amino-terminal sequence of OspC primer having SEQ ID NO. 27; the fusion primer, 5'-GCTGCTAACATTTTGCTTAGGTTTTTTTGGACTTTC-3' (minus strand primer #69-630) (SEQ ID NO. 108); and the sequence primers #520 (SEQ ID NO. 40) and #200 (SEQ ID NO. 18). The chimeric nucleic acid sequence is presented as SEQ ID NO. 109; the chimeric protein encoded by this chimeric nucleic acid sequence is presented as SEQ ID NO. 110.

### Additional Chimeric Nucleic Acid Sequences

Using the methods described above, other chimeric nucleic acid sequences were produced. These chimeric nucleic acid sequences, and the proteins encoded, are summarized in Table III.

**Table III. Chimeric Nucleic acid Sequences and the Encoded Proteins**

| Chimers Generated (base pairs) | SEQ ID NO. (nt) | SEQ ID NO. (protein) |
|---|---|---|
| OspA (52-882) / p93 (1168-2100) | 111 | 112 |
| OspB (45-891) / p41 (122-234) | 113 | 114 |
| OspB (45-891) / p41 (122-295) | 115 | 116 |
| OspB (45-891) / p41 (140-234) | 117 | 118 |
| OspB (45-891) / p41 (140-295) | 119 | 120 |
| OspB (45-891) / p41 (122-234) / OspC (58-633) | 121 | 122 |
| OspA-Tro/OspA-Bo | 137 | 138 |
| OspA-PGau/OspA-Bo | 139 | 140 |
| OspA-B31/OspA-PGau/OspA-B31/ OspA-K48 | 143 | 144 |
| OspA-PGau/OspA-B31/OspA-K48 | 141 | 142 |

### C. Purification of Proteins Generated by Chimeric Nucleic Acid Sequences

The chimeric nucleic acid sequences described above, as well as chimeric nucleic acid sequences produced by the methods described above, are used to produce chimeric proteins encoded by the nucleic acid sequences. Standard methods, such as those described above in Example 3, concerning the expression of proteins from *Borrelia* genes, can be used to express the proteins in a compatible host organism. The chimeric proteins can then be isolated and purified using standard techniques.

If the chimeric protein is soluble, it can be purified on a Sepharose column. Insoluble proteins can be solubilized in guanidine and purified on a Ni²⁺ column; alternatively, they can be solubilized in 10 mM NaPO₄ with 0.1 - 1% TRIXON X 114, and subsequently purified over an S column (Pharmacia). Lipidated proteins were generally purified by the latter method. Solubility was determined by separating both soluble and insoluble fractions of cell lysate on a 12% PAGE gel, and checking for the localization of the protein by Coomasie staining, or by Western blotting with monoclonal antibodies directed to an antigenic polypeptide of the chimeric protein.

### Example 5. Generation of OspC/OspA Chimeric Nucleic Acids and Chimeric Proteins

### A. General Protocol for Creation of Chimeric Nucleic Acid Sequences

A large number of chimeric nucleic acid sequences encoding proteins comprising at least a first and a second polypeptide from *Borrelia burgdorferi* were generated. These chimeric nucleic acid sequences were produced such that the encoded chimeric protein comprised a *Borrelia burgdorferi* OspC polypeptide upstream of (or N-terminal to) a *Borrelia burgdorferi* OspA polypeptide. The chimeric nucleic acid sequences were also produced such that the nucleic acid encoding one polypeptide was in the same reading frame as the nucleic acid sequence encoding the next polypeptide in the chimeric protein.

The general cloning strategy used to construct the chimeric nucleic acid sequences. The desired fragment of OspC was amplified using a 5' primer containing a restriction site suitable for cloning the resultant product into a vector of interest and a 3' primer containing a restriction site suitable for ligating the OspC fragment to the OspA fragment. The OspC product is cloned into a suitable vector. For the OspA poriton of the chimeric nucleic acid, the desired OspA fragment was amplified using a 5' primer containing a restriction site for ligating the resultant OspA fragment to the OspC fragment and a 3' primer containing a restriction site suitable for cloning the resultant OspA product into the vector with the OspC product. The use of a restriction site to allow ligation of the OspC and OspA fragment results in the insertion of 0 to about 3 amino acids between the OspC and OspA fragments.

A specific example of such a construction follows. It is understood that other suitable restriction sites could be used with no more than routine experimentation. The resultant OspC/OspA chimers could have, therefore, the addition of 0 to about 3 amino acids between the OspC and OspA fragments, depending on the restriction site used.

For the OspC portions of the chimeric nucleic acids, desired fragments of OspC genes from various strains or genospecies were PCR amplified using a 5' primer containing an NdeI site and a 3' primer containing a NcoI and a BamHI site. The amplified OspC product was then cloned into the NdeI and BamHI sites of the T7 promoter driven expression vector, pET9c. For the OspA portion of the chimeric nucleic acid, desired fragments of OspA genes a strain of interest or genospecies of interest were PCR amplified using a 5' primer containing an NcoI site and a 3' primer containing a BamHI site. This OspA portion could then be directly cloned into the NcoI and BamHI sites of the pET9c vector containing the desired OspC sequence, thereby producing the desired OspC-OspA construct. By including the sequence for the NcoI restriction site in the primers, a nine nucleotide linker sequence encoding the amino acids Ser-Met-Ala was produced at the junction between the N-terminal OspC sequence and the C-terminal OspA sequence. The use of the NcoI restriction enzyme (CCATGG) in this cloning strategy was a suitable choice as *Borrelia* DNA is an AT-rich and therefore possesses only a few NcoI sites in its genome.

As an example, OspC-OspA chimeric nucleic acids which contain unlipidated OspC B31 were generated using the following primers:
(5'OspC-NdeI): 5'-GT CAT ATG GCT TGT AAT AAT TCA GGG AAA GA-3' (SEQ ID NO:181); and
(3'OspC-Ncol): 5'-T TTC CAT GGA AGG TTT TTT TGG ACT TTC TG-3' (SEQ ID NO:182).

For OspC-OspA chimeric nucleic acids which contain unlipidated OspA B31, the following primers were used:
(5'OspA-NcoI:) 5'-TT TCC ATG GCC AAG CAA AAT GTT AGC AGC C-3' (SEQ ID NO: 183); and
(3'OspA-BamHI): 5'-TAA GGA TCC TTA TTT TAA AGC GTT TTT-3' (SEQ ID NO: 184).

Lipidated versions of the OspC/A chimeras can be constructed by cloning an expression vector that contains a leader sequence containing a lipidation site, such that the leader sequence is linked upstream of the OspC portion of the chimera and in frame with the chimera. The leader sequence comprising a lipidation signal can be, for example, from a gene encoding the OspA, B or C polypeptides.

Chimeric nucleic acid sequences, and the proteins that they encode, which were produced are summarized in table IV. Other additional chimeric nucleic acid sequences, and encoded proteins, are also depicted in Table IV. In further embodiments, chimeric OspC/OspA proteins are constructed wherein a first segment of OspA is from B31 and comprises base pairs from about 88 to about 450, and a second segment of OspA comprises base pairs from about 451 to about 537 of PKo. These chimeras can also comprise additional OspA segments such as the last two segments of SEQ ID NOs 167 or 165 or the last segment of SEQ ID NO: 163.

**Table IV. Chimeric OspC/OspA Nucleic Acid Sequences and Encoded Proteins**

| ¹Chimers Generated | SEQ ID NO. (nt) | SEQ ID NO. (protein ) | FIG. NO. |
|---|---|---|---|
| OspC-B31(bp55-633)/OspA-B31(bp52-822) | 145 | | 55 |
| | | | |
| OspC-B31(aa19-211)/OspA-B31(aa18-273) | | 146 | 55 |
| OspC-B31(bp55-624)/OspA-B31(bp52-822) | 147 | | 56 |
| | | | |
| OspC-B31(aa19-208)/OspA-B31(aa18-273) | | 148 | 56 |
| OspC-C2(bp55-612)/OspA-B31(bp52-822) | 149 | | 57 |
| | | | |
| OspC-C2(aa19-204)/OspA-B31(aa18-273) | | 150 | 57 |
| OspC-B31(bp55-633)/OspA-B31(bp52-651)/OspA-K48(bp652-820) | 151 | | 58 |
| | | | |
| OspC-B3 1(aa19-211)/OspA-B31(aa18-216)/OspA-K48(aa217-273) | | 152 | 58 |
| OspC-C2(bp55-612)/OspA-B31 (bp52-651)/OspA-K48(bp652-820) | 153 | | 59 |
| | | | |
| OspC-C2(aa19-204)/OspA-B31 (aa18-216)/OspA-K48(aa217-273) | | 154 | 59 |
| OspC-B31(bp55-633)/OspA-B31 (bp52-651)/OspA-PKo(bp652-820) | 155 | | 60 |
| | | | |
| OspC-B31(aa19-211)/OspA-B31(aa18-216)/OspA-PKo(aa217-273) | | 156 | 60 |
| OspC-C2(bp55-612)/OspA-B31(52-651)/OspA-PKo(bp652-820) | 157 | | 61 |
| | | | |
| OspC-C2(aa19-204)/OspA-B31 (aal 8-216)/OspA-PKo(aa217-273) | | 158 | 61 |
| OspC-B31(bp55-633)/OspA-K48(bp52-654)/OspA-Tro(bp655-819) | 159 | | 62 |
| | | | |
| OspC-B31(aa19-211)/OspA-K48(aa18-217)/OspA-Tro(aa218-273) | | 160 | 62 |
| OspC-C2(bp55-612/OspA-K48(bp52-654)/OspA-Tro(bp655-819) | 161 | | 63 |
| | | | |
| OspC-C2(aa19-204)/OspA-K48(aa18-217)/OspA-Tro(aa218-273) | | 162 | 63 |
| OspC-C12(bp55-612)/OspA-B31(bp88-492)/OspA-PKo(bp493-537)/OspA-B31(bp538-822) | 163 | | 64 |
| | | | |
| OspC-C12(aal9-204)/OspA-B31(aa30-164)/OspA-PKo(aa165-179)/OspA-B31(aa180-273) | | 164 | 64 |
| OspC-PKo(bp55-639)/OspA-B31(bp88-492)/OspA-PKo(bp493-537)/OspA-B31(bp538-651)/OspA-K48(bp652-825) | 165 | | 65 |
| | | | |
| OspC-PKo(aa19-213)/OspA-B31(aa30-164)/OspA-PKo(aa165-179)/OspA-B31(aa180-216)/OspA-K48(aa217-274) | | 166 | 65 |
| OspC-Tro(bp55-624)/OspA-B31(bp88-492)/OspA-PKo(bp493-537)/OspA-B31(bp538-651)/OspA-PKo(bp652-822) | 167 | | 66 |
| | | | |
| OspC-Tro(aa19-208)/OspA-B31(aa30-164)/OspA-PKo(aa165-179)/OspA-B31(aa180-216)/OspA-PKo(aa217-273) | | 168 | 66 |
| OspC-B31(bp55-633)/OspA-B31(bp394-820) | 169 | | 67 |
| | | | |
| OspC-B31(aa19-211)/OspA-B31(aa132-273) | | 170 | 67 |
| OspC-B31(bp55-631)/OspA-B31(bp394-651)/OspA-K48(652-820) | 171 | | 68 |
| | | | |
| OspC-B31(aa19-211)/OspA-B31(aa132-216)/OspA-K48(217-273) | | 172 | 68 |
| OspC-B31 (bp55-633)/OspA-B31 (bp394-651)/OspA-PKo(652-820) | 173 | | 69 |
| | | | |
| OspC-B31 (aa19-211)/OspA-B31 (aa132-216)/OspA-PKo(217-273) | | 174 | 69 |
| OspC-B3 1(bp55-633)/OspA-K48(bp394-654)/OspA-Tro(655-819) | 175 | | 70 |
| | | | |
| OspC-B31(aa19-211)/OspA-K48(aa132-217)/OspA-Tro(218-273) | | 176 | 70 |
| OspC-B31 (bp55-633)/OspA-B31 (bp88-492)/OspA-PKo(bp493-537)/OspA-B31 (bp541-651)/OspA-PKo(bp652-822) | 177 | | 71 |
| | | | |
| OspC-B31(aa19-211)/OspA-B31(aa30-164)/OspA-PKo(aa165-179, aa164(K>G))/OspA-B31(aa180-216)(aa190(N-del))/OspA- PKo(aa217-273) | | 178 | 71 |
| OspC-C2(bp55-612)/OspA-B31(bp88-492)/OspA-PKo(bp493-537)/OspA-B31(bp541-651)/OspA-PKo(bp652-822) | 179 | | 72 |
| | | | |
| OspC-C2(aa19-204)/OspA-B31(aa30-164)/OspA-PKo(aa165-179, aa164(K>G))/OspA-B31(aa180-216)(aa190(N-del))/OspA-PKo(aa217-273) | | 180 | 72 |

| | | | |
|---|---|---|---|
| ¹Chimers Generated are listed as follows: Nucleotide or polypeptide fragment -strain (sequence in base pairs for the top listing and amino acids for the bottom listing) | | | |

Separate nucleotide or polypeptide fragments in the chimer are separated by a /

### B. Protein Expression

As described in the previous two examples, it is possible to express and purify *Borrelia* proteins such as OspA, OspC and chimeric OspC/OspA polypeptides. This is accomplished by incorporating the desired nucleic acid sequence, which encodes the protein of choice, into an expression plasmid, using standard techniques. This expression plasmid can then be transfected into a compatible host cell in order to express the desired protein.

The purified OspA, OspC or OspC/OspA chimeric proteins, that were used to immunize mice and in the ELISA tests described below, were generated and purified by cloning either OspA, OspC or OspC/OspA chimeric nucleic acid sequences, in frame, into the pET expression plasmid. The expression plasmid was then transfected into the compatible expression cell line *Escherichia coli* strain BL21 (DE3)/(pLysS) or strain B834 (DE3). The BL21 or B834 cells were then grown in 10 ml LB media (5 g/l NaCl, 10 g/l tryptone, 5 g/l yeast extract, 25 mg/l chloramphenicol and 50 mg/l ampicillin) at 37°C, with shaking. When the optical density at 600λ reached 0.3-0.4 units, recombinant protein expression was induced by adding IPTG (isopropyl B-D-thiogalactopyranoside) to a final concentration of 0.5 mM and the cells were grown for an additional three hours. The cultures were harvested by centrifugation at 3800xg for five minutes. The cells were resuspended in 20 mM NaPO₄, pH7.7 and stored at -20°C overnight. Once thawed, the crude extracts were incubated with DNase (2 µg/ml) in the presence of 2.5 mM MgCl₂ at room temperature for thirty minutes and then spun at 14,000 rpm (Eppendorf 5417C) for five minutes.

To purify the OspC proteins described below, the crude extracts from the OspC-expressing cells were loaded onto an anion exchange column (Q Sepharose Fast Flow, 2.2x10cm, Pharmacia) which had been pre-equilibrated with 20 mM Tris-Cl, pH 9.3. The column was washed in the same buffer (20 mM Tris-Cl, pH 9.3) which eluted the OspC protein. The wash fractions that contained OspC were concentrated using Amicon 10K and then were dialyzed with a solution containing 20 mM NaPO₄ pH 8.0 and 250 mM NaCl. The partially purified OspC was then passed over a Ni²⁺ metal affinity column (Chelating Sepharose Fast Flow 2.2x10 cm, Pharmacia) equilibrated with 20 mM NaPO₄ pH 8.0 and 250 mM NaCl. The column was washed using a decreasing pH gradient of 20 mM sodium acetic acid and 250 mM NaCl and the bound OspC eluted around pH 5.7. The OspC fractions were then concentrated by ultrafiltration and stored at -70°C.

For purification of OspA proteins, the same procedure was followed, except that the dialysis step, after the Amicon 10K cutoff, was done in 20 nM NaPO₄, pH 6.0. The partially purified OspA was then applied to a cation exchange column (S Sepharose Fast Flow 2.2x10 cm, Pharmacia) equilibrated with 20 nM NaPO₄, pH 6.0. The column was washed using an increasing NaCl gradient from 0 to 100 mM. The OspA-containing fractions were concentrated by ultrafiltration and stored at - 70°C.

As previously indicated, both lipidated and non-lipidated (truncated, i.e., without the first 17 amino acids) forms of OspC, OspA and OspC/OspA chimeric proteins were generated.

### C. Immunization of Mice and Serologic Characterization Using ELISA (Enzyme-Linked Immunosorbent Assay)

### Immunization of Mice

Mice, either C3H-J or ICR, were immunized with 3 ug of lipidated OspC/OspA chimeric protein or 6 ug of non-lipidated OspC/OspA chimers in 100 ul of aluminum hydroxide adjuvant (concentration of 1.8 mg/ml) by (SC) subcutaneous injection. As a negative control, mice were immunized with 100 ul of aluminum hydroxide adjuvant only. All mice received a total of three injections which were given at two week intervals. One week after the final immunization, blood was drawn from each mouse (including negative controls) and the serum was tested for IgG reacivity using the ELISA method described below, for the presence of anti-OspA antibodies to three different purified OspA proteins *(Borrelia burgdorferi* sensu stricto (B31), *Borrelia garinii* (K48) and *Borrelia afzelli* (PGau). The sera was tested at a dilution of 1:1000.

Mice were immunized with the chimeric proteins described in Table V.

**Table V. Chimeric Proteins Used to Immunize Mice**

| Name | Description (amino acid) | SEQ ID NO.: (nucleic acid) | SEQ ID NO.: (polype p-tide) | Fig. No: |
|---|---|---|---|---|
| OspA | OspA-B31(18-273) | 6 | 7 | 47,48 |
| OspC | OspC-B31(19-211) | 29 | 30 | 47,48 |
| OspC2-OspA | OspC-C2(19-204)/OspA-B31(18-273) | 149 | 150 | 47,48 |
| ¹lipOspAP/Bo | OspA-PGau(1-217)/OspA-Bo(218-273) | 139 | 140 | 49,50 |
| ¹lipOspAB/P | OspA-B31(1-216)/OspA-PKo(217-273) | * | * | 49, 50 |
| OspC-OspAB/P | OspC-B31(19-211)/OspA-B31(18-216)/OspA-PKo(217-273) | 155 | 156 | 49, 50, 52, 53, 54 |
| OspCB31-OspAB31 | OspC-B31(19-211)/OspA-B31(18-273) | 145 | 146 | 51, 52, 53,54 |
| OspC2-OspAB31 | OspC-C2(19-204)/OspA-B31(18-273) | 149 | 150 | 51,52 |
| ¹lip OspA K/T | OspA-K48(1-217)/OspA-Tro(218-273) | * | * | 52 |
| ¹lip OspC-B31 | OspC-B31(1-211) | 29 | 30 | 51 |
| OspCB31-OspABPBP | OspC-B31(19-211)/OspA-B31(30-150)/OspA-PKo(151-179)/OspA-B31(180-216) (190 N deletion)/OspA-PKo(217-273) B31/B31/PKo | 177 | 178 | 53,54 |

| | | | | |
|---|---|---|---|---|
| ¹"lip" means the chimeric protein contains its native N-terminal lipidation signal | | | | |

### Serologic Characterization Using ELISA (Enzyme-Linked Immunosorbent Assay)

### Immobilization of antigen onto ELISA Plates

A solution of purified recombinant OspC or OspA protein from each of the *Borrelia burgdorferi* strains B31 *(Borrelia burgdorferi* sensu stricto), K48 *(Borrelia garinii)* and PGau *(Borrelia afzelli)* was added to sodium phosphate buffer, pH 9.0, and was used to coat a commercial microwell plate (MaxiSorp®, Nunc). The coating procedure was as follows: 100 µl of a solution containing the appropriate OspA or OspC protein (made up at a concentration of 250 ng/ml in the following coating buffer: 100 mM Bis-Tris propane, pH 9.7) was added to each well of a microtiter plate which was incubated for one hour at 37°C. The antigen solution was removed from the wells, the plate was washed three times with phosphate buffered saline (PBS) pH 9.0, and 300 µl of blocking buffer solution was added (3% dry milk, 0.1 % polyoxyethylenesorbitan (referred to herein as Tween 20^{™}), 0.02% NaN₃ in 100 nM Bis-Tris propane, pH 9.7). Following a one hour incubation at 37°C, the plates were washed four times with TBS-Tween 20^{™} wash buffer (20 mM Tris-Cl, pH 7.5, 136 mM NaCl, 0.1% Tween 20^{™} and 0.02% NaN₃) and then were allowed to dry. The plates were then wrapped in plastic and stored at 4°C until they were used.

### ELISA (Enzyme-Linked Immunosorbent Assay) Tests

The standard procedure for the ELISA tests was as follows: mouse serum was diluted 1:1000 in sample dilution buffer (1% dry milk, 136 mM NaCl, 0.1% Tween 20^{™}, 0.02% NaN₃ in 20 mM Tris-Cl, pH7.5) and 100 µl of the diluted serum was added to the ELISA microtiter plate wells that had been coated with antigen as described above. Following incubation for 1 hour at 37°C, the samples were removed and the plates were washed four times in TBS-Tween^{™} (20 mM Tris-Cl, pH 7.5; 136 mM NaCl; 0.1% Tween 20^{™} and 0.02% NaN₃). For the secondary antibody, goat anti-mouse antisera conjugated to alkaline phosphatase-specific for either IgM (Fc) or IgG (Fab), (Jackson Immuno Research Laboratories) was diluted 1:750 in sample dilution buffer (1% dry milk, 136 mM NaCl, 0.1% Tween 20^{™}, 0.02% NaN₃ in 20 mM Tris-Cl, pH7.5) and 100 µl of the diluted secondary antibody was added to each well. Following incubation for thirty minutes at 37°C, the plates were washed three times with TBS-Tween^{™} (20 mM Tris-Cl, pH 7.5; 136 mM NaCl; 0.1% Tween 20^{™} and 0.02% NaN₃) and 100 µl of Phosphatase Substrate solution (5 mg ofp-nitrophenylphosphate tablets dissolved in 1X diethanolamine substrate buffer to yield a 2 mg/ml solution - Kirkegaard Perry Laboratory) was added to each well. The plates were incubated for thirty minutes at 37°C and 100 µl of stop solution (5 % EDTA) was added to each well. The absorbance at 405 nm was read on a microplate reader (Dynatech). A sample was considered positive if it produced an average absorbance greater than the mean of the negative controls plus three standard deviations.

Previous work has demonstrated that it is the carboxy-terminal region of OspA that contains the antigenic sites that provide the immunoprotective response. Thus, in addition to the ELISA test described above, a modified ELISA was performed (herein referred to as the Protective ELISA Test), wherein the purified N-terminal region of B31 OspA (amino acids 18-139) was used to block any antibodies present in the mouse serum that had specificity to this N-terminal OspA region. These protective ELISA tests were performed as above, except that 80 µg/ml of a purified B31 OspA fragment (amino acids 18-139) was added to the diluted mouse serum prior to adding the sera to the antigen-coated ELISA microtiter plate wells.

### Results of ELISA Tests

Using the above-described ELISA tests, it was demonstrated that mice immunized with a non-lipidated OspC/OspA chimeric protein (OspC2-OspA-composed of OspC (a.a.19-204 from strain C2)/OspA (a.a. 18-273 from strain B31) (SEQ ID NO. 150) produced an immune response both to OspA and OspC that was comparable to the immune response generated to non-lipidated OspA (OspA - a.a. 18-273 from strain B31) and non-lipidated OspC (OspC - a.a. 19-211 from strain B31) control proteins (Fig. 47). As indicated in Fig. 47 and described above, mice were immunized with OspA, OspC or OspC2-OspA proteins and immune responses of the sera were measured against B31 OspA antigen (stippled bars) and B31 OspC antigen (solid bars).

Using the above-described Protective ELISA Test, it was also shown that mice immunized with the same non-lipidated OspC/OspA chimeric protein (OspC2-OspA - composed of OspC (a.a. 19-204 from strain C2)/OspA (a.a. 18-273 from strain B31) (SEQ ID NO.150) produced an immune response to the C-terminal portion of OspA that was comparable to the immune response generated to the C-terminal portion of a non-lipidated OspA (OspA - a.a. 18-273 from strain B31) control protein (Fig. 48). As indicated in Fig. 48, mice were immunized with OspA, OspC or OspC2-OspA proteins and immune responses of the sera were measured against B31 OspA antigen. The protective antibody response to B31 OspA antigen is indicated in the stippled bars.

Thus, these results clearly demonstrate that non-lipidated chimeric OspC/OspA proteins are able to induce immune responses in mice that are comparable to the immune response generated against non-lipidated OspC and OspA control proteins.

It had been previously thought that the lipidation signals that are present on *Borrelia burgdorferi* outer surface proteins were required for immunogenicity and that OspC and OspA proteins that lacked this lipidation signal would be less or non-immunogenic. To test this idea, mice were immunized with a non-lipidated OspC/OspA chimeric protein (OspC-OspAB/P - composed of OspC (a.a.19-211 from strain B31)/OspA (a.a. 18-216 from strain B31)/OspA (a.a. 217-273 from strain PKo)(SEQ ID NO: 156) as well as two lipidated OspA proteins, lipOspAP/Bo (composed of OspA (a.a. 1-217 from strain PGau)/OspA (a.a. 218-273 from strain Bo)) and lipOspAB/P (composed of OspA (a.a. 1-216 from strain B31)/OspA (a.a. 217-273 from strain PKo)) and were subjected ELISA tests. Mice immunized with the non-lipidated OspC/OspA chimeric protein (OspC-OspAB/P) produced an immune response to OspA from each of the *Borrelia burgdorferi* strains B31 *(Borrelia burgdorferi* sensu stricto), K48 *(Borrelia garinii)* and PGau *(Borrelia afzelli),* that was equivalent or greater than the immune response generated to the two lipidated OspA control proteins (lipOspAP/Bo and lipOspAb/P) (Fig. 49).

Similar results to these were obtained using the Protective ELISA Test described above. Mice immunized with the non-lipidated OspC/OspA chimeric protein (OspC-OspAB/P) produced an immune response to the C-terminal region of OspA from each of the *Borrelia burgdorferi* strains B31 *(Borrelia burgdorferi* sensu stricto), K48 *(Borrelia garinii)* and PGau *(Borrelia afzelli),* that was equivalent or greater than the immune response generated to the C-terminal region of OspA from the two lipidated OspA control proteins (lipOspAP/Bo and lipOspAb/P) (Fig. 50).

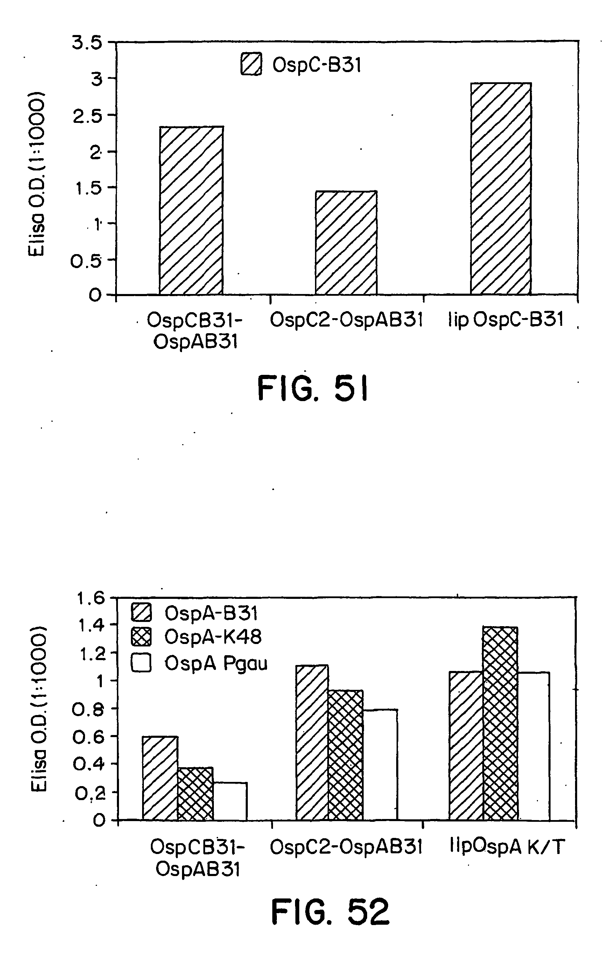

In addition to the comparisons between non-lipidated OspC/OspA chimeric proteins and lipidated OspA control proteins, experiments were also performed to compare non-lipidated OspC/OspA chimeric proteins with a lipidated OspC control protein (Fig. 51). Mice that were immunized with either the non-lipidated OspC/OspA chimeric protein OspCB31-OspAB31 (composed of OspC (a.a. 19-211 from strain B31)/OspA (a.a. 18-273 from strain B31) (SEQ ID NO: 146) or the non-lipidated OspC/OspA chimeric protein OspC2-OspAB31 (composed of OspC (a.a. 19-204 from strain C2)/OspA (a.a. 18-273 from strain B31) (SEQ ID NO:150) produced an immune response to OspC derived from the *Borrelia burgdorferi* strain B31 that was comparable to the immune response produced by a lipidated OspC control protein (lip OspC-B31 - composed of OspC (a.a. 1-211 from strain B31)) (Fig. 51).

Thus, these results clearly demonstrate that non-lipidated chimeric OspC/OspA proteins are able to induce immune responses against OspA and OspC that are comparable to the immune response generated against OspA and OspC using lipidated OspA or OspC control proteins. The use of unlipidated forms of these proteins as vaccine immunogens or diagnostic antigens is highly desirable because the product yield is much greater and the proteins are much easier to purify. For these reasons, the production of these proteins less expensive.

The OspC/OspA chimeric proteins of the present invention are also able to generate immune responses against OspA proteins that are derived from strains that are not represented in the chimeric protein. Mice immunized with the OspC/OspA chimeric proteins, OspCB31-OspAB31 (SEQ ID NO:146) and OspC2-OspAB31 (SEQ ID NO:150), are not only able to generate immune responses that recognize OspA derived from strain B31 *(Borrelia burgdorferi* sensu stricto), but also recognize OspA derived from strain K48 *(Borrelia garinii)* and strain PGau *(Borrelia afzelli)* (Fig. 52). For comparison, mice were also immunized with the lipidated OspA chimeric protein, Lip OspA K/T (composed of OspA (a.a. 1-217 from strain K48)/OspA (a.a. 218-273 from strain Tro)) (Fig. 52).

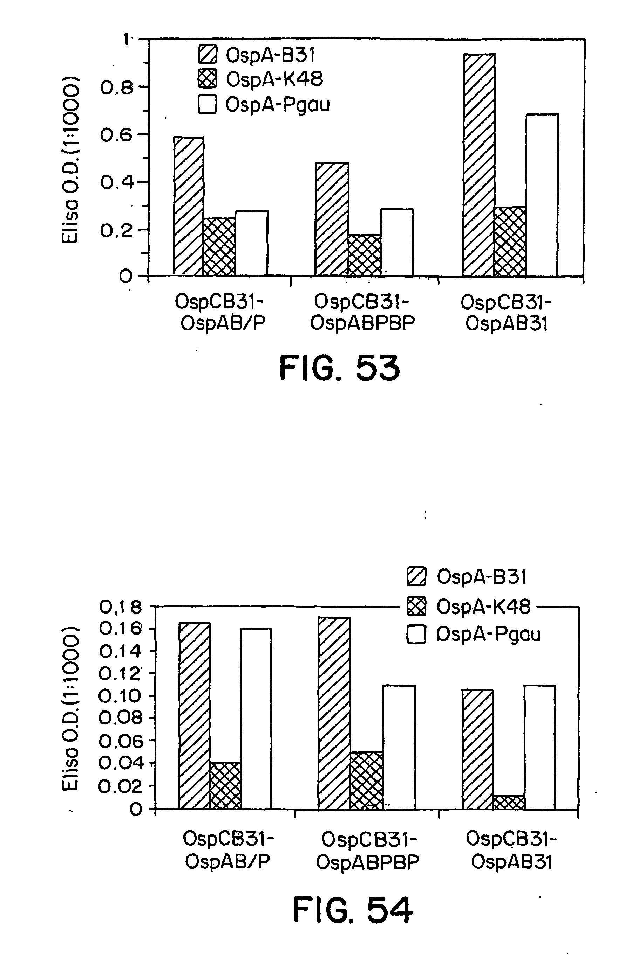

Additional antibody responses to OspA derived from strain B31 (*Borrelia burgdorferi* sensu stricto), strain K48 *(Borrelia garinii*) and strain PGau *(Borrelia afzelli*) are also presented for sera from mice immunized with other OspC/OspA chimeric proteins. Thus, Fig. 53 presents the ELISA results from mice immunized with either OspCB31-OspAB/P (SEQ ID NO:156), OspCB31-OspABPBP (SEQ ID NO:178) or OspCB31-OspAB31 (SEQ ID NO: 146). In each case, sera from the immunized mice was tested against OspA derived from each of strain B31 *(Borrelia burgdorferi* sensu stricto), K48 *(Borrelia garinii)* and PGau *(Borrelia afzelli).* In all cases, a strong immune response was generated (Fig. 53). As with the previously described OspC/OspA chimeric proteins, the three OspC/OspA chimeric proteins used to immunize the mice in Fig. 52 also elicited a strong immune response to the C-terminal region of OspA when examined using the Protective ELISA Test described above (Fig. 54).

### Tick Challege of Immunized Mice

Mice, either C3H-J or JCR, that had been immunized as described above, were also challenged with either laboratory-infected nympha or field nympha. The immunized mice were placed in isolation cages and each mouse received 5-10 nymphs. All of the nymphs were collected at counted after 6 days. Four week after challenge, the mice were bled and sera was tested using commercially-available Western blot strips to *Borrelia burgdorferi* sensu stricto strain B31 (MarDx strips) and/or *Borrelia garinii* (MRL strips). Eight weeks after challenge, the mice were bled, sera was tested again by Western blot and ear punch and bladder samples were cultured. As a positive control, mice which had been immunized with only aluminum hydroxide adjuvant, as described above, were subjected to the same challenge.

The results of the tick challenge studies (Table VI) demonstrate that while immunization with lipidated OspC protein was unable to protect the mice, as evidenced by a positive Western blot signal (in 4 out of 5 mice), immunization with two different OspC/OspA chimeric proteins (SEQ ID NO.146 and SEQ ID NO. 150) did provide protection, as indicated by the absence of Western blot signal (in 0 out of 8 mice and 0 out of 3 mice) (Table VI). The sham positive control showed that the challenge by the ticks was successful in all cases, as evidenced by 100°/ positive signal in Western blots (Table VI).

**Table VI. Effect of Vaccination on Transmission of Borrelia from Ticks**

| Vaccine Candidate | Mouse | Tick-nymph | Seroconversion (Western Blots) Vaccinated | Seroconversion (Western Blots) Sham |
|---|---|---|---|---|
| OspC1-OspAB31 | C3H-J | Long Island | 0+/8 | 8+/8 |
| OspC2-OspAB31 | C3H-J | Long Island | 0+/3 | 4+/4 |
| Lip OspC12 | ICR | Long Island | 4+/5 | 5+/5 |

## Claims

1. An unlipidated chimeric protein comprising at least a first and a second polypeptide, wherein the first polypeptide comprises *Borrelia burgdorferi* OspC and wherein the second polypeptide comprises *Borrelia burgdorferi* OspA, such that:
(a) the OspC polypeptide comprises the N-terminus of the protein, wherein the OspC polypeptide is selected from the group consisting of: amino acid residue 19 to amino acid residue 211, amino acid residue 19 to 204, and amino acid residue 19 to 213, wherein the amino acid numbering is based on the OspC polypeptide of SEO ID NO: 30; and
(b) the OspA polypeptide is either:
(i) selected from the group consisting of: amino acid residue 18 to 273, and amino acid residue 132 to 273, wherein the amino acid numbering is based on the OspA polypeptide of SEQ ID NO: 7; or
(ii) a chimeric polypeptide comprising OspA polypeptides from at least two different strains of *Borrelia burgdorferi.*

2. The chimeric protein of Claims 1, wherein the OspA and OspC polypeptides are from the same strain of *Borrelia burgdorferi;* and/or the OspA and OspC polypeptides are from the same genospecies of *Borrelia burgdorferi.*

3. The chimeric protein of Claims 1, wherein the OspA and OspC polypeptides are from different strains of *Borrelia burgdorferi;* and/or the OspA and OspC polypeptides are from different genospecies of *Borrelia burgdorferi;* and/or
wherein the chimeric OspA polypeptide comprises OspA polypeptides from different genospecies of *Borrelia burgdorferi.*

4. The chimeric protein of any one of Claims 1 to 3, wherein the OspA polypeptide comprises
(a) a *Borrelia burgdorferi* OspA polypeptide from amino acid residue 18 to amino acid residue 216 of a first strain of *Borrelia burgdorferi,* and from amino acid residue 217 to amino acid residue 273 of a second strain of *Borrelia burgdorferi;* or
(b) a *Borrelia burgdorferi* OspA polypeptide from amino acid residue 132 to amino acid residue 217 of a first strain of *Borrelia burgdorferi,* and a *Borrelia burgdorferi* OspA polypeptide from amino acid residue 218 to amino acid residue 273 of a second strain of *Borrelia burgdorferi,* wherein the amino acid numbering is based on the OspA polypeptide sequence of SEQ ID NO: 7.

5. The chimeric protein of any one of Claims 1 to 4, wherein the OspA polypeptide comprises at least four separate OspA polypeptide fragments; and further optionally
wherein the first OspA polypeptide fragment comprises a *Borrelia burgdorferi* OspA polypeptide from amino acid residue 30 to amino acid residue 150, wherein the second OspA polypeptide fragment comprises a *Borrelia burgdorferi* OspA polypeptide from amino acid residue 151 to amino acid residue 179, wherein the third OspA polypeptide fragment comprises a *Borrelia burgdorferi* OspA polypeptide from amino acid residue 180 to amino acid residue 216, and wherein the fourth OspA polypeptide fragment comprises a *Borrelia burgdorferi* OspA polypeptide from amino acid residue 217 to amino acid residue 273, wherein the amino acid numbering is based on the OspA polypeptide sequence of SEQ ID NO: 7.

6. A chimeric protein comprising an amino acid sequence selected from the group consisting of: SEQ ID NOs: 146, 148, 150, 152, 154, 156, 158, 160, 162, 164, 166, 168, 170, 172, 174, 176, 178 and 180.

7. A nucleic acid encoding an unlipidated chimeric protein comprising:
at least a first and a second polypeptide, wherein the first polypeptide comprises *Borrelia burgdorferi* OspC and wherein the second polypeptide comprises *Borrelia burgdorferi* OspA, such that OspC comprises the N-terminus of the encoded protein, wherein:
(a) the OspC polypeptide is selected from the group consisting of: amino acid residue 19 to amino acid residue 211, amino acid residue 19 to 204, and amino acid residue 19 to 213, wherein the amino acid numbering is based on the OspC polypeptide of SEQ ID NO: 30; and
(b) the OspA polypeptide is either:
(i) selected from the group consisting of: amino acid residue 18 to 273, and amino acid residue 132 to 273, wherein the amino acid numbering is based on the OspA polypeptide of SEQ ID NO: 7; or
(ii) the OspA polypeptide is a chimeric polypeptide comprising OspA polypeptides from at least two different strains of *Borrelia burgdorferi.*

8. The nucleic acid of Claim 7, wherein the OspA polypeptide comprises amino acid residue 18 to amino acid residue 216 of a first strain of *Borrelia burgdorferi* and from amino acid residue 217 to amino acid residue 273 of a second strain of *Borrelia burgdorferi,* or
from amino acid residue 132 to amino acid residue 216 of a first strain of *Borrelia burgdorferi* and amino acid residue 217 to amino acid residue 273 of a second strain of *Borrelia burgdorferi,* wherein the amino acid numbering is based on the OspA polypeptide sequence of SEQ ID NO: 7.

9. The nucleic acid of Claim 7 or 8, wherein the OspA polypeptide comprises at least four separate OspA polypeptide fragments, wherein the first OspA polypeptide fragment comprises a *Borrelia burgdorferi* OspA polypeptide from amino acid residue 30 to amino acid residue 150, wherein the second OspA polypeptide comprises a *Borrelia burgdorferi* OspA polypeptide from amino acid residue 151 to amino acid residue 179, wherein the third OspA polypeptide comprises a *Borrelia burgdorferi* OspA polypeptide from amino acid residue 181 to amino acid residue 216, and wherein the fourth OspA polypeptide comprises a *Borrelia burgdorferi* OspA polypeptide from amino acid residue 216 to amino acid residue 273, wherein the amino acid numbering is based on the OspA polypeptide sequence of SEQ ID NO: 7.

10. A nucleic acid of any one of Claims 7 to 9, wherein:
the encoded OspC and OspA polypeptides are from different strains of *Borrelia burgdorferi* and/or the encoded OspC and OspA polypeptides are from different genospecies of *Borrelia burgdorferi.*

11. A nucleic acid of any one of Claims 7 to 10, wherein:
the encoded OspC and OspA polypeptides are from the same strains of *Borrelia burgdorferi:* and/or
the encoded OspC and OspA polypeptides are from the same genospecies of *Borrelia burgdorferi.*

12. A nucleic acid selected from the group consisting of: SEQ ID NOs: 145, 147, 149, 151, 153, 155, 157, 159, 161, 163, 165, 167, 169, 171, 173, 175, 177 and 179.

13. An expression vector comprising either:
(a) an isolated DNA having a nucleotide sequence selected from the group consisting of: SEQ ID NOs: 145, 147, 149, 151, 153, 155, 157, 159, 161, 163, 165, 167, 169, 171, 173, 175, 177 and 179; or
(b) an isolated DNA encoding a polypeptide having an amino acid sequence selected from the group consisting of: SEQ ID NOs : 146, 148, 150, 152, 154, 156, 158, 160, 162, 164, 166, 168, 170, 172, 174, 176, 178 and 180.

14. A host cell comprising a recombinant nucleic acid either:
(a) having a nucleotide sequence selected from the group consisting of: SEQ ID NOs: 145, 147, 149, 151, 153, 155, 157, 159, 161, 163, 165, 167, 169, 171, 173, 175, 177 and 179; or
(b) encoding a protein having an amino acid sequence selected from the group consisting of: SEQ ID NOs: 146, 148, 150, 152, 154, 156, 158, 160, 162, 164, 166, 168, 170, 172, 174, 176, 178 and 180.

15. A method of producing an unlipidated chimeric *Borrelia burgdorferi* protein comprising;
a) selecting a polynucleotide encoding OspC or an antigenic portion thereof;
b) selecting a polynucleotide encoding OspA or an antigenic portion thereof; and
c) ligating the polynucleotide of a) to the polynucleotide of b), to form a chimeric polynucleotide sequence encoding a chimeric OspC-OspA protein such that OspC is upstream of OspA, wherein:
the OspC polypeptide is selected from the group consisting of: amino acid residue 19 to amino acid residue 211, amino acid residue 19 to 204, and amino acid residue 19 to 213, wherein the amino acid numbering is based on the OspC polypeptide of SEQ ID NO: 30; and the OspA polypeptide is either:
(i) selected from the group consisting of: amino acid residue 18 to 273, and amino acid residue 132 to 273, wherein the amino acid numbering is based on the OspA polypeptide of SEQ ID NO: 7, or
(ii) the OspA polypeptide is a chimeric polypeptide comprising OspA polypeptides from at least two different strains of *Borrelia burgdorferi.*

16. The method of Claim 15, wherein:
(a) the chimeric OspC-OspA protein has an amino acid sequence selected from the group consisting of: SEQ ID NOs: 146, 148, 150, 152, 154, 156, 158, 160, 162, 164, 166, 168, 170, 172, 174, 176, 178 and 180; and/or
(b) the method for producing a chimeric protein further comprising maintaining a host cell comprising the chimeric polynucleotide of Claim 15 under conditions suitable for expression of the polynucleotide.

17. Use of at least a first and a second polypeptide for the manufacture of an unlipidated chimeric protein for use in immunizing an animal against Lyme's Disease, wherein the first polypeptide comprises *Borrelia burgdorferi* OspC and wherein the second polypeptide comprises *Borrelia burgdorferi* OspA, such that OspC comprises the N-terminus of the protein, comprising administering the chimeric protein to an animal, wherein:
(a) the OspC polypeptide is selected from the group consisting of: amino acid residue 19 to amino acid residue 211, amino acid residue 19 to 204, and amino acid residue 19 to 213, wherein the amino acid numbering is based on the OspC polypeptide of SEQ ID NO: 30, and
(b) the OspA polypeptide is either:
(i) selected from the group consisting of: amino acid residue 18 to 273, and amino acid residue 132 to 273, wherein the amino acid numbering is based on the OspA polypeptide of SEQ ID NO: 7, or
(ii) the OspA polypeptide is a chimeric polypeptide comprising OspA polypeptides from at least two different strains of *Borrelia burgdorferi.*

18. The use of Claim 17, wherein:
(a) the chimeric protein is administered with a physiologically-acceptable vehicle or carrier;
(b) the chimeric protein is encoded by a nucleic acid selected from the group consisting of: SEQ ID NOs: 145, 147, 149, 151, 153, 155, 157, 159, 161, 163, 165, 167, 169, 171, 173, 175, 177 and 179; or
(c) the chimeric protein comprises an amino acid sequence selected from the group consisting of: SEQ ID NOs: 146, 148, 150, 152, 154, 156, 158, 160, 162, 164, 166, 168, 170, 172, 174, 176, 178 and 180.

19. A method of detecting an unlipidated chimeric protein comprising at least a first and a second polypeptide, wherein the first polypeptide comprises *Borrelia burgdorferi* OspC and wherein the second polypeptide comprises *Borrelia burgdorferi* OspA, such that OspC comprises the N-terminus of the protein, comprising;
a) contacting a host sample with the chimeric protein, under conditions wherein antibodies, if present, in the host sample bind to the chimeric protein forming antigen antibody complexes; and
b) detecting the antigen antibody complexes,
wherein the OspC polypeptide is selected from the group consisting of: amino acid residue 19 to amino acid residue 211, amino acid residue 19 to 204, and amino acid residue 19 to 213, wherein the amino acid numbering is based on the OspC polypeptide of SEQ ID NO: 30, and the OspA polypeptide is selected from the group consisting of: amino acid residue 18 to 273, and amino acid residue 132 to 273, wherein the amino acid numbering is based on the OspA polypeptide of SEQ ID NO: 7, or
the OspA polypeptide is a chimeric polypeptide comprising OspA polypeptides from at least two different strains of *Borrelia burgdorferi.*

20. The method of Claim 19, wherein:
(a) the chimeric protein is encoded by a nucleic acid selected from the group consisting of: SEQ ID NOs: 145, 147, 149, 151, 153, 155, 157, 159, 161, 163, 165, 167, 169, 171, 173, 175, 177 and 179; or
(b) the chimeric protein comprises an amino acid sequence selected from the group consisting of: SEQ ID NOs: 146, 148, 150, 152, 154, 156, 158, 160, 162, 164, 166, 168, 170, 172, 174, 176, 178 and 180.

21. Use of a nucleic acid that encodes an unlipidated chimeric protein comprising a first and a second polypeptide, wherein the first polypeptide comprises *Borrelia burgdorferi* OspC and wherein the second polypeptide comprises *Borrelia burgdorferi* OspA, such that OspC comprises the N-terminus of the protein, for the manufacture of a composition for use in immunizing an animal against Lyme Disease, wherein: (a) the OspC polypeptide is selected from the group consisting of: amino acid residue 19 to amino acid residue 211, amino acid residue 19 to 204, and amino acid residue 19 to 213, wherein the amino acid numbering is based on the OspC polypeptide of SEQ ID NO: 30; and the OspA polypeptide is either:
(i) selected from the group consisting of: amino acid residue 18 to 273, and amino acid residue 132 to 273, wherein the amino acid numbering is based on the OspA polypeptide of SEQ ID NO: 7, or
(ii) the OspA polypeptide is a chimeric polypeptide comprising OspA polypeptides from at least two different strains of *Borrelia burgdorferi* and optionally
wherein the nucleic acid encoding the chimeric protein is selected from the group consisting of: SEQ ID NOs: 145, 147, 149, 151, 153, 155, 157, 159, 161, 163, 165, 167, 169, 171, 173, 175, 177 and 179.

22. A physiological composition to vaccinate against and treat *Borrelia* infection in animals or humans, the composition comprising;
a) either (i) an unlipidated chimeric protein comprising at least a first and a second polypeptide, wherein the first polypeptide comprises *Borrelia burgdorferi* OspC and wherein the second polypeptide comprises *Borrelia burgdorferi* OspA, such that OspC comprises the N-terminus of the protein, wherein the OspC polypeptide is selected from the group consisting of: amino acid residue 19 to amino acid residue 211, amino acid residue 19 to 204, and amino acid residue 19 to 213, wherein the amino acid numbering is based on the OspC polypeptide of SEQ ID NO: 30, and the OspA polypeptide is either selected from the group consisting of: amino acid residue 18 to 273, and amino acid residue 132 to 273, wherein the amino acid numbering is based on the OspA polypeptide of SEQ ID NO: 7, or a chimeric polypeptide comprising OspA polypeptides from at least two different strains of *Borrelia burgdorferi,* the amount being effective to elicit an immune response to *Borrelia burgdorferi;* or (ii) a nucleic acid encoding said chimeric protein;
b) a physiologically-acceptable carrier or vehicle; and optionally
c) an adjuvant.

23. The composition of Claim 22, wherein either:
(a) the chimeric protein is selected from the group consisting of: SEQ ID NOs: 146, 148, 150, 152, 154, 156, 158, 160, 162, 164, 166, 168, 170, 172, 174, 176, 178 and 180; and/or
(b) the nucleic acid is selected from the group consisting of: SEQ ID NOs: 145, 147, 149, 151, 153, 155, 157, 159, 161, 163, 165, 167, 169, 171, 173, 175, 177 and 179.

24. A kit comprising;
a) an unlipidated chimeric protein comprising at least a first and a second polypeptide, wherein the first polypeptide comprises *Borrelia burgdorferi* OspC and wherein the second polypeptide comprises *Borrelia burgdorferi* OspA, such that OspC comprises the N-terminus of the protein, wherein the OspC polypeptide is selected from the group consisting of: amino acid residue 19 to amino acid residue 211, amino acid residue 19 to 204, and amino acid residue 19 to 213, wherein the amino acid numbering is based on the OspC polypeptide of SEQ ID NO: 30, and the OspA polypeptide is either: (i) selected from the group consisting of: amino acid residue 18 to 273, and amino acid residue 132 to 273, wherein the amino acid numbering is based on the OspA polypeptide of SEQ ID NO: 7, or (ii) a chimeric polypeptide comprising OspA polypeptides from at least two different strains of *Borrelia burgdorferi,* and
b) reagents for detecting antibody antigen complexes formed between the chimeric protein and antibodies, if present, in a user-supplied host sample.

25. The kit of Claim 24, wherein the chimeric protein is selected from the group consisting of: SEQ ID NOs: 146, 148, 150, 152, 154, 156, 158, 160, 162, 164, 166, 168, 170, 172, 174, 176, 178 and 180.

## Patentansprüche

1. Unlipidiertes chimäres Protein mit mindestens einem ersten und einem zweiten Polypeptid, wobei das erste Polypeptid das OspC von *Borrelia burgdorferi* umfasst und das zweite Polypeptid das OspA von *Borrelia burgdorferi* umfasst, so dass
(a) das OspC-Polypeptid den N-Terminus des Proteins umfasst, wobei das OspC-Polypeptid ausgewählt ist aus der Gruppe Aminosäurerest 19 bis Aminosäurerest 211, Aminosäurerest 19 bis Aminosäurerest 204 und Aminosäurerest 19 bis Aminosäurerest 213, wobei die Nummerierung der Aminosäuren auf der Grundlage der SEQ ID NO: 30 für das OspC-Polypeptid beruht; und
(b) das OspA-Polypeptid entweder
(i) ausgewählt ist aus der Gruppe Aminosäurerest 18 bis Aminosäurerest 273 und Aminosäurerest 132 bis Aminosäurerest 273, wobei die Nummerierung der Aminosäuren auf der Grundlage der SEQ ID NO: 7 für das OspA-Polypeptid beruht; oder
(ii) ein chimäres Polypeptid ist, das die OspA-Polypeptide aus mindestens zwei unterschiedlichen Stämmen von *Borrelia burgdorferi* umfasst.

2. Chimäres Protein nach Anspruch 1, in welchem die OspA- und OspC-Polypeptide von dem gleichen *Borrelia burgdorferi*-Stamm sind und/oder die OspA- und OspC-Polypeptide von der gleichen Genospezies von *Borrelia burgdorferi* sind

3. Chimäres Protein nach Anspruch 1, in welchem die OspA- und OspC-Polypeptide von unterschiedlichen *Borrelia burgdorferi-*Stämmen sind und/oder die OspA- und OspC-Polypeptide von unterschiedlichen Genospezies von *Borrelia burgdorferi* sind und/oder wobei das chimäre OspA-Polypeptid OspA-Polypeptide aus verschiedenen Genospezies von *Borrelia burgdorferi*-umfasst.

4. Chimäres Protein nach jedem der Ansprüche 1 bis 3, in welchem das OspA-Polypeptid
(a) ein *Borrelia burgdorferi-OspA-Polypeptid* aus den Aminosäureresten 18 bis 216 eines ersten *Borrelia burgdorferi-Stammes* sowie aus den Aminosäureresten 217 bis 273 eines zweiten *Borrelia burgdorferi-Stammes* umfasst oder
(b) ein *Borrelia burgdorferi-*OspA*-*Polypeptid aus den Aminosäureresten 132 bis 217 eines ersten *Borrelia burgdorferi-Stammes* sowie ein *Borrelia burgdorferi*-OspA-Polypeptid aus den Aminosäureresten 218 bis 273 eines zweiten *Borrelia burgdorferi-*Stammes umfasst, wobei die Nummerierung der Aminosäuren auf der Grundlage der SEQ ID NO: 7 für das OspA-Polypeptid beruht.

5. Chimäres Protein nach jedem der Ansprüche 1 bis 4, in welchem das OspA-Polypeptid mindestens vier getrennte OspA-Polypeptid-Fragmente umfasst und in welchem ferner wahlweise
das erste OspA-Polypeptid-Fragment ein *Borrelia burgdorferi-*OspA-Polypeptid aus den Aminosäureresten 30 bis 150 umfasst, das zweite OspA-Polypeptid-Fragment ein *Borrelia burgdorferi-*OspA-Polypeptid aus den Aminosäureresten 151 bis 179 umfasst, das dritte OspA-Polypeptid-Fragment ein *Borrelia burgdorferi*-OspA-Polypeptid aus den Aminosäureresten 180 bis 216 umfasst und das vierte OspA-Polypeptid-Fragment ein *Borrelia burgdorferi-*OspA-Polypeptid aus den Aminosäureresten 217 bis 273 umfasst, wobei die Nummerierung der Aminosäuren auf der Grundlage der SEQ ID NO: 7 für das OspA-Polypeptid beruht.

6. Chimäres Protein mit einer Aminosäuresequenz, die ausgewählt ist aus der Gruppe SEQ ID NOs: 146, 148, 150, 152, 154, 156, 158, 160, 162, 164, 166, 168, 170, 172, 174, 176, 178 und 180.

7. Nucleinsäure, welche ein unlipidiertes chimäres Protein codiert, welches mindestens ein erstes und ein zweites Polypeptid umfasst, wobei das erste Polypeptid das OspC von *Borrelia burgdorferi* umfasst und das zweite Polypeptid das OspA von *Borrelia burgdorferi* umfasst, so dass das OspC-Polypeptid den N-Terminus des codierten Proteins umfasst, wobei
(a) das OspC-Polypeptid ausgewählt ist aus der Gruppe Aminosäurerest 19 bis Aminosäurerest 211, Aminosäurerest 19 bis Aminosäurerest 204 und Aminosäurerest 19 bis Aminosäurerest 213, wobei die Nummerierung der Aminosäuren auf der Grundlage der SEQ ID NO: 30 für das OspC-Polypeptid beruht; und
(b) das OspA-Polypeptid entweder
(i) ausgewählt ist aus der Gruppe Aminosäurerest 18 bis Aminosäurerest 273 und Aminosäurerest 132 bis Aminosäurerest 273, wobei die Nummerierung der Aminosäuren auf der Grundlage der SEQ ID NO: 7 für das OspA-Polypeptid beruht; oder
(ii) das OspA-Polypeptid ein chimäres Polypeptid ist, das die OspA-Polypeptide aus mindestens zwei unterschiedlichen Stämmen von *Borrelia burgdorferi* umfasst.

8. Nucleinsäure nach Anspruch 7, wobei das OspA-Polypeptid die Aminosäurereste 18 bis 216 eines ersten *Borrelia burgdorferi-Stammes* sowie die Aminosäurereste 217 bis 273 eines zweiten *Borrelia burgdorferi-Stammes* umfasst oder
die Aminosäurereste 132 bis 216 eines ersten *Borrelia burgdorferi-Stammes* sowie die Aminosäurereste 217 bis 273 eines zweiten *Borrelia burgdorferi-Stammes* umfasst, wobei die Nummerierung der Aminosäuren auf der Grundlage der OspA-Polypeptidsequenz der SEQ ID NO: 7 beruht.

9. Nucleinsäure nach Anspruch 7 oder 8, wobei das OspA-Polypeptid mindestens vier getrennte OspA-Polypeptid-Fragmente umfasst, wobei das erste OspA-Polypeptid-Fragment ein *Borrelia burgdorferi*-OspA-Polypeptid aus den Aminosäureresten 30 bis 150 umfasst, das zweite OspA-Polypeptid-Fragment ein *Borrelia burgdorferi*-OspA-Polypeptid aus den Aminosäureresten 151 bis 179 umfasst, das dritte OspA-Polypeptid-Fragment ein *Borrelia burgdorferi*-OspA-Polypeptid aus den Aminosäureresten 181 bis 216 umfasst und das vierte OspA-Polypeptid-Fragment ein *Borrelia burgdorferi*-OspA-Polypeptid aus den Aminosäureresten 216 bis 273 umfasst, wobei die Nummerierung der Aminosäuren auf der Grundlage der OspA-Polypeptidsequenz der SEQ ID NO: 7 beruht.

10. Nucleinsäure nach jedem der Ansprüche 7 bis 9, wobei die codierten OspC- und OspA-Polypeptide von unterschiedlichen *Borrelia burgdorferi*-Stämmen sind und/oder die codierten OspC- und OspA-Polypeptide von unterschiedlichen Genospezies von *Borrelia burgdorferi* sind.

11. Nucleinsäure nach jedem der Ansprüche 7 bis 10, wobei die codierten OspC- und OspA-Polypeptide von den gleichen *Borrelia burgdorferi*-Stämmen sind und/oder die codierten OspC- und OspA-Polypeptide von den gleichen Genospezies von *Borrelia burgdorferi* sind

12. Nucleinsäure, die ausgewählt ist aus der Gruppe SEQ ID NOs 145, 147, 149, 151, 153, 155, 157, 159, 161, 163, 165, 167, 169, 171, 173, 175, 177 und 179.

13. Expressionsvektor mit entweder
(a) einer isolierten DNA mit einer Nucleotidsequenz, die ausgewählt ist aus der Gruppe SEQ ID NOs 145, 147, 149, 151, 153, 155, 157, 159, 161, 163, 165, 167, 169, 171, 173, 175, 177 und 179 oder
(b) einer isolierten DNA, welche ein Polypeptid mit einer Aminosäuresequenz codiert, die ausgewählt ist aus der Gruppe SEQ ID NOs: 146, 148, 150, 152, 154, 156, 158, 160, 162, 164, 166, 168, 170, 172, 174, 176, 178 und 180.

14. Wirtszelle, welche eine rekombinante Nucleinsäure umfasst, welche entweder
(a) eine Nucleotidsequenz aufweist, die ausgewählt ist aus der Gruppe SEQ ID NOs 145, 147, 149, 151, 153, 155, 157, 159, 161, 163, 165, 167, 169, 171, 173, 175, 177 und 179 oder
(b) ein Protein mit einer Aminosäuresequenz codiert, die ausgewählt ist aus der Gruppe SEQ ID NOs: 146, 148, 150, 152, 154, 156, 158, 160, 162, 164, 166, 168, 170, 172, 174, 176, 178 und 180.

15. Verfahren zur Produktion eines unlipidierten chimären *Borrelia burgdorferi-*Proteins in den Schritten:
a) Auswählen eines Polynucleotids, welches das OspC oder einen antigenen Teil desselben codiert;
b) Auswählen eines Polynucleotids, welches das OspA oder einen antigenen Teil desselben codiert;
c) Ligieren des Polynucleotids von a) an das Polypeptid von b), um eine chimäre Polynucleotidsequenz zu bilden, welche ein chimäres OspC-OspA-Protein codiert, so dass OspC upstream zu OspA zu liegen kommt, wobei
das OspC-Polypeptid ausgewählt wird aus der Gruppe Aminosäurerest 19 bis Aminosäurerest 211, Aminosäurerest 19 bis Aminosäurerest 204 und Aminosäurerest 19 bis Aminosäurerest 213, wobei die Nummerierung der Aminosäuren auf der Grundlage der SEQ ID NO: 30 für das OspC-Polypeptid beruht; und das OspA-Polypeptid entweder
(i) ausgewählt wird aus der Gruppe Aminosäurerest 18 bis Aminosäurerest 273 und Aminosäurerest 132 bis Aminosäurerest 273, wobei die Nummerierung der Aminosäuren auf der Grundlage der SEQ ID NO: 7 für das OspA-Polypeptid beruht; oder
(ii) das OspA-Polypeptid ein chimäres Polypeptid ist, das die OspA-Polypeptide aus mindestens zwei unterschiedlichen Stämmen von *Borrelia burgdorferi* umfasst.

16. Verfahren nach Anspruch 15, wobei
(a) das chimäre OspC-OspA-Protein eine Aminosäuresequenz aufweist, die ausgewählt wird aus der Gruppe SEQ ID NOs: 146, 148, 150, 152, 154, 156, 158, 160, 162, 164, 166, 168, 170, 172, 174, 176, 178 und 180 und/oder
(b) das Verfahren zur Produktion eines chimären Proteins ferner den Schritt umfasst: Halten einer Wirtszelle mit dem chimären Polynucleotid des Anspruchs 15 unter Bedingungen, die für die Expression des Polynucleotids geeignet sind

17. Verwendung von mindestens einem ersten und einem zweiten Polypeptid für die Herstellung eines unlipidierten chimären Proteins für den Einsatz bei der Immunisierung eines Tieres gegen die Lyme-Krankheit, wobei das erste Polypeptid das OspC von *Borrelia burgdorferi* umfasst und das zweite Polypeptid das OspA von *Borrelia burgdorferi* umfasst, so dass das das OspC den N-Terminus des Proteins enthält, wobei das chimäre Protein einem Tier verabreicht wird, wobei
(a) das OspC-Polypeptid ausgewählt wird aus der Gruppe Aminosäurerest 19 bis Aminosäurerest 211, Aminosäurerest 19 bis Aminosäurerest 204 und Aminosäurerest 19 bis Aminosäurerest 213, wobei die Nummerierung der Aminosäuren auf der Grundlage der SEQ ID NO: 30 für das OspC-Polypeptid beruht, und
(b) das OspA-Polypeptid entweder
(i) ausgewählt wird aus der Gruppe Aminosäurerest 18 bis Aminosäurerest 273 und Aminosäurerest 132 bis Aminosäurerest 273, wobei die Nummerierung der Aminosäuren auf der Grundlage der SEQ ID NO: 7 für das OspA-Polypeptid beruht; oder
(ii) das OspA-Polypeptid ein chimäres Polypeptid ist, das die OspA-Polypeptide aus mindestens zwei unterschiedlichen Stämmen von *Borrelia burgdorferi* umfasst.

18. Verwendung nach Anspruch 17, wobei
(a) das chimäre Protein mit einem physiologisch verträglichen Vehikel oder Träger verabreicht wird;
(b) das chimäre Protein von einer Nucleinsäure codiert wird, die ausgewählt ist aus der Gruppe SEQ ID NOs 145, 147, 149, 151, 153, 155, 157, 159, 161, 163, 165, 167, 169, 171, 173, 175, 177 und 179 oder
(c) das chimäre Protein eine Aminosäuresequenz umfasst, die ausgewählt ist aus der Gruppe SEQ ID NOs: 146, 148, 150, 152, 154, 156, 158, 160, 162, 164, 166, 168, 170, 172, 174, 176, 178 und 180.

19. Verfahren zum Nachweis eines unlipidierten chimären Proteins mit mindestens einem ersten und einem zweiten Polypeptid, wobei das erste Polypeptid das OspC von *Borrelia burgdorferi* umfasst und das zweite Polypeptid das OspA von *Borrelia burgdorferi* umfasst, so dass das OspC-Polypeptid den N-Terminus des Proteins umfasst, in den Schritten:
a) in Kontakt Bringen einer Wirtsprobe mit dem chimären Protein unter Bedingungen, bei denen sich Antikörper, falls in der Wirtsprobe vorhanden, an das chimäre Protein unter Bildung von Antigen-Antikörper-Komplexen binden; und
(b) Nachweisen der Antigen-Antikörper-Komplexe,
wobei das OspC-Polypeptid ausgewählt wird aus der Gruppe Aminosäurerest 19 bis Aminosäurerest 211, Aminosäurerest 19 bis Aminosäurerest 204 und Aminosäurerest 19 bis Aminosäurerest 213, wobei die Nummerierung der Aminosäuren auf der Grundlage der SEQ ID NO: 30 für das OspC-Polypeptid beruht, und
das OspA-Polypeptid ausgewählt wird aus der Gruppe Aminosäurerest 18 bis Aminosäurerest 273 und Aminosäurerest 132 bis Aminosäurerest 273, wobei die Nummerierung der Aminosäuren auf der Grundlage der SEQ ID NO: 7 für das OspA-Polypeptid beruht; oder
das OspA-Polypeptid ein chimäres Polypeptid ist, das die OspA-Polypeptide aus mindestens zwei unterschiedlichen Stämmen von *Borrelia burgdorferi* umfasst.

20. Verfahren nach Anspruch 19, wobei
(a) das chimäre Protein von einer Nucleinsäure codiert wird, die ausgewählt wird aus der Gruppe SEQ ID NOs 145, 147, 149, 151, 153, 155, 157, 159, 161, 163, 165, 167, 169, 171, 173, 175, 177 und 179 oder
(b) das chimäre Protein eine Aminosäuresequenz umfasst, die ausgewählt wird aus der Gruppe SEQ ID NOs: 146, 148, 150, 152, 154, 156, 158, 160, 162, 164, 166, 168, 170, 172, 174, 176, 178 und 180.

21. Verwendung einer Nucleinsäure, welche ein unlipidiertes chimäres Protein mit einem ersten und einem zweiten Polypeptid codiert, wobei das erste Polypeptid das OspC von *Borrelia burgdorferi* umfasst und das zweite Polypeptid das OspA von *Borrelia burgdorferi* umfasst, so dass das OspC-Polypeptid den N-Terminus des Proteins umfasst, zur Herstellung einer Zusammensetzung für einen Einsatz bei der Immunisierung eines Tieres gegen die Lyme-Kramkheit, wobei (a) das OspC-Polypeptid ausgewählt wird aus der Gruppe Aminosäurerest 19 bis Aminosäurerest 211, Aminosäurerest 19 bis Aminosäurerest 204 und Aminosäurerest 19 bis Aminosäurerest 213, wobei die Nummerierung der Aminosäuren auf der Grundlage der SEQ ID NO: 30 für das OspC-Polypeptid beruht, und das OspA-Polypeptid entweder
(i) ausgewählt wird aus der Gruppe Aminosäurerest 18 bis Aminosäurerest 273 und Aminosäurerest 132 bis Aminosäurerest 273, wobei die Nummerierung der Aminosäuren auf der Grundlage der SEQ ID NO: 7 für das OspA-Polypeptid beruht; oder
(ii) das OspA-Polypeptid ein chimäres Polypeptid ist, das die OspA-Polypeptide aus mindestens zwei unterschiedlichen Stämmen von *Borrelia burgdorferi* umfasst, und wobei wahlweise die das chimäre Protein codierende Nucleinsäure ausgewählt wird aus der Gruppe SEQ ID NOs 145, 147, 149, 151, 153, 155, 157, 159, 161, 163, 165, 167, 169, 171, 173, 175, 177 und 179.

22. Physiologische Zusammensetzung zum Impfen gegen eine *Borrelia*-Infektion und zur Behandlung einer *Borrelia*-Infektion bei Tier und Mensch, wobei die Zusammensetzung
a) entweder (i) ein unlipidiertes chimäres Protein mit mindestens einem ersten und einem zweiten Polypeptid umfasst, wobei das erste Polypeptid das OspC von *Borrelia burgdorferi* umfasst und das zweite Polypeptid das OspA von *Borrelia burgdorferi* umfasst, so dass das OspC-Polypeptid den N-Terminus des Proteins umfasst, wobei das OspC-Polypeptid ausgewählt ist aus der Gruppe Aminosäurerest 19 bis Aminosäurerest 211, Aminosäurerest 19 bis Aminosäurerest 204 und Aminosäurerest 19 bis Aminosäurerest 213, wobei die Nummerierung der Aminosäuren auf der Grundlage der SEQ ID NO: 30 für das OspC-Polypeptid beruht, und wobei das OspA-Polypeptid entweder ausgewählt ist aus der Gruppe Aminosäurerest 18 bis Aminosäurerest 273 und Aminosäurerest 132 bis Aminosäurerest 273, wobei die Nummerierung der Aminosäuren auf der Grundlage der SEQ ID NO: 7 für das OspA-Polypeptid beruht, oder ein chimäres Polypeptid ist, welches OspA-Polypeptide aus mindestens zwei unterschiedlichen Stämmen von *Borrelia burgdorferi* umfasst, wobei die Menge wirksam ist, um eine Immunantwort gegen *Borrelia burgdorferi* hervorzurufen; oder (ii) eine Nucleinsäure umfasst, welche das chimäre Protein codiert;
b) einen physiologisch verträglichen Träger oder ein Vehikel und wahlweise
c) ein Adjuvans umfasst

23. Zusammensetzung nach Anspruch 22, wobei entweder
(a) das chimäre Protein ausgewählt ist aus der Gruppe SEQ ID NOs: 146, 148, 150, 152, 154, 156, 158, 160, 162, 164, 166, 168, 170, 172, 174, 176, 178 und 180 und/oder
(b) die Nucleinsäure ausgewählt ist aus der Gruppe SEQ ID NOs 145, 147, 149, 151, 153, 155, 157, 159, 161, 163, 165, 167, 169, 171, 173, 175, 177 und 179.

24. Ein Kit mit
a) einem unlipidierten chimären Protein, das mindestens ein erstes und ein zweites Polypeptid umfasst, wobei das erste Polypeptid das OspC von *Borrelia burgdorferi* umfasst und das zweite Polypeptid das OspA von *Borrelia burgdorferi* umfasst, so dass das OspC-Polypeptid den N-Terminus des Proteins umfasst, wobei das OspC-Polypeptid ausgewählt ist aus der Gruppe Aminosäurerest 19 bis Aminosäurerest 211, Aminosäurerest 19 bis Aminosäurerest 204 und Aminosäurerest 19 bis Aminosäurerest 213, wobei die Nummerierung der Aminosäuren auf der Grundlage der SEQ ID NO: 30 für das OspC-Polypeptid beruht, und wobei das OspA-Polypeptid entweder ausgewählt ist aus der Gruppe Aminosäurerest 18 bis Aminosäurerest 273 und Aminosäurerest 132 bis Aminosäurerest 273, wobei die Nummerierung der Aminosäuren auf der Grundlage der SEQ ID NO: 7 für das OspA-Polypeptid beruht, oder ein chimäres Polypeptid ist, welches OspA-Polypeptide aus mindestens zwei unterschiedlichen Stämmen von *Borrelia burgdorferi* umfasst, und
b) Reagenzien zum Nachweis von Antigen-Antikörper-Komplexen, die sich zwischen dem chimären Protein und Antikörpern, falls welche vorhanden sind, in einer vom Benutzer zugeführten Wirtsprobe gebildet haben.

25. Kit nach Anspruch 24, in welchem das chimäre Protein ausgewählt ist aus der Gruppe SEQ ID NOs: 146, 148, 150, 152, 154, 156, 158, 160, 162, 164, 166, 168, 170, 172, 174, 176, 178 und 180.

## Revendications

1. Protéine chimère non lipidée comportant au moins un premier et un second polypeptide, dans laquelle le premier polypeptide comporte OspC de *Borrelia burgdorferi* et dans laquelle le second polypeptide comporte OspA de *Borrelia burgdorferi,* de sorte que :
(a) le polypeptide OspC correspond à la partie N-terminale de la protéine, dans lequel le polypeptide OspC est choisi parmi le groupe constitué de : résidu d'acide aminé 19 à résidu d'acide aminé 211, résidus d'acide aminé 19 à 204, et résidus d'acide aminé 19 à 213, la numérotation des acides aminés étant basée sur le polypeptide OspC de SEQ ID NO :30, et
(b) le polypeptide OspA est soit :
(i) choisi parmi le groupe constitué de : résidus d'acide aminé 18 à 273, et résidus d'acide aminé 132 à 273, la numérotation des acides aminés étant basée sur le polypeptide OspA de SEQ ID NO :7, ou
(ii) un polypeptide chimère comportant des polypeptides OspA provenant d'au moins deux souches différentes de *Borrelia burgdorferi.*

2. Protéine chimère selon la revendication 1, dans laquelle les polypeptides OspA et OspC proviennent de la même souche de *Borrelia burgdorferi,* et/ou les polypeptides OspA et OspC proviennent de la même *genospecies* de *Borrelia burgdorferi.*

3. Protéine chimère selon la revendication 1, dans laquelle les polypeptides OspA et OspC proviennent de souches différentes de *Borrelia burgdorferi,* et/ou
les polypeptides OspA et OspC proviennent de *genospecies* différentes de *Borrelia burgdorferi,* et/ou
dans laquelle le polypeptide OspA chimère comporte des polypeptides OspA provenant de *genospecies* différentes de *Borrelia burgdorferi.*

4. Protéine chimère selon l'une quelconque des revendications 1 à 3, dans laquelle le polypeptide OspA comporte :
(a) un polypeptide OspA de *Borrelia burgdorferi* du résidu d'acide aminé 18 au résidu d'acide aminé 216 d'une première souche de *Borrelia burgdorferi,* et du résidu d'acide aminé 217 au résidu d'acide aminé 273 d'une seconde souche de *Borrelia burgdorferi,* ou
(b) un polypeptide OspA de *Borrelia burgdorferi* du résidu d'acide aminé 132 au résidu d'acide aminé 217 d'une première souche de *Borrelia burgdorferi,* et un polypeptide OspA de *Borrelia burgdorferi* du résidu d'acide aminé 218 au résidu d'acide aminé 273 d'une seconde souche de *Borrelia burgdorferi,* la numérotation des acides aminés étant basée sur la séquence du polypeptide OspA de SEQ ID NO :7.

5. Protéine chimère selon l'une quelconque des revendications 1 à 4, dans laquelle le polypeptide OspA comporte au moins quatre fragments de polypeptide OspA séparés, et en outre de manière facultative
dans lequel le premier fragment de polypeptide OspA comporte un polypeptide OspA de *Borrelia burgdorferi* du résidu d'acide aminé 30 au résidu d'acide aminé 150, le deuxième fragment de polypeptide OspA comporte un polypeptide OspA de *Borrelia burgdorferi* du résidu d'acide aminé 151 au résidu d'acide aminé 179, le troisième fragment de polypeptide OspA comporte un polypeptide OspA de *Borrelia burgdorferi* du résidu d'acide aminé 180 au résidu d'acide aminé 216, et le quatrième fragment de polypeptide OspA comporte un polypeptide OspA de *Borrelia burgdorferi* du résidu d'acide aminé 217 au résidu d'acide aminé 273, la numérotation des acides aminés étant basée sur la séquence du polypeptide OspA de SEQ ID NO :7.

6. Protéine chimère comportant une séquence d'acides aminés choisie parmi le groupe constitué des SEQ ID NOs: 146, 148, 150, 152, 154, 156, 158, 160, 162, 164, 166, 168, 170, 172, 174, 176, 178 et 180.

7. Acide nucléique codant une protéine chimère non lipidée comportant :
au moins un premier et un second polypeptide, dans laquelle le premier polypeptide comporte OspC de *Borrelia burgdorferi* et dans lequel le second polypeptide comporte OspA de *Borrelia burgdorferi,* de sorte que OspC correspond à la partie N-terminale de la protéine codée, dans lequel :
(a) le polypeptide OspC est choisi parmi le groupe constitué de : résidu d'acide aminé 19 à résidu d'acide aminé 211, résidus d'acide aminé 19 à 204, et résidus d'acide aminé 19 à 213, la numérotation des acides aminés étant basée sur le polypeptide OspC de SEQ ID NO :30, et
(b) le polypeptide OspA est soit :
(i) choisi parmi le groupe constitué de : résidus d'acide aminé 18 à 273, et résidus d'acide aminé 132 à 273, la numérotation des acides aminés étant basée sur le polypeptide OspA de SEQ ID NO :7, ou
(ii) le polypeptide OspA est un polypeptide chimère comportant des polypeptides OspA provenant d'au moins deux souches différentes de *Borrelia burgdorferi.*

8. Acide nucléique selon la revendication 7, dans lequel le polypeptide OspA comporte du résidu d'acide aminé 18 au résidu d'acide aminé 216 d'une première souche de *Borrelia burgdorferi* et du résidu d'acide aminé 217 au résidu d'acide aminé 273 d'une seconde souche de *Borrelia burgdorferi,* ou
du résidu d'acide aminé 132 au résidu d'acide aminé 216 d'une première souche de *Borrelia burgdorferi,* et du résidu d'acide aminé 217 au résidu d'acide aminé 273 d'une seconde souche de *Borrelia burgdorferi,* la numérotation des acides aminés étant basée sur la séquence du polypeptide OspA de SEQ ID NO :7.

9. Acide nucléique selon la revendication 7 ou 8, dans lequel le polypeptide OspA comporte au moins quatre fragments de polypeptide OspA séparés, dans lequel le premier fragment de polypeptide OspA comporte un polypeptide OspA de *Borrelia burgdorferi* du résidu d'acide aminé 30 au résidu d'acide aminé 150, le deuxième polypeptide OspA comporte un polypeptide OspA de *Borrelia burgdorferi* du résidu d'acide aminé 151 au résidu d'acide aminé 179, le troisième polypeptide OspA comporte un polypeptide OspA de *Borrelia burgdorferi* du résidu d'acide aminé 181 au résidu d'acide aminé 216, et le quatrième polypeptide OspA comporte un polypeptide OspA de *Borrelia burgdorferi* du résidu d'acide aminé 216 au résidu d'acide aminé 273, la numérotation des acides aminés étant basée sur la séquence du polypeptide OspA de SEQ ID NO :7.

10. Acide nucléique selon l'une quelconque des revendications 7 à 9, dans lequel :
les polypeptides OspC et OspA codés proviennent de souches différentes de *Borrelia burgdorferi* et/ou les polypeptides OspC ou OspA codés proviennent de *genospecies* différentes de *Borrelia burgdorferi.*

11. Acide nucléique selon l'une quelconque des revendications 7 à 10, dans lequel :
les polypeptides OspC et OspA codés proviennent des mêmes souches de *Borrelia burgdorferi,* et/ou
les polypeptides OspC et OspA codés proviennent de la même *genospecies* de *Borrelia burgdorferi.*

12. Acide nucléique choisi parmi le groupe constitué de : SEQ ID NOs : 145, 147, 149, 151, 153, 155, 157, 159, 161, 163, 165, 167, 169, 171, 173, 176, 177 et 179.

13. Vecteur d'expression comportant soit :
(a) un ADN isolé ayant une séquence nucléotidique choisie parmi le groupe constitué des SEQ ID NOs : 145, 147, 149, 151, 153, 155, 157, 159, 161, 163, 165, 167, 169, 171, 173, 175, 177 et 179, ou
(b) un ADN isolé codant un polypeptide ayant une séquence en acides aminés choisie parmi le groupe constitué des SEQ ID NOs : 146, 148, 150, 152, 154, 156, 158, 160, 162, 164, 166, 168, 170, 172, 174, 176, 178 et 180.

14. Cellule hôte comportant un acide nucléique recombinant :
(a) ayant une séquence nucléotidique choisie parmi le groupe constitué des SEQ ID NOs : 145, 147, 149, 151, 153, 155, 157, 159, 161, 163, 165, 167, 169, 171, 173, 175, 177 et 179, ou
(b) codant une protéine ayant une séquence en acides aminés choisie parmi le groupe constitué des SEQ ID NOs : 146, 148, 150, 152, 154, 156, 158, 160, 162, 164, 166, 168, 170, 172, 174, 176, 178 et 180.

15. Procédé pour produire une protéine chimère non lipidée de *Borrelia burgdorferi* comportant les étapes suivantes :
a) choisir un polynucléotide codant OspC ou une partie antigène de celui-ci,
b) choisir un polynucléotide codant OspA ou une partie antigène de celui-ci, et
c) ligaturer le polynucléotide de a) au polynucléotide de b), pour former une séquence polynucléotidique chimère codant une protéine chimère OspC-OspA de sorte que OspC est en amont de OspA, dans laquelle :
le polypeptide OspC est choisi parmi le groupe constitué de : résidu d'acide aminé 19 à résidu d'acide aminé 211, résidus d'acide aminé 19 à 204, et résidus d'acide aminé 19 à 213, la numérotation des acides aminés étant basée sur le polypeptide OspC de SEQ ID NO :30, et
le polypeptide OspA est soit :
(i) choisi parmi le groupe constitué de : résidus d'acide aminé 18 à 273, et résidus d'acide aminé 132 à 273, la numérotation des acides aminés étant basée sur le polypeptide OspA de SEQ ID NO :7, ou
(ii) le polypeptide OspA est un polypeptide chimère comportant des polypeptides OspA provenant d'au moins deux souches différentes de *Borrelia burgdorferi.*

16. Procédé selon la revendication 15, dans lequel :
(a) la protéine OspC-OspA chimère a une séquence en acides aminés choisie parmi le groupe constitué des SEQ ID NOs : 146, 148, 150, 152, 154, 156, 158, 160, 162, 164, 166, 168, 170, 172, 174, 176, 178 et 180, et/ou
(b) le procédé pour produire une protéine chimère comprend en outre le maintien d'une cellule hôte comportant le polynucléotide chimère de la revendication 15 dans des conditions adaptées à l'expression du polynucléotide.

17. Utilisation d'au moins un premier et un second polypeptide pour la fabrication d'une protéine chimère non lipidée destinée à être utilisée pour immuniser un animal contre la Maladie de Lyme, dans laquelle le premier polypeptide comporte OspC de *Borrelia burgdorferi* et dans laquelle le second polypeptide comporte OspA de *Borrelia burgdorferi,* de sorte que OspC correspond à la partie N-terminale de la protéine, comportant l'administration de la protéine chimère à un animal, dans laquelle : (a) le polypeptide OspC est choisi parmi le groupe constitué de : résidu d'acide aminé 19 à résidu d'acide aminé 211, résidus d'acide aminé 19 à 204, et résidus d'acide aminé 19 à 213, la numérotation des acides aminés étant basée sur le polypeptide OspC de SEQ ID NO :30, et
(b) le polypeptide OspA est soit :
(i) choisi parmi le groupe constitué de : résidus d'acide aminé 18 à 273, et résidus d'acide aminé 132 à 273, la numérotation des acides aminés étant basée sur le polypeptide OspA de SEQ ID NO :7, ou
(ii) le polypeptide OspA est un polypeptide chimère comportant des polypeptides OspA provenant d'au moins deux souches différentes de *Borrelia burgdorferi.*

18. Utilisation selon la revendication 17, dans laquelle :
(a) la protéine chimère est administrée avec un véhicule ou support physiologiquement acceptable ;
(b) la protéine chimère est codée par un acide nucléique choisi parmi le groupe constitué des SEQ ID NOs : 145, 147, 149, 151, 153, 155, 157, 159, 161, 163, 165, 167, 169, 171, 173, 175, 177 et 179, ou
(c) la protéine chimère comporte une séquence en acides aminés choisie parmi le groupe constitué des SEQ ID NOs : 146, 148, 150, 152, 154, 156, 158, 160, 162, 164, 166, 168, 170, 172, 174, 176, 178 et 180.

19. Procédé de détection d'une protéine chimère non lipidée comportant au moins un premier et un second polypeptides, dans lequel le premier polypeptide comporte OspC de *Borrelia burgdorferi* et dans lequel le second polypeptide comporte OspA de *Borrelia burgdorferi,* de sorte que OspC correspond à la partie N-terminale de la protéine, comportant les étapes suivantes :
a) mettre en contact un échantillon hôte avec la protéine chimère, dans des conditions dans lesquelles les anticorps, s'ils sont présents dans l'échantillon hôte, se lient à la protéine chimère en formant des complexes antigène-anticorps, et
b) détecter les complexes antigène-anticorps,
dans lequel le polypeptide OspC est choisi parmi le groupe constitué de : résidu d'acide aminé 19 à résidu d'acide aminé 211, résidu d'acide aminé 19 à 204, et résidu d'acide aminé 19 à 213, la numérotation des acides aminés étant basée sur le polypeptide OspC de SEQ ID NO :30, et
le polypeptide OspA est choisi parmi le groupe constitué de : résidus d'acide aminé 18 à 273, et résidus d'acide aminé 132 à 273, la numérotation des acides aminés étant basée sur le polypeptide OspA de SEQ ID NO :7, ou
le polypeptide OspA est un polypeptide chimère comportant des polypeptides OspA provenant d'au moins deux souches différentes de *Borrelia burgdorferi.*

20. Procédé selon la revendication 19, dans lequel :
(a) la protéine chimère est codée par un acide nucléique choisi parmi le groupe constitué des SEQ ID NOs : 145, 147, 149, 151, 153, 155, 157, 159, 161, 163, 165, 167, 169, 171, 173, 175, 177 et 179, ou
(b) la protéine chimère comporte une séquence en acides aminés choisie parmi le groupe constitué des SEQ ID NOs : 146, 148, 150, 152, 154, 156, 158, 160, 162, 164, 166, 168, 170, 172, 174, 176, 178 et 180.

21. Utilisation d'un acide nucléique qui code une protéine chimère non lipidée comportant un premier et un second polypeptide, dans laquelle le premier polypeptide comporte OspC de *Borrelia burgdorferi* et dans laquelle le second polypeptide comporte OspA de *Borrelia burgdorferi,* de sorte que OspC correspond à la partie N-terminale de la protéine, pour la fabrication d'une composition destinée à être utilisée pour immuniser un animal contre la Maladie de Lyme, dans laquelle : (a) le polypeptide OspC est choisi parmi le groupe constitué de : résidu d'acide aminé 19 à résidu d'acide aminé 211, résidu d'acide aminé 19 à 204, et résidu d'acide aminé 19 à 213, la numérotation des acides aminés étant basée sur le polypeptide OspC de SEQ ID NO :30, et
le polypeptide OspA est soit :
(i) choisi parmi le groupe constitué de : résidu d'acide aminé 18 à 273, et résidu d'acide aminé 132 à 273, la numérotation des acides aminés étant basée sur le polypeptide OspA de SEQ ID NO :7, ou
(ii) le polypeptide OspA est un polypeptide chimère comportant des polypeptides OspA provenant d'au moins deux souches différentes de *Borrelia burgdorferi,* et de manière facultative
dans laquelle l'acide nucléique codant la protéine chimère est choisi parmi le groupe constitué des SEQ ID NOs :145, 147, 149, 151, 153, 155, 157, 159, 161, 163, 165, 167, 169, 171, 173, 175, 177 et 179.

22. Composition physiologique pour la vaccination et le traitement contre une infection par *Borrelia* chez des animaux ou êtres humains, la composition comportant
a) soit (i) une protéine chimère non lipidée comportant au moins un premier et un second polypeptide, dans laquelle le premier polypeptide comporte OspC de *Borrelia burgdorferi* et dans laquelle le second polypeptide comporte OspA de *Borrelia burgdorferi,* de sorte que OspC correspond à la partie N-terminale de la protéine, dans laquelle le polypeptide OspC est choisi parmi le groupe constitué de : résidu d'acide aminé 19 à résidu d'acide aminé 211, résidus d'acide aminé 19 à 204, et résidus d'acide aminé 19 à 213, la numérotation des acides aminés étant basée sur le polypeptide OspC de SEQ ID NO :30, et le polypeptide OspA est choisi soit parmi le groupe constitué de : résidus d'acide aminé 18 à 273, et résidus d'acide aminé 132 à 273, la numérotation des acides aminés étant basée sur le polypeptide OspA de SEQ ID NO :7, ou un polypeptide chimère comportant des polypeptides OspA provenant d'au moins deux souches différentes de *Borrelia burgdorferi,* la quantité étant efficace pour provoquer une réponse immunitaire envers *Borrelia burgdorferi,* ou (ii) un acide nucléique codant ladite protéine chimère,
b) un support ou véhicule physiologiquement acceptable, et de manière facultative
c) un adjuvant.

23. Composition selon la revendication 22, dans laquelle :
(a) la protéine chimère est choisie parmi le groupe constitué des SEQ ID NOs : 146, 148, 150, 152, 154, 156, 158, 160, 162, 164, 166, 168, 170, 172, 174, 176, 178 et 180, et/ou
(b) l'acide nucléique est choisi parmi le groupe constitué des SEQ ID NOs : 145, 147, 149, 151, 153, 155, 157, 159, 161, 163, 165, 167, 169, 171, 173, 175, 177 et 179.

24. Kit comportant :
a) une protéine chimère non lipidée comportant au moins un premier et un second polypeptide, dans lequel le premier polypeptide comporte OspC de *Borrelia burgdorferi* et dans lequel le second polypeptide comporte OspA de *Borrelia burgdorferi,* de sorte que OspC correspond à la partie N-terminale de la protéine, dans lequel le polypeptide OspC est choisi parmi le groupe constitué de : résidu d'acide aminé 19 à résidu d'acide aminé 211, résidus d'acide aminé 19 à 204, et résidus d'acide aminé 19 à 213, la numérotation des acides aminés étant basée sur le polypeptide OspC de SEQ ID NO :30, et le polypeptide OspA est soit : (i) choisi parmi le groupe constitué de : résidus d'acide aminé 18 à 273, et résidus d'acide aminé 132 à 273, la numérotation des acides aminés étant basée sur le polypeptide OspA de SEQ ID NO :7, ou (ii) un polypeptide chimère comportant des polypeptides OspA provenant d'au moins deux souches différentes de *Borrelia burgdorferi,* et
b) des réactifs pour détecter des complexes anticorps-antigène formés entre la protéine chimère et des anticorps, éventuellement présents dans un échantillon hôte fourni par l'utilisateur.

25. Kit selon la revendication 24, dans lequel la protéine chimère est choisie parmi le groupe constitué des SEQ ID NOs : 146, 148, 150, 152, 154, 156, 158, 160, 162, 164, 166, 168, 170, 172, 174, 176, 178 et 180.
